# EUROPEAN PATENT APPLICATION

(11) **EP 3 862 023 A1**
(43) Date of publication of application: **11.08.2021**
(21) Application number: 21155131.2
(22) Date of filing: 04.02.2021
(51) Int. Cl.: A61K 47/68, A61P 35/00

(54) **CONJUGATES OF CELL-BINDING MOLECULES WITH CYTOTOXIC AGENTS**

(30) Priority: 05.02.2020 US 202016782456
(71) Applicant: Hangzhou DAC Biotech Co, Ltd, Hangzhou 310018 (CN)
(72) Inventor: ZHAO, Robert Yongxin, Lexington, Massachusetts 02421 (US); ZHANG, Yue, Hangzhou City, Zhejiang Province Province 310018 (CN); MA, Yourang, Hangzhou City, Zhejiang Province 310018 (CN)
(74) Representative: Haseltine Lake Kempner LLP

(57) **Abstract**

A conjugate of a potent cytotoxic agent with a cell-binding molecule having a structure represented by Formula (I), wherein T, L, m, n, -----, R¹, R², R³, R⁴, R⁵, R⁶, R⁷, R⁸, R⁹, R¹⁰, R¹¹, R¹², and R¹³ are as defined herein, can be used for targeted treatment of cancer, autoimmune disease, and infectious disease.

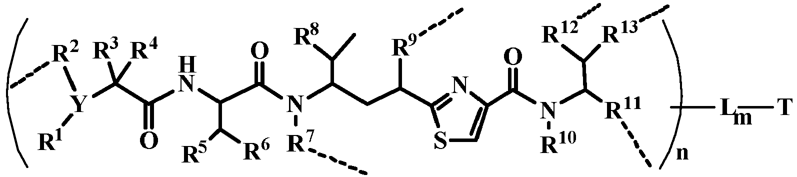

## Description

### CROSS REFERENCE OF RELATED APPLICATIONS

This application is a continuation-in-part of U.S. patent application Ser. No. 16/407,367, filed on May 9, 2019, entitled "CONJUGATES OF CELL BINDING MOLECULES WITH CYTOTOXIC AGENTS," which in turn is a continuation of U.S. patent application Ser. No. 14/253,881, filed on April 16, 2014, now U.S. Patent No. 10,399,941, which in turn is a continuation of PCT/IB2012/053554, filed on July 12, 2012. The entire content of each of the prior applications is hereby incorporated by reference.FIELD OF THE INVENTION

This invention relates to conjugates of potent antimitotic agents with a cell-surface receptor binding molecules for targeted therapy. The invention also relates to use of the compositions comprising binding molecule-antimitotic agent conjugates for treating cancer, autoimmune disease, and infectious disease.

### BACKGROUND OF THE INVENTION

The targeted delivery of highly active cytotoxic drugs by antibodies or other cell-surface receptor binding agents to specific sites of disease in human body, which in turn can dramatically increase therapeutic windows of the cytotoxic drugs, has proven a particularly promising approach for targeted treatment (Van den Mooter, T. et al Expert Opin Biol Ther. 2015, 15, 749-60). In particular, since US FDA approvals of Adcetris (brentuximab vedotin) in 2011 and Kadcyla (ado-trastuzumab emtansine) in 2013, almost every major pharmaceutical and biotech company has adopted the applications of antibody-drug conjugate (ADC) for targeted treatment of cancers (Chari, R. et al, Angew. Chem., Int. Ed. 2014, 53, 3796-3827; Sievers, E. L. et al. Annu Rev Med. 2013, 64, 15-29; Mehrling, T. Future Oncol, 2015, 11, 549). So far, the majority of ADCs in clinical evaluation utilize the highly potent tubulin-interacting agents, maytansinoids or auristatins. A few ADCs in the clinic have incorporated other potent effector molecules, such as the topoisomerase 1 inhibitor SN-38 or the DNA interacting agents calicheamicin and pyrrolobenzodiazepines (Anderl, J. et al, Methods Mol Biol. 2013;1045:51-70; Thomas, A., et al, Lancet Oncol. 2016 Jun;17(6):e254-e262).

Several short peptidic compounds that found to have biological activity have been isolated from natural sources. One of them, Tubulysins (structures shown below), which were the first time isolated by Hofle and Reichenbach et al. (GBF Braunschweig) from a culture browth of the myxobacterial strains of Archangium gephyra (F. Sasse et al. J. Antibiot. 2000, 53, 879-885; WO9813375), are members of group of antimitotic peptides that inhibit tubulin polymerization in dividing cells, and thus inducing apoptosis. With the exceptional potency exceeding that of vinblastine, taxol and epothilones (Wipf, et al, Org. Lett. 2004, 6, 4057-60; Peltier, et al, J. Am.

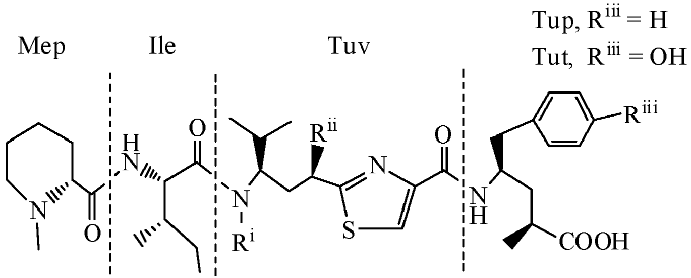

| Tubulysin | Rⁱ | Rⁱⁱ | Rⁱⁱⁱ |
|---|---|---|---|
| A | CH₂OCOCH₂CH(CH₃)₂ | OCOCH₃ | OH |
| B | CH₂OCOCH₂CH₂CH₃ | OCOCH₃ | OH |
| C | CH₂OCOCH₂CH₃ | OCOCH₃ | OH |
| D | CH₂OCOCH₂CH(CH₃)₂ | OCOCH₃ | H |
| E | CH₂OCOCH₂CH₂CH₃ | OCOCH₃ | H |
| F | CH₂OCOCH₂CH₃ | OCOCH₃ | H |
| G | CH₂OCOCH=CH₂ | OCOCH₃ | OH |
| H | CH₂OCOCH₃ | OCOCH₃ | H |
| I | CH₂OCOCH₃ | OCOCH₃ | OH |
| U | H | OCOCH₃ | H |
| V | H | OH | H |
| Z | H | OH | OH |
| Pretubulysin | CH₃ | H | H |

(The structures of existing tubulysin compounds)

Chem. Soc. 2006, 128, 16018-9; Wipf, et al, Org. Lett., 2007, 9, 1605-1607; Wang, et al, Chem. Biol. Drug Des. 2007, 70, 75-86; Pando, et al, Org. Lett. 2009, 11, 5567-9), these antimitotic peptides are exciting leads for targeted therapies. Structurally, the tetrapeptide tubulysins are comprising of *N*-methylpipecolinic acid (Mep) at the N-terminus, isoleucine (Ile) as the second residue, the unique thiazole-containing tubuvaline (Tuv) as the third residue, and two possible γ-amino acids at the C-terminus (tubutyrosine (Tut) or tubuphenylalanine (Tup)). Despite several tubulysins have recently been synthesized, significant general toxicities (>20% animal body weight loss) of the existing tubulysins at doses required for achieving a therapeutic effect compromise their efficacy (US Patent appl. 2010/0048490). We have been interested in the art of a conjugate of a cell surface binding ligand, particularly using an antibody to conjugate with tubulysin derivatives for having significantly lower general toxicity, yet useful therapeutic efficiency. Although the natural tubulysins are ideal payloads for ADCs with their extreme potency in tens picomolar ranges of IC₅₀ values against many cell lines, we found that the natural tubulysin conjugates were hardly metabolized in animal livers, resulting in severe liver toxicity. A simpler analog, such as using 2-(dimethylamino)-2-methylpropanoic acid to replace 1-methylpiperidine-2-carboxylic acid at the far left side of natural tubulysin structures did not alternate much potency of the compounds conjugated to an antibody, but reduced significant liver toxicity of the conjugates. Here this patent discloses these tubulysin conjugates with a cell surface binding ligand and using these conjugates for treating cancer and immune disorders.

### SUMMARY OF THE INVENTION

In one illustrative embodiment of the invention provides a conjugate of formula (I): 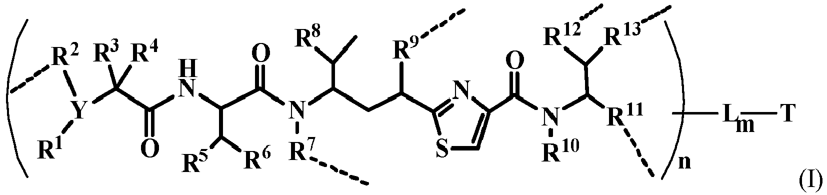
or their pharmaceutically acceptable salts, hydrates, or hydrated salts; or the polymorphic crystalline structures of these compounds; or their optical isomers, racemates, diastereomers or enantiomers thereof;
Wherein T is a targeting or binding ligand; L is a releasable linker; ----- is a linkage bond that L connects to an atom inside the bracket independently; n is 1-20 and m is 1-10;
Inside the bracket is a potent antimitotic agent wherein R¹, R², R³, and R⁴ are independently linear or branched C₁-C₈ of alkyl, alkylalcohol; C₂-C₈ of heteroalkyl, alkylcycloalkyl, heterocycloalkyl, alkyl ether, alkyl carboxylate, alkyl amine, alkyl ester, alkyl amide; C₃-C₈ of aryl, Ar-alkyl, heterocyclic, carbocyclic, cycloalkyl, heteroalkylcycloalkyl, alkylcarbonyl; or two R's: R¹R², R³R⁴, R⁵R⁶, or R¹²R¹³ independently together form a 3~7 membered carbocyclic, cycloalkyl, heterocyclic, heterocycloalkyl, aromatic or heteroaromatic ring system; Y is N or C; In addition, R¹, R², R³, and R⁴ can be independently absent;
Wherein R⁵, R⁶, R⁸ and R¹⁰ are independently selected from H and linear or branched Ci-C₄ of alkyl or C₂-C₄ of heteroalkyl;
Wherein R⁷ is selected from H, R¹⁴, or -R¹⁴C(=O)X¹R¹⁵; -R¹⁴X¹R¹⁵; X¹ is selected from O, S, S-S, NH, or NR¹⁴;
Wherein R⁹ is H, -O-, -OR¹⁴, -OC(=O)R¹⁴-, -OC(=O)NHR¹⁴-, -OC(=O)NR¹⁴R¹⁵-, -OC(=O) R¹⁴SSR¹⁵-, OP(=O)(OR¹⁴)-, or OR¹⁴OP(=O)(OR¹⁵);
Wherein R¹¹ is H, R¹⁴, -R¹⁴C(=O)R¹⁶, -R¹⁴C(=O)X²R¹⁶, -R¹⁴X²R¹⁶, -R¹⁴C(=O)X², wherein X² is -O-, -S-, -NH-, -NHS(O₂), -N(R¹⁴)-, -O-R¹⁴-, -S-R¹⁴-, -S(=O)-R¹⁴-, or -NHR¹⁴-;
Wherein R¹² is H, R¹⁴, -O-, -S-, -N-, =N-, =NNH-, -OH, -SH, -NH₂, =NH, =NNH₂,-NH(R¹⁴), -OR¹⁴, -C(O)O-, -C(O)OR¹⁶-,-COR¹⁶, -COOR¹⁴-, C(O)NH-, C(O)NH₂, C(O)NHR¹⁴,-SR¹⁴, -S(=O)R¹⁴, -P(=O)(OR¹⁶)₂, -OP(=O)(OR¹⁶)₂, -CH₂OP(=O)(OR¹⁶)₂, -SO₂R¹⁶;
Wherein R¹³ is linear or branched C₁-C₁₀ of alkyl, alkyl acid, alkyl amide, alkyl amine; or C₂-C₁₀ of heteroalkyl; or C₃-C₁₀ of Ar; Ar refers to an aromatic or hetero aromatic group, composed of one or several rings, comprising four to ten carbon atoms, preferentially four to six carbon atoms. The term of hetero aromatic group refers to an aromatic group that has one or several carbon atoms replaced by hetero atoms, preferentially one, two or three carbon atoms replaced by O, N, Si, Se, P or S, more preferentially O, S, N. The term aryl or Ar also refers to an aromatic group, wherein one or several H atoms can be replaced independently by R¹⁷, F, Cl, Br, I, OR¹⁶, SR¹⁶, NR¹⁶R¹⁷, N=NR¹⁶, N= R¹⁶, NR¹⁶R¹⁷, NO₂, SOR¹⁶R¹⁷, SO₂R¹⁶, SO₃R¹⁶, OSO₃R¹⁶, PR¹⁶R¹⁷, POR¹⁶R¹⁷, PO₂R¹⁶R¹⁷, OP(O)(OR¹⁷)₂, OCH₂OP(O)(OR¹⁷)₂, OC(O)OP(O)(OR¹⁷)₂, PO(OR¹⁶)(OR¹⁷), OP(O)(OR¹⁷)OP(O)(OR¹⁷)₂, OC(O)R¹⁷ or OC(O)NHR¹⁷;
Wherein R¹⁴ and R¹⁵ are independently H; linear or branched C₁-C₈ of alkyl; C₂-C₈ of alkenyl, alkynyl, heteroalkyl, heterocyclic, carbocyclic; C₃-C₈ of aryl, cycloalkyl, alkylcycloalkyl, heterocycloalkyl, heteroaralkyl, heteroalkylcycloalkyl, alkylcarbonyl;
Wherein when R¹⁴ is bivalent, it is a R¹⁴ that is further connected to an additional functional group of one to four amino acid units, or (CH₂CH₂O)ᵣ, r is an integer ranging from 0 to 12, or C₄-C₁₂ of glycosides, or C₁-C₈ of carboxylic acid;
Wherein R¹⁶ is H, OH, R¹⁴ or one to four amino acid units;
Wherein R¹⁷ is H, linear or branched C₁-C₈ of alkyl; C₂-C₈ of alkenyl, alkynyl, heteroalkyl, heterocyclic; C₃-C₈ of aryl, carbocyclic, cycloalkyl, alkylcycloalkyl, heterocycloalkyl, heteroalkylcycloalkyl, heteroaralkyl, alkylcarbonyl or C₄-C₁₂ of glycosides, or pharmaceutical salts.

In another embodiment, the linker L of the potent antimitotic agent- binding molecule conjugates has the formula: --Ww-(Aa)r--Vv-; wherein: --W-- is a Stretcher unit; w is 0 or 1; each --Aa-- is independently an Amino Acid unit; r is independently an integer ranging from 0 to 12; --V-- is a Spacer unit; and v is 0, 1 or 2. The Stretcher unit W may independently contain a self-immolative spacer, peptidyl units, a hydrazone bond, disulfide or thiolether bonds.

In another embodiment, the cell-surface binding molecule T may be of any kind presently known, or which become known cell binding ligands, such as peptides and non-peptides. Generally the binding molecule T is an antibody; a single chain antibody; an antibody fragment that binds to the target cell; a monoclonal antibody; a single chain monoclonal antibody; or a monoclonal antibody fragment that binds the target cell; a chimeric antibody; a chimeric antibody fragment that binds to the target cell; a domain antibody; a domain antibody fragment that binds to the target cell; adnectins that mimic antibodies; DARPins; a lymphokine; a hormone; a vitamin; a growth factor; a colony stimulating factor; or a nutrient-transport molecule (a transferrin); a binding peptide, or protein, or antibody, or small molecule attached on albumin, polymers, dendrimers, liposomes, nanoparticles, vesicles, (viral) capsids. Preferably the binding molecule T is a monoclonal antibody.

In yet another aspect, a compound of formula I∼VII or a pharmaceutically acceptable salt or solvate thereof is used for treating cancer, an autoimmune disease or an infectious disease in a human or an animal.

### BRIEF DESCRIPTION OF THE DRAWINGS

Figure 1 shows the general synthesis of Tuv component of a Tubulysin analog.
Figure 2 shows the synthesis of tubulysin components.
Figure 3 shows the synthesis of tubulysin components.
Figure 4 shows the synthesis of components of tubulysin analogs.
Figure 5 shows the synthesis of components of tubulysin analogs.
Figure 6 shows the synthesis of components of tubulysin analogs.
Figure 7 shows the synthesis of components of tubulysin analogs.
Figure 8 shows the synthesis of components of tubulysin analogs containing a conjugate linker.
Figure 9 shows the synthesis of components of tubulysin analogs and their conjugations to an antibody.
Figure 10 shows the synthesis of components of a linker.
Figure 11 shows the synthesis of Tubulysin analogs containing a linker and their conjugations to an antibody.
Figure 12 shows the synthesis of Tubulysin analogs containing a linker and their conjugations to an antibody.
Figure 13 shows the synthesis of Tubulysin analogs containing a linker and their conjugations to an antibody.
Figure 14 shows the synthesis of tubulysin analogs containing a linker and their conjugations to an antibody.
Figure 15 shows the synthesis of components of a linker and their linkage to Tubulysin analogs containing a linker as well the conjugation to an antibody.
Figure 16 shows the synthesis of components of Tup and Tuv analogs.
Figure 17 shows the synthesis of components of Tuv analogs..
Figure 18 shows the synthesis of tubulysin analogs containing a linker and their conjugations to an antibody.
Figure 19 shows the synthesis of tubulysin analogs containing a linker and their conjugations to an antibody.
Figure 20 shows the synthesis of components of Tubulysin analogs.
Figure 21 shows the synthesis of components of Tubulysin analogs.

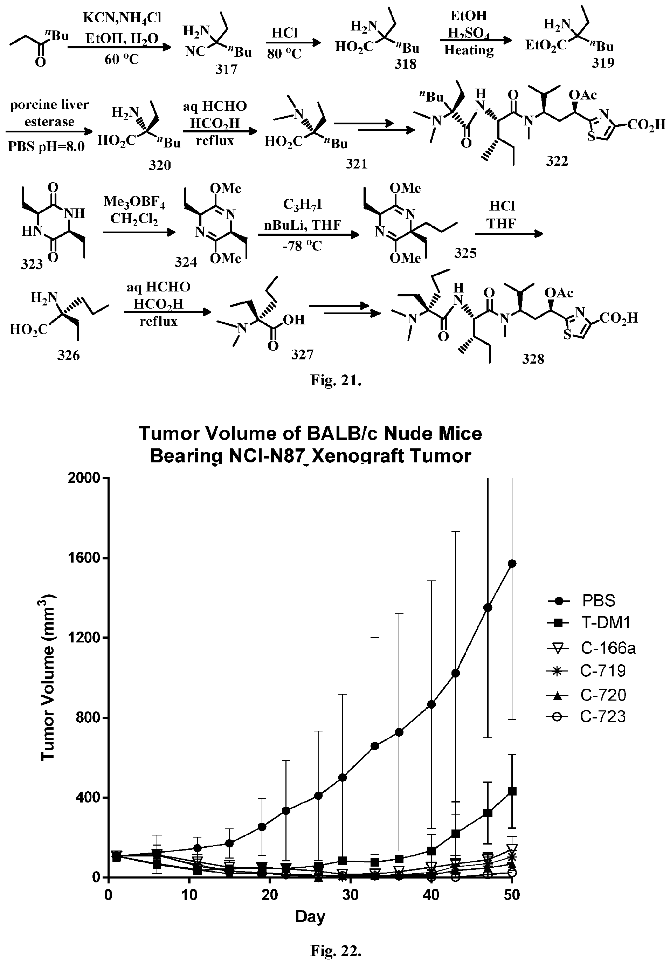

Figure 22 shows the comparison of the anti-tumor effect of conjugate compounds C-166a, C-719, C-720, and C-723 with T-DM1 using human gastric tumor N87 cell model, i.v., one injection at dosing of 6 mg/kg for conjugates C-166a, C-719, C-720, C-723 and T-DM1. Four conjugates tested here demonstrated better anti-tumor activity than T-DM1. All 6/6 animals at the groups of compounds C-166a, C-719, C-720, and C-723 had completely no tumor measurable at day 22 till day 36, and all of them can inhibit the tumor growth for over 48 days. In contrast T-DM1 at dose of 6 mg/Kg was not able to eliminate the tumors and it only inhibited the tumor growth for 31 days.
Figures 23 (a) and (b) shows an acute toxicity study on ADC conjugates T-DM1, C-166a, C-719, C-720, and C-723 through observing changes in body weight (BW) of mice treated with dose of 75 mg/Kg (Fig 24-a) and 150 mg/Kg (Fig 24-b) in 12 days. The body weight changes demonstrated that conjugate C-723 was more toxic at both doses than T-DM1; conjugate C-720 was similar toxic to T-DM1 at dose of 75 mg/Kg and less toxic than T-DM1 at dose of 150 mg/Kg; and both conjugate C-166a and conjugate C-719 are much less toxic than T-DM1 at both the tested doses.

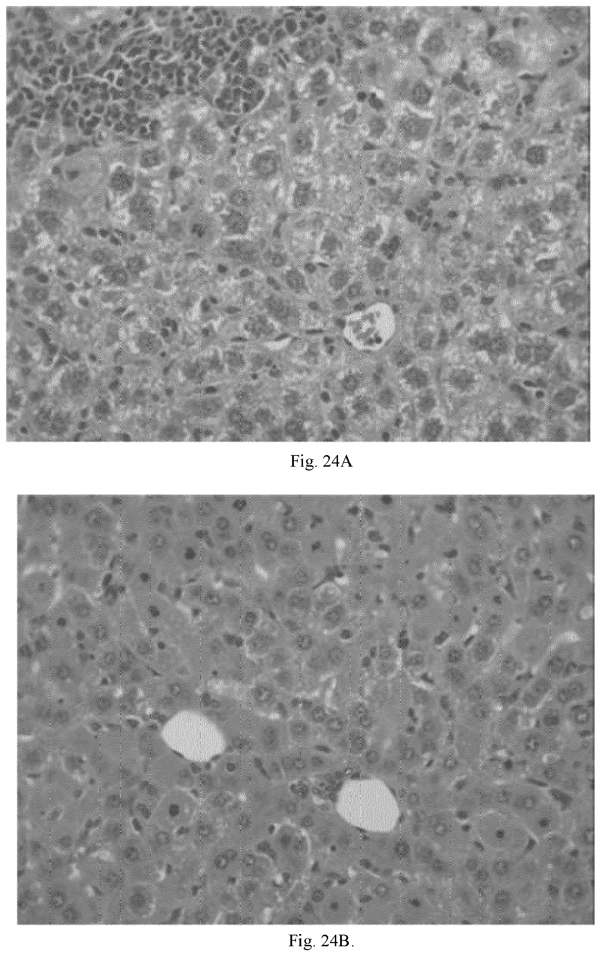

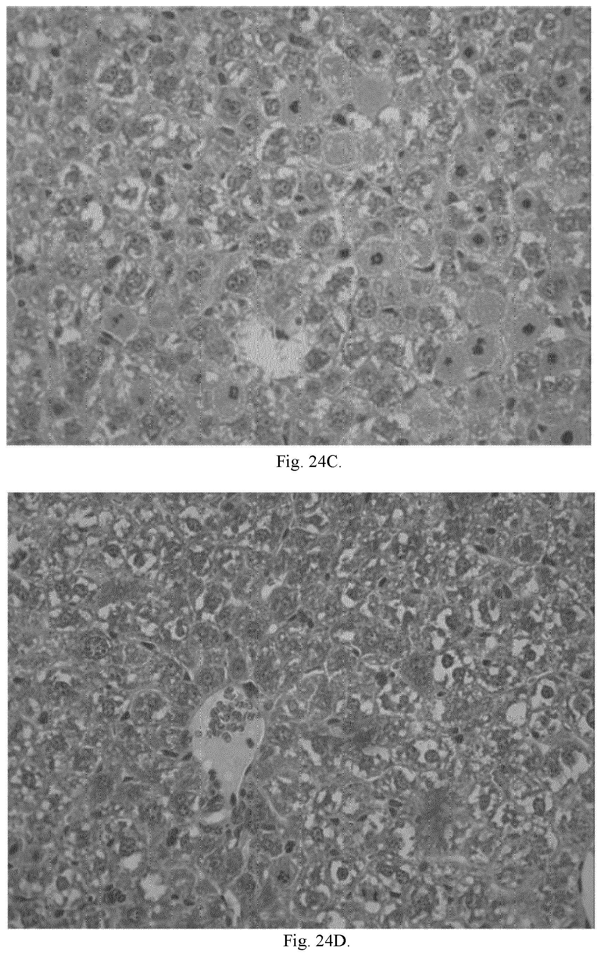

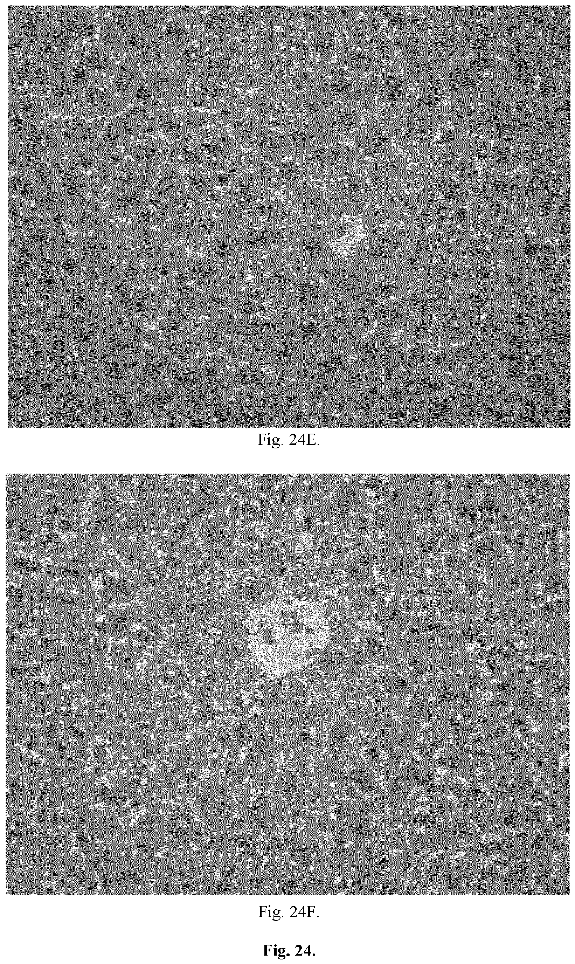

Figures 24A and 24B show the liver pathogen of the mice treated with dose of 75 mg/Kg of conjugate compounds T-DM1, C-166a, C-719, C-720, and C-723 in comparison with PBS buffer on day 5. The pictures were enlarged by 40 fold. As the pictures indicated: (1). T-DM1 group (in Fig. 24A), pathology of T-DM1 75mg/kg group indicated hepatocyte swelling and multifocal necrosis. The lobule structures were not clear. The central venules contained the swollen hepatocytes, red blood cells and red-colored remaining. The nucleuses of hepatocyte were in different sizes and stains. Hepatocytes exhibited the blurred boundaries, increased volume, and eosinophilic-stained plasma . Part of the liver nucleus disappeared. An obvious proliferative phase was seen; (2) In C-723 group (in Fig. 24B), scattered single cell necrosis and water-degeneration are main pathological behaviors. In swelling area, hepatic lobule structure is lost, and a large number of red blood cells are congested in the central venules. Hepatocytes are swollen, borderline unclear and eosinophilic staining. The nuclei vary in sizes and colors. Mild proliferation is observed. (3). Pathology in C-720 group (in Fig. 24C) exhibits the exudate in the central vein of the lobule, disorder in plates arrangement of hepatocytes and hepatocyte hyperplasia. Hypertrophies of Kupffer's cells were occasionally observed. (4). in both C-719 ((in Fig. 24D) and C-166a (in Fig. 24E) groups, hepatic lobular structure was slightly disordered. Hepatic sinuses were visible. Inflammatory cell infiltration was observed in the wall of the bile ducts. Hypertrophy of Kupffer's cells was rare. Hepatocytes were mildly swollen. The microscopic structure is similar to what was seen in the control PBS group (in Fig. 24F).

### DETAILED DESCRIPTION OF THE INVENTION

### DEFINITIONS

"Alkyl" refers to an aliphatic hydrocarbon group or univalent groups derived from alkane by removal of one or two hydrogen atoms from carbon atoms. It may be straight or branched having C₁-C₈ (1 to 8 carbon atoms) in the chain. "Branched" means that one or more lower C numbers of alkyl groups such as methyl, ethyl or propyl are attached to a linear alkyl chain. Exemplary alkyl groups include methyl, ethyl, *n*-propyl, *i*-propyl, *n*-butyl, *t*-butyl, *n*-pentyl, 3-pentyl, octyl, nonyl, decyl, cyclopentyl, cyclohexyl, 2,2-dimethylbutyl, 2,3-dimethylbutyl, 2,2-dimethylpentyl, 2,3-dimethylpentyl, 3,3-dimethylpentyl, 2,3,4-trimethylpentyl, 3-methyl-hexyl, 2,2-dimethylhexyl, 2,4-dimethylhexyl, 2,5-dimethylhexyl, 3,5-dimethylhexyl, 2,4-dimethylpentyl, 2-methylheptyl, 3-methylheptyl, *n*-heptyl, isoheptyl, *n*-octyl, and isooctyl. A C₁-C₈ alkyl group can be unsubstituted or substituted with one or more groups including, but not limited to, -C₁-C₈ alkyl,-O-(C₁-C₈ alkyl), -aryl, -C(O)R', -OC(O)R', -C(O)OR', -C(O)NH₂, -C(O)NHR', -C(O)N(R')₂, -NHC(O)R', -SR', -S(O)₂R', -S(O)R', -OH, -halogen, -N₃, -NH₂, -NH(R'), -N(R')₂ and -CN; where each R' is independently selected from -C₁-C₈ alkyl and aryl.

"Halogen" refers to fluorine, chlorine, bromine or iodine atom; preferably fluorine and chlorine atom.

"Heteroalkyl" refers to C₂-C₈ alkyl in which one to four carbon atoms are independently replaced with a heteroatom from the group consisting of O, S and N.

"Carbocycle" refers to a saturated or unsaturated ring having 3 to 8 carbon atoms as a monocycle or 7 to 13 carbon atoms as a bicycle. Monocyclic carbocycles have 3 to 6 ring atoms, more typically 5 or 6 ring atoms. Bicyclic carbocycles have 7 to 12 ring atoms, arranged as a bicycle [4,5], [5,5], [5,6] or [6,6] system, or 9 or 10 ring atoms arranged as a bicycle [5,6] or [6,6] system. Representative C₃-C₈ carbocycles include, but are not limited to, -cyclopropyl, - cyclobutyl, -cyclopentyl, -cyclopentadienyl, -cyclohexyl, -cyclohexenyl, -1,3-cyclohexadienyl, - 1,4-cyclohexadienyl, -cycloheptyl, -1,3-cycloheptadienyl, -1,3,5-cycloheptatrienyl, -cyclooctyl, and -cyclooctadienyl.

A "C₃-C₈ carbocycle" refers to a 3-, 4-, 5-, 6-, 7- or 8-membered saturated or unsaturated nonaromatic carbocyclic ring. A C₃-C₈ carbocycle group can be unsubstituted or substituted with one or more groups including, but not limited to, -C₁-C₈ alkyl,-O-(C₁-C₈ alkyl), -aryl, -C(O)R',-OC(O)R', -C(O)OR', -C(O)NH₂, -C(O)NHR', -C(O)N(R')₂, -NHC(O)R', -SR, -S(O)R',-S(O)₂R',-OH, -halogen, -N₃, -NH₂, -NH(R'), -N(R')₂ and -CN; where each R' is independently selected from -C₁-C₈ alkyl and aryl.

"Alkenyl" refers to an aliphatic hydrocarbon group containing a carbon-carbon double bond which may be straight or branched having 2 to 8 carbon atoms in the chain. Exemplary alkenyl groups include ethenyl, propenyl, n-butenyl, i-butenyl, 3-methylbut-2-enyl, n-pentenyl, hexylenyl, heptenyl, octenyl.

"Alkynyl" refers to an aliphatic hydrocarbon group containing a carbon-carbon triple bond which may be straight or branched having 2 to 8 carbon atoms in the chain. Exemplary alkynyl groups include ethynyl, propynyl, *n*-butynyl, 2-butynyl, 3-methylbutynyl, 5-pentynyl, *n*-pentynyl, hexylynyl, heptynyl, and octynyl.

"Alkylene" refers to a saturated, branched or straight chain or cyclic hydrocarbon radical of 1-18 carbon atoms, and having two monovalent radical centers derived by the removal of two hydrogen atoms from the same or two different carbon atoms of a parent alkane. Typical alkylene radicals include, but are not limited to: methylene (-CH₂-), 1,2-ethyl (-CH₂CH₂-), 1,3-propyl (-CH₂CH₂CH₂-), 1,4-butyl (-CH₂CH₂CH₂CH₂-), and the like.

"Alkenylene" refers to an unsaturated, branched or straight chain or cyclic hydrocarbon radical of 2-18 carbon atoms, and having two monovalent radical centers derived by the removal of two hydrogen atoms from the same or two different carbon atoms of a parent alkene. Typical alkenylene radicals include, but are not limited to: 1,2-ethylene (-CH=CH-).

"Alkynylene" refers to an unsaturated, branched or straight chain or cyclic hydrocarbon radical of 2-18 carbon atoms, and having two monovalent radical centers derived by the removal of two hydrogen atoms from the same or two different carbon atoms of a parent alkyne. Typical alkynylene radicals include, but are not limited to: acetylene, propargyl and 4-pentynyl.

"Aryl" or Ar refers to an aromatic or hetero aromatic group, composed of one or several rings, comprising three to fourteen carbon atoms, preferentially six to ten carbon atoms. The term of "hetero aromatic group" refers one or several carbon on aromatic group, preferentially one, two, three or four carbon atoms are replaced by O, N, Si, Se, P or S, preferentially by O, S, and N. The term aryl or Ar also refers to an aromatic group, wherein one or several H atoms are replaced independently by -R', -halogen, -OR', or -SR', -NR'R", -N=NR', -N=R', -NR'R",-NO₂,-S(O)R', -S(O)₂R', -S(O)₂OR', -OS(O)₂OR', -PR'R", -P(O)R'R", -P(OR')(OR"),-P(O)(OR')(OR") or -OP(O)(OR')(OR") wherein R', R" are independently H, alkyl, alkenyl, alkynyl, heteroalkyl, aryl, arylalkyl, carbonyl, or pharmaceutical salts.

"Heterocycle" refers to a ring system in which one to four of the ring carbon atoms are independently replaced with a heteroatom from the group of O, N, S, Se, B, Si and P. Preferable heteroatoms are O, N and S. Heterocycles are also described in The Handbook of Chemistry and Physics, 78th Edition, CRC Press, Inc., 1997-1998, p. 225 to 226, the disclosure of which is hereby incorporated by reference. Preferred nonaromatic heterocyclic include epoxy, aziridinyl, thiiranyl, pyrrolidinyl, pyrazolidinyl, imidazolidinyl, oxiranyl, tetrahydrofuranyl, dioxolanyl, tetrahydropyranyl, dioxanyl, dioxolanyl, piperidyl, piperazinyl, morpholinyl, pyranyl, imidazolinyl, pyrrolinyl, pyrazolinyl, thiazolidinyl, tetrahydrothiopyranyl, dithianyl, thiomorpholinyl, dihydropyranyl, tetrahydropyranyl, dihydropyranyl, tetrahydropyridyl, dihydropyridyl, tetrahydropyrimidinyl, dihydrothiopyranyl, azepanyl, as well as the fused systems resulting from the condensation with a phenyl group.

The term "heteroaryl" or aromatic heterocycles refers to a 3 to 14, preferably 5 to 10 membered aromatic hetero, mono-, bi-, or multi-cyclic ring. Examples include pyrrolyl, pyridyl, pyrazolyl, thienyl, pyrimidinyl, pyrazinyl, tetrazolyl, indolyl, quinolinyl, purinyl, imidazolyl, thienyl, thiazolyl, benzothiazolyl, furanyl, benzofuranyl, 1,2,4-thiadiazolyl, isothiazolyl, triazolyl, tetrazolyl, isoquinolyl, benzothienyl, isobenzofuryl, pyrazolyl, carbazolyl, benzimidazolyl, isoxazolyl, pyridyl-*N*-oxide, as well as the fused systems resulting from the condensation with a phenyl group.

"Alkyl", "cycloalkyl", "alkenyl", "alkynyl", "aryl", "heteroaryl", "heterocyclic" and the like refer also to the corresponding "alkylene", "cycloalkylene", "alkenylene", "alkynylene", "arylene", "heteroarylene", "heterocyclene" and the likes which are formed by the removal of two hydrogen atoms.

"Arylalkyl" refers to an acyclic alkyl radical in which one of the hydrogen atoms bonded to a carbon atom, typically a terminal or sp³ carbon atom, is replaced with an aryl radical. Typical arylalkyl groups include, benzyl, 2-phenylethan-1-yl, 2-phenylethen-1-yl, naphthylmethyl, 2-naphthylethan-1-yl, 2-naphthylethen-1-yl, naphthobenzyl, 2-naphthophenylethan-1-yl and the like.

"Heteroarylalkyl" refers to an acyclic alkyl radical in which one of the hydrogen atoms bonded to a carbon atom, typically a terminal or sp³ carbon atom, is replaced with a heteroaryl radical. Examples of heteroarylalkyl groups are 2-benzimidazolylmethyl, 2-furylethyl.

Examples of a "hydroxyl protecting group" includes, methoxymethyl ether, 2-methoxyethoxymethyl ether, tetrahydropyranyl ether, benzyl ether, *p*-methoxybenzyl ether, trimethylsilyl ether, triethylsilyl ether, triisopropylsilyl ether, *t*-butyldimethylsilyl ether, triphenylmethylsilyl ether, acetate ester, substituted acetate esters, pivaloate, benzoate, methanesulfonate and *p*-toluenesulfonate.

"Leaving group" refers to a functional group that can be substituted by another functional group. Such leaving groups are well known in the art, and examples include, a halide (e.g., chloride, bromide, and iodide), methanesulfonyl (mesyl), *p*-toluenesulfonyl (tosyl), trifluoromethylsulfonyl (triflate), and trifluoromethylsulfonate. A preferred leaving group is selected from nitrophenol; N-hydroxysuccinimide (NHS); phenol; dinitrophenol; pentafluorophenol; tetrafluorophenol; difluorophenol; monofluorophenol; pentachlorophenol; triflate; imidazole; dichlorophenol; tetrachlorophenol; 1-hydroxybenzotriazole; tosylate; mesylate; 2-ethyl-5-phenylisoxazolium-3'-sulfonate, anhydrides formed its self, or formed with the other anhydride, e.g. acetyl anhydride, formyl anhydride; or an intermediate molecule generated with a condensation reagent for peptide coupling reactions or for Mitsunobu reactions.

The following abbreviations may be used herein and have the indicated definitions: Boc, tert-butoxy carbonyl; BroP, bromotrispyrrolidinophosphonium hexafluorophosphate; CDI, 1,1'-carbonyldiimidazole; DCC, dicyclohexylcarbodiimide; DCE, dichloroethane; DCM, dichloromethane; DEAD is diethylazodicarboxylate, DIAD, diisopropylazodicarboxylate; DIBAL-H, diisobutyl-aluminium hydride; DIPEA or DEA, diisopropylethylamine; DEPC, diethyl phosphorocyanidate; DMA, N,N-dimethyl acetamide; DMAP, 4-(N, N-dimethylamino)pyridine; DMF, N,N-dimethylformamide; DMSO, dimethylsulfoxide; DTPA is diethylenetriaminepentaacetic acid; DTT, dithiothreitol; EDC, 1-(3-dimethylaminopropyl)-3-ethylcarbodiimide hydrochloride; ESI-MS, electrospray mass spectrometry; EtOAc is ethyl acetate; Fmoc is N-(9-fluorenylmethoxycarbonyl); HATU, O-(7-azabenzotriazol-1-yl)-N, N, N', N'-tetramethyluronium hexafluorophosphate; HOBt, 1-hydroxybenzotriazole; HPLC, high pressure liquid chromatography; NHS, N-Hydroxysuccinimide; MeCN is acetonitrile; MeOH is methanol; MMP, 4-methylmorpholine; PAB, p-aminobenzyl; PBS, phosphate-buffered saline (pH 7.0∼7.5); Ph is phenyl; phe is L-phenylalanine; PyBrop is bromo-tris-pyrrolidino-phosphonium hexafluorophosphate; PEG, polyethylene glycol; SEC, size-exclusion chromatography; TCEP, tris(2-carboxyethyl)phosphine; TFA, trifluoroacetic acid; THF, tetrahydrofuran; Val, valine; TLC is thin layer chromatography; UV is ultraviolet.

The "amino acid(s)" can be natural and/or unnatural amino acids, preferably alpha-amino acids. Natural amino acids are those encoded by the genetic code, which are alanine, arginine, asparagine, aspartic acid, cysteine, glutamic acid, glutamine, glycine, histidine, isoleucine, leucine, lysine, methionine, phenylalanine, proline, serine, threonine, tyrosine. tryptophan and valine. The unnatural amino acids are derived forms of proteinogenic amino acids. Examples include hydroxyproline, lanthionine, 2-aminoisobutyric acid, dehydroalanine, gamma-aminobutyric acid (the neurotransmitter), ornithine, citrulline, beta alanine (3-aminopropanoic acid), gamma-carboxyglutamate, selenocysteine (present in many noneukaryotes as well as most eukaryotes, but not coded directly by DNA), pyrrolysine (found only in some archaea and one bacterium), N-formylmethionine (which is often the initial amino acid of proteins in bacteria, mitochondria, and chloroplasts), 5-hydroxytryptophan, L-dihydroxyphenylalanine, triiodothyronine, L-3,4-dihydroxyphenylalanine (DOPA), and O-phosphoserine. The term amino acid also includes amino acid analogs and mimetics. Analogs are compounds having the same general H₂N(R)CHCO₂H structure of a natural amino acid, except that the R group is not one found among the natural amino acids. Examples of analogs include homoserine, norleucine, 3-aminopropanoic acid, 4-aminobutanoic acid, 5-aminopentanoic acid, 6-aminohexanoic acid, 7-aminoheptanoic acid, methionine-sulfoxide, and methionine methyl sulfonium. Preferably, an amino acid mimetic is a compound that has a structure different from the general chemical structure of an alpha-amino acid but functions in a manner similar to one. The term "unnatural amino acid" is intended to represent the "D" stereochemical form, the natural amino acids being of the "L" form. When 1∼8 amino acids are used in this patent application, amino acid sequence is then preferably a cleavage recognition sequence for a protease. Many cleavage recognition sequences are known in the art. See, e.g., Matayoshi et al. Science 247: 954 (1990); Dunn et al. Meth. Enzymol. 241: 254 (1994); Seidah et al. Meth. Enzymol. 244: 175 (1994); Thornberry, Meth. Enzymol. 244: 615 (1994); Weber et al. Meth. Enzymol. 244: 595 (1994); Smith et al. Meth. Enzymol. 244: 412 (1994); and Bouvier et al. Meth. Enzymol. 248: 614 (1995); the disclosures of which are incorporated herein by reference. In particular, the sequence is selected from the group consisting of Val-Cit, Ala-Val, Ala-Ala, Val-Val, Val-Ala-Val, Lys-Lys, Ala-Asn-Val, Val-Leu-Lys, Cit-Cit, Val-Lys, Ala-Ala-Asn, Asp-Lys, Asp-Glu, Glu-Lys, Lys, Cit, Ser, and Glu.

The "glycoside" is a molecule in which a sugar group is bonded through its anomeric carbon to another group via a glycosidic bond. Glycosides can be linked by an O- (an O-glycoside), N- (a glycosylamine), S-(a thioglycoside), or C- (a C-glycoside) glycosidic bond. Its core the empirical formula is C*ₘ*(H₂O)*ₙ* (where *m* could be different from *n,* and m and n are < 36), Glycoside herein includes glucose (dextrose), fructose (levulose) allose, altrose, mannose, gulose, iodose, galactose, talose, galactosamine, glucosamine, sialic acid, *N*-acetylglucosamine, sulfoquinovose (6-deoxy-6-sulfo-D-glucopyranose), ribose, arabinose, xylose, lyxose, sorbitol, mannitol, sucrose, lactose, maltose, trehalose, maltodextrins, raffinose, Glucuronic acid (glucuronide), and stachyose. It can be in D form or L form, 5 atoms cyclic furanose forms, 6 atoms cyclic pyranose forms, or acyclic form, α-isomer (the -OH of the anomeric carbon below the plane of the carbon atoms of Haworth projection), or a β-isomer (the -OH of the anomeric carbon above the plane of Haworth projection). It is used herein as a monosaccharide, disaccharide, polyols, or oligosaccharides containing 3-6 sugar units.

The term "antibody," as used herein, refers to a full-length immunoglobulin molecule or an immunologically active portion of a full-length immunoglobulin molecule, i.e., a molecule that contains an antigen binding site that immunospecifically binds an antigen of a target of interest or part thereof, such targets including but not limited to, cancer cell or cells that produce autoimmune antibodies associated with an autoimmune disease. The immunoglobulin disclosed herein can be of any type (e.g. IgG, IgE, IgM, IgD, IgA and IgY), class (e.g., IgG1, IgG2, IgG3, IgG4, IgA1 and IgA2) or subclass of immunoglobulin molecule. The immunoglobulins can be derived from any species. Preferably, however, the immunoglobulin is of human, murine, or rabbit origin. Antibodies useful in the invention are preferably monoclonal, and include, but are not limited to, polyclonal, monoclonal, bispecific, human, humanized or chimeric antibodies, single chain antibodies, Fv, Fab fragments, F(ab') fragments, F(ab')₂ fragments, fragments produced by a Fab expression library, anti-idiotypic (anti-Id) antibodies, CDR's, and epitope-binding fragments of any of the above which immunospecifically bind to cancer cell antigens, viral antigens or microbial antigens.

An "enantiomer", also known as an "optical isomer", is one of two stereoisomers that are mirror images of each other that are non-superposable (not identical), much as one's left and right hands are the same except for being reversed along one axis (the hands cannot be made to appear identical simply by reorientation). A single chiral atom or similar structural feature in a compound causes that compound to have two possible structures which are non-superposable, each a mirror image of the other. The presence of multiple chiral features in a given compound increases the number of geometric forms possible, though there may be some perfect-mirror-image pairs. Enantiopure compounds refer to samples having, within the limits of detection, molecules of only one chirality. When present in a symmetric environment, enantiomers have identical chemical and physical properties except for their ability to rotate plane-polarized light (+/-) by equal amounts but in opposite directions (although the polarized light can be considered an asymmetric medium). They are sometimes called optical isomers for this reason. A mixture of *equal parts* of an optically active isomer and its enantiomer is termed racemic and has zero net rotation of plane-polarized light because the positive rotation of each (+) form is exactly counteracted by the negative rotation of a (-) one. Enantiomer members often have different chemical reactions with other enantiomer substances. Since many biological molecules are enantiomers, there is sometimes a marked difference in the effects of two enantiomers on biological organisms. In drugs, for example, often only one of a drug's enantiomers is responsible for the desired physiologic effects, while the other enantiomer is less active, inactive, or sometimes even productive of adverse effects. Owing to this discovery, drugs composed of only one enantiomer ("enantiopure") can be developed to enhance the pharmacological efficacy and sometimes eliminate some side effects.

Isotopes are variants of a particular chemical element which differs in neutron number. All isotopes of a given element have the same number of protons in each atom. Each atomic number identifies a specific element, but not the isotope; an atom of a given element may have a wide range in its number of neutrons. The number of nucleons (both protons and neutrons) in the nucleus is the atom's mass number, and each isotope of a given element has a different mass number. For example, carbon-12, carbon-13 and carbon-14 are three isotopes of the element carbon with mass numbers 12, 13 and 14 respectively. The atomic number of carbon is 6, which means that every carbon atom has 6 protons, so that the neutron numbers of these isotopes are 6, 7 and 8 respectively. Hydrogen atom has three isotopes of protium (¹H), deuterium (²H), and tritium (³H), which deuterium has twice the mass of protium and tritium has three times the mass of protium. Isotopic substitution can be used to determine the mechanism of a chemical reaction and via the kinetic isotope effect. Isotopic substitution can be used to study how the body affects a specific xenobiotic/chemical after administration through the mechanisms of absorption and distribution, as well as the metabolic changes of the substance in the body (e.g. by metabolic enzymes such as cytochrome P450 or glucuronosyltransferase enzymes), and the effects and routes of excretion of the metabolites of the drug. This study is called pharmacokinetics (PK). Isotopic substitution can be used to study of the biochemical and physiologic effects of drugs. The effects can include those manifested within animals (including humans), microorganisms, or combinations of organisms (for example, infection). This study is called pharmacodynamics (PD). The effects can include those manifested within animals (including humans), microorganisms, or combinations of organisms (for example, infection). Both together influence dosing, benefit, and adverse effects of the drug. isotopes can contain a stable (non-radioactive) or an unstable element. Isotopic substitution of a drug may have a different thrapeutical efficacy of the original drug.

"Pharmaceutically" or "pharmaceutically acceptable" refer to molecular entities and compositions that do not produce an adverse, allergic or other untoward reaction when administered to an animal, or a human, as appropriate.

"Pharmaceutically acceptable solvate" or "solvate" refer to an association of one or more solvent molecules and a disclosed compound. Examples of solvents that form pharmaceutically acceptable solvates include, but are not limited to, water, isopropanol, ethanol, methanol, DMSO, ethyl acetate, acetic acid and ethanolamine.

"Pharmaceutically acceptable excipient" includes any carriers, diluents, adjuvants, or vehicles, such as preserving or antioxidant agents, fillers, disintegrating agents, wetting agents, emulsifying agents, suspending agents, solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions as suitable therapeutic combinations.

As used herein, "pharmaceutical salts" refer to derivatives of the disclosed compounds wherein the parent compound is modified by making acid or base salts thereof. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, tartaric, citric, methanesulfonic, benzenesulfonic, glucuronic, glutamic, benzoic, salicylic, toluenesulfonic, oxalic, fumaric, maleic, lactic and the like. Further addition salts include ammonium salts such as tromethamine, meglumine, epolamine, etc., metal salts such as sodium, potassium, calcium, zinc or magnesium.

The pharmaceutical salts of the present invention can be synthesized from the parent compound which contains a basic or acidic moiety by conventional chemical methods. Generally, such salts can be prepared via reaction the free acidic or basic forms of these compounds with a stoichiometric amount of the appropriate base or acid in water or in an organic solvent, or in a mixture of the two. Generally, non-aqueous media like ether, ethyl acetate, ethanol, isopropanol, or acetonitrile are preferred. Lists of suitable salts are found in Remington's Pharmaceutical Sciences, 17th ed., Mack Publishing Company, Easton, PA, 1985, p. 1418, the disclosure of which is hereby incorporated by reference.

"Administering" or "administration" refers to any mode of transferring, delivering, introducing or transporting a pharmaceutical drug or other agent to a subject. Such modes include oral administration, topical contact, intravenous, intraperitoneal, intramuscular, intralesional, intranasal, subcutaneous or intrathecal administration. Also contemplated by the present invention is utilization of a device or instrument in administering an agent. Such device may utilize active or passive transport and may be slow-release or fast-release delivery device.

In the context of cancer, the term "treating" includes any or all of: preventing growth of tumor cells or cancer cells, preventing replication of tumor cells or cancer cells, lessening of overall tumor burden and ameliorating one or more symptoms associated with the disease.

In the context of an autoimmune disease, the term "treating" includes any or all of: preventing replication of cells associated with an autoimmune disease state including, but not limited to, cells capable of producing an autoimmune antibody, lessening the autoimmune-antibody burden and ameliorating one or more symptoms of an autoimmune disease.

In the context of an infectious disease, the term "treating" includes any or all of: preventing the growth, multiplication or replication of the pathogen that causes the infectious disease and ameliorating one or more symptoms of an infectious disease.

Examples of a "mammal" or "animal" include, but are not limited to, a human, rat, mouse, guinea pig, monkey, pig, goat, cow, horse, dog, cat, bird and fowl.

### DRUG-LINKER- BINDING LIGAND CONJUGATES

As stated above, this invention provides a cell surface binding molecule - antimitotic (cytotoxic) agent conjugate of Formula (I): 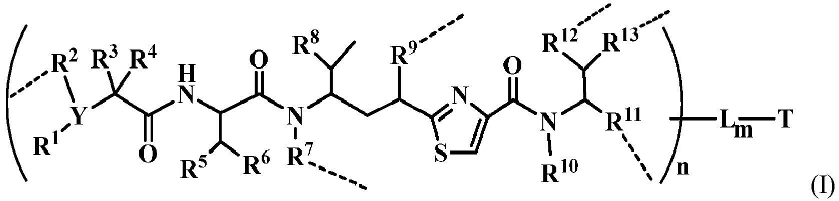
or their pharmaceutically acceptable salts, hydrates, or hydrated salts; or the polymorphic crystalline structures of these compounds; or their optical isomers, racemates, diastereomers or enantiomers thereof;
Wherein T is a targeting or binding ligand; L is a releasable linker; ----- is a linkage bond that L connects to an atom inside the bracket independently; n is 1∼20 and m is 1∼10;
Inside the bracket is a potent antimitotic agent wherein R¹, R², R³, and R⁴ are independently linear or branched C₁-C₈ of alkyl, alkylalcohol; C₂-C₈ of heteroalkyl, alkylcycloalkyl, heterocycloalkyl, alkyl ether, alkyl carboxylate, alkyl amine, alkyl ester, alkyl amide; C₃-C₈ of aryl, Ar-alkyl, heterocyclic, carbocyclic, cycloalkyl, heteroalkylcycloalkyl, alkylcarbonyl; or two R's: R¹R², R³R⁴, R⁵R⁶, or R¹²R¹³ together independently form a 3∼7 membered carbocyclic, cycloalkyl, heterocyclic, heterocycloalkyl, aromatic or heteroaromatic ring system; Y is N or C; In addition, R¹, R², R³, and R⁴ can be independently absent;
Wherein R⁵, R⁶, R⁸ and R¹⁰ are independently selected from H and linear or branched Ci-C₄ of alkyl or C₂-C₄ of heteroalkyl;
Wherein R⁷ is selected from H, R¹⁴, or -R¹⁴C(=O)X¹R¹⁵; -R¹⁴X¹R¹⁵; X¹ is selected from O, S, S-S, NH, or NR¹⁴;
Wherein R⁹ is H, -O-, -OR¹⁴, -OC(=O)R¹⁴-, -OC(=O)NHR¹⁴-, -OC(=O)NR¹⁴R¹⁵-, -OC(=O) R¹⁴SSR¹⁵-, OP(=O)(OR¹⁴)-, or OR¹⁴OP(=O)(OR¹⁵);
Wherein R¹¹ is H, R¹⁴, -R¹⁴C(=O)R¹⁶, -R¹⁴C(=O)X²R¹⁶, -R¹⁴X²R¹⁶, -R¹⁴C(=O)X², wherein X² is -O-, -S-, -NH-, -NHS(O₂), -NHS(O), -N(R¹⁴)-, -O-R¹⁴-, -S-R¹⁴-, -S(=O)-R¹⁴-, or -NHR¹⁴-;
Wherein R¹² is H, R¹⁴, -O-, -S-, -N-, =N-, =NNH-, -OH, -SH, -NH₂, =NH, =NNH₂,-NH(R¹⁴), -OR¹⁴, -C(O)O-, -C(O)OR¹⁶-,-COR¹⁶, -COOR¹⁴-, C(O)NH-, C(O)NH₂, C(O)NHR¹⁴,-SR¹⁴, -S(=O)R¹⁴, -P(=O)(OR¹⁶)₂, -OP(=O)(OR¹⁶)₂, -CH₂OP(=O)(OR¹⁶)₂, -SO₂R¹⁶;
Wherein R¹³ is linear or branched C₁-C₁₀ of alkyl, alkyl acid, alkyl amide, alkyl amine; or C₂-C₁₀ of heteroalkyl; or C₃-C₁₀ of Ar; Ar refers to an aromatic or hetero aromatic group, composed of one or several rings, comprising four to ten carbon atoms, preferentially four to six carbon atoms. The term of hetero aromatic group refers to an aromatic group that has one or several carbon atoms replaced by hetero atoms, preferentially one, two or three carbon atoms replaced by O, N, Si, Se, P or S, more preferentially O, S, N. The term aryl or Ar also refers to an aromatic group, wherein one or several H atoms can be replaced independently by R¹⁷, F, Cl, Br, I, OR¹⁶, SR¹⁶, NR¹⁶R¹⁷, N=NR¹⁶, N= R¹⁶, NR¹⁶R¹⁷, NO₂, SOR¹⁶R¹⁷, SO₂R¹⁶, SO₃R¹⁶, OSO₃R¹⁶, PR¹⁶R¹⁷, POR¹⁶R¹⁷, PO₂R¹⁶R¹⁷, OP(O)(OR¹⁷)₂, OCH₂OP(O)(OR¹⁷)₂, OC(O)OP(O)(OR¹⁷)₂, PO(OR¹⁶)(OR¹⁷), OP(O)(OR¹⁷)OP(O)(OR¹⁷)₂, OC(O)R¹⁷ or OC(O)NHR¹⁷;
Wherein R¹⁴ and R¹⁵ are independently H; linear or branched C₁-C₈ of alkyl; C₂-C₈ of alkenyl, alkynyl, heteroalkyl, heterocyclic, carbocyclic; C₃-C₈ of aryl, cycloalkyl, alkylcycloalkyl, heterocycloalkyl, heteroaralkyl, heteroalkylcycloalkyl, alkylcarbonyl;
Wherein when R¹⁴ is bivalent, it is a R¹⁴ that is further connected to an additional functional group of one to four amino acid units, or (CH₂CH₂O)ᵣ, r is an integer ranging from 0 to 12, or C₄-C₁₂ of glycosides, or C₁-C₈ of carboxylic acid;
Wherein R¹⁶ is H, OH, R¹⁴ or one to four amino acid units;
Wherein R¹⁷ is H, linear or branched C₁-C₈ of alkyl; C₂-C₈ of alkenyl, alkynyl, heteroalkyl, heterocyclic; C₃-C₈ of aryl, carbocyclic, cycloalkyl, alkylcycloalkyl, heterocycloalkyl, heteroalkylcycloalkyl, heteroaralkyl, alkylcarbonyl or C₄-C₁₂ of glycosides, or pharmaceutical salts.

In another embodiment, conjugates of antimitotic agents have the formula (II) 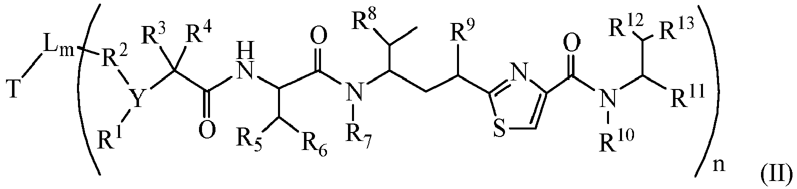
or their pharmaceutically acceptable salts, hydrates, or hydrated salts; or the polymorphic crystalline structures of these compounds; or their optical isomers, racemates, diastereomers or enantiomers thereof;
Wherein T, L, n, m, Y, R¹, R², R³, R⁴, R⁵, R⁶, R⁸, R¹⁰, R¹³, R¹⁴, R¹⁵, R¹⁶ and R¹⁷ are defined the same as in the Formula (I);
Wherein R⁷ is selected from H, R¹⁴, or -R¹⁴C(=O)X¹R¹⁵; -R¹⁴X¹R¹⁵; X¹ is selected from O, S, S-S, NH, or NR¹⁴;
Wherein R⁹ is H, -OH, -OR¹⁴, -OC(=O)R¹⁴, -OC(=O)NHR¹⁴, -OC(=O)NR¹⁴R¹⁵, -OC(=O) R¹⁴SSR¹⁵, OP(=O)(OR¹⁴)₂, or OR¹⁴OP(=O)(OR¹⁵);
Wherein R¹¹ is H, R¹⁴, -R¹⁴C(=O)R¹⁶, -R¹⁴C(=O)X²R¹⁶, -R¹⁴X²R¹⁶, -R¹⁴C(=O)X², wherein X² is -O-, -S-, -NH-, -NHS(O₂), -N(R¹⁴)-, -O-R¹⁴-, -S-R¹⁴-, -S(=O)-R¹⁴-, or -NHR¹⁴-;
Wherein R¹² is H, R¹⁴, -O-, -S-, -N-, =N-, =NNH-, -OH, -SH, -NH₂, =NH, =NNH₂,-NH(R¹⁴), -OR¹⁴, -C(O)O-, -C(O)OR¹⁶-,-COR¹⁶, -COOR¹⁴-, C(O)NH-, C(O)NH₂, C(O)NHR¹⁴,-SR¹⁴, -S(=O)R¹⁴, -P(=O)(OR¹⁶)₂, -OP(=O)(OR¹⁶)₂, -CH₂OP(=O)(OR¹⁶)₂, -SO₂R¹⁶.

Illustrative compounds inside the bracket of formula (II) have preferred structures below: 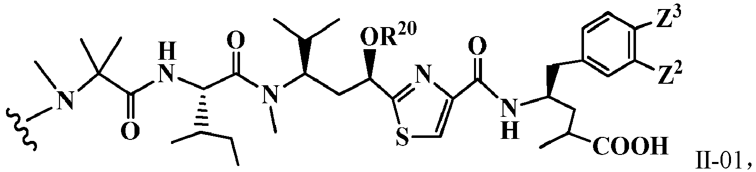 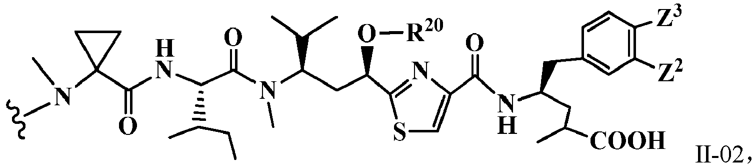 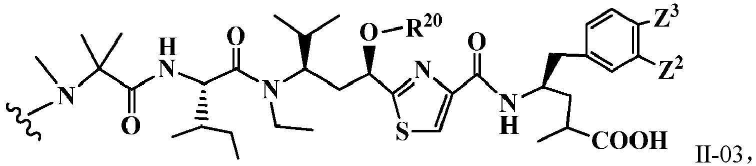 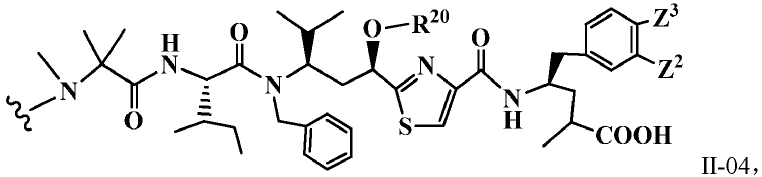 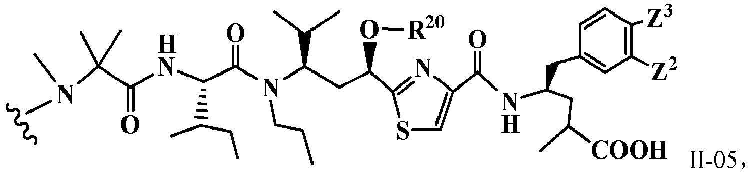 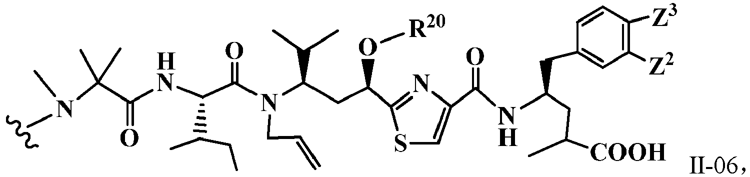 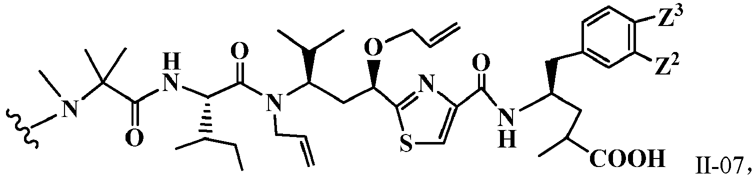 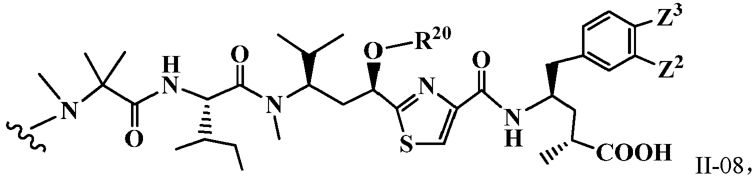 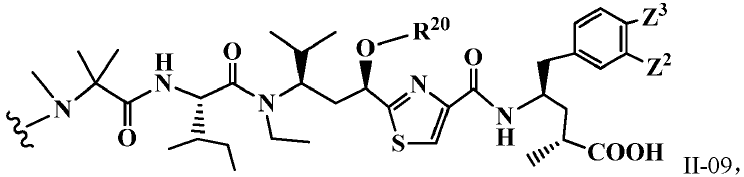 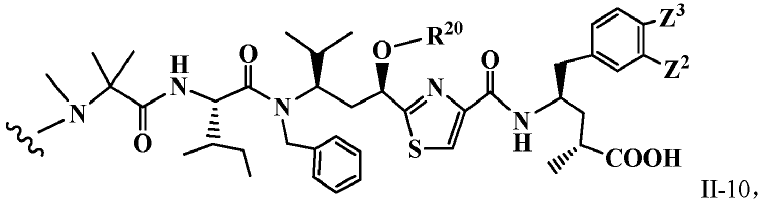 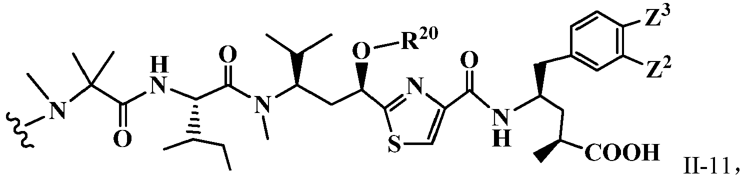 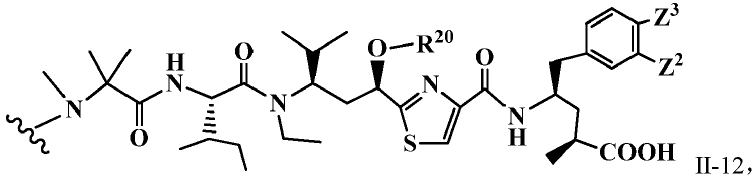 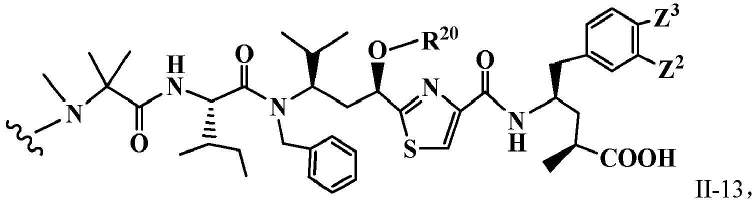 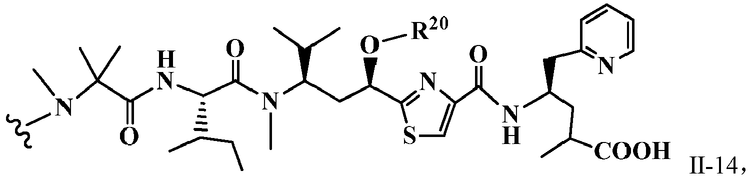 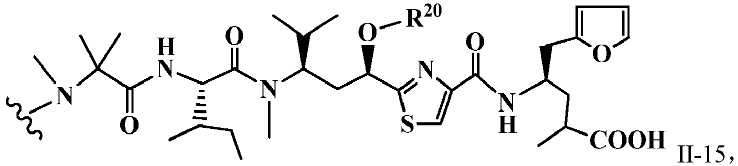 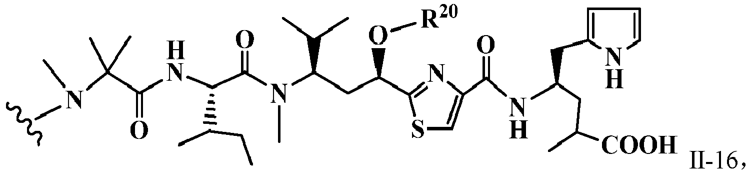 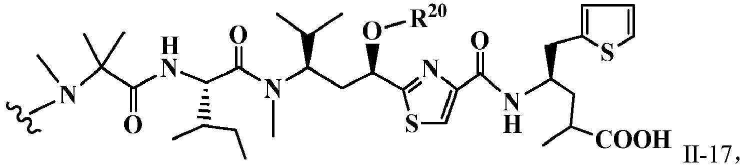 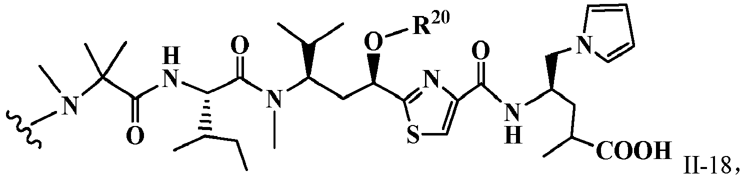 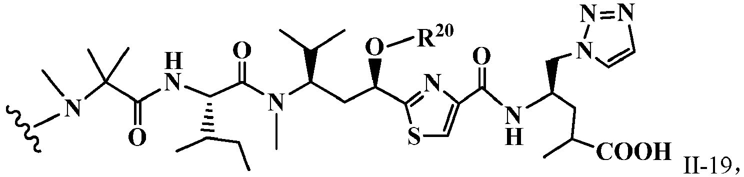 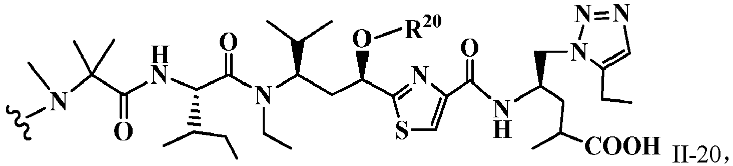 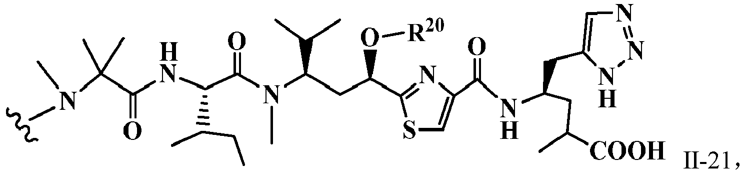 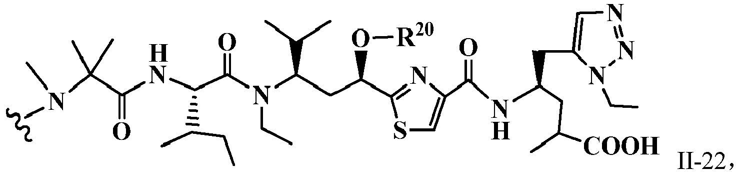 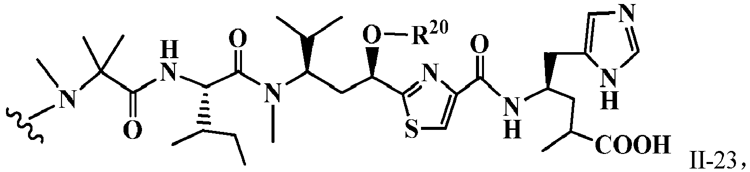 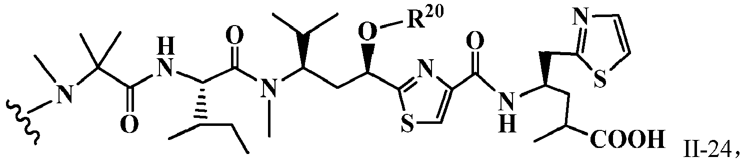 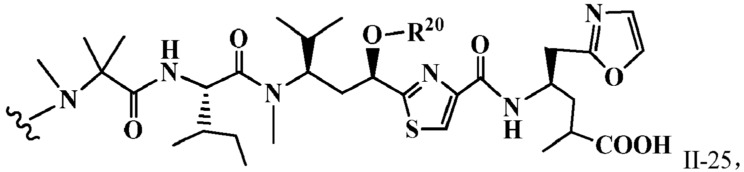 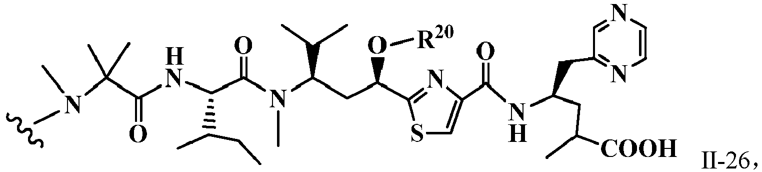 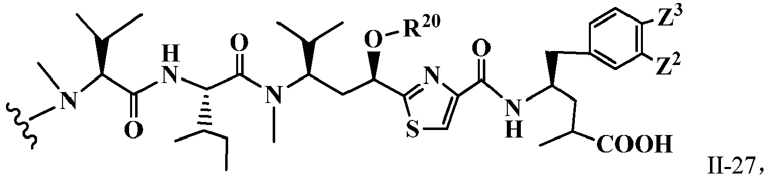 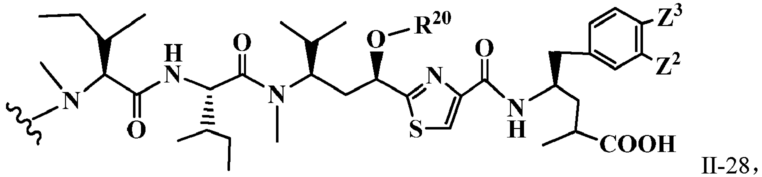 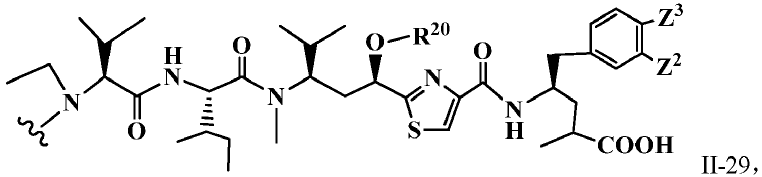 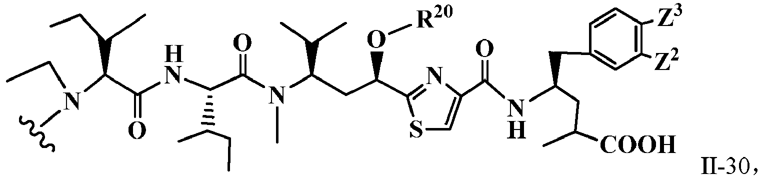 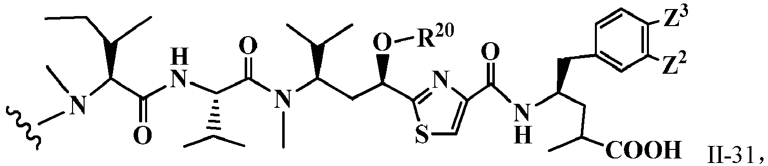 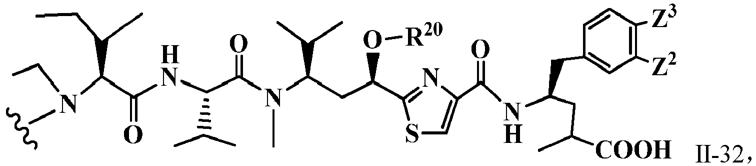 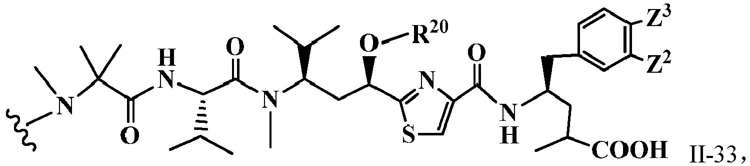 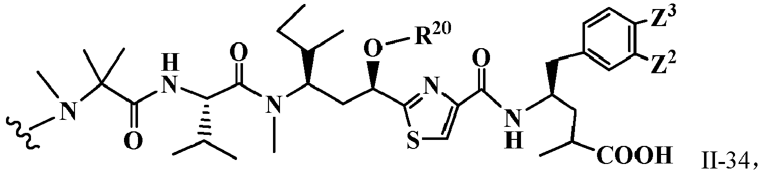 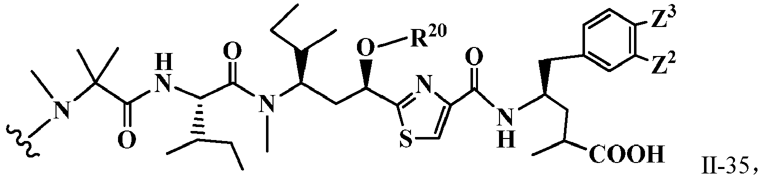 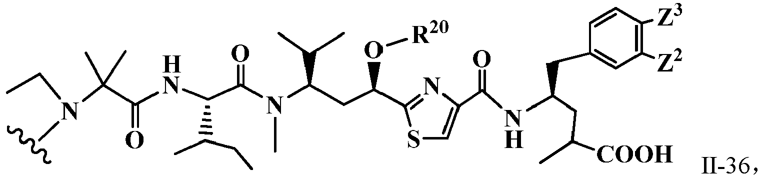 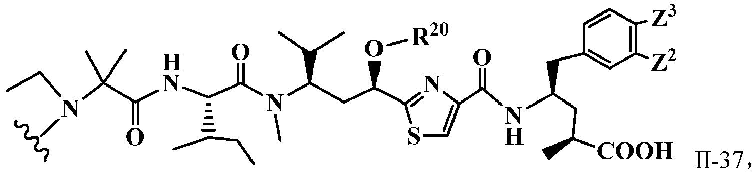 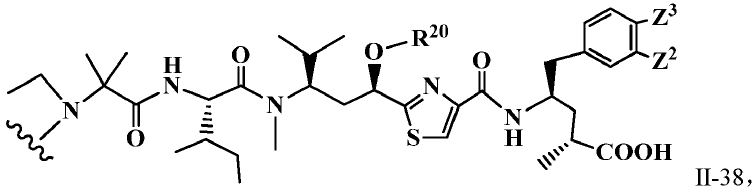 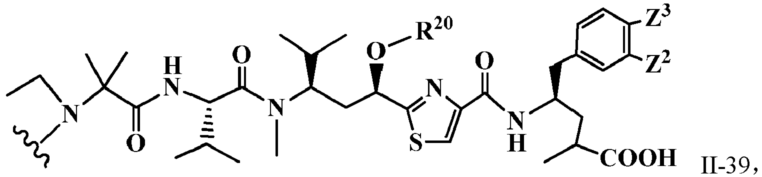 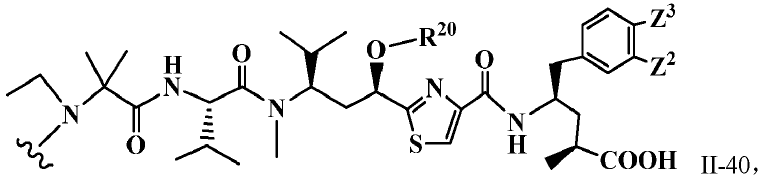 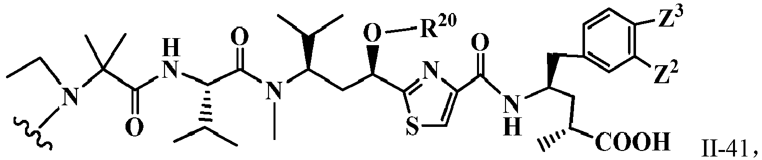 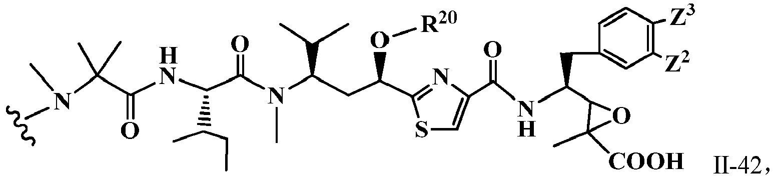 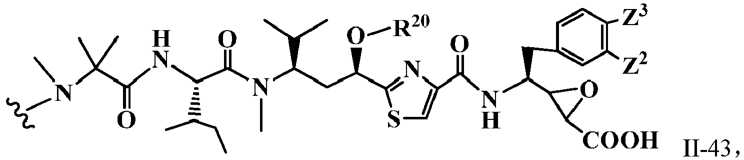 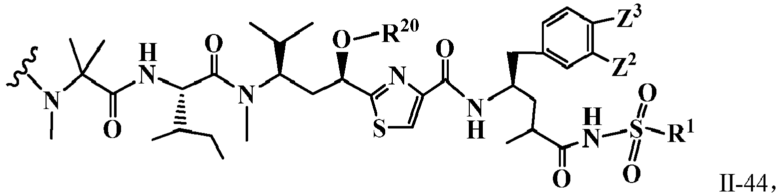 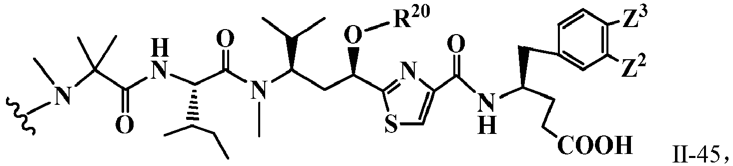 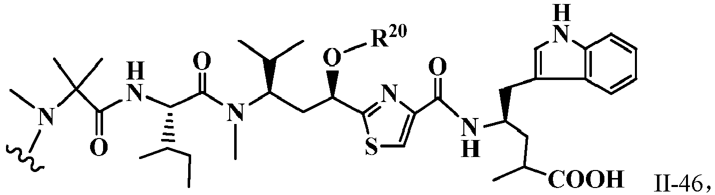 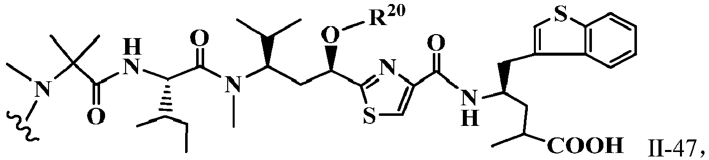 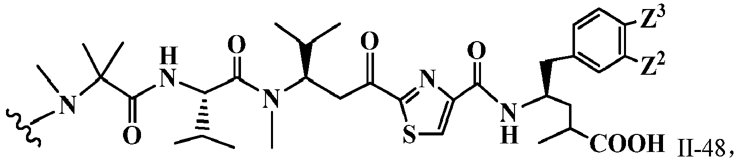 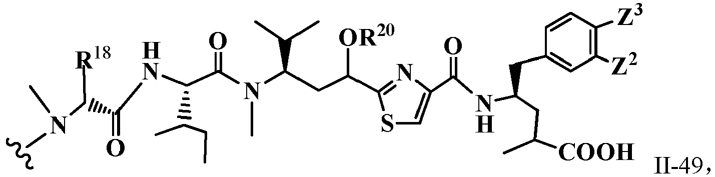 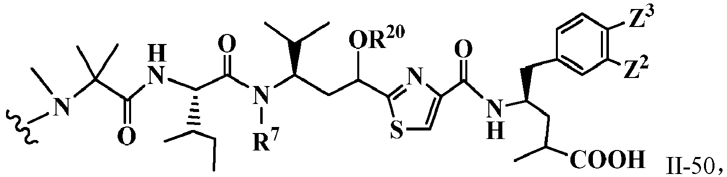 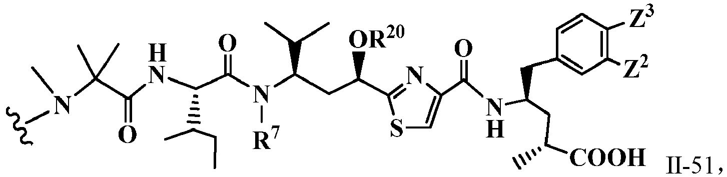 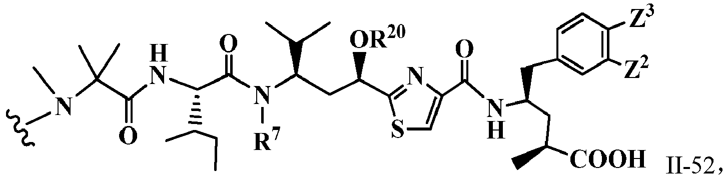 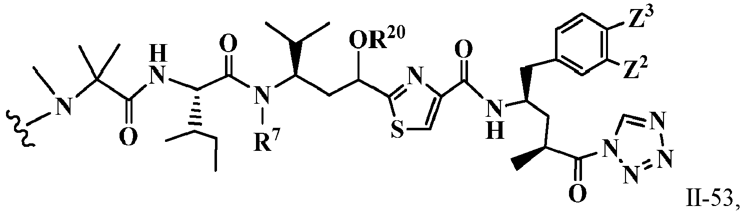 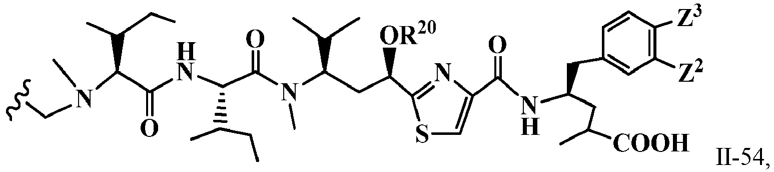 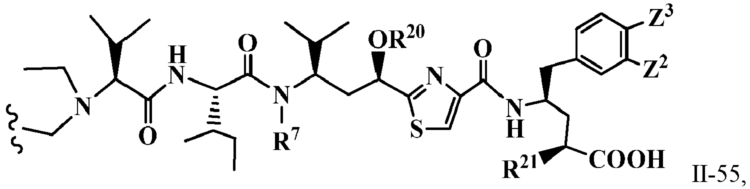 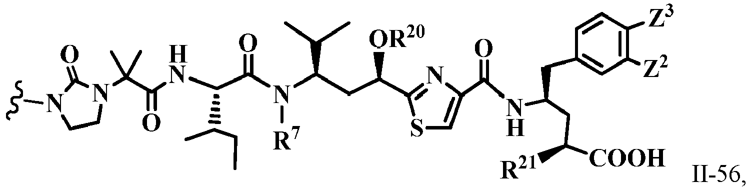 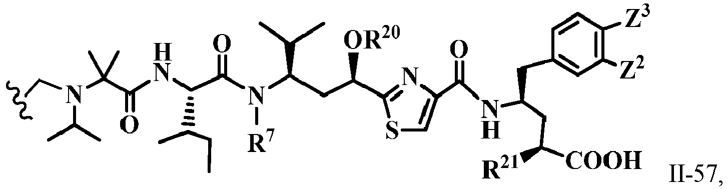 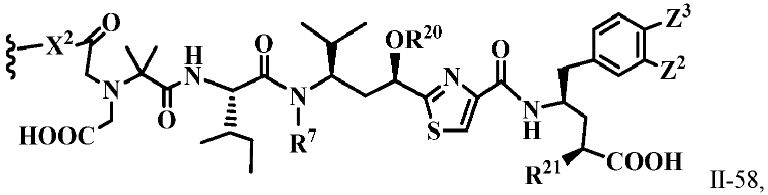 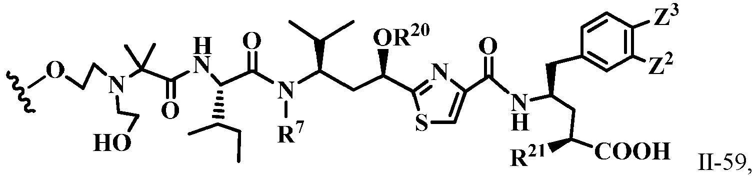 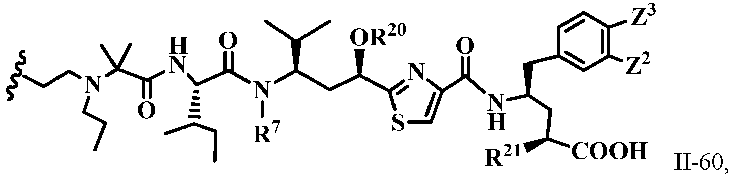 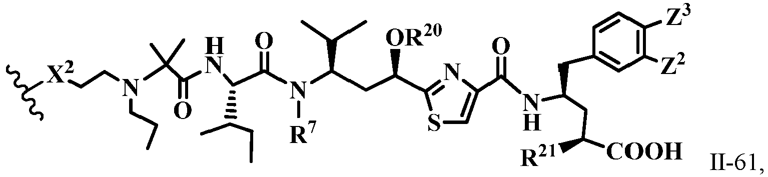 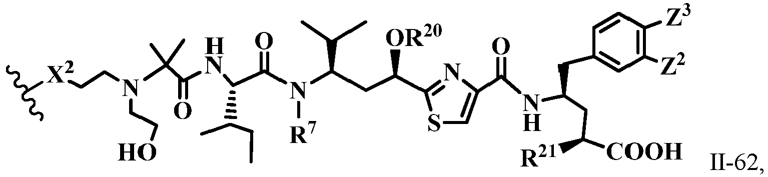 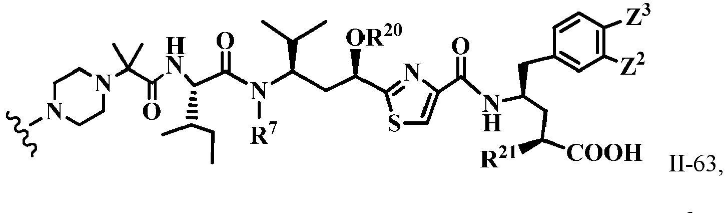 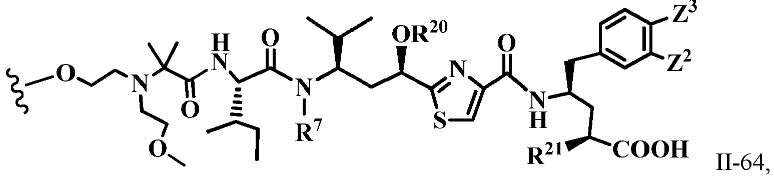 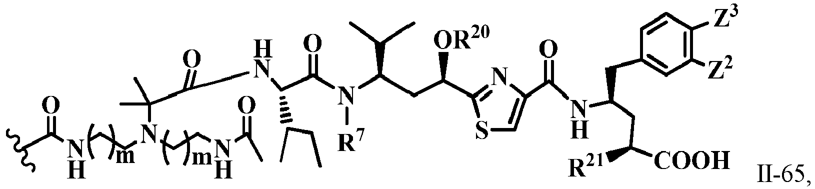 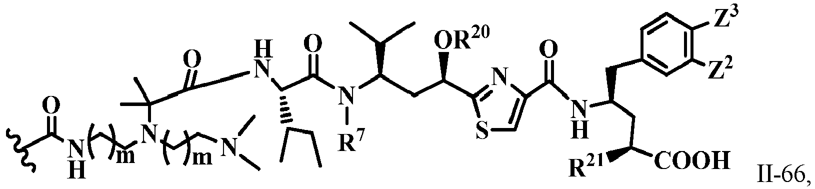 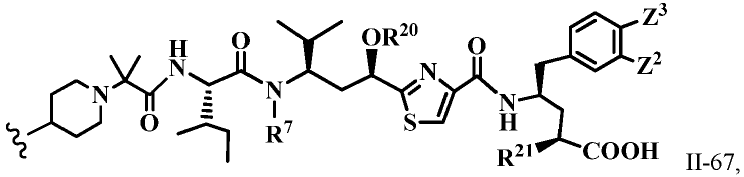 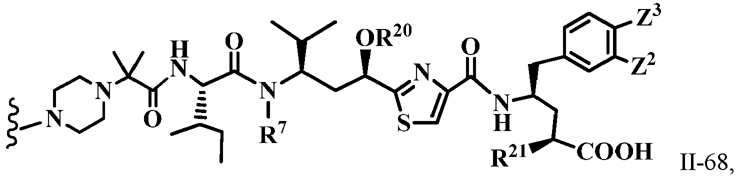 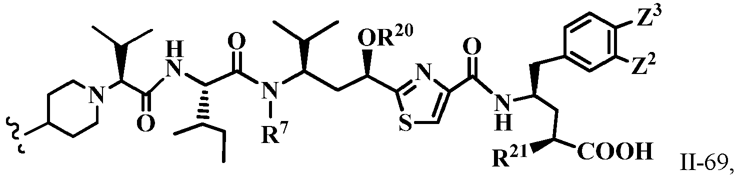
or their pharmaceutically acceptable salts, hydrates, or hydrated salts; or the polymorphic crystalline structures of these compounds; or their optical isomers, racemates, diastereomers or enantiomers thereof;
wherein R²⁰ is H; C₁-C₈ of linear or branched alkyl, heteroalkyl, or acyl (-C(O)R¹⁷); C₂-C₈ of linear or branched alkenyl, alkynyl, alkylcycloalkyl, heterocycloalkyl; C₃-C₈ linear or branched of aryl, Ar-alkyl, heterocyclic, carbocyclic, cycloalkyl, heteroalkylcycloalkyl, alkylcarbonyl, heteroaryl; carbonate (-C(O)OR¹⁷), carbamate (-C(O)NR¹⁷R¹⁸); or 1-8 carbon atoms of carboxylate, esters, ether, or amide; or 1∼8 amino acids; or polyethyleneoxy unit of formula (OCH₂CH₂)ₚ or (OCH₂CH(CH₃))ₚ, wherein p is an integer from 0 to about 1000; or R²⁰ is absent and the oxygen forms a ketone, or combination above thereof; wherein R²¹ is H, C₁-C₈ of linear or branched alkyl;
wherein Z³ and Z³ are independently H, OH, NH₂, OR¹⁷, NHR¹⁷, COOH, COOR¹⁷, C(O)R¹⁷, C(O)NHR¹⁷, C(O)NHNHR¹⁷, C(O)NH₂, R¹⁸, OCH₂OP(O)(OR¹⁸)₂, OC(O)OP(O)(OR¹⁸)₂, OPO(OR¹⁸)₂, NHPO(OR¹⁸)₂, OP(O)(OR¹⁸)OP(O)(OR¹⁸)₂, OC(O)R¹⁸, OC(O)NHR¹⁸, OSO₂(OR¹⁸), O-(C₄-C₁₂-glycoside), C₁-C₈ of linear or branched alkyl or heteroalkyl; C₂-C₈ of linear or branched alkenyl, alkynyl, alkylcycloalkyl, heterocycloalkyl; C₃-C₈ linear or branched of aryl, Ar-alkyl, heterocyclic, carbocyclic, cycloalkyl, heteroalkylcycloalkyl, alkylcarbonyl, heteroaryl; carbonate (-C(O)OR¹⁷), carbamate (-C(O)NR¹⁷R¹⁸); R¹⁷ and R¹⁸ are independently H, C₁-C₈ linear or branched alkyl or heteroalkyl; C₂-C₈ of linear or branched alkenyl, alkynyl, alkylcycloalkyl, heterocycloalkyl; C₃-C₈ linear or branched of aryl, Ar-alkyl, heterocyclic, carbocyclic, cycloalkyl, heteroalkylcycloalkyl, alkylcarbonyl, heteroaryl; carbonate (-C(O)OR¹⁷), carbamate (-C(O)NR¹⁷R¹⁸); R¹⁹ is H, OH, NH₂, OSO₂(OR¹⁸), XCH₂OP(O)(OR¹⁸)₂, XPO(OR¹⁸)₂, XC(O)OP(O)(OR¹⁸)₂, XC(O)R¹⁸, XC(O)NHR¹⁸, *C*₁∼*C*₈ alkyl or carboxylate; C₂∼C₈ alkenyl, alkynyl, alkylcycloalkyl, heterocycloalkyl; C₃∼C₈ aryl or alkylcarbonyl; or pharmaceutical salts; X is O, S, NH, NHNH, NHR¹⁷, or CH_{2;} R⁷ is defined the same above;
wherein " 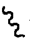 " is the site that linked to a linker L of Formula (II).

In another embodiment, a conjugate of a cell binding molecule-antimitotic agent has the Formula (III): 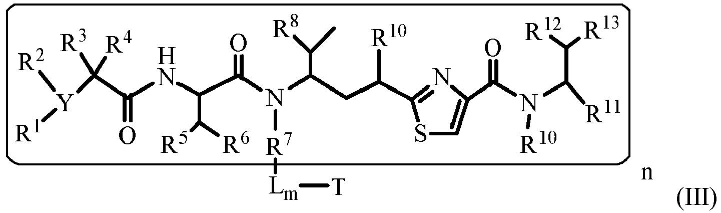
or their pharmaceutically acceptable salts, hydrates, or hydrated salts; or the polymorphic crystalline structures of these compounds; or their optical isomers, racemates, diastereomers or enantiomers thereof;
wherein T, L, m, Y, R¹, R², R³, R⁴, R⁵, R⁶, R⁸, R⁹, R¹⁰, R¹¹, R¹², R¹³ and n are defined the same as in Formula (I) and (II);
Wherein R⁷ is independently selected from -R¹⁴-, or -R¹⁴C(=O)X¹R¹⁵- or -R¹⁴X¹R¹⁵-, wherein R¹⁴ and R¹⁵ are independently linear or branched C₁∼C₈ of alkyl, heteroalkyl; C₂∼C₈ of alkenyl, alkynyl; C₃∼C₈ of aryl, heterocyclic, carbocyclic, cycloalkyl, heterocycloalkyl, heteroaralkyl heteroalkylcycloalkyl, alkylcarbonyl; X¹ is selected from O, S, S-S, NH, or NR¹⁴.

Illustrative compounds inside the bracket of Formula (III) have the structures: 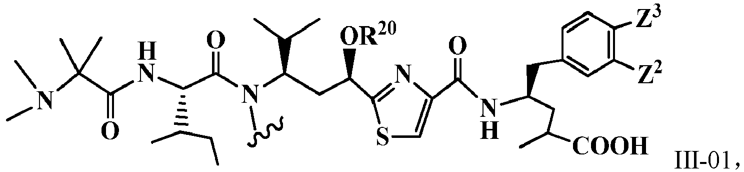 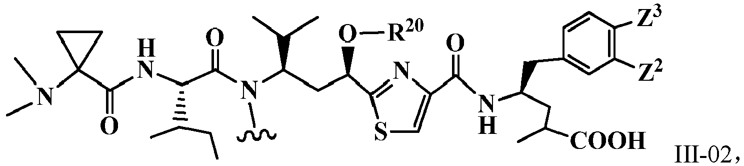 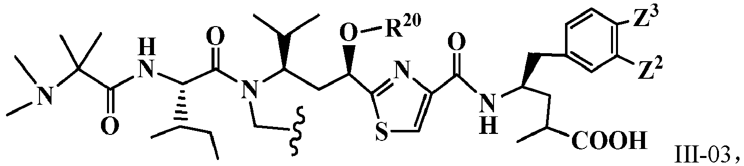 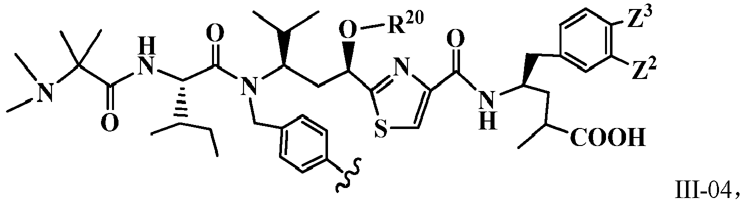 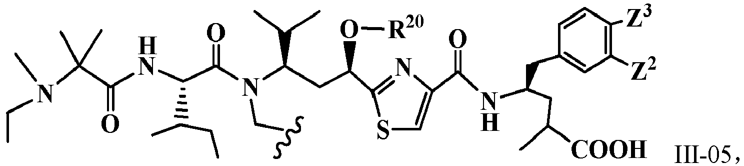 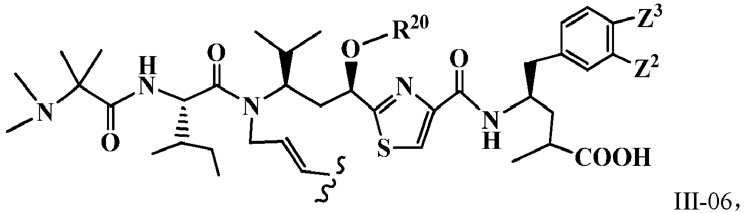 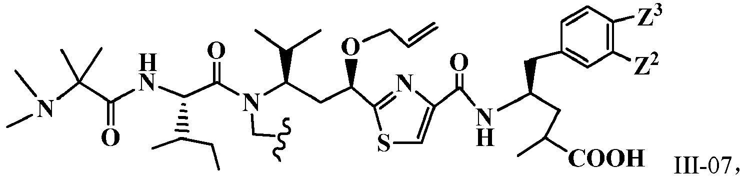 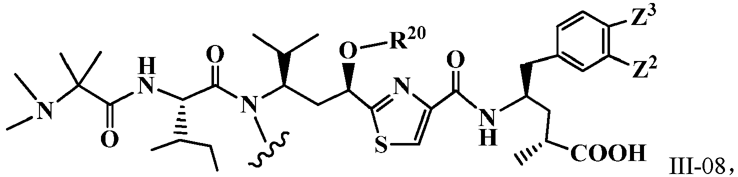 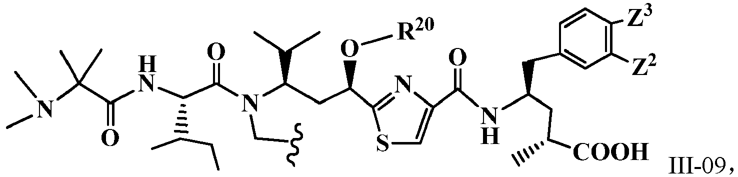 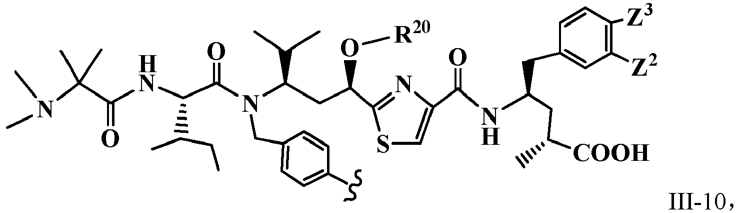 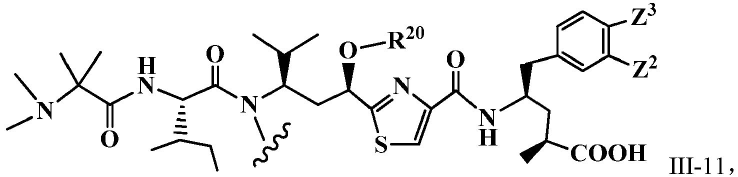 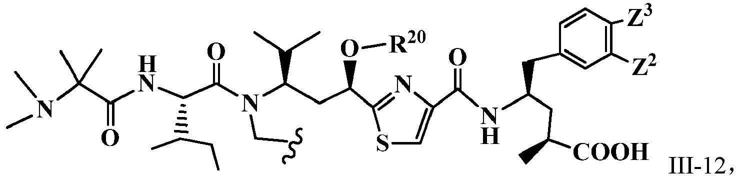 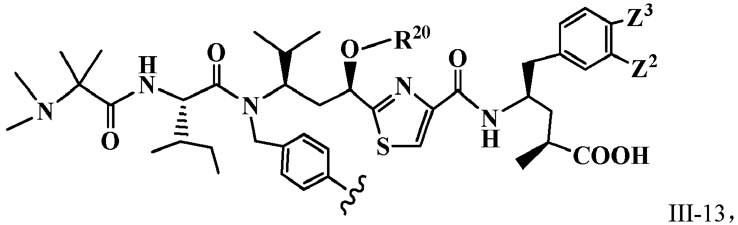 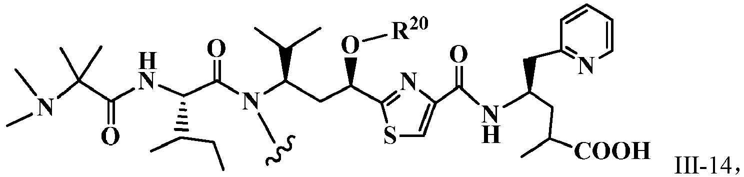 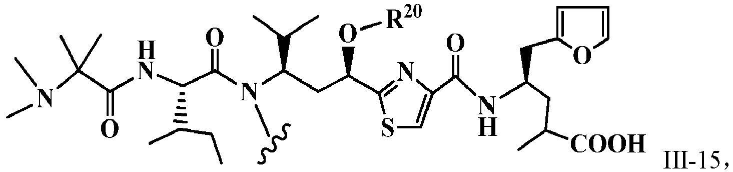 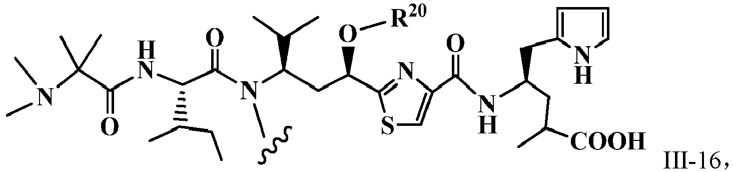 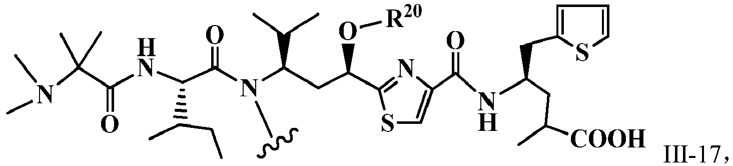 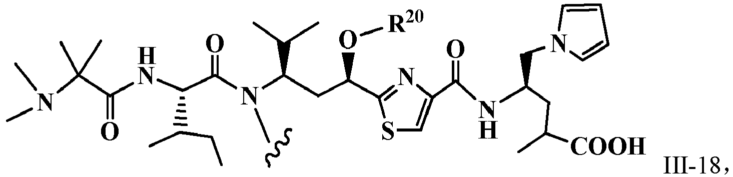 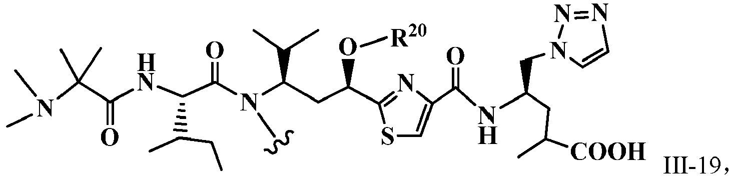 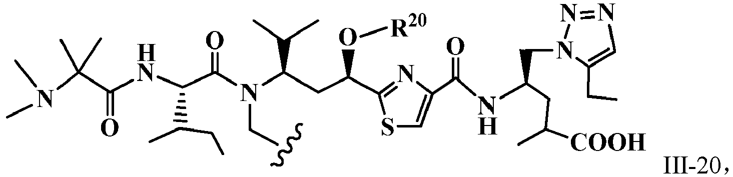 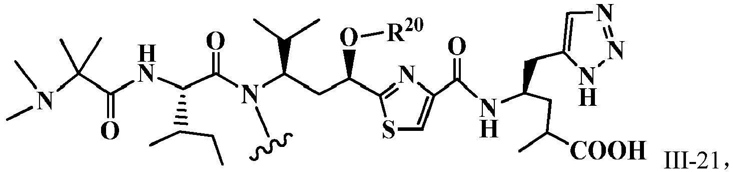 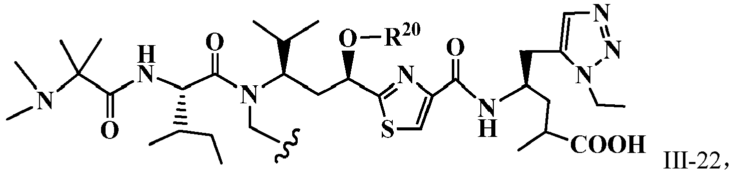 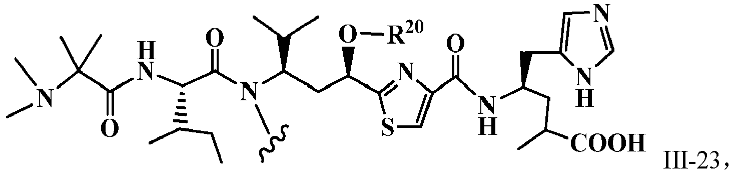 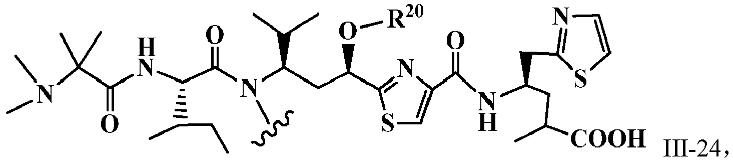 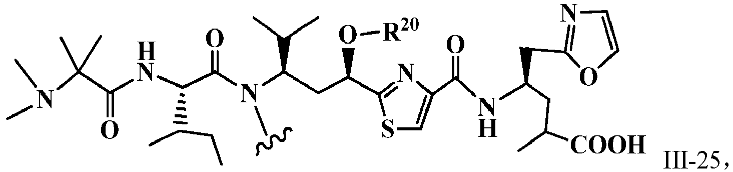 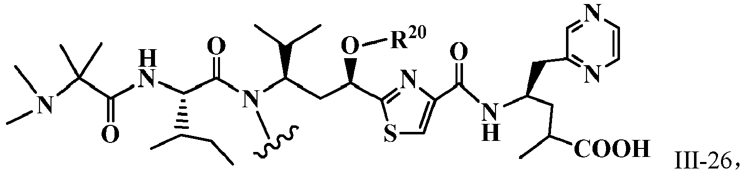 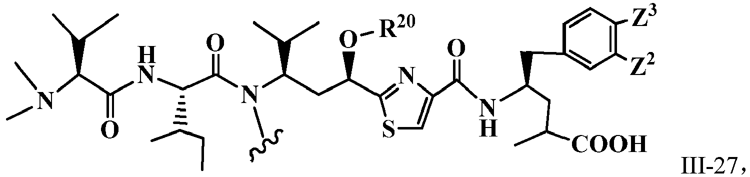 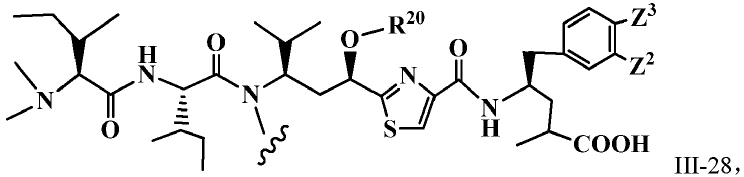 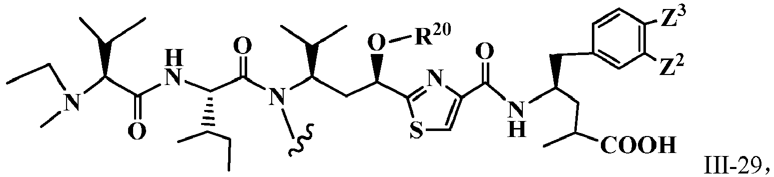 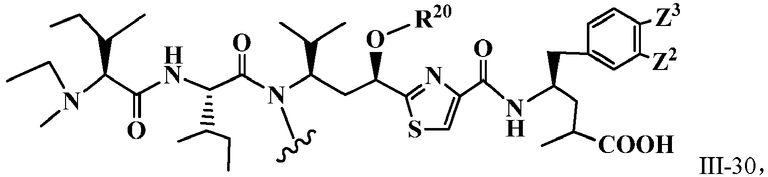 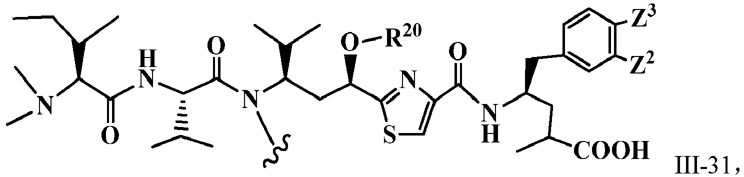 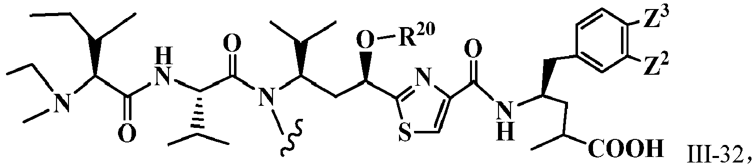 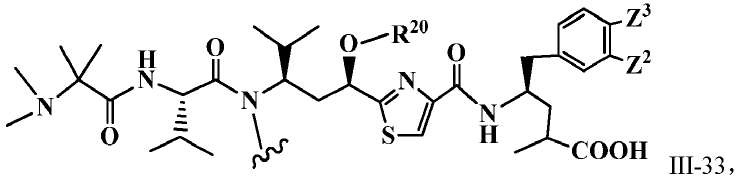 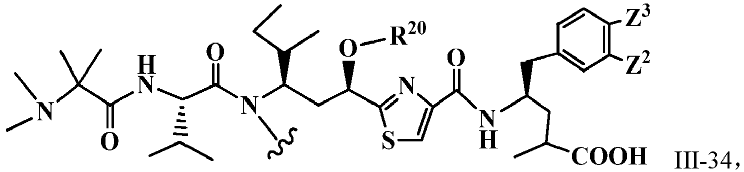 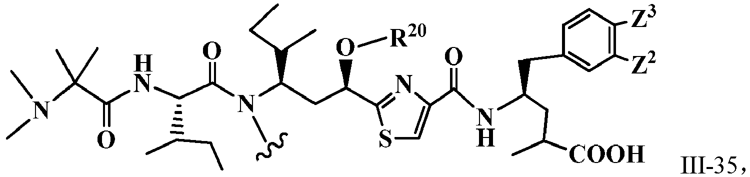 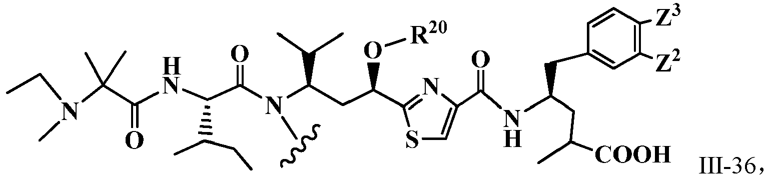 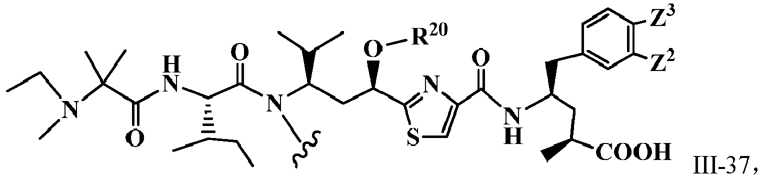 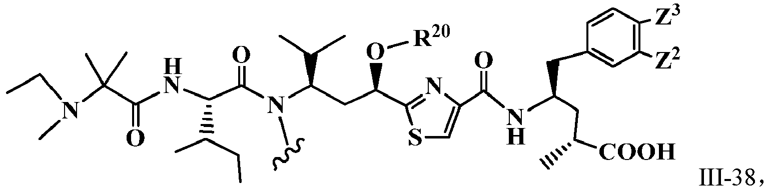 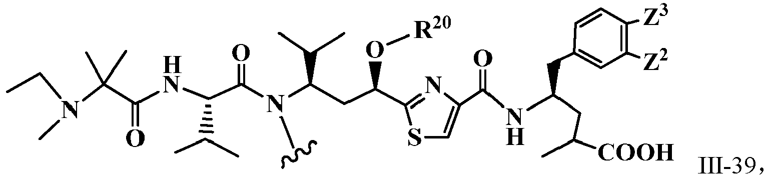 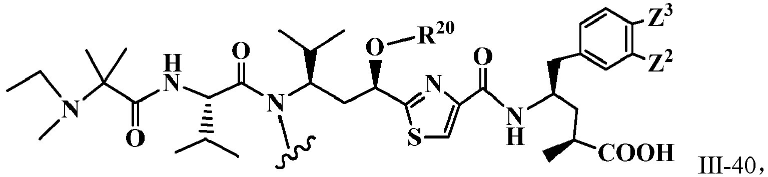 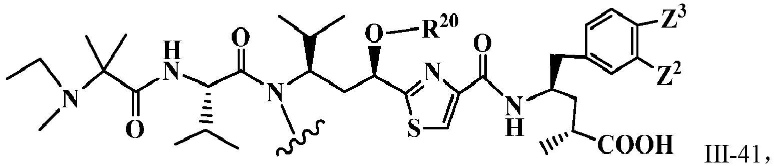 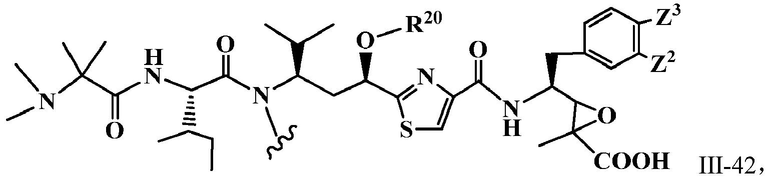 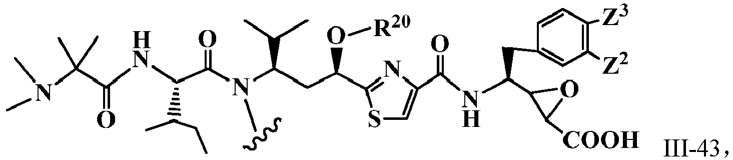 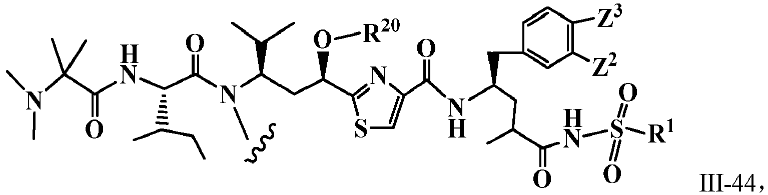 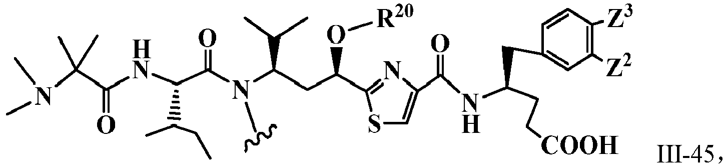 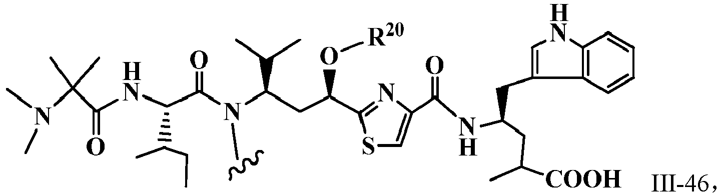 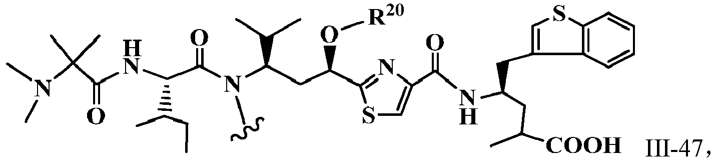 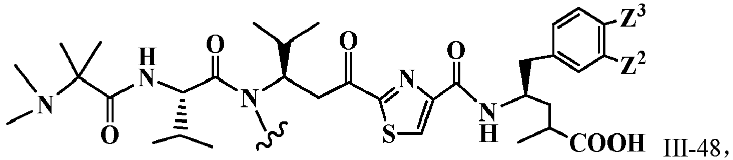 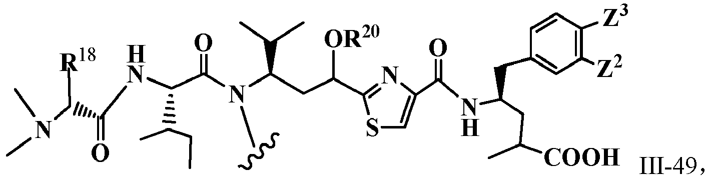 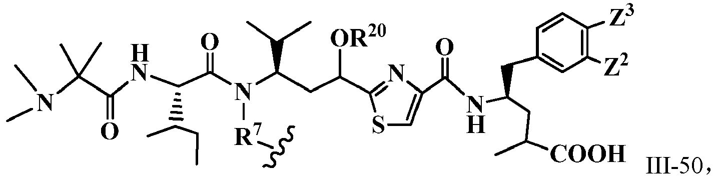 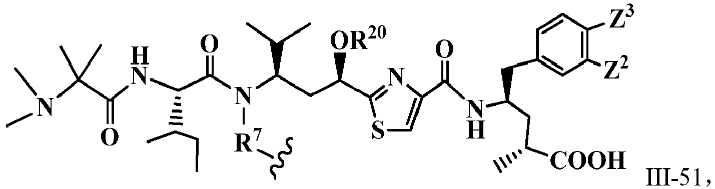 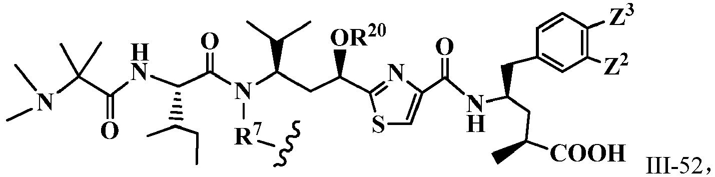 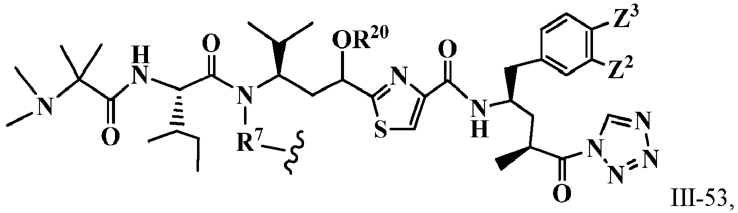 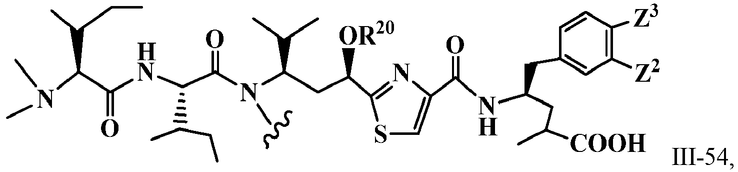 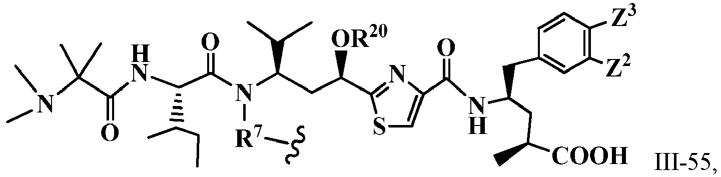 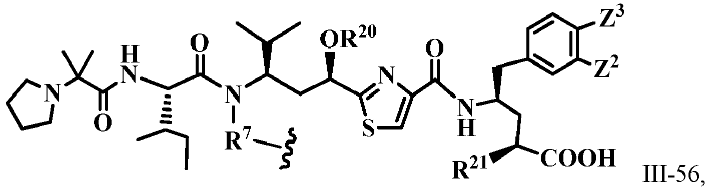 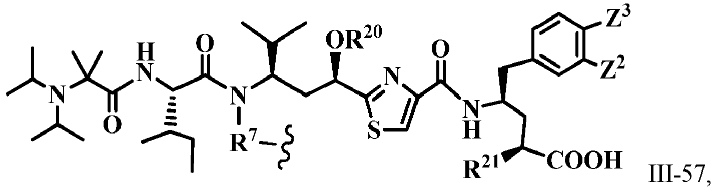 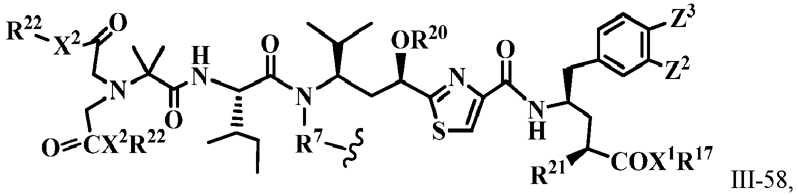 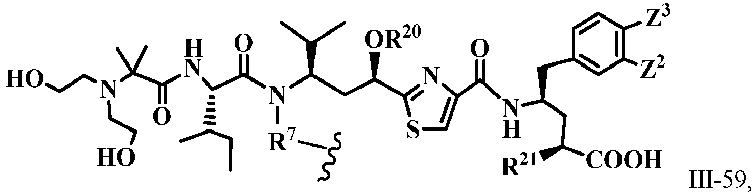 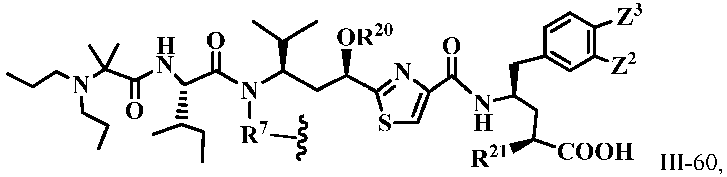 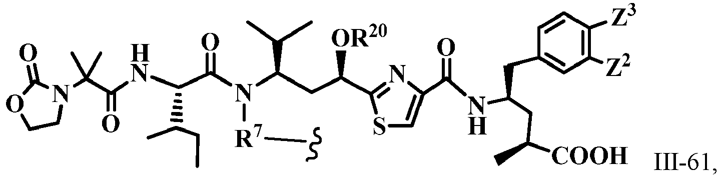 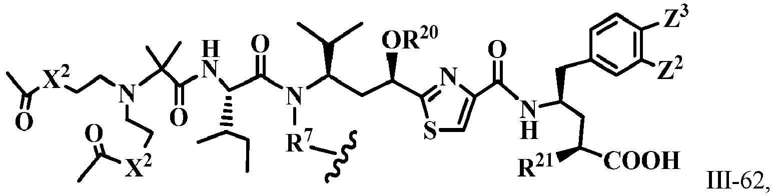 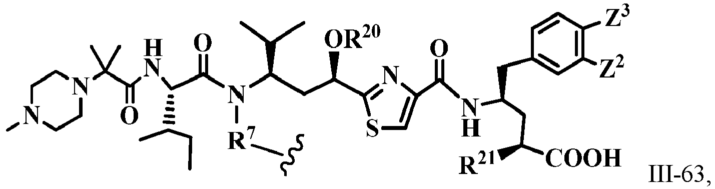 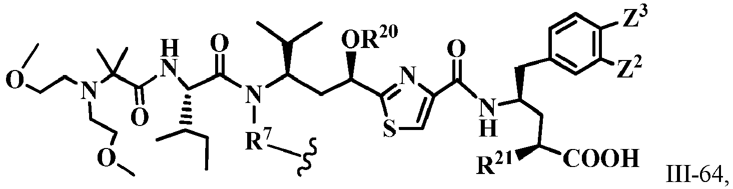 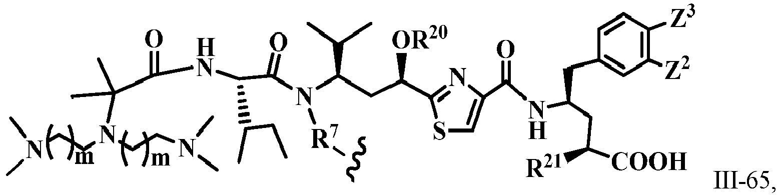 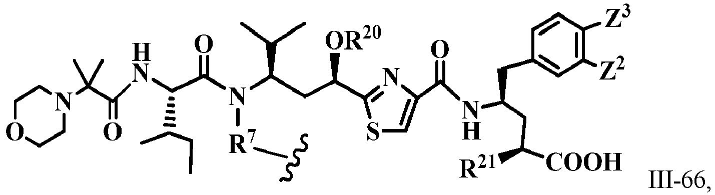 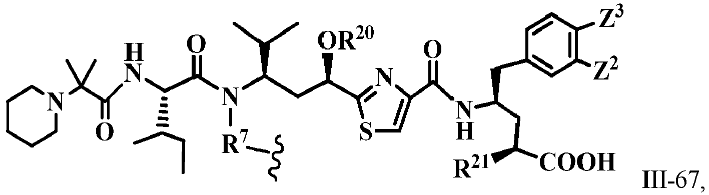 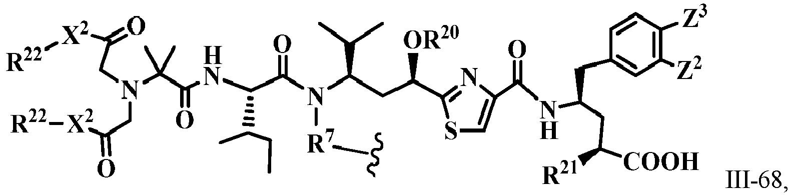
or their pharmaceutically acceptable salts, hydrates, or hydrated salts; or the polymorphic crystalline structures of these compounds; or their optical isomers, racemates, diastereomers or enantiomers thereof;
wherein R²⁰ is H; C₁-C₈ of linear or branched alkyl, heteroalkyl, or acyl (-C(O)R¹⁷); C₂-C₈ of linear or branched alkenyl, alkynyl, alkylcycloalkyl, heterocycloalkyl; C₃∼C₈ linear or branched of aryl, Ar-alkyl, heterocyclic, carbocyclic, cycloalkyl, heteroalkylcycloalkyl, alkylcarbonyl, heteroaryl; carbonate (-C(O)OR¹⁷), carbamate (-C(O)NR¹⁷R¹⁸); or 1-8 carbon atoms of carboxylate, esters, ether, or amide; or 1∼8 amino acids; or polyethyleneoxy unit of formula (OCH₂CH₂)ₚ or (OCH₂CH(CH₃))ₚ, wherein p is an integer from 0 to about 1000; or R²⁰ is absent and the oxygen forms a ketone, or combination above thereof;
wherein R²¹ and R²² are independently H, C₁-C₈ of linear or branched alkyl;
Z³ and Z³ are independently H, OH, NH₂, OR¹⁷, NHR¹⁷, COOH, COOR¹⁷, C(O)R¹⁷, C(O)NHR¹⁷, C(O)NHNHR¹⁷, C(O)NH₂, R¹⁸, OCH₂OP(O)(OR¹⁸)₂, OC(O)OP(O)(OR¹⁸)₂, OPO(OR¹⁸)₂, NHPO(OR¹⁸)₂, OP(O)(OR¹⁸)OP(O)(OR¹⁸)₂, OC(O)R¹⁸, OC(O)NHR¹⁸, OSO₂(OR¹⁸), O-(C₄-C₁₂-glycoside), C₁-C₈ of linear or branched alkyl or heteroalkyl; C₂-C₈ of linear or branched alkenyl, alkynyl, alkylcycloalkyl, heterocycloalkyl; C₃-C₈ linear or branched of aryl, Ar-alkyl, heterocyclic, carbocyclic, cycloalkyl, heteroalkylcycloalkyl, alkylcarbonyl, heteroaryl; carbonate (-C(O)OR¹⁷), carbamate (-C(O)NR¹⁷R¹⁸); R¹⁷ and R¹⁸ are independently H, C₁-C₈ linear or branched alkyl or heteroalkyl; C₂-C₈ of linear or branched alkenyl, alkynyl, alkylcycloalkyl, heterocycloalkyl; C₃∼C₈ linear or branched of aryl, Ar-alkyl, heterocyclic, carbocyclic, cycloalkyl, heteroalkylcycloalkyl, alkylcarbonyl, heteroaryl; carbonate (-C(O)OR¹⁷), carbamate (-C(O)NR¹⁷R¹⁸); R¹⁹ is H, OH, NH₂, OSO₂(OR¹⁸), XCH₂OP(O)(OR¹⁸)₂, XPO(OR¹⁸)₂, XC(O)OP(O)(OR¹⁸)₂, XC(O)R¹⁸, XC(O)NHR¹⁸, *C*₁∼C₈ alkyl or carboxylate; C₂∼C₈ alkenyl, alkynyl, alkylcycloalkyl, heterocycloalkyl; C₃∼C₈ aryl or alkylcarbonyl; or pharmaceutical salts; X, X¹ and X² are independently O, S, NH, NHNH, or CH₂.

In another embodiment, a cell binding molecule-antimitotic agent conjugate has the Formula (IV): 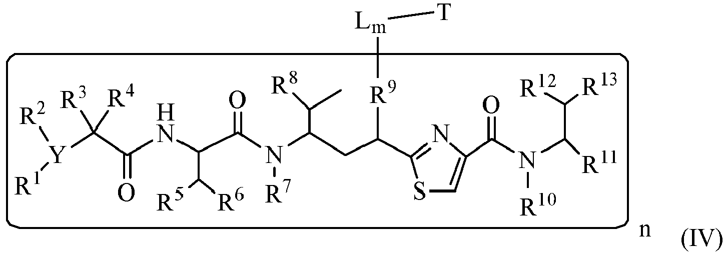
or their pharmaceutically acceptable salts, hydrates, or hydrated salts; or the polymorphic crystalline structures of these compounds; or their optical isomers, racemates, diastereomers or enantiomers thereof;
wherein T, L, m, R¹, R², R³, R⁴, R⁵, R⁶, R⁷, R⁸, R¹⁰, R¹¹, R¹², R¹³ and n are defined the same as in Formula (II);
Wherein R⁹ is independently H, -O-, -OR¹⁴-, -OC(=O)R¹⁴-, -OC(=O)NHR¹⁴-,-OC(=O)NR¹⁴R¹⁵-, -OC(=O)R¹⁴SSR¹⁵-, -OP(=O)(OR¹⁴)O-, wherein R¹⁴, R¹⁵ are independently H, C₁∼C₈ of alkyl, heteroalkyl; C₃∼C₈ of aryl, heteroaryl, heterocyclic, carbocyclic, cycloalkyl, alkylcycloalkyl, heterocycloalkyl, heteroalkylcycloalkyl, heteroaralkyl, alkylcarbonyl or pharmaceutical salts.

Illustrative compounds inside the bracket of Formula (IV) have the structures: 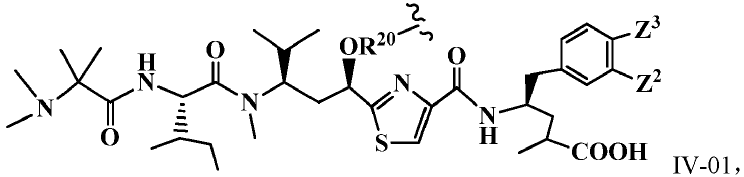 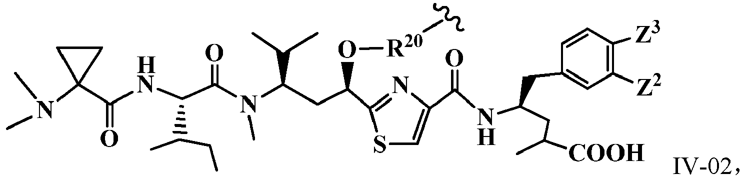 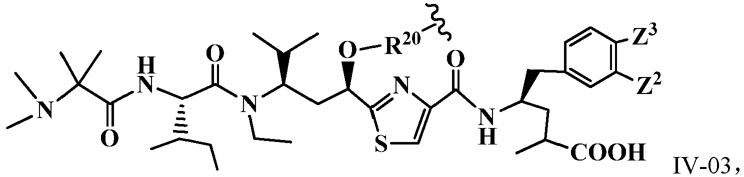 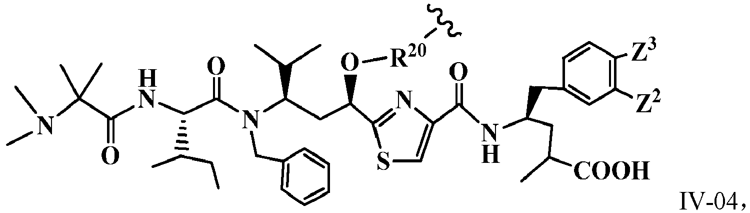 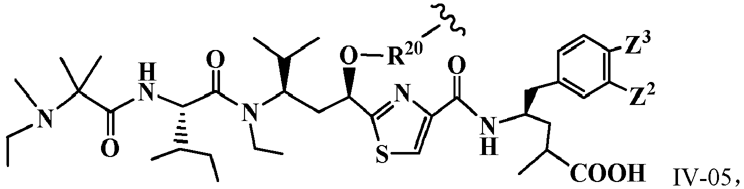 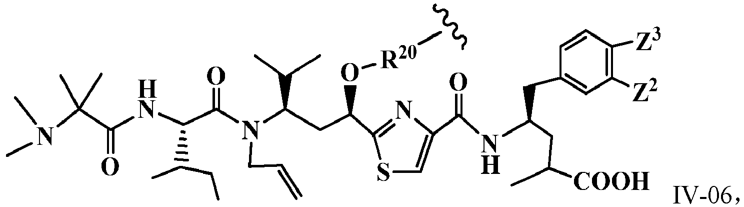 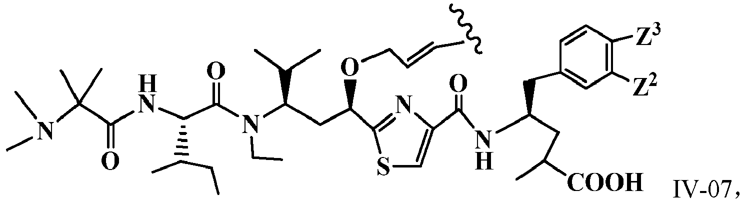 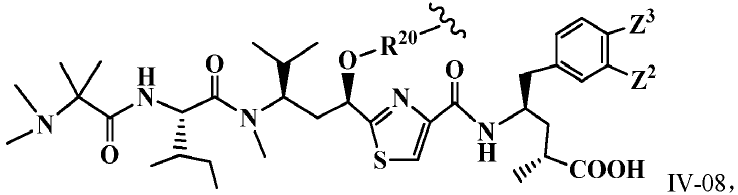 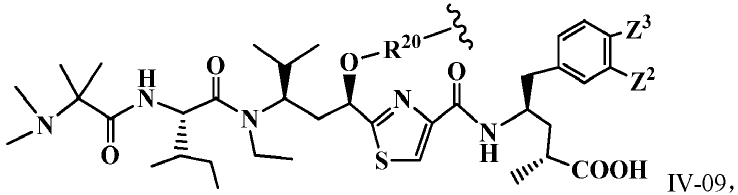 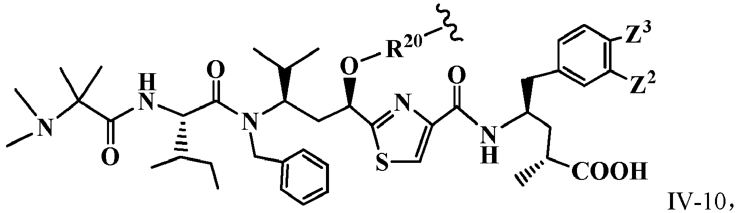 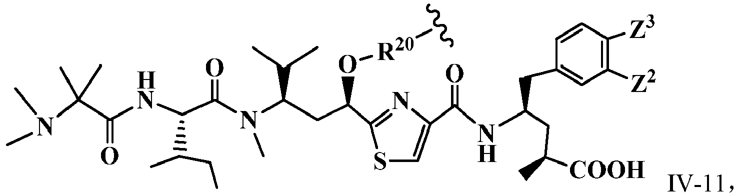 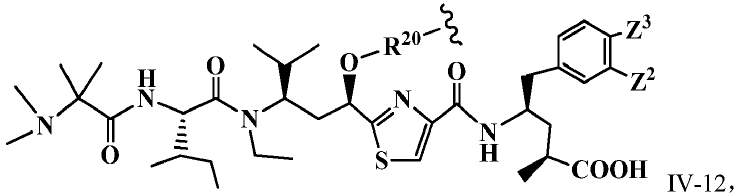 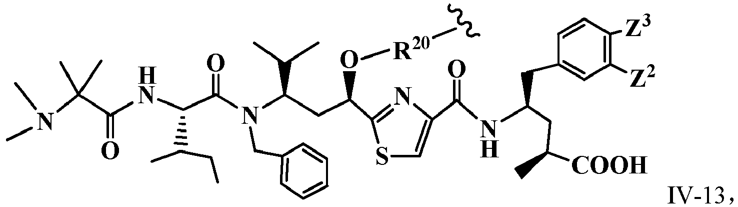 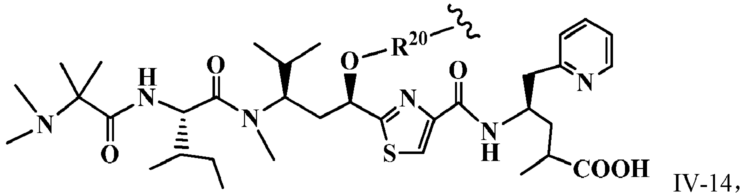 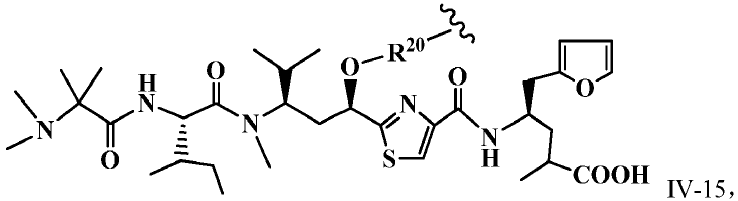 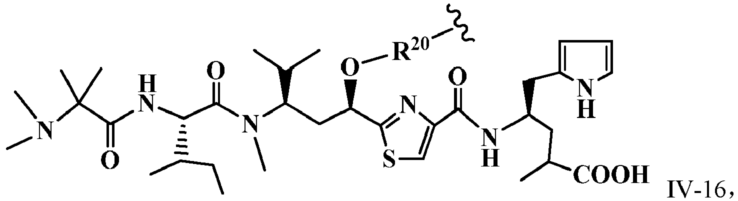 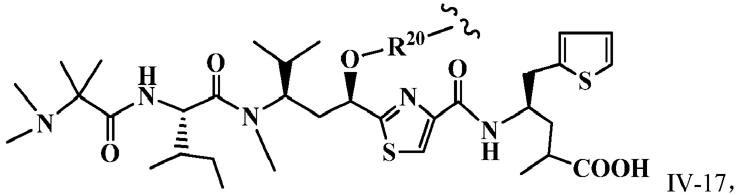 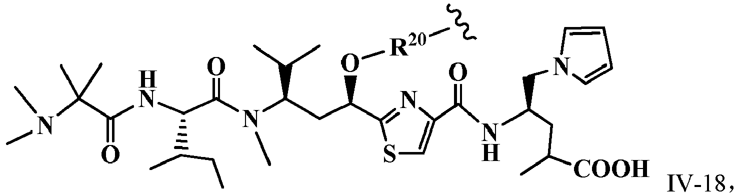 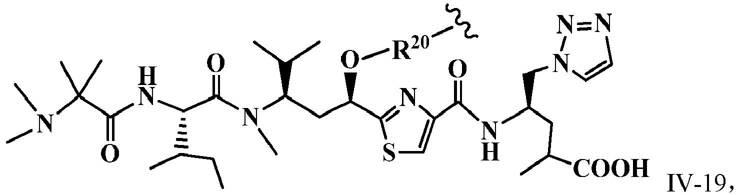 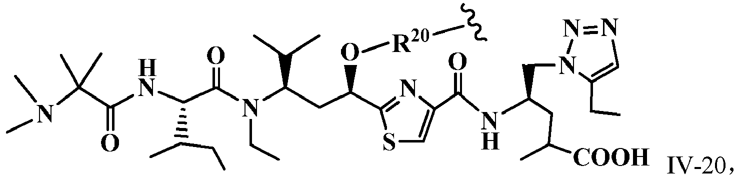 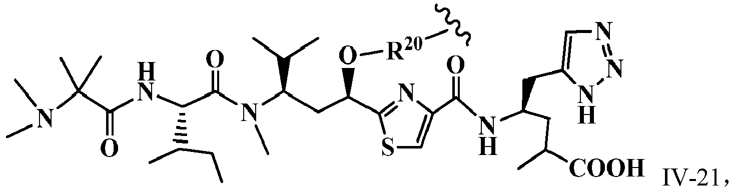 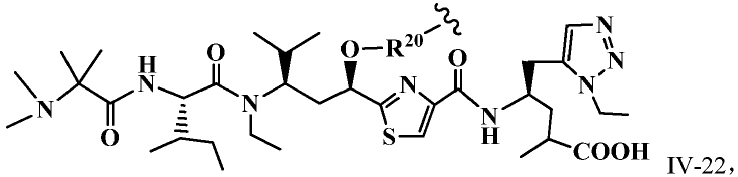 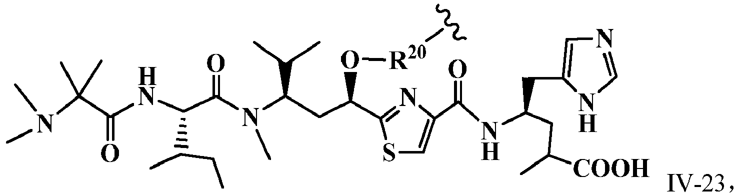 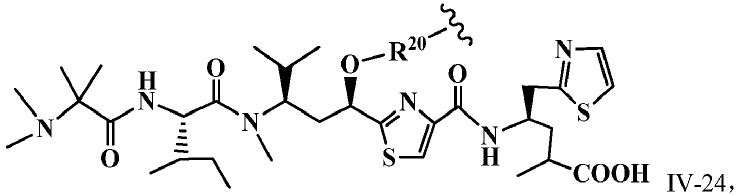 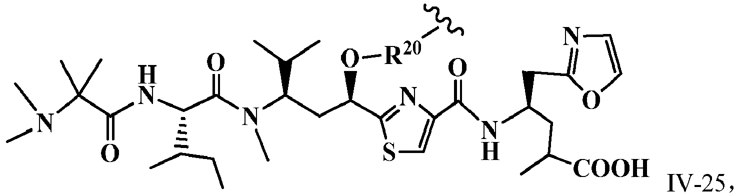 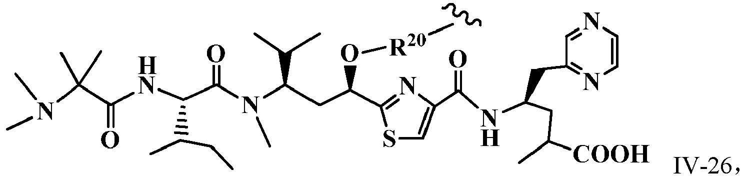 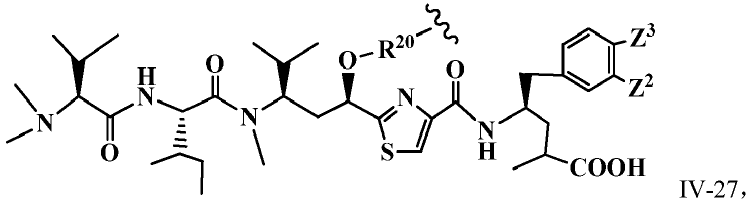 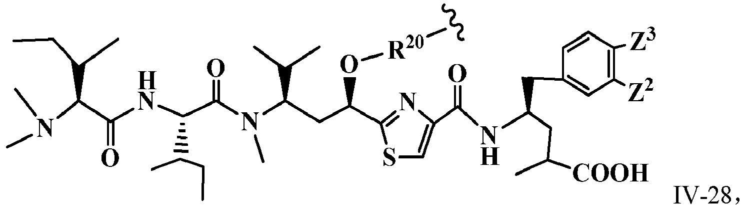 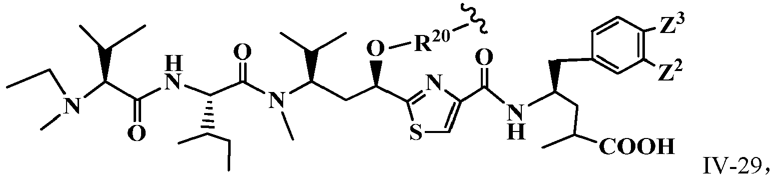 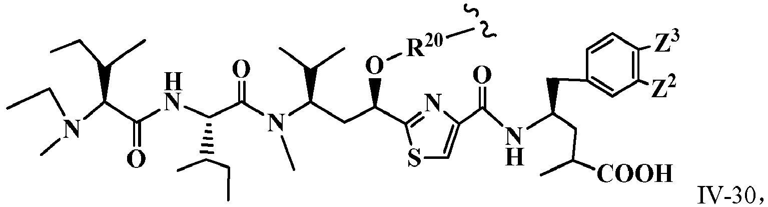 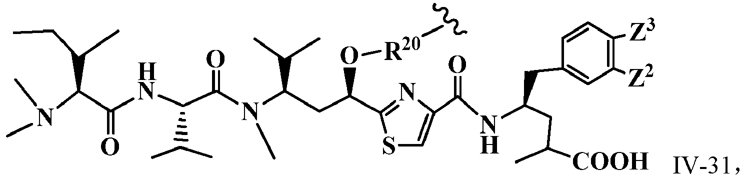 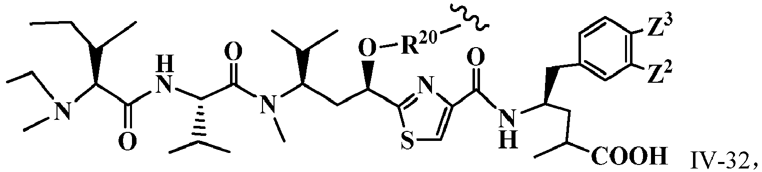 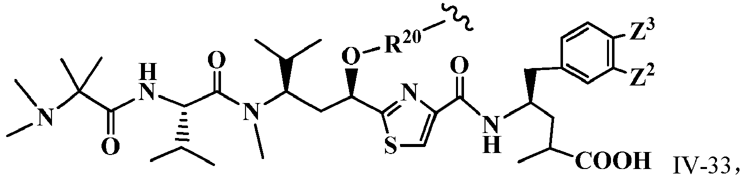 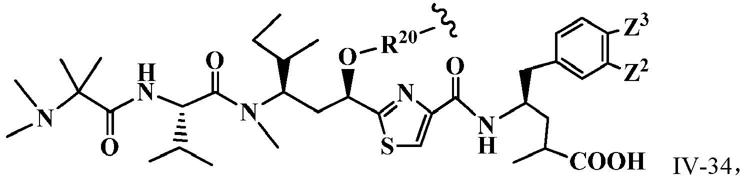 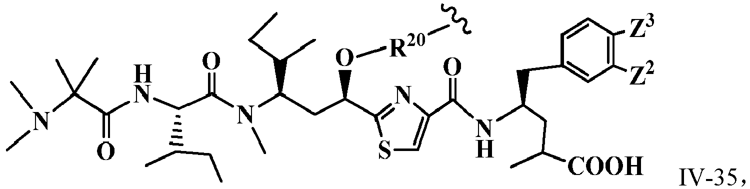 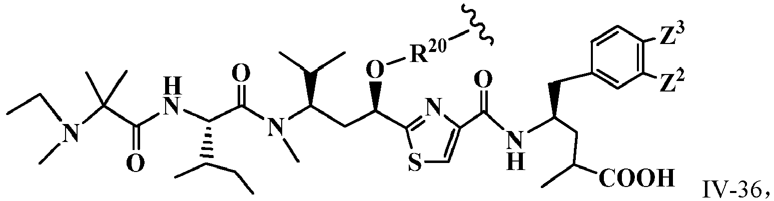 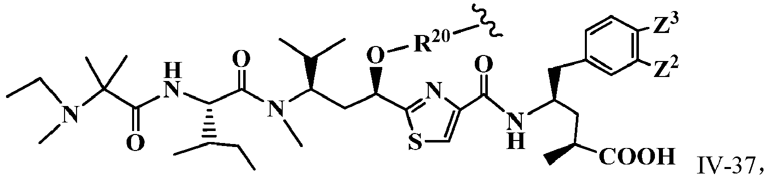 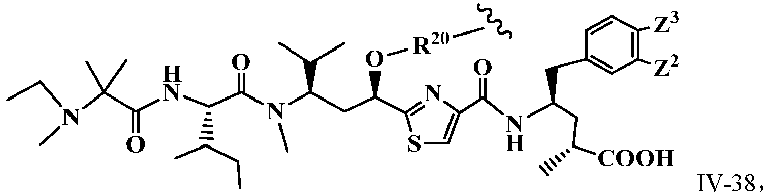 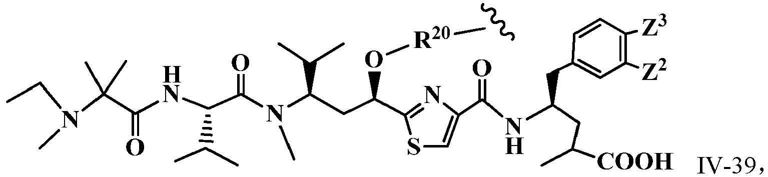 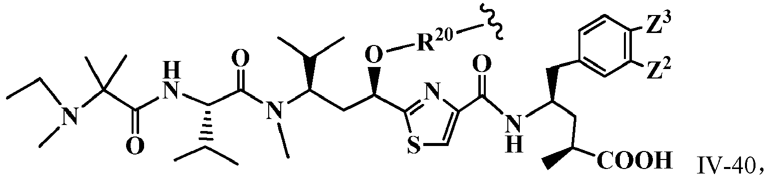 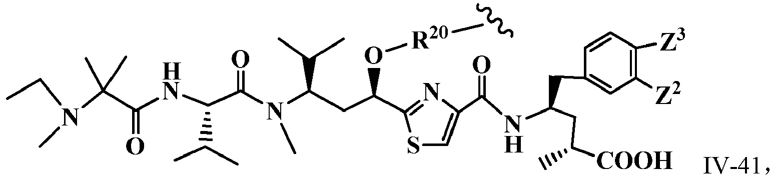 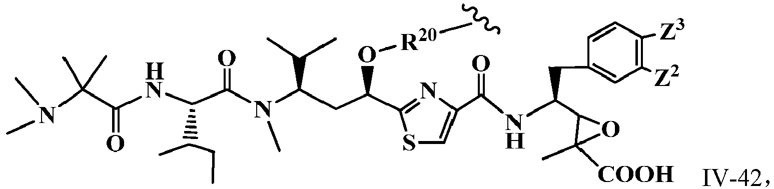 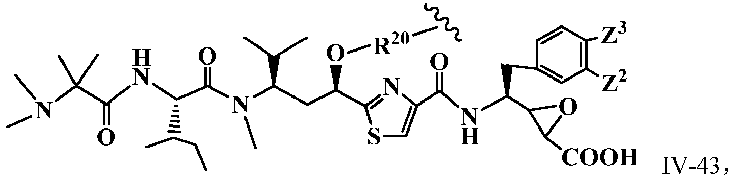 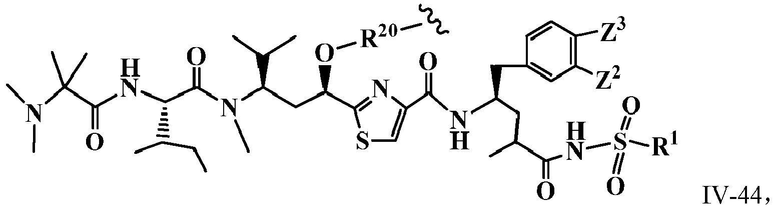 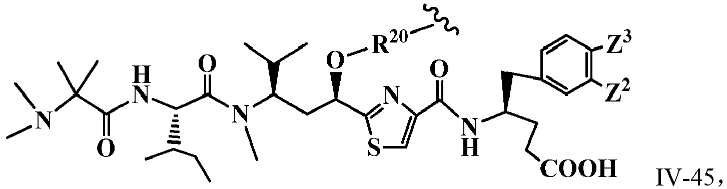 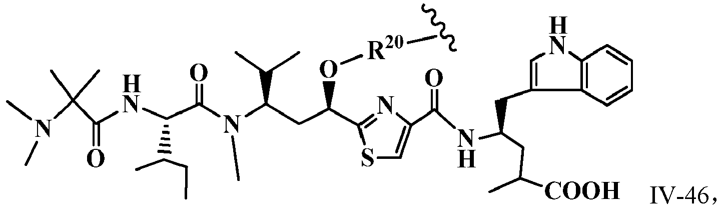 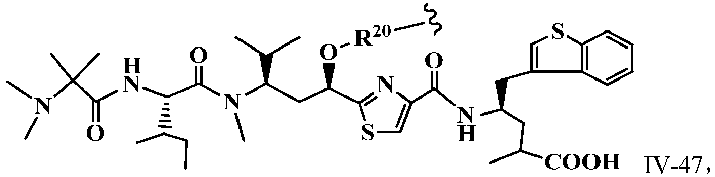 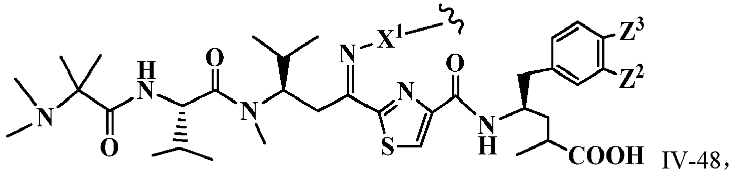 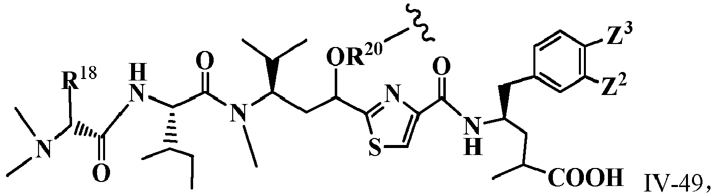 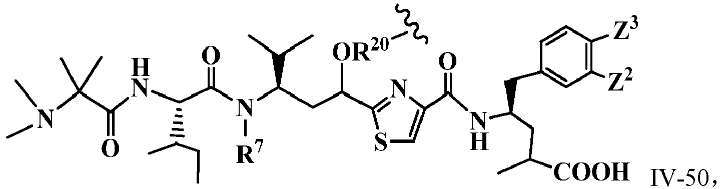 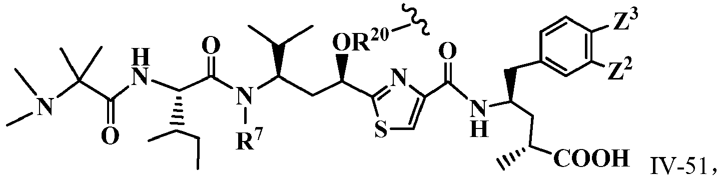 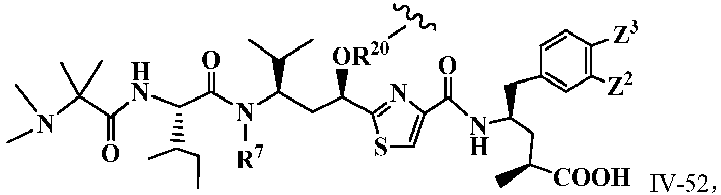 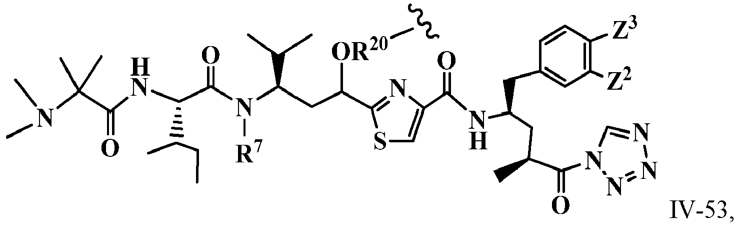 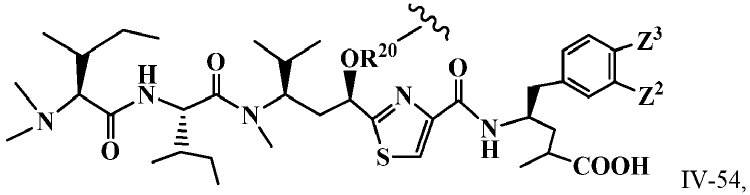 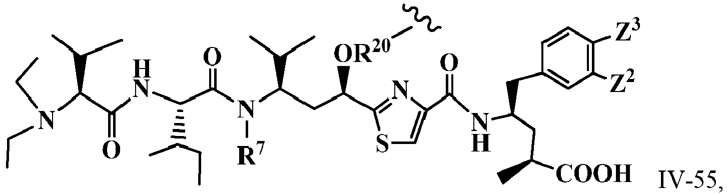 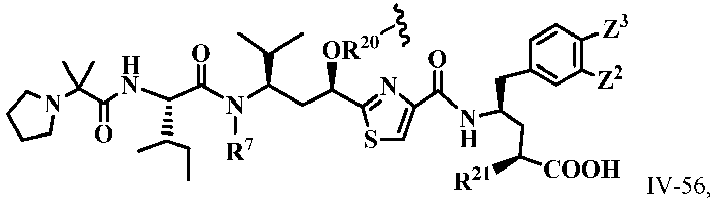 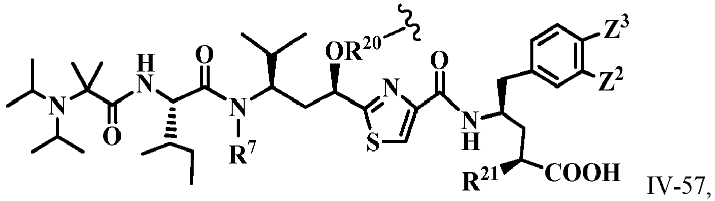 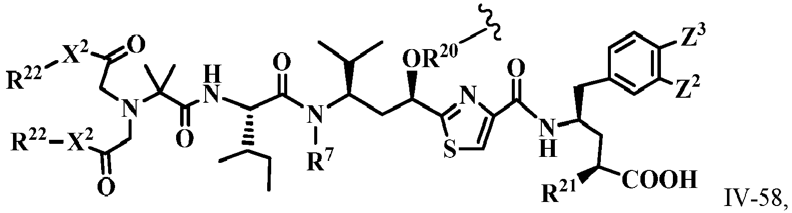 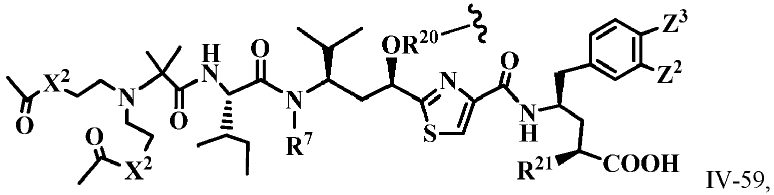 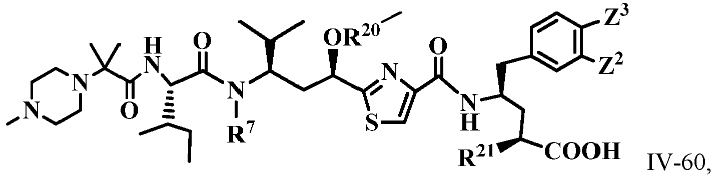 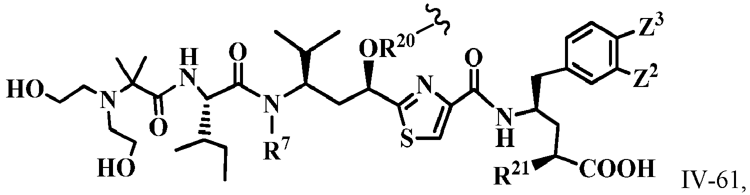 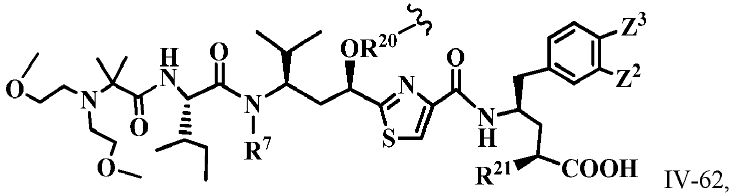 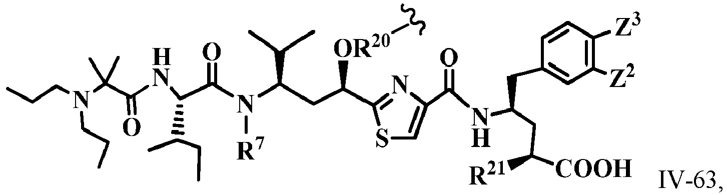 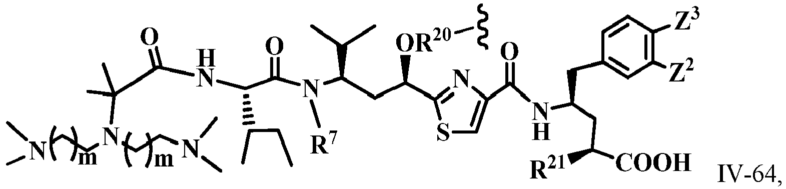 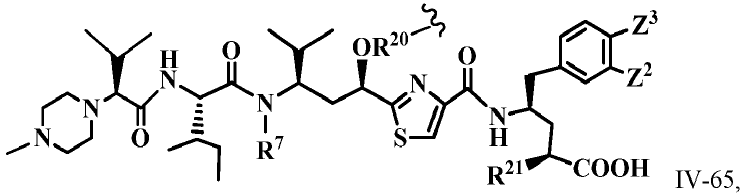 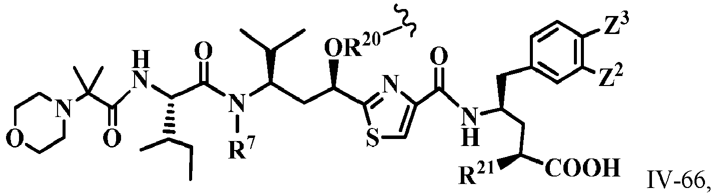 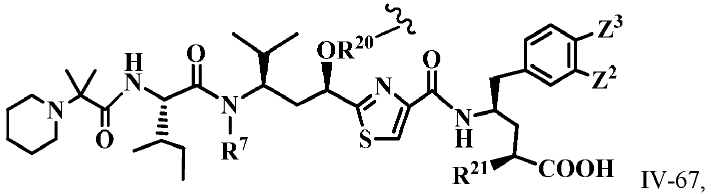 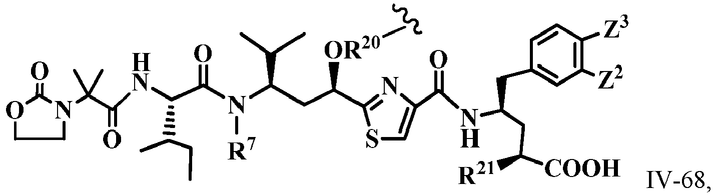
or their pharmaceutically acceptable salts, hydrates, or hydrated salts; or the polymorphic crystalline structures of these compounds; or their optical isomers, racemates, diastereomers or enantiomers thereof;
wherein " 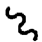 ", R⁷, R²⁰, R²¹, R²², Z², Z³, and X² are defined the same as above.

In another embodiment, a cell-binding molecule -antimitotic agent conjugate has the formula (V): 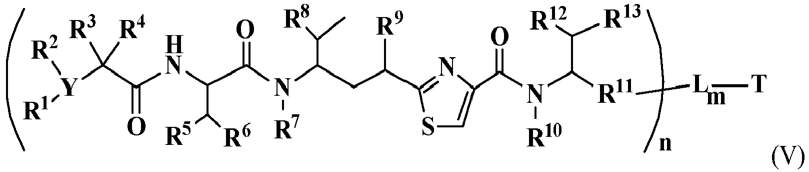
or their pharmaceutically acceptable salts, hydrates, or hydrated salts; or the polymorphic crystalline structures of these compounds; or their isotopes, optical isomers, racemates, diastereomers or enantiomers thereof;
wherein T, L, m, Y, R¹, R², R³, R⁴, R⁵, R⁶, R⁷, R⁸, R⁹, R¹⁰, R¹², R¹³ and n are defined the same as in Formula (II);
Wherein R¹¹ is -R¹⁴-, -R¹⁴C(=O)R¹⁷-, -R¹⁴C(=O)X²R¹⁷-, -R¹⁴X²R¹⁷-, - R¹⁴C(=O)X²-, wherein R¹⁷ is independently H, OH, C₁∼C₈ of alkyl; C₂∼C₈ of alkenyl, alkynyl, heteroalkyl; C₃∼C₈ of aryl, arylene, heterocyclic, carbocyclic, heterocycloalkyl; or an amino acid, or two amino acid units; X² is -O-, -S-, -NH-, -NHS(O₂)-, -NHS(O)-, -N(R¹⁴)-, -O-R¹⁴-, -S-R¹⁴-, -S(=O)-R¹⁴-, or -NHR¹⁴-; R¹⁴ is H, C₁∼C₈ of alkyl, heteroalkyl; C₂∼C₈ of alkenyl, alkynyl; C₃∼C₈ of aryl, heterocyclic, carbocyclic, cycloalkyl, alkylcycloalkyl, heterocycloalkyl, heteroalkylcycloalkyl, heteroaralkyl, alkylcarbonyl.

Illustrative compounds inside the bracket of Formula (V) have the structures: 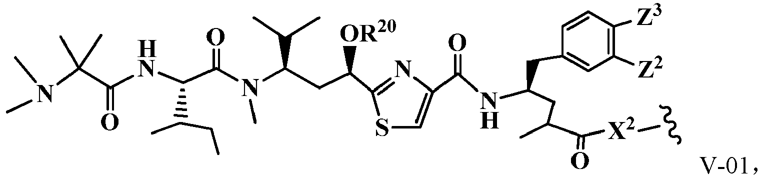 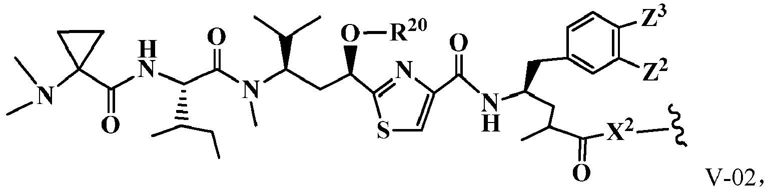 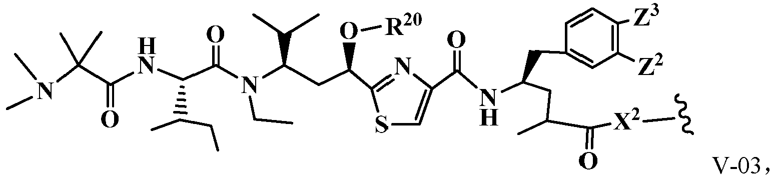 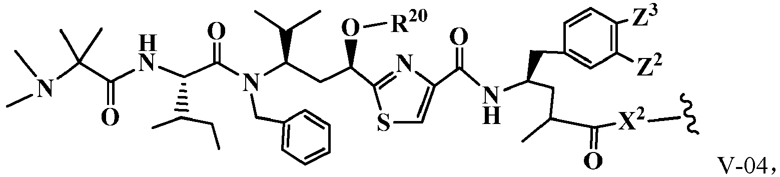 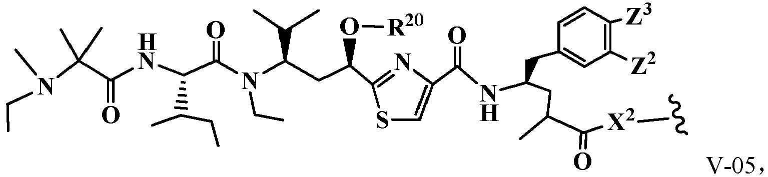 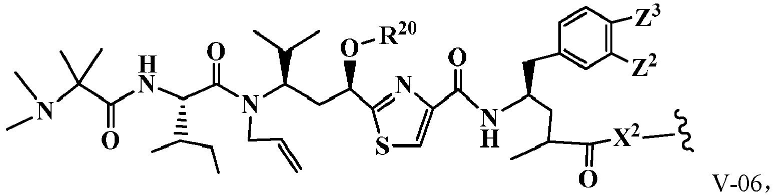 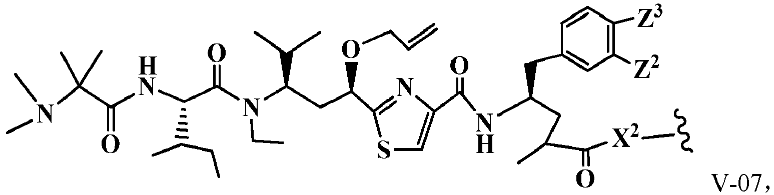 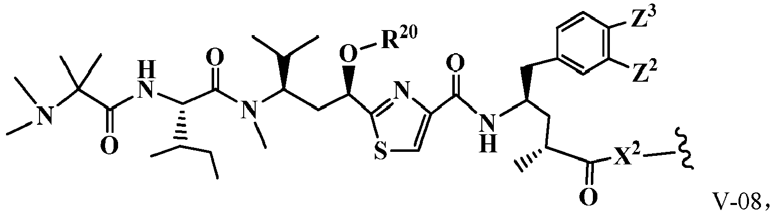 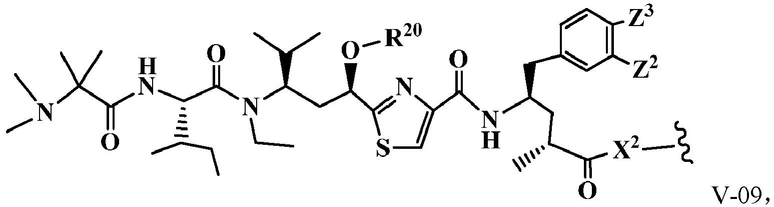 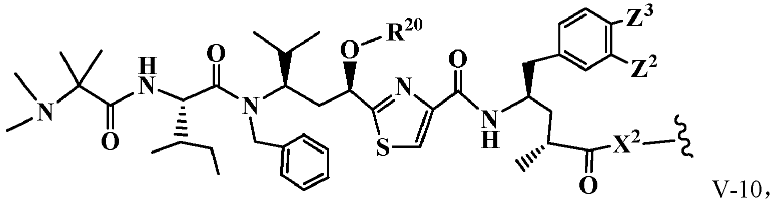 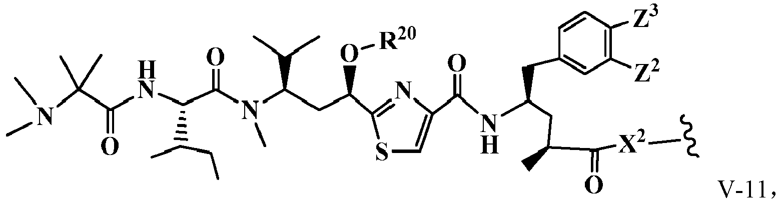 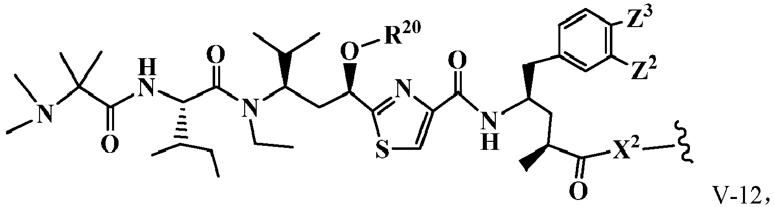 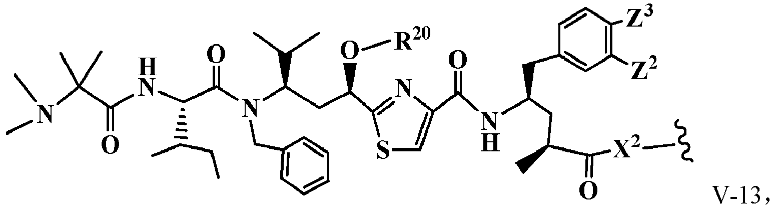 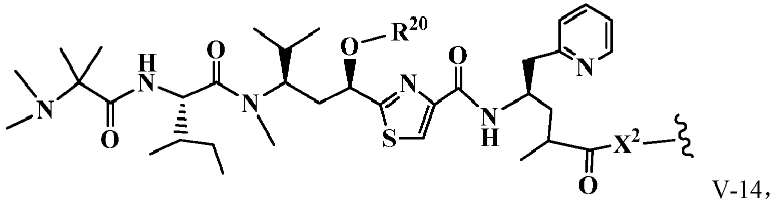 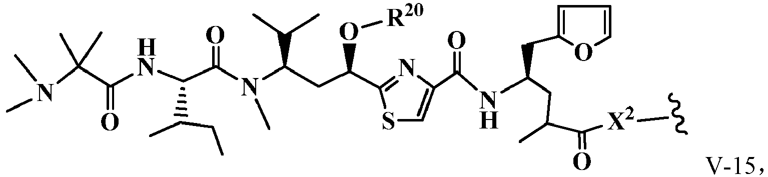 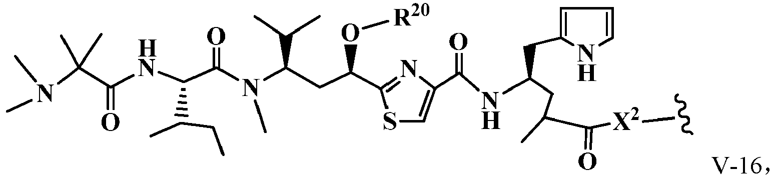 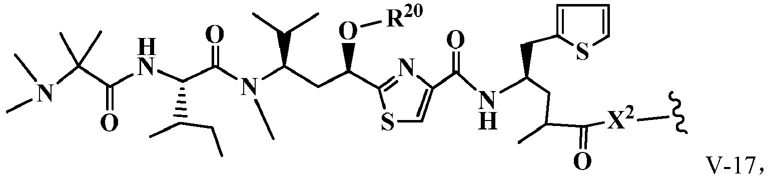 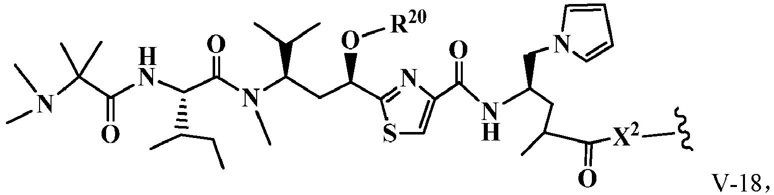 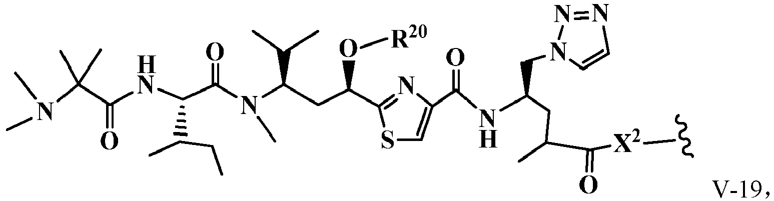 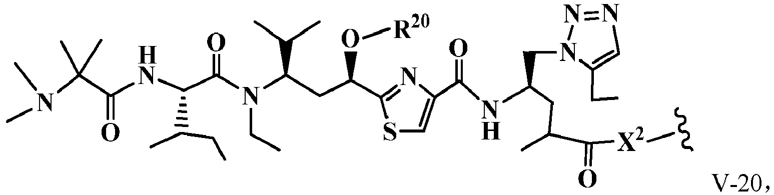 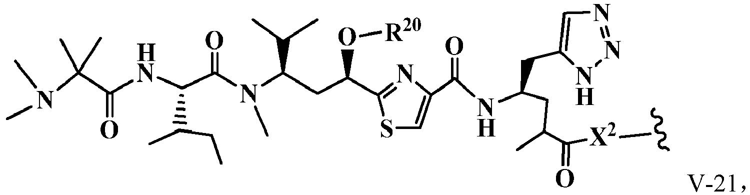 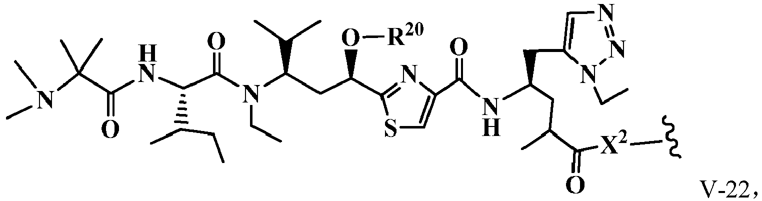 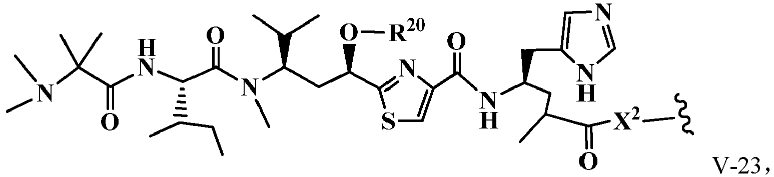 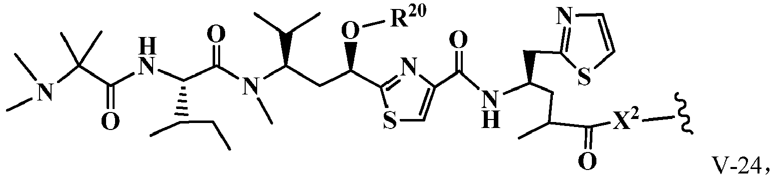 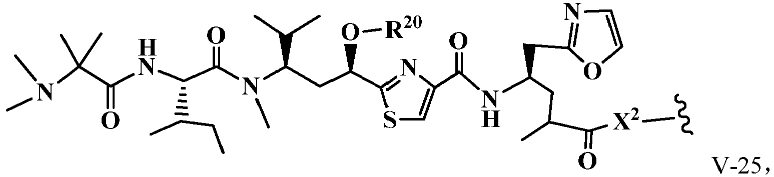 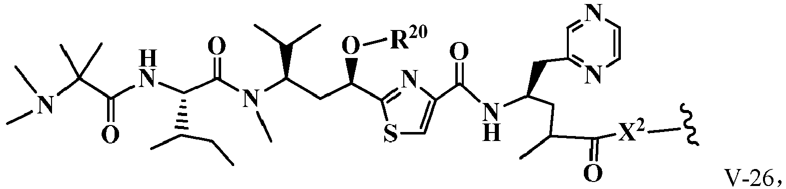 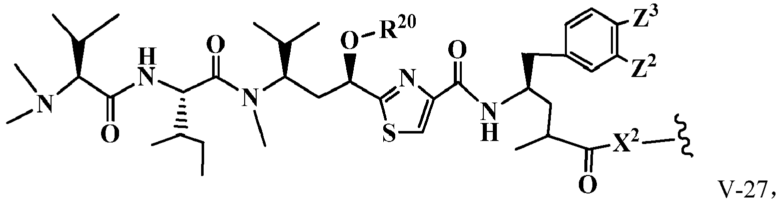 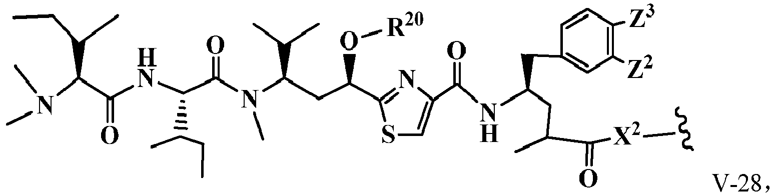 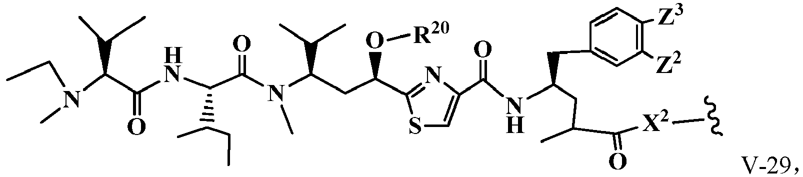 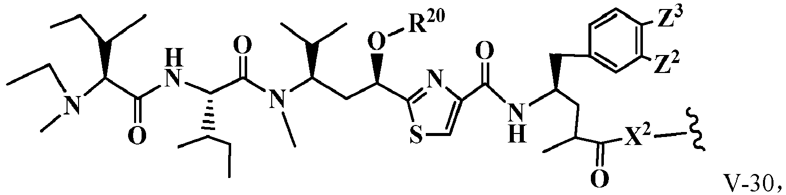 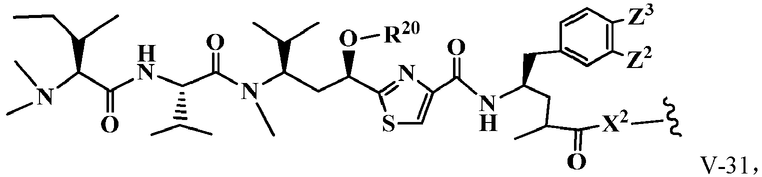 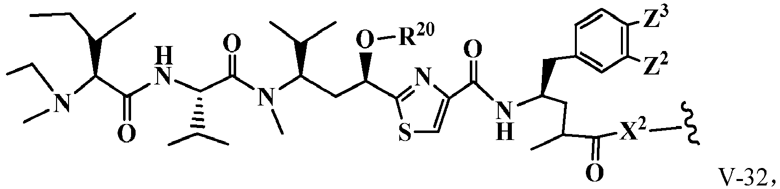 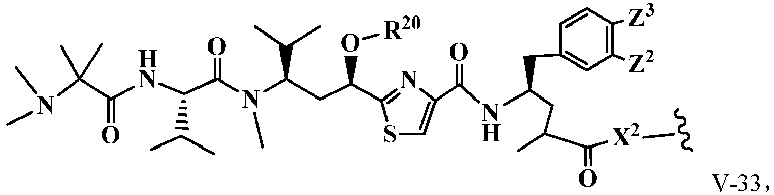 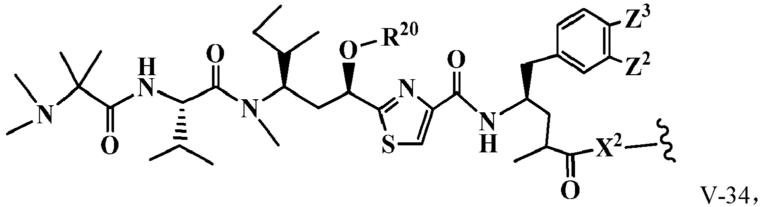 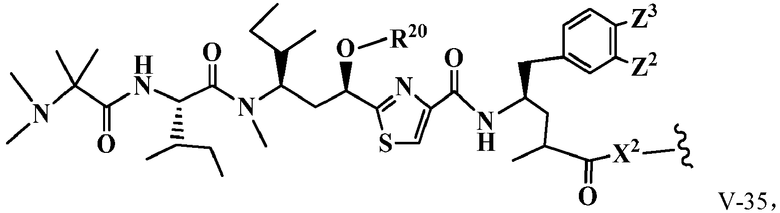 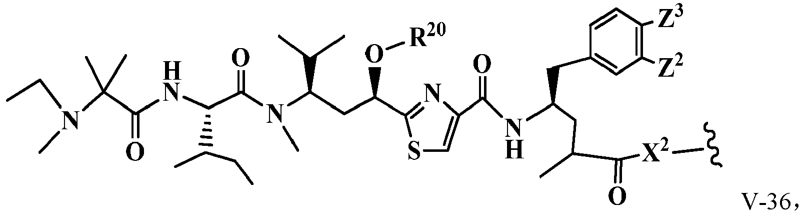 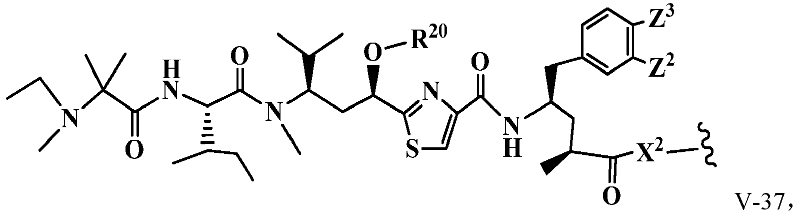 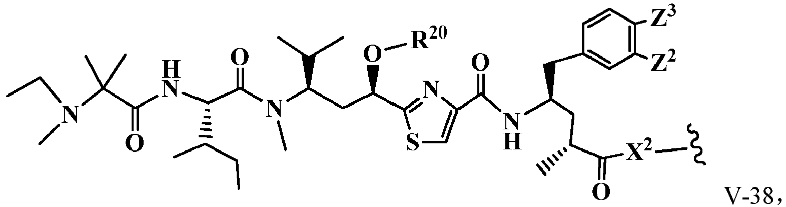 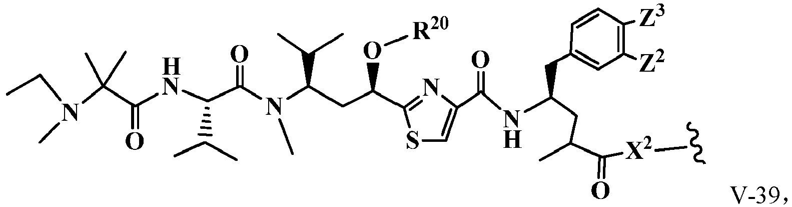 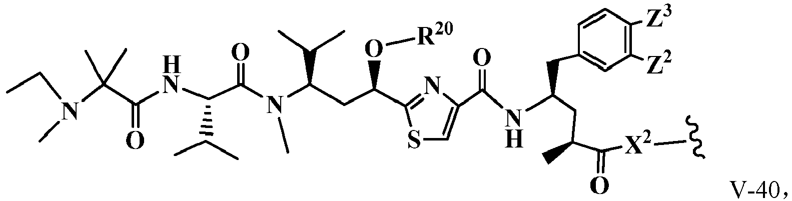 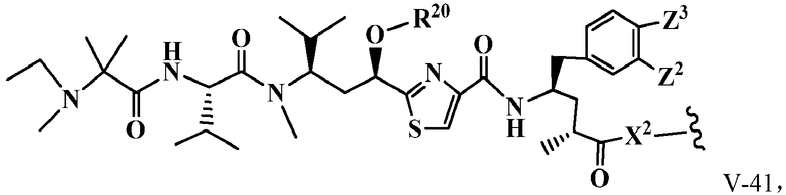 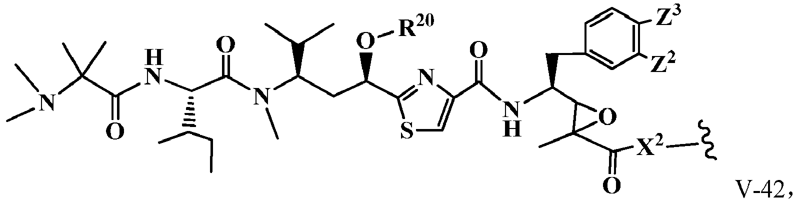 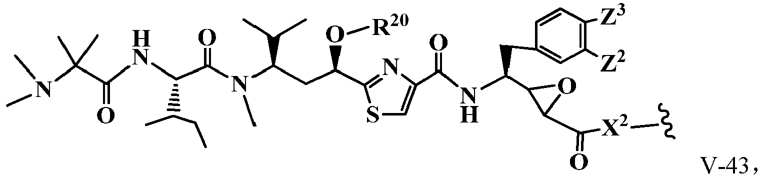 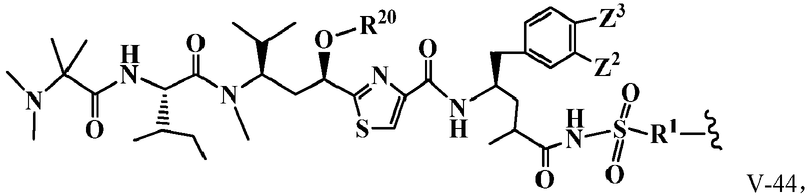 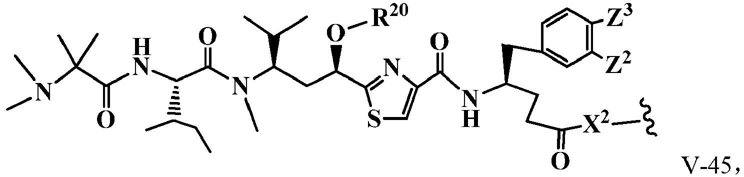 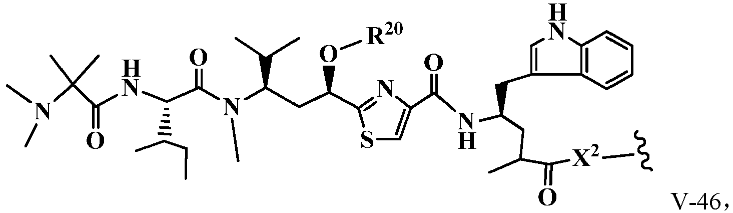 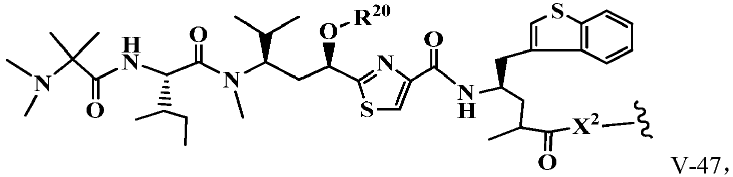 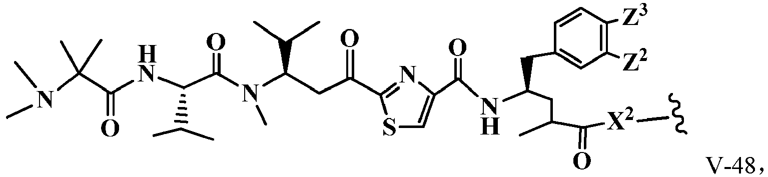 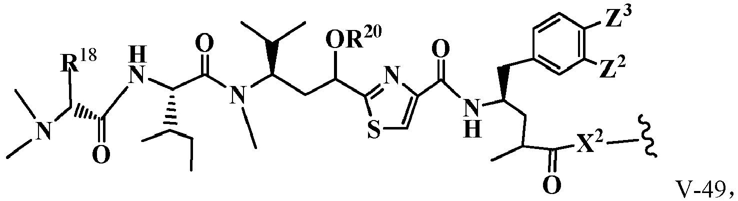 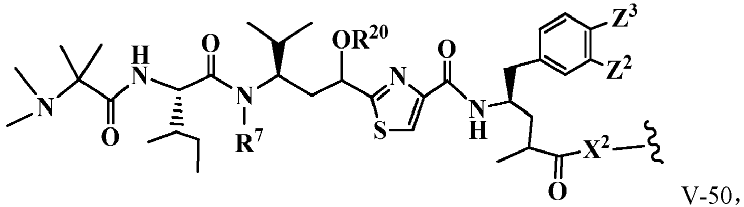 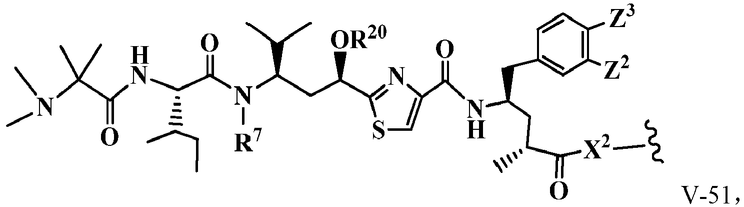 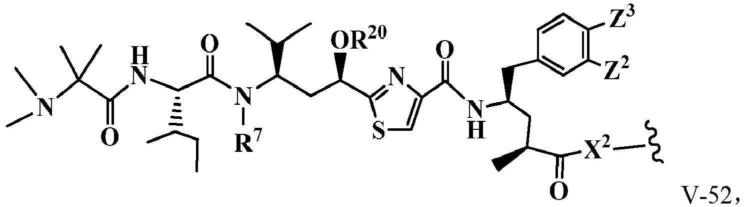 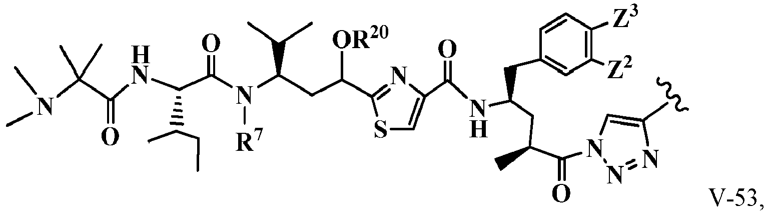 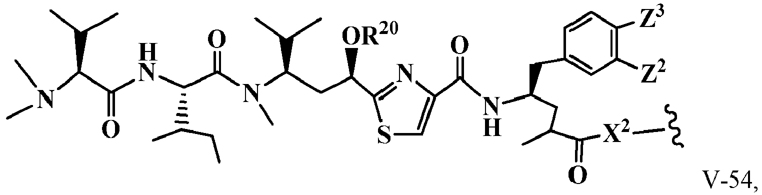 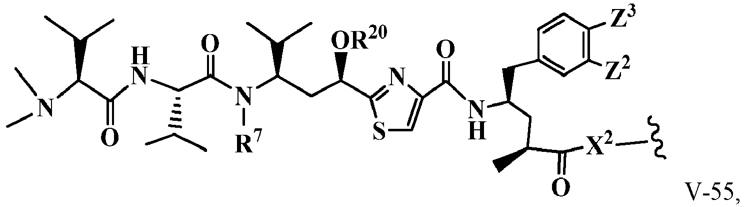 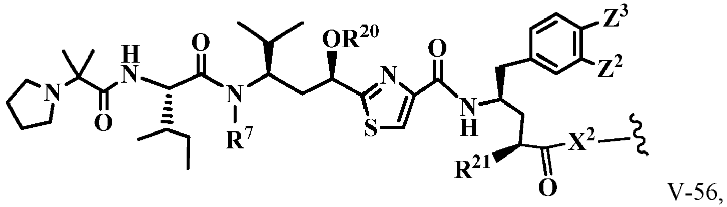 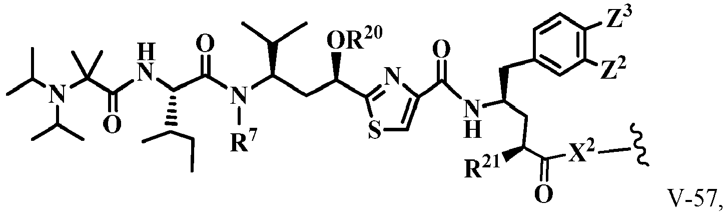 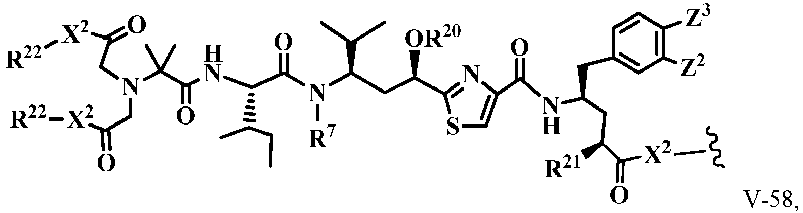 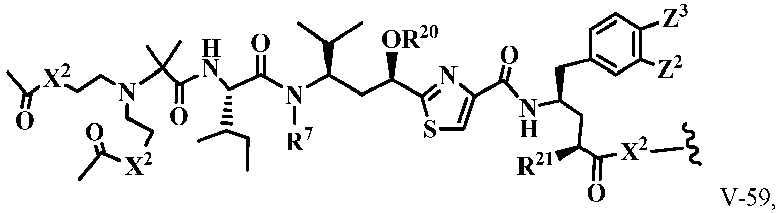 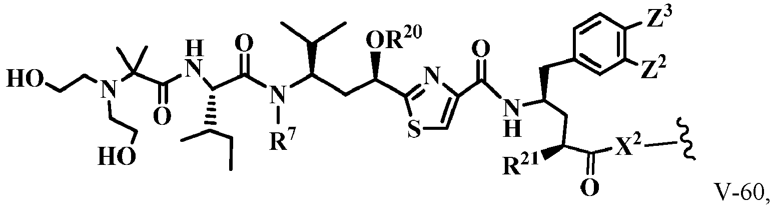 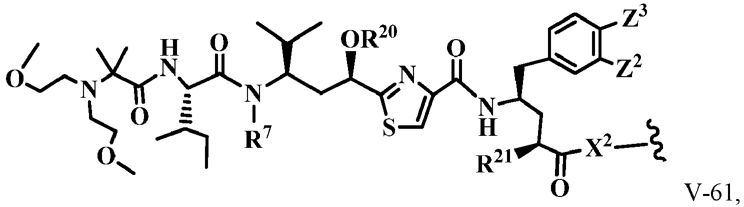 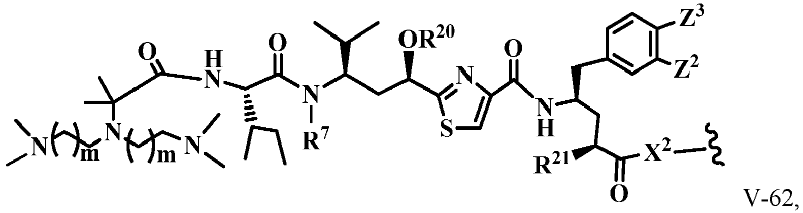 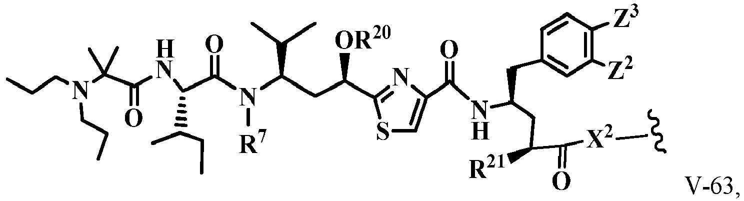 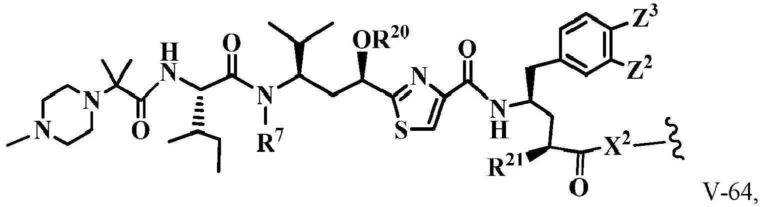 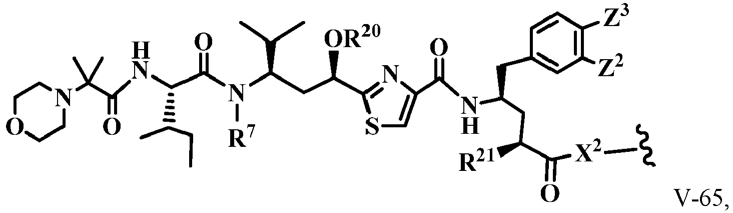 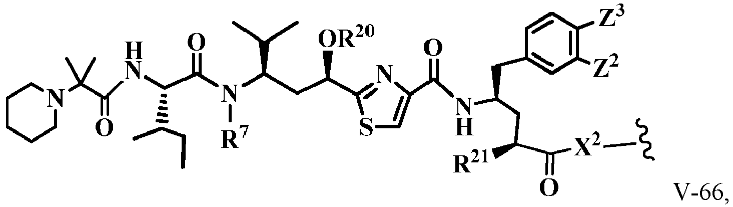 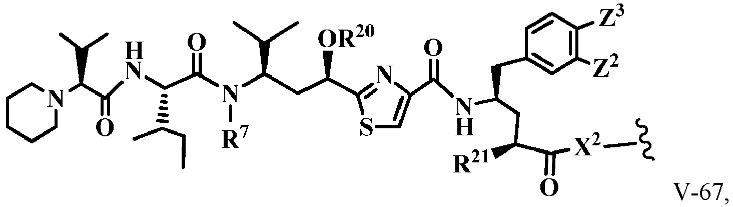 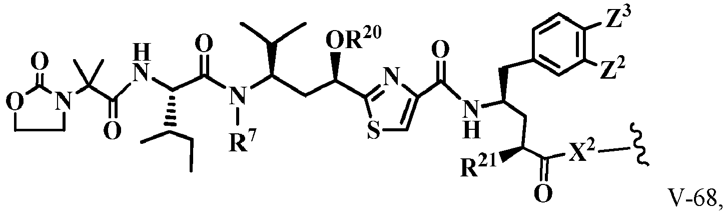
or their pharmaceutically acceptable salts, hydrates, or hydrated salts; or the polymorphic crystalline structures of these compounds; or their optical isomers, racemates, diastereomers or enantiomers thereof;
wherein " 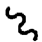 ", R⁷, R²⁰, R²¹, R²², Z², Z³, and X² are defined the same as above.

In another embodiment, a conjugates of a cell binding-antimitotic agent have the formula (VI) 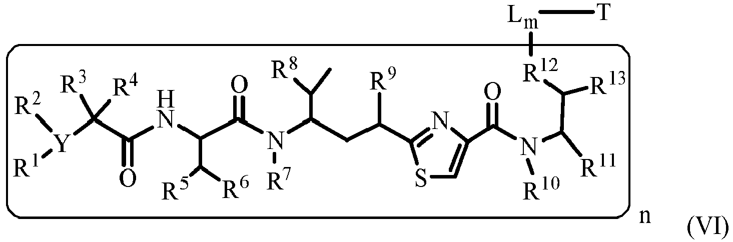
or their pharmaceutically acceptable salts, hydrates, or hydrated salts; or the polymorphic crystalline structures of these compounds; or their optical isomers, racemates, diastereomers or enantiomers thereof;
wherein T, L, m, Y, R¹, R², R³, R⁴, R⁵, R⁶, R⁷, R⁸, R⁹, R¹¹, R¹³ and n are defined the same as in Formula (II);
wherein R¹² is independently R¹⁴, -O-, -S-, -NH-, =N-, =NNH-, -N(R¹⁴)-, -OR¹⁴-, C(O)O-, C(O)NH-, C(O)NR¹⁴-, -SR¹⁴-, -S(=O)R¹⁴-, -NHR¹⁴-, -CH₂OP(=O)(OR¹⁵)-, -P(=O)(OR¹⁵)-,-OP(=O)(OR¹⁵)O-, -SO₂R¹⁴, R¹⁴, R¹⁵ are independently C₁∼C₈ of alkyl, heteroalkyl; C₂∼C₈ of alkenyl, alkynyl; C₃∼C₈ of aryl, heterocyclic, carbocyclic, cycloalkyl, alkylcycloalkyl, heterocycloalkyl, heteroaralkyl, heteroalkylcycloalkyl, alkylcarbonyl.

Illustrative compounds inside the bracket of Formula (VI) have the structures: 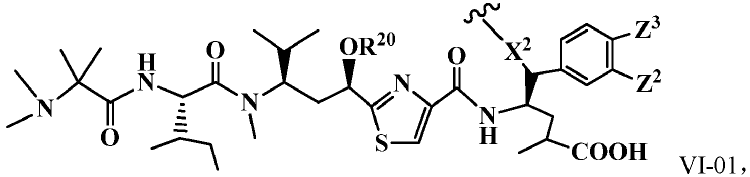 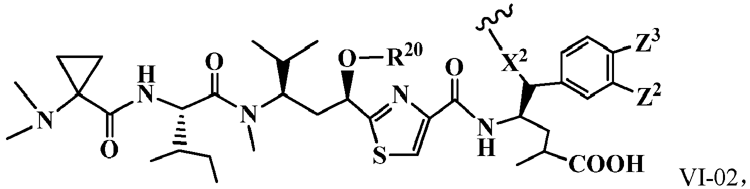 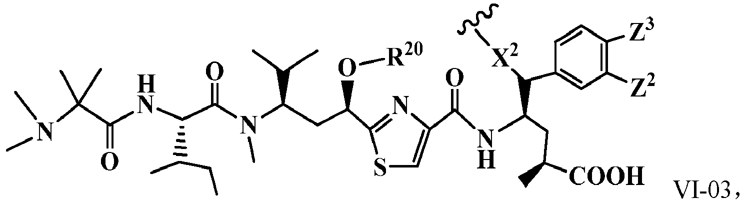 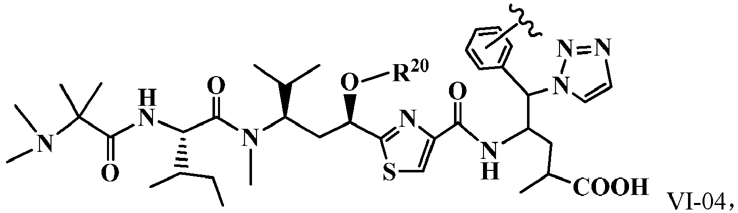 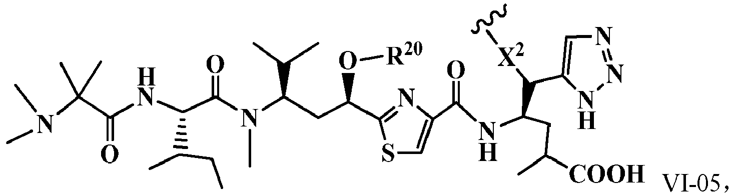 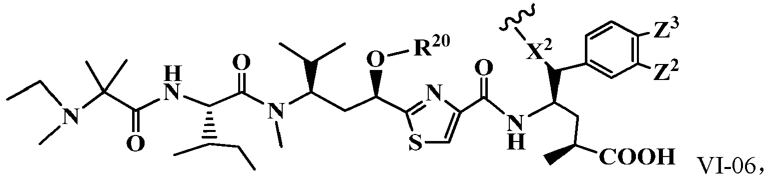 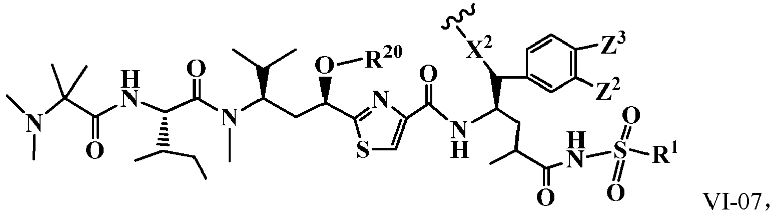 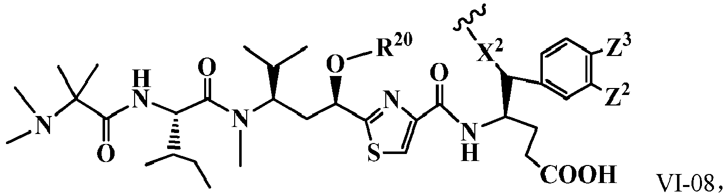 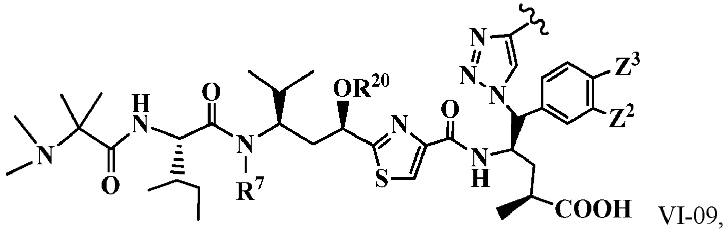 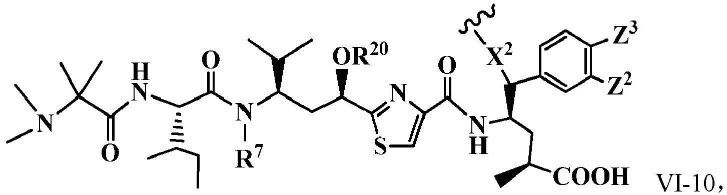 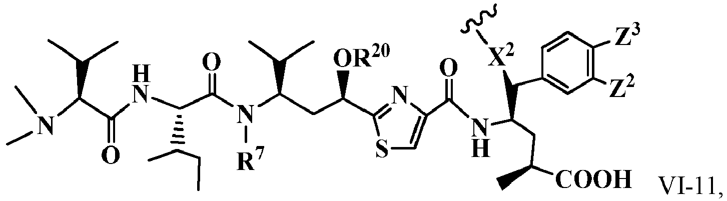 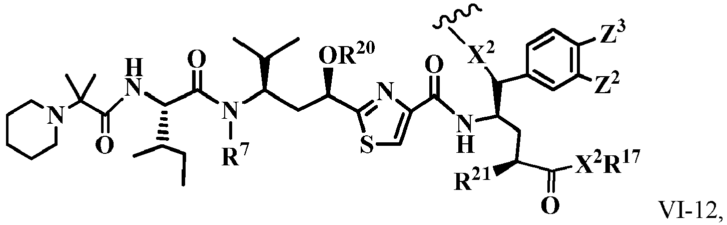
or their pharmaceutically acceptable salts, hydrates, or hydrated salts; or the polymorphic crystalline structures of these compounds; or their optical isomers, racemates, diastereomers or enantiomers thereof;
wherein R⁷, R¹⁷, R²⁰, R²¹, Z², Z³, and X² are defined the same as above.

In another embodiment, the conjugates of the cell-surface binding molecule-antimitotic agents have the Formula (VII): 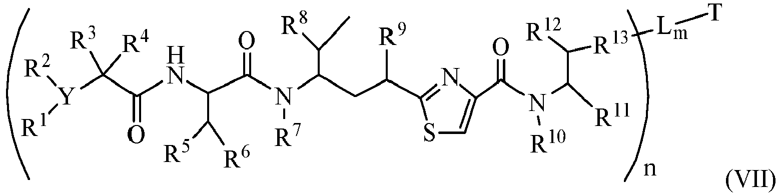
or their pharmaceutically acceptable salts, hydrates, or hydrated salts; or the polymorphic crystalline structures of these compounds; or their optical isomers, racemates, diastereomers or enantiomers thereof,
Wherein T, L, n, m, Y, R¹, R², R³, R⁴, R⁵, R⁶, R⁸, R¹⁰, R¹¹, and R¹² are defined the same as in Formula (II);
Wherein R¹³ is C₁∼C₁₀ of alkyl, heteroalkyl, alkyl acid, alkyl amide, alkyl amine, or Ar; Ar refers to a aromatic or hetero aromatic group, composed of one or several rings, comprising four to ten carbon, preferentially four to six carbon atoms. The term of hetero aromatic group refers one or several carbon on aromatic group, preferentially one, two or three carbon atoms are replaced by O, N, Si, Se, P or S, preferentially O, S, N. The term aryl or Ar also refers to a aromatic group, wherein one or several H atoms are replaced independently by R¹⁸, F, Cl, Br, I, OR¹⁶, SR¹⁶, NR¹⁶R¹⁸, N=NR¹⁶, N=R¹⁶, NR¹⁶R¹⁸, NO₂, SOR¹⁶R¹⁸, SO₂R¹⁶, SO₃R¹⁶, OSO₃R¹⁶, PR¹⁶R¹⁸, POR¹⁶R¹⁸, PO₂R¹⁶R¹⁸, OPO₃R¹⁶R¹⁸, or PO₃R¹⁶R¹⁸ wherein R¹⁶, R¹⁸ are independently H, C₁∼C₈ of alkyl; C₂∼C₈ of alkenyl, alkynyl, heteroalkyl; C₃∼C₈ of aryl, heterocyclic, carbocyclic, cycloalkyl, alkylcycloalkyl, heterocycloalkyl, heteroaralkyl, heteroalkylcycloalkyl, alkylcarbonyl; or C₄ ∼ C₁₂ glycosides; or pharmaceutical salts.

Illustrative examples of compounds inside the bracket of Formula (VII) have the structures: 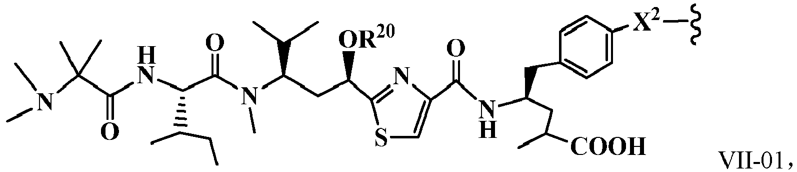 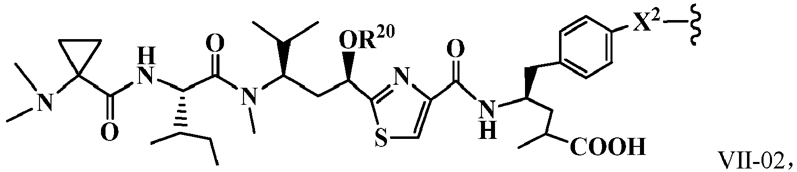 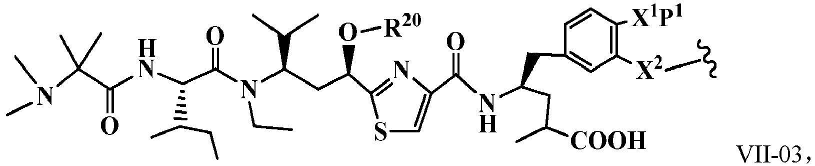 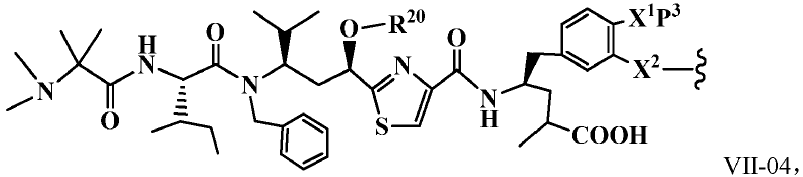 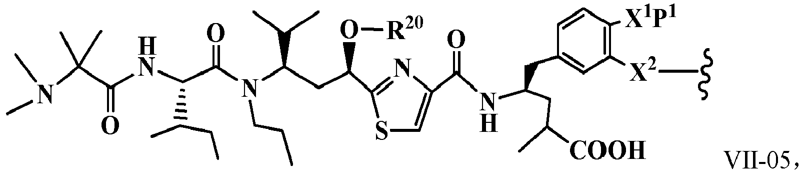 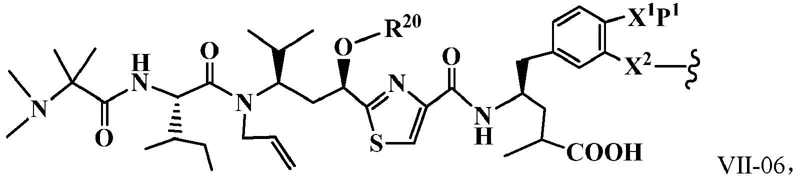 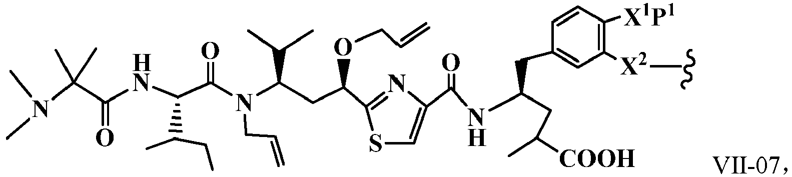 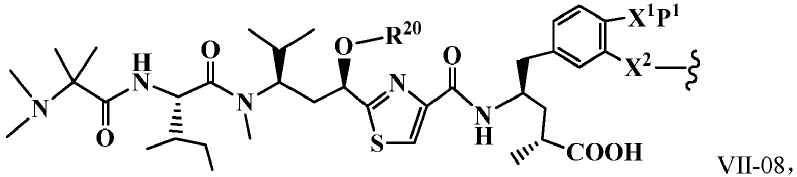 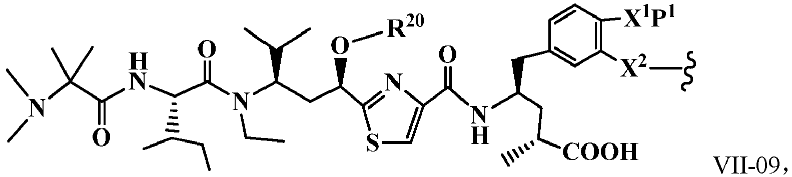 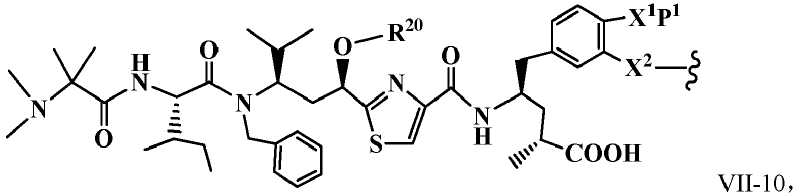 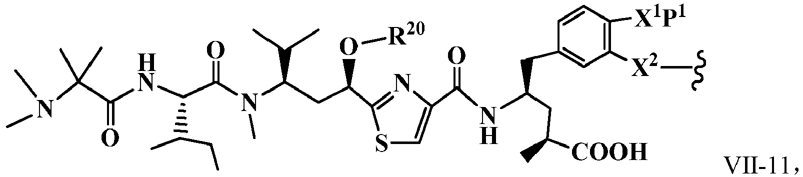 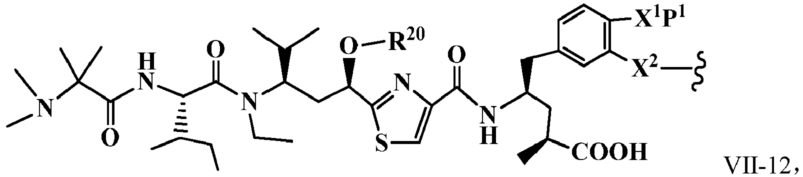 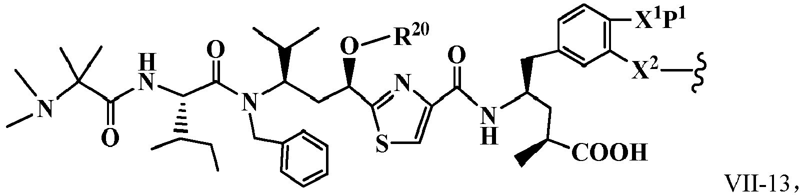 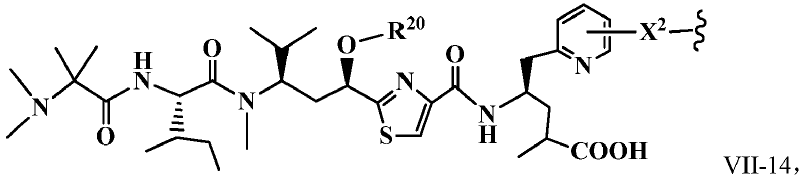 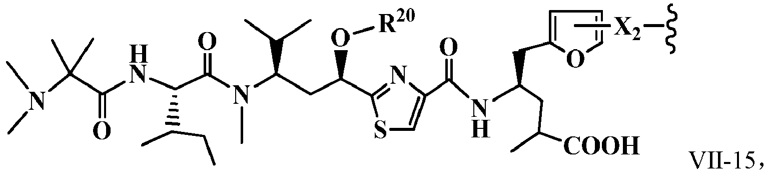 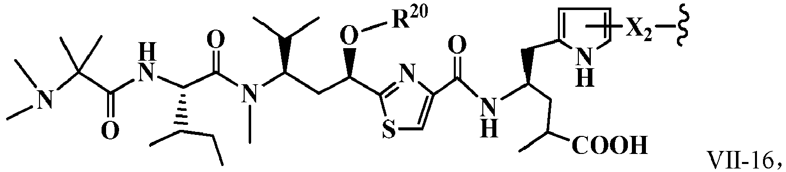 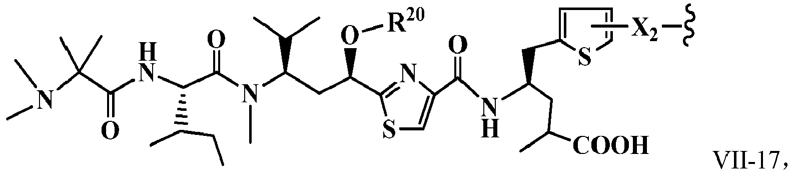 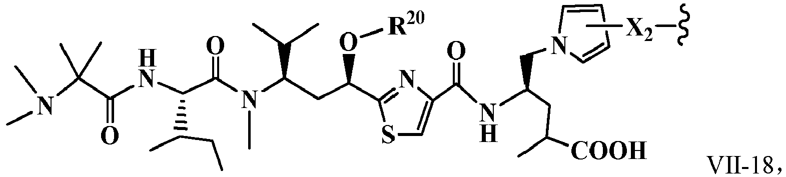 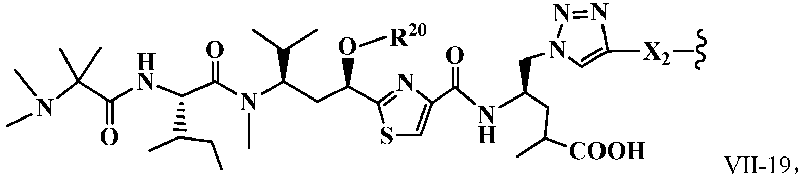 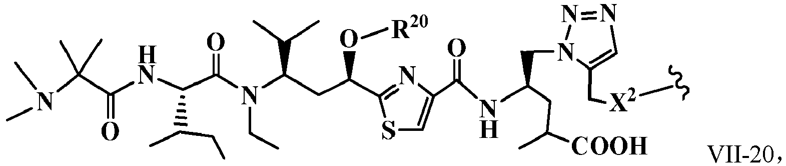 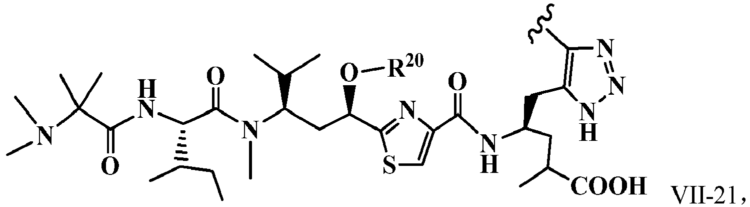 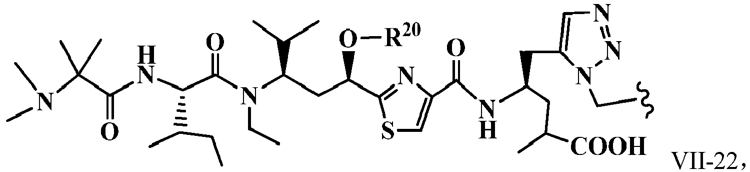 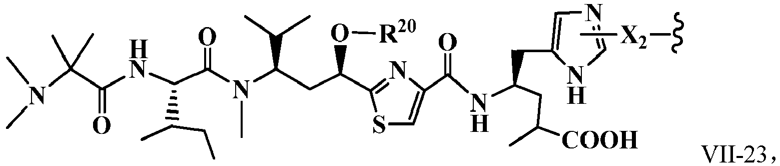 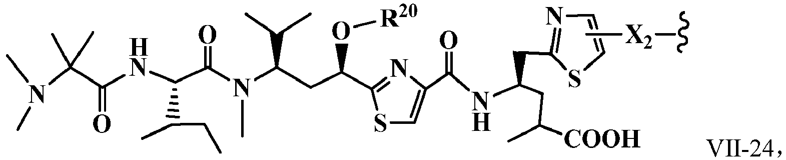 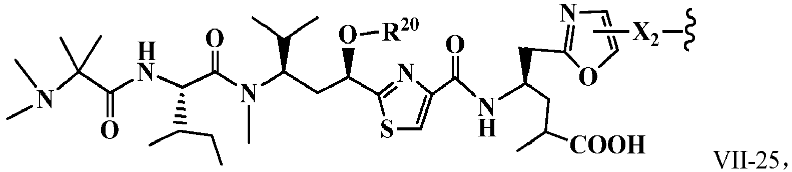 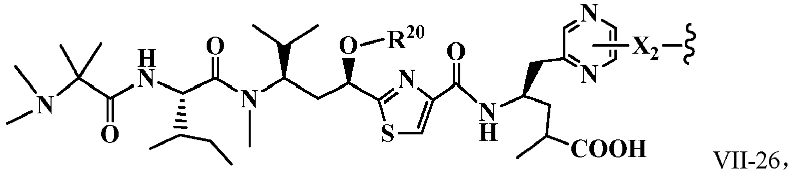 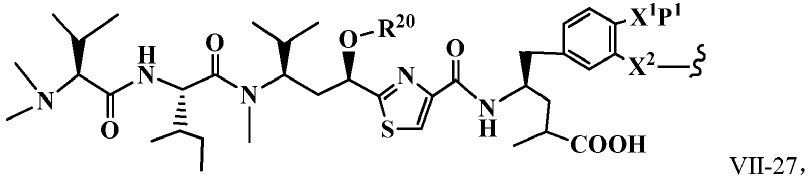 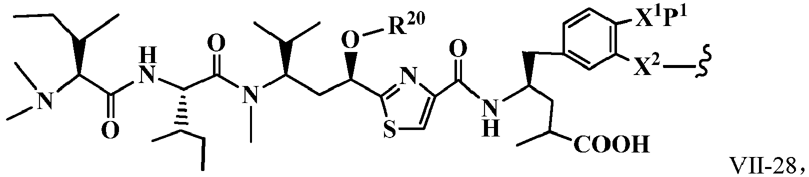 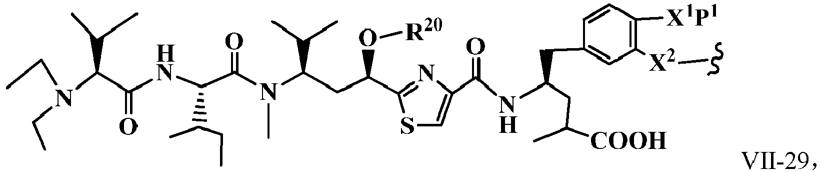 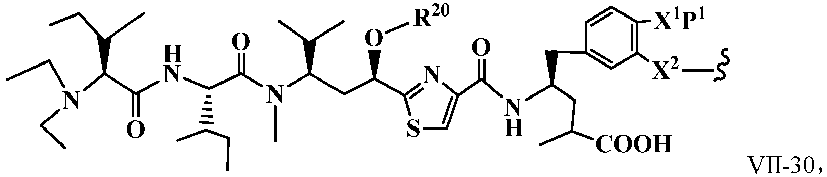 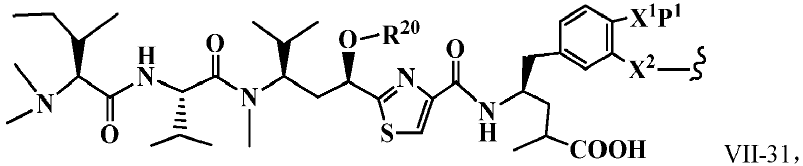 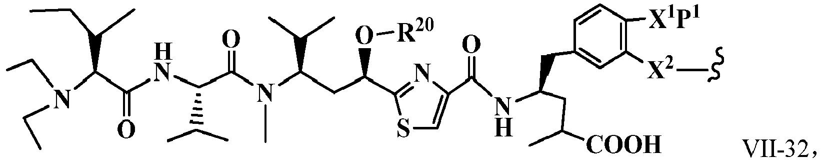 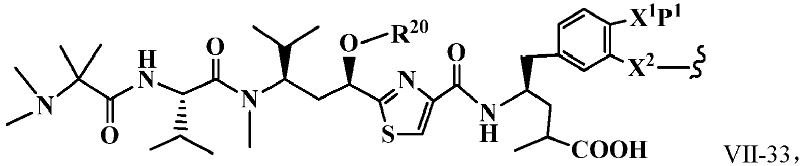 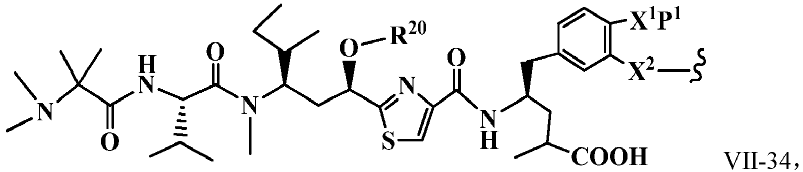 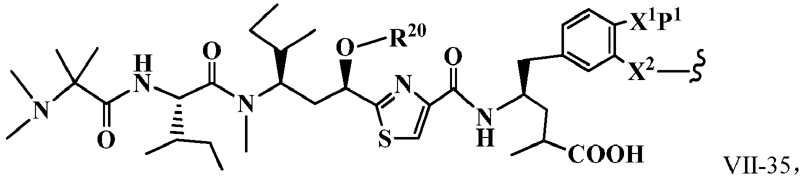 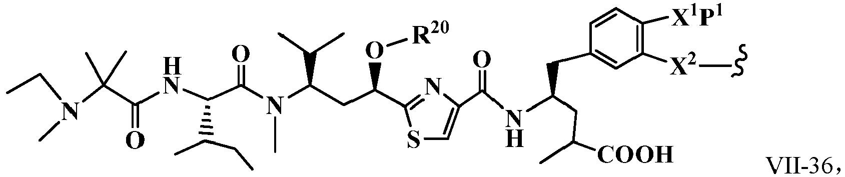 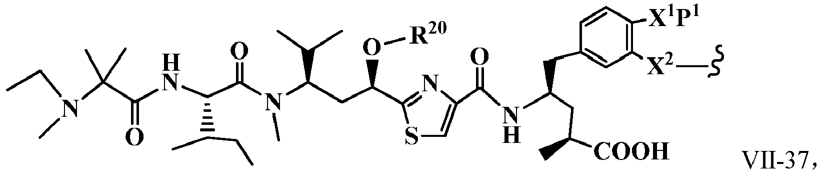 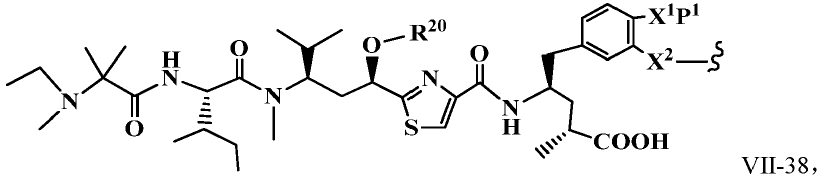 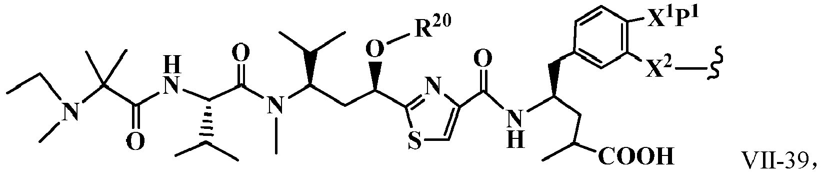 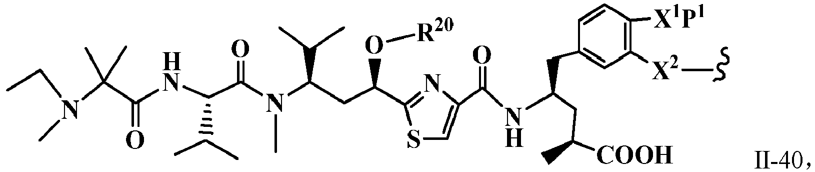 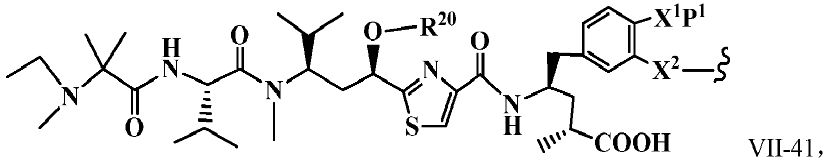 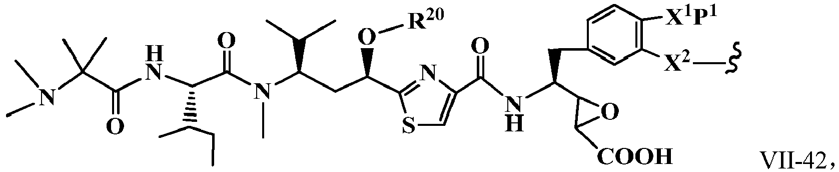 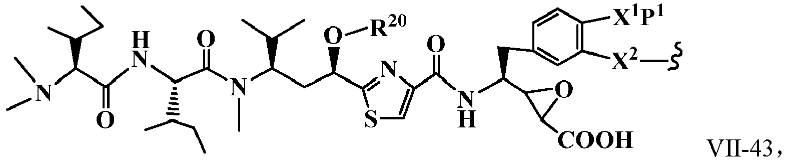 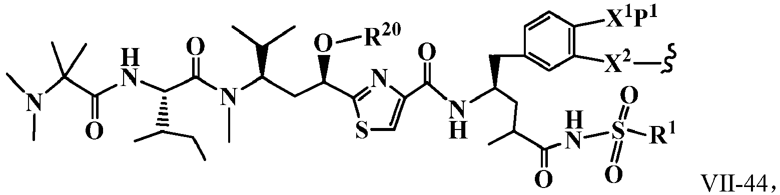 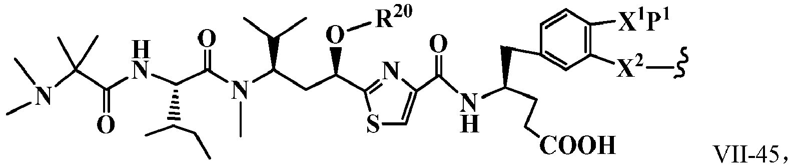 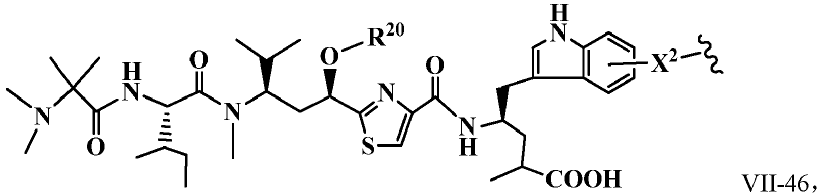 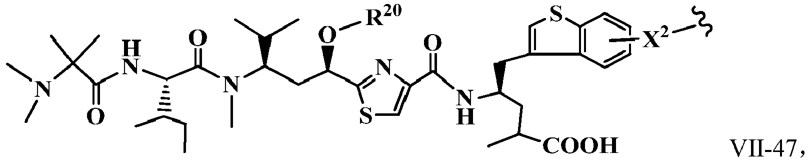 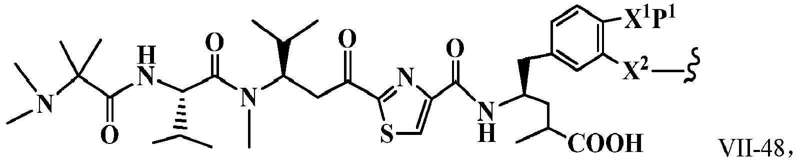 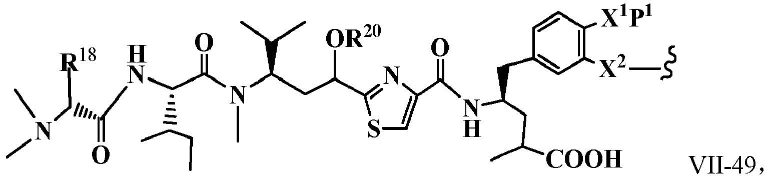 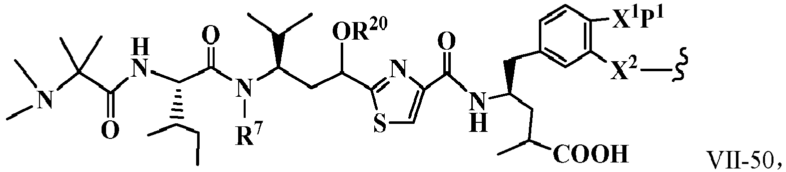 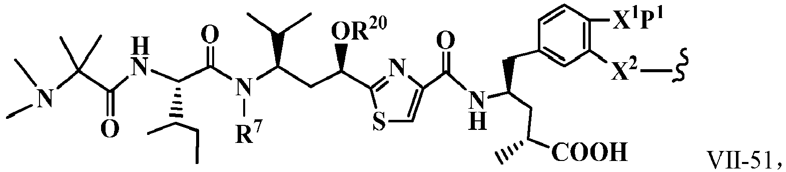 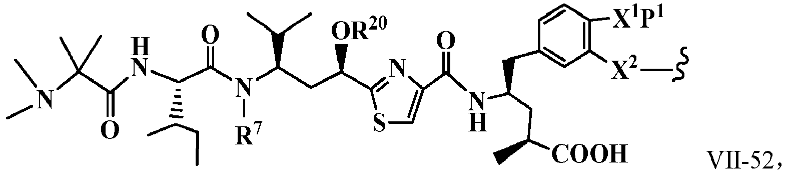 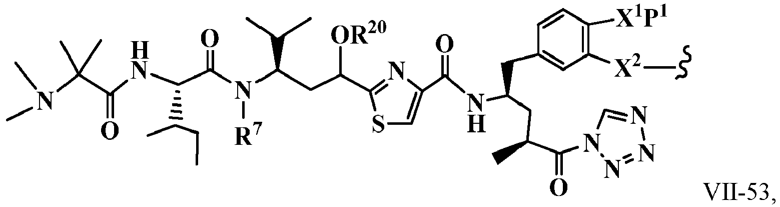 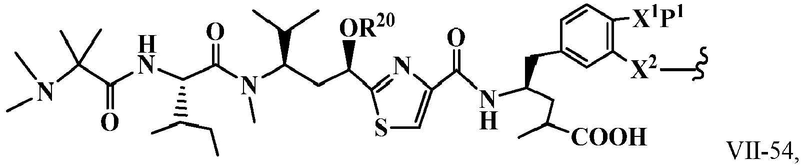 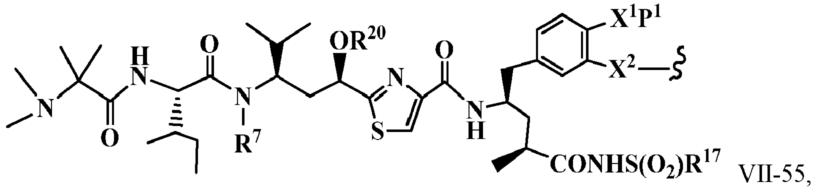 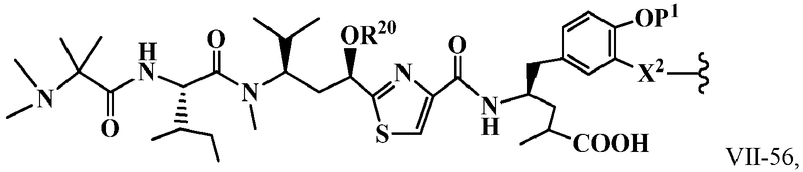 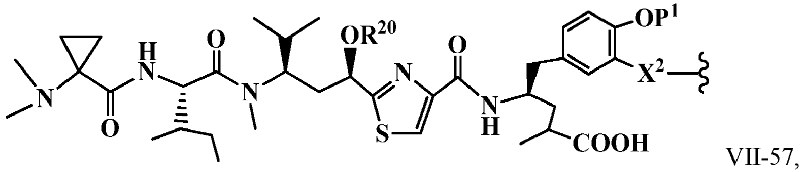 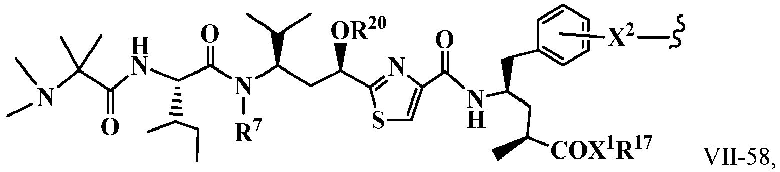 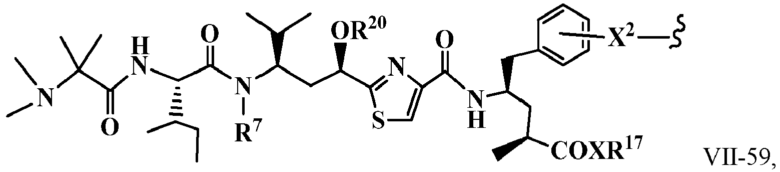 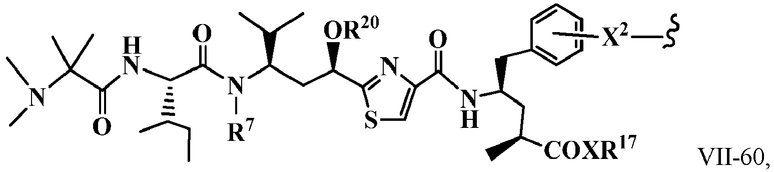 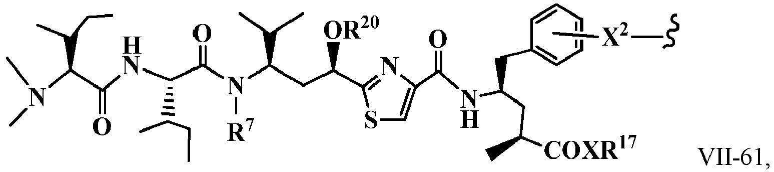 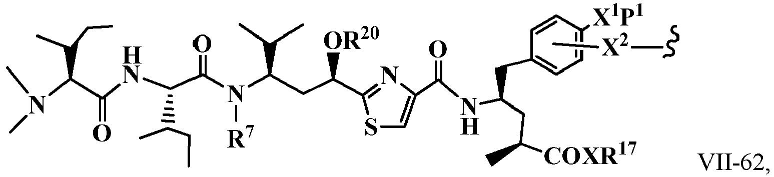 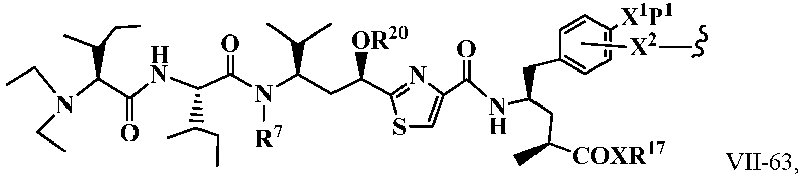 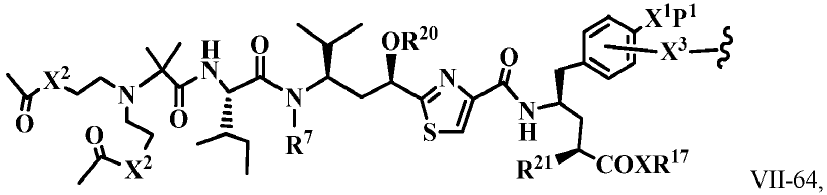 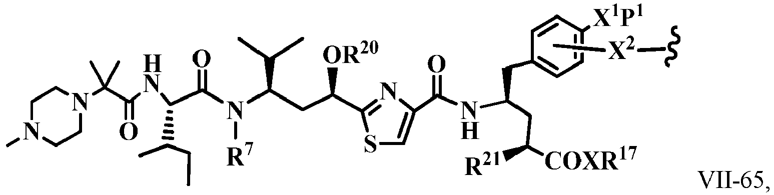 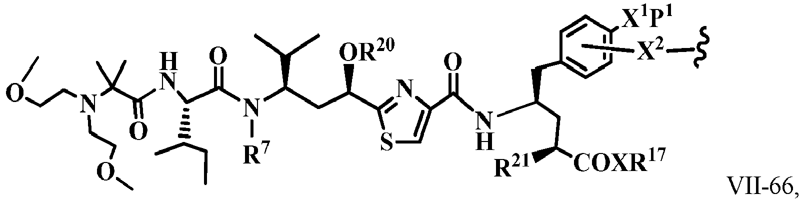 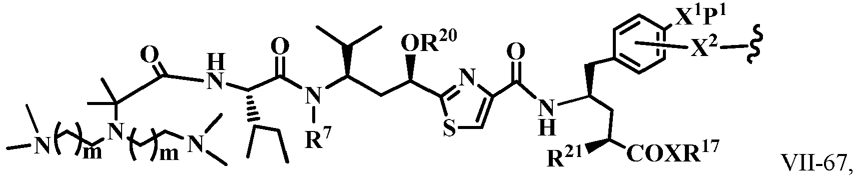 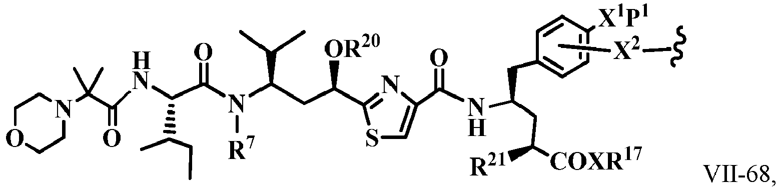 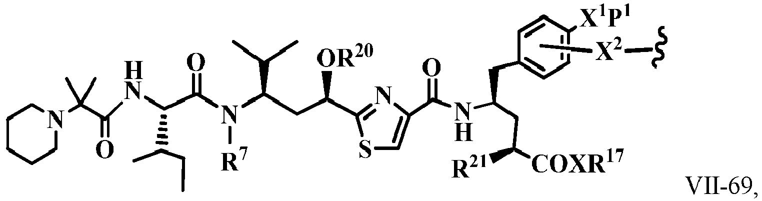 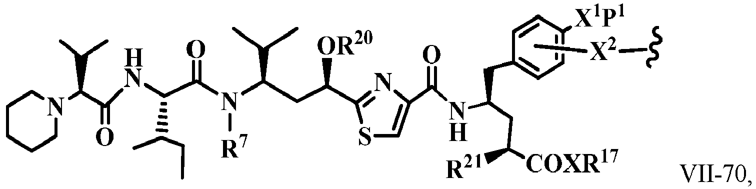 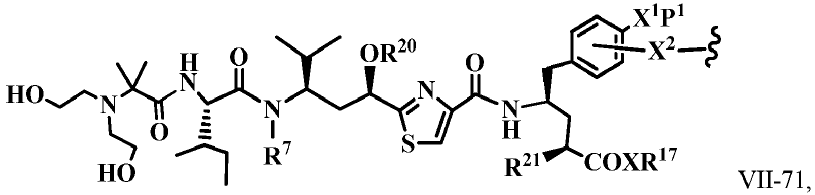 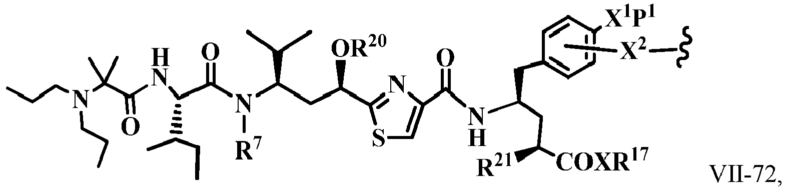 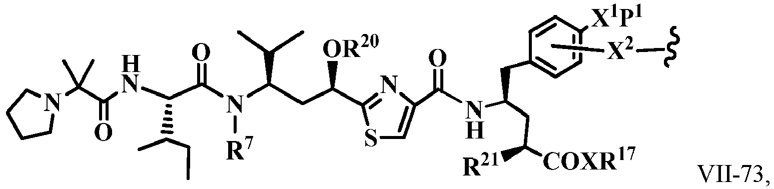 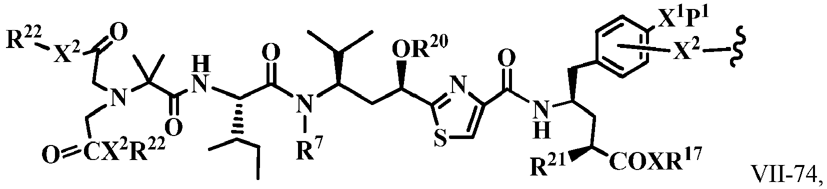 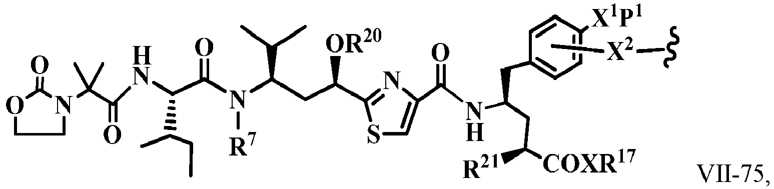 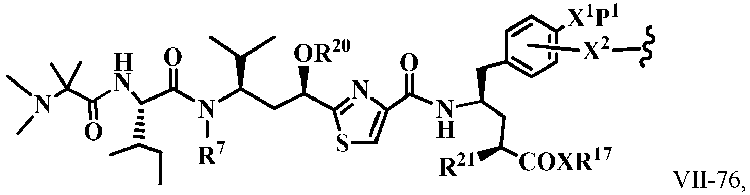 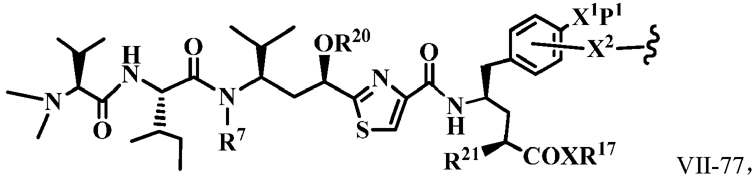
or their pharmaceutically acceptable salts, hydrates, or hydrated salts; or the polymorphic crystalline structures of these compounds; or their optical isomers, racemates, diastereomers or enantiomers thereof;
wherein " 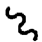 ", R⁷, R²⁰, Z², Z³, and X² are defined the same as above; X, X¹, and X³, are independently O, S, NH, NHNH, NHR¹⁷, CH₂ or absent; P¹ is H, R¹⁷, P(O)(OH)₂, P(O)(X¹R¹⁷)₂, CH₂P(O)(OH)₂, S(O²)(X¹R¹⁷), C₆H₁₂O₅ (glycoside), (CH₂CH₂O)ₚR¹⁷, wherein p is selected from 0 -100, and R¹⁷ is defined above; in addition X¹P¹ can be absent (together is H).

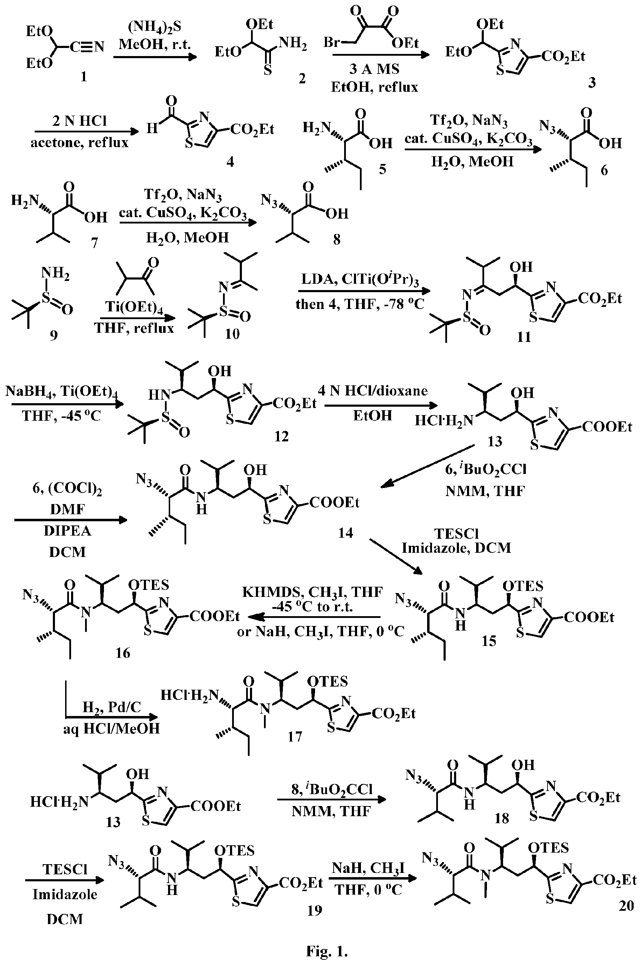

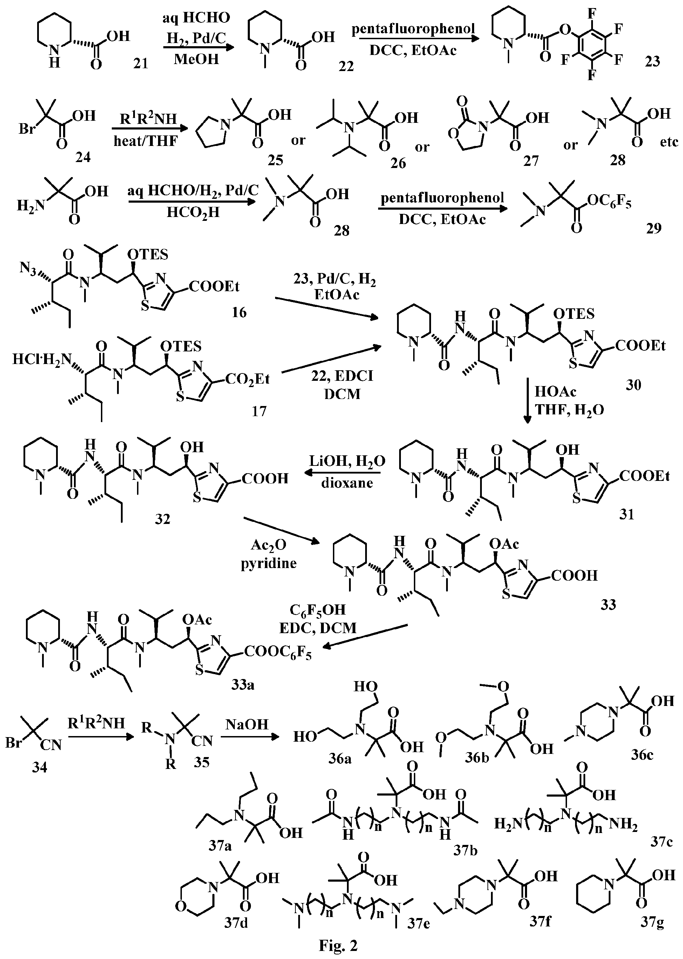

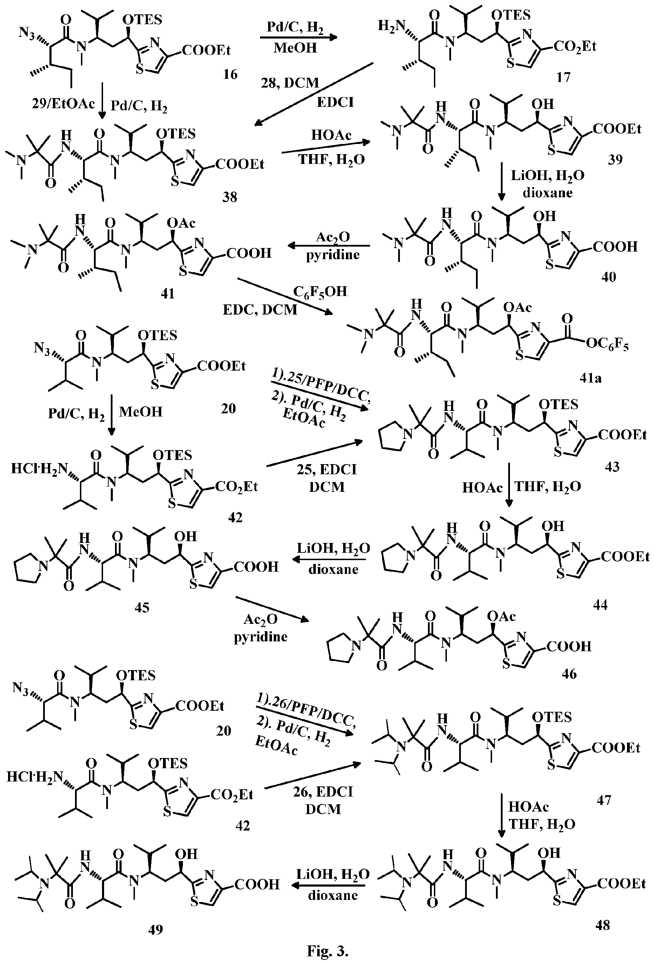

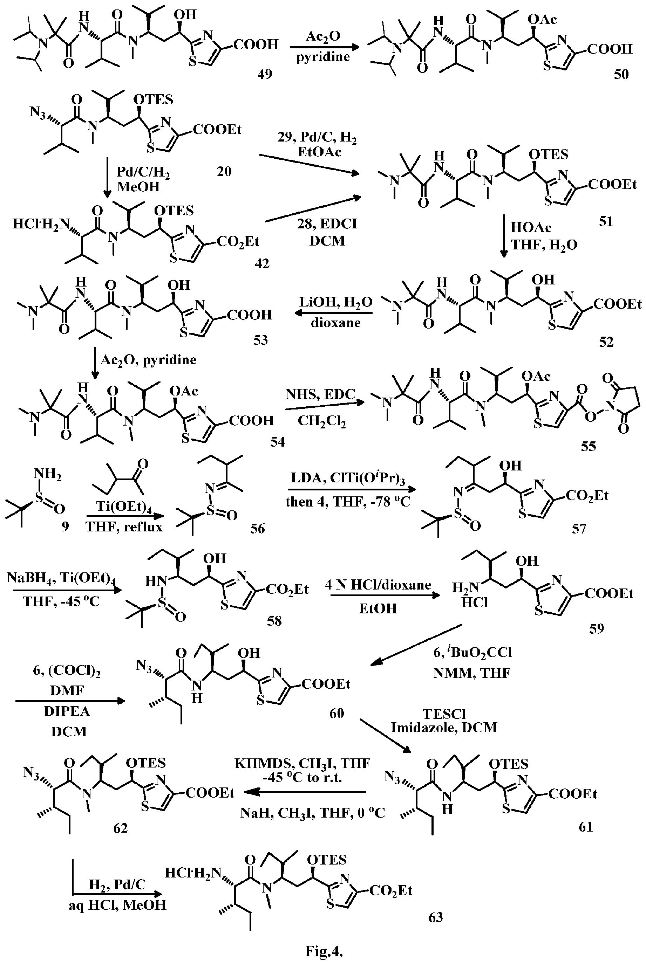

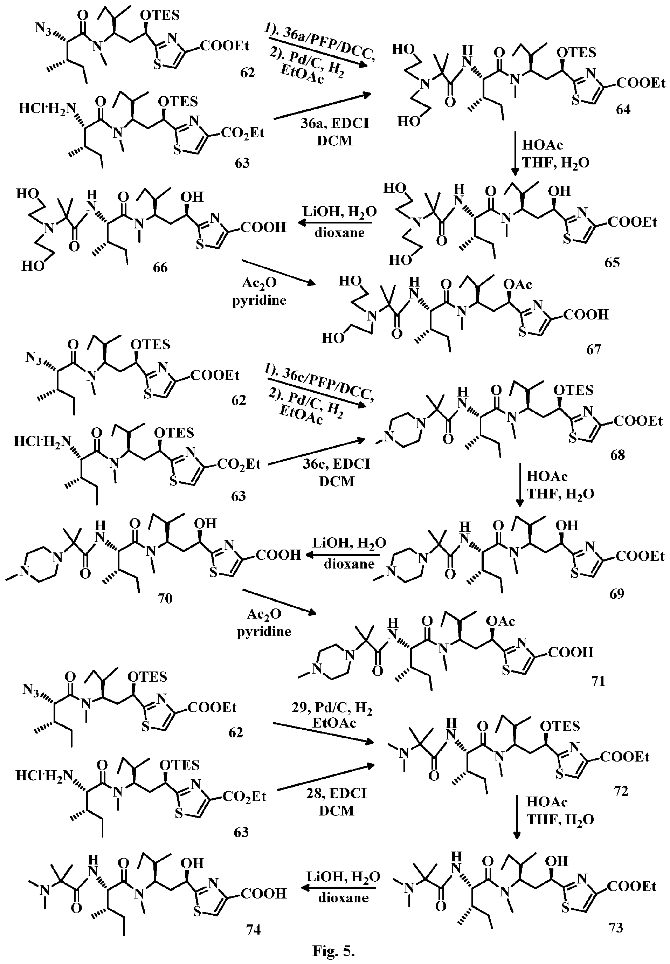

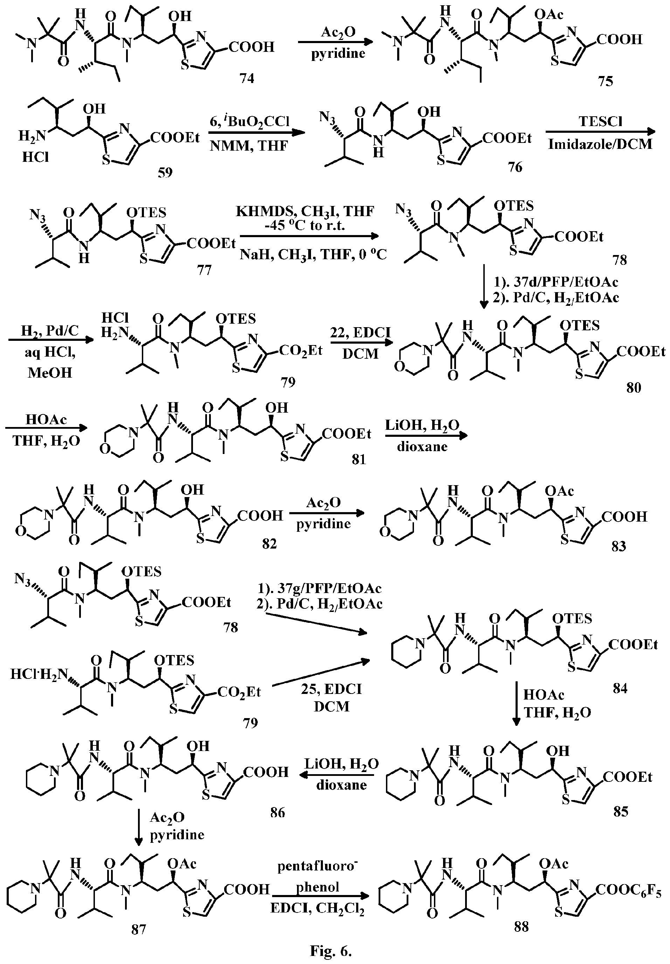

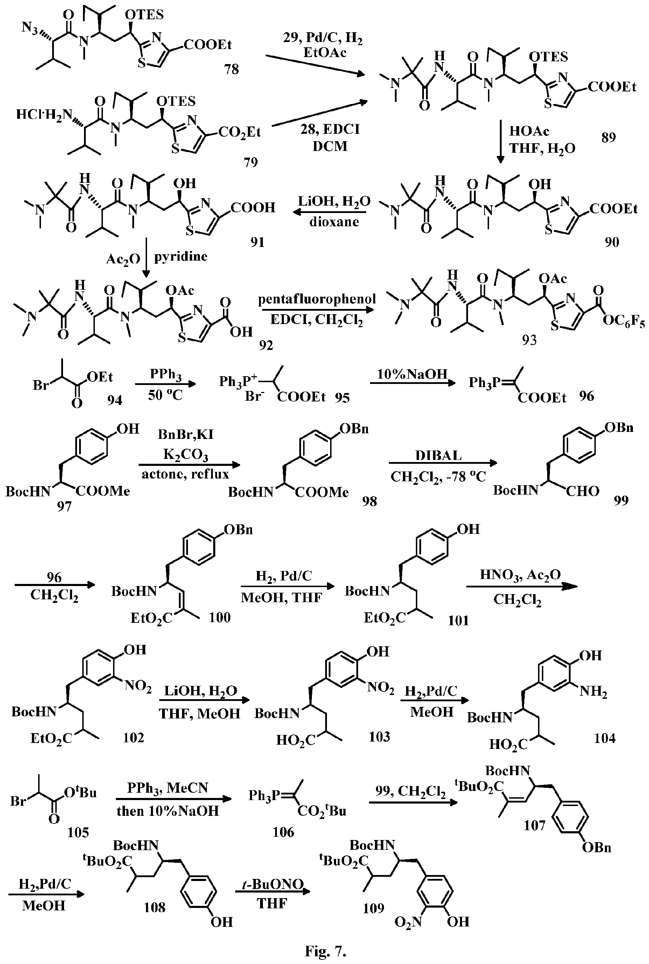

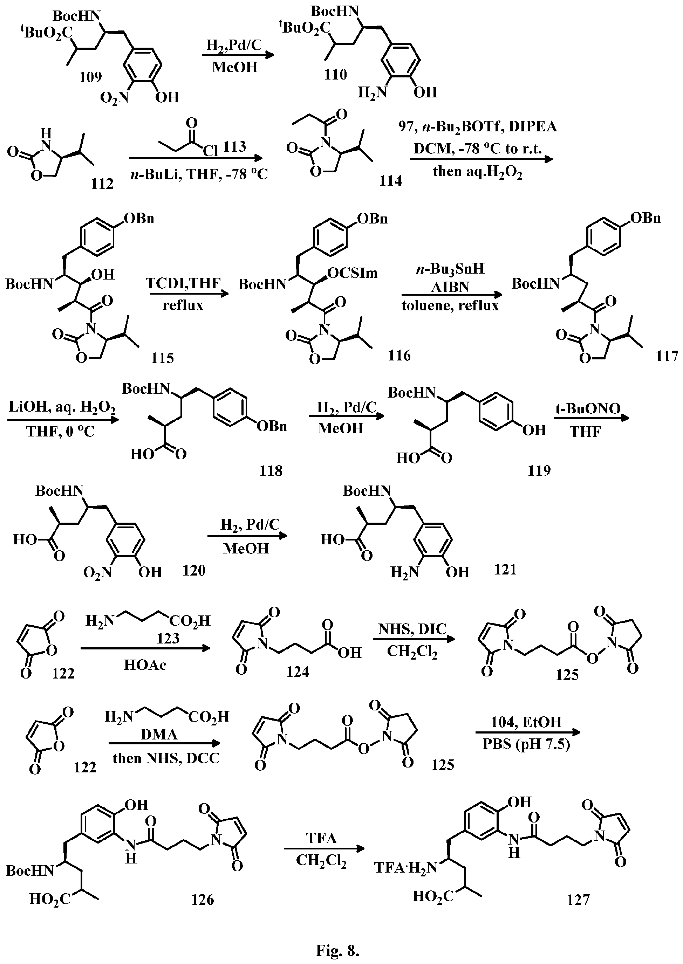

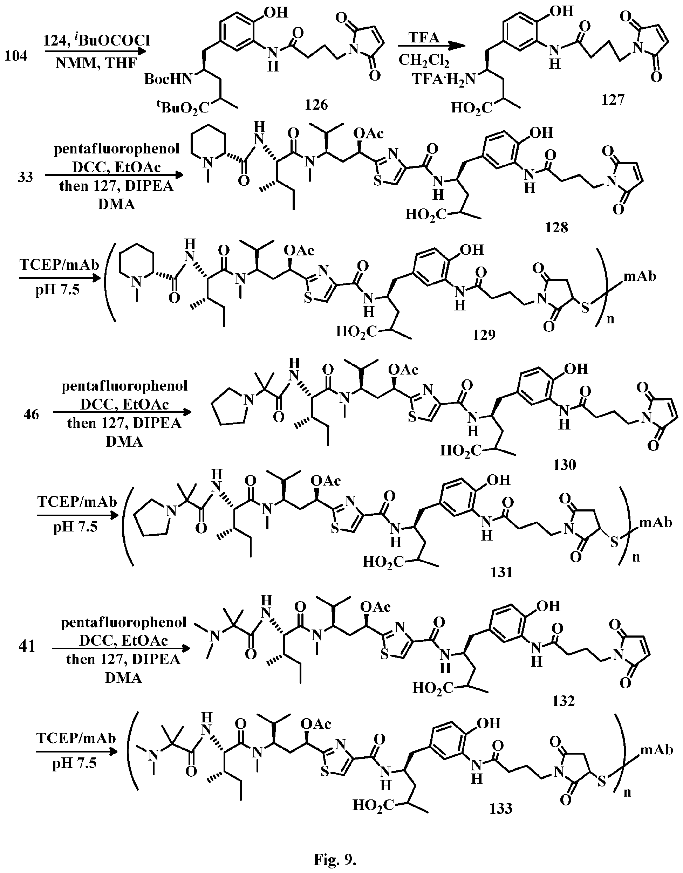

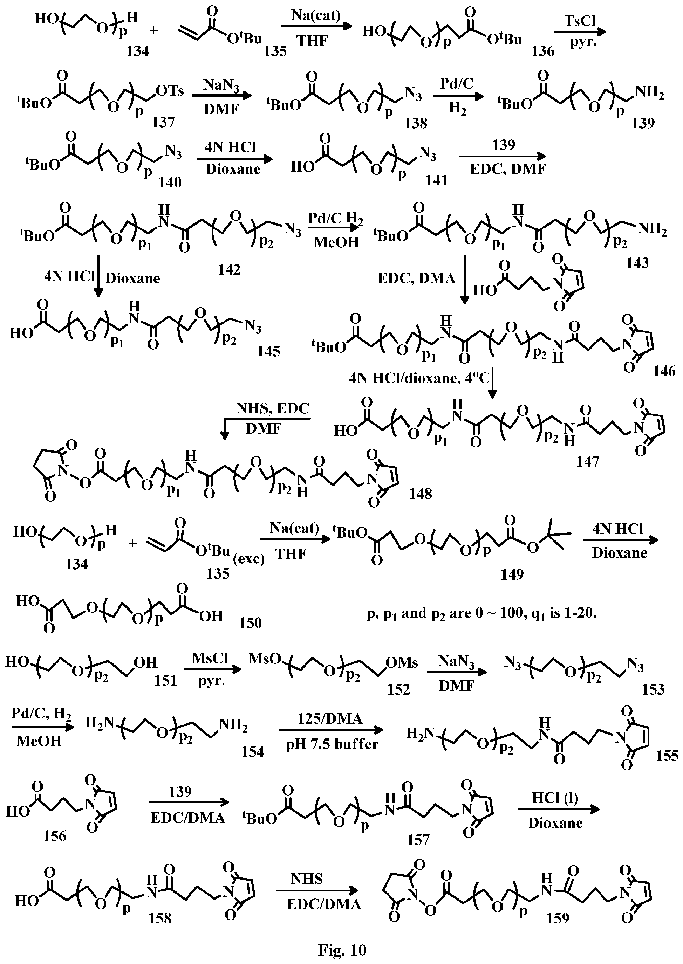

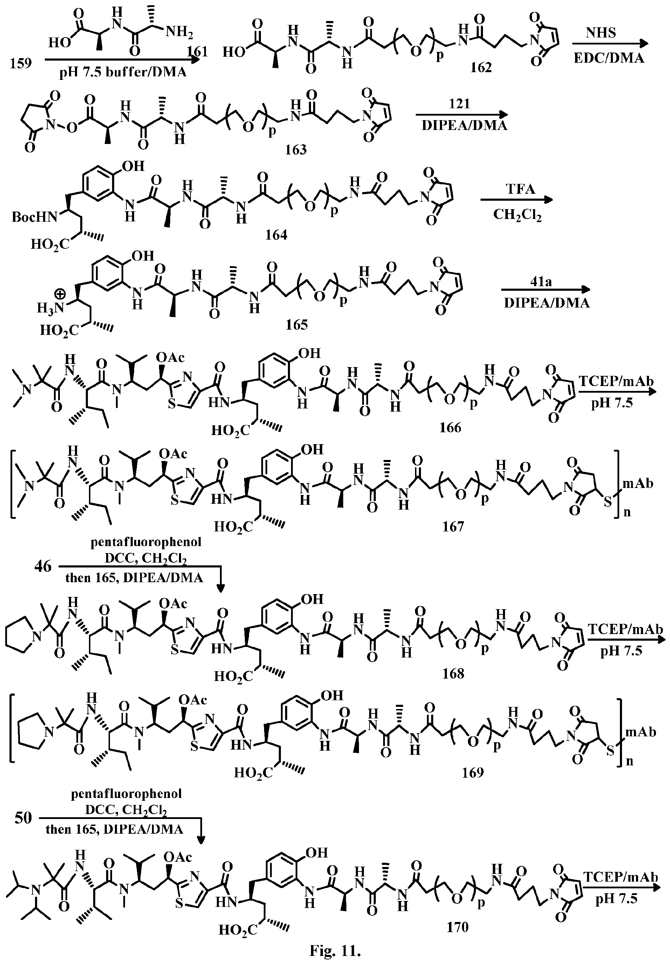

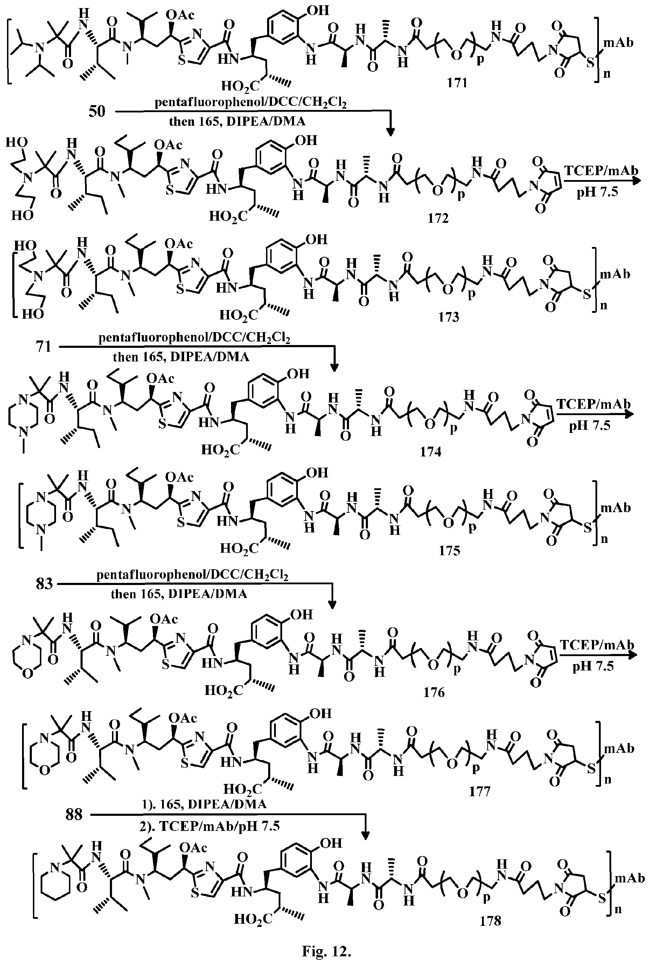

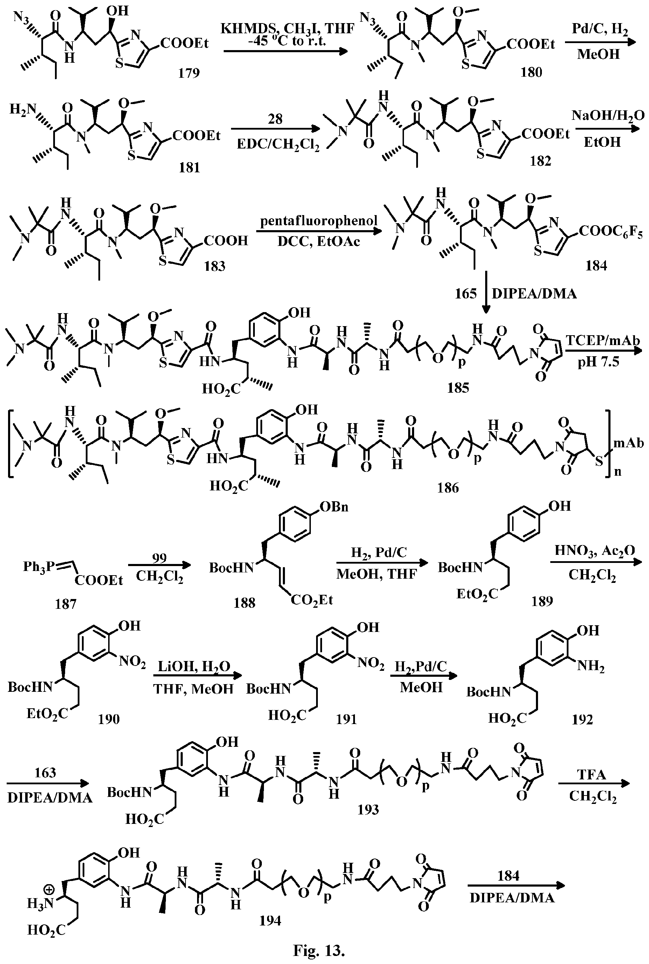

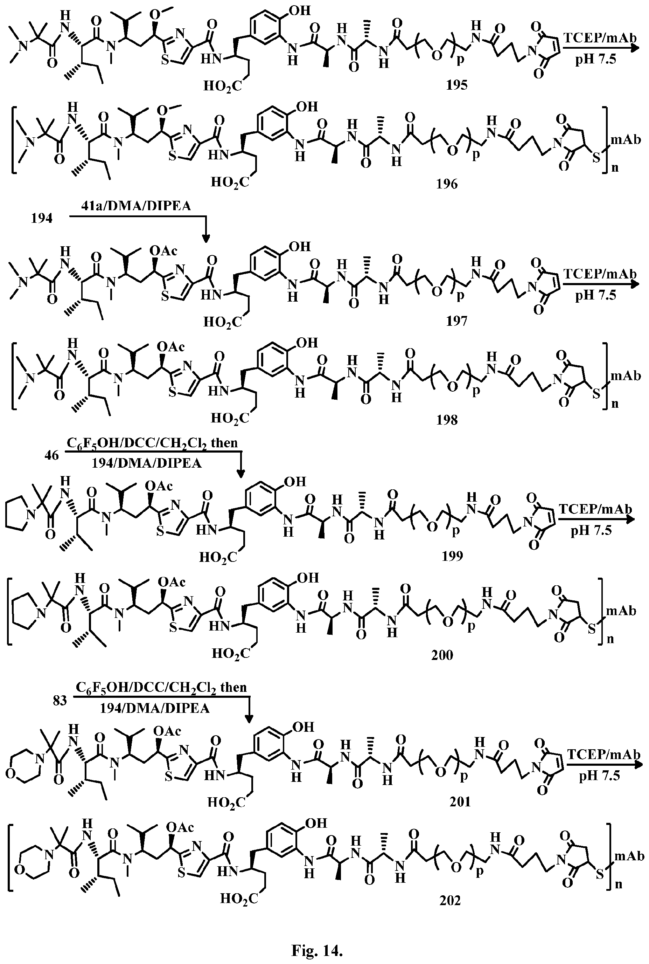

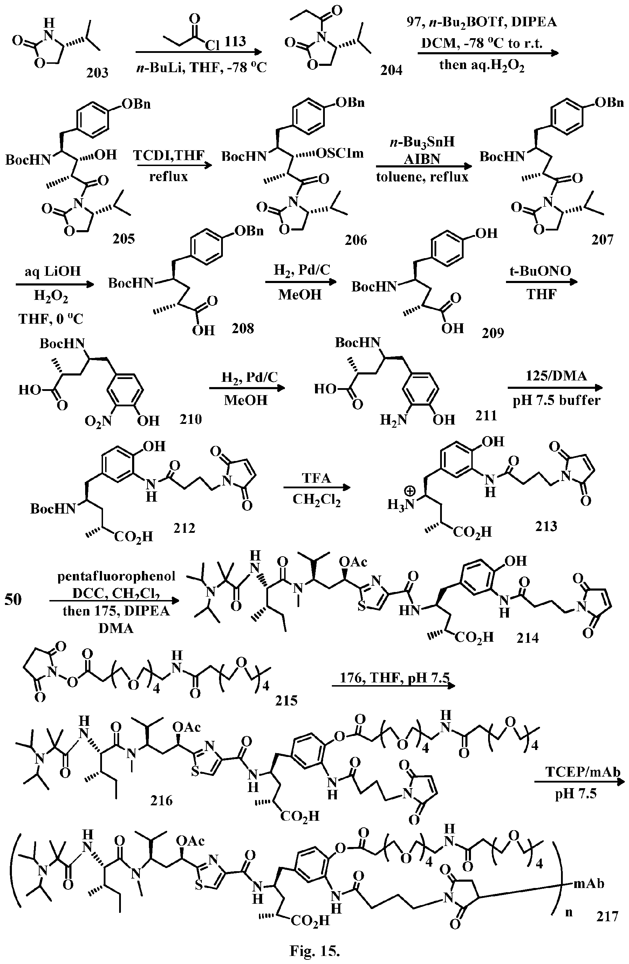

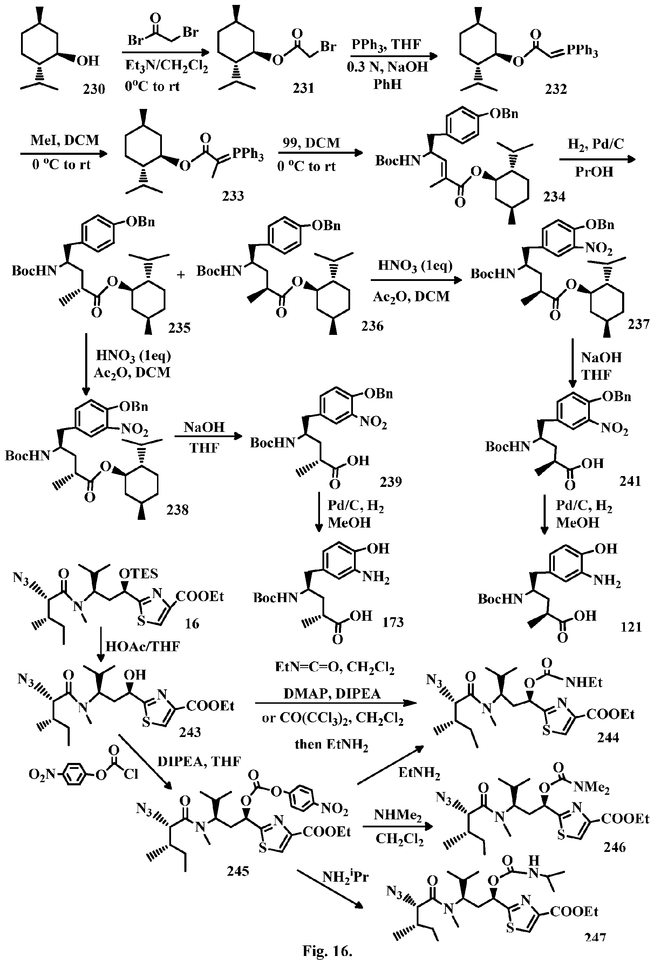

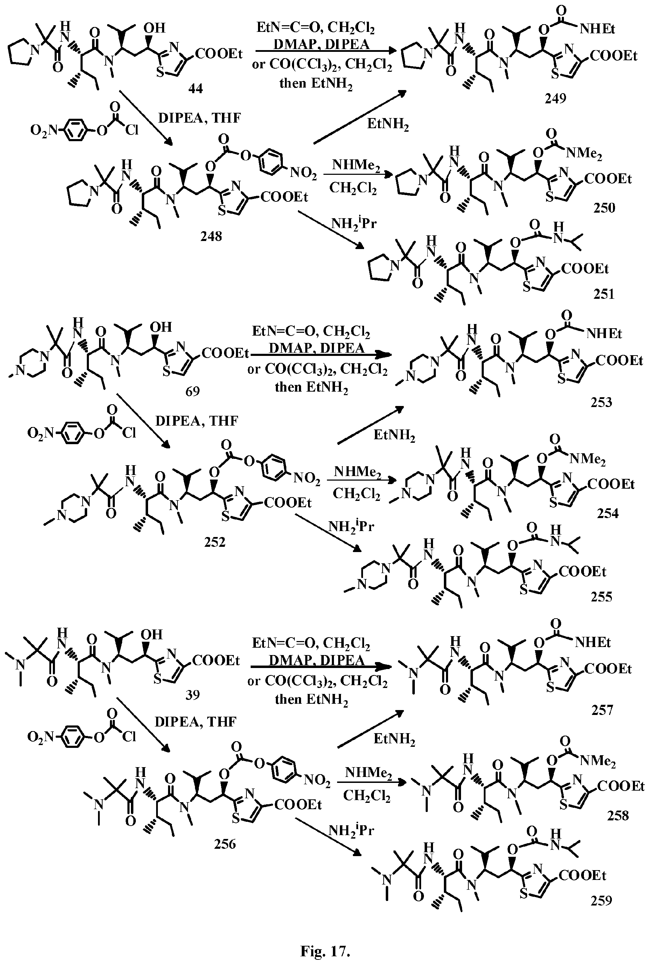

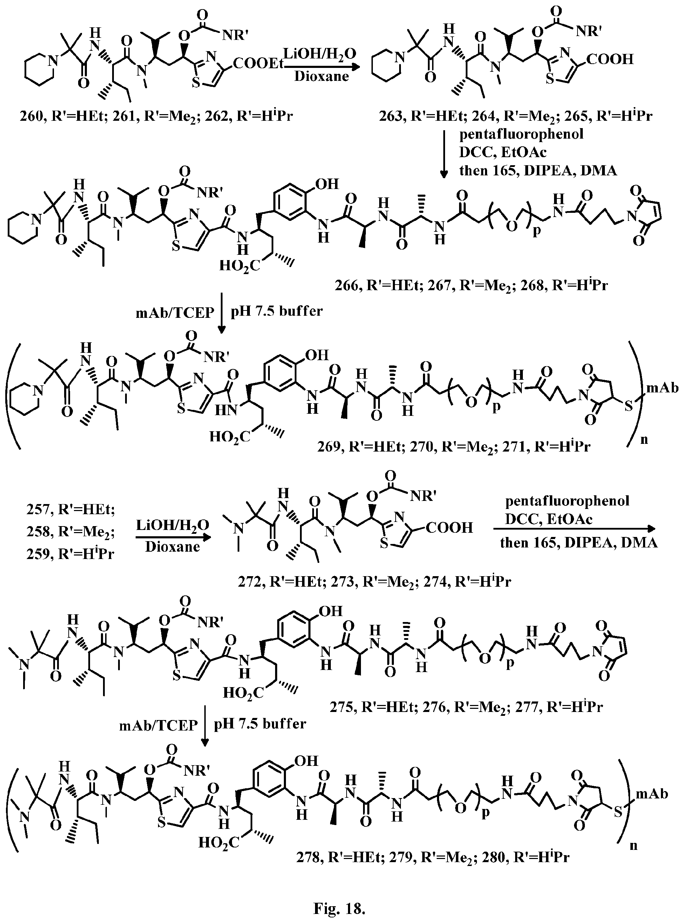

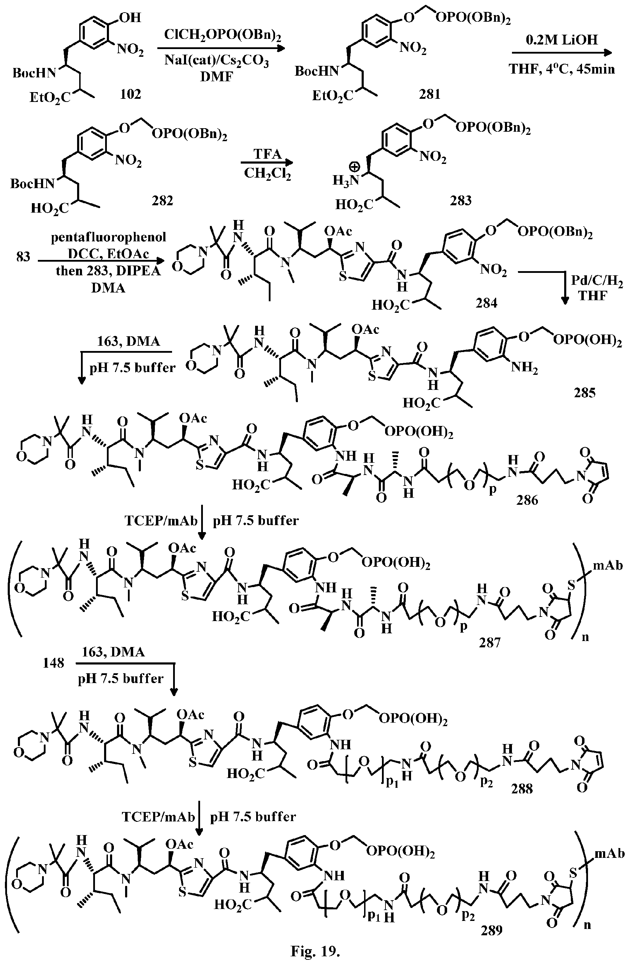

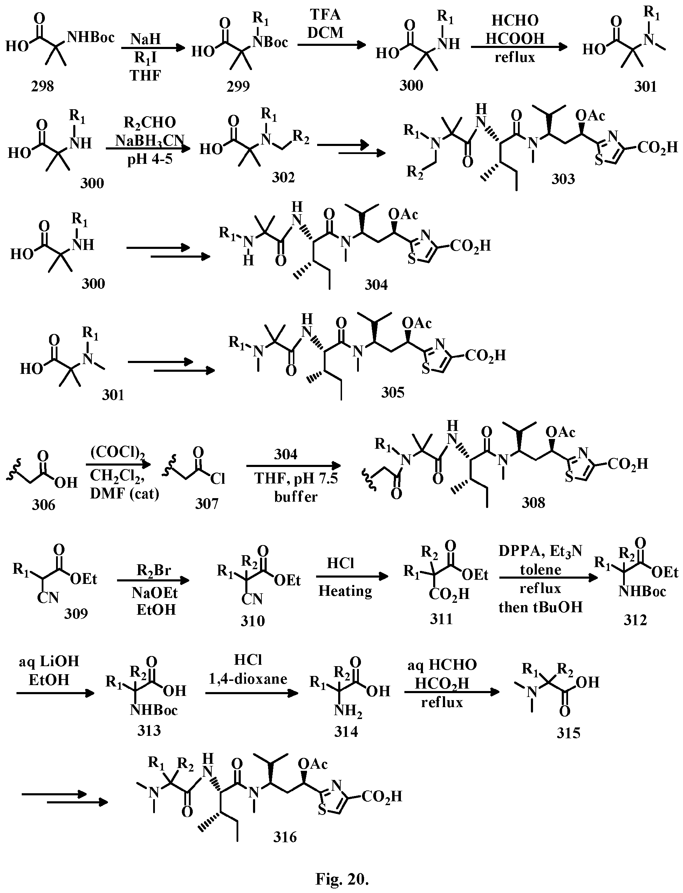

In another embodiment, the synthetic routes to produce the antimitotic agents and their conjugation to a cell-surface receptor binding molecules of the present invention are exampled, but not limited to, as shown in Figs. 1-22.

In another embodiment, the releasable linker (L) is a chain of atoms selected from C, N, O, S, Si, and P that covalently connects the cell-surface binding ligand (T) to the potent antimitotic agents. The linker may have a wide variety of lengths, such as in the range from about 2 to about 100 atoms. The atoms used in forming the linker may be combined in all chemically relevant ways, such as forming alkylene, alkenylene, and alkynylene, ethers, polyoxyalkylene, esters, amines, imines, polyamines, hydrazines, hydrazones, amides, ureas, semicarbazides, carbazides, alkoxyamines, alkoxylamines, urethanes, amino acids, acyloxylamines, hydroxamic acids, and many others. In addition, it is to be understood that the atoms forming the releasable linker (L) may be either saturated or unsaturated, or may be radicals, or may be cyclized upon each other to form divalent cyclic structures, including cyclo alkanes, cyclic ethers, cyclic amines, arylenes, heteroarylenes, and the like in the linker.

The term releasable linker refers to a linker that includes at least one bond that can be broken under physiological conditions, such as a pH-labile, acid-labile, base-labile, oxidatively labile, metabolically labile, biochemically labile, or enzyme-labile bond. It is appreciated that such physiological conditions resulting in bond breaking do not necessarily include a biological or metabolic process, and instead may include a standard chemical reaction, such as a hydrolysis or substitution reaction, for example, an endosome having a lower pH than cytosolic pH, and/or disulfide bond exchange reaction with a intracellular thiol, such as the amillimolar range of abundant of glutathione inside the malignant cells.

The releasable linker L of conjugates may have the formula: --Ww-(Aa)r--Vv-- wherein:--W-- is a Stretcher unit; w is 0 or 1; each --Aa-- is independently an Amino Acid unit; r is independently an integer ranging from 0 to 12; --V-- is a Spacer unit; and v is 0, 1 or 2.

The Stretcher unit (--W--), when present, links a targeted binding molecular unit (T) to an amino acid unit (--Aa--), or links V when an Aa is not present. The Stretcher unit W may independently contain a self-immolative spacer, peptidyl units, a hydrazone bond, disulfide or thiolether bonds. In this regard a binding molecular (T) has a functional group that can form a bond with a functional group of a Stretcher. Useful functional groups that can be present on a binding molecular, either naturally or via chemical manipulation include, but are not limited to, sulfhydryl (--SH), amino, hydroxyl, carbonyl, the anomeric hydroxyl group of a carbohydrate, and carboxyl. Preferred functional groups are sulfhydryl, carboxy and amino. Sulfhydryl groups can be generated by reduction of an intramolecular disulfide bond of a Ligand. Alternatively, sulfhydryl groups can be generated by reaction of an amino group of a lysine moiety of a binding molecular using 2-iminothiolane (Traut's reagent) or thiolactone or another sulfhydryl generating reagent, such as modifies T with a disulfide bond linker, or a thiol ester following by reduction or hydrolysis respectively.

Illustrative examples of W linked to T have the structures: 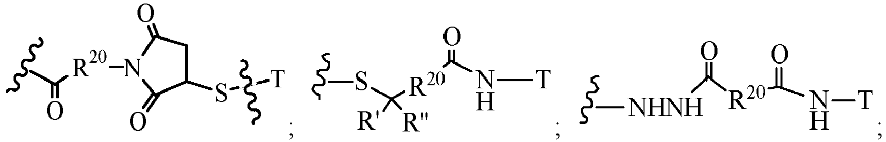 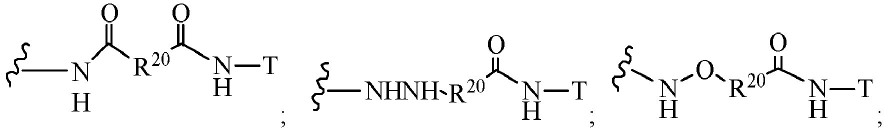 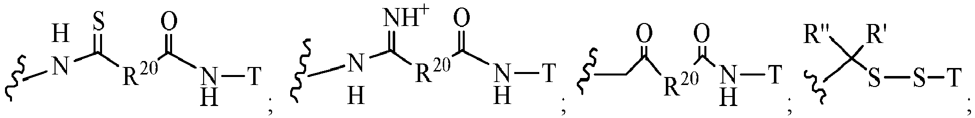 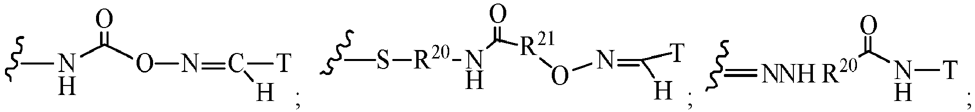 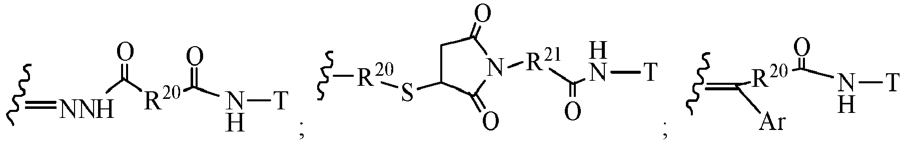 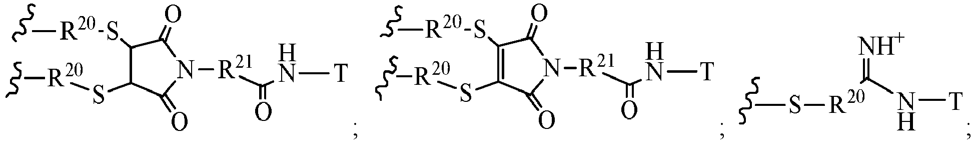 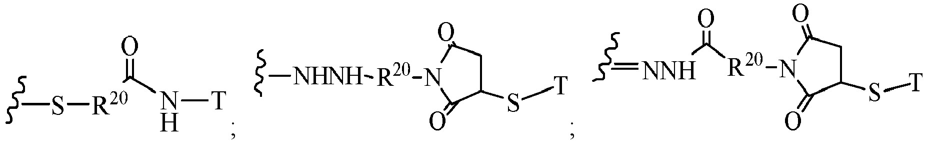 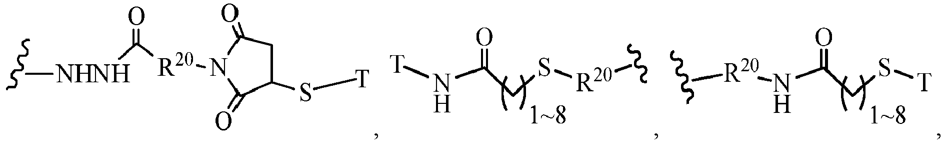 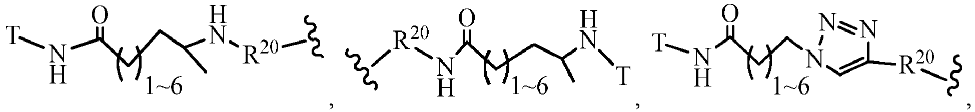 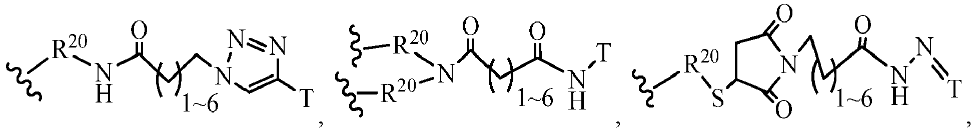 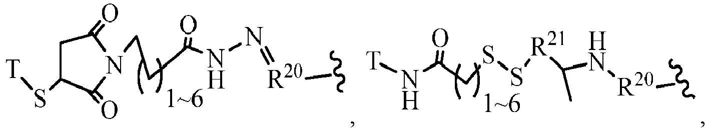 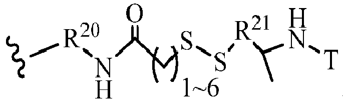 wherein R²⁰ and R²¹ are independently selected from -C₁∼C₉ alkylene-, -C₁∼C₇ carbocyclo-, -O-(C₁∼C₈ alkyl)-, -arylene-, -C₁∼C₉ alkylene-arylene-, -arylene, -C₁∼C₉ alkylene-, -C₁∼C₉ alkylene-(C₁∼C₈ carbocyclo)-, -(C₃∼C₇ carbocyclo)-C₁∼C₉ alkylene-,-C₃∼C₈ heterocyclo-, -C₁∼C₁₀ alkylene-(C₃∼C₈ heterocyclo)-, -(C₃∼C₈ heterocyclo)- C₁∼C₉ alkylene-, -(CH₂CH₂O)ₖ-, -(CH(CH₃)CH₂O)ₖ-, and -(CH₂CH₂O)ₖ-CH₂-; k is an integer ranging from 1-20.; R' and R" are independently H or CH₃.

In another embodiment, conjugation of W to T covalently as illustrated above can be via various chemical reactions.

Examples of the formation of amide linkages:

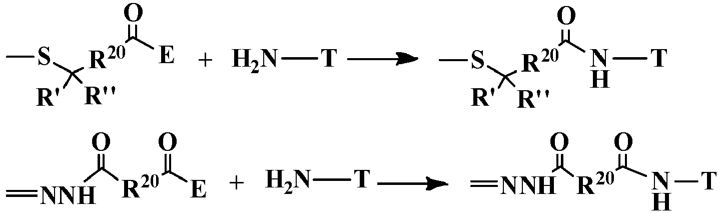

Wherein the Stretcher unit contains a reactive site of E, which can form an amide bond with a primary or secondary amino group of a Ligand. Example of the reactive E, includes, but is not limited to, such as hydroxysuccinimidyl esters (NHS, Sulfo-NHS, etc), 4-nitrophenyl esters, pentafluorophenyl esters, tetrafluorophenyl (includes sulfo-tetrafluorophenyl) esters, anhydrides, acid chlorides, sulfonyl chlorides, isocyanates and isothiocyanates.

Examples of thiol ether or disulfide bond linkages:

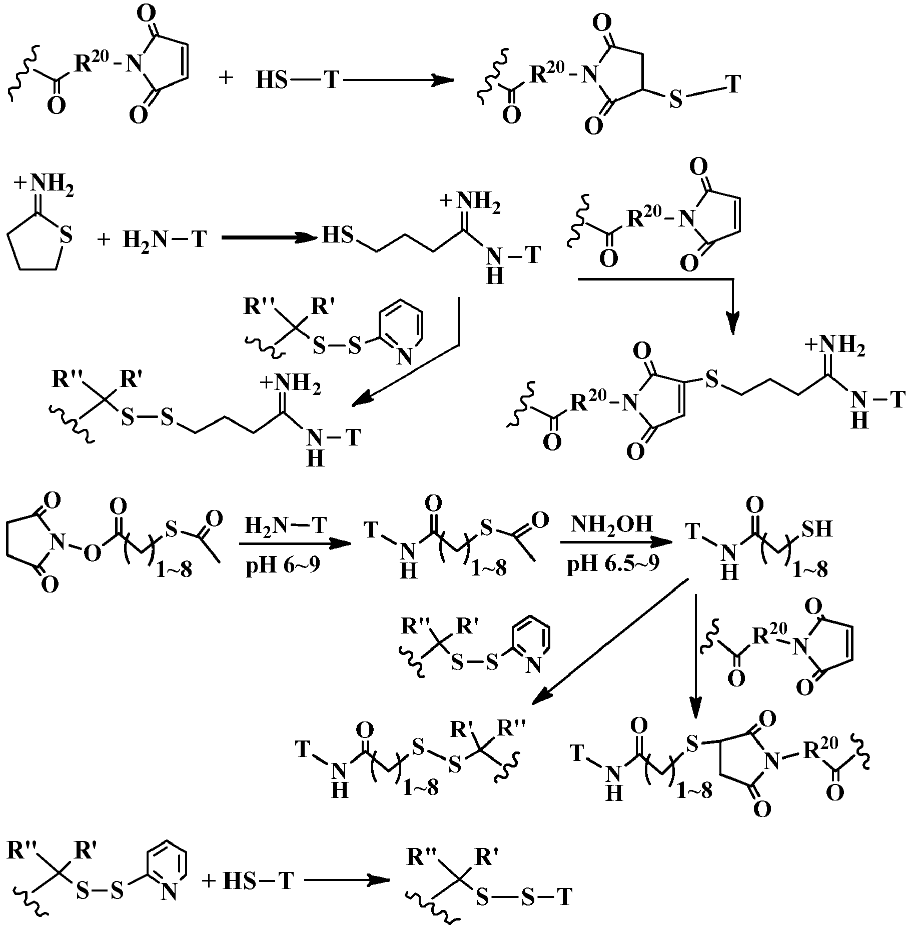

Wherein the Stretcher unit contains a sulfhydryl reactive site, which can form a thiol ether or disulfide bond with a thiol group which is generated by reduction of an intramolecular disulfide bond of the binding ligand T, or generated by a chemical modification on the binding ligand T.

In yet another aspect of the invention, the reactive group of the Stretcher contains a reactive site that is reactive to an aldehyde (--CHO) or a ketone (-C(=O)R) group that can be chemically modified on a binding molecular T. For example, a carbohydrate on a binding molecular T can be mildly oxidized using a reagent such as sodium periodate to generate an aldehyde or a ketone (-C(=O)R) group; or an amine on an amino acid at the N-termini of antibodies (or proteins or peptides) can react with pyridoxal 5'-phosphate (PLP) in a buffer solution to introduce ketone groups (Scheck & Francis, ACS Chem. Biol. 2007, 2, 247-251). The resulting (--C=O) unit can be condensed with a Stretcher that contains a functionality such as a hydrazide, an oxime, a primary or secondary amine, a hydrazine, a thiosemicarbazone, a hydrazine carboxylate, and an arylhydrazide.

Examples of the conjugation of the hydrazone, or the oxime or imine linkages: 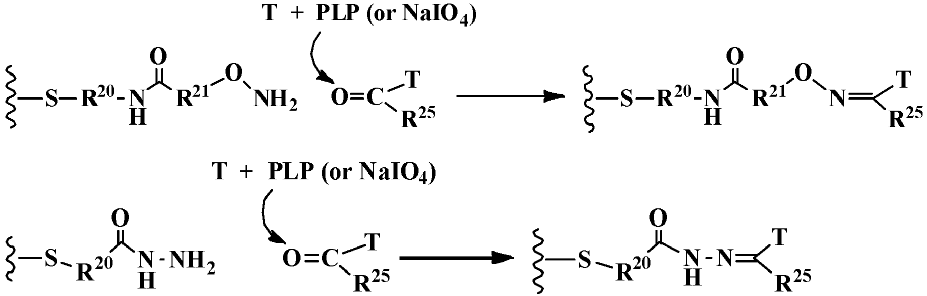 wherein R²⁰ and R²¹ are described above, R²⁵ is an organic substituent of an amino acid.

In another aspect of the invention, the Stretchers (which may contain a spacer V and/or an amino acid) can be linked to the binding molecules (T), followed by conjugation of a potent antimitotic agent to the binding molecule-stretcher moiety in an aqueous buffered solution. Examples of these kinds of two-step conjugations (a drug linked to R¹⁶ is omitted here): 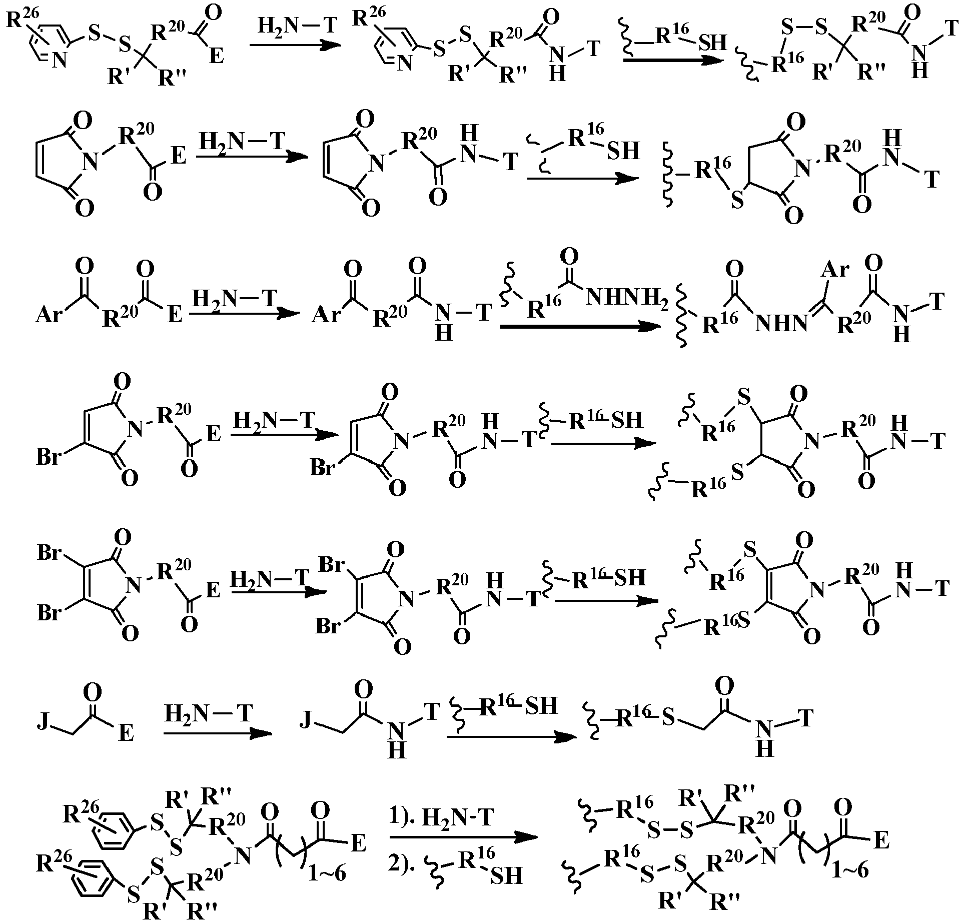 wherein E includes, but is not limited to, such as hydroxysuccinimidyl esters (NHS, Sulfo-NHS, etc), 4-nitrophenyl esters, pentafluorophenyl esters, tetrafluorophenyl (includes sulfo-tetrafluorophenyl) esters, anhydrides, acid chlorides, sulfonyl chlorides, isocyanates and isothiocyanates. R' and R" are independently H or CH₃; R²⁰, R¹⁶ and Ar are defined in various embodiment throughout this inventions; R²⁶ is H, or F, or NO₂ independently; J is F, Cl, Br, I, tosylate (TsO) or mesylate (MsO) independently and wherein 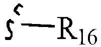 bears at least one antimitotic agent/drug

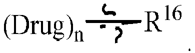

In another aspect of the invention, the Stretchers can be linked to a potent antimitotic agent first, followed by conjugation of the binding molecules (T) in an aqueous pH 3 ∼ 10 (preferably pH 5 ∼ 8.5) buffered solution containing up to 50% of organic cosolvents. Examples of these kinds of two-step conjugations: 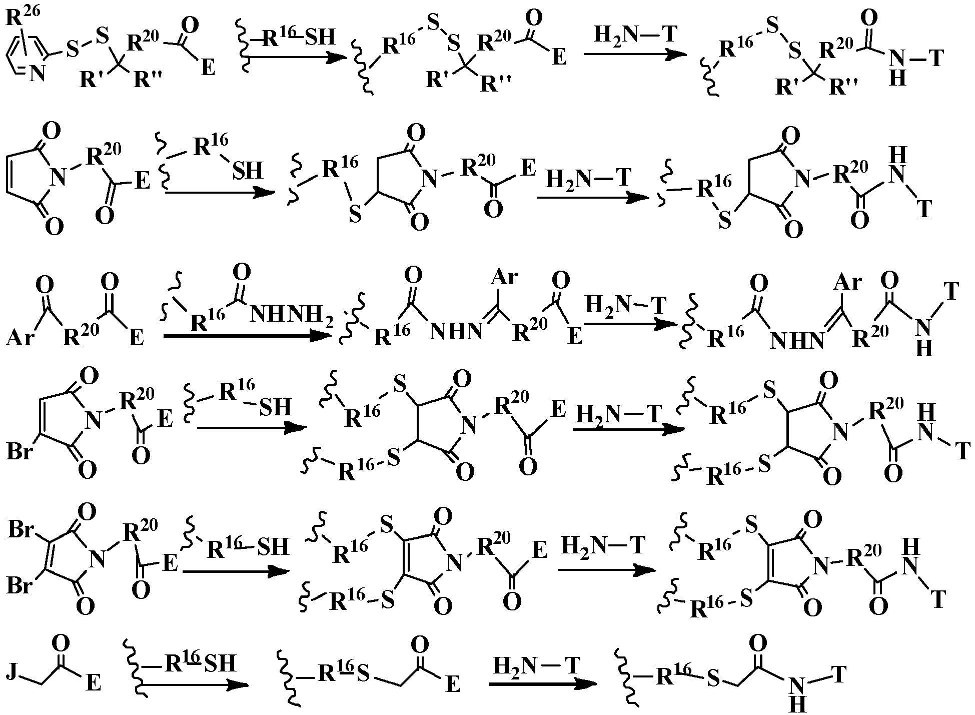 wherein E includes, but is not limited to, such as hydroxysuccinimidyl esters (NHS, Sulfo-NHS, etc), 4-nitrophenyl esters, pentafluorophenyl esters, tetrafluorophenyl (includes sulfo-tetrafluorophenyl) esters, anhydrides, acid chlorides, sulfonyl chlorides, isocyanates and isothiocyanates. R' and R" are independently H or CH₃; R¹⁶, R²⁰ and Ar are defined in various embodiment throughout this inventions; R²⁶ is H, or F, or NO₂ independently; J is F, Cl, Br, I, tosylate (TsO) or mesylate (MsO) independently and wherein 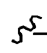 -R₁₆ bears at least one antimitotic agent/drug.

The Amino Acid unit (--Aa--), when present, links the Stretcher unit to the Spacer unit if the Spacer unit is present, links the Stretcher unit to the antimitotic agent unit if the Spacer unit is absent, and links the binding molecule (T) unit to the antimitotic agent unit if the Stretcher unit and Spacer unit are absent. --(Aa)r-- is a natural or unnatural amino acid, dipeptide, tripeptide, tetrapeptide, pentapeptide, hexapeptide, heptapeptide, octapeptide, nonapeptide, decapeptide, undecapeptide or dodecapeptide unit, and r is an integer ranging from 0 to 12. The term amino acid as used herein refers generally to aminoalkylcarboxylate, where the alkyl radical is optionally substituted, such as with alkyl, acyl, hydroxy alkyl, sulfhydrylalkyl, aminoalkyl, carboxyalkyl, and the like, The structures of the natural and unnatural amino acids and peptides are described in the book: G. C. Barrett and D. T. Elmore, "Amino Acid and Peptide", Cambridge University Press, 2004. In addition, amino acid refers to beta, gamma, and longer amino acids with intra chain containing methyl, benzyl, hydroxymethyl, thiomethyl, carboxyl, carboxylmethyl, guanidinopropyl, and the like. More preferably the amino acid is selected from asparagine, aspartic acid, cysteine, glycine, glutamic acid, lysine, glutamine, arginine, serine, ornithine, threonine, and the like.

The Amino Acid unit of the Invention can be enzymatically cleaved by one or more enzymes, including a tumor-associated protease, to liberate the antimitotic agent, which in one embodiment is protonated in vivo upon release to provide an antimitotic agent.

The Spacer unit (--V--), when present, links an Amino Acid unit to the antimitotic agent when an Amino Acid unit is present. Alternately, the Spacer unit links the Stretcher unit to antimitotic agent when the Amino Acid unit is absent. The Spacer unit also links antimitotic agent to the binding molecule (T) when both the Amino Acid unit and Stretcher unit are absent. The spacer linkers may contain function groups that substantially increase the water solubility, biological transport, preferential renal clearance, uptake, absorption, biodistribution, and/or bioavailability of the conjugate are described herein. Spacer units are of two general types: self-immolative and non-self-immolative. A non-self-immolative Spacer unit is one in which part or all of the Spacer unit remains bound to antimitotic agent after cleavage, particularly enzymatic, of an Amino Acid unit from the antimitotic agent-Linker- binding molecule conjugate or the antimitotic agent-Linker Compound. The self-immolative unit includes aromatic compounds that are electronically similar to the para-aminobenzyl-carbamoyl (PAB) groups, 2-aminoimidazol-5-methanol derivatives, heterocyclic PAB analogs, beta-glucuronide, and ortho or para-aminobenzylacetals; or one of the following structures: 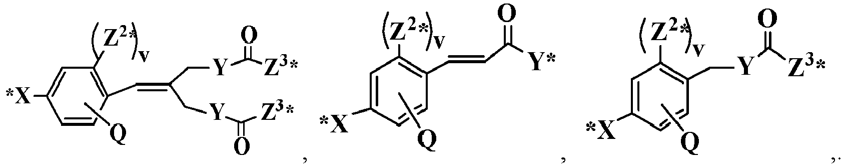 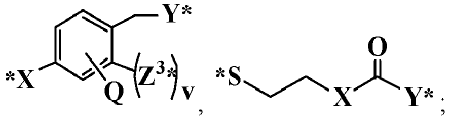 wherein the (*) atom is the point of attachment of additional spacer or releasable linker units, the antimitotic agent, and/or the binding molecule (T); X, Y and Z³ are independently NH, O, or S; Z² is H, NH, O or S independently. v is 0 or 1; Q is independently H, OH, C₁∼C₆ alkyl, (OCH₂CH₂)ₙ F, Cl, Br, I, OR¹⁷, or SR¹⁷, NR¹⁷R¹⁸, N=NR¹⁷, N=R¹⁷, NR¹⁷R¹⁸, NO₂, SOR¹⁷R¹⁸, SO₂R¹⁷, SO₃R¹⁷, OSO₃R¹⁷, PR¹⁷R¹⁸, POR¹⁷R¹⁸, PO₂R¹⁷R¹⁸, OPO(OR¹⁷)(OR¹⁸), or OCH₂PO(OR¹⁷(OR¹⁸) wherein R¹⁷, R¹⁸ are independently H, C₁∼C₈ of alkyl; C₂∼C₈ of alkenyl, alkynyl, heteroalkyl; C₃∼C₈ of aryl, heterocyclic, carbocyclic, cycloalkyl, heterocycloalkyl, heteroaralkyl, alkylcarbonyl; or pharmaceutical cation salts

Examples of the non-self-immolative spacer linkers: 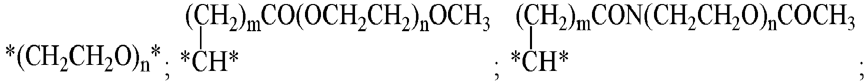 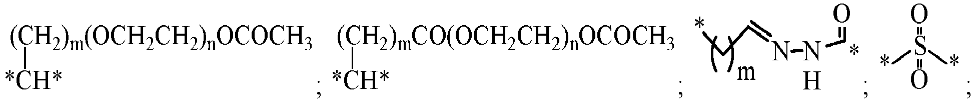 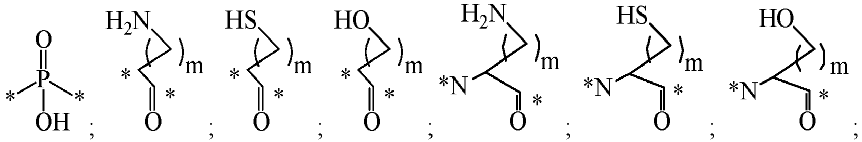 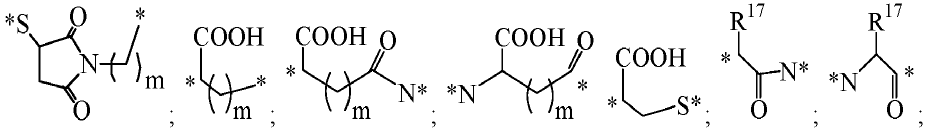 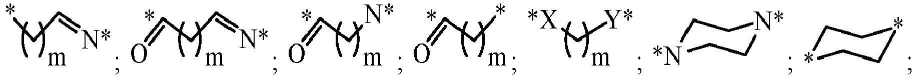 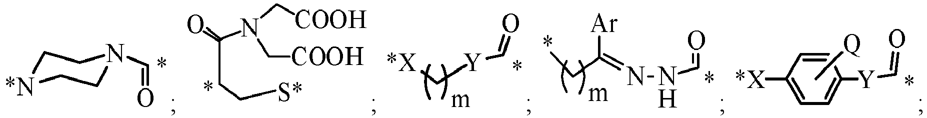 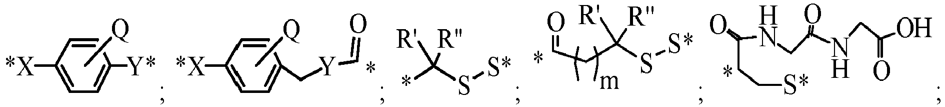 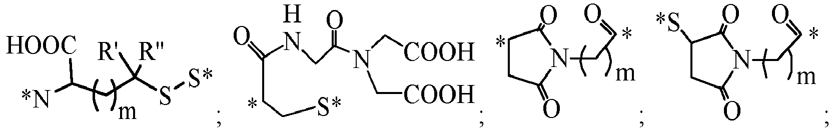 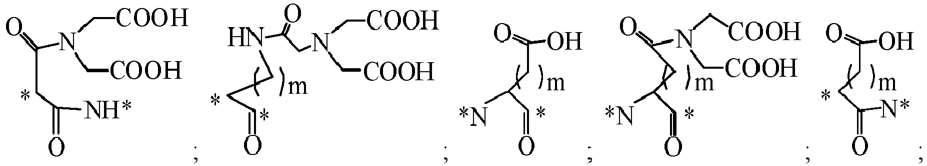 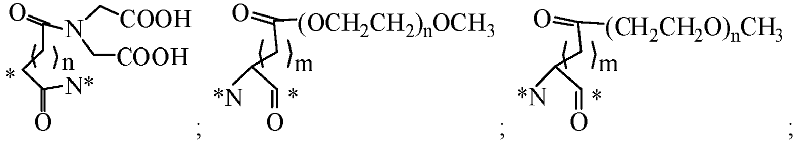 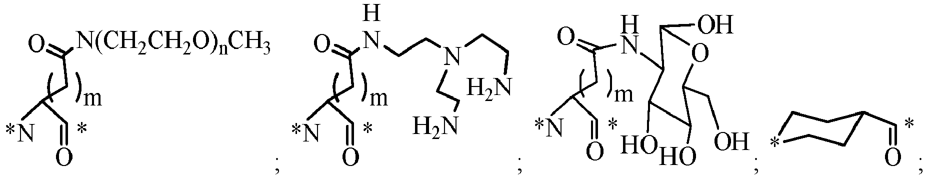 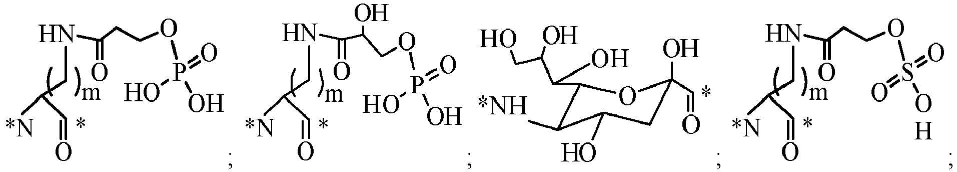 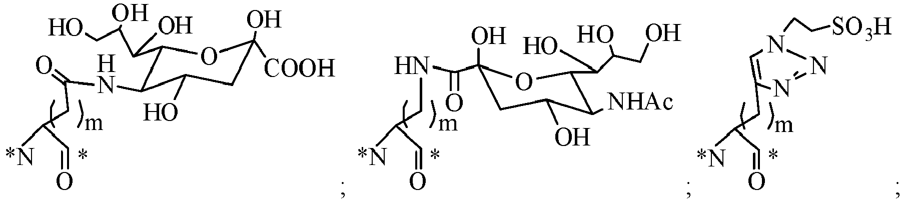 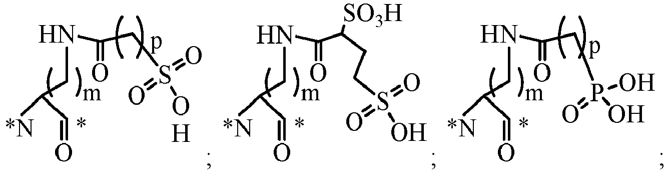 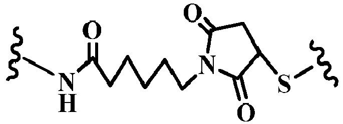 6-maleimidocaproyl (MC), 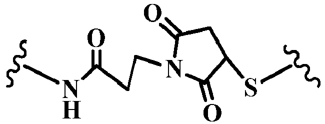 maleimido propanoyl (MP), 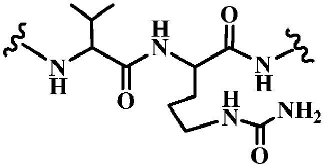 valine-citrulline (val-cit), 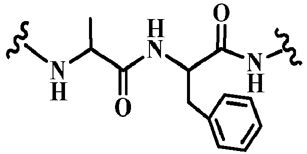 alanine-phenylalanine (ala-phe), 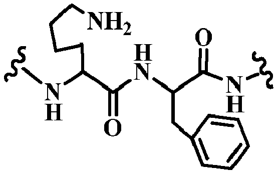 lysine-phenylalanine (lys-phe), 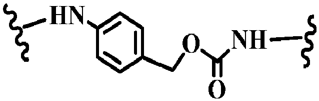 p-aminobenzyloxycarbonyl (PAB), 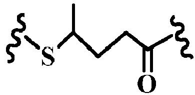 4-thio-pentanoate (SPP), 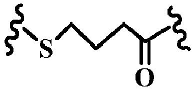 4-thio-butyrate (SPDB), 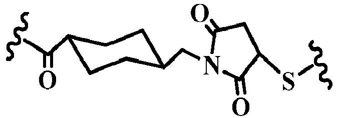 4-(N-maleimidomethyl)cyclo-hexane-1-carboxylate (MCC), 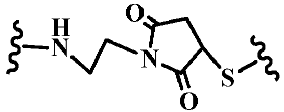 maleimidoethyl (ME), 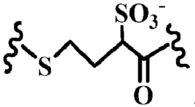 4-thio-2-hydroxysulfonyl-butyrate (2-Sulfo-SPDB), 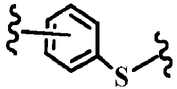 aryl-thiol (PySS), 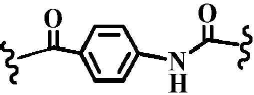 (4-acetyl)aminobenzoate (SIAB), 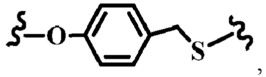 oxylbenzylthio, 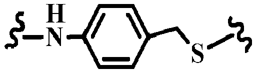 aminobenzylthio, 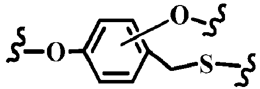 dioxylbenzylthio, 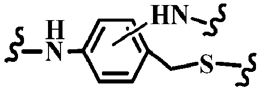 diaminobenzylthio, 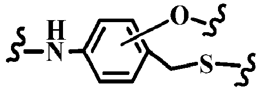 amino-oxylbenzylthio, 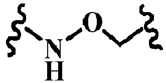 alkoxy amino (AOA), 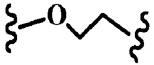 ethyleneoxy (EO), 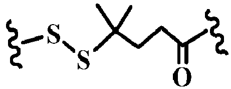 4-methyl-4-dithio-pentanoic (MPDP), 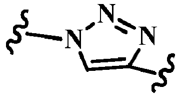 triazole, 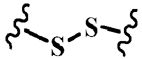 dithio, 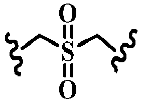 alkylsulfonyl, 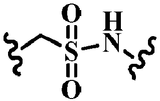 alkylsulfonamide, 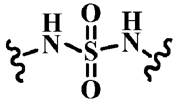 sulfon-bisamide, 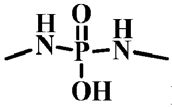 Phosphondiamide, 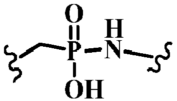 alkylphosphonamide, 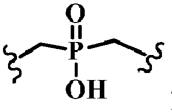 phosphinic acid, 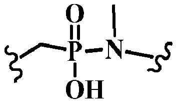 N-methylphosphonamidic acid, 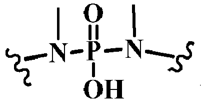 N,N' -dimethylphosphon-amidic acid, 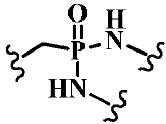 N,N' -dimethylphosphondiamide, 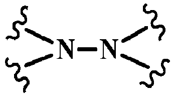 hydrazine, 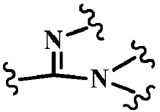 acetimidamide; 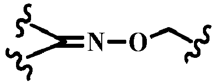 oxime, 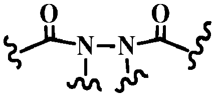 acetylacetohydrazide, 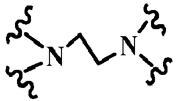 aminoethyl-amine, 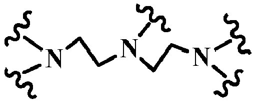 aminoethyl-aminoethyl-amine, 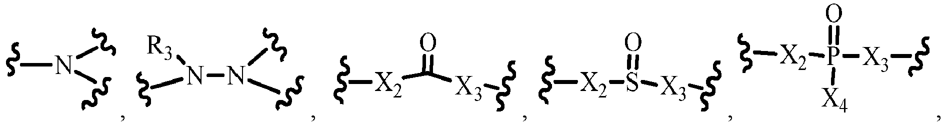 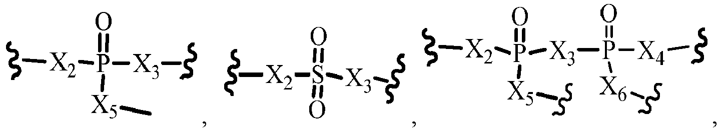 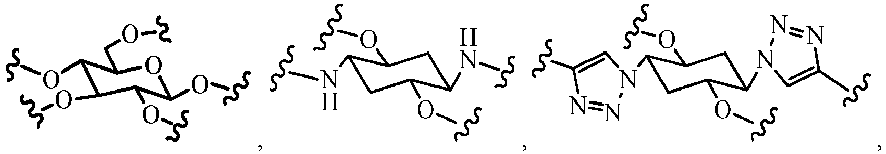 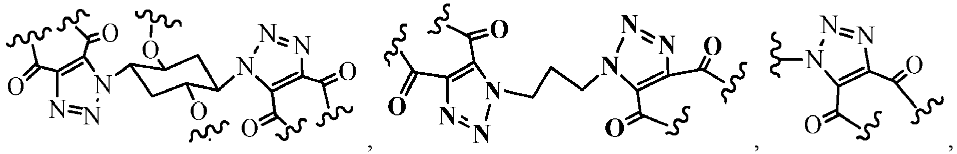 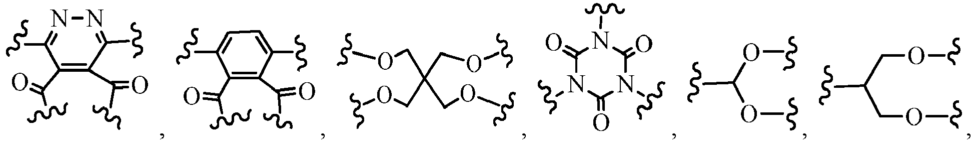 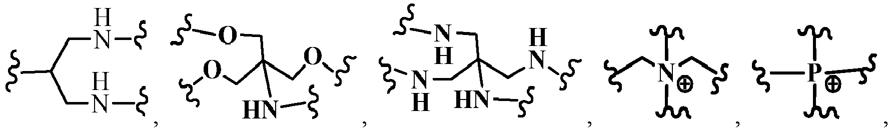 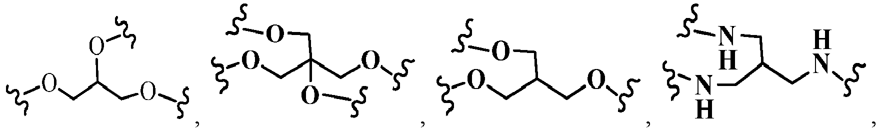 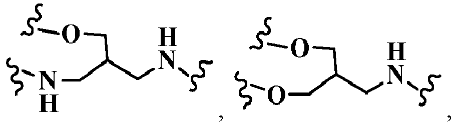 or L- or D-, natural or unnatural peptides containing 1-20 the same or different amino acids;

Wherein the "*" and " 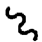 " atom are the point of attachment of additional spacer or releasable linkers, the antimitotic agents, and/or the binding molecules; m is 1∼10; n is 1∼20; X₂, X₃, X₄, X₅, or X₆, are independently selected from NH; NHNH; N(R₁₂); N(R₁₂)N(R_{12'}); O; S; C₁-C₆ of alkyl; C₂-C₆ of heteroalkyl, alkylcycloalkyl, heterocycloalkyl; C₃∼C₈ of aryl, Ar-alkyl, heterocyclic, carbocyclic, cycloalkyl, heteroalkylcycloalkyl, alkylcarbonyl, heteroaryl; CH₂OR₁₂, CH₂SR₁₂, CH₂NHR₁₂, or 1∼8 amino acids; wherein R₁₂ and R_{12'} are independently H; C₁-C₈ of alkyl; C₂-C₈ of hetero-alkyl, alkylcycloalkyl, heterocycloalkyl; C₃-C₈ of aryl, Ar-alkyl, heterocyclic, carbocyclic, cycloalkyl, heteroalkylcycloalkyl, alkylcarbonyl, heteroaryl; or 1-8 carbon atoms of esters, ether, or amide; or polyethyleneoxy unit of formula (OCH₂CH₂)ₚ or (OCH₂CH(CH₃))ₚ, wherein p is an integer from 0 to about 1000, or combination above thereof.

A releasable component of the linker L that at least one bond in L can be broken under physiological conditions: a pH-labile, acid-labile, base-labile, oxidatively labile, metabolically labile, biochemically labile or enzyme-labile bond, which having one of the following structures:
-(CR₁₅R₁₆)ₘ(Aa)r(CR₁₇R₁₈)ₙ(OCH₂CH₂)ₜ-, -(CR₁₅R₁₆)ₘ(CR₁₇R₁₈)ₙ(Aa)r(OCH₂CH₂)ₜ-, -(Aa)ᵣ-(CR₁₅R₁₆)ₘ(CR₁₇R₁₈)ₙ(OCH₂CH₂)ₜ-, -(CR₁₅R₁₆)ₘ(CR₁₇R₁₈)ₙ(OCH₂CH₂)ᵣ(Aa)ₜ-,-(CR₁₅R₁₆)ₘ(CR₁₇=CR₁₈)(CR₁₉R₂₀)ₙ(Aa)ₜ(OCH₂CH₂)ᵣ-, -(CR₁₅R₁₆)ₘ(NR₁₁CO)(Aa)ₜ(CR₁₉R₂₀)ₙ-(OCH₂CH₂)ᵣ-, -(CR₁₅R₁₆)ₘ(Aa)ₜ(NR₂₁CO)(CR₁₉R₂₀)ₙ(OCH₂CH₂)ᵣ-, -(CR₁₅R₁₆)ₘ(OCO)(Aa)ₜ-(CR₁₉R₂₀)ₙ(OCH₂CH₂)ᵣ-,-(CR₁₅R₁₆)ₘ(OCNR₁₇)(Aa)ₜ(CR₁₉R₂₀)ₙ(OCH₂CH₂)ᵣ-,-(CR₁₅R₁₆)ₘ-(CO)(Aa)ₜ-(CR₁₉R₂₀)ₙ(OCH₂CH₂)ᵣ-,-(CR₁₅R₁₆)ₘ(NR₂₁CO)(Aa)ₜ(CR₁₉R₂₀)ₙ(OCH₂CH₂)ᵣ-,-(CR₁₅R₁₆)ₘ-(OCO)(Aa)ₜ(CR₁₉R₂₀)ₙ-(OCH₂CH₂)ᵣ-, -(CR₁₅R₁₆)ₘ(OCNR₁₇)(Aa)ₜ(CR₁₉R₂₀)ₙ-(OCH₂CH₂)ᵣ-, -(CR₁₅R₁₆)ₘ(CO)(Aa)ₜ(CR₁₉R₂₀)ₙ-(OCH₂CH₂)ᵣ-, -(CR₁₅R₁₆)ₘ-phenyl-CO(Aa)ₜ-(CR₁₇R₁₈)ₙ-, -(CR₁₅R₁₆)ₘ-furyl-CO(Aa)ₜ(CR₁₇R₁₈)ₙ-, -(CR₁₅R₆)ₘ-oxazolyl-CO(Aa)ₜ(CR₁₇R₁₈)ₙ-,-(CR₁₅R₁₆)ₘ-thiazolyl-CO(Aa)ₜ(CCR₁₇R₁₈)ₙ-, -(CR₁₅R₁₆)ₜ-thienyl-CO(CR₁₇R₁₈)ₙ-, -(CR₁₅R₁₆)ₜ-imidazolyl-CO-(CR₁₇R₁₈)ₙ-, -(CR₁₅R₁₆)ₜ-morpholino-CO(Aa)ₜ-(CR₁₇R₁₈)ₙ-, -(CR₁₅R₁₆)ₜ-piperazino-CO(Aa)ₜ(CR₁₇R₁₈)ₙ-, -(CR₁₅R₁₆)ₜ-N-methylpiperazin-CO(Aa)ₜ(CR₁₇R₁₈)ₙ-,-(CR₁₅R₁₆)ₘ-(Aa)tphenyl-, -(CR₁₅R₁₆)ₘ-(Aa)ₜfuryl-, -(CR₁₅R₁₆)ₘ-oxazolyl(Aa)ₜ-, -(CR₁₅R₁₆)ₘ-thiazolyl(Aa)ₜ-, -(CR₁₅R₁₆)ₘ-thienyl-(Aa)ₜ-, -(CR₁₅R₁₆)ₘ-imidazolyl(Aa)ₜ-, -(CR₁₅R₁₆)ₘ-morpholino-(Aa)ₜ-, -(CR₁₅R₁₆)ₘ-piperazino-(Aa)ₜ-, -(CR₁₅R₁₆)ₘ-N-methylpiperazino-(Aa)ₜ₋ ,-K(CR₁₅R₁₆)ₘ(Aa)r(CR₁₇R₁₈)ₙ(OCH₂CH₂)ₜ-, -K(CR₁₅R₁₆)ₘ(CR₁₇R₁₈)ₙ(Aa)ᵣ(OCH₂CH₂)ₜ-, -K(Aa)ᵣ-(CR₁₅R₁₆)ₘ(CR₁₇R₁₈)ₙ(OCH₂CH₂)ₜ-, -K(CR₁₅R₁₆)ₘ(CR₁₇R₁₈)ₙ(OCH₂CH₂)ᵣ(Aa)ₜ--K(CR₁₅R₁₆)ₘ-(CR₁₇=CR₁₈)(CR₁₉R₂₀)ₙ(Aa)ₜ(OCH₂CH₂)ᵣ, -K(CR₁₅R₁₆)ₘ(NR₁₁CO)(Aa)ₜ-(CR₁₉R₂₀)ₙ(OCH₂CH₂)ᵣ-, -K(CR₅R₆)ₘ(Aa)ₜ(NR₂₁CO)(CR₁₉R₂₀)ₙ(OCH₂CH₂)ᵣ-, -K(CR₁₅R₁₆)ₘ-(OCO)(Aa)ₜ(CR₁₉R₂₀)ₙ-(OCH₂CH₂)ᵣ-,-K(CR₁₅R₁₆)ₘ(OCNR₁₇)(Aa)ₜ(CR₁₉R₂₀)ₙ-(OCH₂CH₂)ᵣ-, -K(CR₁₅R₁₆)ₘ(CO)(Aa)ₜ-(CR₁₉R₂₀)ₙ(OCH₂CH₂)ᵣ-, -K(CR₁₅R₁₆)ₘ(NR₂₁CO)(Aa)ₜ-(CR₁₉R₂₀)ₙ-(OCH₂CH₂)ᵣ-, -K(CR₁₅R₁₆)ₘ-(OCO)(Aa)ₜ(CR₁₉R₂₀)ₙ(OCH₂CH₂)ᵣ-, -K(CR₁₅R₁₆)ₘ-(OCNR₁₇)(Aa)ₜ-(CR₁₉R₂₀)ₙ(OCH₂CH₂)ᵣ-, -K-(CR₁₅R₁₆)ₘ(CO)(Aa)ₜ(CR₁₉R₂₀)ₙ(OCH₂CH₂)ᵣ-, -K(CR₁₅R₁₆)ₘ-phenyl-CO(Aa)ₜ(CR₁₇R₁₈)ₙ-, -K-(CR₁₅R₁₆)ₘ-furyl-CO(Aa)ₜ(CR₁₇R₁₈)ₙ-, -K(CR₁₅R₁₆)ₘ-oxazolyl-CO(Aa)ₜ(CR₁₇R₁₈)ₙ-,-K(CR₁₅R₁₆)ₘ-thiazolyl-CO(Aa)ₜ-(CR₁₇R₁₈)ₙ-,-K(CR₁₅R₁₆)ₜ-thienyl-CO(CR₁₇R₁₈)ₙ-, -K(CR₁₅R₁₆)timidazolyl-CO-(CR₁₇R₁₈)ₙ-, -K(CR₅R₆)ₜmorpholino-CO(Aa)ₜ-(CR₁₇R₁₈)ₙ-, -K(CR₁₅R₁₆)ₜ-piperazino-CO(Aa)ₜ-(CR₁₇R₁₈)ₙ-, -K(CR₁₅R₁₆)ₜ-N-methylpiperazin-CO(Aa)ₜ(CR₁₇R₁₈)ₙ-, -K(CR₁₅R₁₆)ₘ-(Aa)ₜphenyl, -K-(CR₁₅R₁₆)ₘ-(Aa)ₜfuryl-, -K(CR₁₅R₁₆)ₘ-oxazolyl-(Aa)ₜ-, -K(CR₁₅R₁₆)ₘ-thiazolyl(Aa)ₜ-, -K(CR₁₅R₁₆)ₘ-thienyl-(Aa)ₜ-, -K(CR₁₅R₁₆)ₘ-imidazolyl(Aa)ₜ-, -K(CR₁₅R₁₆)ₘ-Morpholino(Aa)ₜ-, -K(CR₁₅R₁₆)ₘpiperazino(Aa)ₜG, -K(CR₅R₆)ₘ-N-methyl-piperazino(Aa)t-; wherein m, Aa, m, n, R₁₃, R¹⁴, and R₁₅ are described above; t and r here are 0 - 100 independently; R₁₆, R₁₇, R₁₈, R₁₉, and R₂₀ are independently chosen from H; halide; C₁∼C₈ of alkyl or heteroalkkyl, C₂∼C₈ of aryl, alkenyl, alkynyl, ether, ester, amine or amide, C₃∼C₈ of aryl, which optionally substituted by one or more halide, CN, NR₁₂R_{12'}, CF₃, OR₁₂, Aryl, heterocycle, S(O)R₁₂, SO₂R₁₂, -CO₂H, -SO₃H, -OR₁₂, -CO₂R₁₂, -CONR₁₂, -PO₂R₁₂R₁₃, -PO₃H or P(O)R₁₂R_{12'}R₁₃; K is NR₁₂, -SS-, -C(=O)-, -C(=O)NH-, -C(=O)O-, -C=NH-O-, -C=N-NH-, -C(=O)NH-NH-, O, S, Se, B, Het (heterocyclic or heteroaromatic ring having C₃-C₁₂); or peptides containing the same or different 1 - 20 amino acids.

The binding molecule (T) may be of any kind presently known, or that become known, molecule that binds to, complexes with or reacts with a moiety of a cell population sought to be therapeutically or otherwise biologically modified. The binding molecule unit acts to deliver the antimitotic agents to the particular target cell population with which the binding molecule (T) reacts.

The cell-binding agents, T include, but are not limited to, large molecular weight proteins such as, for example, full-length antibodies (polyclonal and monoclonal antibodies); single chain antibodies; fragments of antibodies such as Fab, Fab', F(ab')₂, Fᵥ, [Parham, J. Immunol. 131, 2895-2902 (1983)], fragments produced by a Fab expression library, anti-idiotypic (anti-Id) antibodies, CDR's, and epitope-binding fragments of any of the above which immuno-specifically bind to cancer cell antigens, viral antigens or microbial antigens; interferons (such as type I, II, III); peptides; lymphokines such as IL-2, IL-3, IL-4, IL-6, GM-CSF, interferon-gamma (IFN-γ); hormones such as insulin, TRH (thyrotropin releasing hormones), MSH (melanocyte-stimulating hormone), steroid hormones, such as androgens and estrogens, melanocyte-stimulating hormone (MSH); growth factors and colony-stimulating factors such as epidermal growth factors (EGF), granulocyte-macrophage colony-stimulating factor (GM-CSF), transforming growth factors (TGF), such as TGFα, TGFβ, insulin and insulin like growth factors (IGF-I, IGF-II) G-CSF, M-CSF and GM-CSF [Burgess, Immunology Today, 5, 155-158 (1984)]; vaccinia growth factors (VGF); fibroblast growth factors (FGFs); smaller molecular weight proteins, poly-peptide, peptides and peptide hormones, such as bombesin, gastrin, gastrin-releasing peptide; platelet-derived growth factors; interleukin and cytokines, such as interleukin-2 (IL-2), interleukin-6 (IL-6), leukemia inhibitory factors, granulocyte-macrophage colony-stimulating factor (GM-CSF); vitamins, such as folate; apoproteins and glycoproteins, such as transferrin {O'Keefe et al, 260 J. Biol. Chem. 932-937 (1985)}; sugar-binding proteins or lipoproteins, such as lectins; cell nutrient-transport molecules; and small molecular inhibitors, such as prostate-specific membrane antigen (PSMA) inhibitors and small molecular tyrosine kinase inhibitors (TKI), non-peptides or any other cell binding molecule or substance, such as bioactive polymers (Dhar, et al, Proc. Natl. Acad. Sci. 2008, 105, 17356-61); dendrimers (Lee, et al, Nat. Biotechnol. 2005, 23, 1517-26; Almutairi, et al; Proc. Natl. Acad. Sci. 2009, 106, 685-90); nanoparticles (Liong, et al, ACS Nano, 2008, 19, 1309-12; Medarova, et al, Nat. Med. 2007, 13, 372-7; Javier, et al, Bioconjugate Chem. 2008, 19, 1309-12); liposomes (Medinai, et al, Curr. Phar. Des. 2004, 10, 2981-9); viral capsides (Flenniken, et al, Viruses Nanotechnol. 2009, 327, 71-93). In general monoclonal antibodies are preferred as a cell-surface binding agent if an appropriate one is available.

Preferably, T is selected from the group consisting of an antibody, a single chain antibody, an antibody fragment that binds to a target cell, a monoclonal antibody, a single chain monoclonal antibody, a monoclonal antibody fragment that binds to the target cell, a chimeric antibody, a chimeric antibody fragment that binds to the target cell, a domain antibody, a domain antibody fragment that binds to the target cell, an adnectin that mimics antibody, DARPins, a lymphokine, a hormone, a vitamin, a growth factor, a colony stimulating factor, a nutrient-transport molecule (a transferrin), and/or a cell-binding peptide, protein, or small molecule attached or coated on an albumin, a polymer, a dendrimer, a liposome, a nanoparticle, a vesicle, or on a (viral) capsid.

In further preferably, the cell binding agent/molecule, T is capable of targeting against a tumor cell, a virus infected cell, a microorganism infected cell, a parasite infected cell, an autoimmune disease cell, an activated tumor cells, a myeloid cell, an activated T-cell, an affecting B cell, or a melanocyte, or any disease cells expressing any one of the following antigens or receptors: CD1, CD1a, CD1b, CD1c, CD1d, CD1e,CD2, CD3, CD3d, CD3e, CD3g, CD4, CD5,CD6, CD7, CD8, CD8a, CD8b, CD9, CD10, CD11a, CD11b, CD11c, CD11d, CD12w, CD13, CD14, CD15, CD16, CD16a, CD16b, CDw17, CD18, CD19, CD20, CD21, CD22, CD23, CD24, CD25, CD26, CD27, CD28, CD29, CD30, CD31,CD32,CD32a, CD32b, CD33, CD34, CD35, CD36, CD37, CD38, CD39, CD40, CD41,CD42,CD42a, CD42b, CD42c, CD42d, CD43, CD44, CD45, CD46, CD47, CD48, CD49b, CD49c, CD49c, CD49d, CD49f, CD50, CD51, CD52, CD53, CD54, CD55,CD56, CD57, CD58, CD59, CD60, CD60a, CD60b, CD60c, CD61,CD62E, CD62L, CD62P, CD63, CD64, CD65, CD65s, CD66, CD66a, CD66b, CD66c, CD66d, CD66e, CD66f, CD67, CD68, CD69, CD70, CD71, CD72, CD73, CD74, CD75, CD75s, CD76, CD77, CD78, CD79, CD79a, CD79b, CD80, CD81, CD82, CD83, CD84, CD85, CD85a, CD85b, CD85c, CD85d, CD85e, CD85f, CD85g, CD85g, CD85i, CD85j, CD85k, CD85m, CD86, CD87, CD88, CD89, CD90, CD91,CD92, CD93, CD94, CD95, CD96, CD97, CD98, CD99, CD100, CD101, CD102, CD103, CD104, CD105, CD106, CD107, CD107a, CD107b, CD108, CD109, CD110, CD111, CD112, CD113, CD114, CD115, CD116, CD117, CD118, CD119, CD120, CD120a, CD120b, CD121, CD121a, CD121b, CD122, CD123, CD123a, CD124, CD125, CD126, CD127, CD128, CD129, CD130, CD131, CD132, CD133, CD134, CD135, CD136, CD137, CD138, CD139, CD140, CD140a, CD140b, CD141, CD142, CD143, CD144, CD145, CDw145, CD146, CD147, CD148, CD149, CD150, CD151, CD152, CD153, CD154, CD155, CD156, CD156a, CD156b, CD156c, CD156d, CD157, CD158, CD158a, CD158b1, CD158b2, CD158c, CD158d, CD158e1, CD158e2, CD158f2, CD158g, CD158h, CD158i, CD158j, CD158k, CD159, CD159a, CD159b, CD159c, CD160, CD161, CD162, CD163, CD164, CD165, CD166, CD167, CD167a, CD167b, CD168, CD169, CD170, CD171, CD172, CD172a, CD172b, CD172g, CD173, CD174, CD175, CD175s, CD176, CD177, CD178, CD179, CD179a, CD179b, CD180, CD181, CD182, CD183, CD184, CD185, CD186, CDw186, CD187, CD188, CD189, CD190, CD191, CD192, CD193, CD194, CD195, CD196, CD197, CD198, CD199, CDw198, CDw199, CD200, CD201, CD202, CD202(a,b), CD203, CD203c, CD204, CD205, CD206, CD207, CD208, CD209, CD210, CDw210a, CDw210b, CD211, CD212, CD213, CD213a₁, CD213a₂, CD214, CD215, CD216, CD217, CD218, CD218a, CD218, CD21b9, CD220, CD221, CD222, CD223, CD224, CD225, CD226, CD227, CD228, CD229, CD230, CD231, CD232, CD233, CD234, CD235, CD235a, CD235b, CD236, CD237, CD238, CD239, CD240, CD240ce, CD240d, CD241, CD242, CD243, CD244, CD245, CD246, CD247, CD248, CD249, CD250, CD251, CD252, CD253, CD254,CD255, CD256, CD257, CD258, CD259, CD260, CD261, CD262, CD263, CD264, CD265, CD266, CD267, CD268, CD269, CD270, CD271, CD272, CD273, CD274, CD275, CD276, CD277, CD278, CD279, CD281, CD282, CD283, CD284, CD285, CD286, CD287, CD288, CD289, CD290, CD291, CD292, CD293, CD294, CD295, CD296, CD297, CD298, CD299, CD300, CD300a, CD300b, CD300c, CD301, CD302, CD303, CD304, CD305, CD306, CD307, CD307a, CD307b, CD307c, CD307d, CD307e, CD307f, CD308, CD309, CD310, CD311, CD312, CD313, CD314, CD315, CD316, CD317, CD318, CD319, CD320, CD321, CD322, CD323, CD324, CD325, CD326, CD327, CD328, CD329, CD330, CD331, CD332, CD333, CD334, CD335, CD336, CD337, CD338, CD339, CD340, CD341, CD342, CD343, CD344, CD345, CD346, CD347, CD348, CD349, CD350, CD351, CD352, CD353, CD354, CD355, CD356, CD357, CD358, CD359, CD360, CD361, CD362, CD363, CD364, CD365, CD366, CD367, CD368, CD369, CD370, CD371, CD372, CD373, CD374, CD375, CD376, CD377, CD378, CD379, CD381, CD382, CD383, CD384, CD385, CD386, CD387, CD388, CD389, CRIPTO, CRIPTO, CR, CR1, CRGF, CRIPTO, CXCR5, LY64, TDGF1, 4-1BB, APO2, ASLG659, BMPR1B, 4-1BB, 5AC, 5T4 (Trophoblastic glycoprotein, TPBG, 5T4, Wnt-Activated Inhibitory Factor 1 or WAIF1), Adenocarcinoma antigen, AGS-5, AGS-22M6, Activin receptor-like kinase 1, AFP, AKAP-4, ALK, Alpha integrin, Alpha v beta6, Amino-peptidase N, Amyloid beta, Androgen receptor, Angiopoietin 2, Angiopoietin 3, Annexin A1, Anthrax toxin protective antigen, Anti-transferrin receptor, AOC3 (VAP-1), B7-H3, Bacillus anthracis anthrax, BAFF (B-cell activating factor), BCMA, B-lymphoma cell, bcr-abl, Bombesin, BORIS, C5, C242 antigen, CA125 (carbohydrate antigen 125, MUC16), CA-IX (or CAIX, carbonic anhydrase 9), CALLA, CanAg, Canis lupus familiaris IL31, Carbonic anhydrase IX, Cardiac myosin, CCL11(C-C motif chemokine 11), CCR4 (C-C chemokine receptor type 4), CCR5, CD3E (epsilon), CEA (Carcinoembryonic antigen), CEACAM3, CEACAM5 (carcino-embryonic antigen), CFD (Factor D), Ch4D5, Cholecystokinin 2 (CCK2R), CLDN18 (Claudin-18), CLDN18.1 (Claudin-18.1), CLDN18.2 (Claudin-18.2), Clumping factor A, cMet, CRIPTO, FCSF1R (Colony stimulating factor 1 receptor), CSF2 (colony stimulating factor 2, Granulocyte-macrophage colony-stimulating factor (GM-CSF)), CSP4, CTLA4 (cytotoxic T-lymphocyte-associated protein 4), CTAA16.88 tumor antigen, CXCR4, C-X-C chemokine receptor type 4, cyclic ADP ribose hydrolase, Cyclin B1, CYP1B1, Cytomegalovirus, Cytomegalovirus glycoprotein B, Dabigatran, DLL3 (delta-like-ligand 3), DLL4 (delta-like-ligand 4), DPP4 (Dipeptidyl-peptidase 4), DR5 (Death receptor 5), E. coli shiga toxin type-1, E. coli shiga toxin type-2, ED-B, EGFL7 (EGF-like domain-containing protein 7), EGFR, EGFRII, EGFRvIII, Endoglin, Endothelin B receptor, Endotoxin, EpCAM (epithelial cell adhesion molecule), EphA2, Episialin, ERBB2 (Epidermal Growth Factor Receptor 2), ERBB3, ERG (TMPRSS2 ETS fusion gene), Escherichia coli, ETV6-AML, FAP (Fibroblast activation protein alpha), fibroblast surface antigen, FCGR1, alpha-Fetoprotein, Fibrin II, beta chain, Fibronectin extra domain-B, FOLR (folate receptor), Folate receptor alpha, Folate hydrolase, Fos-related antigen IF protein of respiratory syncytial virus, Frizzled receptor, Fucosyl GM1, GD2 ganglioside, G-28 (a cell surface antigen glycolipid), GD3 idiotype, GloboH, Glypican 3, N-glycolylneuraminic acid, GM3, GMCSF receptor α-chain, Growth differentiation factor, GP100, GPNMB (Trans-membrane glycoprotein NMB), GUCY2C (Guanylate cyclase 2C, guanylyl cyclase C(GC-C), intestinal Guanylate cyclase, Guanylate cyclase-C receptor, Heat-stable enterotoxin receptor (hSTAR)), Heat shock proteins, Hemagglutinin, Hepatitis B surface antigen, Hepatitis B virus, HER1 (human epidermal growth factor receptor 1), HER2, HER2/neu, HER3 (ERBB-3), IgG4, HGF/SF (Hepatocyte growth factor/scatter factor), HHGFR, HIV-1, Histone complex, HLA-DR (human leukocyte antigen), HLA-DR10, HLA-DRB , HMWMAA, Human chorionic gonadotropin, HNGF, Human scatter factor receptor kinase, HPV E6/E7, Hsp90, hTERT, ICAM-1 (Intercellular Adhesion Molecule 1), Idiotype, IGF1R (IGF-1, insulin-like growth factor 1 receptor), IGHE, IFN-γ, Influenza hemagglutinin, IgE, IgE Fc region, IGHE, interleukins (comprising IL-1, IL-2, IL-3, IL-4, IL-5, IL-6, IL-6R, IL-7, IL-8, IL-9, IL-10, IL-11, IL-12, IL-13, IL-15, IL-17, IL-17A, IL-18, IL-19, IL-20, IL-21, IL-22, IL-23, IL-27, or IL-28), IL31RA, ILGF2 (Insulin-like growth factor 2), Integrins (α4, α_{IIb}β₃, αvβ3, α₄β₇, α5β1, α6β4, α7β7, αllβ3, α5β5, αvβ5), Interferon gamma-induced protein, ITGA2, ITGB2, KIR2D, Kappa Ig, LCK, Le, Legumain, Lewis-Y antigen, LFA-1 (Lymphocyte function-associated antigen 1, CD11a), LHRH, LINGO-1, Lipoteichoic acid, LIV1A, LMP2, LTA, MAD-CT-1, MAD-CT-2, MAGE-1, MAGE-2, MAGE-3, MAGE A1, MAGE A3, MAGE 4, MART1, MCP-1, MIF (Macrophage migration inhibitory factor, or glycosylation-inhibiting factor (GIF)), MS4A1 (membrane-spanning 4-domains subfamily A member 1), MSLN (mesothelin), MUC1(Mucin 1, cell surface associated (MUC1) or polymorphic epithelial mucin (PEM)), MUC1-KLH, MUC16 (CA125), MCP1(monocyte chemotactic protein 1), MelanA/MART1, ML-IAP, MPG, MS4A1 (membrane-spanning 4-domains subfamily A), MYCN, Myelin-associated glycoprotein, Myostatin, NA17, NARP-1, NCA-90 (granulocyte antigen), Nectin-4 (ASG-22ME), NGF, Neural apoptosis-regulated proteinase 1, NOGO-A, Notch receptor, Nucleolin, Neu oncogene product, NY-BR-1, NY-ESO-1, OX-40, OxLDL (Oxidized low-density lipoprotein), OY-TES1, P21, p53 nonmutant, P97, Page4, PAP, Paratope of anti-(N-glycolylneuraminic acid), PAX3, PAX5, PCSK9, PDCD1 (PD-1, Programmed cell death protein 1), PDGF-Rα (Alpha-type platelet-derived growth factor receptor), PDGFR-β, PDL-1, PLAC1, PLAP-like testicular alkaline phosphatase, Platelet-derived growth factor receptor beta, Phosphate-sodium co-transporter, PMEL 17, Polysialic acid, Proteinase3 (PR1), Prostatic carcinoma, PS (Phosphatidylserine), Prostatic carcinoma cells, Pseudomonas aeruginosa, PSMA, PSA, PSCA, Rabies virus glycoprotein, RHD (Rh polypeptide 1 (RhPI)), Rhesus factor, RANKL, RhoC, Ras mutant, RGS5, ROBO4, Respiratory syncytial virus, RON, ROR1, Sarcoma translocation breakpoints, SART3, Sclerostin, SLAMF7 (SLAM family member 7), Selectin P, SDC1 (Syndecan 1), sLe(a), Somatomedin C, SIP (Sphingosine-1-phosphate), Somatostatin, Sperm protein 17, SSX2, STEAP1 (six-transmembrane epithelial antigen of the prostate 1), STEAP2, STn, TAG-72 (tumor associated glycoprotein 72), Survivin, T-cell receptor, T cell transmembrane protein, TEM1 (Tumor endothelial marker 1), TENB2, Tenascin C (TN-C), TGF-α, TGF-β (Transforming growth factor beta), TGF-β1, TGF-β2 (Transforming growth factor-beta 2), Tie (CD202b), Tie2, TIM-1 (CDX-014), Tn, TNF, TNF-α, TNFRSF8, TNFRSF10B (tumor necrosis factor receptor superfamily member 10B), TNFRSF-13B (tumor necrosis factor receptor superfamily member 13B), TPBG (trophoblast glycoprotein), TRAIL-R1 (Tumor necrosis apoptosis Inducing ligand Receptor 1), TRAILR2 (Death receptor 5 (DR5)), tumor-associated calcium signal transducer 2, tumor specific glycosylation of MUC1, TWEAK receptor, TYRP1(glycoprotein 75), TRP-1 (Trop1), TRP-2 (Trop2), Tyrosinase, VCAM-1, VEGF, VEGF-A, VEGF-2, VEGFR-1, VEGFR2, or vimentin, WT1, XAGE 1, or cells expressing any insulin growth factor receptors, or any epidermal growth factor receptors.

In the process of the conjugation, prior to conjugating with the antimitotic agents of this invention, the cell-binding molecules can be modified through attachment of a more specific peptide, a protein, or a drug, or the other functional molecules with a heterobifunctional cross linker such as with linkers of Amine-to-Nonselective (succinimidyl (NHS)-diazirine (SDA), NHS ester /Azide), Amine-to-Sulfhydryl (NHS ester/maleimide, NHS ester/ pyridyldithiol, NHS esters/ haloacetyl), Sulfhydryl-to-Carbohydrate (Maleimide/Hydrazide, Pyridyldithiol /Hydrazide), Hydroxyl-to-Sulfhydryl (Isocyanate / Maleimide), Amine-to-DNA (NHS ester/ Psoralen), Amine-to-Carboxyl (Carbodiimide).

In the SDA linkage modification, the NHS ester of a SDA linker reacts with primary an amine group of a binding molecule backbone in pH 6∼9 buffer to form a stable amide bond upon release of NHS. Then photoactivation of the diarzirine with long-wave UV light (330-370nm) creates a reactive carbene intermediate that can react with an amine group of a more specific peptide or a protein or the other functional molecule. The order of these two steps can be different as this: an amine group of a functional molecule reacts with a SDA linker first following by photoactive reaction of a binding molecule with long-wave UV light (330-370nm). The SDA crosslinkers can be cleavable (with a disulfide bond inside such as SDAD linker).

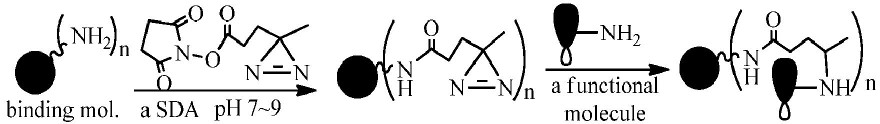

In the NHS ester /Azide linkage modification, the NHS ester of the linker reacts with primary an amine group of a binding molecule backbone in pH 6∼9 buffer to form a stable amide. Then an alkynyl group on a more specific peptide or a protein or the other functional molecule reacts to the azide on the other side of the linker via Azide-Alkyne Huisgen Cycloaddition to form a 1,2,3-triazole linkage (click chemistry). Also, the NHS ester of the linker reacts with primary an amine group of a functional molecule in pH 6∼9 buffer to form a stable amide. Then an alkynyl group being linked on a binding molecule reacts to the azide on the other side of the linker via 5 Azide-Alkyne Huisgen Cycloaddition to form a 1,2,3-triazole linkage.

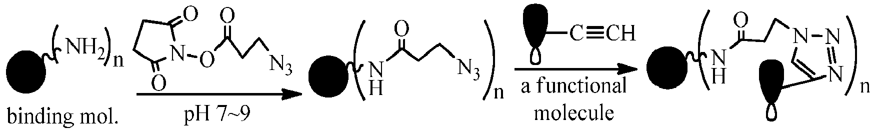

In the Amine-to-Sulfhydryl linkage modification, the NHS ester of the linker reacts with a primary amine group of a binding molecule backbone in pH 6∼9 buffer to form a stable amide bond. Then a sulfhydryl on a more specific peptide or a protein or the other functional molecule reacts to the maleimide, or pyridyldithiol, or haloacetyl on the other side of the Amine-tosulfhydryl linker at pH 4.5 - 8.5 to form a thioether or a disulfide bond. The conjugation with the Amine-to-Sulfhydryl linker can be in different orders. For instance, an amine group of a functional molecule can be reacted with the linker to form an amide bond first, following by reaction with a sulfhydryl on a binding molecule. Also a sulfhydryl group of a functional molecule can be reacted with the linker to form a thioether or a disulfide bond at pH 4.5 - 7 first, following by reaction with an amine group on a binding molecule at pH 6 - 9 to form an amide bond.

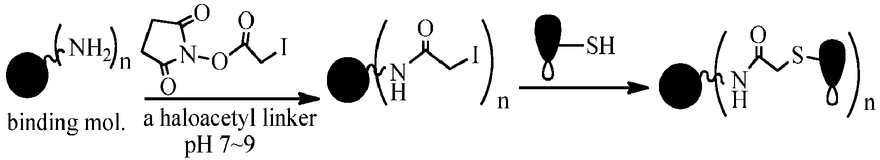

In the Sulfhydryl-to-Carbohydrate linkage modification, the sulfhydryl group of a binding molecule can be reacted with the maleimide or the pyridyldithiol on the linker to form a thioether or a disulfide bond at pH 4.5 - 8 first, Then a carbonyl (aldehyde/ketone) group on a functional molecule reacts with the hydrazide to form an hydrazone bond. Also the sulfhydryl group on a functional molecule can react with the linker to form a thioether or a disulfide bond at pH 4.5 - 8 first, following by reaction with a carbohydrate, or an oxidized carbohydrate, or an carbonyl (aldehyde/ketone) group on a binding molecule form an hydrazone bond.

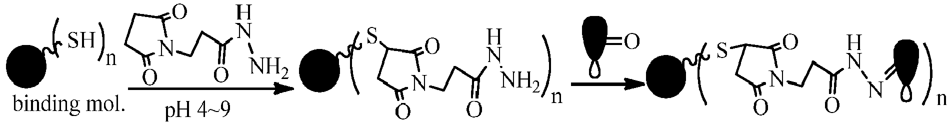

In the Hydroxyl-to-Sulfhydryl linkage modification, the sulfhydryl group of a binding molecule can be reacted with the maleimide or the pyridyldithiol on the linker to form a thioether or a disulfide bond at pH 6 - 8 first, Then a hydroxy group on a functional molecule reacts with the isocyanate on the linker to form a carbamate bond at pH 8 ∼9. Also the sulfhydryl group on a functional molecule can react with the linker to form a thioether or a disulfide bond at pH 6∼ 8 first, following by reaction with a hydroxy on a binding molecule form a carbamate bond at pH 8∼9.

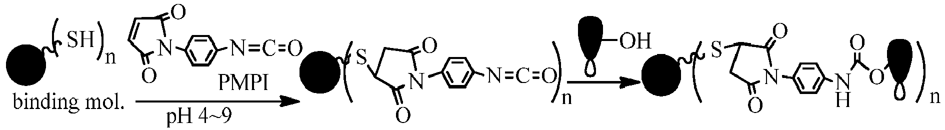

In yet another aspect of the invention, the production of antibodies used in the present invention involves *in vivo* or *in vitro* procedures or combinations thereof. Methods for producing polyclonal anti-receptor peptide antibodies are well-known in the art, such as in U.S. Pat. No. 4,493,795 (to Nestor et al). A monoclonal antibody is typically made by fusing myeloma cells with the spleen cells from a mouse that has been immunized with the desired antigen (Kohler, G.; Milstein, C. (1975). Nature 256: 495-497). The detailed procedures are described in "Antibodies--A Laboratory Manual", Harlow and Lane, eds., Cold Spring Harbor Laboratory Press, New York (1988), which is incorporated herein by reference. Particularly monoclonal antibodies are produced by immunizing mice, rats, hamsters or any other mammal with the antigen of interest such as the intact target cell, antigens isolated from the target cell, whole virus, attenuated whole virus, and viral proteins. Splenocytes are typically fused with myeloma cells using polyethylene glycol (PEG) 6000. Fused hybrids are selected by their sensitivity to HAT (hypoxanthine-aminopterin-thymine). Hybridomas producing a monoclonal antibody useful in practicing this invention are identified by their ability to immunoreact specified receptors or inhibit receptor activity on target cells.

A monoclonal antibody used in the present invention can be produced by initiating a monoclonal hybridoma culture comprising a nutrient medium containing a hybridoma that secretes antibody molecules of the appropriate antigen specificity. The culture is maintained under conditions and for a time period sufficient for the hybridoma to secrete the antibody molecules into the medium. The antibody-containing medium is then collected. The antibody molecules can then be further isolated by well-known techniques, such as using protein-A affinity chromatography; anion, cation, hydrophobic, or size exclusive chromatographies (particularly by affinity for the specific antigen after Protein A, and sizing column chromatography); centrifugation, differential solubility, or by any other standard technique for the purification of proteins.

Media useful for the preparation of these compositions are both well-known in the art and commercially available and include synthetic culture media. An exemplary synthetic medium is Dulbecco's minimal essential medium (DMEM; Dulbecco et al., Virol. 8:396 (1959)) supplemented with 4.5 gm/1 glucose, 20 mm glutamine, 20% fetal calf serum and with an antifoaming agent, such as polyoxyethylene-polyoxypropylene block copolymer.

In addition, antibody-producing cell lines can also be created by techniques other than fusion, such as direct transformation of B lymphocytes with oncogenic DNA, or transfection with an oncovirus, such as Epstein-Barr virus (EBV, also called human herpesvirus 4 (HHV-4)) or Kaposi's sarcoma-associated herpesvirus (KSHV). See, U.S. Pat. Nos. 4,341,761; 4,399,121; 4,427,783; 4,444,887; 4,451,570; 4,466,917; 4,472,500; 4,491,632; 4,493,890. A monoclonal antibody may also be produced via an anti-receptor peptide or peptides containing the carboxyl terminal as described well-known in the art. See Niman et al., Proc. Natl. Acad. Sci. USA, 80: 4949-4953 (1983); Geysen et al., Proc. Natl. Acad. Sci. USA, 82: 178-182 (1985); Lei et al. Biochemistry 34(20): 6675-6688, (1995). Typically, the anti-receptor peptide or a peptide analog is used either alone or conjugated to an immunogenic carrier, as the immunogen for producing anti-receptor peptide monoclonal antibodies.

There are also a number of other well-known techniques for making monoclonal antibodies as binding molecules in this invention. Particularly useful are methods of making fully human antibodies. One method is phage display technology which can be used to select a range of human antibodies binding specifically to the antigen using methods of affinity enrichment. Phage display has been thoroughly described in the literature and the construction and screening of phage display libraries are well known in the art, see, e.g., Dente et al, Gene. 148(1):7-13 (1994); Little et al, Biotechnol Adv. 12(3):539-55 (1994); Clackson et al., Nature 352:264-628 (1991); Huse et al., Science 246:1275-1281 (1989).

Monocolonal antibodies derived by hybridoma technique from another species than human, such as mouse, can be humanized to avoid human anti-mouse antibodies when infused into humans. Among the more common methods of humanization of antibodies are complementarity-determining region grafting and resurfacing. These methods have been extensively described, see e.g. U.S. Pat. Nos. 5,859,205 and 6,797,492; Liu et al, Immunol Rev. 222:9-27 (2008); Almagro et al, Front Biosci. 1;13:1619-33 (2008); Lazar et al, Mol Immunol. 44(8):1986-98 (2007); Li et al, Proc. Natl. Acad. Sci. USA. 103(10):3557-62 (2006) each incorporated herein by reference. Fully human antibodies can also be prepared by immunizing transgenic mice, rabbits, monkeys, or other mammals, carrying large portions of the human immunoglobulin heavy and light chains, with an immunogen. Examples of such mice are: the Xenomouse. (Abgenix, Inc.), the HuMAb-Mouse (Medarex/BMS), the VelociMouse (Regeneron), see also U.S. Pat. No. 6,596,541 , 6,207,418, No. 6,150,584, No. 6,111,166, No. 6,075,181, No. 5,922,545, Nos. 5,661,016, 5,545,806, 5,436,149 and 5,569,825. In human therapy, murine variable regions and human constant regions can also be fused to construct called "chimeric antibodies" that are considerably less immunogenic in man than murine mAbs (Kipriyanov et al, Mol Biotechnol. 26:39-60 (2004); Houdebine, Curr Opin Biotechnol. 13:625-9 (2002) each incorporated herein by reference). In addition, site-directed mutagenesis in the variable region of an antibody can result in an antibody with higher affinity and specificity for its antigen (Brannigan et al, Nat Rev Mol Cell Biol. 3:964-70, (2002)); Adams et al, J Immunol Methods. 231:249-60 (1999)) and exchanging constant regions of a mAb can improve its ability to mediate effector functions of binding and cytotoxicity.

Antibodies immunospecific for a malignant cell antigen can also be obtained commercially or produced by any method known to one of skill in the art such as, e.g., chemical synthesis or recombinant expression techniques. The nucleotide sequence encoding antibodies immunospecific for a malignant cell antigen can be obtained commercially, e.g., from the GenBank database or a database like it, the literature publications, or by routine cloning and sequencing.

Apart from an antibody, a peptide or protein that bind/block/target or in some other way interact with the epitopes or corresponding receptors on a targeted cell can be used as a binding molecule. These peptides or proteins could be any random peptide or proteins that have an affinity for the epitopes or corresponding receptors and they don't necessarily have to be of the immunoglobulin family. These peptides can be isolated by similar techniques as for phage display antibodies (Szardenings, J Recept Signal Transduct Res. 2003; 23(4):307-49). The use of peptides from such random peptide libraries can be similar to antibodies and antibody fragments. The binding molecules of peptides or proteins may be conjugated on or linked to a large molecules or materials, such as, but is not limited, an albumin, a polymer, a liposome, a nano particle, as long as such attachment permits the peptide or protein to retain its antigen binding specificity.

Examples of antibodies used for conjugation of antimitotic agents in this prevention for treating cancer, autoimmune disease, and infectious disease include, but are not limited to, 3F8 (anti-GD2), Abagovomab (anti CA-125), Abciximab (anti CD41 (integrin alpha-IIb), Adalimumab (anti-TNF-a), Adecatumumab (anti-EpCAM, CD326), Afelimomab (anti-TNF-a); Afutuzumab (anti-CD20), Alacizumab pegol (anti-VEGFR2), ALD518 (anti-IL-6), Alemtuzumab (Campath, MabCampath, anti- CD52), Altumomab (anti-CEA), Anatumomab (anti-TAG-72), Anrukinzumab (IMA-638, anti-IL-13), Apolizumab (anti-HLA-DR), Arcitumomab (anti-CEA), Aselizumab (anti-L-selectin (CD62L), Atlizumab (tocilizumab, Actemra, RoActemra, anti-IL-6 receptor), Atorolimumab (anti-Rhesus factor), Bapineuzumab (anti-beta amyloid), Basiliximab (Simulect, antiCD25 (α chain of IL-2 receptor), Bavituximab (anti-phosphatidylserine), Bectumomab (LymphoScan, anti-CD22), Belimumab (Benlysta, LymphoStat-B, anti-BAFF), Benralizumab (anti-CD125), Bertilimumab (anti-CCL11 (eotaxin-1)), Besilesomab (Scintimun, anti-CEA-related antigen), Bevacizumab (Avastin, anti-VEGF-A), Biciromab (FibriScint, anti-fibrin II beta chain), Bivatuzumab (anti-CD44 v6), Blinatumomab (BiTE, anti-CD19), Brentuximab (cAC10, anti-CD30 TNFRSF8), Briakinumab (anti-IL-12, IL-23) Canakinumab (Ilaris, anti-IL-1), Cantuzumab (C242, anti-CanAg), Capromab, Catumaxomab (Removab, anti-EpCAM, anti-CD3), CC49 (anti-TAG-72), Cedelizumab (anti-CD4), Certolizumab pegol (Cimzia anti-TNF-a), Cetuximab (Erbitux, IMC-C225, anti-EGFR), Citatuzumab bogatox (anti-EpCAM), Cixutumumab (anti-IGF-1), Clenoliximab (anti-CD4), Clivatuzumab (anti-MUCl), Conatumumab (anti-TRAIL-R2), CR6261 (anti-Influenza A hemagglutinin), Dacetuzumab (anti-CD40), Daclizumab (Zenapax, anti-CD25 (α chain of IL-2 receptor)), Daratumumab (anti-CD38 (cyclic ADP ribose hydrolase), Denosumab (Prolia, anti-RANKL), Detumomab (anti-B-lymphoma cell), Dorlimomab, Dorlixizumab, Ecromeximab (anti-GD3 ganglioside), Eculizumab (Soliris, anti-C5), Edobacomab (anti-endotoxin), Edrecolomab (Panorex, MAb17-1A, anti-EpCAM), Efalizumab (Raptiva, anti-LFA-1 (CD11a), Efungumab (Mycograb, anti-Hsp90), Elotuzumab (anti-SLAMF7), Elsilimomab (anti-IL-6), Enlimomab pegol (anti-ICAM-1 (CD54)), Epitumomab (anti-episialin), Epratuzumab (anti-CD22), Erlizumab (anti-ITGB2 (CD18)), Ertumaxomab (Rexomun, anti-HER2/neu, CD3), Etaracizumab (Abegrin, anti-integrin αᵥβ₃), Exbivirumab (anti-hepatitis B surface antigen), Fanolesomab (NeutroSpec, anti-CD 15), Faralimomab (anti-interferon receptor), Farletuzumab (anti-folate receptor 1), Felvizumab (anti-respiratory syncytial virus), Fezakinumab (anti-IL-22), Figitumumab (anti-IGF-1 receptor), Fontolizumab (anti-IFN-y), Foravirumab (anti-rabies virus glycoprotein), Fresolimumab (anti-TGF-β), Galiximab (anti-CD80), Gantenerumab (anti- beta amyloid), Gavilimomab (anti-CD147 (basigin)), Gemtuzumab (anti-CD33), Girentuximab (anti-carbonic anhydrase 9), Glembatumumab (CR011, anti-GPNMB), Golimumab (Simponi, anti-TNF-a), Gomiliximab (anti-CD23 (IgE receptor)), anti-HLA-DR antibody, Ibalizumab (anti-CD4), Ibritumomab (anti-CD20), Igovomab (Indimacis-125, anti-CA-125), Imciromab (Myoscint, anti-cardiac myosin), Infliximab (Remicade, anti-TNF-a), Intetumumab (anti-CD51), Inolimomab (anti-CD25 (α chain of IL-2 receptor)), Inotuzumab (anti-CD22), Ipilimumab (anti-CD152), Iratumumab (anti- CD30 (TNFRSF8)), Keliximab (anti-CD4), Labetuzumab (CEA-Cide, anti-CEA), Lebrikizumab (anti- IL-13), Lemalesomab (anti-NCA-90 (granulocyte antigen)), Lerdelimumab (anti-TGF beta 2), Lexatumumab (anti-TRAIL-R2), Libivirumab (anti-hepatitis B surface antigen), Lintuzumab (anti-CD33), Lucatumumab (anti-CD40), Lumiliximab (anti- CD23 (IgE receptor), Mapatumumab (anti-TRAIL-R1), Maslimomab (anti- T-cell receptor), Matuzumab (anti-EGFR), Mepolizumab (Bosatria, anti-IL-5), Metelimumab (anti-TGF beta 1), Milatuzumab (anti-CD74), Minretumomab (anti-TAG-72), Mitumomab (BEC-2, anti-GD3 ganglioside), Morolimumab (anti-Rhesus factor), Motavizumab (Numax, anti-respiratory syncytial virus), Muromonab-CD3 (Orthoclone OKT3, anti-CD3), Nacolomab (anti-C242), Naptumomab (anti-5T4), Natalizumab (Tysabri, anti-integrin α₄), Nebacumab (anti-endotoxin), Necitumumab (anti-EGFR), Nerelimomab (anti-TNF-a), Nimotuzumab (Theracim, Theraloc, anti-EGFR), Nofetumomab, Ocrelizumab (anti-CD20), Odulimomab (Afolimomab, anti-LFA-1 (CD11a)), Ofatumumab (Arzerra, anti-CD20), Olaratumab (anti-PDGF-R α), Omalizumab (Xolair, anti-IgE Fc region), Oportuzumab (anti-EpCAM), Oregovomab (OvaRex, anti-CA-125), Otelixizumab (anti-CD3), Pagibaximab (anti-lipoteichoic acid), Palivizumab (Synagis, Abbosynagis, anti-respiratory syncytial virus), Panitumumab (Vectibix, ABX-EGF, anti-EGFR), Panobacumab (anti- *Pseudomonas aeruginosa*), Pascolizumab (anti-IL-4), Pemtumomab (Theragyn, anti-MUCl), Pertuzumab (Omnitarg, 2C4, anti-HER2/neu), Pexelizumab (anti-C5), Pintumomab (anti-adenocarcinoma antigen), Priliximab (anti-CD4), Pritumumab (anti-vimentin), PRO 140 (anti-CCR5), Racotumomab (1E10, anti-(N-glycolylneuraminic acid (NeuGc, NGNA)-gangliosides GM3)), Rafivirumab (anti-rabies virus glycoprotein), Ramucirumab (anti-VEGFR2), Ranibizumab (Lucentis, anti-VEGF-A), Raxibacumab (anti-anthrax toxin, protective antigen), Regavirumab (anti-cytomegalovirus glycoprotein B), Reslizumab (anti-IL-5), Rilotumumab (anti-HGF), Rituximab (MabThera, Rituxanmab, anti-CD20), Robatumumab (anti-IGF-1 receptor), Rontalizumab (anti-IFN-a), Rovelizumab (LeukArrest, anti-CD 11, CD18), Ruplizumab (Antova, anti-CD154 (CD40L)), Satumomab (anti-TAG-72), Sevirumab (anti-cytomegalovirus), Sibrotuzumab (anti-FAP), Sifalimumab (anti-IFN-a), Siltuximab (anti-IL-6), Siplizumab (anti-CD2), (Smart) MI95 (anti-CD33), Solanezumab (anti-beta amyloid), Sonepcizumab (anti-sphingosine-1-phosphate), Sontuzumab (anti-episialin), Stamulumab (anti-myostatin), Sulesomab (LeukoScan, (anti-NCA-90 (granulocyte antigen), Tacatuzumab (anti-alpha-fetoprotein), Tadocizumab (anti-integrin α_{IIb}β₃), Talizumab (anti-IgE), Tanezumab (anti-NGF), Taplitumomab (anti-CD19), Tefibazumab (Aurexis, (anti-clumping factor A), Telimomab, Tenatumomab (anti-tenascin C), Teneliximab (anti-CD40), Teplizumab (anti-CD3), TGN1412 (anti-CD28), Ticilimumab (Tremelimumab, (anti-CTLA-4), Tigatuzumab (anti-TRAIL-R2), TNX-650 (anti-IL-13), Tocilizumab (Atlizumab, Actemra, RoActemra, (anti-IL-6 receptor), Toralizumab (anti-CD154 (CD40L)), Tositumomab (anti-CD20), Trastuzumab (Herceptin, (anti-HER2/neu), Tremelimumab (anti-CTLA-4), Tucotuzumab celmoleukin (anti-EpCAM), Tuvirumab (anti-hepatitis B virus), Urtoxazumab (anti- *Escherichia coli*), Ustekinumab (Stelara, anti-IL-12, IL-23), Vapaliximab (anti-AOC3 (VAP-1)), Vedolizumab, (anti-integrin α₄β₇), Veltuzumab (anti-CD20), Vepalimomab (anti-AOC3 (VAP-1), Visilizumab (Nuvion, anti-CD3), Vitaxin (anti-vascular integrin avb3), Volociximab (anti-integrin α₅β₁), Votumumab (HumaSPECT, anti-tumor antigen CTAA16.88), Zalutumumab (HuMax-EGFr, (anti-EGFR), Zanolimumab (HuMax-CD4, anti-CD4), Ziralimumab (anti-CD147 (basigin)), Zolimomab (anti-CD5), Etanercept (Enbrel®), Alefacept (Amevive®), Abatacept (Orencia®), Rilonacept (Arcalyst), 14F7 [anti-IRP-2 (Iron Regulatory Protein 2)], 14G2a (anti-GD2 ganglioside, from Nat. Cancer Inst. for melanoma and solid tumors), J591 (anti-PSMA, Weill Cornell Medical School for prostate cancers), 225.28S [anti-HMW-MAA (High molecular weight-melanoma-associated antigen), Sorin Radiofarmaci S.R.L. (Milan, Italy) for melanoma], COL-1 (anti-CEACAM3, CGM1, from Nat. Cancer Inst. USA for colorectal and gastric cancers), CYT-356 (Oncoltad®, for prostate cancers), HNK20 (OraVax Inc. for respiratory syncytial virus), ImmuRAIT (from Immunomedics for NHL), Lym-1 (anti-HLA-DR10, Peregrine Pharm. for Cancers), MAK-195F [anti-TNF (tumor necrosis factor; TNFA, TNF-alpha; TNFSF2), from Abbott / Knoll for Sepsis toxic shock], MEDI-500 [T10B9, anti-CD3, TRαβ (T cell receptor alpha/beta), complex, from Medlmmune Inc for Graft-versus-host disease], RING SCAN [anti-TAG 72 (tumor associated glycoprotein 72), from Neoprobe Corp. for Breast, Colon and Rectal cancers], Avicidin (anti-EPCAM (epithelial cell adhesion molecule), anti-TACSTDl (Tumor-associated calcium signal transducer 1), anti-GA733-2 (gastrointestinal tumor-associated protein 2), anti-EGP-2 (epithelial glycoprotein 2); anti-KSA; KS1/4 antigen; M4S; tumor antigen 17-1A; CD326, from NeoRx Corp. for Colon, Ovarian, Prostate cancers and NHL]; anti-Trop-2-humanized antibody hRS7, LymphoCide (Immunomedics, NJ), Smart ID10 (Protein Design Labs), Oncolym (Techniclone Inc, CA), Allomune (BioTransplant, CA), anti-VEGF (Genentech, CA); CEAcide (Immunomedics, NJ), IMC-1C11 (ImClone Systems) and Cetuximab (ImClone).

Other antibodies as binding ligands include, but are not limited to, are antibodies against the following antigens: Aminopeptidase N (CD13), Annexin A1, B7-H3 (CD276, various cancers), CA125 (ovarian), CA15-3 (carcinomas), CA19-9 (carcinomas), L6 (carcinomas), Lewis Y (carcinomas), Lewis X (carcinomas), alpha fetoprotein (carcinomas), CA242 (colorectal), placental alkaline phosphatase (carcinomas), prostate specific antigen (prostate), prostatic acid phosphatase (prostate), epidermal growth factor (carcinomas), CD2 (Hodgkin's disease, NHL lymphoma, multiple myeloma), CD3 epsilon (T cell lymphoma, lung, breast, gastric, ovarian cancers, autoimmune diseases, malignant ascites), CD19 (B cell malignancies), CD20 (non-Hodgkin's lymphoma), CD22 (leukemia, lymphoma, multiple myeloma, SLE), CD30 (Hodgkin's lymphoma), CD33 (leukemia, autoimmune diseases), CD38 (multiple myeloma), CD40 (lymphoma, multiple myeloma, leukemia (CLL)), CD51 (Metastatic melanoma, sarcoma), CD52 (leukemia), CD56 (small cell lung cancers, ovarian cancer, Merkel cell carcinoma, and the liquid tumor, multiple myeloma), CD66e (cancers), CD70 (metastatic renal cell carcinoma and non-Hodgkin lymphoma), CD74 (multiple myeloma), CD80 (lymphoma), CD98 (cancers), mucin (carcinomas), CD221 (solid tumors), CD227 (breast, ovarian cancers), CD262 (NSCLC and other cancers), CD309 (ovarian cancers), CD326 (solid tumors), CEACAM3 (colorectal, gastric cancers), CEACAM5 (carcinoembryonic antigen; CEA, CD66e) (breast, colorectal and lung cancers), DLL4 (Δ-like-4), EGFR (Epidermal Growth Factor Receptor, various cancers), CTLA4 (melanoma), CXCR4 (CD184, Heme-oncology, solid tumors), Endoglin (CD105, solid tumors), EPCAM (epithelial cell adhesion molecule, bladder, head, neck, colon, NHL prostate, and ovarian cancers), ERBB2 (Epidermal Growth Factor Receptor 2; lung, breast, prostate cancers), FCGR1 (autoimmune diseases), FOLR (folate receptor, ovarian cancers), GD2 ganglioside (cancers), G-28 (a cell surface antigen glyvolipid, melanoma), GD3 idiotype (cancers), Heat shock proteins (cancers), HER1 (lung, stomach cancers), HER2 (breast, lung and ovarian cancers), HLA-DR10 (NHL), HLA-DRB (NHL, B cell leukemia), human chorionic gonadotropin (carcinoma), IGF1R (insulin-like growth factor 1 receptor, solid tumors, blood cancers), IL-2 receptor (interleukin 2 receptor,T-cell leukemia and lymphomas), IL-6R (interleukin 6 receptor, multiple myeloma, RA, Castleman's disease, IL6 dependent tumors), Integrins (ανβ3, α5β1, α6β4, αllβ3, α5β5, αvβ5, for various cancers), MAGE-1 (carcinomas), MAGE-2 (carcinomas), MAGE-3 (carcinomas), MAGE 4 (carcinomas), anti-transferrin receptor (carcinomas), p97 (melanoma), MS4A1 (membrane-spanning 4-domains subfamily A member 1, Non-Hodgkin's B cell lymphoma, leukemia), MUC1 orMUC1-KLH (breast, ovarian, cervix, bronchus and gastrointestinal cancer), MUC16 (CA125) (Ovarian cancers), CEA (colorectal), gp100 (melanoma), MART1 (melanoma), MPG (melanoma), MS4A1 (membrane-spanning 4-domains subfamily A, small cell lung cancers, NHL), Nucleolin, Neu oncogene product (carcinomas), P21 (carcinomas), Paratope of anti-(N-glycolylneuraminic acid, Breast, Melanoma cancers), PLAP-like testicular alkaline phosphatase (ovarian, testicular cancers), PSMA (prostate tumors), PSA (prostate), ROBO4, TAG 72 (tumour associated glycoprotein 72, AML, gastric, colorectal, ovarian cancers), T cell transmembrane protein (cancers), Tie (CD202b), TNFRSF10B (tumor necrosis factor receptor superfamily member 10B, cancers), TNFRSF13B (tumor necrosis factor receptor superfamily member 13B, multiple myeloma, NHL, other cancers, RA and SLE), TPBG (trophoblast glycoprotein, Renal cell carcinoma), TRAIL-R1 (Tumor necrosis apoprosis Inducing ligand Receptor 1,lymphoma, NHL, colorectal, lung cancers), VCAM-1 (CD106, Melanoma), VEGF, VEGF-A, VEGF-2 (CD309) (various cancers). Some other tumor associated antigens recognized by antibodies have been reviewed (Gerber, et al, mAbs 1:3, 247-253 (2009); Novellino et al, Cancer Immunol Immunother. 54(3),187-207 (2005). Franke, et al, Cancer Biother Radiopharm. 2000, 15, 459-76). Examples of these antigens that antibodies against are: Many other **C**luster of **D**ifferentiations (CD4, CD5, CD6, CD7, CD8, CD9, CD10, CD11a, CD11b, CD11c, CD12w, CD14, CD15, CD16, CDw17, CD18, CD21, CD23, CD24, CD25, CD26, CD27, CD28, CD29, CD31, CD32, CD34, CD35, CD36, CD37, CD41, CD42, CD43, CD44, CD45, CD46, CD47, CD48, CD49b, CD49c, CD53, CD54, CD55, CD58, CD59, CD61, CD62E, CD62L, CD62P, CD63, CD68, CD69, CD71, CD72, CD79, CD81, CD82, CD83, CD86, CD87, CD88, CD89, CD90, CD91, CD95, CD96, CD100, CD103, CD105, CD106, CD109, CD117, CD120, CD127, CD133, CD134, CD135, CD138, CD141, CD142, CD143, CD144, CD147, CD151, CD152, CD154, CD156, CD158, CD163, CD166, .CD168, CD184, CDw186, CD195, CD202 (a, b), CD209, CD235a, CD271, CD303, CD304), Annexin A1, Nucleolin, Endoglin (CD105), ROBO4, Amino-peptidase N, Δ-like-4 (DLL4), VEGFR-2 (CD309), CXCR4 9CD184), Tie2, B7-H3, WT1, MUC1, LMP2, HPV E6 E7, EGFRvIII, HER-2/neu, Idiotype, MAGE A3, p53 nonmutant, NY-ESO-1, GD2, CEA, MelanA/MART1, Ras mutant, gp100, p53 mutant, Proteinase3 (PR1), bcr-abl, Tyrosinase, Survivin, hTERT, Sarcoma translocation breakpoints, EphA2, PAP, ML-IAP, AFP, EpCAM, ERG (TMPRSS2 ETS fusion gene), NA17, PAX3, ALK, Androgen receptor, Cyclin B1, Polysialic acid, MYCN, RhoC, TRP-2, GD3, Fucosyl GM1, Mesothelin, PSCA, MAGE A1, sLe(a), CYP1B1, PLAC1, GM3, BORIS, Tn, GloboH, ETV6-AML, NY-BR-1, RGS5, SART3, STn, Carbonic anhydrase IX, PAX5, OY-TES1, Sperm protein 17, LCK, HMWMAA, AKAP-4, SSX2, XAGE 1, B7H3, Legumain, Tie 2, Page4, VEGFR2, MAD-CT-1, FAP, PDGFR-β, MAD-CT-2, Fos-related antigen 1.

In another specific embodiment, the antimitotic agent- binding molecule conjugates of the invention are used in accordance with the compositions and methods of the invention for the treatment of cancers. The cancers include, but are not limited, Adrenocortical Carcinoma, Anal Cancer, Bladder Cancer, Brain Tumor (Adult, Brain Stem Glioma, Childhood, Cerebellar Astrocytoma, Cerebral Astrocytoma, Ependymoma, Medulloblastoma, Supratentorial Primitive Neuroectodermal and Pineal Tumors, Visual Pathway and Hypothalamic Glioma), Breast Cancer, Carcinoid Tumor, Gastrointestinal, Carcinoma of Unknown Primary, Cervical Cancer, Colon Cancer, Endometrial Cancer, Esophageal Cancer, Extrahepatic Bile Duct Cancer, Ewings Family of Tumors (PNET), Extracranial Germ Cell Tumor, Eye Cancer, Intraocular Melanoma, Gallbladder Cancer, Gastric Cancer (Stomach), Germ Cell Tumor, Extragonadal, Gestational Trophoblastic Tumor, Head and Neck Cancer, Hypopharyngeal Cancer, Islet Cell Carcinoma, Kidney Cancer (renal cell cancer), Laryngeal Cancer, Leukemia (Acute Lymphoblastic, Acute Myeloid, Chronic Lymphocytic, Chronic Myelogenous, Hairy Cell), Lip and Oral Cavity Cancer, Liver Cancer, Lung Cancer (Non-Small Cell, Small Cell, Lymphoma (AIDS-Related, Central Nervous System, Cutaneous T-Cell, Hodgkin's Disease, Non-Hodgkin's Disease, Malignant Mesothelioma, Melanoma, Merkel Cell Carcinoma, Metastatic Squamous Neck Cancer with Occult Primary, Multiple Myeloma, and Other Plasma Cell Neoplasms, Mycosis Fungoides, Myelodysplastic Syndrome, Myeloproliferative Disorders, Nasopharyngeal Cancer, Neuroblastoma, Oral Cancer, Oropharyngeal Cancer, Osteosarcoma, Ovarian Cancer (Epithelial, Germ Cell Tumor, Low Malignant Potential Tumor), Pancreatic Cancer (Exocrine, Islet Cell Carcinoma), Paranasal Sinus and Nasal Cavity Cancer, Parathyroid Cancer, Penile Cancer, Pheochromocytoma Cancer, Pituitary Cancer, Plasma Cell Neoplasm, Prostate Cancer Rhabdomyosarcoma, Rectal Cancer, Renal Cell Cancer (kidney cancer), Renal Pelvis and Ureter (Transitional Cell), Salivary Gland Cancer, Sezary Syndrome, Skin Cancer, Skin Cancer (Cutaneous T-Cell Lymphoma, Kaposi's Sarcoma, Melanoma), Small Intestine Cancer, Soft Tissue Sarcoma, Stomach Cancer, Testicular Cancer, Thymoma (Malignant), Thyroid Cancer, Urethral Cancer, Uterine Cancer (Sarcoma), Unusual Cancer of Childhood, Vaginal Cancer, Vulvar Cancer, Wilms' Tumor.

In another specific embodiment, the antimitotic agent- binding molecule conjugates of the invention are used in accordance with the compositions and methods of the invention for the treatment or prevention of an autoimmune disease. The autoimmune diseases include, but are not limited, Achlorhydra Autoimmune Active Chronic Hepatitis, Acute Disseminated Encephalomyelitis, Acute hemorrhagic leukoencephalitis, Addison's Disease, Agammaglobulinemia, Alopecia areata, Amyotrophic Lateral Sclerosis, Ankylosing Spondylitis, Anti-GBM/TBM Nephritis, Antiphospholipid syndrome, Antisynthetase syndrome, Arthritis, Atopic allergy, Atopic Dermatitis, Autoimmune Aplastic Anemia, Autoimmune cardiomyopathy, Autoimmune hemolytic anemia, Autoimmune hepatitis, Autoimmune inner ear disease, Autoimmune lymphoproliferative syndrome, Autoimmune peripheral neuropathy, Autoimmune pancreatitis, Autoimmune polyendocrine syndrome Types I, II, & III, Autoimmune progesterone dermatitis, Autoimmune thrombocytopenic purpura, Autoimmune uveitis, Balo disease/Balo concentric sclerosis, Bechets Syndrome, Berger's disease, Bickerstaff's encephalitis, Blau syndrome, Bullous Pemphigoid, Castleman's disease, Chagas disease, Chronic Fatigue Immune Dysfunction Syndrome, Chronic inflammatory demyelinating polyneuropathy, Chronic recurrent multifocal ostomyelitis, Chronic lyme disease, Chronic obstructive pulmonary disease, Churg-Strauss syndrome, Cicatricial Pemphigoid, Coeliac Disease, Cogan syndrome, Cold agglutinin disease, Complement component 2 deficiency, Cranial arteritis, CREST syndrome, Crohns Disease (a type of idiopathic inflammatory bowel diseases), Cushing's Syndrome, Cutaneous leukocytoclastic angiitis, Dego's disease, Dercum's disease, Dermatitis herpetiformis, Dermatomyositis, Diabetes mellitus type 1, Diffuse cutaneous systemic sclerosis, Dressler's syndrome, Discoid lupus erythematosus, Eczema, Endometriosis, Enthesitis-related arthritis, Eosinophilic fasciitis, Epidermolysis bullosa acquisita, Erythema nodosum, Essential mixed cryoglobulinemia, Evan's syndrome, Fibrodysplasia ossificans progressiva, Fibromyalgia, Fibromyositis, Fibrosing aveolitis, Gastritis, Gastrointestinal pemphigoid, Giant cell arteritis, Glomerulonephritis, Goodpasture's syndrome, Graves' disease, Guillain-Barre syndrome, Hashimoto's encephalitis, Hashimoto's thyroiditis, Haemolytic anaemia, Henoch-Schonlein purpura, Herpes gestationis, Hidradenitis suppurativa, Hughes syndrome (See Antiphospholipid syndrome), Hypogammaglobulinemia, Idiopathic Inflammatory Demyelinating Diseases, Idiopathic pulmonary fibrosis, Idiopathic thrombocytopenic purpura (See Autoimmune thrombocytopenic purpura), IgA nephropathy (Also Berger's disease), Inclusion body myositis, Inflammatory demyelinating polyneuopathy, Interstitial cystitis, Irritable Bowel Syndrome , Juvenile idiopathic arthritis, Juvenile rheumatoid arthritis, Kawasaki's Disease, Lambert-Eaton myasthenic syndrome, Leukocytoclastic vasculitis, Lichen planus, Lichen sclerosus, Linear IgA disease (LAD), Lou Gehrig's Disease (Also Amyotrophic lateral sclerosis), Lupoid hepatitis, Lupus erythematosus, Majeed syndrome, Ménière's disease, Microscopic polyangiitis, Miller-Fisher syndrome, Mixed Connective Tissue Disease, Morphea, Mucha-Habermann disease, Muckle-Wells syndrome, Multiple Myeloma, Multiple Sclerosis, Myasthenia gravis, Myositis, Narcolepsy, Neuromyelitis optica (Devic's Disease), Neuromyotonia, Occular cicatricial pemphigoid, Opsoclonus myoclonus syndrome, Ord thyroiditis, Palindromic rheumatism, PANDAS (Pediatric Autoimmune Neuropsychiatric Disorders Associated with Streptococcus), Paraneoplastic cerebellar degeneration, Paroxysmal nocturnal hemoglobinuria, Parry Romberg syndrome, Parsonnage-Turner syndrome, Pars planitis, Pemphigus, Pemphigus vulgaris, Pernicious anaemia, Perivenous encephalomyelitis, POEMS syndrome, Polyarteritis nodosa, Polymyalgia rheumatica, Polymyositis, Primary biliary cirrhosis, Primary sclerosing cholangitis, Progressive inflammatory neuropathy, Psoriasis, Psoriatic Arthritis, Pyoderma gangrenosum, Pure red cell aplasia, Rasmussen's encephalitis, Raynaud phenomenon, Relapsing polychondritis, Reiter's syndrome, Restless leg syndrome, Retroperitoneal fibrosis, Rheumatoid arthritis, Rheumatoid fever, Sarcoidosis, Schizophrenia, Schmidt syndrome, Schnitzler syndrome, Scleritis, Scleroderma, Sjögren's syndrome, Spondyloarthropathy, Sticky blood syndrome, Still's Disease, Stiff person syndrome, Subacute bacterial endocarditis, Susac's syndrome, Sweet syndrome, Sydenham Chorea, Sympathetic ophthalmia, Takayasu's arteritis, Temporal arteritis (giant cell arteritis), Tolosa-Hunt syndrome, Transverse Myelitis, Ulcerative Colitis (a type of idiopathic inflammatory bowel diseases), Undifferentiated connective tissue disease, Undifferentiated spondyloarthropathy, Vasculitis, Vitiligo, Wegener's granulomatosis, Wilson's syndrome, Wiskott-Aldrich syndrome

In another specific embodiment, a binding molecule used for the conjugate for the treatment or prevention of an autoimmune disease includes, but are not limited to, anti-elastin antibody; Abys against epithelial cells antibody; Anti-Basement Membrane Collagen Type IV Protein antibody; Anti-Nuclear Antibody; Anti ds DNA; Anti ss DNA, Anti Cardiolipin Antibody IgM, IgG; anti-celiac antibody; Anti Phospholipid Antibody IgK, IgG; Anti SM Antibody; Anti Mitochondrial Antibody; Thyroid Antibody; Microsomal Antibody, T-cells antibody; Thyroglobulin Antibody, Anti SCL-70; Anti-Jo; Anti-U.sub.lRNP; Anti-La/SSB; Anti SSA; Anti SSB; Anti Perital Cells Antibody; Anti Histones; Anti RNP; C-ANCA; P-ANCA; Anti centromere; Anti-Fibrillarin, and Anti GBM Antibody, Anti-ganglioside antibody; Anti-Desmogein 3 antibody; Anti-p62 antibody; Anti-sp100 antibody; Anti-Mitochondrial(M2) antibody; Rheumatoid factor antibody; Anti-MCV antibody; Anti-topoisomerase antibody; Anti-neutrophil cytoplasmic (cANCA) antibody.

In certain preferred embodiments, the binding molecule for the conjugate in the present invention, can bind to both a receptor or a receptor complex expressed on an activated lymphocyte which is associated with an autoimmune disease. The receptor or receptor complex can comprise an immunoglobulin gene superfamily member (e.g. CD2, CD3, CD4, CD8, CD19, CD22, CD28, CD79, CD90, CD152/CTLA-4, PD-1, or ICOS), a TNF receptor superfamily member (e.g. CD27, CD40, CD95/Fas, CD134/OX40, CD137/4-1BB, INF-R1, TNFR-2, RANK, TACI, BCMA, osteoprotegerin, Apo2/TRAIL-R1, TRAIL-R2, TRAIL-R3, TRAIL-R4, and APO-3), an integrin, a cytokine receptor, a chemokine receptor, a major histocompatibility protein, a lectin (C-type, S-type, or I-type), or a complement control protein.

In another specific embodiment, useful binding ligands that are immunospecific for a viral or a microbial antigen are humanized or human monoclonal antibodies. As used herein, the term "viral antigen" includes, but is not limited to, any viral peptide, polypeptide protein (e.g. HIV gp120, HIV nef, RSV F glycoprotein, influenza virus neuramimidase, influenza virus hemagglutinin, HTLV tax, herpes simplex virus glycoprotein (e.g. gB, gC, gD, and gE) and hepatitis B surface antigen) that is capable of eliciting an immune response. As used herein, the term "microbial antigen" includes, but is not limited to, any microbial peptide, polypeptide, protein, saccharide, polysaccharide, or lipid molecule (e.g., a bacterial, fungi, pathogenic protozoa, or yeast polypeptide including, e.g., LPS and capsular polysaccharide 5/8) that is capable of eliciting an immune response. Examples of antibodies available 1 for the viral or microbial infection include, but are not limited to, Palivizumab which is a humanized anti-respiratory syncytial virus monoclonal antibody for the treatment of RSV infection; PRO542 which is a CD4 fusion antibody for the treatment of HIV infection; Ostavir which is a human antibody for the treatment of hepatitis B virus; PROTVIR which is a humanized IgG.sub. 1 antibody for the treatment of cytomegalovirus; and anti-LPS antibodies.

The binding molecules -antimitotic agent conjugates of this invention can be used in the treatment of infectious diseases. These infectious diseases include, but are not limited to, Acinetobacter infections, Actinomycosis, African sleeping sickness (African trypanosomiasis), AIDS (Acquired immune deficiency syndrome), Amebiasis, Anaplasmosis, Anthrax, Arcanobacterium haemolyticum infection, Argentine hemorrhagic fever, Ascariasis, Aspergillosis, Astrovirus infection, Babesiosis, Bacillus cereus infection, Bacterial pneumonia, Bacterial vaginosis, Bacteroides infection, Balantidiasis, Baylisascaris infection, BK virus infection, Black piedra, Blastocystis hominis infection, Blastomycosis, Bolivian hemorrhagic fever, Borrelia infection, Botulism (and Infant botulism), Brazilian hemorrhagic fever, Brucellosis, Burkholderia infection, Buruli ulcer, Calicivirus infection (Norovirus and Sapovirus), Campylobacteriosis, Candidiasis (Moniliasis; Thrush), Cat-scratch disease, Cellulitis, Chagas Disease (American trypanosomiasis), Chancroid, Chickenpox, Chlamydia, Chlamydophila pneumoniae infection, Cholera, Chromoblastomycosis, Clonorchiasis, Clostridium difficile infection, Coccidioidomycosis, Colorado tick fever, Common cold (Acute viral rhinopharyngitis; Acute coryza), Creutzfeldt-Jakob disease, Crimean-Congo hemorrhagic fever, Cryptococcosis, Cryptosporidiosis, Cutaneous larva migrans, Cyclosporiasis, Cysticercosis, Cytomegalovirus infection, Dengue fever, Dientamoebiasis, Diphtheria, Diphyllobothriasis, Dracunculiasis, Ebola hemorrhagic fever, Echinococcosis, Ehrlichiosis, Enterobiasis (Pinworm infection), Enterococcus infection, Enterovirus infection, Epidemic typhus, Erythema infectiosum (Fifth disease), Exanthem subitum, Fasciolopsiasis, Fasciolosis, Fatal familial insomnia, Filariasis, Food poisoning by Clostridium perfringens, Free-living amebic infection, Fusobacterium infection, Gas gangrene (Clostridial myonecrosis), Geotrichosis, Gerstmann-Sträussler-Scheinker syndrome, Giardiasis, Glanders, Gnathostomiasis, Gonorrhea, Granuloma inguinale (Donovanosis), Group A streptococcal infection, Group B streptococcal infection, Haemophilus influenzae infection, Hand, foot and mouth disease (HFMD), Hantavirus Pulmonary Syndrome, Helicobacter pylori infection, Hemolytic-uremic syndrome, Hemorrhagic fever with renal syndrome, Hepatitis A, Hepatitis B, Hepatitis C, Hepatitis D, Hepatitis E, Herpes simplex, Histoplasmosis, Hookworm infection, Human bocavirus infection, Human ewingii ehrlichiosis, Human granulocytic anaplasmosis, Human metapneumovirus infection, Human monocytic ehrlichiosis, Human papillomavirus infection, Human parainfluenza virus infection, Hymenolepiasis, Epstein-Barr Virus Infectious Mononucleosis (Mono), Influenza, Isosporiasis, Kawasaki disease, Keratitis, Kingella kingae infection, Kuru, Lassa fever, Legionellosis (Legionnaires' disease), Legionellosis (Pontiac fever), Leishmaniasis, Leprosy, Leptospirosis, Listeriosis, Lyme disease (Lyme borreliosis), Lymphatic filariasis (Elephantiasis), Lymphocytic choriomeningitis, Malaria, Marburg hemorrhagic fever, Measles, Melioidosis (Whitmore's disease), Meningitis, Meningococcal disease, Metagonimiasis, Microsporidiosis, Molluscum contagiosum, Mumps, Murine typhus (Endemic typhus), Mycoplasma pneumonia, Mycetoma, Myiasis, Neonatal conjunctivitis (Ophthalmia neonatorum), (New) Variant Creutzfeldt-Jakob disease (vCJD, nvCJD), Nocardiosis, Onchocerciasis (River blindness), Paracoccidioidomycosis (South American blastomycosis), Paragonimiasis, Pasteurellosis, Pediculosis capitis (Head lice), Pediculosis corporis (Body lice), Pediculosis pubis (Pubic lice, Crab lice), Pelvic inflammatory disease, Pertussis (Whooping cough), Plague, Pneumococcal infection, Pneumocystis pneumonia, Pneumonia, Poliomyelitis, Prevotella infection, Primary amoebic meningoencephalitis, Progressive multifocal leukoencephalopathy, Psittacosis, Q fever, Rabies, Rat-bite fever, Respiratory syncytial virus infection, Rhinosporidiosis, Rhinovirus infection, Rickettsial infection, Rickettsialpox, Rift Valley fever, Rocky mountain spotted fever, Rotavirus infection, Rubella, Salmonellosis, SARS (Severe Acute Respiratory Syndrome), Scabies, Schistosomiasis, Sepsis, Shigellosis (Bacillary dysentery), Shingles (Herpes zoster), Smallpox (Variola), Sporotrichosis, Staphylococcal food poisoning, Staphylococcal infection, Strongyloidiasis, Syphilis, Taeniasis, Tetanus (Lockjaw), Tinea barbae (Barber's itch), Tinea capitis (Ringworm of the Scalp), Tinea corporis (Ringworm of the Body), Tinea cruris (Jock itch), Tinea manuum (Ringworm of the Hand), Tinea nigra, Tinea pedis (Athlete's foot), Tinea unguium (Onychomycosis), Tinea versicolor (Pityriasis versicolor), Toxocariasis (Ocular Larva Migrans), Toxocariasis (Visceral Larva Migrans), Toxoplasmosis, Trichinellosis, Trichomoniasis, Trichuriasis (Whipworm infection), Tuberculosis, Tularemia, Ureaplasma urealyticum infection, Venezuelan equine encephalitis, Venezuelan hemorrhagic fever, Viral pneumonia, West Nile Fever, White piedra (Tinea blanca), Yersinia pseudotuberculosis infection, Yersiniosis, Yellow fever, Zygomycosis.

The binding molecules, proffered antibodies described in this patent that are against pathogenic strains include, but are not limit, Acinetobacter baumannii, Actinomyces israelii, Actinomyces gerencseriae and Propionibacterium propionicus, Trypanosoma brucei, HIV (Human immunodeficiency virus), Entamoeba histolytica, Anaplasma genus, Bacillus anthracis, Arcanobacterium haemolyticum, Junin virus, Ascaris lumbricoides, Aspergillus genus, Astroviridae family, Babesia genus, Bacillus cereus, multiple bacteria, Bacteroides genus, Balantidium coli, Baylisascaris genus, BK virus, Piedraia hortae, Blastocystis hominis, Blastomyces dermatitides, Machupo virus, Borrelia genus, Clostridium botulinum, Sabia, Brucella genus, usually Burkholderia cepacia and other Burkholderia species, Mycobacterium ulcerans, Caliciviridae family, Campylobacter genus, usually Candida albicans and other Candida species, Bartonella henselae, Group A Streptococcus and Staphylococcus, Trypanosoma cruzi, Haemophilus ducreyi, Varicella zoster virus (VZV), Chlamydia trachomatis, Chlamydophila pneumoniae, Vibrio cholerae, Fonsecaea pedrosoi, Clonorchis sinensis, Clostridium difficile, Coccidioides immitis and Coccidioides posadasii, Colorado tick fever virus, rhinoviruses, coronaviruses, CJD prion, Crimean-Congo hemorrhagic fever virus, Cryptococcus neoformans, Cryptosporidium genus, Ancylostoma braziliense; multiple parasites, Cyclospora cayetanensis, Taenia solium, Cytomegalovirus, Dengue viruses (DEN-1, DEN-2, DEN-3 and DEN-4) - Flaviviruses, Dientamoeba fragilis, Corynebacterium diphtheriae, Diphyllobothrium, Dracunculus medinensis, Ebolavirus, Echinococcus genus, Ehrlichia genus, Enterobius vermicularis, Enterococcus genus, Enterovirus genus, Rickettsia prowazekii, Parvovirus B19, Human herpesvirus 6 and Human herpesvirus 7, Fasciolopsis buski, Fasciola hepatica and Fasciola gigantica, FFI prion, Filarioidea superfamily, Clostridium perfringens, Fusobacterium genus, Clostridium perfringens; other Clostridium species, Geotrichum candidum, GSS prion, Giardia intestinalis, Burkholderia mallei, Gnathostoma spinigerum and Gnathostoma hispidum, Neisseria gonorrhoeae, Klebsiella granulomatis, Streptococcus pyogenes, Streptococcus agalactiae, Haemophilus influenzae, Enteroviruses, mainly Coxsackie A virus and Enterovirus 71, Sin Nombre virus, Helicobacter pylori, Escherichia coli O157:H7, Bunyaviridae family, Hepatitis A Virus, Hepatitis B Virus, Hepatitis C Virus, Hepatitis D Virus, Hepatitis E Virus, Herpes simplex virus 1, Herpes simplex virus 2, Histoplasma capsulatum, Ancylostoma duodenale and Necator americanus, Hemophilus influenzae, Human bocavirus, Ehrlichia ewingii, Anaplasma phagocytophilum, Human metapneumovirus, Ehrlichia chaffeensis, Human papillomavirus, Human parainfluenza viruses, Hymenolepis nana and Hymenolepis diminuta, Epstein-Barr Virus, Orthomyxoviridae family, Isospora belli, Kingella kingae, Klebsiella pneumoniae, Klebsiella ozaenas, Klebsiella rhinoscleromotis, Kuru prion, Lassa virus, Legionella pneumophila, Legionella pneumophila, Leishmania genus, Mycobacterium leprae and Mycobacterium lepromatosis, Leptospira genus, Listeria monocytogenes, Borrelia burgdorferi and other Borrelia species, Wuchereria bancrofti and Brugia malayi, Lymphocytic choriomeningitis virus (LCMV), Plasmodium genus, Marburg virus, Measles virus, Burkholderia pseudomallei, Neisseria meningitides, Metagonimus yokagawai, Microsporidia phylum, Molluscum contagiosum virus (MCV), Mumps virus, Rickettsia typhi, Mycoplasma pneumoniae, numerous species of bacteria (Actinomycetoma) and fungi (Eumycetoma), parasitic dipterous fly larvae, Chlamydia trachomatis and Neisseria gonorrhoeae, vCJD prion, Nocardia asteroides and other Nocardia species, Onchocerca volvulus, Paracoccidioides brasiliensis, Paragonimus westermani and other Paragonimus species, Pasteurella genus, Pediculus humanus capitis, Pediculus humanus corporis, Phthirus pubis, Bordetella pertussis, Yersinia pestis, Streptococcus pneumoniae, Pneumocystis jirovecii, Poliovirus, Prevotella genus, Naegleria fowleri, JC virus, Chlamydophila psittaci, Coxiella burnetii, Rabies virus, Streptobacillus moniliformis and Spirillum minus, Respiratory syncytial virus, Rhinosporidium seeberi, Rhinovirus, Rickettsia genus, Rickettsia akari, Rift Valley fever virus, Rickettsia rickettsii, Rotavirus, Rubella virus, Salmonella genus, SARS coronavirus, Sarcoptes scabiei, Schistosoma genus, Shigella genus, Varicella zoster virus, Variola major or Variola minor, Sporothrix schenckii, Staphylococcus genus, Staphylococcus genus, Staphylococcus aureus, Streptococcus pyogenes, Strongyloides stercoralis, Treponema pallidum, Taenia genus, Clostridium tetani, Trichophyton genus, Trichophyton tonsurans, Trichophyton genus, Epidermophyton floccosum, Trichophyton rubrum, and Trichophyton mentagrophytes, Trichophyton rubrum, Hortaea werneckii, Trichophyton genus, Malassezia genus, Toxocara canis or Toxocara cati, Toxoplasma gondii, Trichinella spiralis, Trichomonas vaginalis, Trichuris trichiura, Mycobacterium tuberculosis, Francisella tularensis, Ureaplasma urealyticum, Venezuelan equine encephalitis virus, Vibrio colerae, Guanarito virus, West Nile virus, Trichosporon beigelii, Yersinia pseudotuberculosis, Yersinia enterocolitica, Yellow fever virus, Mucorales order (Mucormycosis) and Entomophthorales order (Entomophthoramycosis), Pseudomonas aeruginosa, Campylobacter (Vibrio) fetus, Aeromonas hydrophila, Edwardsiella tarda, Yersinia pestis, Shigella dysenteriae, Shigella flexneri, Shigella sonnei, Salmonella typhimurium, Treponema pertenue, Treponema carateneum, Borrelia vincentii, Borrelia burgdorferi, Leptospira icterohemorrhagiae, Pneumocystis carinii, Brucella abortus, Brucella suis, Brucella melitensis, Mycoplasma spp., Rickettsia prowazeki, Rickettsia tsutsugumushi, Clamydia spp.; pathogenic fungi (Aspergillus fumigatus, Candida albicans, Histoplasma capsulatum); protozoa (Entomoeba histolytica, Trichomonas tenas, Trichomonas hominis, Tryoanosoma gambiense, Trypanosoma rhodesiense, Leishmania donovani, Leishmania tropica, Leishmania braziliensis, Pneumocystis pneumonia, Plasmodium vivax, Plasmodium falciparum, Plasmodium malaria); or Helminiths (Schistosoma japonicum, Schistosoma mansoni, Schistosoma haematobium, and hookworms).

Other antibodies as a binding ligand in this invention for treatment of viral disease include, but are not limited to, antibodies against antigens of pathogenic viruses, including as examples and not by limitation: Poxyiridae, Herpesviridae, Adenoviridae, Papovaviridae, Enteroviridae, Picornaviridae, Parvoviridae, Reoviridae, Retroviridae, influenza viruses, parainfluenza viruses, mumps, measles, respiratory syncytial virus, rubella, Arboviridae, Rhabdoviridae, Arenaviridae, Non-A/Non-B Hepatitis virus, Rhinoviridae, Coronaviridae, Rotoviridae, Oncovirus [such as, HBV (Hepatocellular carcinoma), HPV (Cervical cancer, Anal cancer), Kaposi's sarcoma-associated herpesvirus (Kaposi's sarcoma), Epstein-Barr virus (Nasopharyngeal carcinoma, Burkitt's lymphoma, Primary central nervous system lymphoma), MCPyV (Merkel cell cancer), SV40 (*Simian virus 40*), HCV (Hepatocellular carcinoma), HTLV-I (Adult T-cell leukemia/ lymphoma)], Immune disorders caused virus: [such as Human Immunodeficiency Virus (AIDS)]; Central nervous system virus: [such as, JCV (Progressive multifocal leukoencephalopathy), MeV (Subacute sclerosing panencephalitis), LCV (Lymphocytic choriomeningitis), Arbovirus encephalitis, Orthomyxoviridae (probable) (Encephalitis lethargica), RV (Rabies), Chandipura virus, Herpesviral meningitis, Ramsay Hunt syndrome type II; Poliovirus (Poliomyelitis, Post-polio syndrome), HTLV-I (Tropical spastic paraparesis)]; Cytomegalovirus (Cytomegalovirus retinitis, HSV (Herpetic keratitis)); Cardiovascular virus [such as CBV (Pericarditis, Myocarditis)]; Respiratory system/acute viral nasopharyngitis/viral pneumonia: [Epstein-Barr virus (EBV infection/Infectious mononucleosis), Cytomegalovirus; SARS coronavirus (Severe acute respiratory syndrome) Orthomyxoviridae: Influenzavirus A/B/C (Influenza/Avian influenza), Paramyxovirus: Human parainfluenza viruses (Parainfluenza), RSV (Human respiratory syncytial virus), hMPV]; Digestive system virus [MuV (Mumps), Cytomegalovirus (Cytomegalovirus esophagitis); Adenovirus (Adenovirus infection); Rotavirus, Norovirus, Astrovirus, Coronavirus; HBV (Hepatitis B virus), CBV, HAV (Hepatitis A virus), HCV (Hepatitis C virus), HDV (Hepatitis D virus), HEV (Hepatitis E virus), HGV (Hepatitis G virus)]; Urogenital virus [such as, BK virus, MuV (Mumps)].

According to a further object, the present invention also concerns pharmaceutical compositions comprising the conjugate of the invention together with a pharmaceutically acceptable carrier for treatment of cancer and autoimmune disorders. The method for treatment of cancer and autoimmune disorders can be practiced *in vitro, in vivo,* or *ex vivo.* Examples of *in vitro* uses include treatments of cell cultures in order to kill all cells except for desired variants that do not express the target antigen; or to kill variants that express undesired antigen. Examples of *ex vivo* uses include treatments of hematopoietic stem cells (HSC) prior to the performance of the transplantation (HSCT) into the same patient in order to kill diseased or malignant cells. For instance, clinical *ex vivo* treatment to remove tumor cells or lymphoid cells from bone marrow prior to autologous transplantation in cancer treatment or in treatment of autoimmune disease, or to remove T cells and other lymphoid cells from allogeneic bone marrow or tissue prior to transplant in order to prevent graft-versus-host disease, can be carried out as follows. Bone marrow is harvested from the patient or other individual and then incubated in medium containing serum to which is added the conjugate of the invention, concentrations range from about 1 pM to 0.1 mM, for about 15 minutes to about 48 hours at about 37 °C. The exact conditions of concentration and time of incubation (=dose) are readily determined by the skilled clinicians. After incubation the bone marrow cells are washed with medium containing serum and returned to the patient by i.v. infusion according to known methods. In circumstances where the patient receives other treatment such as a course of ablative chemotherapy or total-body irradiation between the time of harvest of the marrow and reinfusion of the treated cells, the treated marrow cells are stored frozen in liquid nitrogen using standard medical equipment.

### FORMULATION AND APPLICATION

The conjugates of the patent application are formulated to liquid, or suitable to be lyophilized and subsequently be reconstituted to a liquid formulation . The contemplated excipients, which may be utilized in the aqueous pharmaceutical compositions of the patent application include, for example, flavoring agents, antimicrobial agents, sweeteners, antioxidants, antistatic agents, lipids such as phospholipids or fatty acids, steroids such as cholesterol, protein excipients such as serum albumin (human serum albumin), recombinant human albumin, gelatin, casein, salt-forming counterions such sodium and the like. These and additional known pharmaceutical excipients and/or additives suitable for use in the formulations of the invention are known in the art, e.g., as listed in "The Handbook of Pharmaceutical Excipients, 4th edition, Rowe et al., Eds., American Pharmaceuticals Association (2003); and Remington: the Science and Practice of Pharmacy, 21th edition, Gennaro, Ed., Lippincott Williams & Wilkins (2005).

A pharmaceutical container or vessel is used to hold the pharmaceutical formulation of any of conjugates of the patent application. The vessel is a vial, bottle, pre-filled syringe, pre-filled or auto-injector syringe. The liquid formula can be freeze-dried or drum-dryed to a form of cake or powder in a borosilicate vial or soda lime glass vial. The solid powder can also be prepared by efficient spray drying, and then packed to a vial or a pharmaceutical container for storage and distribution.

In a further embodiment, the invention provides a method for preparing a formulation comprising the steps of: (a) lyophilizing the formulation comprising the conjugates, excipients, and a buffer system; and (b) reconstituting the lyophilized mixture of step (a) in a reconstitution medium such that the reconstituted formulation is stable. The formulation of step (a) may further comprise a stabilizer and one or more excipients selected from a group comprising bulking agent, salt, surfactant and preservative as hereinabove described. As reconstitution media, several diluted organic acids or water, i.e. sterile water, bacteriostatic water for injection (BWFI) or may be used. The reconstitution medium may be selected from water, i.e. sterile water, bacteriostatic water for injection (BWFI) or the group consisting of acetic acid, propionic acid, succinic acid, sodium chloride, magnesium chloride, acidic solution of sodium chloride, acidic solution of magnesium chloride and acidic solution of arginine, in an amount from about 10 to about 250 mM.

A liquid pharmaceutical formulation of the conjugates of the patent application should exhibit a variety of pre-defined characteristics. One of the major concerns in liquid drug products is stability, as proteins/antibodies tend to form soluble and insoluble aggregates during manufacturing and storage. In addition, various chemical reactions can occur in solution (deamidation, oxidation, clipping, isomerization etc.) leading to an increase in degradation product levels and/or loss of bioactivity. Preferably, a conjugate in either liquid or loyphilizate formulation should exhibit a shelf life of more than 6 months at 25°C. More preferred a conjugate in either liquid or loyphilizate formulation should exhibit a shelf life of more than 12 months at 25°C. Most preferred liquid formulation should exhibit a shelf life of about 24 to 36 months at 2-8° C and the loyphilizate formulation should exhibit a shelf life of about preferably up to 60 months at 2-8° C. Both liquid and loyphilizate formulations should exhibit a shelf life for at least two years at -20° C, or -70° C.

In certain embodiments, the formulation is stable following freezing (e. g., -20°C, or -70° C.) and thawing of the formulation, for example following 1, 2 or 3 cycles of freezing and thawing. Stability can be evaluated qualitatively and/or quantitatively in a variety of different ways, including evaluation of drug/antibody(protein) ratio and aggregate formation (for example using UV, size exclusion chromatography, by measuring turbidity, and/or by visual inspection); by assessing charge heterogeneity using cation exchange chromatography, image capillary isoelectric focusing (icIEF) or capillary zone electrophoresis; amino-terminal or carboxy-terminal sequence analysis; mass spectrometric analysis, or matrix-assisted laser desorption ionization/time-of-flight mass spectrometry (MALDI/TOF MS), or HPLC-MS/MS; SDS-PAGE analysis to compare reduced and intact antibody; peptide map (for example tryptic or LYS--C) analysis; evaluating biological activity or antigen binding function of the antibody; etc. Instability may involve any one or more of: aggregation, deamidation (e.g. Asn deamidation), oxidation (e.g. Met oxidation), isomerization (e.g. Asp isomerization), clipping/hydrolysis/fragmentation (e.g. hinge region fragmentation), succinimide formation, unpaired cysteine(s), N-terminal extension, C-terminal processing, glycosylation differences, etc.

A stable conjugate should also "retains its biological activity" in a pharmaceutical formulation, if the biological activity of the conjugate at a given time, e. g. 12 month, within about 20%, preferably about 10% (within the errors of the assay) of the biological activity exhibited at the time the pharmaceutical formulation was prepared as determined in an antigen binding assay, and/or in vitro, cytotoxic assay, for example.

For clinical in vivo use, the conjugate via the linkers of the invention will be supplied as solutions or as a lyophilized solid that can be redissolved in sterile water for injection. Examples of suitable protocols of conjugate administration are as follows. Conjugates are given daily, weekly, biweekly, triweekly, once every four weeks or monthly for 8∼54 weeks as an i.v. bolus. Bolus doses are given in 50 to 1000 ml of normal saline to which human serum albumin (e.g. 0.5 to 1 mL of a concentrated solution of human serum albumin, 100 mg/mL) can optionally be added. Dosages will be about 50 µg to 20 mg/kg of body weight per week, i.v. (range of 10 µg to 200 mg/kg per injection). 4∼54 weeks after treatment, the patient may receive a second course of treatment. Specific clinical protocols with regard to route of administration, excipients, diluents, dosages, times, etc., can be determined by the skilled clinicians.

Examples of medical conditions that can be treated according to the *in vivo* or *ex vivo* methods of killing selected cell populations include malignancy of any types of cancer, autoimmune diseases, graft rejections, and infections (viral, bacterial or parasite).

The amount of a conjugate which is required to achieve the desired biological effect, will vary depending upon a number of factors, including the chemical characteristics, the potency, and the bioavailability of the conjugates, the type of disease, the species to which the patient belongs, the diseased state of the patient, the route of administration, all factors which dictate the required dose amounts, delivery and regimen to be administered.

In general terms, the conjugates via the linkers of this invention may be provided in an aqueous physiological buffer solution containing 0.1 to 10% w/v conjugates for parenteral administration. Typical dose ranges are from 1 µg/kg to 0.1 g/kg of body weight daily; weekly, biweekly, triweekly, or monthly, a preferred dose range is from 0.01 mg/kg to 20 mg/kg of body weight weekly, biweekly, triweekly, or monthly, an equivalent dose in a human. The preferred dosage of drug to be administered is likely to depend on such variables as the type and extent of progression of the disease or disorder, the overall health status of the particular patient, the relative biological efficacy of the compound selected, the formulation of the compound, the route of administration (intravenous, intramuscular, or other), the pharmacokinetic properties of the conjugates by the chosen delivery route, and the speed (bolus or continuous infusion) and schedule of administrations (number of repetitions in a given period of time).

The conjugates of the present invention are also capable of being administered in unit dose forms, wherein the term "unit dose" means a single dose which is capable of being administered to a patient, and which can be readily handled and packaged, remaining as a physically and chemically stable unit dose comprising either the active conjugate itself, or as a pharmaceutically acceptable composition, as described hereinafter. As such, typical total daily/weekly/biweekly/monthly dose ranges are from 0.01 to 100 mg/kg of body weight. By way of general guidance, unit doses for humans range from 1 mg to 3000 mg per day, or per week, per two weeks (biweekly), triweekly, or per month. Preferably the unit dose range is from 1 to 500 mg administered one to four times a month and even more preferably from 1 mg to 100 mg, once a week, or once biweekly, or once triweekly. Conjugates provided herein can be formulated into pharmaceutical compositions by admixture with one or more pharmaceutically acceptable excipients. Such unit dose compositions may be prepared for use by oral administration, particularly in the form of tablets, simple capsules or soft gel capsules; or intranasally, particularly in the form of powders, nasal drops, or aerosols; or dermally, for example, topically in ointments, creams, lotions, gels or sprays, or via trans-dermal patches. The compositions may conveniently be administered in unit dosage form and may be prepared by any of the methods well known in the pharmaceutical art, for example, as described in Remington: The Science and Practice of Pharmacy, 21th ed.; Lippincott Williams & Wilkins: Philadelphia, PA, 2005.

Preferred formulations include pharmaceutical compositions in which a compound of the present invention is formulated for oral or parenteral administration. For oral administration, tablets, pills, powders, capsules, troches and the like can contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavoring agent such as peppermint, or methyl salicylate. Capsules can be in the form of a hard capsule or soft capsule, which are generally made from gelatin blends optionally blended with plasticizers, as well as a starch capsule. In addition, dosage unit forms can contain various other materials that modify the physical form of the dosage unit, for example, coatings of sugar, shellac, or enteric agents. Other oral dosage forms syrup or elixir may contain sweetening agents, preservatives, dyes, colorings, and flavorings. In addition, the active compounds may be incorporated into fast dissolve, modified-release or sustained-release preparations and formulations, and wherein such sustained-release formulations are preferably bi-modal. Preferred tablets contain lactose, cornstarch, magnesium silicate, croscarmellose sodium, povidone, magnesium stearate, or talc in any combination.

Liquid preparations for parenteral administration include sterile aqueous or non-aqueous solutions, suspensions, and emulsions. The liquid compositions may also include binders, buffers, preservatives, chelating agents, sweetening, flavoring and coloring agents, and the like. Non-aqueous solvents include alcohols, propylene glycol, polyethylene glycol, vegetable oils such as olive oil, and organic esters such as ethyl oleate. Aqueous carriers include mixtures of alcohols and water, buffered media, and saline. In particular, biocompatible, biodegradable lactide polymer, lactide/glycolide copolymer, or polyoxyethylene-polyoxypropylene copolymers may be useful excipients to control the release of the active compounds. Intravenous vehicles can include fluid and nutrient replenishers, electrolyte replenishers, such as those based on Ringer's dextrose, and the like. Other potentially useful parenteral delivery systems for these active compounds include ethylene-vinyl acetate copolymer particles, osmotic pumps, implantable infusion systems, and liposomes.

Alternative modes of administration include formulations for inhalation, which include such means as dry powder, aerosol, or drops. They may be aqueous solutions containing, for example, polyoxyethylene-9-lauryl ether, glycocholate and deoxycholate, or oily solutions for administration in the form of nasal drops, or as a gel to be applied intranasally. Formulations for buccal administration include, for example, lozenges or pastilles and may also include a flavored base, such as sucrose or acacia, and other excipients such as glycocholate. Formulations suitable for rectal administration are preferably presented as unit-dose suppositories, with a solid based carrier, such as cocoa butter, and may include a salicylate. Formulations for topical application to the skin preferably take the form of an ointment, cream, lotion, paste, gel, spray, aerosol, or oil. Carriers which can be used include petroleum jelly, lanolin, polyethylene glycols, alcohols, or their combinations. Formulations suitable for transdermal administration can be presented as discrete patches and can be lipophilic emulsions or buffered, aqueous solutions, dissolved and/or dispersed in a polymer or an adhesive.

In a specific embodiment, a conjugate of the invention is administered concurrently with the other known or will be known therapeutic agents such as the chemotherapeutic agent, the radiation therapy, immunotherapy agents, autoimmune disorder agents, anti-infectious agents or the other antibody-drug conjugates, resulting in a *synergistic effect.* In another specific embodiment, the *synergistic* drugs or radiation therapy are administered prior or subsequent to administration of a conjugate, in one aspect at least an hour, 12 hours, a day, a week, biweeks, triweeks, a month, in further aspects several months, prior or subsequent to administration of a conjugate of the invention.

In other embodiments, the *synergistic* drugs include, but not limited to:
1). Chemotherapeutic agents: a). Alkylating agents: such as Nitrogen mustards: chlorambucil, chlornaphazine, cyclophosphamide, dacarbazine, estramustine, ifosfamide, mechlorethamine, mechlorethamine oxide hydrochloride, mannomustine, mitobronitol, melphalan, mitolactol, pipobroman, novembichin, phenesterine, prednimustine, thiotepa, trofosfamide, uracil mustard; CC-1065 (including its adozelesin, carzelesin and bizelesin synthetic analogues); Duocarmycin (including the synthetic analogues, KW-2189 and CBI-TMI); Benzodiazepine dimers (e.g., dimmers of pyrrolobenzodiazepine (PBD) or tomaymycin, indolinobenzodiazepines, imidazobenzothiadiazepines, or oxazolidino-benzodiazepines); Nitrosoureas: (carmustine, lomustine, chlorozotocin, fotemustine, nimustine, ranimustine); Alkylsulphonates: (busulfan, treosulfan, improsulfan and piposulfan); Triazenes: (dacarbazine); Platinum containing compounds: (carboplatin, cisplatin, oxaliplatin); aziridines, such as benzodopa, carboquone, meturedopa, and uredopa; ethylenimines and methylamelamines including altretamine, triethylenemel-amine, trietylenephosphoramide, triethylenethio-phosphaoramide and trimethylolomel-amine]; b). Plant Alkaloids: such as Vinca alkaloids: (vincristine, vinblastine, vindesine, vinorelbine, navelbin); Taxoids: (paclitaxel, docetaxol) and their analogs, Maytansinoids (DM1, DM2, DM3, DM4, maytansine and ansamitocins) and their analogs, cryptophycins (particularly cryptophycin 1 and cryptophycin 8); epothilones, eleutherobin, discodermo-lide, bryostatins, dolostatins, auristatins, amatoxins, cephalostatins; pancratistatin; a sarcodictyin; spongistatin; c). DNA Topoisomerase Inhibitors: such as [Epipodophyllins: (9-aminocamptothecin, camptothecin, crisnatol, daunomycin, etoposide, etoposide phosphate, irinotecan, mitoxantrone, novantrone, retinoic acids (retinols), teniposide, topotecan, 9-nitrocamptothecin (RFS 2000)); mitomycins: (mitomycin C)]; d). Anti-metabolites: such as {[Anti-folate: DHFR inhibitors: (methotrexate, trimetrexate, denopterin, pteropterin, aminopterin (4-aminopteroic acid) or the other folic acid analogues); IMP dehydrogenase Inhibitors: (mycophenolic acid, tiazofurin, ribavirin, EICAR); Ribonucleotide reductase Inhibitors: (hydroxyurea, deferoxamine)]; [Pyrimidine analogs: Uracil analogs: (ancitabine, azacitidine, 6-azauridine, capecitabine (Xeloda), carmofur, cytarabine, dideoxyuridine, doxifluridine, enocitabine, 5-Fluorouracil, floxuridine, ratitrexed (Tomudex)); Cytosine analogs: (cytarabine, cytosine arabinoside, fludarabine); Purine analogs: (azathioprine, fludarabine, mercaptopurine, thiamiprine, thioguanine)]; folic acid replenisher, such as frolinic acid}; e). Hormonal therapies: such as {Receptor antagonists: [Anti-estrogen: (megestrol, raloxifene, tamoxifen); LHRH agonists: (goscrclin, leuprolide acetate); Anti-androgens: (bicalutamide, flutamide, calusterone, dromostanolone propionate, epitiostanol, goserelin, leuprolide, mepitiostane, nilutamide, testolactone, trilostane and other androgens inhibitors)]; Retinoids/Deltoids: [Vitamin D3 analogs: (CB 1093, EB 1089 KH 1060, cholecalciferol, ergocalciferol); Photodynamic therapies: (verteporfin, phthalocyanine, photosensitizer Pc4, demethoxy-hypocrellin A); Cytokines: (Interferon-alpha, Interferon-gamma, tumor necrosis factor (TNFs), human proteins containing a TNF domain)]}; f). Kinase inhibitors, such as BIBW 2992 (anti-EGFR/Erb2), imatinib, gefitinib, pegaptanib, sorafenib, dasatinib, sunitinib, erlotinib, nilotinib, lapatinib, axitinib, pazopanib. vandetanib, E7080 (anti-VEGFR2), mubritinib, ponatinib (AP24534), bafetinib (INNO-406), bosutinib (SKI-606), cabozantinib, vismodegib, iniparib, ruxolitinib, CYT387, axitinib, tivozanib, sorafenib, bevacizumab, cetuximab, Trastuzumab, Ranibizumab, Panitumumab, ispinesib; g). A poly (ADP-ribose) polymerase (PARP) inhibitors, such as olaparib, niraparib, iniparib, talazoparib, veliparib, veliparib, CEP 9722 (Cephalon's), E7016 (Eisai's), BGB-290 (BeiGene's), 3-aminobenzamide.
   h). antibiotics, such as the enediyne antibiotics (e.g. calicheamicins, especially calicheamicin γ1, δ1, α1 and β1, see, e.g., J. Med. Chem., 39 (11), 2103-2117 (1996), Angew Chem Intl. Ed. Engl. 33:183-186 (1994); dynemicin, including dynemicin A and deoxydynemicin; esperamicin, kedarcidin, C-1027, maduropeptin, as well as neocarzinostatin chromophore and related chromoprotein enediyne antiobiotic chromomophores), aclacinomysins, actinomycin, authramycin, azaserine, bleomycins, cactinomycin, carabicin, carminomycin, carzinophilin; chromomycins, dactinomycin, daunorubicin, detorubicin, 6-diazo-5-oxo-L-norleucine, doxorubicin, morpholino-doxorubicin, cyanomorpholino-doxorubicin, 2-pyrrolino-doxorubicin and deoxydoxorubicin, epirubicin, esorubicin, idarubicin, marcellomycin, nitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; i). Others: such as Polyketides (acetogenins), especially bullatacin and bullatacinone; gemcitabine, epoxomicins (e. g. carfilzomib), bortezomib, thalidomide, lenalidomide, pomalidomide, tosedostat, zybrestat, PLX4032, STA-9090, Stimuvax, allovectin-7, Xegeva, Provenge, Yervoy, Isoprenylation inhibitors (such as Lovastatin), Dopaminergic neurotoxins (such as 1-methyl-4-phenylpyridinium ion), Cell cycle inhibitors (such as staurosporine), Actinomycins (such as Actinomycin D, dactinomycin), Bleomycins (such as bleomycin A2, bleomycin B2, peplomycin), Anthracyclines (such as daunorubicin, doxorubicin (adriamycin), idarubicin, epirubicin, pirarubicin, zorubicin, mtoxantrone, MDR inhibitors (such as verapamil), Ca²⁺ ATPase inhibitors (such as thapsigargin), Histone deacetylase inhibitors (Vorinostat, Romidepsin, Panobinostat, Valproic acid, Mocetinostat (MGCD0103), Belinostat, PCI-24781, Entinostat, SB939, Resminostat, Givinostat, AR-42, CUDC-101, sulforaphane, Trichostatin A); Thapsigargin, Celecoxib, glitazones, epigallocatechin gallate, Disulfiram, Salinosporamide A.; Anti-adrenals, such as aminoglutethimide, mitotane, trilostane; aceglatone; aldophosphamide glycoside; aminolevulinic acid; amsacrine; arabinoside, bestrabucil; bisantrene; edatraxate; defofamine; demecolcine; diaziquone; eflornithine (DFMO), elfomithine; elliptinium acetate, etoglucid; gallium nitrate; gacytosine, hydroxyurea; ibandronate, lentinan; lonidamine; mitoguazone; mitoxantrone; mopidamol; nitracrine; pentostatin; phenamet; pirarubicin; podophyllinic acid; 2-ethylhydrazide; procarbazine; PSK®; razoxane; rhizoxin; sizofiran; spirogermanium; tenuazonic acid; triaziquone; 2, 2',2"-trichlorotriethylamine; trichothecenes (especially T-2 toxin, verrucarin A, roridin A and anguidine); urethane, siRNA, antisense drugs, and a nucleolytic enzyme.
2). An anti-autoimmune disease agent includes, but is not limited to, cyclosporine, cyclosporine A, aminocaproic acid, azathioprine, bromocriptine, chlorambucil, chloroquine, cyclophosphamide, corticosteroids (e.g. amcinonide, betamethasone, budesonide, hydrocortisone, flunisolide, fluticasone propionate, fluocortolone danazol, dexamethasone, Triamcinolone acetonide, beclometasone dipropionate), DHEA, enanercept, hydroxychloroquine, infliximab, meloxicam, methotrexate, mofetil, mycophenylate, prednisone, sirolimus, tacrolimus.
3). An anti-infectious disease agent includes, but is not limited to, a). Aminoglycosides: amikacin, astromicin, gentamicin (netilmicin, sisomicin, isepamicin), hygromycin B, kanamycin (amikacin, arbekacin, bekanamycin, dibekacin, tobramycin), neomycin (framycetin, paromomycin, ribostamycin), netilmicin, spectinomycin, streptomycin, tobramycin, verdamicin; b). Amphenicols:azidamfenicol, chloramphenicol, florfenicol, thiamphenicol; c). Ansamycins: geldanamycin, herbimycin; d). Carbapenems: biapenem, doripenem, ertapenem, imipenem/cilastatin, meropenem, panipenem; e). Cephems: carbacephem (loracarbef), cefacetrile, cefaclor, cefradine, cefadroxil, cefalonium, cefaloridine, cefalotin or cefalothin, cefalexin, cefaloglycin, cefamandole, cefapirin, cefatrizine, cefazaflur, cefazedone, cefazolin, cefbuperazone, cefcapene, cefdaloxime, cefepime, cefminox, cefoxitin, cefprozil, cefroxadine, ceftezole, cefuroxime, cefixime, cefdinir, cefditoren, cefepime, cefetamet, cefmenoxime, cefodizime, cefonicid, cefoperazone, ceforanide, cefotaxime, cefotiam, cefozopran, cephalexin, cefpimizole, cefpiramide, cefpirome, cefpodoxime, cefprozil, cefquinome, cefsulodin, ceftazidime, cefteram, ceftibuten, ceftiolene, ceftizoxime, ceftobiprole, ceftriaxone, cefuroxime, cefuzonam, cephamycin (cefoxitin, cefotetan, cefmetazole), oxacephem (flomoxef, latamoxef); f). Glycopeptides: bleomycin, vancomycin (oritavancin, telavancin), teicoplanin (dalbavancin), ramoplanin; g). Glycylcyclines: e. g. tigecycline; g). β-Lactamase inhibitors: penam (sulbactam, tazobactam), clavam (clavulanic acid); i). Lincosamides: clindamycin, lincomycin; j). Lipopeptides: daptomycin, A54145, calcium-dependent antibiotics (CDA); k). Macrolides: azithromycin, cethromycin, clarithromycin, dirithromycin, erythromycin, flurithromycin, josamycin, ketolide (telithromycin, cethromycin), midecamycin, miocamycin, oleandomycin, rifamycins (rifampicin, rifampin, rifabutin, rifapentine), rokitamycin, roxithromycin, spectinomycin, spiramycin, tacrolimus (FK506), troleandomycin, telithromycin; 1). Monobactams: aztreonam, tigemonam; m). Oxazolidinones: linezolid; n). Penicillins: amoxicillin, ampicillin (pivampicillin, hetacillin, bacampicillin, metampicillin, talampicillin), azidocillin, azlocillin, benzylpenicillin, benzathine benzylpenicillin, benzathine phenoxymethyl-penicillin, clometocillin, procaine benzylpenicillin, carbenicillin (carindacillin), cloxacillin, dicloxacillin, epicillin, flucloxacillin, mecillinam (pivmecillinam), mezlocillin, meticillin, nafcillin, oxacillin, penamecillin, penicillin, pheneticillin, phenoxymethylpenicillin, piperacillin, propicillin, sulbenicillin, temocillin, ticarcillin; o). Polypeptides: bacitracin, colistin, polymyxin B; p). Quinolones: alatrofloxacin, balofloxacin, ciprofloxacin, clinafloxacin, danofloxacin, difloxacin, enoxacin, enrofloxacin, floxin, garenoxacin, gatifloxacin, gemifloxacin, grepafloxacin, kano trovafloxacin, levofloxacin, lomefloxacin, marbofloxacin, moxifloxacin, nadifloxacin, norfloxacin, orbifloxacin, ofloxacin, pefloxacin, trovafloxacin, grepafloxacin, sitafloxacin, sparfloxacin, temafloxacin, tosufloxacin, trovafloxacin; q). Streptogramins: pristinamycin, quinupristin/dalfopristin); r). Sulfonamides: mafenide, prontosil, sulfacetamide, sulfamethizole, sulfanilimide, sulfasalazine, sulfisoxazole, trimethoprim, trimethoprim-sulfamethoxazole (co-trimoxazole); s). Steroid antibacterials: e.g. fusidic acid; t). Tetracyclines: doxycycline, chlortetracycline, clomocycline, demeclocycline, lymecycline, meclocycline, metacycline, minocycline, oxytetracycline, penimepicycline, rolitetracycline, tetracycline, glycylcyclines (e.g. tigecycline); u). Other types of antibiotics: annonacin, arsphenamine, bactoprenol inhibitors (Bacitracin), DADAL/AR inhibitors (cycloserine), dictyostatin, discodermolide, eleutherobin, epothilone, ethambutol, etoposide, faropenem, fusidic acid, furazolidone, isoniazid, laulimalide, metronidazole, mupirocin, mycolactone, NAM synthesis inhibitors (e. g. fosfomycin), nitrofurantoin, paclitaxel, platensimycin, pyrazinamide, quinupristin/dalfopristin, rifampicin (rifampin), tazobactam tinidazole, uvaricin;
4). Anti-viral drugs: a). Entry/fusion inhibitors: aplaviroc, maraviroc, vicriviroc, gp41 (enfuvirtide), PRO 140, CD4 (ibalizumab); b). Integrase inhibitors: raltegravir, elvitegravir, globoidnan A; c). Maturation inhibitors: bevirimat, vivecon; d). Neuraminidase inhibitors: oseltamivir, zanamivir, peramivir; e). Nucleosides &nucleotides: abacavir, aciclovir, adefovir, amdoxovir, apricitabine, brivudine, cidofovir, clevudine, dexelvucitabine, didanosine (ddl), elvucitabine, emtricitabine (FTC), entecavir, famciclovir, fluorouracil (5-FU), 3'-fluoro-substituted 2', 3'-dideoxynucleoside analogues (e.g. 3'-fluoro-2',3'-dideoxythymidine (FLT) and 3'-fluoro-2',3'-dideoxyguanosine (FLG), fomivirsen, ganciclovir, idoxuridine, lamivudine (3TC),1-nucleosides (e.g. *β*-1-thymidine and *β*-1-2'-deoxycytidine), penciclovir, racivir, ribavirin, stampidine, stavudine (d4T), taribavirin (viramidine), telbivudine, tenofovir, trifluridine valaciclovir, valganciclovir, zalcitabine (ddC), zidovudine (AZT); f). Non-nucleosides: amantadine, ateviridine, capravirine, diarylpyrimidines (etravirine, rilpivirine), delavirdine, docosanol, emivirine, efavirenz, foscarnet (phosphonoformic acid), imiquimod, interferon alfa, loviride, lodenosine, methisazone, nevirapine, NOV-205, peginterferon alfa, podophyllotoxin, rifampicin, rimantadine, resiquimod (R-848), tromantadine; g). Protease inhibitors: amprenavir, atazanavir,boceprevir, darunavir, fosamprenavir, indinavir, lopinavir, nelfinavir, pleconaril, ritonavir, saquinavir, telaprevir (VX-950), tipranavir; h). Other types of anti-virus drugs: abzyme, arbidol, calanolide a, ceragenin, cyanovirin-n, diarylpyrimidines, epigallocatechin gallate (EGCG), foscarnet, griffithsin, taribavirin (viramidine), hydroxyurea, KP-1461, miltefosine, pleconaril, portmanteau inhibitors, ribavirin, seliciclib.
5). The radioisotopes for radiotherapy. Examples of radioisotopes (radionuclides) are ³H, ¹¹C, ¹⁴C, ¹⁸F, ³²P, ³⁵S, ⁶⁴Cu, ⁶⁸Ga, ⁸⁶Y, ⁹⁹Tc, ¹¹¹In, ¹²³I, ¹²⁴I, ¹²⁵I, ¹³¹I, ¹³³Xe, ¹⁷⁷Lu, ²¹¹At, or ²¹³Bi. Radioisotope labeled antibodies are useful in receptor targeted imaging experiments or can be for targeted treatment such as with the antibody-radioisotope conjugates (Wu et al (2005) Nature Biotechnology 23(9): 1137-46). The cell binding molecules, e.g. an antibody can be labeled with ligand reagents that bind, chelate or otherwise complex a radioisotope metal, using the techniques described in Current Protocols in Immunology, Volumes 1 and 2, Coligen et al, Ed. Wiley-Interscience, New York, Pubs. (1991). Chelating ligands which may complex a metal ion include DOTA, DOTP, DOTMA, DTPA and TETA (Macrocyclics, Dallas, Tex. USA).
6). Another cell-binding molecule-drug conjugate as a synergy therapy. The preferred synergic conjugate can be a conjugate having a cytotoxic agent of a tubulysin analog, maytansinoid analog, taxanoid (taxane) analog, CC-1065 analog, daunorubicin and doxorubicin compound, amatoxin analog, benzodiazepine dimer (e.g., dimers of pyrrolobenzodiazepine (PBD), tomaymycin, anthramycin, indolinobenzodiazepines, imidazobenzothiadiazepines, or oxazolidinobenzodiazepines), calicheamicins and the enediyne antibiotic compound, actinomycin, azaserine, bleomycins, epirubicin, tamoxifen, idarubicin, dolastatins, auristatins (e.g. monomethyl auristatin E, MMAE , MMAF, auristatin PYE, auristatin TP, Auristatins 2-AQ, 6-AQ, EB (AEB), and EFP (AEFP)), duocarmycins, geldanamycins, methotrexates, thiotepa, vindesines, vincristines, hemiasterlins, nazumamides, microginins, radiosumins, topoisomerase I inhibitors, alterobactins, microsclerodermins, theonellamides, esperamicins, PNU-159682, and their analogues and derivatives above thereof.
7). Other immunotheraphy drugs: e.g. imiquimod, interferons (e.g. α, β), granulocyte colony-stimulating factors, cytokines, Interleukins (IL-1 ∼ IL-35), antibodies (e. g. trastuzumab, pertuzumab, bevacizumab, cetuximab, panitumumab, infliximab, adalimumab, basiliximab, daclizumab, omalizumab, PD-1 or PD-L1), Protein-bound drugs (e.g., Abraxane), an antibody conjugated with drugs selected from calicheamicin derivative, of maytansine derivatives (DM1 and DM4), CC-1065, SN-38, exatecan, topotecan, topoisomerase I inhibitors, duocarmycin, PBD or IGN minor groove binders, potent taxol derivatives, doxorubicin, auristatin antimitotic drugs (e. g. Trastuzumab-DM1, Trastuzumab deruxtecan (DS-8201a), Inotuzumab ozogamicin, Brentuximab vedotin, Sacituzumab govitecan, Glembatumumab vedotin, lorvotuzumab mertansine, AN-152 LMB2, TP-38, VB4-845, Cantuzumab mertansine, AVE9633, SAR3419, CAT-8015 (anti-CD22), IMGN388, Mirvetuximab soravtansine (IMGN853), Enfortumab vedotin, milatuzumab-doxorubicin, SGN-75 (anti-CD70), anti-Her3-exetecan, anti-Trop2-exetecan, nnti-CD79b-MMAE, anti-Her2-MMAE, anti-trop2-MMAE, anti-Her2-MMAF, anti-trop2-MMAF, anti-CD22-calicheamicin derivative, anti-CD22-MMAE, anti-Her2-auristatin derivatives, anti-Mucl- auristatin derivatives, anti-cMet- auristatin derivatives, or anti-Claudin18.2-auristatin derivatives).
8). The pharmaceutically acceptable salts, acids or derivatives of any of the above drugs.

In another synergistic immunotherapy, an antibody of a checkpoint inhibitor, TCR (T cell receptors) T cells, or CARs (chimeric antigen receptors) T cells, or of B cell receptor (BCR), Natural killer (NK) cells, or the cytotoxic cells, or an antibody of anti- CD3, CD4, CD8, CD16 (FcγRIII), CD19, CD20, CD22, CD25, CD27, CD30, CD33, CD37, CD38, CD40, CD40L, CD45RA, CD45RO, CD56, CD57, CD57^{bright}, CD70, CD79, CD79b, CD123, CD125, CD138, TNFβ, Fas ligand, MHC class I molecules (HLA-A, B, C), VEGF, or NKR-P1 antigen is preferred to use along with the conjugates of the present patent for synergistic therapy.

In yet another embodiment, a pharmaceutical composition comprising a therapeuticcally effective amount of the conjugate of Formula (I) ∼ (VII) or any conjugates described through the present patent can be administered concurrently with the other therapeutic agents such as the chemotherapeutic agent, the radiation therapy, immunotherapy agents, autoimmune disorder agents, anti-infectious agents or the other conjugates for synergistically effective treatment or prevention of a cancer, or an autoimmune disease, or an infectious disease.

According to a still further object, the present invention is also concerned with the process of preparation of the conjugate of the invention. The conjugate and process of the present invention may be prepared in a number of ways well known to those skilled in the art. The antimitotic agents used in the conjugate can be synthesized, for example, by application or adaptation of the methods described below, or variations thereon as appreciated by the skilled artisan. The appropriate modifications and substitutions will be readily apparent and well known or readily obtainable from the scientific literature to those skilled in the art. In particular, such methods can be found in R.C. Larock, Comprehensive Organic Transformations, 2nd Edition, Wiley-VCH Publishers, 1999.

In the reactions described hereinafter, it may be necessary to protect reactive functional groups, for example hydroxy, amino, imino, thio or carboxy groups, where these are desired in the final product, to avoid their unwanted participation in the reactions. Conventional protecting groups may be used in accordance with standard practice, for examples see P. G. Wuts and T.W. Greene, Greene's Protective Groups in Organic Synthesis, Wiley-Interscience; 4th edition (2006). Some reactions may be carried out in the presence of a base, or an acid or in a suitable solvent. There is no particular restriction on the nature of the base, acid and solvent to be used in this reaction, and any base, acid or solvent conventionally used in reactions of this type may equally be used here, provided that it has no adverse effect on other parts of the molecule. The reactions can take place over a wide range of temperatures. In general, we find it convenient to carry out the reaction at a temperature of from -80°C to 150°C (more preferably from about room temperature to 100°C). The time required for the reaction may also vary widely, depending on many factors, notably the reaction temperature and the nature of the reagents. However, provided that the reaction is effected under the preferred conditions outlined above, a period of from 3 hours to 20 hours will usually suffice.

The work-up of the reaction can be carried out by conventional means. For example, the reaction products may be recovered by distilling off the solvent from the reaction mixture or, if necessary after distilling off the solvent from the reaction mixture, pouring the residue into water followed by extraction with a water-immiscible organic solvent and distilling off the solvent from the extract. Additionally, the product can, if desired, be further purified by various well known techniques, such as recrystallization, reprecipitation or the various chromatography techniques, notably column chromatography or preparative thin layer chromatography. The synthesis of the antimitotic agents and their conjugates of this invention are illustrated in Figs. 1-21.

The conjugates of the cell-binding molecules with potent antimitotic agents are further illustrated but not restricted by the description in the following examples.

### EXPERIMENTAL MATERIALS:

Mass spectra were obtained using a Bruker Esquire 3000 system. NMR spectra were recorded on a Bruker AVANCE300 spectrometer. Chemical shifts are reported in ppm relative to TMS as an internal standard. Ultraviolet spectra were recorded on a Hitachi U1200 spectrophotometer. HPLC was performed using an Agilent 1100 HPLC system equipped with a fraction collector and a variable wavelength detector. Thin layer chromatography was performed on Analtech GF silica gel TLC plates. Aminal acids and their derivatives as well as preloaded resins were either from Merck Chemicals International Co, or Synthetech Co., or Peptides International Inc or Chembridge International Co. or Sigma -Aldrich Co. Some of the linkers, Linkers of NHS ester /Maleimide (AMAS, BMPS, GMBS, MBS, SMCC, EMCS or Sulfo-EMCS, SMPB, SMPH, LC-SMCC, Sulfo-KMUS, SM(PEG)4, SM(PEG)6, SM(PEG)8, SM(PEG)12, SM(PEG)24); NHS ester /Pyridyldithiol (SPDP, LC-SPDP or Sulfo-LC-SPDP, SMPT, Sulfo-LC-SMPT); NHS esters /Haloacetyl (SIA, SBAP, SIAB or Sulfo-SIAB); NHS ester /Diazirine (SDA or Sulfo-SDA, LC-SDA or Sulfo-LC-SDA, SDAD or Sulfo-SDAD); Maleimide /Hydrazide (BMPH, EMCH, MPBH, KMUH); Pyridyldithiol /Hydrazide (PDPH); Isocyanate /Maleimide (PMPI) were purchased from Thermo Fisher Scientific Co. SPDB, SPP linkers were made according to the references (Cumber, A. et al, Bioconjugate Chem., 1992, 3, 397-401). T-DM1 and Trastuzumab was from Genentech. All other chemicals or anhydrous solvents were from Sigma-Aldrich International or Aladdin Chemical (Shanghai) Ltd.

### Example 1. Synthesis of di-tert-butyl-1,2-bis(2-(tert-butoxy)-2-oxoethyl) hydrazine- 1,2-dicarboxylate.

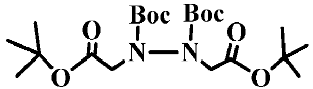

To di-tert-butyl hydrazine-1,2-dicarboxylate (8.01 g, 34,4 mmol) in DMF (150 ml) was added NaH (60% in oil, 2.76 g, 68.8 mmol). After stirred at RT for 30 min, tert-butyl 2-bromoacetate (14.01 g, 72.1 mmol) was added. The mixture was stirred overnight, quenched with addition of methanol (3 ml), concentrated, diluted with EtOAc (100 ml) and water (100 ml), separated, and the aqueous layer was extracted with EtOAc (2 x 50 ml). The organic layers were combined, dried over MgSO₄, filtered, evaporated, and purified purified by SiO₂ column chromatography (EtOAc/Hexane1:5 to 1:3) to afforded the title compound (12.98 g, 82% yield) as a colorless oil.MS ESI m/z calcd for C₂₂H₄₁N₂O₈ [M+H]⁺ 461.28, found 461.40.

### Example 2. Synthesis of 2,2'-(hydrazine-1,2-diyl)diacetic acid.

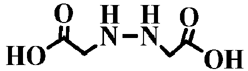

Di-tert-butyl 1,2-bis(2-(tert-butoxy)-2-oxoethyl)hydrazine-1,2-dicarboxylate (6.51 g, 14.14 mmol) in 1,4-dioxane (40 ml) was added HCl (12 M, 10 ml). The mixture was stirred for 30 min, diluted with dioxane (20 ml) and toluene (40 ml), evaporated and co-evaporated with dioxane (20 ml) and toluene (40 ml) to dryness to afford the crude title product for the next step without further production (2.15 g, 103% yield, ∼93% pure). MS ESI m/z calcd for C₄H₉N₂O₄ [M+H]⁺ 149.05, found 149.40.

### Example 3. Synthesis of 2,2'-(1,2-bis((E)-3-bromoacryloyl)hydrazine-1,2-diyl)diacetic acid.

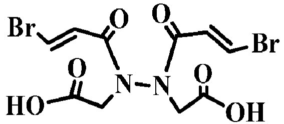

To a solution of 2,2'-(hydrazine-1,2-diyl)diacetic acid (1.10 g, 7.43 mmol) in the mixture of THF (50 ml) and NaH₂PO₄ (0.1 M, 80 ml, pH 6.0) was added(E)-3-bromoacryloyl bromide (5.01 g, 23.60 mmol). The mixture was stirred for 6 h, concentrated and purified on SiO₂ column eluted with H₂O/CH₃CN (1:9) containing 3% formic acid to afford the title compound (2.35 g, 77% yield, ∼93% pure). MS ESI m/z calcd for C₁₀H₁₁Br₂N₂O₆ [M+H]⁺ 412.89, found 413.50.

### Example 4. Synthesis of 2,2'-(1,2-bis((E)-3-bromoacryloyl)hydrazine-1,2-diyl)diacetyl chloride.

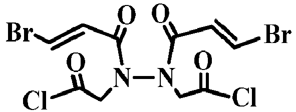

2,2'-(1,2-Bis((E)-3-bromoacryloyl)hydrazine-1,2-diyl)diacetic acid (210 mg, 0.509 mmol) in dichloroethane (15 ml) was added (COCl)₂ (505 mg, 4.01 mmol), followed by addition of 0.040 ml of DMF. After stirred at RT for 2 h, the mixture was concentrated and co-evaporated with dichloroethane (2 x 20 ml) and toluene (2 x 15 ml) to dryness to afford the title crude product (which is not stable) for the next step without further purification (245 mg, 107% yield). MS ESI m/z calcd for C₁₀H₉Br₂Cl₂N₂O₄ [M+H]⁺ 448.82, 450.82, 452.82, 454.82, found 448.60, 450.60, 452.60, 454.60.

### Example 5. Synthesis of tert-butyl 2,8-dioxo-1,5-oxazocane-5-carboxylate.

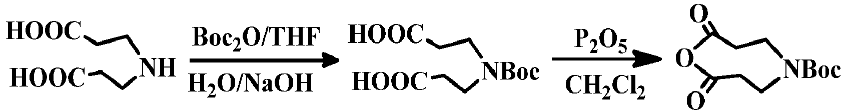

To a solution of 3,3'-azanediyldipropanoic acid(10.00 g, 62.08 mmol) in 1.0 M NaOH (300 ml) at 4 °C was added di-tert-butyl dicarbonate (22.10 g, 101.3 mmol) in 200 ml THF in 1 h. After addition, the mixture was kept to stirring for 2 h at 4 °C. The mixture was carefully acidified to pH ∼4 with 0.2 M H₃PO₄, concentrated in vacuo, extracted with CH₂Cl₂, dried over Na₂SO₄, evaporated and purified with flash SiO₂ chromatography eluted with AcOH/MeOH/CH₂Cl₂ (0.01:1:5) to afford 3,3'-((tert-butoxycarbonyl)azanediyl)dipropanoic acid (13.62 g, 84% yield). ESI MS m/z C₁₁H₁₉NO₆ [M+H]⁺, cacld. 262.27, found 262.40.

To a solution of 3,3'-((tert-butoxycarbonyl)azanediyl)dipropanoic acid (8.0 g, 30.6 mmol) in CH₂Cl₂ (500 ml) at 0 °C was added phosphorus pentoxide (8.70 g, 61.30 mmol). The mixture was stirred at 0 °C for 2 h and then r.t. for 1 h, filtered through short SiO₂ column, and rinsed the column with EtOAc/CH₂Cl₂ (1:6). The filtrate was concentrated and triturated with EtOAc/hexane to afford the title compound (5.64 g, 74% yield). ESI MS m/z C₁₁H₁₇NO₅ [M+H] ⁺, cacld. 244.11, found 244.30.

### Example 6. Synthesis of 2,5-dioxopyrrolidin-1-yl propiolate.

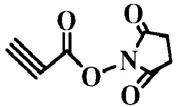

Propiolic acid(5.00 g, 71.4 mmol), NHS (9.01g, 78.3 mmol) and EDC (20.0 g, 104.1 mmol) in CH₂Cl₂ (150 ml) and DIPEA (5 ml, 28.7 mmol) was stirred for overnight, evaporated and purified by SiO₂ column chromatography (EtOAc/Hexane 1:4) to afforded the title compound (9.30 g, 79% yield) as a colorless oil. ¹H NMR (500 MHz, CDCl₃) δ 2.68 (s, 1H), 2.61 (s, 4H). MS ESI m/z calcd for C₇H₅NaNO₄ [M+Na]⁺ 190.02, found 190.20.

### Example 7. Synthesis of tert-butyl 2-propioloylhydrazinecarboxylate.

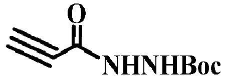

Propiolic acid(5.00 g, 71.4 mmol), tert-butyl hydrazinecarboxylate (9.45g, 71.5 mmol) and EDC (20.0 g, 104.1 mmol) in CH₂Cl₂ (150 ml) and DIPEA (5 ml, 28.7 mmol) was stirred for overnight, evaporated and purified by SiO₂ column chromatography (EtOAc/Hexane 1:5) to afforded the title compound (7.92 g, 84% yield) as a colorless oil. ¹H NMR (500 MHz, CDCl₃) δ 8.76 (m, 2H),2.68 (s, 1H), 1.39 (s, 9H). MS ESI m/z calcd for C₅H₁₂NaN₂O₂ [M+Na]⁺ 155.09, found 155.26.

### Example 8. Synthesis of propiolohydrazide, HCl salt.

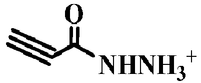

tert-butyl 2-propioloylhydrazinecarboxylate(4.01 g, 30.35 mmol) dissolved in 1,4-dioxane (12 mL) was treated with 4 ml of HCl (conc.) at 4 °C. The mixture was stirred for 30 min, diluted with Dioxane (30 ml) and toluene (30 ml) and concentrated under vacuum. The crude mixture was purified on silica gel using a mixture of methanol (from 5% to 10%) and 1% formic acid in methylene chloride as the eluant to give title compound (2.11 g, 83% yield), ESI MS m/z C₃H₅N₂O [M+H]⁺, cacld. 85.03, found 85.30.

### Example 9. Synthesis of compound 2.

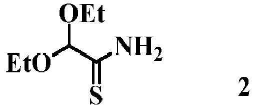

In a 10-L reactor 2,2-diethoxyacetonitrile (1.00 kg, 7.74 mol, 1.0 eq.) was mixed with (NH₄)₂S (48% aqueous solution, 1.41 kg, 9.29 mol, 1.2 eq.) in methanol (6.0 L) at room temperature. The internal temperature increased to 33 °C and then dropped back to r.t. After stirring overnight, the reaction mixture was concentrated under vacuum and the residue was taken up in ethyl acetate (5 L) and washed with saturated NaHCO₃ solution (4 × 1.0 L). The aqueous layer was back-extracted with ethyl acetate (5 × 1.0 L). The organic phases were combined and washed with brine (3 L), dried over anhydrous Na₂SO₄ and concentrated. The resulting solid was collected by vacuum filtration and washed with petroleum ether. The filtrate was concentrated and triturated with petroleum ether to yield a few crops of white or light yellow solid. All crops were combined to give 1.1 kg of desired product (87% yields). ¹H NMR (500 MHz, CDCl₃) δ 7.81 (d, *J* = 71.1 Hz, 2H), 5.03 (s, 1H), 3.73 (dq, *J* = 9.4, 7.1 Hz, 2H), 3.64 (dq, *J* = 9.4, 7.0 Hz, 2H), 1.25 (t, *J* = 7.1 Hz, 6H).

### Example 10. Synthesis of compound 3.

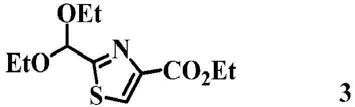

In a 5-L 3-neck round bottle flask, equipped with a reflux condenser and an additional funnel, ethyl bromopyruvate (80% purity, 404 mL, 2.57 mol, 1.2 eq.) was added over 30 min. to a mixture of molecular sieves (3Å, 500 g) and thioamide(350 g, 2.14 mol, 1.0 eq.) in 3 L EtOH. During addition, the internal temperature increased slightly. The reaction mixture was then heated to reflux and stirred for 30 min. After cooling to r.t. the reaction mixture was filter over celite and the filter cake washed with ethyl acetate. The filtrate was concentrated under vacuum. Two batches of the crude product were combined and mixed with silica gel (1.5 kg) and loaded on a silica gel (10 kg packed) column and eluted with ethyl acetate/ petroleum ether (10-20%) to give thiazole carboxylate as a brown oil (509 g, 92% yield).

### Example 11. Synthesis of compound 4.

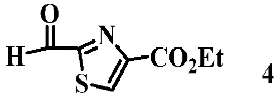

A solution of acetal (300 g, 1.16 mol) in acetone (3.0 L) was heated to reflux and 4N HCl (250 mL) was added over 1.0 h to the refluxing solution. TLC analysis indicated complete consumption of the starting material. The reaction mixture was concentrated under reduced pressure and phases were separated. The organic phase was diluted with ethyl acetate (1.5 L) and washed with saturated NaHCO₃ solution (1.0 L), water (1.0 L) and brine (1.0 L), and then dried over anhydrous Na₂SO₄. All of the aqueous phases were combined and extracted with ethyl acetate. The extracts were combined and dried over anhydrous Na₂SO₄. The organic solutions were filtered and concentrated under reduced pressure. The crude product was triturated with petroleum ether and diethyl ether (5:1) and the resulting solid was collected by vacuum filtration and washed with petroleum ether and ethyl acetate (10:1). The filtrate was concentrated and chromatographed using 0-15% ethyl acetate/petroleum ether to give another crop of desired product. All white to light yellow solids were combined and weighed 40 g (43% yield).¹H NMR (500 MHz, CDCl₃) δ 10.08 - 10.06 (m, 1H), 8.53 - 8.50 (m, 1H), 4.49 (q, *J* = 7.1 Hz, 2H), 1.44 (t, *J* = 7.1 Hz, 3H). MS ESI m/z calcd for C₇H₈NO₃S [M+H]⁺ 186.01; found 186.01.

### Example 12. Synthesis of compound 6.

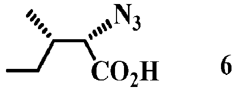

NaN₃ (740 g, 11.4 mol) was dissolved in water (2.0 L) and dichloromethane (2.0 L) was added and cooled at 0 °C, to which Tf₂O(700 mL, 4.10 mol, 1.8 eq.) was added over 1.5 h. After addition was completed, the reaction was stirred at 0 °C for 3 h. The organic phase was separated and the aqueous phase was extracted with dichloromethane (2 × 500 mL). The combined organic phases were washed with saturated NaHCO₃ solution (3 × 1.0 L). This dichloromethane solution of triflyl azide was added to a mixture of (L)-isoleucine (300 g, 2.28 mol, 1.0 eq.), K₂CO₃ (472 g, 3.42 mol, 1.5 eq.), CuSO₄·5H₂O (5.7 g, 22.8 mmol, 0.01 eq.) in water (3.0 L) and methanol (3.0 L) at r.t. During addition, the internal temperature increased slightly. And the mixture was then stirred at r.t. for 16 h. The organic solvents were removed under reduced pressure and the aqueous phase was acidified to pH 6-6.5 with concentrated HCl (about 280 mL added) and then diluted with phosphate buffer (0.25 M, pH 6.2, 6.0 L), washed with EtOAc (6 × 2.0 L) to remove the sulfonamide by-product. The solution was acidified to pH 3 with concentrated HCl (about 400 mL added), extracted with EtOAc (4 × 2.0 L). The combined organic layers were washed with brine (2.0 L) and dried over anhydrous Na₂SO₄, filtered and concentrated to give product **6** (320 g, 89% yield) as a light yellow oil. ¹H NMR (500 MHz, CDCl₃) δ 12.01 (s, 1H), 3.82 (d, *J* = 5.9 Hz, 1H), 2.00 (ddd, *J* = 10.6, 8.6, 5.5 Hz, 1H), 1.54 (dqd, *J* = 14.8, 7.5, 4.4 Hz, 1H), 1.36 - 1.24 (m, 1H), 1.08 - 0.99 (m, 3H), 0.97 - 0.87 (m, 3H).

### Example 13. Synthesis of compound 10.

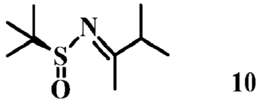

To a solution of (S)-2-methylpropane-2-sulfinamide (100 g, 0.825 mol, 1.0 eq.) in 1 L THF was added Ti(OEt)₄ (345 mL, 1.82 mol, 2.2 eq.) and 3-methyl-2-butanone (81 mL, 0.825 mol, 1.0 eq.) under N₂ at r.t. The reaction mixture was refluxed for 16 h, then cooled to r.t. and poured onto iced water (1L). The mixture was filtered and the filter cake was washed with EtOAc. The organic layer was separated, dried over anhydrous Na₂SO₄ and concentrated to give a residue which was purified by vacuum distillation (15-20 torr, 95 °C) to afforded product **10** (141 g, 90% yield) as a yellow oil. ¹H NMR (500 MHz, CDCl₃) δ 2.54 - 2.44 (m, 1H), 2.25 (s, 3H), 1.17 (s, 9H), 1.06 (dd, *J* = 6.9, 5.1 Hz, 6H). MS ESI m/z calcd for C₉H₁₉NaNOS [M+Na]⁺ 212.12; found 212.11.

### Example 14. Synthesis of compound 11.

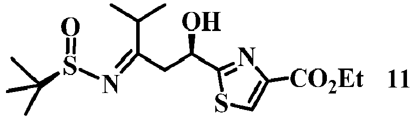

To a solution of diisopropylamine (264 mL, 1.87 mol, 1.65 eq.) in dry THF (1L) was added *n*-butyllithium (2.5 M, 681 mL, 1.70 mol, 1.5 eq.) at -78 °C under N₂. The reaction mixture was warmed to 0 °C over 30 min and then cooled back to -78 °. Compound **10** (258 g, 1.36mol, 1.2 eq.) was added, and rinsed with THF (50 mL). The reaction mixture was stirred for 1 h before ClTi(O*ⁱ*Pr)₃ (834 g, 3.17 mol, 2.8 eq.) in THF (1.05 L) was added dropwise. After stirring for 1 h, compound **4** (210 g, 1.13 mol, 1.0 eq.) dissolved in THF (500 mL) was added dropwise in about 1 hours and the resulting reaction mixture was stirred for 3 h. The completion of the reaction was indicated by TLC analysis. The reaction was quenched by a mixture of acetic acid and THF (v/v 1:1, 300 mL), then poured onto brine (2 L), extracted with EtOAc (8 × 1L). The organic phase was washed with water and brine, dried over anhydrous Na₂SO₄, filtered and concentrated. The residue was purified by column chromatography (DCM/EtOAc/PE 2:1:2) to afforded the compound **11** (298 g, 74% yield) as a colorless oil. ¹H NMR (500 MHz, CDCl₃) δ 8.13 (s, 1H), 6.63 (d, *J=* 8.2 Hz, 1H), 5.20 - 5.11 (m, 1H), 4.43 (q, *J* = 7.0 Hz, 2H), 3.42 - 3.28 (m, 2H), 2.89 (dt, *J* = 13.1, 6.5 Hz, 1H), 1.42 (t, *J* = 7.1 Hz, 3H), 1.33 (s, 9H), 1.25 - 1.22 (m, 6H). MS ESI m/z calcd for C₁₆H₂₆NaN₂O₄S₂ [M+Na]⁺ 397.13, found 397.11.

### Example 15. Synthesis of compound 12.

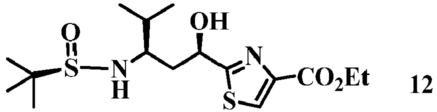

A solution of compound **11** (509 g, 1.35 mol, 1.0 eq.) dissolved in THF (200 mL) was cooled to -78 °C. Ti(OEt)₄ (570 mL, 2.72 mol, 2.0 eq.) was added slowly. After completion of the addition, the mixture was stirred for 1 h, before NaBH₄ (51.3 g, 1.36 mol, 1.0 eq.) was added in portions over 90 min. The reaction mixture was stirred at -78 °C for 3 h. TLC analysis showed starting material still remained. EtOH (50 mL) was added slowly, and the reaction was stirred for 1.5 h and then poured onto brine (2 L, with 250 mL HOAc) and warmed to r.t. After filtration over Celite, the organic phase was separated and washed with water and brine, dried over anhydrous Na₂SO₄, filtered, and concentrated. The residue was purified by column chromatography (EtOAc/PE 1:1) to deliver product **12** (364 g, 71% yield) as a white solid. ¹H NMR (500 MHz, CDCl₃) δ 8.10 (s, 1H), 5.51 (d, *J* = 5.8 Hz, 1H), 5.23 - 5.15 (m, 1H), 4.41 (q, *J* = 7.0 Hz, 2H), 3.48 - 3.40 (m, 1H), 3.37 (d, *J* = 8.3 Hz, 1H), 2.29 (t, *J* = 13.0 Hz, 1H), 1.95 - 1.87 (m, 1H), 1.73 - 1.67 (m, 1H), 1.40 (t, *J* = 7.1 Hz, 3H), 1.29 (s, 9H), 0.93 (d, *J* = 7.3 Hz, 3H), 0.90 (d, *J* = 7.2 Hz, 3H). MS ESI m/z calcd for C₁₆H₂₈NaN₂O₄S₂ [M+Na]⁺ 399.15, found 399.14.

### Example 16. Synthesis of compound 13.

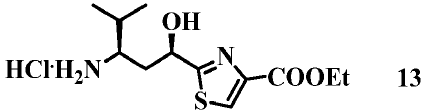

To a solution of compound **12** (600 g, 1.60 mol, 1.0 eq.) in ethanol (590 mL) was added 4 N HCl in dioxane (590 mL) slowly at 0 °C. The reaction was allowed to warm to r.t. and stirred for 2.5 h. A white precipitate crushed out and was collected by filtration and washed with EtOAc. The filtrate was concentrated and triturated with EtOAc. Two crops of white solid were combined and weighed 446 g (90% yield).

### Example 17. Synthesis of compound 14.

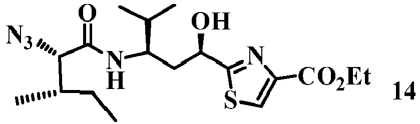

Compound **10:** Azido-Ile-OH (**6**, 153g, 0.97 mol, 2.0 eq.) was dissolved in THF (1.5 L) and cooled to 0 °C, to which NMM (214 mL, 1.94 mol, 4.0 eq.) and isobutylchloroformate (95 mL, 0.73 mol, 2.0 eq.) were added in sequence. The reaction was stirred at 0 °C for 1.0 h. Compound **13** (150 g, 0.49 mmol, 1.0 eq.) was added in portions. After stirring at 0 °C for 30 min, the reaction was warmed to r.t. and stirred for 2 h. Water was added at 0 °C to quench the reaction and the resulting mixture was extracted with EtOAc for three times. The combined organic layers were washed with IN HCl, saturated NaHCO₃ and brine, dried over anhydrous Na₂SO₄, filtered and concentrated. The residue was purified by column chromatography (0-30% EtOAc/PE) to give a white solid (140 g, 70% yield). ¹H NMR (500 MHz, CDCl₃) δ 8.14 (s, 1H), 6.57 (d, *J* = 8.9 Hz, 1H), 4.91 (d, *J* = 11.1 Hz, 1H), 4.44 (dd, *J* = 13.2, 6.3 Hz, 2H), 4.08 - 3.95 (m, 2H), 2.21 (dd, *J* = 24.4, 11.5 Hz, 2H), 1.90 - 1.79 (m, 3H), 1.42 (t, *J* = 6.6 Hz, 3H), 1.37 - 1.27 (m, 2H), 1.11 (d, *J* = 6.4 Hz, 3H), 1.01 - 0.94 (m, 9H). MS ESI m/z calcd for C₁₈H₃₀N₅O₄S [M+H]⁺ 412.19, found 412.19.

### Example 18. Synthesis of compound 15.

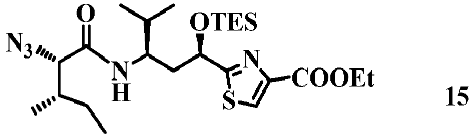

Compound **11:** To a solution of compound **14** (436 g, 1.05 mol, 1.0 eq.) in CH₂Cl₂ (50 mL) was added imidazole (94 g, 1.37 mmol, 1.3 eq.), followed by chlorotriethylsilane (222 mL, 1.32 mol, 1.25 eq.) at 0 °C. The reaction mixture was allowed to warm to r.t. over 1 hour and stirred for an additional hour. Brine was added to the reaction mixture, the organic layer was separated and the aqueous layer was extracted with EtOAc. The combined organic phases were dried, filtered, concentrated under reduced pressure, and purified by column chromatography with a gradient of 15-35% EtOAc in petroleum ether to afford product **15** (557.4 g, 95% yield) as a colorless oil. ¹H NMR (500 MHz, CDCl₃) δ 8.12 (s, 1H), 6.75 (d, *J* = 8.0 Hz, 1H), 5.20 - 5.12 (m, 1H), 4.44 (q, *J* = 7.0 Hz, 2H), 4.06 - 3.97 (m, 1H), 3.87 (d, *J* = 3.8 Hz, 1H), 2.14 (d, *J* = 3.8 Hz, 1H), 2.01 - 1.91 (m, 3H), 1.42 (t, *J* = 7.1 Hz, 3H), 1.34 - 1.25 (m, 2H), 1.06 (d, *J* = 6.8 Hz, 3H), 1.00 - 0.93 (m, 18H), 0.88 (dd, *J* = 19.1, 6.8 Hz, 6H). MS ESI m/z calcd for C₂₄H₄₄N₅O₄SSi [M+H]⁺ 526.28, found 526.28.

### Example 19. Synthesis of compound 16.

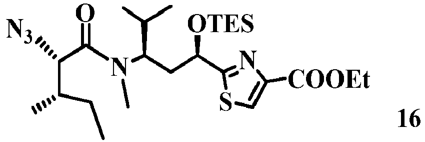

To a solution of **15** (408 g, 0.77 mol, 1.0 eq.) and methyl iodide (145 mL, 2.32 mol, 3.0 eq.) in THF (4 L) was added sodium hydride (60% dispersion in mineral oil, 62.2 g, 1.55 mol, 2.0 eq.) at 0 °C The resulting mixture was stirred at 0 °C overnight and then poured onto ice-water cooled saturated ammonium chloride (5 L) with vigorous stirring. The mixture was then extracted with EtOAc (3 × 500 mL) and the organic layers were dried, filtered, concentrated and purified by column chromatography with a gradient of 15-35% EtOAc in petroleum ether to afford product **16** (388 g, 93% yield) as a light yellow oil. ¹H NMR (500 MHz, CDCl₃) δ 8.09 (s, 1H), 4.95 (d, *J* = 6.6 Hz, 1H), 4.41 (q, *J* = 7.1 Hz, 2H), 3.56 (d, *J* = 9.5 Hz, 1H), 2.98 (s, 3H), 2.27 - 2.06 (m, 4H), 1.83 - 1.70 (m, 2H), 1.41 (t, *J* = 7.2 Hz, 3H), 1.29 (ddd, *J* = 8.9, 6.8, 1.6 Hz, 3H), 1.01 (d, *J* = 6.6 Hz, 3H), 0.96 (dt, *J* = 8.0, 2.9 Hz, 15H), 0.92 (d, *J* = 6.6 Hz, 3H), 0.90 (d, *J* = 6.7 Hz,3H). MS ESI m/z calcd for C₂₅H₄₆N₅O₄SSi [M+H]⁺ 540.30, found 540.30.

### Example 20. Synthesis of compound 17.

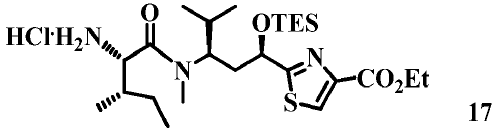

To a solution of compound **16** (1.01 g, 1.87 mmol) in methanol (15 mL) was added 0.1N HCl dropwise until a neutral pH was reached. After addition of Pd/C (10 wt%, 583 mg), the mixture was stirred under H₂ (1 atm) at room temperature for 16 h. The Pd/C was then removed by filtration, with washing of the filter pad with methanol. The filtrate was concentrated under reduced pressure and the residue was re-dissolved in EtOAc (50 mL), dried over anhydrous Na₂SO₄, filtered and concentrated to afford compound **17** (900 mg, 94% yield) as a pale yellow oil.

### Example 21. Synthesis of compound 22.

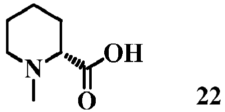

To a solution of D-pipecolinic acid (10.0 g, 77.4 mmol, 1.0 eq.) in methanol (100 mL) was added formaldehyde (37% aqueous solution, 30.8 mL, 154.8 mmol, 2.0 eq.), followed by Pd/C (10 wt%, 1.0 g). The reaction mixture was stirred under H₂ (1 atm) overnight, and then filtered through Celite, with washing of the filter pad with methanol. The filtrate was concentrated under reduced pressure to afford compound **22** (10.0 g, 90% yield) as a white solid.

### Example 22. Synthesis of compound 23.

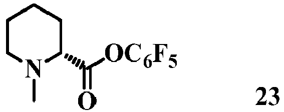

To a solution of D-*N*-methyl pipecolinic acid (2.65 g, 18.5 mmol) in EtOAc (50 mL) were added pentafluorophenol (3.75 g, 20.4 mmol) and DCC (4.21 g, 20.4 mmol). The reaction mixture was stirred at r.t. for 16 h, and then filtered over Celite. The filter pad was washed with 10 mL of EtOAc. The filtrate was used immediately without further purification or concentration.

### Example 23. Synthesis of compound 28.

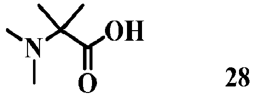

A mixture of 2-amino-2-methylpropanoic acid (500 g, 4.85 mol, 1.0 eq.), aqueous formaldehyde (37%, 1.0 L, 12.1 mol, 2.5 eq.) and formic acid (1.0 L) was heated to reflux (80 °C) for 3.0 h. 6 N HCl (850 mL) was then added at r.t. and the reaction mixture was concentrated. The resulting solid was collected by filtration with washing of ethyl acetate for three times (1.0 L). The solid was dissolved in water (1.5 L) and neutralized to pH 7.0 with 4N NaOH (about 1.0 L solution). The solution was concentrated and co-evaporated with ethanol (2.0 L) to remove residual water. MeOH (2.0 L) was added to the residue and the solid (NaCl) was filtered off with washing of ethyl acetate. The filtrate was concentrated under reduced pressure to give a white solid 639.2 g, which contains some NaCl and was used without further treatment.

### Example 24. Synthesis of compound 29.

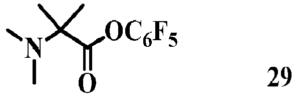

To a solution of 2-(dimethylamino)-2-methylpropanoic acid (97 g, 0.74 mol) in EtOAc (1 L) were added pentafluorophenol (163 g, 0.88 mol) and DIC (126 mL, 0.81 mol). The reaction mixture was stirred at r.t. for 24 h, and then filtered over Celite. The filter pad was washed with 10 mL of EtOAc. The filtrate was used immediately without further purification or concentration.

### Example 25. Synthesis of compound 30.

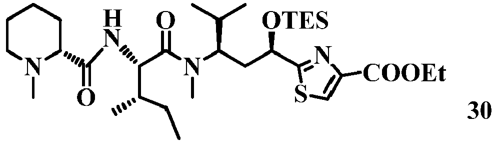

Dry Pd/C (10 wt%, 300 mg) and azide compound **16** (3.33 g, 6.61 mmol) were added to pentafluorophenyl ester **23** in EtOAc. The reaction mixture was stirred under hydrogen atmosphere for 27 h, and then filtered through a plug of Celite, with washing of the filter pad with EtOAc. The combined organic portions were concentrated and purified by column chromatography with a gradient of 0-5% methanol in EtOAc to deliver compound **30** (3.90 g, 86% yield). MS ESI m/z calcd for C₃₂H₅₉N₄O₅SSi [M+H]⁺ 639.39, found 639.39.

### Example 26. Synthesis of compound 31.

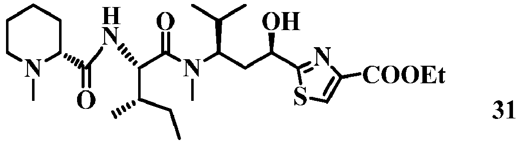

The coupled product compound **30** (3.90 g, 6.1 mmol) was dissolved in AcOH/water/THF (v/v/v 3:1:1, 100 mL), and stirred at r.t. for 48 h. The reaction was then concentrated and purified by column chromatography (2:98 to 15:85 MeOH/EtOAc) to afford compound **31** (2.50 g, 72% yield over 2 steps). MS ESI m/z calcd for C₂₆H₄₅N₄O₅S [M+H]⁺ 525.30, found 525.33.

### Example 27. Synthesis of compound 32.

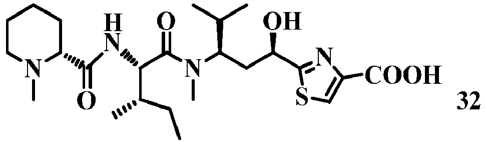

An aqueous solution of LiOH (0.4 N, 47.7 mL, 19.1 mmol, 4.0 eq.) was added to a solution of compound **31** (2.50 g, 4.76 mmol, 1.0 eq.) in dioxane (47.7 mL) at 0 °C. The reaction mixture was stirred at r.t. for 2 h and then concentrated. Column chromatography (100% CH₂Cl₂ to CH₂Cl₂/MeOH/NH₄OH 80:20:1) afforded compound **32** (2.36 g, 99% yield) as an amorphous solid. MS ESI m/z calcd for C₂₄H₄₁N₄O₅S [M+H]⁺ 497.27, found 497.28.

### Example 28. Synthesis of compound 33.

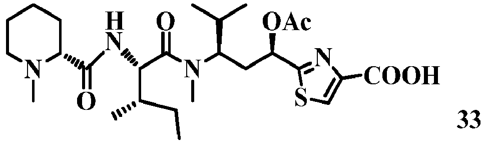

To a solution of compound **32** (2.36 g, 4.75 mmol) in pyridine (50 mL) at 0 °C, acetic anhydride (2.25 mL, 24 mmol) was added slowly. The reaction mixture was allowed to warm to r.t. over 2 h and stirred at r.t. for 24 h. The reaction was concentrated and then treated with dioxane/water (v/v 1:1, 10 mL) for 1 h to destroy possible anhydride. After concentration the residue was purified by column chromatography (100% CH₂Cl₂ to CH₂Cl₂/MeOH/NH₄OH 50:50:1) to afford compound **33** (2.25 g, 88% yield) as an amorphous white solid. MS ESI m/z calcd for C₂₆H₄₃N₄O₆S [M+H]⁺ 539.28, found 539.28.

### Example 29. Synthesis of compound 38.

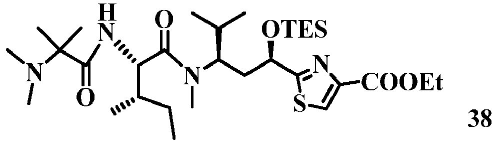

To the EtOAc solution of pentafluorophenyl ester **29,** compound **16** (200 g, 0.37 mol) and dry Pd/C (10 wt%, 10 g) were added. The reaction mixture was stirred under hydrogen atmosphere (1 atm) for 27 h, and then filtered through a plug of Celite, with washing of the filter pad with EtOAc. The combined organic portions were concentrated and purified by column chromatography with a gradient of 0-5% methanol in EtOAc to deliver compound **38** (184 g, 79% yield). MS ESI m/z calcd for C₃₁H₅₈N₄O₅SSi [M+H]⁺ 627.39, found 627.39.

### Example 30. Synthesis of compound 39.

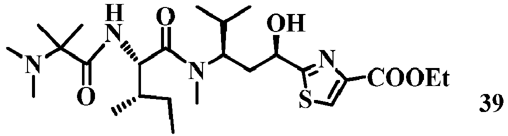

Compound **38** (200 g, 0.32 mmol) was dissolved in AcOH/water/THF (v/v/v 3:1:1, 638 mL), and stirred at r.t. for 4 days. After the reaction was concentrated, toluene was added and concentrated again; this step was repeated two times to afford compound **39,** which was used directly in the next step. MS ESI m/z calcd for C₂₅H₄₅N₄O₅S [M+H]⁺ 513.30, found 513.30.

### Example 31. Synthesis of compound 40.

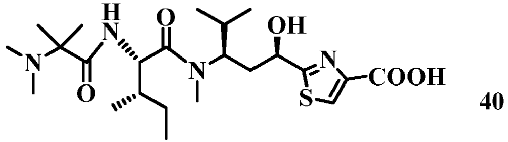

An aqueous solution of LiOH (0.4 N, 600 mL, 2.55 mol, 8.0 eq.) was added to a solution of compound **39** (160 g, 0.319 mol, 1.0 eq.) in MeOH (1.2 L) at 0 °C. The reaction mixture was stirred at r.t. for 2 h and then concentrated. Column chromatography (pure CH₂Cl₂ to 80:20:1 CH₂Cl₂/MeOH/NH₄OH) afforded compound **40** (140 g, 91% yield for two steps) as an amorphous solid. MS ESI m/z calcd for C₂₃H₄₀N₄O₅S [M+H]⁺ 485.27, found 485.27.

### Example 32. Synthesis of compound 41.

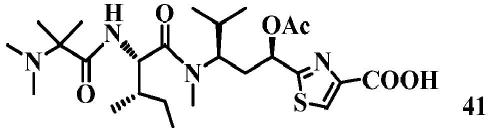

A solution of compound **27** (143 g, 0.30 mol, 1.0 eq.) and DMAP (0.36 g, 2.95 mmol, 0.01 eq.) in anhydrous THF (1.4 L) and anhydrous DMF (75 mL) was cooled to 0 °C, to which TEA (82.2 mL, 0.59 mmol, 2.0 eq.) and acetic anhydride (56 mL, 0.59 mmol, 2.0 eq.) were added. The reaction mixture was allowed to warm to r.t. and stirred for 24 h, and then concentrated. Column chromatography (5-50% MeOH/DCM) delivered compound **41** (147 g, 95% yield) as an amorphous solid. MS ESI m/z calcd for C₂₅H₄₄N₄O₆S [M+H]⁺ 527.28, found 527.28.

### Example 33. Synthesis of compound 41a.

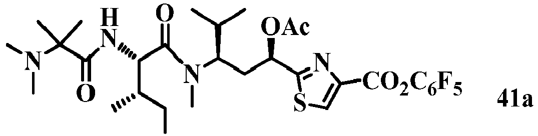

To a solution of compound **41** (5.0 g, 9.5 mmol, 1.0 eq) in anhydrous DCM (100 mL) was added EDC (4.6 g, 23.8 mmol, 2.5 eq) and pentafluorophenol (4.4 g, 23.8 mmol, 2.5 eq) at room temperature under N₂. The mixture was stirred at room temperature for 2 h, and then diluted in DCM (100 mL), washed with water (2 × 200 mL) and brine (200 mL), dried over anhydrous sodium sulfate, filtered, concentrated and purified by SiO₂ column chromatography (50% EtOAc/PE) to give compound **41a** as a white solid (5.2 g, 79% yield) MS ESI m/z calcd for C₃₁H₄₂F₅N₄O₆S [M+H]⁺: 693.27, found:693.27.

### Example 34. Synthesis of compound 95.

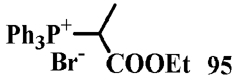

In a 500 mL round-bottomed flask equipped with a magnetic stir bar was added triphenylphosphine (100 g, 381 mmol, 1.0 eq.) and ethyl 2-bromopropionate (100 mL, 762 mmol, 2.0 eq.). The mixture was then heated to 50 °C under N₂ atmosphere overnight. After the white solid (PPh₃) was dissolved, a large amount of white solid was generated. Trituration with petroleum ether/EtOAc and filtration gave compound **95** as a white solid (135 g, 80% yield). MS ESI m/z calcd for C₂₃H₂₄O₂P [M-Br]⁺ 363.15, found 363.13.

### Example 35. Synthesis of compound 96.

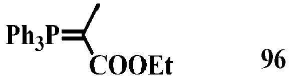

A solution of compound **95** (135.42 g, 305.7 mmol) in dichloromethane (500 mL) was added slowly into 10% NaOH solution (450 mL) with vigorous stirring. The organic solution rapidly turned bright yellow. After 30 minutes, TLC analysis showed that the reaction was completed. Layers were separated and the aqueous layer was further extracted with CH₂Cl₂ (2 × 200 mL). Combined organic layers were washed with brine, dried over anhydrous Na₂SO₄ and concentrated to give a yellow solid **96** (104 g, 94% yield). MS ESI m/z calcd for C₂₃H₂₄O₂P [M+H]⁺ 362.14, found 363.13. The crude product was used directly in the next step.

### Example 36. Synthesis of compound 98.

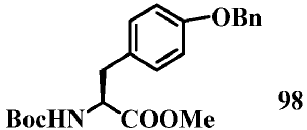

To a mixture of Boc-L-Tyr-OMe (670 g, 2.27mol, 1.0 eq.), K₂CO₃ (358 g, 2.5mol, 1.1 eq.) and KI (38 g, 0.227mol, 0.1 eq.) in acetone (3L) was added benzyl bromide (283 mL, 2.38mol, 1.05 eq.) slowly. The mixture was then refluxed overnight. Water (6L) was added and the reaction mixture was extracted with EtOAc (5 × 100 L). The combined organic layers were washed with brine (2L), dried over anhydrous Na₂SO₄, filtered, concentrated and purified by SiO₂ column chromatography (4:1 hexanes/EtOAc) to give a white solid **98** (795 g, 91% yield). ¹H NMR (500 MHz, CDCl₃) δ 7.43 (d, *J* = 7.0 Hz, 2H), 7.38 (t, *J* = 7.4 Hz, 2H), 7.32 (t, *J* = 7.2 Hz, 1H), 7.04 (d, *J* = 8.5 Hz, 2H), 6.91 (d, *J* = 8.6 Hz, 2H), 5.04 (s, 2H), 4.55 (d, *J* = 6.9 Hz, 1H), 3.71 (s, 3H), 3.03 (qd, *J* = 14.0, 5.8 Hz, 2H), 1.43 (s, 9H). ESI: m/z: calcd for C₂₂H₂₈NO₅ [M+H]⁺: 386.19, found 386.19.

### Example 37. Synthesis of compound 99.

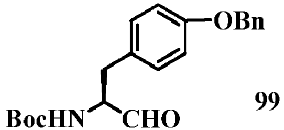

To a solution of ester **98** (380 g, 987 mmol, 1.0 eq.) in anhydrous dichloromethane (1L) at -78°C was added DIBAL (1.0 M in hexanes, 2.9 L, 2.9 eq.) over 3 h. After the addition was completed, the mixture was quenched with 3 L of ethanol. IN HCl was added dropwise until pH 4 was reached. The resulting mixture was allowed to warm to 0 °C. Layers were separated and the aqueous layer was further extracted with EtOAc (3 × 3 L). The combined organic solution was washed with brine, dried over anhydrous Na₂SO₄, and concentrated. Trituration with PE/EtOAc and filtration gave a white solid **99** (263 g, 75% yield). ¹H NMR (500 MHz, CDCl₃) δ 9.65 (s, 1H), 7.45 (d, J = 7.1 Hz, 2H), 7.41 (t, J = 7.4 Hz, 2H), 7.35 (t, J = 7.1 Hz, 1H), 7.11 (d, J = 8.6 Hz, 2H), 6.95 (d, J = 8.6 Hz, 2H), 5.07 (s, 2H), 4.42 (dd, J = 12.4, 6.1 Hz, 1H), 3.09 (d, J = 6.2 Hz, 2H), 1.46 (s, 9H). ESI: m/z: calcd for C₂₁H₂₆NO₄ [M+H]⁺: 356.18, found 356.19.

### Example 38. Synthesis of compound 100.

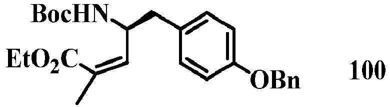

To a solution of aldehyde **99** (81.4 g, 229 mmol, 1.0 eq.) in anhydrous dichloromethane (800 mL) at room temperature was added ylide **96** (2.0 eq.) in anhydrous dichloromethane (800 mL) over 30 min. The mixture was stirred at room temperature overnight then concentrated and purified by SiO₂ column chromatography (6:1 petroleum ether/ EtOAc) to give a white solid **100** (63.4 g, 63% yield). ¹H NMR (500 MHz, CDCl₃) δ 7.45 - 7.41 (m, 2H), 7.40 - 7.35 (m, 2H), 7.33 (d, *J* = 7.2 Hz, 1H), 7.10 - 7.06 (m, 2H), 6.92 - 6.88 (m, 2H), 6.50 (dd, *J* = 8.8, 1.3 Hz, 1H), 5.04 (s, 2H), 4.57 (s, 2H), 4.18 (q, *J* = 7.1 Hz, 2H), 2.86 (d, *J* = 8.5 Hz, 1H), 2.72 (dd, *J* = 13.6, 6.8 Hz, 1H), 1.71 (d, *J* = 1.4 Hz, 3H), 1.41 (d, *J* = 2.2 Hz, 9H), 1.28 (td, *J* = 7.5,5.1 Hz, 4H). MS ESI m/z calcd for C₂₆H₃₃NaNO₅ [M+Na]⁺ 462.24, found 462.22.

### Example 39. Synthesis of compound 101.

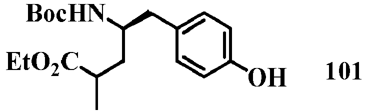

In a hydrogenation bottle, Pd/C (1.83 g, 10 wt%, 50% water) was added to a solution of compound **100** (30.2 g, 68.9 mmol) in THF (100 mL) and methanol (300 mL). The mixture was shaken under 1 atm H₂ overnight, filtered through Celite (filter aid), and the filtrate was concentrated to afford compound **101** (25.0 g, theoretical yield) as a colorless oil. ¹H NMR (500 MHz, CDCl₃) δ 6.99 (d, *J* = 7.0 Hz, 2H), 6.72 (d, *J* = 7.6 Hz, 2H), 4.39 (s, 1H), 4.18 - 4.04 (m, 2H), 3.82 (s, 1H), 2.60 (dd, *J* = 37.2, 20.9 Hz, 4H), 1.95 - 1.81 (m, 1H), 1.39 (s, 11H), 1.24 (dd, *J* = 9.5, 4.3 Hz, 3H), 1.13 (t, *J* = 8.9 Hz, 3H). MS ESI m/z calcd for C₁₉H₃₁NO₅ [M+H]⁺ 352.20, found 352.19.

### Example 40. Synthesis of compound 102.

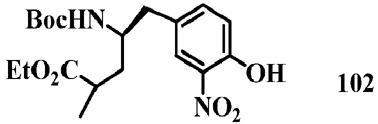

To a solution of compound **101** (5.96 g, 35.9 mmol, 1.0 eq.) in anhydrous dichloromethane (200 mL)was added Ac₂O (3.2 mL, 33.9 mmol, 2.0 eq.) and HNO₃ (65%-68%, 3.5 mL, 50.79 mmol, 3.0 eq.) at room temperature. The mixture was stirred at room temperature for 30min, and TLC analysis showed that the reaction was completed. The reaction solution was washed with water (3 × 200 mL), and the aqueous layer was back-extracted with dichloromethane (3 × 100 mL). The combined dichloromethane solution was washed with brine, dried over anhydrous Na₂SO₄, filtered, concentrated and purified by SiO₂ column chromatography (5:1 hexanes/ EtOAc) to give compound **102** as a yellow solid (4.18 g, 72% yield).¹H NMR (500 MHz, CDCl₃) δ 10.49 (s, 1H), 7.89 (s, 1H), 7.44 (d, *J* = 8.4 Hz, 1H), 7.09 (d, *J* = 8.6 Hz, 1H), 4.32 (d, *J* = 8.3 Hz, 1H), 4.12 (dd, *J* = 14.0, 7.0 Hz, 2H), 3.80 (s, 1H), 2.76 (dd, *J* = 13.0, 6.8 Hz, 2H), 2.59 (s, 1H), 1.88 (s, 1H), 1.37 (t, *J* = 8.7 Hz, 9H), 1.25 (dd, *J* = 13.5, 6.9 Hz, 4H), 1.16 (t, *J* = 8.0 Hz, 3H). MS ESI m/z calcd for C₁₉H₂₈NaN₂O₇ [M+Na]⁺ 419.19, found 419.17.

### Example 41. Synthesis of compound 103.

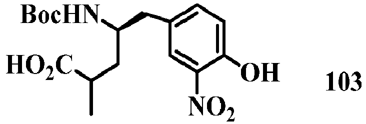

To a solution of ester **102** (15.3 g, 38.6 mmol, 1.0 eq.) in THF (100 mL) and methanol (100 mL) was added LiOH·H₂O (16.3 g, 389 mmol, 10.0 eq.) in water (190 mL) at room temperature. The mixture was stirred at room temperature for 40 min. and then diluted with water (400 mL) and IN KHSO₄ was added dropwise until pH 3-4 was reached. After extraction with EtOAc (3 × 300 mL), the organic phase was washed with brine, dried over anhydrous Na₂SO₄, filtered, concentrated to give **103** as a yellow solid (14.4 g, theoretical yield). ¹H NMR (500 MHz, CDCl₃) δ 10.48 (s, 1H), 7.98 - 7.88 (m, 1H), 7.42 (dd, *J* = 18.4, 8.2 Hz, 1H), 7.14 - 7.03 (m, 1H), 4.48 (d, *J* = 8.6 Hz, 1H), 3.90 (s, 1H), 2.82 - 2.53 (m, 3H), 1.97 - 1.82 (m, 2H), 1.42 - 1.27 (m, 10H), 1.21 (d, *J* = 6.7 Hz, 4H). MS ESI m/z calcd for C₁₇H₂₃N₂O₇ [M-H]⁻ 367.16, found 367.14.

### Example 42. Synthesis of compound 104.

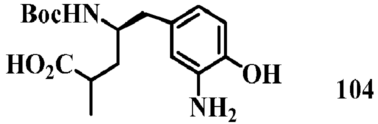

In a hydrogenation bottle, Pd/C (2.60 g, 10 wt%, 50% water) was added to a solution of compound **103** (26.0 g, 70.6 mmol, 1.0 eq.) in methanol (260 mL). The mixture was shaken overnight under 1 atm H₂ then filtered through Celite (filter aid), the filtrate was concentrated to afford compound **104** as a green oil (24.0 g, >100 % yield).

### Example 43. Synthesis of compound 106.

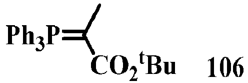

A mixture of *tert*-butyl-2-bromopropanoate (255 g, 1.22 mol, 1.0 eq.) and triphenyl phosphine (320 g, 1.22 mol, 1.0 eq.) in dry acetonitrile (1L) was stirred at room temperature for 18 h. Acetonitrile was removed under reduced pressure and toluene was added to crash out a white precipitate. Toluene was then decanted off and the white solid was dissolved in dichloromethane (1L) and transferred to a separatory funnel. 10% NaOH (1L) was added to the funnel, and the organic layer immediately turned yellow after shaking. The organic layer was separated and the aqueous layer was extracted with dichloromethane (1L) once. The dichloromethane layers were combined and washed with brine (400 mL) once, then dried over Na₂SO₄, filtered and concentrated, giving the ylide **106** as a yellow solid (280g, 58%).

### Example 44. Synthesis of compound 107.

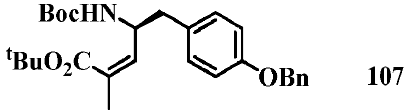

Aldehyde **99** (450 g, 1.27 mol, 1.0 eq.) was dissolved in dry dichloromethane (3L), to which *tert*-butyl ester ylide **106** (546 g, 1.40 mmol, 1.1 eq.) was added and the solution was stirred at r.t. overnight as determined complete by TLC. Purification by column chromatography (10-50% EtOAc/hexanes) afforded compound **107** (444 g, 75% yield) as a white solid. ESI m/z calcd for C₂₈H₃₈NO₅ [M+H]⁺: 468.27, found 468.22.

### Example 45. Synthesis of compound 108.

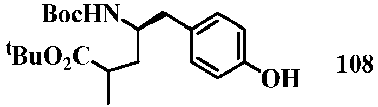

Compound **107** (63 g, 0.13mol) was dissolved in methanol (315 mL) and hydrogenated (1 atm H₂) with Pd/C catalyst (10 wt%, 6.3 g) at r.t. overnight. The catalyst was filtered off and the filtrate were concentrated under reduced pressure to afford compound **108** (45.8 g, 93% yield).

### Example 46. Synthesis of compound 109.

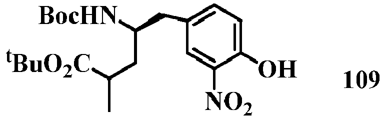

To a solution of compound **108** (390 g, 1.03 mol, 1.0 eq.) in THF (4 L) *tert*-butyl nitrite (1.06 kg, 10.3 mol, 10 eq.) was added at r.t. and the reaction was stirred overnight. After removal of THF, the residue was purified by column chromatography (10-50% EtOAc/hexanes) to afford compound **109** (314 g, 72% yield) as a light yellow solid.

### Example 47. Synthesis of compound 110.

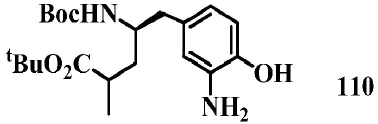

To a solution of **109** (166 g, 0.392 mol, 1.0 eq.) in EtOAc (500 mL) was added Pd/C (10 wt%, 16 g) under nitrogen, and the reaction flask was evacuated and purged with hydrogen for 3 times. The reaction mixture was stirred under hydrogen (1 atm) at r.t. for 16 h and then filtered over Celite and concentrated to afford product **110** (146 g, 97% yield) as a light yellow foam. ¹H NMR (400 MHz, CDCl₃) δ 6.62 (d, J = 7.9 Hz, 1H), 6.55 (s, 1H), 6.43 (d, J = 7.3 Hz, 1H), 4.39 (dd, J = 53.0, 44.2 Hz, 1H), 3.77 (s, 4H), 2.72 - 2.29 (m, 3H), 1.83 - 1.58 (m, 1H), 1.40 (d, J = 7.6 Hz, 18H), 1.24 (s, 1H), 1.06 (t, J = 5.7 Hz, 3H). MS ESI m/z calcd for C₂₁H₃₅N₂O₅ [M+H]⁺ 394.25, found 395.25.

### Example 48. Synthesis of compound 114.

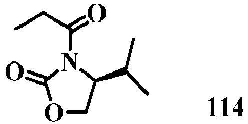

To a solution of (S)-4-isopropyloxazolidin-2-one (5.00 g, 38.7 mmol, 1.0 eq.) in anhydrous THF (200 mL)at -78 °C was added *n*-BuLi (2.5 M in hexanes, 17.0 mL, 1.2 eq.) in 30 min under N₂. The mixture was stirred at -78°C for 1 h, and then propionyl chloride (4.0 mL, 42.58 mmol, 1.1 eq.) was added dropwise. After the mixture was stirred at -78°C for another 1 h, TLC analysis indicated the reaction completed. Saturated ammonium chloride solution (250 mL) was added and extracted with EtOAc (3 × 100 mL). The combined organic layers were washed with 1N NaOH solution (200 mL) and brine (300 mL), dried over anhydrous Na₂SO₄, filtered, concentrated and purified by column chromatography (7:1 hexanes/ EtOAc) to give compound **114** as a colourless oil (6.36 g, 89% yield).MS ESI m/z calcd for C₉H₁₆NO₃ [M+H]⁺ 186.10, found 186.10. ¹H NMR (400 MHz, CDCl₃) δ 4.48 - 4.39 (m, 1H), 4.27 (t, J = 8.7 Hz, 1H), 4.21 (dd, J = 9.1, 3.1 Hz, 1H), 3.06 - 2.82 (m, 2H), 2.38 (dtd, J = 14.0, 7.0, 4.0 Hz, 1H), 1.17 (t, J = 7.4 Hz, 3H), 0.90 (dd, J = 17.0, 7.0 Hz, 6H).

### Example 49. Synthesis of compound 115.

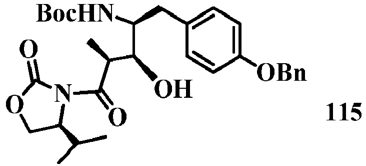

To a solution of (S)-4-isopropyl-3-propionyloxazolidin-2- one (2.00 g, 11.9 mmol, 1.1 eq.) in anhydrous dichloromethane (20 mL)at 0°C was added DIPEA (2.3 mL, 12.9 mmol, 1.2 eq.) and *n*-Bu₂BOTf (1.0 M in dichloromethane, 12.0 mL, 1.1 eq.) under N₂.The mixture was stirred at 0 °C for 45 min, then cooled to -78°C, to which a solution of compound **99** (4.24 mL, 10.8 mmol, 1.0 eq.) in dichloromethane was added dropwise. The mixture was stirred at -78 °C for 1 h and then warmed slowly to room temperature. The mixture was stirred at room temperature overnight, and PBS (0.1M, pH 7.0, 100 mL) was added. After phase separation, the aqueous phase was further extracted with dichloromethane (3 × 50 mL). The combined organic layers were washed with brine (200 mL), dried over anhydrous Na₂SO₄, filtered and concentrated. The crude product was re-dissolved in methanol (100 mL) and treated with H₂O₂ (30% aqueous solution, 26 mL, 23 eq.) at 0 °C for 3 h. The methanol was removed by rotary evaporation and water (100 mL) was added. The resulting mixture was extracted with EtOAc (3 × 100 mL). The combined organic layers were washed with brine (300 mL), dried over anhydrous Na₂SO₄, filtered, concentrated and purified by SiO₂ column chromatography (3:1 hexanes/EtOAc) to give compound **115** as a foamy solid(2.70 g, 49% yield). ¹H NMR (400 MHz, CDCl₃) δ 7.52 - 7.26 (m, 5H), 7.15 (d, *J* = 7.4 Hz, 2H), 6.93 (d, *J* = 7.3 Hz, 2H), 5.05 (s, 2H), 4.69 (d, *J* = 7.0 Hz, 1H), 4.47 (s, 1H), 4.36 (t, *J* = 7.8 Hz, 1H), 4.17 (d, *J* = 8.5 Hz, 1H), 3.93 (d, *J* = 7.1 Hz, 1H), 3.85 (s, 2H), 2.84 (d, *J* = 6.9 Hz, 2H), 2.31 (s, 1H), 1.40 - 1.37 (m, 9H), 1.31 (s, 3H), 0.92 (dd, *J* = 13.4, 6.6 Hz,6H). MS ESI m/z calcd for C₃₀H₄₁N₂O₇ [M+H]⁺ 541.28, found 541.30.

### Example 50. Synthesis of compound 116.

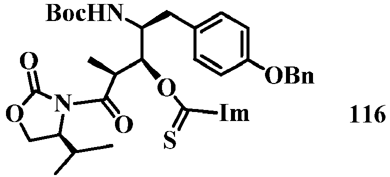

A mixture of compound **115** (2.50 g, 4.63 mmol, 1.0 eq.) and 1,1'-thiocarbonyldiimidazole (2.48 g, 13.89 mmol, 3.0 eq.) in anhydrous THF (46 mL) was refluxed overnight. Water (100 mL) was added and the resulting mixture was extracted with EtOAc (3 × 50 mL). The combined organic layers were washed with brine (200 mL), dried over anhydrous Na₂SO₄, filtered, concentrated and purified by SiO₂ column chromatography (3:1 hexanes/EtOAc) to give compound **116** as a yellow foam (2.33 g, 77% yield). ¹H NMR (400 MHz, CDCl₃) δ 8.41 (s, 1H), 7.67 (s, 1H), 7.36 (dt, *J* = 16.0, 6.9 Hz, 6H), 7.09 (s, 1H), 7.05 (d, *J* = 8.4 Hz, 2H), 6.86 (d, *J* = 8.4 Hz, 2H), 6.32 (d, *J* = 9.5 Hz, 1H), 5.01 (s, 2H), 4.56 - 4.43 (m, 2H), 4.32 (ddd, *J* = 16.2, 15.6, 7.8 Hz, 3H), 4.19 (d, *J* = 8.7 Hz, 1H), 2.96 (dd, *J* = 14.6, 4.4 Hz, 1H), 2.49 (dd, *J* = 14.5, 10.5 Hz, 1H), 2.29 (td, *J* = 13.4, 6.7 Hz, 1H), 1.31 (s, 3H), 1.29 (s, 9H), 0.91 (dd, *J* = 13.9, 6.9 Hz, 6H). MS ESI m/z calcd for C₃₄H₄₃N₄O₇S[M+H]⁺ 651.27, found 651.39.

### Example 51. Synthesis of compound 117.

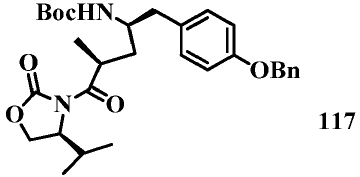

To a solution of compound **116** (1.90 g, 2.92 mmol, 1.0 eq.) in anhydrous toluene (30 mL)was added *n*-Bu₃SnH (1.6 mL, 5.84 mmol, 2.0 eq.) and azodiisobutyronitrile (0.05 g, 0.584 mmol, 0.1 eq.) in sequence. The mixture was refluxed for 2.5 h and then cconcentrated and purified by SiO₂ column chromatography (5:1 hexanes/EtOAc) to give compound **117** as a white foam (1.21 g, 79% yield). ¹H NMR (400 MHz, CDCl₃) δ 7.36 (ddd, *J* = 24.5, 14.5, 7.1 Hz, 5H), 7.08 (d, *J* = 8.5 Hz, 2H), 6.90 (d, *J* = 8.5 Hz, 2H), 5.04 (d, *J* = 5.1 Hz, 2H), 4.48 (d, *J* = 4.2 Hz, 1H), 4.33 (t, *J* = 8.4 Hz, 1H), 4.22 (d, *J* = 9.7 Hz, 1H), 4.15 (d, *J* = 8.8 Hz, 1H), 3.81 (s, 2H), 2.73 (dd, *J* = 14.1, 5.9 Hz, 1H), 2.61 (dd, *J* = 14.0, 7.2 Hz, 1H), 2.29 (dq, *J* = 13.5, 6.8 Hz, 1H), 2.11 - 2.00 (m, 1H), 1.35 (s, 9H), 1.20 (d, *J* = 6.9 Hz, 3H), 0.89 (dd, *J* = 14.0, 6.9 Hz, 6H). MS ESI m/z calcd for C₃₀H₄₁N₂O₆ [M+H]⁺ 525.28, found 525.37.

### Example 52. Synthesis of compound 118.

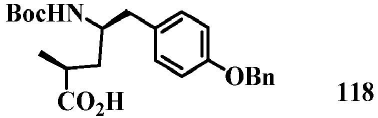

To a solution of compound **117** (1.20 g, 2.29 mmol, 1.0 eq) in THF (30 mL) were added LiOH (0.192 g, 4.58 mmol, 2.0 eq.) in water (6 mL) and H₂O₂ (30% aqueous solution, 1.4 mL, 6.0 eq.). After 3 h of stirring at 0 °C, sodium bisulfite solution (1.5 M, 30 mL) was added to quench the reaction. After 30 min, 1 N KHSO₄ was added dropwise until pH 4 was reached. The reaction mixture was then extracted with EtOAc (3 × 50 mL). The EtOAc solution was washed with brine, dried over anhydrous Na₂SO₄, filtered, concentrated and purified by SiO₂ column chromatography (3:1 hexanes/EtOAc, containing 1% HOAc) to give compound **118** as a white solid (0.78 g, 82% yield). ¹H NMR (400 MHz, CDCl₃) δ 7.46 - 7.28 (m, 5H), 7.07 (d, *J* = 7.7 Hz, 2H), 6.91 (d, *J* = 7.8 Hz, 2H), 4.52 (d, *J* = 8.5 Hz, 1H), 3.87 (d, *J* = 41.8 Hz, 1H), 2.82 - 2.43 (m, 3H), 1.85 (t, *J* = 12.2 Hz, 1H), 1.41 (s, 9H), 1.17 (d, *J* = 6.9 Hz, 3H). MS ESI m/z calcd for C₂₄H₃₂NO₅ [M+H]⁺ 414.22, found 414.21.

### Example 53. Synthesis of compound 119.

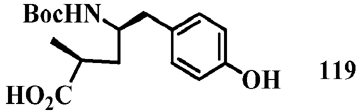

A mixture of compound **118** (0.77 g, 1.86 mmol, 1.0 eq.) and Pd/C (10%, 0.25 g) in methanol (15 mL) was hydrogenated under 1 atm H₂ pressure for 16 h and then filtered through Celite (filter aid). The filtrate was concentrated to afford compound **119** as a white solid (0.58 g, 96% yield). ¹H NMR (400 MHz, CDCl₃) δ 7.00 (d, *J* = 7.5 Hz, 2H), 6.80 (s, 2H), 4.51 (d, *J* = 9.0 Hz, 1H), 3.88 (s, 1H), 2.66 (dd, *J=* 65.6, 22.6 Hz, 4H), 1.88 (t, *J=* 12.2 Hz, 1H), 1.42 (s, 9H), 1.14 (d, *J=* 6.6 Hz, 3H). MS ESI m/z calcd for C₁₇H₂₆NO₅ [M+H]⁺: 324.17, found 324.16.

### Example 54. Synthesis of compound 120.

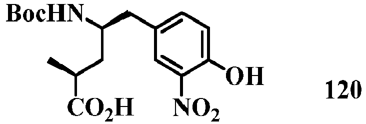

To a solution of compound **119** (0.57 g, 1.76 mmol, 1.0 eq.) in THF (10 mL)was added t-BuONO (0.63 mL, 5.28 mmol, 3.0 eq.) at 0°C. The reaction was stirred at 0°C for 1 hr then room temperature 1 h. After water (50 mL) was added, the reaction mixture was extracted with EtOAc (3 × 30 mL). The combined organic layers were washed with brine (100 mL), dried over anhydrous Na₂SO₄, filtered, concentrated and purified by SiO₂ column chromatography (2:1 hexanes/EtOAc, containing 1% HOAc) to give compound **120** as a yellow solid (0.50 g, 77% yield). ¹H NMR (400 MHz, DMSO) δ7.92 (s, 1H), 7.47 (d, *J* = 8.3 Hz, 1H), 7.05 (d, *J* = 8.5 Hz, 1H), 3.73 (s, 1H), 2.78 (dd, *J* = 13.6, 5.3 Hz, 1H), 2.69 - 2.47 (m, 2H), 1.87 (t, *J* = 11.9 Hz, 1H), 1.47 - 1.37 (m, 1H), 1.32 (s, 9H), 1.17 (d, *J=* 7.2 Hz, 3H). MS ESI m/z calcd for C₁₇H₂₅N₂O₇ [M+H]⁺ 369.15, found 369.14.

### Example 55. Synthesis of compound 121.

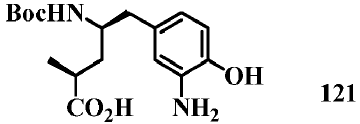

A mixture of compound **120** (0.50 g, 1.36 mmol, 1.0 eq.) and Pd/C (10 wt%, 0.02 g) in methanol (10 mL) was hydrogenated (1 atm H₂) at r.t. for 1 h, and then filtered through Celite (filter aid). The filtrate was concentrated to afford compound **121** as white foam (0.43 g, 93% yield). MS ESI m/z calcd for C₁₇H₂₇N₂O₅ [M+H]+ 339.18, found 339.17. ¹H NMR (400 MHz, MeOD) δ 6.60 (d, *J* = 7.9 Hz, 2H), 6.44 (d, *J* = 7.3 Hz, 1H), 3.71 (d, *J* = 6.3 Hz, 1H), 2.62 - 2.37 (m, 3H), 1.83 (ddd, *J* = 13.7, 9.9, 3.7 Hz, 1H), 1.39 (s, 9H), 1.13 (d, *J* = 7.1 Hz, 3H).

### Example 56. Synthesis of compound 124.

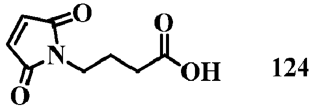

To a solution of maleic anhydride (268 g, 2.73mol) in acetic acid (1L) was added 4-aminobutanoic acid (285 g, 2.76 mol). After stirring at r.t. for 30 min, the reaction was refluxed for 1.5 h, cooled to r.t. and evaporated under vacuum to give a residue, which was taken up in EA, washed with water and brine, and dried over anhydrous Na₂SO₄, filtered and concentrated. The crude product was crystallized from EtOAc and PE to give a white solid (400 g, 80 % yield). ¹H NMR (500 MHz, CDCl₃) δ 6.71 (s, 2H), 3.60 (t, J = 6.7 Hz, 2H), 2.38 (t, J = 7.3 Hz, 2H), 2.00 - 1.84 (m, 2H).

### Example 57. Synthesis of compound 125.

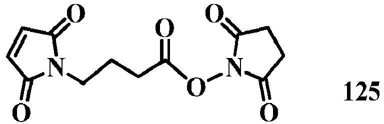

Compound **124** (400 g, 2.18 mol, 1.0 eq.) was dissolved in CH₂Cl₂ (1.5 L), to which N-hydroxysuccinimide (276 g, 2.40 mmol, 1.1 eq.) and DIC (303 g, 2.40 mol, 1.1 eq.) were added at r.t. and stirred overnight. The reaction was concentrated and purified by column chromatography (1:2 petroleum ether/ EtOAc) to give NHS ester **125** as a white solid (382 g, 63% yield). ¹H NMR (500 MHz, CDCl₃) δ 6.74 (s, 2H), 3.67 (t, *J=* 6.8 Hz, 2H), 2.85 (s, 4H), 2.68 (t, *J* = 7.5 Hz, 2H), 2.13 - 2.03 (m, 2H).

### Example 58. Synthesis of compound 126.

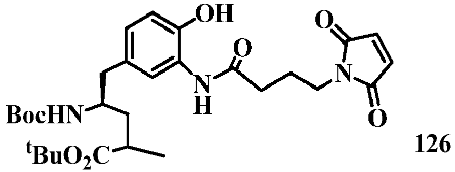

To a solution of **124** (60 g, 328 mmol, 1.3eq.) in THF (600 mL) was added NMM (85.3 mL, 984 mmol, 3.0 eq.) at 0 °C with stirring, followed by isobutyl chloroformate (44.6 mL, 426 mmol, 1.3 eq.) dropwise. After stirring at 0 °C for 2 h, the resulting mixture was added dropwise to a solution of **104** (102 g, 259 mmol, 1.0 eq.) in THF (400 mL) while keeping the temperature at 0 °C. After the addition was completed, the reaction was stirred for additional 30 min. and then quenched with water (300 mL), extracted with EtOAc (3 × 300 mL). The combined organic layers were dried, filtered, concentrated and purified by column chromatography with a gradient of 9-35% EtOAc/PE to afford compound **126** (104 g, 73% yield) as a light yellow solid. ¹H NMR (400 MHz, CDCl₃) δ 8.86 (s, 1H), 8.40 (d, J = 17.3 Hz, 1H), 6.87 (s, 3H), 6.70 (s, 2H), 4.53 - 4.16 (m, 0H), 3.79 (s, 1H), 3.62 (t, J = 6.1 Hz, 1H), 2.63 (s, 1H), 2.40 (t, J = 6.9 Hz, 1H), 2.12 - 1.88 (m, 4H), 1.84 - 1.64 (m, 1H), 1.38 (t, J = 9.6 Hz, 6H), 1.06 (t, J = 6.0 Hz, 3H).

### Example 59. Synthesis of compound 127.

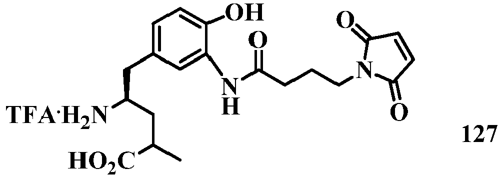

Compound **126** (12.7 g, 22.7mmol) dissolved in CH₂Cl₂ (20 mL) was treated with TFA (40 mL) at 0 °C and the reaction was warmed to r.t. and stirred for 3h. The mixture was concentrated and co-evaporated with toluene three times. The residue was triturated with diethyl ether and a light yellow solid **127** was collected (11.4 g, theoretical yield).

### Example 60. Synthesis of compound 128.

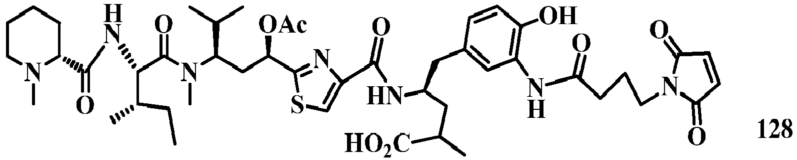

To a solution of carboxylic acid **33** (40 mg, 0.074 mmol, 1.0 eq.) in EtOAc was added pentafluorophenol (27 mg, 0.148mmol, 2.0 eq.) and DCC (23 mg, 0.111mmol, 1.5 eq.). The reaction mixture was stirred at r.t. for 16 h and then filtered over a Celite pad, with washing of the pad with EtOAc. The filtrate was concentrated and re-dissolved in DMA (6 mL), then compound **127** (56.6 mg, 0.13 mmol) and DIPEA (47.4 µL, 0.18mmol) were added. The reaction mixture was stirred at r.t. for 24 h and then concentrated and purified by reverse phase HPLC (C₁₈ column, 10-100% acetonitrile/water) to afford compound **128** (43 mg, 63% yield) as a white solid. MS ESI m/z calcd for C₄₆H₆₆N₇O₁₁S [M+H]⁺ 924.45, found 924.45.

### Example 61. Synthesis of compound 132.

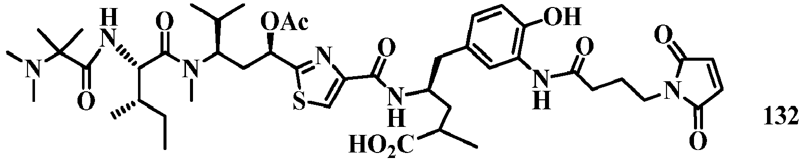

To a solution of compound **41a** (11 g, 15.9 mmol, 1.0 eq.) and compound **127** (12.3 g, 23.8 mmol, 1.5 eq.) in DMF (100 mL) was added DIPEA (6.9 mL, 39.7 mmol, 2.5 eq.) at 0 °C. The reaction mixture was warmed to r.t. and stirred for 1h. The mixture was concentrated under vacuum and purified on silica gel column (100% DCM to 10% MeOH/DCM) to give compound **132** (10 g, 69% yield) as an amorphous solid. MS ESI m/z calcd for C₄₅H₆₅N₇O₁₁S [M+H]⁺ 912.45, found 912.45.

### Example 62. Synthesis of compound 204.

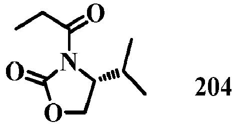

To a solution of (R)-4-isopropyloxazolidin-2-one **(203)** (25.0g, 0.194mol, 1.0eq) in anhydrous THF (1150 mL) was added *n*-BuLi (85.0 mL, 0.213mol, 1.1eq) at -78 °C under N₂ and the mixture was stirred at the same temperature for 1 h, a large number of white solids formed. Then propionyl chloride (20.0 mL, 0.232mol, 1.2eq) was added at -78°C and the mixture was stirred at the same temperature for 1 h. After the consumption of (S)-4-isopropyloxazolidin-2-one monitored by TLC, the solution was poured into saturated ammonium chloride solution (1.2 L) and the mixture was extracted with EA (700 mL, 350 mL × 2). The organic extract was washed with 1.0 N NaOH solution (1.0 L) and brine (1.0 L), dried over anhydrous sodium sulfate, filtered, concentrated in vacuo and purified by SiO₂ column chromatography (PE :EA = 10:1) to give the title compound as a colorless oil (32.6 g, 90.8%). ESI m/z: calcd for C₉H₁₇NO₃ [M+H]⁺: 186.1, found 186.1. ¹H NMR (400 MHz, CDCl₃) δ 4.48 - 4.37 (m, 1H), 4.27 (t, *J=* 8.7 Hz, 1H), 4.21 (dd, *J* = 9.1, 3.1 Hz, 1H), 3.04 - 2.82 (m, 2H), 2.45 - 2.30 (m, 1H), 1.17 (t, *J=* 7.4 Hz, 3H), 0.90 (dd, *J* = 17.1, 7.0 Hz, 6H).

### Example 63. Synthesis of compound 205.

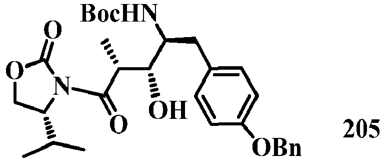

To a solution of (R)-4-isopropyl-3-propionyloxazolidin-2-one (18.4 g, 99.5 mmol, 1.1 eq) in anhydrous DCM (200 mL) were added Bu₂BOTf (1 M dichloromethane solution, 100 mL, 100 mmol, 1.1eq) and DIPEA(19 mL, 108.6mmol, 1.2eq) at 0 °C under N₂, and the mixture was stirred at the same temperature for 45 min. A solution of aldehyde **99**(32.2 g, 90.5 mmol, 1.0 eq) in dichloromethane (320 mL) was added at -78°C and stirred at the same temperature for 1 h, then the solution was allowed to slowly warm to room temperature for 15 hours. The mixture was poured into 700 mL of potassium phosphate buffer (pH 7.0) and extracted with ethyl acetate. The organic extract was washed with brine, dried over anhydrous sodium sulfate, filtered, and then concentrated in vacuo. The residue was dissolved in methanol (730 mL) and cooled to 0°C, then 30% H₂O₂ aqueous solution (225 mL) was added slowly, and the mixture was stirred at the same temperature for 3 hours. After addition of water (750 mL), the mixture was concentrated in vacuo to remove methanol. The resulting aqueous solution was extracted with ethyl acetate (500 mL, 150 mL × 2), and the organic extract was washed with 5% sodium hydrogen carbonate solution and brine, dried over anhydrous sodium sulfate, filtered, concentrated in vacuo and purified by SiO₂ column chromatography (PE :EA = 3:1) to give the title compound as a white foam (31.7 g, 64.8%). ESI m/z: calcd for C₃₀H₄₁N₂O₇ [M+H]⁺: 541.3, found 541.3. ¹H NMR (400 MHz, CDCl₃) δ 7.49 - 7.29 (m, 5H), 7.17 (t, *J* = 10.7 Hz, 2H), 6.93 (d, *J* = 7.0 Hz, 2H), 5.06 (s, 2H), 4.28 (dd, *J* = 44.4, 36.4 Hz, 3H), 4.04 - 3.52 (m, 3H), 3.11 - 2.73 (m, 2H), 2.35 (s, 1H), 1.41 (t, *J* = 16.3 Hz, 9H), 0.91(dd, *J*=15.6, 6.4Hz, 5H).

### Example 64. Synthesis of compound 206.

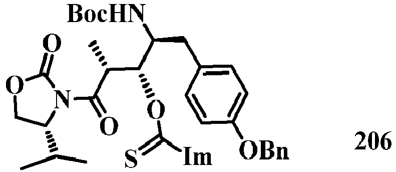

To a solution of compound **205** (28.3 g, 52.3 mmol, 1.0 eq) in anhydrous THF (350 mL) was added 1,1-thiocarbonyl diimidazole (TCDI) (35.1 g, 157.0 mmol, 3.0 eq), and the mixture was heated under reflux overnight. After the consumption of starting material monitored by TLC, the mixture was concentrated in vacuo and purified by SiO₂ column chromatography (PE :EA = 3:1) to give the title compound as a pale yellow foam (26.1 g, 76.8 %). ESI m/z: calcd for C₃₄H₄₃N₄O₇S [M+H]⁺: 651.3, found 651.3. ¹H NMR (400 MHz, CDCl₃) δ 8.21 (s, 1H), 7.43 (d, *J* = 11.8 Hz, 1H), 7.42 - 7.28 (m, 5H), 7.06 (d, *J* = 8.3 Hz, 2H), 7.01 (s, 1H), 6.80 (d, *J* = 8.3 Hz, 2H), 6.17 (dd, *J* = 8.5, 2.9 Hz, 1H), 4.96 (s, 2H), 4.42 - 4.04 (m, 5H), 2.83 (dd, *J* = 14.2, 6.2 Hz, 1H), 2.69 (dd, *J* = 14.2, 7.1 Hz, 1H), 2.32 (dd, *J* = 6.8, 4.2 Hz, 1H), 1.37 (s, 9H), 1.30 (d, *J* = 6.9 Hz, 3H), 0.87 (dd, *J* = 9.9, 7.0 Hz, 6H).

### Example 65. Synthesis of compound 207.

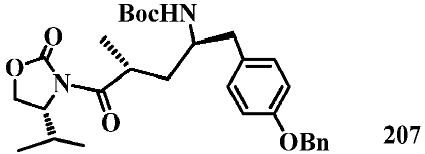

To a solution of compound **206** (26.0 g, 40.0 mmol, 1.0 eq) in anhydrous toluene (350 mL) was added *n*-Bu₃SnH (21.5 mL, 80.0 mmol, 2.0 eq) and 2,2'-azobis(2-methylpropionitrile) (AIBN) (0.066 g, 0.01 eq) under N₂, and the mixture was heated under reflux for 1 hour. After the consumption of starting material monitored by TLC, the mixture was concentrated in vacuo and purified by SiO₂ column chromatography (PE :EA = 5:1) to give the title compound as a white foam (6.0 g, 37.3 %). ESI m/z: calcd for C₃₀H₄₁N₂O₆ [M+H]⁺: 525.3, found 525.3. ¹H NMR (400 MHz, CDCl₃) δ 7.37 (ddd, *J* = 25.1, 15.1, 7.1 Hz, 5H), 7.08 (d, *J* = 7.9 Hz, 2H), 6.89 (d, *J* = 8.4 Hz, 2H), 5.03 (s, 2H), 4.61 (d, *J* = 8.4 Hz, 1H), 4.40 (s, 1H), 4.32 - 4.08 (m, 2H), 3.91 - 3.66 (m, 2H), 2.83 (d, *J* = 8.4 Hz, 1H), 2.60 (t, *J* = 10.1 Hz, 1H), 2.33 (s, 1H), 1.71 (s, 1H), 1.41 (s, 9H), 1.15 (d, *J* = 6.5 Hz, 3H), 0.87 (dd, *J* = 17.0, 7.0 Hz, 6H).

### Example 66. Synthesis of compound 208.

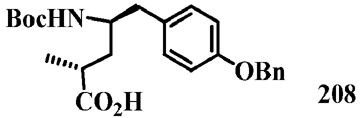

To a solution of compound **207** (7.84 g, 15.0 mmol, 1.0 eq) in THF (90 mL) and water(30 mL) was added LiOH·H₂O (1.57 g, 37.5mmol, 2.5eq) in 30% H₂O₂ aqueous solution (11.4 mL, 112.5 mmol, 7.5eq) at 0 °C, and the mixture was stirred at the same temperature for 3 hours. After addition of 1.5M Na₂SO₃ solution (160 mL) at 0 °C, the mixture was stirred at the same temperature for 30 min. then IN KHSO₄ was added slowly until pH 4. The resulting aqueous solution was extracted with EA (200 mL, 75 mL × 2), and the organic extract was washed with brine, dried over anhydrous sodium sulfate, filtered, concentrated in vacuo and purified by SiO₂ column chromatography (PE :EA = 2:1) to give the title compound as a white solid (6.18 g, 100%). ESI m/z: calcd for C₂₄H₃₂N₁O₅ [M+H]⁺: 414.2, found 414.2. ¹H NMR (400 MHz, CDCl₃) δ 7.39 (ddd, *J* = 24.5, 15.0, 7.2 Hz, 5H), 7.11 (d, *J* = 7.8 Hz, 2H), 6.93 (d, *J* = 8.3 Hz, 2H), 5.06 (s, 2H), 4.44 (t, *J* = 8.3 Hz, 1H), 3.83 (d, *J* = 69.4 Hz, 1H), 2.85 - 2.61 (m, 2H), 2.61 - 2.40 (m, 1H), 1.99 - 1.70 (m, 1H), 1.39 (d, *J* = 26.1 Hz, 9H), 1.19 (s, 3H).

### Example 67. Synthesis of compound 209.

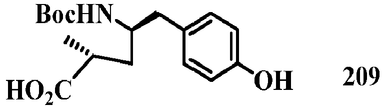

To a solution of compound **208** (6.18 g, 15.0 mmol, 1.0 eq) in MeOH (50 mL) was added Pd/C (0.6 g, 10% Pd/C) in a hydrogenation bottle. The mixture was shaken under 1 atm hydrogen atmosphere overnight, then filtered. The filtrate was concentrated to give the title compound as colourless oil (4.8 g, 99% yield). ESI m/z: calcd for C₁₇H₂₆N₁O₅ [M+H]⁺: 324.2, found 324.2. ¹H NMR (400 MHz, CDCl₃) δ 6.97 (d, *J* = 6.5 Hz, 2H), 6.74 (d, *J* = 8.2 Hz, 2H), 3.93 - 3.66 (m, 1H), 2.58 (tdd, *J* = 19.5, 12.9, 7.4 Hz, 3H), 1.75 (ddd, *J* = 20.1, 16.3, 7.7 Hz, 1H), 1.37 (d, *J* = 21.5 Hz, 9H), 1.11 (d, *J* = 7.0 Hz, 3H).

### Example 68. Synthesis of compound 210.

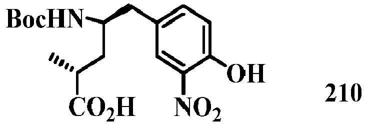

To a solution of compound **209** (4.8 g, 15.0 mmol, 1.0 eq) in anhydrous THF (75 mL) was added slowly *t*-BuONO (18.0 mL, 150 mmol, 10.0 eq) at 0 °C under N₂, and the mixture was stirred at the same temperature for 3 hours. After the consumption of starting material monitored by TLC, IN KHSO₄ was added slowly to the mixture until pH 4. The resulting aqueous solution was extracted with EA (150 mL, 75 mL × 2), and the organic extract was washed with brine, dried over anhydrous sodium sulfate, filtered, concentrated in vacuo and the residue was purified by SiO₂ column chromatography (PE :EA = 3:1) to give the title compound as a yellow solid (3.6 g, 65.4%). ESI m/z: calcd for C₁₇H₂₅N₂O₇ [M+H]⁺: 369.2, found 369.2. ¹H NMR (400 MHz, MeOD) δ 7.93 (d, *J* = 2.0 Hz, 1H), 7.48 (dd, *J* = 8.6, 2.1 Hz, 1H), 7.06 (d, *J* = 8.5 Hz, 1H), 3.83 - 3.71 (m, 1H), 2.82 (dd, *J* = 13.6, 5.0 Hz, 1H), 2.66 - 2.41 (m, 2H), 1.84 (ddd, *J* = 14.0, 10.6, 5.6 Hz, 1H), 1.65 - 1.51 (m, 1H), 1.28 (d, *J* = 24.9 Hz, 9H), 1.15 (d, *J* = 7.0 Hz, 3H).

### Example 69. Synthesis of compound 211.

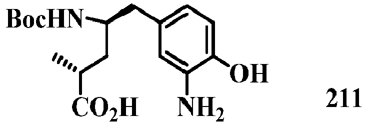

To a solution of compound **210** (3.2 g, 7.74 mmol, 1.0 eq) in MeOH (20 mL) was added Pd/C (0.2 g, 10% Pd/C) in a hydrogenation bottle. The mixture was shaken under 1 atm H₂ atmosphere for 3 h. After consumption of starting material monitored by TLC, the mixture was filtered and the filtrate was concentrated to give the title compound as white foam (2.3 g, 92.0% yield). ESI m/z: calcd for C₁₇H₂₇N₂O₅ [M+H]⁺: 339.2, found 339.2. ¹H NMR (400 MHz, MeOD) δ 6.61 (d, *J* = 8.0 Hz, 2H), 6.45 (d, *J* = 6.3 Hz, 1H), 3.72 (d, *J* = 7.3 Hz, 1H), 2.68 - 2.34 (m, 3H), 1.81 - 1.66 (m, 1H), 1.56 - 1.45 (m, 1H), 1.36 (d, *J* = 29.0 Hz, 9H), 1.08 (d, *J* = 6.9 Hz, 3H).

### Example 70. Synthesis of compound 390.

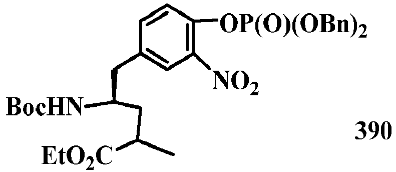

To a solution of compound **102** (1.00 g, 2.52 mmol) in acetonitrile (10 mL) was added CCl₄ (2.2 mL, 22.7 mmol, 9.0 eq.) at -25 °C. After stirring for 10 min, diisopropylethylamine (0.88 mL, 5.04 mmol, 2.0 eq.) and DMAP (0.03 g, 0.252 mmol, 0.1 eq.) were added, followed by dibenzyl phosphite (0.84 mL, 3.78 mmol, 1.5 eq.). The reaction mixture was allowed to reach r.t. over 1.5 h, and then quenched by a solution of KH₂PO₄ (0.5 M, 50 mL). The reaction mixture was extracted with EtOAc (3 × 50 mL). The combined organic extracts were dried over anhydrous Na₂SO₄ and concentrated under reduced pressure. The residue was purified by flash column chromatography (10-50% EtOAc/PE) to afford compound **390** (1.60 g, 96% yield) as a colorless oil. MS ESI m/z calcd for C₃₃H₄₁N₂O₁₀P [M+H]⁺ 657, found 657.

### Example 71. Synthesis of compound 391.

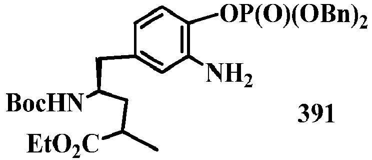

To a solution of compound **390** (1.60 g, 2.43 mmol) in methanol (20 mL) was added Pd/C (10 wt%, 160 mg). The reaction mixture was stirred under H₂ atmosphere (1 atm) at r.t. for 3 h, then filtered through Celite and concentrated under reduced pressure to afford compound **391** (1.00 g, 91% yield) as a white solid. MS ESI m/z calcd for C₁₉H₃₁N₂O₈P [M-H]⁻ 447, found 447.

### Example 72. Synthesis of compound 392.

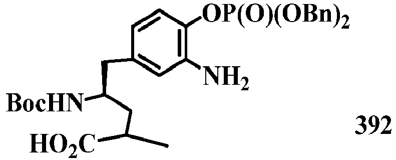

A solution of compound **391** (730 mg, 1.63 mmol) in ethanol (10 mL) was treated with 1 N NaOH (16 mL, 16.3 mmol, 10 eq.) at r.t. overnight, and then concentrated under reduced pressure. The residue was taken up in water (20 mL) and acidified to pH 6 by 1 N HCl. The aqueous solution was concentrated under reduced pressure and the residue was triturated with MeOH/EtOAc (80:20, 5 mL), compound **392** (0.68 g, 99% yield) was collected from filtration as a white solid. MS ESI m/z calcd for C₁₇H₂₇N₂O₈P [M-H]⁻ 417, found 417.

### Example 73. Synthesis of compound 399.

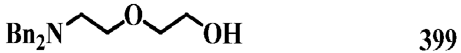

2-(2-aminoethoxy)ethanol (21.00 g, 200 mmol, 1.0 eq.) and K₂CO₃ (83.00 g, 600 mmol, 3.0 eq.) in acetonitrile (350 mL) was added BnBr (57.0 mL, 480 mmol, 2.4 eq.). The mixture was refluxed overnight. Water (1 L) was added and extracted with EtOAc (3 × 300 mL). The combined organic layers were washed with brine (1000 mL), dried over anhydrous Na₂SO₄, filtered, concentrated and purified by SiO₂ column chromatography (4:1 hexanes/ EtOAc) to give a colourless oil (50.97 g, 89.2% yield). MS ESI m/z calcd for C₁₈H₂₃NO₂Na [M + Na]⁺ 309.17, found 309.19.

### Example 74. Synthesis of compound 400.

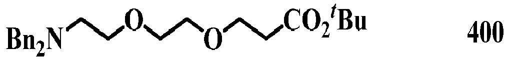

To a mixture of 2-(2-(dibenzylamino)ethoxy)ethanol (47.17 g, 165.3 mmol, 1.0 eq.), *tert-*butyl acrylate (72.0 mL, 495.9 mmol, 3.0 eq.) and *n*-Bu₄NI (6.10 g, 16.53 mmol, 0.1 eq.) in DCM (560 mL) was added sodium hydroxide solution (300 mL, 50%). The mixture was stirred overnight. The organic layer was separated and the water layer was extracted with EtOAc (3 × 100 mL). The organic layers were washed with water(3 × 300 mL) and brine (300 mL), dried over anhydrous Na₂SO₄, filtered, concentrated and purified by SiO₂ column chromatography (7:1 hexanes/ EtOAc) to give a colourless oil (61.08 g, 89.4% yield). MS ESI m/z calcd for C₂₅H₃₆NO₄ [M + H]⁺ 414.2566, found 414.2384.

### Example 75. Synthesis of compound 401.

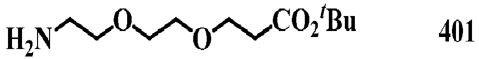

To a solution of tert-butyl 3-(2-(2-(dibenzylamino)ethoxy)ethoxy) propanoate (20.00 g, 48.36 mmol, 1.0 eq.) in THF (30 mL) and MeOH (60 mL) was added Pd/C (2.00 g, 10 wt%, 50% wet) in a hydrogenation bottle. The mixture was shaken overnight, filtered through Celite (filter aid), and the filtrate was concentrated to afford a colourless oil (10.58 g, 93.8% yield). MS ESI m/z calcd for C₁₁H₂₄NO₄ [M + H]⁺ 234.1627, found 234.1810.

### Example 76. Synthesis of compound 402.

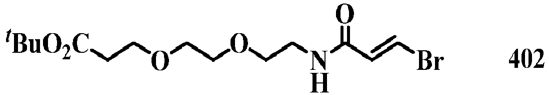

To a solution of (E)-3-bromoacrylic acid(0.15 g, 1 mmol), DMAP (0.15 g, 1.2 mmol) and DCC (0.21 g, 1 mmol) in DCM (10 ml), compound **401** (0.23g, 1mmol) were added at 0 °C. The reaction mixture was allowed to warm to r.t. and stirred overnight. The crude product was concentrated and purified by SiO₂ column chromatography with a gradient of EA/ DCM to give the title product **402** (0.31g, 85% yield). ESI MS m/z: calcd for C₁₄H₂₅BrNO₅ [M+H]⁺: 366.08, found 366.08.

### Example 77. Synthesis of compound 403.

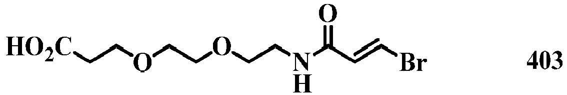

Compound **402** (0.31 g, 0.84 mmol) was dissolved in fomic acid (4 mL) at 0 °C then H₂O (2 mL) was added. The reaction mixture was allowed to warm to r.t. and stirred overnight. The crude product was concentrated and used for the next step without further purification. ESI MS m/z: calcd for C₁₀H₁₇BrNO₅ [M+H]⁺: 310.02, found 310.03.

### Example 78. Synthesis of compound 404.

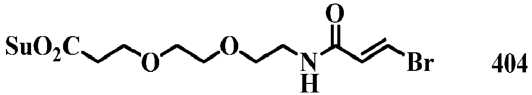

Compound **303** (0.12 g, 0.39 mmol), NHS (0.067 g, 0.58 mmol) and EDCI (0.11 g, 0.58 mmol) were dissolved in DCM (10 mL) and the mixture was stirred at r.t. overnight, concentrated and purified by SiO₂ column chromatography to give the title product **404** (0.13 g, 82% yield). ESI MS m/z: calcd for C₁₄H₂₀BrN₂O₇ [M+H]⁺: 407.04, found 407.04.

### Example 79. Synthesis of compound 426.

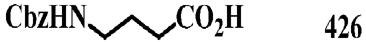

A solution of 4-aminobutyric acid (7.5 g, 75 mmol) and NaOH (6 g, 150 mmol) in H₂O (40 mL) was cooled to 0 °C and treated with a solution of CbzCl (16.1 g, 95 mmol) in THF (32 ml) dropwise. After 1 h, the reaction was allowed to warm to r.t. and stirred for 3 h. THF was removed under vacuum, the pH of the aqueous solution was adjusted to 1.5 by addition of 6 N HCl. The solution was extracted with ethyl acetate, and the organic layer was washed with brine, dried and concentrated to give compound **426** (16.4 g, 92% yield). MS ESI m/z calcd for C₁₂H₁₆NO₅ [M+H]⁺ 238.10, found 238.08.

### Example 80. Synthesis of compound 427.

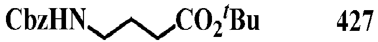

DMAP (0.8 g, 6.56 mmol) and DCC (17.1 g, 83 mmol) were added to a solution of 4-(((benzyloxy)carbonyl)amino)butanoic acid (16.4 g, 69.2 mmol) and *t*-BuOH (15.4 g, 208 mmol) in DCM (100 mL). After stirring at r.t. overnight, the reaction was filtered and filtrate concentrated. The residue was dissolved in ethyl acetate and the washed with IN HCl, brine and dried over Na₂SO₄. Concentration and purification by column chromatography (10 to 50% EtOAc/hexanes) yielded compound **427** (7.5 g, 37% yield). MS ESI m/z calcd for C₁₆H₂₃NO₄Na [M+Na]⁺ 316.16, found 316.13.

### Example 81. Synthesis of compound 428.

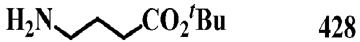

*Tert-Butyl* 4-(((benzyloxy)carbonyl)amino)butanoate (560 mg, 1.91 mmol) was dissolved in MeOH (50 mL), and mixed with Pd/C catalyst (10 wt%, 100 mg) then hydrogenated (1 atm) at r.t. for 3 h. The catalyst was filtered off and all volatiles were removed under vacuum to afford compound **428** (272 mg, 90% yield). MS ESI m/z calcd for C₈H₁₈NO₂ [M+H]⁺ 160.13, found 160.13.

### Example 82. Synthesis of compound 430.

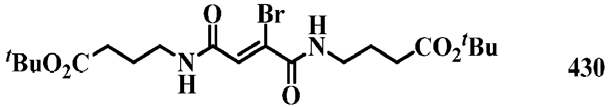

*Tert-Butyl* 4-aminobutanoate (477 mg, 3 mmol) and 2,3-dibromosuccinic acid (414 mg, 1.5 mmol) was dissolved in DCM (35 mL), to which DIPEA (1.16 g, 9 mmol) and EDC (0.86 g, 4.5 mmol) were added. The resulting solution was stirred at r.t. overnight and then washed with brine, dried over Na₂SO₄. Filtration, concentration and purification by column chromatography (pure DCM to 10% MeOH/DCM) yielded compound **430** (160 mg, 22% yield). MS ESI m/z calcd for C₂₀H₃₄BrN₂O₆[M+H]⁺ 477.15, found 477.16.

### Example 83. Synthesis of compound 431.

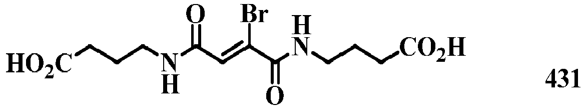

Compound **430** (80 mg, 0.168 mmol) was dissolved in DCM (5 mL) and treated with formic acid (8 mL) at 38 °C overnight. All volatiles were removed under vacuum to afford compound **431** (61 mg, 99% yield). MS ESI m/z calcd for C₁₂H₁₈BrN₂O₆ [M+H]⁺ 365.03, found 365.05.

### Example 84. Synthesis of compound 432.

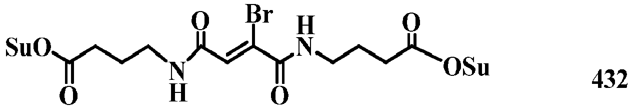

NHS (60 mg, 0.504 mmol) and EDCI (97 mg, 0.504 mmol) were added to a solution of compound **431** (61 mg, 0.168 mmol) in DCM (10 mL). After stirring at r.t. overnight, the reaction mixture was concentrated and purified by column chromatography (0 to 10% MeOH/DCM) to afford compound **432** (72 mg, 77% yield). MS ESI m/z calcd for C₂₀H₂₄BrN₄O₁₀ [M+H]⁺ 559.06, found 559.78.

### Example 85. Synthesis of compound 433.

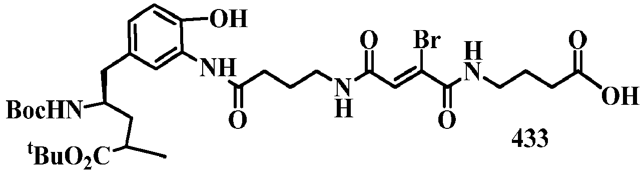

NaH₂PO₄ (0.1M in water, 1 mL) was added to a solution of compound **432** (36 mg, 0.065 mmol) and compound **110** (25 mg, 0.063 mmol) in EtOH (5 mL).The resulting solution was stirred at r.t. overnight. All volatiles were removed under vacuum and the residue was purified by column chromatography (0 to 10% MeOH/DCM) to yield compound **433** (19.7 mg, 41% yield). MS ESI m/z 741.35 ([M+H]⁺).

### Example 86. Synthesis of compound 435.

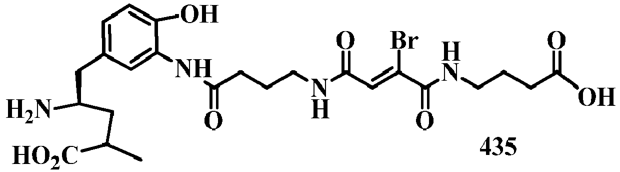

Compound **433** (18 mg, 0.024 mmol) was dissolved in DCM (2 mL) and treated with TFA (2 mL) at r.t. for 2 h. All volatiles were removed under vacuum to afford compound **435** (14 mg, 98% yield), which was use directly in the next step. MS ESI m/z 585.22 ([M+H]⁺).

### Example 87. Synthesis of compound 437.

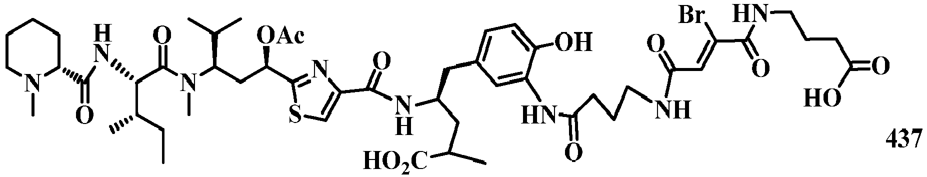

Compound **435** (14 mg, 0.0239 mmol) and perfluorophenyl ester **33a** (18 mg, 0.0255 mmol) were dissolved in DMA (5 mL). To the mixture, DIPEA (10 mg, 0.077 mmol) was added. The resulting mixture was stirred at r.t. overnight, concentrated and purified by preparative HPLC (C₁₈ column, 10-90% MeCN/H₂O) to afford compound **437** (12.8 mg, 48% yield). MS ESI m/z 1105.50 ([M+H]⁺).

### Example 88. Synthesis of compound 441.

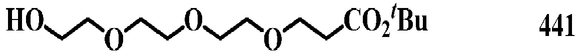

To a solution of 2,2'-(ethane-1,2-diylbis(oxy))diethanol (55.0 mL, 410.75 mmol, 3.0 eq.) in anhydrous THF (200 mL) was added sodium (0.1 g). The mixture was stirred until Na disappeared and then *tert*-butyl acrylate (20.0 mL, 137.79 mmol, 1.0 eq.) was added dropwise. The mixture was stirred overnight and then quenched by HCl solution (20.0 mL, IN) at 0°C. THF was removed by rotary evaporation, brine (300 mL) was added and the resulting mixture was extracted with EtOAc (3 × 100 mL). The organic layers were washed with brine (3 × 300 mL), dried over anhydrous Na₂SO₄, filtered and concentrated to afford a colourless oil (30.20 g, 79.0% yield), which was used without further purification. MS ESI m/z calcd for C₁₃H₂₇O₆ [M + H]⁺ 278.1729, found 278.1730.

### Example 89. Synthesis of compound 442.

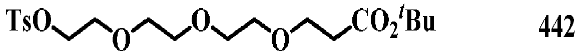

To a solution of tert-butyl 3-(2-(2-(2-hydroxyethoxy)ethoxy)ethoxy) propanoate (30.20 g, 108.5 mmol, 1.0 eq.) and TsCl (41.37 g, 217.0 mmol, 2.0 eq.) in anhydrous DCM (220 mL) at 0 °C was added TEA (30.0 mL, 217.0 mmol, 2.0 eq.). The mixture was stirred at room temperature overnight, and then washed with water (3 × 300 mL) and brine (300 mL), dried over anhydrous Na₂SO₄, filtered, concentrated and purified by SiO₂ column chromatography (3:1 hexanes/ EtOAc) to give a colourless oil (39.4 g, 84.0% yield).MS ESI m/z calcd for C₂₀H₃₃O₈S [M + H]⁺ 433.1818, found 433.2838.

### Example 90. Synthesis of compound 443.

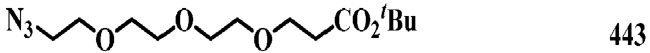

To a solution of tert-butyl 3-(2-(2-(2-(tosyloxy)ethoxy)ethoxy)ethoxy) propanoate (39.4 g, 91.1 mmol, 1.0 eq.) in anhydrous DMF(100 mL) was added NaN₃ (20.67 g, 316.6 mmol, 3.5 eq.). The mixture was stirred at room temperature overnight. Water (500 mL) was added and extracted with EtOAc (3 × 300 mL). The combined organic layers were washed with water (3 × 900 mL) and brine (900 mL), dried over anhydrous Na₂SO₄, filtered, concentrated and purified by SiO₂ column chromatography (5:1 hexanes/ EtOAc) to give a light yellow oil (23.8 g, 85.53% yield).MS ESI m/z calcd for C₁₃H₂₅O₃N₅Na [M + Na]⁺ 326.2, found 326.2.

### Example 91. Synthesis of compound 444.

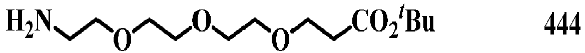

Raney-Ni (7.5 g, suspended in water) was washed with water (three times) and isopropyl alcohol (three times) and mixed with compound **443** (5.0 g, 16.5 mmol) in isopropyl alcohol. The mixture was stirred under a H₂ balloon at r.t. for 16 h and then filtered over a Celite pad, with washing of the pad with isopropyl alcohol. The filtrate was concentrated and purified by column chromatography (5-25% MeOH/DCM) to give light yellow oil (2.60 g, 57% yield). MS ESI m/z calcd for C₁₃H₂₈NO₅ [M+H]⁺ 279.19; found 279.19.

### Example 92. Synthesis of compound 445.

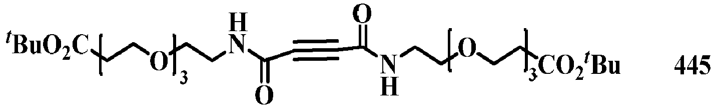

Acetylenedicarboxylic acid (0.35 g, 3.09 mmol, 1.0 eq.) was dissolved in NMP (10 mL) and cooled to 0 °C, to which compound **444** (2.06 g, 7.43 mmol, 2.4 eq.) was added, followed by DMTMM (2.39 g, 8.65 mmol, 2.8 eq.) in portions. The reaction was stirred at 0 °C for 6 h and then diluted with ethyl acetate and washed with water and brine. The organic solution was concentrated and triturated with a mixture solvent of ethyl acetate and petroleum ether. The solid was filtered off and the filtrate was concentrated and purified by column chromatography (80-90% EA/PE) to give a light yellow oil (2.26 g, >100% yield), which was used without further purification. MS ESI m/z calcd for C₃₀H₅₃N₂O₁₂ [M+H]⁺ 633.35; found 633.30.

### Example 93. Synthesis of compound 446.

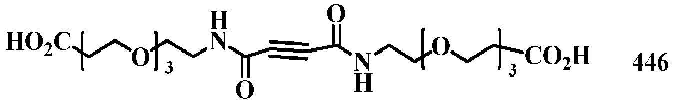

Compound **445** (2.26 g) was dissolved in dichloromethane (15 mL) and cooled to 0 °C then treated with TFA (15 mL). The reaction was warmed to r.t. and stirred for 45 min, and then the solvent and residual TFA was removed on rotovap. The crude product was purified by column chromatography (0-15% MeOH/DCM) to give light yellow oil (1.39 g, 86% yield for two steps). MS ESI m/z calcd for C₂₂H₃₇N₂O₁₂ [M+H]⁺ 521.23; found 521.24.

### Example 94. Synthesis of compound 480.

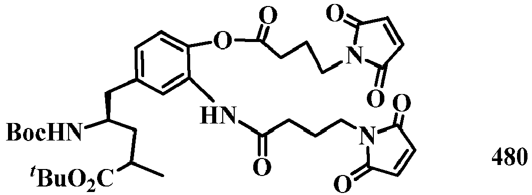

Compound **110** (68 mg, 0.17 mmol), compound **124** (94.5 mg, 0.52 mmol) and HATU (162 mg, 0.425 mmol) were dissolved in DCM (50 mL). TEA (73ul, 0.52mmol) was then added. The reaction mixture was stirred at r.t. overnight. Then the solvent was removed under reduced pressure and the residue was purified by SiO₂ column to give the title product **480** (98 mg, 80% yield). ESI m/z calcd for C₃₇H₄₉N₄O₁₁ [M+H]⁺: 725.33, found 725.34.

### Example 95. Synthesis of compound 481.

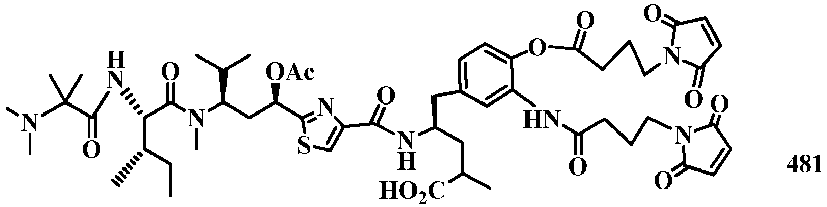

Compound **480** (98 mg, 0.135 mmol) dissolved in DCM (1.0 mL) was treated with TFA (1.0 mL) at r.t. for 2h, then concentrated and redissolved in DMA (1 mL), to which pentafluorophenyl ester **41a** (44 mg, 0.06 mmol) and DIPEA (45.8 µL, 0.27 mmol) were added. The reaction was stirred overnight and then concentrated. The residue was purified by prep-HPLC with a gradient of MeCN/H₂O to give the title product **481** (37 mg, 55% yield). ESI m/z calcd for C₅₃H₇₃N₈O₁₄S [M+H]⁺: 1077.49, found 1077. 50.

### Example 96. Synthesis of compound 484.

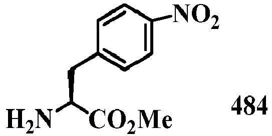

To a solution of (S)-2-amino-3-(4-nitrophenyl)propanoic acid (13.2 g, 62.8 mmol) in methanol (120 mL) was added thionyl chloride (9 mL, 125.6 mmol) at 0 °C. The reaction mixture was heated to reflux and stirred for 1 h, then concentrated under vacuum and suspended in ethyl acetate (50 mL). The mixture was then filtered to afford the title compound as a white solid (14.5 g, 91% yield). ESI m/z calcd for C₁₀H₁₃N₂O₄ [M+H]⁺: 225.08, found 225.08.

### Example 97. Synthesis of compound 485.

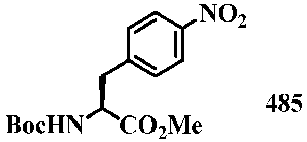

To a solution of compound **484** (9.5 g, 36.4 mmol) in THF (200 mL) was added triethylamine (12.6 mL, 91.1 mmol). After the mixture was stirred for 30 minutes, di-*tert*-butyl dicarbonate (12.5 mL, 54.7 mmol) was added, and the reaction mixture was stirred for 1 h, then diluted with ethyl acetate (200 mL), washed with 1 N HCl (30 mL), water (30 mL), dried over sodium sulfate, filtered and concentrated under vacuum to afford the title compound as a white solid (11.4 g, 97% yield). ESI m/z calcd for C₁₅H₂₁N₂O₆ [M+H]⁺: 325.13, found 325.13.

### Example 98. Synthesis of compound 486.

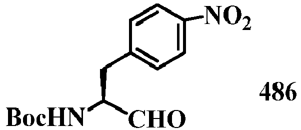

To a solution of compound **485** (14 g, 43.2 mmol) in anhydrous dichloromethane (150 mL) was added DIBAL-H (108 mL, 108 mmol) at -78 °C. The reaction mixture was stirred at -78 °C for 30 min., then poured into ice water (200 mL), extracted with ethyl acetate (3 × 80 mL). The combined organic phase was washed with IN HCl(2 × 50 mL), water (50 mL), dried over sodium sulfate, filtered, concentrated under vacuum, and purified by silica gel column chromatography to afford the title compound (8.6 g, 68% yield). ESI m/z calcd for C₁₄H₁₉N₂O₅ [M+H]⁺: 295.12, found 295.12.

### Example 99. Synthesis of compound 487.

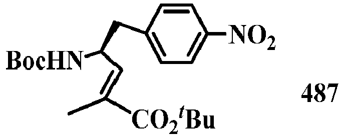

To a solution of compound **106** (8.1 g, 20.8 mmol) in DCM (100 mL) was added compound **486** (5.2 g, 17.8 mmol) at 0 °C. The reaction mixture was warmed to r.t. and stirred for 30 min. then concentrated under vacuum and purified by silica gel column to afford the title compound as a yellow solid (5.9 g, 82% yield). ESI m/z calcd for C₂₁H₃₁N₂O₆ [M+H]⁺: 406.21, found 406.21.

### Example 100. Synthesis of compound 488.

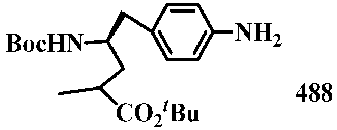

To a solution of compound **487** (4 g, 9.85 mmol) in MeOH (40 mL) was added Pd/C (0.4 g, 10 wt%) in a hydrogenation bottle. The mixture was stirred under 1 atm H₂ overnight, filtered through Celite (filter aid), and the filtrate was concentrated to afford compound **488** (3.6g, yield∼100%). ESI m/z: calcd for C₂₁H₃₅N₂O₄ [M+H]⁺: 379.25, found 379.25.

### Example 101. Synthesis of compound 489.

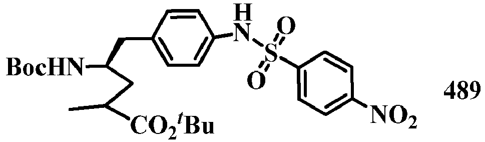

To a solution of compound **488** (3.6 g, 9.52 mmol) and triethylamine (1.3 mL, 9.52 mmol) in dichloromethane (50 mL) was added 4-nitrobenzenesulfonyl chloride (2.1 g, 9.52 mmol) at 0 °C. The reaction mixture was warmed to r.t. and stirred for 1 h, then diluted with DCM (50 mL), washed with IN HCl (20 mL), water (20 mL), dried over sodium sulfate, filtered and concentrated under vacuum, then purified by silica gel column chromatography to afford the title compound as a yellow solid (4 g, 75% yield). ESI m/z calcd for C₂₇H₃₈N₃O₈S [M+H]⁺: 564.23, found 564.23.

### Example 102. Synthesis of compound 490.

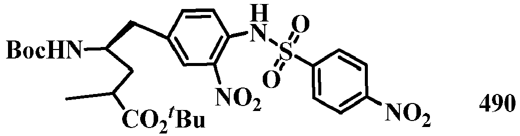

To a solution of compound **489** (3.6 g, 6.39 mmol) in acetonitrile (40 mL) was added *tert-*butyl nitrite (2.29 mL, 19.1 mmol). The reaction mixture was warmed to 45 °C and stirred for 6 hours. The reaction was then concentrated under vacuum and purified by silica gel column chromatography to afford the title compound (3 g, 79% yield).ESI m/z calcd for C₂₇H₃₇N₄O₁₀S [M+H]⁺: 609.22, found 609.22.

### Example 103. Synthesis of compound 491.

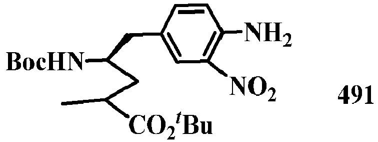

To a solution of compound **490** (3.0 g, 4.92 mmol) in acetonitrile/DMSO (30 mL/1 mL) were added 4-methoxy thiophenol (2.76 g, 19.7 mmol) and potassium carbonate (2.7 g, 19.7 mmol). The reaction mixture was stirred at the room temperature overnight, then diluted with ethyl acetate (100 mL), washed with water (20 mL), brine (20 mL), dried over sodium sulfate, filtered and concentrated under vacuum, and purified by silica gel column chromatography to afford the title compound (1.7 g, 85% yield). ESI m/z calcd for C₂₁H₃₄N₃O₆ [M+H]⁺: 424.24, found 424.24.

### Example 104. Synthesis of compound 492.

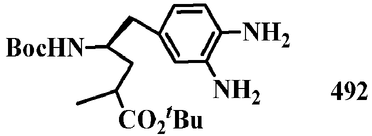

To a solution of compound **491** (100 mg, 0.236 mmol) in MeOH (4 mL) was added Pd/C (10 mg, 10 wt%) in a hydrogenation bottle. The mixture was stirred under 1 atm H₂ overnight, filtered through Celite (filter aid), and the filtrate was concentrated to afford the title compound (92.9 mg, ∼100% yield). ESI m/z calcd for C₂₁H₃₆N₃O₄ [M+H]⁺: 394.26, found 394.26.

### Example 105. Synthesis of compound 493.

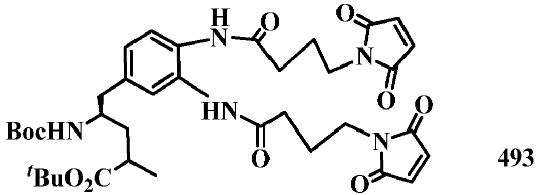

Compound **492** (66 mg, 0.17 mmol), compound **124** (94.5 mg, 0.52 mmol) and HATU (162 mg, 0.425 mmol) were dissolved in DCM (50 mL). TEA (73ul, 0.52mmol) was then added. The reaction mixture was stirred at r.t. overnight, the solvent was removed under reduced pressure and the residue was purified by SiO₂ column to give the title product **493** (98 mg, 80% yield). ESI m/z calcd for C₃₇H₅₀N₅O₁₀ [M+H]⁺: 724.35, found 724.35.

### Example 106. Synthesis of compound 494.

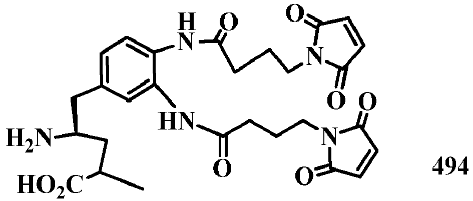

Compound **493** (98 mg, 0.135 mmol) dissolved in DCM (1.0 mL) was treated with TFA (1.0 mL) at r.t. for 2 h, then concentrated to give compound **494,** which was used in the next step without further purification.

### Example 107. Synthesis of compound 495.

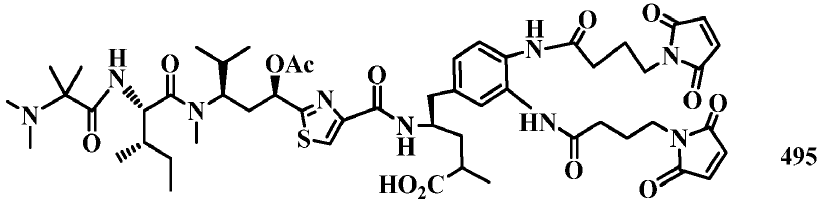

To a solution of compound **494** (76.9 mg, 0.135 mmol) in DMA (1 mL) was added pentafluorophenyl ester **41a** (44 mg, 0.06 mmol) and DIPEA (45.8 µL, 0.27 mmol). The reaction was stirred overnight, then concentrated and the residue was purified by prep-HPLC with a gradient of MeCN/H₂O to give the title product **495** (37 mg, 55% yield). ESI m/z calcd for C₅₃H₇₄N₉O₁₃S [M+H]⁺: 1076.50, found 1076.50.

### Example 108. Synthesis of compound 509.

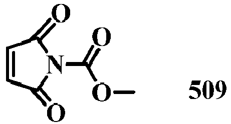

To a solution of maleimide (6.35 g, 65.4 mmol, 1.0 eq.) in EtOAc (120 mL) were added N-methyl morpholine (8.6 mL, 78.5 mmol, 1.2 eq.) and methyl chloroformate (6.0 mL, 78.5 mmol, 1.2 eq.) at 0 °C. The reaction was stirred at 0 °C for 30 min and r.t. 1 h. The solid was filtered off and filtrate concentrated. The residue was dissolved in CH₂Cl₂ and filtered through a silica gel plug and eluted with CH₂Cl₂ to remove the color. The appropriate fractions were concentrated and resulted solid was triturated with 10% EtOAc/PE to give a white solid (9.00 g, 89% yield).

### Example 109. Synthesis of compound 510.

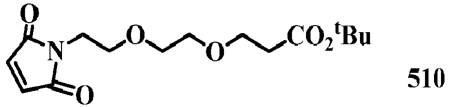

A mixture of compound **401** (8.16 g, 35.0 mmol, 1.0 eq.) and saturated NaHCO₃ (40 mL) was cooled to 0 °C, to which compound **509** (5.43 g, 35.0 mmol, 1.0 eq.) was added in portions. After stirring at 0 °C for 1 h, the reaction was warmed to r.t. and stirred for 1 h. The reaction was extracted with DCM (3 × 100 mL) and the organic extract was washed with brine, dried over anhydrous Na₂SO₄, concentrated and purified by SiO₂ column chromatography to give a white solid (6.76 g, 62% yield). MS ESI m/z calcd for C₁₅H₂₃NO₆ [M+H]⁺ 314.15, found 314.15.

### Example 110. Synthesis of compound 511.

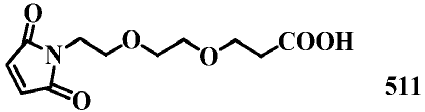

A solution of compound **510** (1.85 g, 5.9 mmol) was dissolved in DCM (20 mL) and treated with TFA (7 mL) at r.t. for 16 h, then concentrated and purified by SiO₂ column chromatography (11:1 DCM/MeOH) to give a white foam (1.47 g, 97% yield). MS ESI m/z calcd for C₁₁H₁₅NO₆ [M+H]⁺ 258.09, found 258.09.

### Example 111. Synthesis of compound 519.

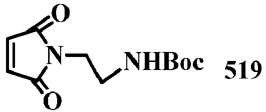

A mixture of *N*-Boc-ethylenediamine (5.6 mL, 35.4 mmol, 1.1 eq.) and saturated NaHCO₃ (60 mL) was cooled to 0 °C, to which compound **509** (5.00 g, 32.2 mmol, 1.0 eq.) was added in portions. After stirring at 0 °C for 30 min, the reaction was warmed to r.t. and stirred for 1 h. The precipitate was collected by filtration and washed with cold water, then dissolved in EtOAc and washed with brine, dried over anhydrous Na₂SO₄ and concentrated to give a white solid (6.69 g, 87% yield).

### Example 112. Synthesis of compound 520.

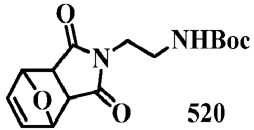

A solution of compound **519** (6.00 g, 25.0 mmol), furan (18.0 mL) in toluene (120 mL) in a high pressure tube was heated to reflux and stirred for 16 h. The colorless solution turned yellow during reaction. The mixture was then cooled to r.t. and concentrated. The resulting white solid was triturated with ethyl ether to give compound **520** (6.5 g, 84% yield).

### Example 113. Synthesis of compound 521.

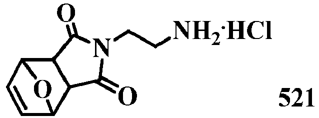

A solution of compound **520** (9.93 g, 32.2 mmol) was dissolved in dioxane (15 mL) and treated with concentrated HCl (15 mL) at r.t. for 3 h. The reaction was concentrated and the resulting solid was collected by filtration, with washing of the filter cake with EtOAc. The solid was dried in an oven (50 °C) overnight to give compound **521** (6.94 g, 88% yield).

### Example 114. Synthesis of compound 522.

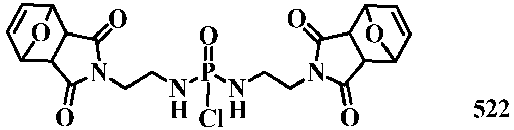

To a solution of compound **521** (0.85 g, 3.47 mmol) in THF (10 mL) was added POCl₃ (162 µL, 1.73 mmol) at -10 °C, followed by TEA (966 µL, 6.95 mmol). The reaction was stirred at -10 °C for 3h, and then the solution was diluted with DCM (20 mL) and filtered over Celite, the filtrate was concentrated to give compound **522,** which was used in the next step directly. ESI m/z calcd for C₂₀H₂₃ClN₄O₇P [M+H]⁺: 497.09, found 497. 09.

### Example 115. Synthesis of compound 523.

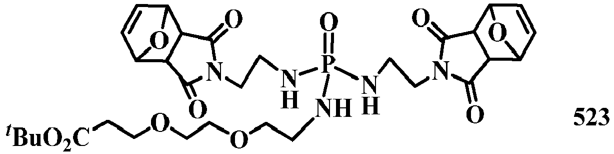

Compound **522** (0.50 g, 1.0 mmol) and DIPEA (0.4 mL, 2.4 mmol) were dissolved in DCM (5.0 mL) at 0 °C, and then compound **401** (0.23 g, 1.0 mmol) was added. The reaction was stirred at 0 °C for 2.5h, then concentrated and purified by SiO₂ column to give the title product **523** (0.30 g, 43%). ESI m/z calcd for C₃₁H₄₅N₅O₁₁P [M+H]⁺: 694.28, found 694.28.

### Example 116. Synthesis of compound 524.

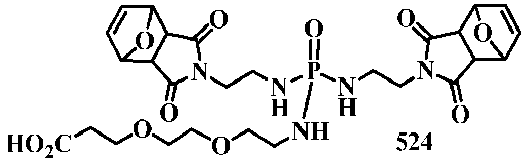

Compound **523** (0.30 g, 0.5 mmol) was dissolved in DCM (3 mL), and treated with TFA (3 mL) at r.t. for 2h, then concentrated to give compound **524,** which was used in the next step without further purification.

### Example 117. Synthesis of compound 525.

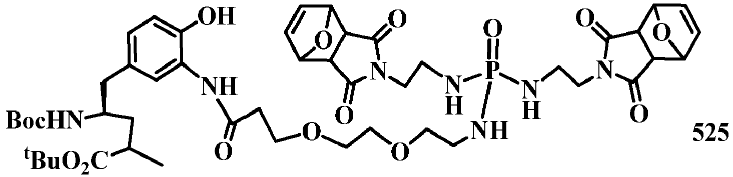

Compound **524** (40 mg, 0.063 mmol), compound **110** (40 mg, 0.10 mmol), HATU (24 mg, 0.063 mmol) were dissolved in DCM (5 mL), and then TEA (27.8 µL, 0.2 mmol) was added. The reaction mixture was stirred at r.t. overnight. Then the solvent was removed under reduced pressure and the residue was purified by SiO₂ column to give the title product **525** (53.4 mg, 84% yield). ESI m/z calcd for C₄₈H₆₉N₇O₁₅P [M+H]⁺: 1014.45, found 1014.45.

### Example 118. Synthesis of compound 526.

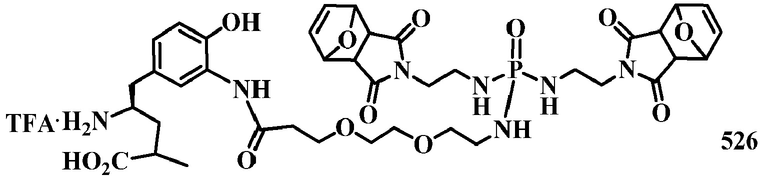

Compound **525** (53.4 mg, 0.053 mmol) was dissolved in DCM (2 mL), and treated with TFA (2 mL) at r.t. for 2 h, then concentrated to give compound **526,** which was used in the next step without further purification.

### Example 119. Synthesis of compound 527.

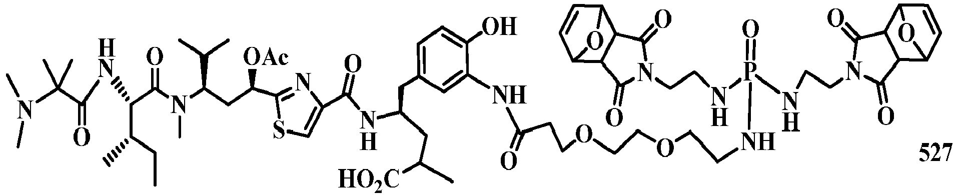

To a solution of compound **526** (45.0 mg, 0.053 mmol) in DMA (1mL) were added pentafluorophenyl ester **41a** (37.0 mg, 0.053mmol) and DIPEA (17 µL, 0.1 mmol). The reaction was stirred overnight and concentrated. The residue was purified by prep-HPLC with a gradient of MeCN/H₂O to give the title product **527** (26.2 mg, 36% yield). ESI m/z calcd for C₆₄H₉₃N₁₁O₁₈PS [M+H]⁺: 1366.61, found 1366. 61.

### Example 120. Synthesis of compound 528.

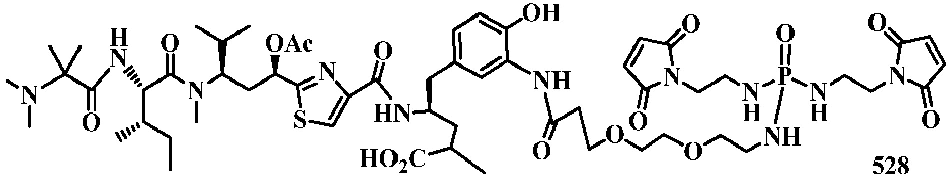

Compound **527** (8.0 mg, 0.0058 mmol) was dissolved in toluene (5.0 mL) and heated to reflux overnight, then concentrated and purified by prep-HPLC with a gradient of MeCN/H₂O to give the title product **528** (6.4 mg, 90% yield). ESI m/z calcd for C₅₆H₈₅N₁₁O₁₆PS [M+H]⁺: 1230.56, found 1230. 56.

### Example 121. Synthesis of compound 529.

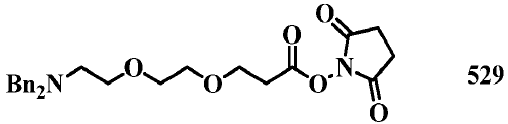

To a solution of tert-butyl 3-(2-(2-(dibenzylamino)ethoxy)ethoxy)propanoate (5.00 g, 12.1 mmol) in 10 mL DCM was added 5 mL of TFA. The reaction mixture was stirred at r.t. for 1 h, and then concentrated. The crude product was dissolved in DCM (50 mL), to which NHS (4.25 g, 37 mmol) and EDCI (7.10 g, 37 mmol) were added. The reaction mixture was stirred at r.t. overnight, then concentrated and purified by SiO₂ column with a gradient of DCM/MeOH to give the title compound **529** (5.00 g, 91%). ESI m/z calcd for C₂₅H₃₁N₂O₆ [M+H]⁺: 455.21, found 455.21.

### Example 122. Synthesis of compound 530.

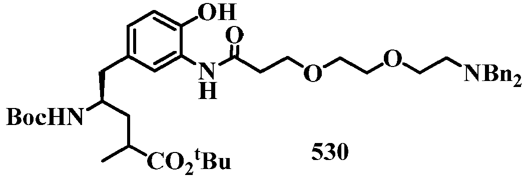

To a solution of compound **110** (1.00 g, 2.5 mmol, 1.0 eq.) in EtOH (10mL) were added compound **529** (1.80 g, 3.9 mmol, 1.5 eq.) and 0.1M NaH₂PO₄ (2 mL) at r.t. The reaction mixture was stirred at r.t. overnight, and then concentrated. The residue was diluted with H₂O (100 mL), then extracted with EtOAc (3 × 50mL). The combined the organic layers were dried over Na₂SO₄, filtered and concentrated, purified by SiO₂ column with a gradient of DCM/MeOH to give the title compound **530** (0.93 g, 50%). ESI m/z calcd for C₄₂H₅₀N₃O₈ [M+H]⁺: 734.43, found 734.43.

### Example 123. Synthesis of compound 531.

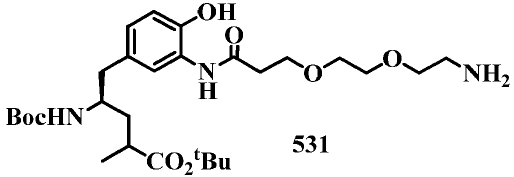

In a hydrogenation bottle, Pd/C (0.093 g, 10 wt%) was added to a solution of compound **530** (0.93 g, 1.27 mmol) in EtOAc (20 mL). The mixture was shaken overnight under 1 atm H₂ then filtered through Celite (filter aid), the filtrate was concentrated to afford compound **531** (0.57 g, 81%) and used in the next step without further purification. ESI m/z calcd for C₂₈H₄₈N₃O₈ [M+H]⁺:554.34, found 554.34.

### Example 124. Synthesis of compound 537.

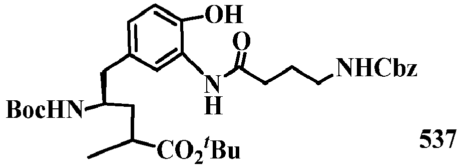

HATU (39.9 g, 105 mmol) was added to a solution of 4-(((benzyloxy)carbonyl)amino) butanoic acid (26.1 g, 110 mmol) in DMF (300 mL). After stirring at r.t. for 30 min, the mixture was added to a solution of compound **110** (39.4 g, 100 mmol) and TEA (20.2 g, 200 mmol) in DMF (300 mL).The resulting mixture was stirred at r.t. for 2 h. Water was then added, extracted with EtOAc, the organic layer was washed with brine, dried over Na₂SO₄. Purification by column chromatography (20% to 70% EA/PE) yielded the title product as a white solid (45 g, 73% yield). ESI m/z calcd for C₃₃H₄₈N₃O₈ [M+H]⁺: 614.34, found 614.15.

### Example 125. Synthesis of compound 538.

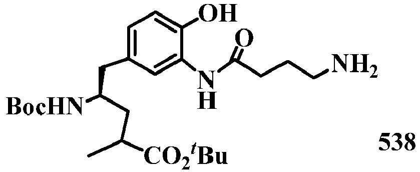

Compound **537** (100 g, 163mmol) was dissolved in methanol (500 mL) and hydrogenated (1 atm) with Pd/C catalyst (10 wt%, 10 g) at r.t. overnight. The catalyst was filtered off and the filtrate were concentrated under reduced pressure to afford compound **538** (75.8 g, 97% yield) as a brown foamy solid. ¹H NMR (400 MHz, CDCl₃) δ 7.11 (s, 1H), 6.83 (d, J = 10.3 Hz, 2H), 5.04 - 4.52 (m, 6H), 3.90 - 3.56 (m, 1H), 2.81 (d, J = 5.3 Hz, 2H), 2.63 (dd, J = 12.5, 6.1 Hz, 2H), 2.54-2.26 (dd, J = 14.0, 7.6 Hz, 4H), 1.94-1.64 (m, 3H), 1.44 - 1.36 (m, 18H), 1.08 (d, J = 6.9 Hz, 3H). ESI m/z calcd for C₂₅H₄₂N₃O₆ [M+H]⁺: 480.30, found 480.59.

### Example 126. Synthesis of compound 539.

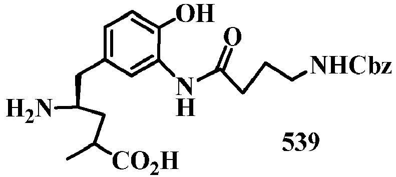

To a solution of compound **537** (1.00 g, 1.63 mmol) in 1 mL DCM was added 2 mL TFA, the reaction mixture was stirred at r.t. for 1.h, and then concentrated. The resulting crude product **539** was used in the next step without further purification. ESI m/z calcd for C₂₄H₃₂N₃O₆ [M+H]⁺: 458.22, found 458.22.

### Example 127. Synthesis of compound 540.

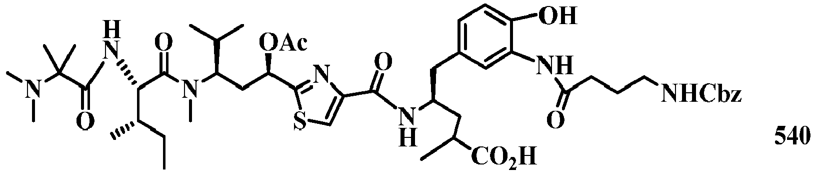

To a solution of compound **539** (0.42 g, 0.92 mmol) in DMF (3 mL) were added pentafluorophenyl ester **41a** (0.63 g, 0.91 mmol) and DIPEA (0.46 mL, 2.73 mmol). The reaction was stirred at r.t. overnight, then concentrated and purified by SiO₂ column with a gradient of DCM/MeOH to give the title compound **540** (0.67 g, 75% yield) as a yellow oil. ESI m/z calcd for C₄₉H₇₂N₇O₁₁S [M+H]⁺: 966.49, found 966.49.

### Example 128. Synthesis of compound 541.

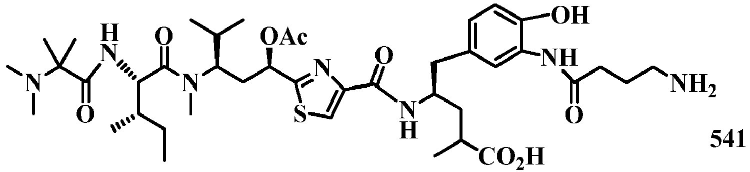

In a hydrogenation bottle, Pd/C (0.02 g, 10 wt%) was added to a solution of compound **540** (0.40 g, 0.41 mmol) in MeOH (15 mL). a drop of IN HCl was then added to adjust pH to around 4. The mixture was shaken overnight under 1 atm H₂ then filtered through Celite (filter aid), the filtrate was concentrated to afford compound **541,** which was used in the next step without further purification. ESI m/z calcd for C₄₁H₅₆N₇O₉S [M+H]⁺: 832.46, found 832.46.

### Example 129. Synthesis of compound 587.

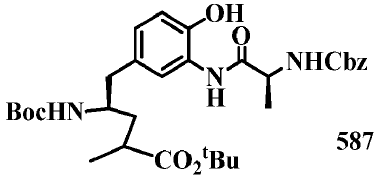

Compound **110** (0.30 g, 0.76 mmol), compound Z-L-Ala-OH(0.17 g, 0.76 mmol) and HATU (0.29 g, 0.76 mmol) were dissolved in DCM (20 mL), to which TEA (110 µL, 0.8 mmol) was added. The reaction mixture was stirred at r.t. overnight. Then the solvent was removed under reduced pressure and the residue was purified by SiO₂ column to give the title product **587** (0.43 g, 95% yield). ESI m/z calcd for C₃₂H₄₆N₃O₈ [M+H]⁺: 600.32, found 600.32.

### Example 130. Synthesis of compound 627.

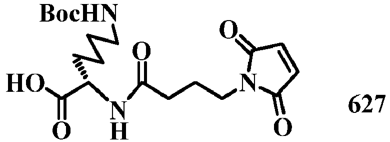

To a solution of H-Lys(Boc)-OH (1.00 g, 3.8 mmol, 1.0 eq.) in EtOH (16 mL) was added compound **125** (1.00 g, 5.6 mmol, 1.5 eq.) at r.t. After 0.1 M NaH₂PO₄ (3 mL) was added, the reaction mixture was stirred at r.t. overnight. The reaction was concentrated under vacuum, and the residues was purified by SiO₂ column with a gradient of DCM/MeOH to give the title compound **627** (1.62 g, theoretical yield). ESI m/z calcd for C₁₉H₃₀N₃O₇ [M+H]⁺: 412.20, found 412.20.

### Example 131. Synthesis of compound 628.

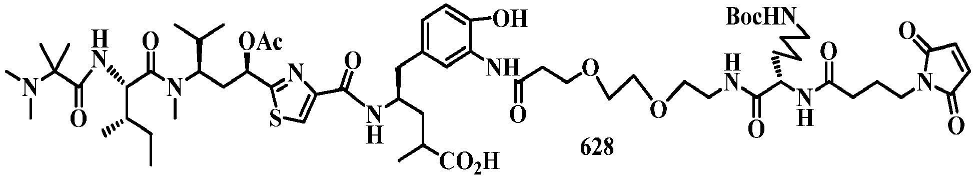

To a solution of carboxylic acid **627** (0.24 g, 0.58 mmol) in EtOAc (10 mL) were added pentafluorophenol (0.21 g, 1.17 mmol) and DCC (0.24 g, 1.17 mmol). The reaction mixture was stirred at r.t. overnight, and then filtered with washing of the filter cake with EtOAc, and the filtrate was concentrated. The resulting PFP-ester(32 mg, 0.056 mmol) was dissolved in 1mL DMF, to which compound **531** (50 mg, 0.056 mmol) and *i*-Pr₂EtN (29 µL, 0.168 mmol) were added. The reaction mixture was stirred at r.t. for 2 h and concentrated. The residue was purified by HPLC with a gradient of MeCN/H₂O to give the title compound **628** (3 mg, 4% yield). ESI m/z calcd for C₆₃H₉₉N₁₀O₁₇S [M+H]⁺: 1299.68, found 1299.68.

### Example 132. Synthesis of compound 629.

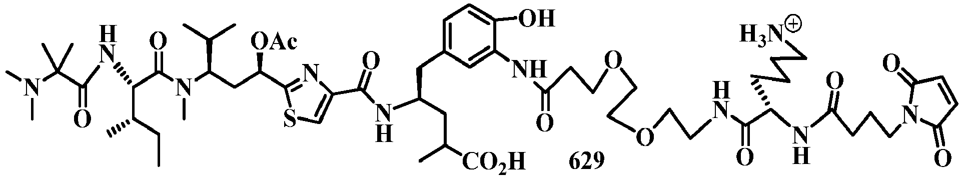

To a solution of compound **628** (3 mg, 0.002 mmol) in 0.5 mL DCM was added 1 mL TFA , the reaction mixture was stirred at r.t. for 1h, then concentrated. The crude product was purified by HPLC with a gradient of MeCN/H₂O to give the title compound **629** (1.43 mg, 52% yield). ESI m/z calcd for C₅₈H₉₁N₁₀O₁₅S [M+H]⁺:1199.63, found 1199.62.

### Example 133. Synthesis of compound 632.

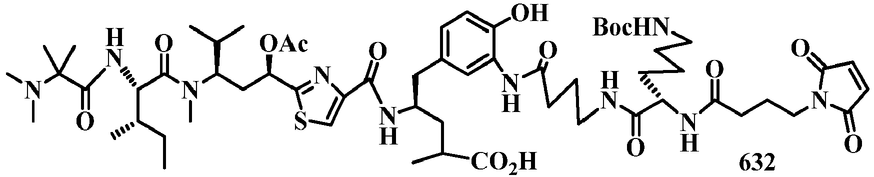

The pentafluorophenyl ester of compound **627** (0.11 g, 0.19 mmol) was dissolved in 1mL DMF, to which compound **541** (0.21 g, 0.25 mmol) and *i*-Pr₂EtN (86 uL, 0.5 mmol) were added. The reaction mixture was stirred at r.t. for 2 h and concentrated. The residue was purified by prep-HPLC with a gradient of MeCN/H₂O to give the title product **632** (20 mg, 9%). ESI m/z calcd for C₆₀H₉₃N₁₀O₁₅S [M+H]⁺: 1225.65, found 1225.66.

### Example 134. Synthesis of compound 633.

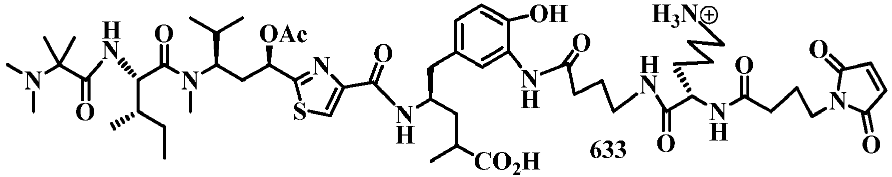

To a solution of compound **632** (20 mg, 0.016 mmol) in 1 mL DCM was added 2 mL TFA. The reaction mixture was stirred at r.t. for 1h, then concentrated, and the crude product was purified by prep-HPLC with a gradient of MeCN/H₂O to give the title compound **633** (8.9 mg, 18% yield). ESI m/z calcd for C₅₅H₈₅N₁₀O₁₃S [M+H]⁺: 1125.59, found 1125.59.

### Example 135. Synthesis of compound 636.

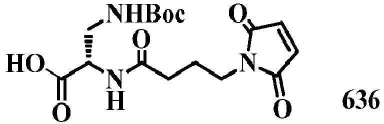

To a solution of H-Dap(Boc)-OH (1.00 g, 4.9 mmol, 1.0 eq.) in EtOH (30 mL) was added compound **125** (2.00 g, 7.3 mmol, 1.5 eq.) at r.t. Then 0.1M NaH₂PO₄ (6 mL) was added, and the reaction mixture was stirred at r.t. overnight. The solvents were removed under vacuum, and the residues was purified by SiO₂ column with a gradient of DCM/MeOH to give the title compound **636** (1.41 g, 78%). ESI m/z calcd for C₁₆H₂₄N₃O₇ [M+H]⁺: 370.15, found 370.15.

### Example 136. Synthesis of compound 637.

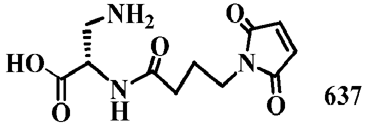

To a solution of compound **636** (1.41 g, 3.8 mmol) in 2 mL DCM was added 5 mL TFA. The reaction mixture was stirred at r.t. for 1h, and then concentrated. The crude product **637** was used in the next step without further purification. ESI m/z calcd for C₁₁H₁₆N₃O₅ [M+H]⁺: 270.10, found 270.10.

### Example 137. Synthesis of compound 638.

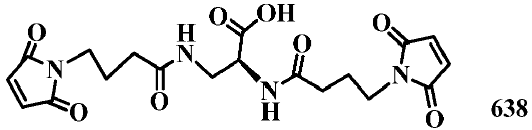

To a solution of above compound **637** in EtOH (20 mL) was added compound **125** (1.90 g, 6.9 mmol, 1.5 eq.) at r.t. Then 0.1M NaH₂PO₄ (4 mL) was added, and the reaction mixture was stirred at r.t. overnight. After the solvents were removed under vacuum, then the residues was purified by HPLC with a gradient of H₂O/MeCN to give the title compound **638** (0.45 g, 22% yield). ESI m/z calcd for C₁₉H₂₃N₄O₈ [M+H]⁺: 435.14, found 435.14.

### Example 138. Synthesis of compound 639.

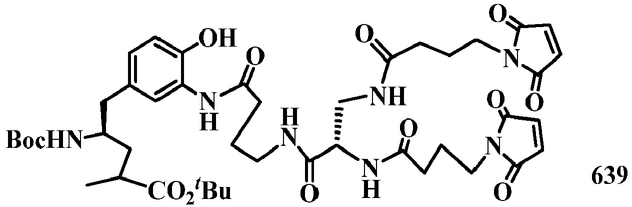

To a solution of compound **638** (0.15 g, 0.34 mmol), compound **538** (0.17 g, 0.34 mmol) and HATU (0.16 g, 0.41 mmol) in DMF (2 mL), TEA (95 µL, 0.68 mmol) was added. After stirring at r.t. for 1 h, the reaction was concentrated under reduced pressure and the residue was purified by prep-HPLC with a gradient of MeCN/H₂O to give the title compound **639** (34 mg, 11% yield). ESI m/z calcd for C₄₄H₆₂N₇O₁₃ [M+H]⁺: 896.43, found 896.42.

### Example 139. Synthesis of compound 640.

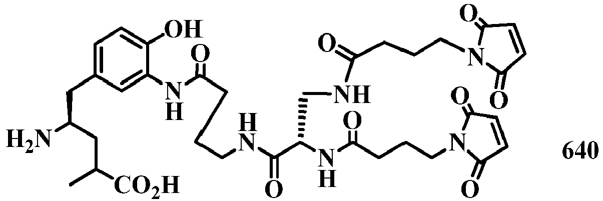

To a solution of compound **639** (34 mg, 0.04 mmol) in 0.5 mL DCM was added 1 mL TFA. The reaction mixture was stirred at r.t. for 2h, and then concentrated to afford the title compound **640**, which was used in the next step without further purification. ESI m/z calcd for C₃₅H₄₆N₇O₁₁ [M+H]⁺: 740.30, found 740.32.

### Example 140. Synthesis of compound 641.

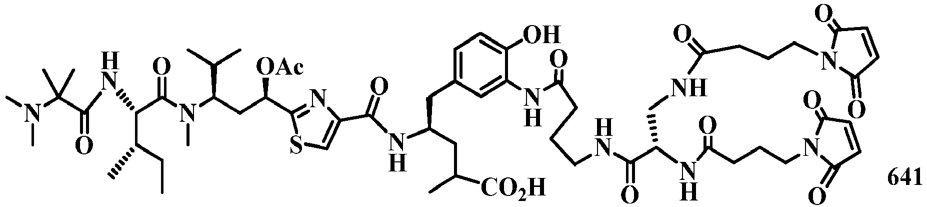

To the solution of compound **640** in DMA (2 mL) was added pentafluorophenyl ester **41a** (28 mg, 0.04 mmol), followed by DIPEA (21 µL, 0.12 mmol). The reaction was stirred overnight and then concentrated and purified by prep-HPLC with a gradient of MeCN/H₂O to give the title compound **641** (14.4 mg, 29%). ESI m/z calcd for C₆₀H₈₆N₁₁O₁₆S [M+H]⁺: 1248.59, found 1248.60.

### Example 141. Synthesis of compound 644.

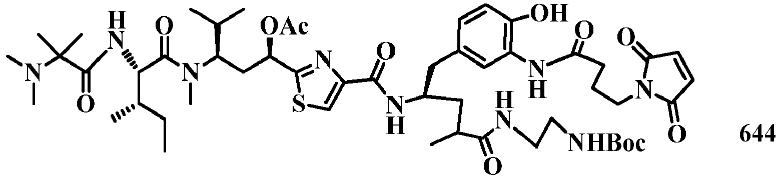

To a solution of compound **132** (0.300 g, 0.329 mmol, 1.0 eq.) and tert-butyl (2-aminoethyl)carbamate hydrochloride (0.063 g, 0.395mmol, 1.2 eq.) in anhydrous DCM (30 mL) at 0 °C was added EDCI (0.189 g, 0.988mmol, 3.0 eq.). After stirring for 10 minutes, the reaction was warmed to room temperature and stirred overnight. The reaction was diluted with DCM and washed with water and brine, dried over anhydrous Na₂SO₄, concentrated and purified by SiO₂ column chromatography (DCM /MeOH) to give compound **644** as a yellow foamy solid (0.132 g, 54% yield). ESI m/z calcd for C₅₂H₈₀N₉O₁₂S[M+H]⁺: 1054.6, found: 1054.6.

### Example 142. Synthesis of compound 645.

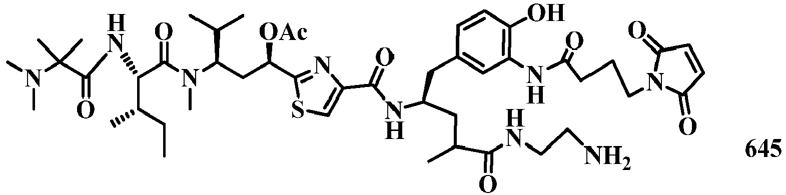

To a solution of compound **644** (0.132 g, 0.125 mmol, 1.0 eq.) in DCM (4.5 mL) at r.t. was added TFA (1.5 mL) and stirred for 1 h. The reaction was diluted with anhydrous toluene and concentrated, and this operation was repeated for three times to give yellow oil which was purified on prep-HPLC (C₁₈ column, mobile phase A: water, mobile phase B: acetonitrile, from 10% of B to 80% of B in 60 min). The fractions were pooled and lyophilized to give compound **645** (111 mg, 93% yield). ESI m/z calcd for C₄₇H₇₂N₉O₁₀S [M+H]⁺: 954.5, found: 954.5.

### Example 143. Synthesis of compound 648.

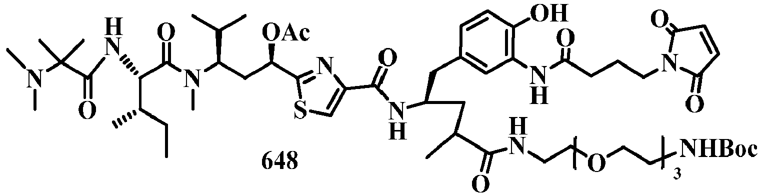

To a solution of compound **645** (0.050 g, 0.0549 mmol, 1.0 eq.) and *tert*-butyl (2-(2-(2-(2-aminoethoxy)ethoxy)ethoxy)ethyl)carbamate (0.024 g, 0.0824mmol, 1.5 eq.) in anhydrous DCM (10 mL) at 0 °C was added EDCI (0.032 g, 0.1647mmol, 3.0 eq.). After stirring for 10 minutes, the reaction was warmed to r.t. and stirred overnight. The mixture was then diluted with DCM and washed with water and brine, dried over anhydrous Na₂SO₄, concentrated and purified by SiO₂ column chromatography (DCM/MeOH) to give the title compound as a yellow foamy solid (0.030 g, 46% yield). ESI m/z calcd for C₅₈H₉₂N₉O₁₅S [M+H]⁺: 1186.6, found: 1186.6.

### Example 144. Synthesis of compound 649.

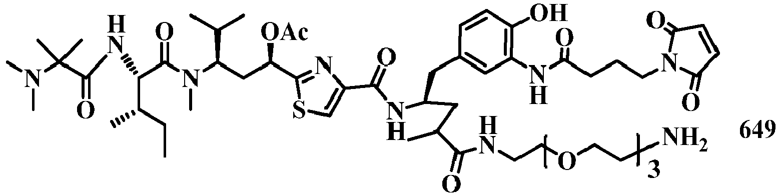

To a solution of compound **648** (0.030 g, 0.0253 mmol, 1.0 eq.) in DCM (3.0 mL) at r.t. was added TFA (1.0 mL). The reaction was stirred for 1 h and then diluted with anhydrous toluene and concentrated, this operation was repeated for three times to give a yellow oil, which was purified on prep-HPLC (C₁₈ column, mobile phase A: water, mobile phase B: acetonitrile, from 10% of B to 80% of B in 60 min). The fractions were pooled and lyophilized to give compound **649** (11.7 mg, 43% yield). ESI m/z calcd for C₅₃H₈₄N₉O₁₃S [M+H]⁺: 1086.6, found: 1086.6.

### Example 145. Synthesis of compound 652.

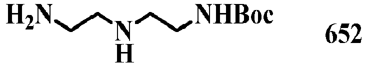

To a solution of N-(2-aminoethyl)ethane-1,2-diamine (28.7 g, 275 mmol, 10.0 eq.) and DMAP (0.034 g, 0.000275mmol, 0.01 eq.) in anhydrous DCM (350 mL) at 0 °C was added Boc₂O (6.0 g, 0.0275mmol, 1.0 eq.) in anhydrous DCM (100 mL) over 3 h. The reaction was then warmed to r.t. and stirred overnight, concentrated and purified by SiO₂ column chromatography (DCM/MeOH) to give the title compound as a yellow oil (4.5 g, 80% yield). ESI m/z calcd for C₉H₂₂N₃O₂ [M+H]⁺: 204.2, found:204.2.

### Example 146. Synthesis of compound 653.

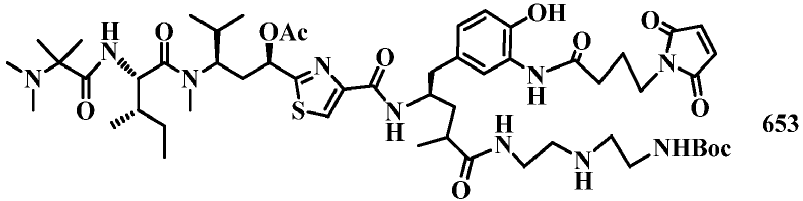

To a solution of compound **645** (0.060 g, 0.0658 mmol, 1.0 eq.) and tert-butyl (2-((2-aminoethyl)amino)ethyl)carbamate (0.016 g, 0.0790 mmol, 1.2 eq.) in anhydrous DCM (6 mL) at 0 °C was added EDCI (0.038 g, 0.1974 mmol, 3.0 eq.). After stirring for 10 minutes, the reaction was warmed to r.t. and stirred overnight. The mixture was concentrated and purified on prep-HPLC (C₁₈ column, mobile phase A: water, mobile phase B: acetonitrile, from 10% of B to 80% of B in 60 min). The fractions were pooled and lyophilized to give the title compound **653** (48 mg, 66% yield). ESI m/z calcd for C₅₄H₈₅N₁₀O₁₂S [M+H]⁺: 1097.6, found: 1097.6.

### Example 147. Synthesis of compound 654.

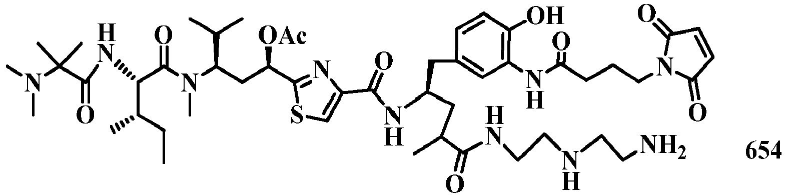

To a solution of compound **653** (0.048g, 0.0437mmol, 1.0 eq.) in DCM (3.0 mL) at r.t. was added TFA (1.0 mL). After stirring for 1 h, the reaction was diluted with anhydrous toluene and concentrated, and this operation was repeated for three times to give a yellow oil, which was purified on prep-HPLC (C₁₈ column, mobile phase A: water, mobile phase B: acetonitrile, from 10% of B to 80% of B in 60 min). The fractions were pooled and lyophilized to give the title compound **654** (111 mg, 93% yield). ESI m/z calcd for C₄₉H₇₇N₁₀O₁₀S [M+H]⁺: 997.5, found: 997.5.

### Example 148. Synthesis of compound 658.

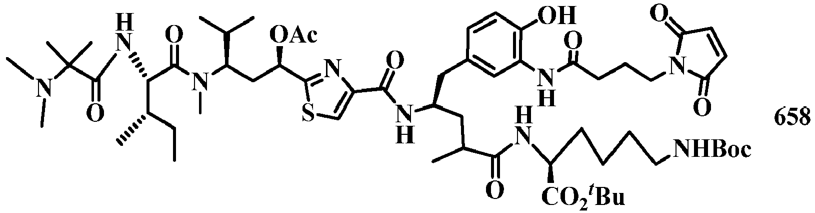

To a solution of compound **645** (0.400 g, 0.439 mmol, 1.0 eq.) and H-Lys(Boc)-O*^{t}*Bu·HCl (0.135 g, 0.528 mmol, 1.2 eq.) in anhydrous DCM (40 mL) at 0 °C was added EDCI (0.189 g, 1.317mmol, 3.0 eq.). After stirring for 10 min, the reaction was warmed to r.t. and stirred overnight. The mixture was diluted with DCM and washed with water and brine, dried over anhydrous Na₂SO₄, concentrated and purified by SiO₂ column chromatography (DCM/MeOH) to give compound **658** as a yellow oil (0.43 g, 82% yield). ESI m/z calcd for C₆₀H₉₄N₉O₁₄S [M+H]⁺: 1196.7, found: 1196.7.

### Example 149. Synthesis of compound 559.

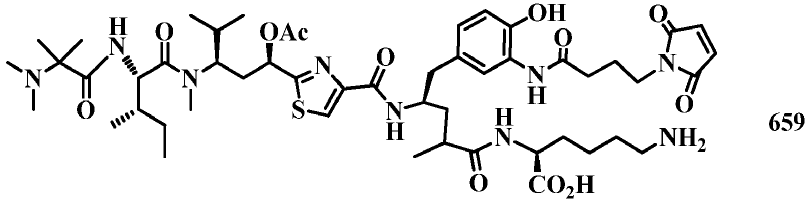

To a solution of compound **658** (0.230 g, 0.192 mmol, 1.0 eq.) in DCM (6.0 mL) at r.t. was added TFA (2.0 mL) and the reaction was stirred for 3 h and then diluted with toluene and concentrated, this operation was repeated for three times to give a yellow oil, which was purified on prep-HPLC (C₁₈column, mobile phase A: water, mobile phase B: acetonitrile, from 10% of B to 80% of B in 60 min). The fractions were pooled and lyophilized to give the title compound (153 mg, 76% yield). ESI m/z calcd for C₅₁H₇₈N₉O₁₂S [M+H]⁺: 1040.5, found:1040.5.

### Example 150. Synthesis of compound 662.

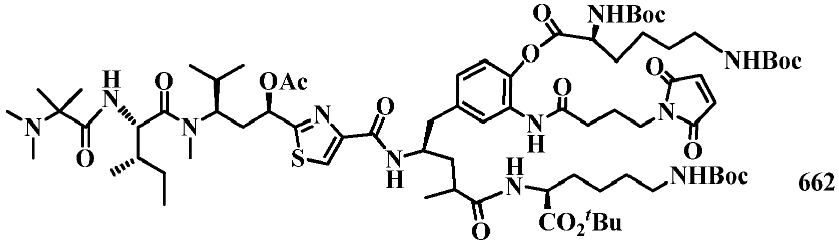

To a solution of compound **658** (0.200 g, 0.167 mmol,1.0 eq.) and Boc-L-Lys(Boc)-OH (0.070 g, 0.200 mmol, 1.2 eq.) in anhydrous DCM (10 mL) at 0 °C was added HATU (0.095 g, 0.250 mmol, 1.5 eq.) and TEA (46 µL, 0.334mmol, 2.0 eq.). The reaction was stirred for 10 min at 0 °C and stirred for 10 minutes, then warmed to r.t. and stirred overnight. The mixture was diluted with DCM and washed with water and brine, dried over anhydrous Na₂SO₄, concentrated and purified by SiO₂ column chromatography (DCM/MeOH) to give compound **662** as a colourless oil (0.270 g, theoretical yield). ESI m/z calcd for C₇₆H₁₂₂N₁₁O₁₉S [M+H]⁺: 1524.9, found: 1524.9.

### Example 151. Synthesis of compound 663.

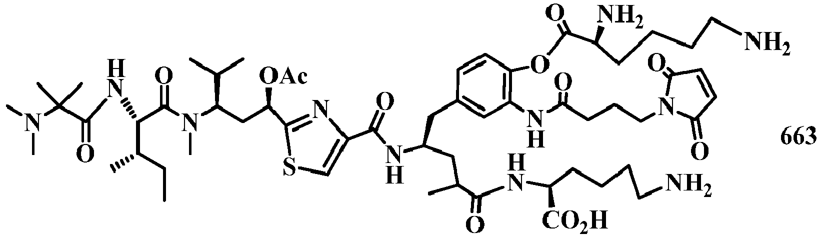

To a solution of compound **662** (0.270 g, 0.177 mmol, 1.0 eq.) in DCM (6.0 mL) at r.t. was added TFA (2.0 mL) and stirred for 4 h. The mixture was diluted with anhydrous toluene and concentrated, this operation was repeated for three times to give yellow oil, which was purified on prep-HPLC (C₁₈ column, mobile phase A: water, mobile phase B: acetonitrile, from 10% of B to 80% of B in 60 min). The fractions were pooled and lyophilized to give the title compound (172 mg, 83% yield). ESI m/z calcd for C₅₇H₉₀N₁₁O₁₃S [M+H]⁺: 1168.6, found: 1168.6.

### Example 152. Synthesis of compound 666.

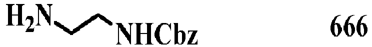

To a solution of ethane-1,2-diamine (30.0 g, 0.5 mol, 10.0 eq.) in anhydrous DCM (500 mL) at 0 °C was added CbzCl (8.53 g, 0.050 mol, 1.0 eq.) in anhydrous DCM (250 mL) over 7 h. The reaction was then warmed to r.t. and stirred overnight. The mixture was washed with water and brine, dried over anhydrous Na₂SO₄, and concentrated to give benzyl (2-aminoethyl)carbamate as a white solid (7.0 g, 94% yield). ESI m/z calcd for C₁₀H₁₄N₂O₂ [M+H]⁺: 195.1, found: 195.2.

### Example 153. Synthesis of compound 667.

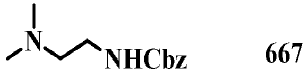

To a solution of compound **666** (7.0 g, 35.8 mmol, 1.0 eq.) and 37% HCHO (aq) (14mL, 0.1772mmol, 5.0 eq.) in MeOH (120 mL) at 0 °C was added NaBH₃CN (3.9 g, 0.0620 mol, 1.6 eq.), then HOAc (3 mL) was added to adjust pH ∼7.0. The mixture was warmed to r.t. and stirred overnight, then concentrated. The residue was dissolved in DCM (200 mL), and washed with water and brine, dried over anhydrous Na₂SO₄, concentrated and purified by SiO₂ column chromatography (DCM/MeOH) to give the title compound as a light yellow oil (6.4 g, 80% yield). ESI m/: calcd for C₁₂H₁₈N₂O₂ [M+H]⁺: 224.1, found:224.1.

### Example 154. Synthesis of compound 668.

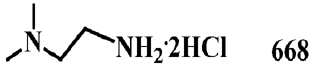

Compound **667** (3.0 g, 13.4 mmol, 1.0 eq.) and Pd/C (0.3 g, 10% Pd/C, 50% wet) were mixed with HCl (3 mL) and MeOH (100 mL) in a hydrogenation bottle and shaken at 100 psi H₂ atmosphere for 5 h. Then the mixture was filtered over Celite and the filtrate was concentrated to give the title compound as a yellow solid (2.1 g, 98% yield). ¹H NMR (400 MHz, D₂O) δ 3.33 (d, *J=* 4.6 Hz, 2H), 3.27 (s, 2H), 2.79 (s, 6H).

### Example 155. Synthesis of compound 669.

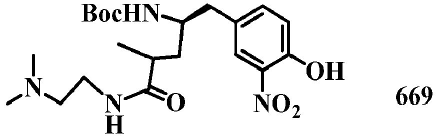

To a solution of compound **103** (0.58 g, 1.58 mmol, 1.0 eq.) and compound **668** (0.051 g, 3.15 mmol, 2.0 eq.) in anhydrous DMF (10 mL) at 0 °C were added HATU (0.090 g, 2.37 mmol, 1.5 eq.) and TEA(0.656 mL, 4.74 mmol, 3.0 eq.). After stirring for 10 minutes, the reaction was warmed to r.t. and stirred for 90 minutes. The mixture was diluted with H₂O and extracted with EA (3 × 100 mL). The combined organic layers were washed with water and brine, dried over anhydrous Na₂SO₄, concentrated to give the title compound as a yellow foamy solid (0.67 g, 97% yield). ESI m/z calcd for C₂₁H₃₅N₄O₆ [M+H]⁺: 439.2, found:439.2.

### Example 156. Synthesis of compound 670.

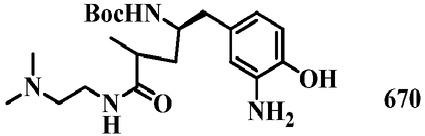

Pd/C (0.2 g, 10% Pd/C, 50% wet) was added to a solution of compound **669** (0.60 g, 13.7 mmol, 1.0 eq.) in EA (10 mL). The mixture was shaken at 100 psi H₂ atmosphere for 4 h. Then the mixture was filtered over Celite and the filtrate was concentrated to give the title compound as green oil (5.50 g, 98% yield). ESI m/z calcd for C₂₁H₃₇N₄O₆₄ [M+H]⁺: 409.3, found:409.3.

### Example 157. Synthesis of compound 671.

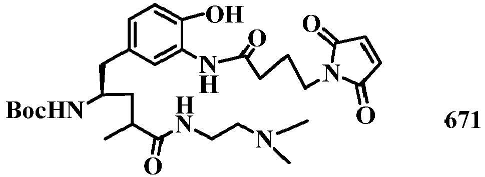

To a solution of compound **670** (0.50 g, 1.22 mmol, 1.0 eq.) in 95% EtOH (10 mL) and 0.1M NaH₂PO₄ (2 mL) was added compound **125** (0.683 g, 2.44 mmol, 2.0 eq.) and the reaction was stirred overnight and then concentrated and purified by SiO₂ column chromatography (DCM/MeOH) to give the title compound as a light yellow oil (0.624 g, 89% yield). ESI m/z calcd for C₂₉H₄₄N₅O₇ [M+H]⁺: 574.3, found: 574.3.

### Example 158. Synthesis of compound 672.

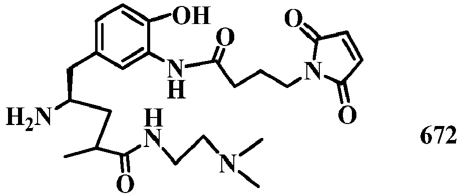

To a solution of compound **671** (0.20 g, 0.349 mmol, 1.0eq) in DCM (6.0 mL) at r.t. was added TFA (2.0 mL) and the reaction was stirred for 2 h, then diluted with anhydrous toluene and concentrated, this operation was repeated for three times to give the title compound as a yellow oil (165 mg, theoretical yield). ESI m/z calcd for C₂₄H₃₆N₅O₅ [M+H]⁺: 474.3, found: 474.3.

### Example 159. Synthesis of compound 673.

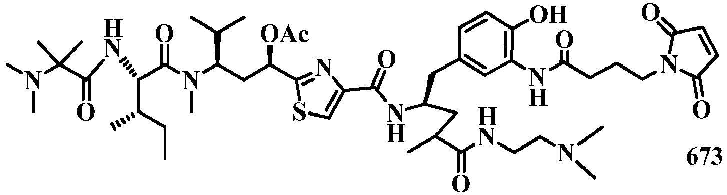

To a solution of compound **672** (0.165 g, 0.349 mmol,1.0 eq.) in anhydrous DMF (2 mL) at 0 °C was added compound **41a** (0.290 g, 1.047 mmol, 1.2 eq.) in anhydrous DMF (3 mL) and the reaction was stirred for 10 minutes, then warmed to r.t. and stirred for 1 h. The reaction mixture was concentrated and purified on prep-HPLC (C₁₈ column, mobile phase A: water, mobile phase B: acetonitrile, from 10% of B to 80% of B in 60 min). The fractions were pooled and lyophilized to give the title compound (58 mg, 17% yield) as a light yellow foamy solid. ESI m/z calcd for C₄₉H₇₆N₉O₁₀S [M+H]⁺: 982.5, found: 982.5.

### Example 160. Synthesis of compound 704.

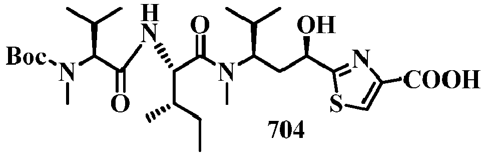

(S)-2-((tert-butoxycarbonyl)(methyl)amino)-3-methylbutanoic acid (33 mg, 0.14 mmol), DCC (32 mg, 0.154 mmol) and pentafluorophenol (39 mg, 0.21 mmol) were dissolved in ethyl acetate (20 mL) and the reaction was stirred at room temperature overnight. The reaction was then concentrated to dryness to give compound (S)-perfluorophenyl 2-((tert-butoxycarbonyl) (methyl)amino)-3-methylbutanoate, which was dissolved in 2 mL of DMA, and a solution of compound 2-((1R,3R)-3-((2S,3S)-2-amino-N,3-dimethylpentanamido)-1-hydroxy-4-methylpentyl)thiazole-4-carboxylic acid (52 mg, 0.14 mmol) in 3 mL of DMA and DIPEA (48.5 µL, 0.28mmol) were added. The reaction was stirred at room temperature overnight and then concentrated. The residue was diluted with 1 mL of acetonitrile and purified by reverse phase HPLC with a gradient of MeCN/H₂O to afford compound **704** (40.2 mg, 49% yield) . ESI: m/z: calcd for C₂₈H₄₉N₄O₇S [M+H]⁺: 585.32, found 585.32.

### Example 161. Synthesis of compound 705.

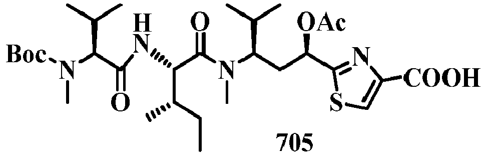

To a solution of compound **704** (40 mg, 0.069 mmol) in pyridine (8 mL) at 0 °C was added acetic anhydride (20.4 mg, 0.2 mmol), and the reaction was warmed to room temperature and stirred overnight, then concentrated. The residue was purified by column chromatography (MeOH / DCM) to afford the title compound **705** (48.1 mg, ∼100% yield). ESI: m/z: calcd for C₃₀H₅₁N₄O₈S [M+H]⁺: 627.33, found 627.33.

### Example 162. Synthesis of compound 708.

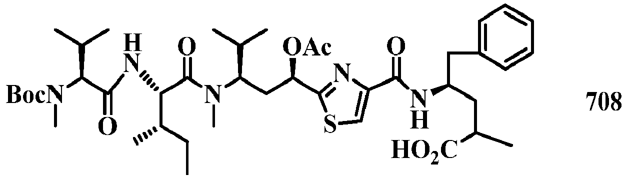

Compound **705** (48.1 mg, 0.077 mmol) DCC (17.4 mg, 0.085 mmol) and pentafluorophenol (21.2 mg, 0.115 mmol) were dissolved in ethyl acetate (10 mL) and the reaction was stirred overnight at room temperature, then concentrated to dryness to give compound perfluorophenyl 2-((6S,9S,12R,14R)-9-((S)-sec-butyl)-6,12-diisopropyl-2,2,5,11-tetramethyl-4,7,10,16-tetraoxo-3,15-dioxa-5,8,11-triazaheptadecan-14-yl)thiazole-4-carboxylate, which was dissolved in 4 mL of DMA, and a solution of compound (4R)-4-amino-2-methyl-5-phenylpentanoic acid, trifluoroacetic acid salt (20.7 mg, 0.1 mmol) in 3 mL of DMA and DIPEA (26.8 µL, 0.154 mmol) were added. The reaction was stirred at room temperature overnight and then concentrated. The residue was diluted with 1 mL of acetonitrile and purified by reverse phase HPLC with a gradient of MeCN/H₂O to afford compound **708** (33 mg, 84% yield). ESI: m/z: calcd for C₄₂H₆₆N₅O₉S [M+H]⁺: 816.45, found 816.45.

### Example 163. Synthesis of compound 709.

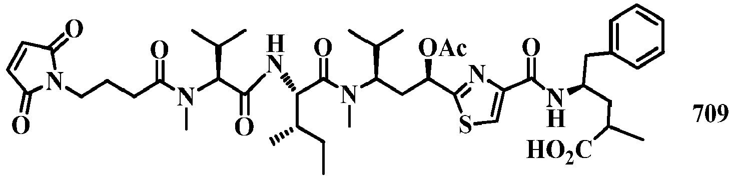

Compound **708** from previous step was dissolved in DCM (1 mL) and treated with TFA (1 mL) at r.t. for 2 h. The reaction was concentrated and the residue was dissolved in EtOH (20 mL) . Compound **125** (30.8 mg, 0.11 mmol) and 0.1 M NaH₂PO₄ (4 mL) were added and the resulting mixture was stirred at r.t. overnight, then concentrated and the residue was purified by column chromatography (MeOH / DCM) to afford the title compound **709** (28.5 mg, 42% yield). ESI m/z: calcd for C₄₅H₆₅N₆O₁₀S [M+H]⁺: 881.44, found 881.44.

### Example 164. Synthesis of compound 712.

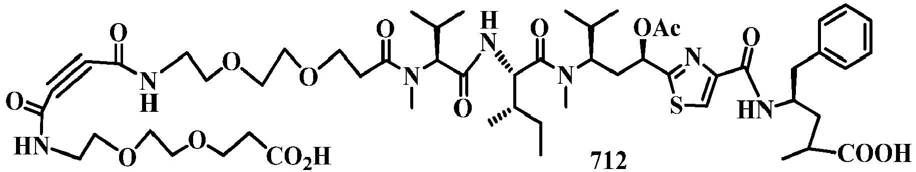

To a solution of compound **708** (63 mg, 0.077 mmol) in DCM (1 mL) was treated with TFA (1 mL) at room temperature for 2 h, then concentrated and the residue was dissolved in DMA (4 mL). Compound **711** (65.8 mg, 0.11 mmol) and DIPEA (27 µL, 0.154mmol) were added and the reaction was stirred at room temperature overnight, then concentrated and the residue was purified by reverse phase HPLC with a gradient of MeCN/H₂O to afford compound **712** (14 mg, 16% yield). ESI: m/z: calcd for C₅₅H₈₄N₇O₁₆S [M+H]⁺: 1130.56, found 1130.57.

### Example 165. Synthesis of tert-butyl 17-(2,5-dioxo-2,5-dihydro-1H-pyrrol-1-yl)-14-oxo-4,7,10-trioxa-13-azaheptadecan-1-oate (compound 157a).

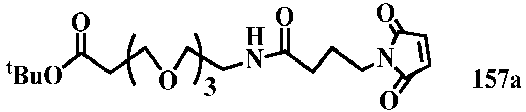

Compound **444** (27.92 g, 0.1 mol) and compound **124** (18.3 g, 0.1 mol) was dissolved in DCM (300 mL), to which DIPEA (12.9 g, 0.1 mol) and EDC (38.6 g, 0.2 mol) were added. The resulting solution was stirred at r.t. overnight and then washed with brine, dried over Na₂SO₄. Filtration, concentration and purification by column chromatography (5% EtOAc/DCM to 20% EtOAc/DCM) yielded compound **157a** (38.03 g, 86% yield). MS ESI m/z calcd for C₂₁H₃₅N₂O₈ [M+H]⁺ 443.2394, found 443.2412.

### Example 166. Synthesis of 17-(2,5-dioxo-2,5-dihydro-1H-pyrrol-1-yl)-14-oxo-4, 7,10-trioxa-13-azaheptadecan-1-oic acid (compound 158a).

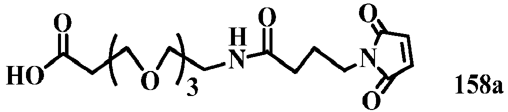

To a solution of compound **157a** (38.0 g, 85.9 mmol) in 150 mL DCM was added 50 mL TFA. The reaction mixture was stirred at r.t. for 1h, and then diluted with toluene (50 ml), concentrated in vacuo and purification by column chromatography (10% MeOH/DCM to 20% MeOH/DCM) yielded compound **158a** (27.53 g, 83% yield). MS ESI m/z calcd for C₁₇H₂₇N₂O₈ [M+H]⁺ 387.1768, found 387.1792.

### Example 167. Synthesis of 2,5-dioxopyrrolidin-1-yl 17-(2,5-dioxo-2,5-dihydro-1H-pyrrol-1-yl)-14-oxo-4,7,10-trioxa-13-azaheptadecan-1-oate (compound 159a).

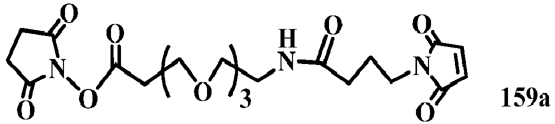

NHS (3.20 g, 27.82 mmol) and EDCI (9.70 g, 50.4 mmol) were added to a solution of compound **158a** (10.10 g, 26.15 mmol) in DCM (80 mL). After stirring at r.t. overnight, the reaction mixture was concentrated and purified by column chromatography (5 to 20% EtOAc/DCM) to afford compound **159a** (10.73 g, 85% yield). MS ESI m/z calcd for C₂₁H₃₀N₃O₁₀ [M+H]⁺ 484.1942, found 484.1978.

### Example 168. Synthesis of (2S,SS)-23-(2,5-dioxo-2,5-dihydro-1H-pyrrol-1-yl)-2,5-dimethyl-4,7,20-trioxo-10,13,16-trioxa-3,6,19-triazatricosan-1-oic acid (compound 162a).

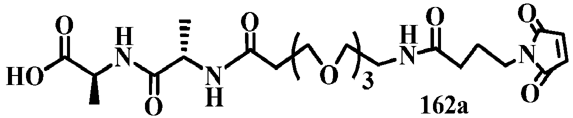

Compound **161** ((S)-Ala-(S)-Ala, 5.01 g, 31.2 mmol) in the mixture of ethanol (100 ml) and pH 7.5 buffer (0.1 M NaH₂PO₄/Na₂HPO₄, 100 ml), was added compound 159a (15.08 g, 31.20 mmol) in four portions in 2 hours. After addition, the mixture was continued to stir for 4 hours, concentrated in vacuo, and purified by column chromatography (10 to 20% MeOH/DCM) to afford compound **162a** (13.18 g, 80% yield). MS ESI m/z calcd for C₂₃H₃₇N₄O₁₀ [M+H]⁺ 529.2511, found 529.2545.

### Example 169. Synthesis of (2S,5S)-2,5-dioxopyrrolidin-1-yl 23-(2,5-dioxo-2,5-dihydro-1H-pyrrol-1-yl)-2,5-dimethyl-4,7,20-trioxo-10,13,16-trioxa-3,6,19-triazatricosan-1-oate (163a).

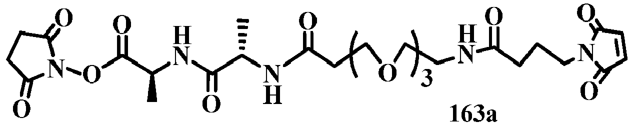

NHS (1.60 g, 13.91 mmol) and EDCI (5.90 g, 30.7 mmol) were added to a solution of compound **162a** (6.50 g, 12.30 mmol) in DCM (70 mL). After stirring at r.t. overnight, the reaction mixture was concentrated and purified by column chromatography (5 to 20% EtOAc/DCM) to afford compound **163a** (6.61 g, 86% yield). MS ESI m/z calcd for C₂₇H₄₀N₅O₁₂ [M+H]⁺ 626.2672, found 626.2698.

### Example 170. Synthesis of (2S,4R)-4-((tert-butoxycarbonyl)amino)-5-(3-((2S,5S)-23-(2,5-dioxo-2,5-dihydro-1H-pyrrol-1-yl)-2,5-dimethyl-4,7,20-trioxo-10,13,16-trioxa-3,6,19-triazatricosanamido)-4-hydroxyphenyl)-2-methylpentanoic acid (164a).

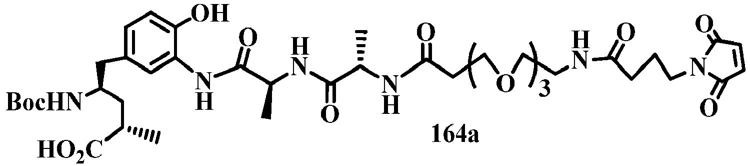

Compound **121** (3.57 g, 10.55 mmol) in the mixture of ethanol (70 ml) and pH 7.5 buffer (0.1 M NaH₂PO₄/Na₂HPO₄, 60 ml), was added compound **163a** (6.60 g, 10.55 mmol) in four portions in 2 hours. After addition, the mixture was continued to stir for 4 hours, concentrated in vacuo, and purified by column chromatography (15 to 25% MeOH/DCM) to afford compound **164a** (7.25 g, 81% yield). MS ESI m/z calcd for C₄₀H₆₁N₆O₁₄ [M+H]⁺ 849.4267, found 849.4295.

### Example 171. Synthesis of (2R,4S)-4-carboxy-1-(3-((2S,5S)-23-(2,5-dioxo-2,5-dihydro-1H-pyrrol-1-yl)-2,5-dimethyl-4,7,20-trioxo-10,13,16-trioxa-3,6,19-triazatricosanamido)-4-hydroxyphenyl)pentan-2-aminium, trifluoroacetate (165a).

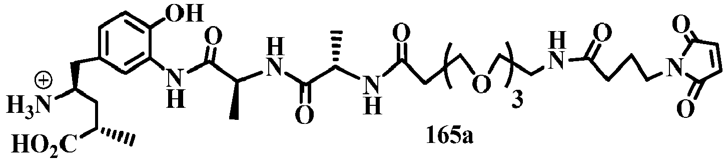

To a solution of compound **164a** (0.20 g, 0.349 mmol, 1.0eq) in DCM (6.0 mL) at r.t. was added TFA (2.0 mL) and the reaction was stirred for 2 h, then diluted with anhydrous toluene and concentrated, this operation was repeated for three times to give the title compound as a yellow oil (165 mg, theoretical yield) for the next step without further purification. ESI m/z calcd for C₃₅H₅₄N₆O₁₂ [M+H]⁺: 750.3795, found: 750.3825.

### Example 172. Synthesis of (2S,4R)-4-(2-((6S,9R,11R)-6-((S)-sec-butyl)-9-isopropyl-2,3,3,8-tetramethyl-4,7,13-trioxo-12-oxa-2,5,8-triazatetradecan-11-yl)thiazole-4-carboxamido)-5-(3-((2S,5S)-23-(2,5-dioxo-2,5-dihydro-1H-pyrrol-1-yl)-2,5-dimethyl-4,7,20-trioxo-10,13,16-trioxa-3,6,19-triazatricosanamido)-4-hydroxyphenyl)-2-methylpentanoic acid (166a).

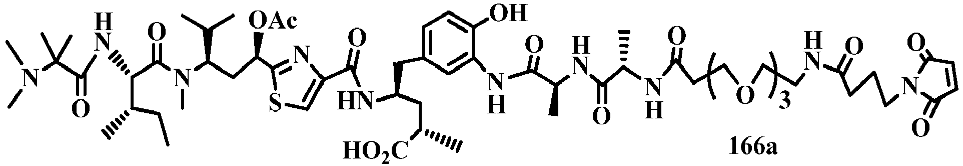

Compound **165a** (45 mg, 0.0600 mmol) and perfluorophenyl ester **41a** (45 mg, 0.0650 mmol) were dissolved in DMA (5 mL). To the mixture, DIPEA (20 mg, 0.154 mmol) was added. The resulting mixture was stirred at r.t. overnight, concentrated and purified by preparative HPLC (C₁₈ column, 10-90% MeCN/H₂O) to afford compound **166a** (49.1 mg, 65% yield). ESI m/z calcd for C₆₀H₉₃N₁₀O₁₇S [M+H]⁺: 1256.6442, found: 1256.6510.

### Example 173. Synthesis of perfluorophenyl 2-methyl-2-(pyrrolidin-1-yl)propanoate 713.

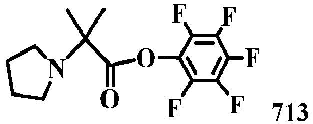

2-Methyl-2-(pyrrolidin-1-yl)propanoic acid **25** (401 mg, 2.50 mmol), EDC (654 mg, 3.40 mmol) and pentafluorophenol (480 mg, 2.60 mmol) were dissolved in dichloromethane (45 mL) and the reaction was stirred overnight at room temperature, then concentrated to dryness to give compound perfluorophenyl 2-methyl-2-(pyrrolidin-1-yl)propanoate 713 (662 mg, 82% yield). MS ESI m/z calcd for C₁₄H₁₅F₅NO₂ [M+H]⁺ 324.1024, found 324.1045.

### Example 174. Synthesis of ethyl 2-((5R,7R,10S)-10-((S)-sec-butyl)-3,3-diethyl-7-isopropyl-8,13-dimethyl-9,12-dioxo-13-(pyrrolidin-1-yl)-4-oxa-8,11-diaza-3-silatetradecan-5-yl)thiazole-4-carboxylate 714.

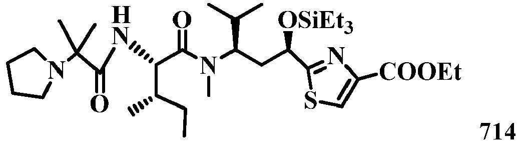

To the EtOAc solution (40 ml) of pentafluorophenyl ester **713** (650 mg, 2.01 mmol), compound **16** (1.085 g, 2.01 mmol) and dry Pd/C (10 wt%, 100 mg) were added. The reaction mixture was stirred under hydrogen atmosphere (1 atm) for 24 h, and then filtered through a plug of Celite, with washing of the filter pad with EtOAc. The combined organic portions were concentrated and purified by column chromatography with a gradient of 0-5% methanol in CH₂Cl₂ to deliver compound **714** (1.10 g, 84% yield). MS ESI m/z calcd for C₃₃H₆₁N₄O₅SSi [M+H]⁺ 653.4133, found 653.4148.

### Example 175. Synthesis of ethyl 2-((1R,3R)-3-((2S,3S)-N,3-dimethyl-2-(2-methyl-2-(pyrrolidin-1-yl)propanamido)pentanamido)-1-hydroxy-4-methylpentyl)thiazole-4-carboxylate, 715.

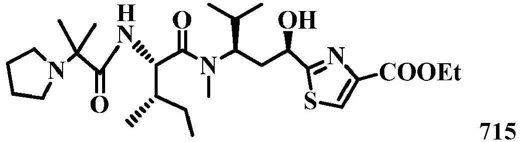

Compound **714** (1.10 g, 1.68 mmol) was dissolved in AcOH/water/THF (v/v/v 3:1:1, 25 mL), and stirred at r.t. for 2 days. After the reaction was concentrated, toluene was added and concentrated again; this step was repeated two times to afford compound **715,** which was used directly in the next step. MS ESI m/z calcd for C₂₇H₄₇N₄O₅S [M+H]⁺ 539.3268, found 539.3295.

### Example 176. Synthesis of 2-((1R,3R)-3-((2S,3S)-N,3-dimethyl-2-(2-methyl-2-(pyrrolidin-1-yl)propanamido)pentanamido)-1-hydroxy-4-methylpentyl)thiazole-4-carboxylic acid, 716.

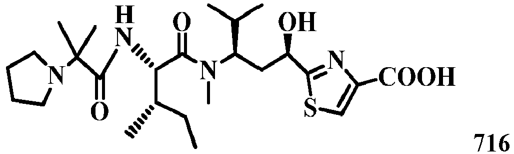

An aqueous solution of LiOH (0.4 N, 5 mL) was added to a solution of compound **715** (0.85 g, 1.65 mmol) in MeOH (20 mL) at 0 °C. The reaction mixture was stirred at r.t. for 2 h and then concentrated. Column chromatography (pure CH₂Cl₂ to 80:20:1 CH₂Cl₂/MeOH/NH₄OH) afforded compound **716** (773 mg, 90% yield for two steps) as an amorphous solid. MS ESI m/z calcd for C₂₅H₄₃N₄O₅S [M+H]⁺ 511.2955, found 511.2980.

### Example 177. Synthesis of 2-((1R,3R)-1-acetoxy-3-((2S,3S)-N,3-dimethyl-2-(2-methyl-2-(pyrrolidin-1-yl)propanamido)pentanamido)-4-methylpentyl)thiazole-4-carboxylic acid, 717.

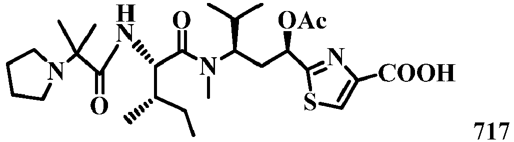

A solution of compound **716** (765 mg, 1.50 mmol) and DMAP (180 mg, 1.48 mmol,) in anhydrous THF (30 mL) and anhydrous DMF (15 mL) was cooled to 0 °C, to which DIPEA (3.0 mL, 17.2 mmol) and acetic anhydride (1.0 g, 9.79 mmol) were added. The reaction mixture was allowed to warm to r.t. and stirred for 4 h, and then concentrated. Column chromatography (5-50% MeOH/DCM) delivered compound **717** (785 mg, 95% yield) as an amorphous solid. MS ESI m/z calcd for C₂₇H₄₅N₄O₆S [M+H]⁺ 553.3061, found 553.3095.

### Example 178. Synthesis of perfluorophenyl 2-((1R,3R)-1-acetoxy-3-((2S,3S)-N,3-dimethyl-2-(2-methyl-2-(pyrrolidin-1-yl)propanamido)pentanamido)-4-methylpentyl)thiazole-4-carboxylate, 718.

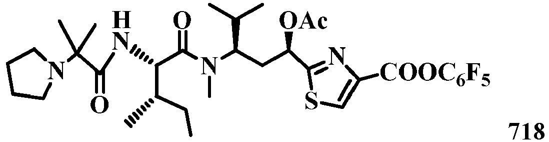

To a solution of compound **717** (775 mg, 1.40 mmol) in anhydrous DCM (10 mL) was added EDC (805 mg, 4.19 mmol) and pentafluorophenol (276 mg, 1.50 mmol) at room temperature under N₂. The mixture was stirred at room temperature for 4 h, and then diluted in DCM (100 mL), washed with water (2 × 200 mL) and brine (200 mL), dried over anhydrous sodium sulfate, filtered, concentrated and purified by SiO₂ column chromatography (50% EtOAc/PE) to give compound **718** as a white solid (815 mg, 81% yield) MS ESI m/z calcd for C₃₃H₄₄F₅N₄O₆S [M+H]⁺: 719.2901, found: 719.2945.

### Example 179. Synthesis of (2S,4R)-4-(2-((1R,3R)-1-acetoxy-3-((2S,3S)-N,3-dimethyl-2-(2-methyl-2-(pyrrolidin-1-yl)propanamido)pentanamido)-4-methylpentyl)thiazole-4-carboxamido)-5-(3-((2S,5S)-23-(2,5-dioxo-2,5-dihydro-1H-pyrrol-1-yl)-2,5-dimethyl-4,7,20-trioxo-10,13,16-trioxa-3,6,19-triazatricosanamido)-4-hydroxyphenyl)-2-methylpentanoic acid.

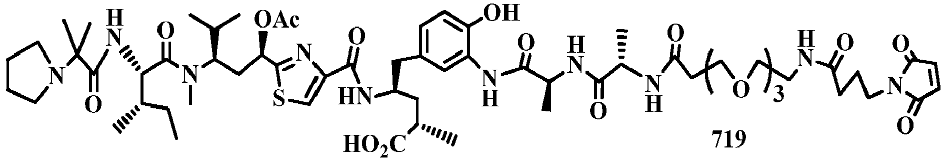

In a solution of compound **718** (153 mg, 0.213 mmol) and compound **165a** (160 mg, 0.213 mmol) in 7 mL of DMA, DIPEA (100 µL, 0.575 mmol) were added. The reaction was stirred at room temperature overnight and then concentrated. The residue was diluted with 1 mL of acetonitrile and purified by reverse phase HPLC with a gradient of MeCN/H₂O (10% MeCN to 40% MeCN in 45 min, d2 cm x L25 cm, C-18 column, 8 ml/min) to afford compound **719** (166.1 mg, 61% yield). ESI: m/z: calcd for C₆₂H₉₅N₁₀O₁₇S [M+H]⁺: 1282.6598, found 1282.6630.

### Example 180. Synthesis of (2S,4R)-4-(2-((1R,3R)-1-acetoxy-3-((2S,3S)-N,3-dimethyl-2-((R)-1-methylpiperidine-2-carboxamido)pentanamido)-4-methylpentyl)thiazole-4-carboxamido)-5-(3-((2S,5S)-23-(2,5-dioxo-2,5-dihydro-1H-pyrrol-1-yl)-2,5-dimethyl-4,7,20-trioxo-10,13,16-trioxa-3,6,19-triazatricosanamido)-4-hydroxyphenyl)-2-methylpentanoic acid, 720.

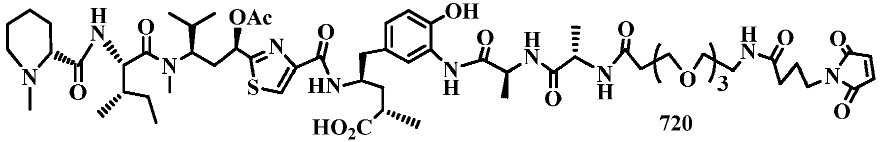

Compound **33** (30.2 mg, 0.056 mmol), EDC (25.0 mg, 0.130 mmol) and pentafluorophenol (11 mg, 0.060 mmol) were dissolved in dichloromethane (4 mL) and the reaction was stirred overnight at room temperature, then concentrated to dryness to give compound perfluorophenyl 2-((1R,3R)-1-acetoxy-3-((2S,3S)-N,3-dimethyl-2-((R)-1-methylpiperidine-2-carboxamido)pentanamido)-4-methylpentyl)thiazole-4-carboxylate, **33a,** which was dissolved in 4 mL of DMA, and a solution of compound **165a** (160 mg, 0.213 mmol) in 3 mL of DMA and DIPEA (26.8 µL, 0.154 mmol) were added. The reaction was stirred at room temperature overnight and then concentrated. The residue was diluted with 1 mL of acetonitrile and purified by reverse phase HPLC with a gradient of MeCN/H₂O (10% MeCN to 40% MeCN in 45 min, d2 cm x L25 cm, C-18 column, 8 ml/min) to afford compound **720** (133.1 mg, 48% yield). ESI: m/z: calcd for C₆₁H₉₃N₁₀O₁₇S [M+H]⁺: 1269.6442, found 1282.6630.

### Example 181. Synthesis of (2S,4R)-tert-butyl 4-((tert-butoxycarbonyl)amino)-5-(4-((2S,5S)-23-(2,5-dioxo-2,5-dihydro-1H-pyrrol-1-yl)-2,5-dimethyl-4,7,20-trioxo-10,13,16-trioxa-3,6,19-triazatricosanamido)phenyl)-2-methylpentanoate, 721.

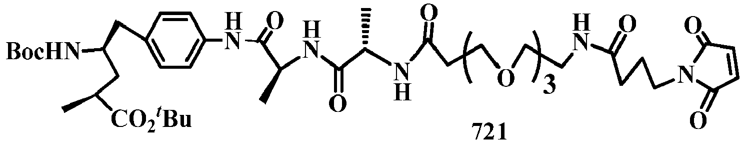

Compound **488** (310 mg, 0.82 mmol) in the mixture of ethanol (70 ml) and pH 7.5 buffer (0.1 M NaH₂PO₄/Na₂HPO₄, 60 ml), was added compound **163a** (660 mg, 1.055 mmol) in four portions in 2 hours. After addition, the mixture was continued to stir for 4 hours, concentrated in vacuo, and purified by column chromatography (15 to 25% EtOAc/DCM) to afford compound **714** (604,5 mg, 83% yield). MS ESI m/z calcd for C₄₄H₆₉N₆O₁₃ [M+H]⁺ 889.4923, found 889.4965.

### Example 182. Synthesis of (2S,4R)-4-amino-5-(4-((2S,5S)-23-(2,5-dioxo-2,5-dihydro-1H-pyrrol-1-yl)-2,5-dimethyl-4,7,20-trioxo-10,13,16-trioxa-3,6,19-triazatricosanamido)phenyl)-2-methylpentanoic acid, trifluoroacetic acid salt.

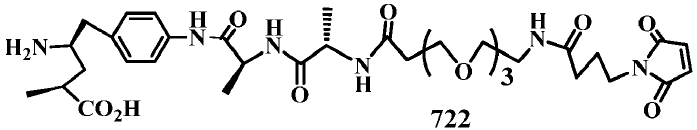

To a solution of compound **721** (0.20 g, 0.225 mmol, 1.0eq) in DCM (6.0 mL) at r.t. was added TFA (2.0 mL) and the reaction was stirred for 2 h, then diluted with anhydrous toluene and concentrated, this operation was repeated for three times to give the title compound as a yellow oil (165 mg, theoretical yield) for the next step without further purification. ESI m/z calcd for C₃₅H₅₃N₆O₁₁ [M+H]⁺: 732.3773, found: 732.3795.

### Example 183. Synthesis of (2S,4R)-4-(2-((1R,3R)-1-acetoxy-3-((2S,3S)-N,3-dimethyl-2-((R)-1-methylpiperidine-2-carboxamido)pentanamido)-4-methylpentyl)thiazole-4-carboxamido)-5-(4-((2S,5S)-23-(2,5-dioxo-2,5-dihydro-1H-pyrrol-1-yl)-2,5-dimethyl-4,7,20-trioxo-10,13,16-trioxa-3,6,19-triazatricosanamido)phenyl)-2-methylpentanoic acid (723, as a reference control).

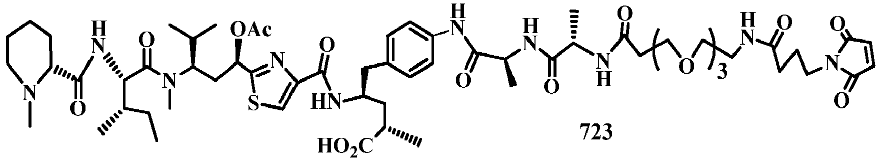

Compound **33** (30.2 mg, 0.056 mmol), EDC (25.0 mg, 0.130 mmol) and pentafluorophenol (11 mg, 0.060 mmol) were dissolved in dichloromethane (4 mL) and the reaction was stirred overnight at room temperature, then concentrated to dryness to give compound perfluorophenyl 2-((1R,3R)-1-acetoxy-3-((2S,3S)-N,3-dimethyl-2-((R)-1-methylpiperidine-2-carboxamido)pentanamido)-4-methylpentyl)thiazole-4-carboxylate, which was dissolved in 4 mL of DMA, and a solution of compound **722** (160 mg, 0.213 mmol) in 3 mL of DMA and DIPEA (26.8 µL, 0.154 mmol) were added. The reaction was stirred at room temperature overnight and then concentrated. The residue was diluted with 1 mL of acetonitrile and purified by reverse phase HPLC with a gradient of MeCN/H₂O (10% MeCN to 40% MeCN in 45 min, d2 cm x L25 cm, C-18 column, 8 ml/min) to afford compound **723** (47.7 mg, 68% yield). ESI: m/z: calcd for C₆₁H₉₃N₁₀O₁₆S [M+H]⁺: 1253.6492, found 1253.6540.

### Example 184. General method of preparation of antibody conjugates of compounds 128, 132, 437, 481, 495, 528, 629, 633, 641, 645, 649, 654, 659, 663, 673, 709, 712, 166a, 719, 720 and 723.

To a mixture of 2.0 mL of 10 mg/ml Herceptin in pH 6.0∼8.0, were added of 0.70∼2.0 mL PBS buffer of 100 mM NaH₂PO₄, pH 6.5∼8.5 buffers, TCEP (14-35 µL, 20 mM in water) and the compounds 128, 132, 437, 481, 495, 528, 629, 633, 641, 645, 649, 654, 659, 663, 673, 709, 712, 166a, 719, 720 and 723 (14-28 µL, 20 mM in DMA independently, followed by addition of 4-(azidomethyl)benzoic acid (14-50µL, 20 mM in pH 7.5, PBS buffer). The mixture was incubated at RT for 4∼18 h, then DHAA (135 µL, 50 mM) was added in. After continuous incubation at RT overnight, the mixture was purified on G-25 column eluted with 100 mM NaH₂PO₄, 50 mM NaCl pH 6.0∼7.5 buffer to afford 12.2∼18.6 mg of the conjugate compounds C-128, C-132, C-437, C-481, C-495, C-528, C-629, C-633, C-641, C-644, C-645, C-649, C-654, C-659, C-663, C-673, C-709, C-712, C-166a, C-719, C-720 and C-723, (80%∼93% yield) accordingly in 13.4∼15.8 ml of the NaH₂PO₄ buffer. The drug/antibody ratio (DAR) was 3.4∼3.9 for the conjugates, wherein DAR was determined via UPLC-QTOF mass spectrum. It was 94∼99% monomer analyzed by SEC HPLC (Tosoh Bioscience, Tskgel G3000SW, 7.8 mm ID x 30 cm, 0.5 ml/min, 100 min) and a single band measured by SDS-PAGE gel.

### Example 185. In vitro cytotoxicity evaluation of conjugate C-128, C-132, C-437, C-481, C-495, C-528, C-629, C-633, C-641, C-644, C-645, C-649, C-654, C-659, C-663, C-673, C-709, C-712, C-166a, C-719, C-720 and C-723, in comparison with T-DM1.

The cell line used in the cytotoxicity assays was NCI-N87, a human gastric carcinoma cell line; the cells were grown in RPMI-1640 with 10% FBS. To run the assay, the cells (180 µl, 6000 cells) were added to each well in a 96-well plate and incubated for 24 hours at 37°C with 5% CO₂. Next, the cells were treated with test compounds (20 µl) at various concentrations in appropriate cell culture medium (total volume, 0.2 mL). The control wells contain cells and the medium but lack the test compounds. The plates were incubated for 120 hours at 37°C with 5% CO₂. MTT (5mg/ml) was then added to the wells (20 µl) and the plates were incubated for 1.5hr at 37°C. The medium was carefully removed and DMSO (180 µl) was added afterward. After it was shaken for 15min, the absorbance was measured at 490 nm and 570 nm with a reference filter of 620 nm. The inhibition% was calculated according to the following equation: inhibition% = [1-(assay-blank)/(control-blank)] × 100. The results are listed in Table 1.

**Table 1. The Structures of the Her2-tubulysin analog conjugates of the patent application along with their results of the IC₅₀ cytotoxicity against NCI-N87 cells:**

| Compound # | Structures and its IC50 against NCI-N87 cells |
|---|---|
| 129 (C-128) | 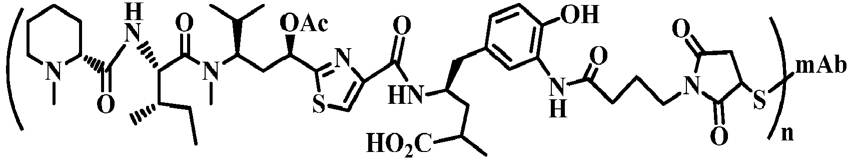 |
| | IC₅₀=0.14 nM, (n = 3.5) |
| 133 (C-132) | 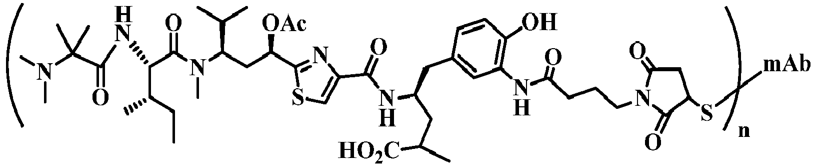 |
| | IC₅₀=0.17 nM, (n = 3.6). |
| C-437 | 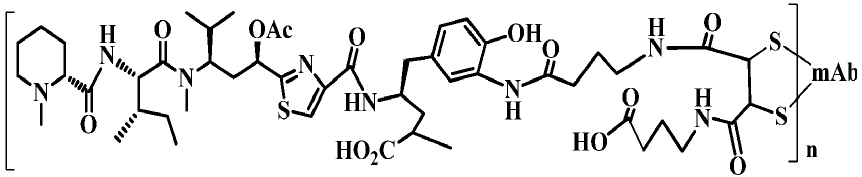 |
| | IC₅₀ =3.67 nM, (n = 3.8). |
| C-481 | 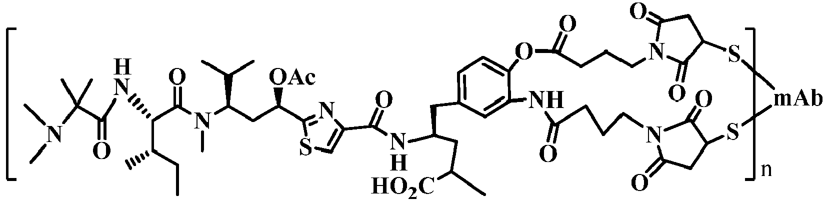 |
| | IC₅₀ = 0.73 nM, (n = 3.8). |
| C-495 | 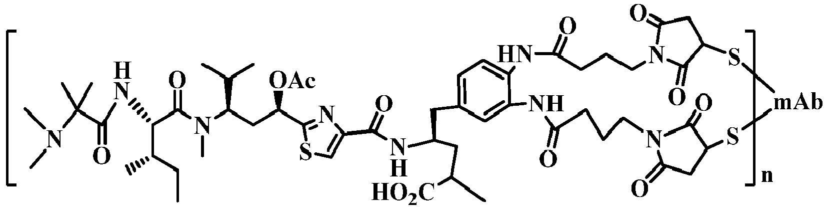 |
| | IC₅₀ = 13.06 nM, (n = 3.9). |
| C-528 | 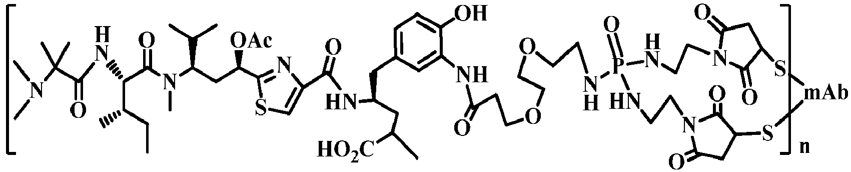 |
| | IC₅₀ = 1.35 nM, (n = 3.8). |
| C-629 | 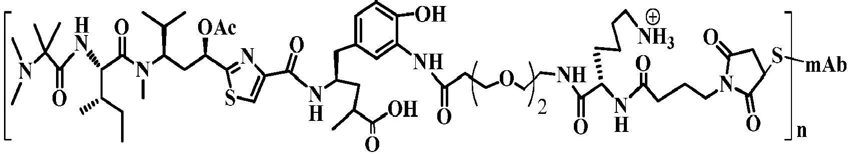 |
| | IC₅₀ =0.18 nM, (n = 3.7). |
| C-633 | 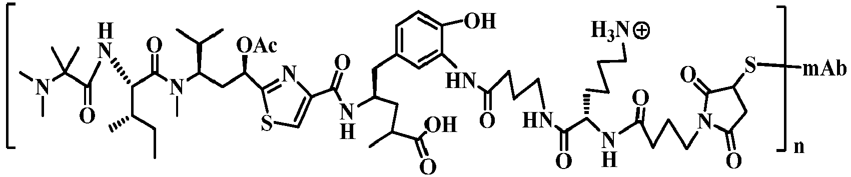 |
| | IC₅₀ = 0.11 nM, (n = 3.6). |
| C-641 | 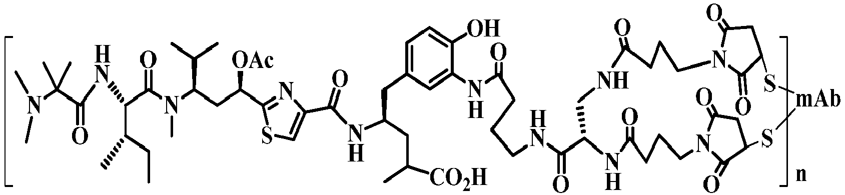 |
| | IC₅₀ = 0.15 nM, (n = 3.6). |
| C-645 | 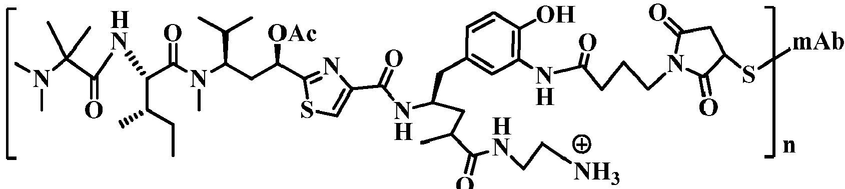 |
| | IC₅₀ = 3.56 nM, (n = 3.8). |
| C-649 | 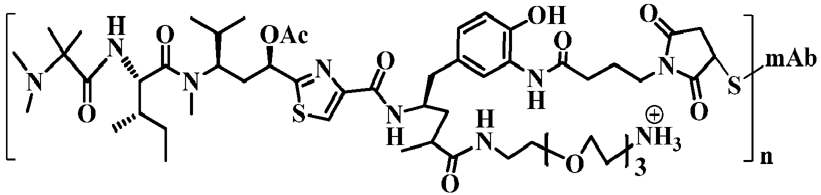 |
| | IC₅₀ =9.01 nM, (n = 3.8). |
| C-654 | 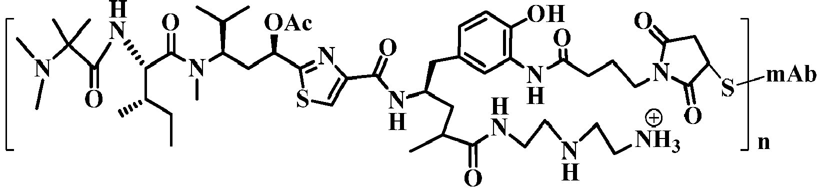 |
| | IC₅₀=3.51nM, (n=3.6). |
| C-659 | 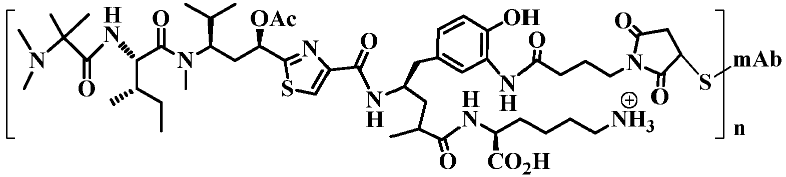 |
| | IC₅₀ =2.3 nM, (n = 3.8). |
| C-663 | 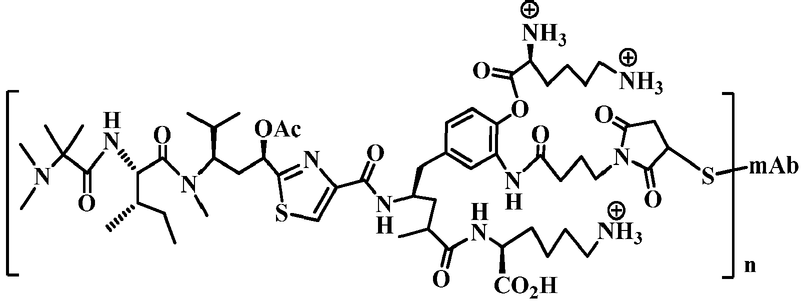 |
| | IC₅₀ =4.21nM, (n = 3.8). |
| C-673 | 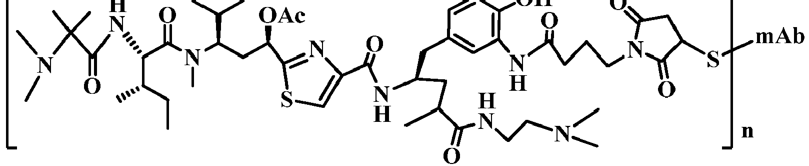 |
| | IC₅₀ = 1.35 nM, (n = 3.7). |
| C-709 | 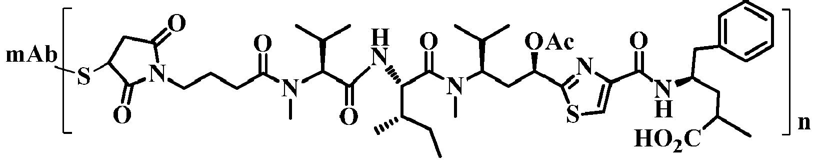 |
| | IC₅₀ =9.6 nM, (n = 3.6). |
| C-712 | 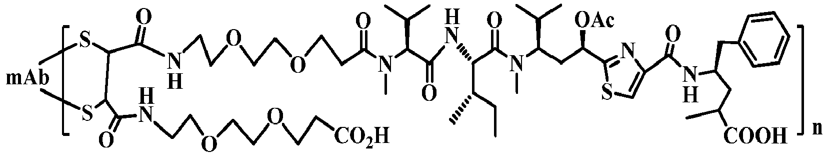 |
| | IC₅₀ =11.2 nM, (n = 3.6). |
| C-166a | 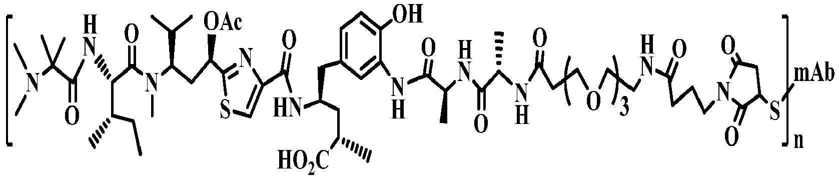 |
| | IC₅₀=0.12 nM, (n = 3.6). |
| C-719 | 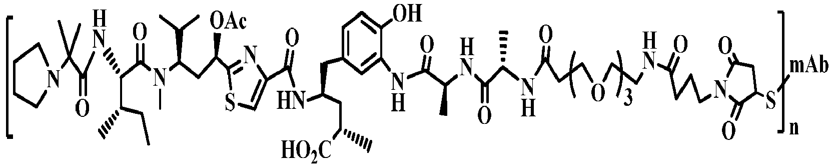 |
| | IC₅₀=0.10 nM, (n = 3.6). |
| C-720 | 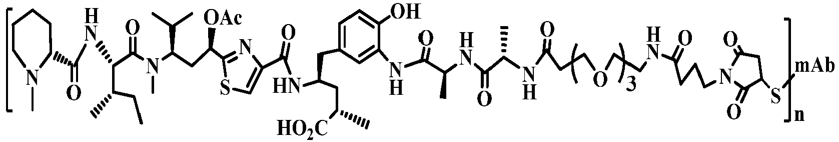 |
| | IC₅₀ =0.08 nM, (n = 3.6). |
| C-723 | 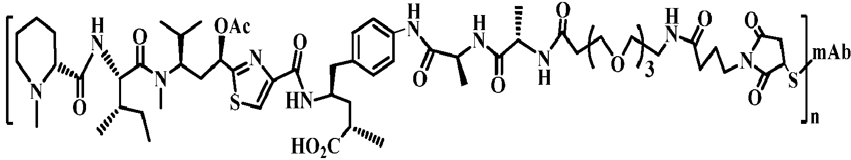 |
| | IC₅₀=0.07 nM, (n = 3.6). |

### Example 186. Antitumor Activity In vivo (BALB/c Nude Mice bearing NCI-N87 Xenograft tumor).

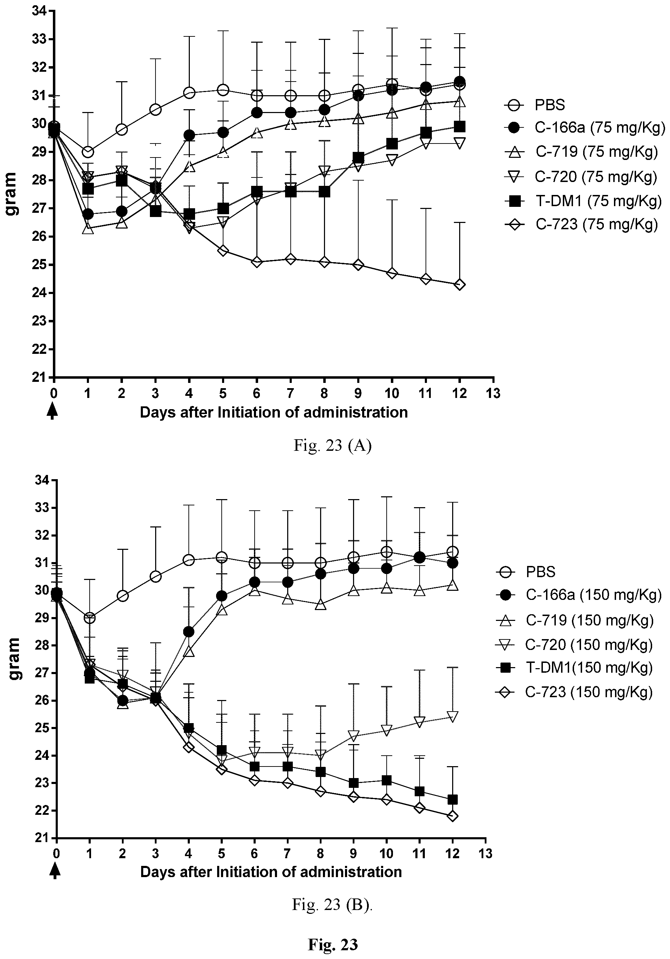

The in vivo efficacy of conjugates C-166a, C-719, C-720, C-723 along with T-DM1 were evaluated in a human gastric carcinoma N-87 cell line tumor xenograft models. Five-week-old female BALB/c Nude mice (40 animals) were inoculated subcutaneously in the area under the right shoulder with N-87 carcinoma cells (5 × 10⁶ cells/mouse) in 0.1mL of serum-free medium. The tumors were grown for 7 days to an average size of 125 mm³. The animals were then randomly divided into 6 groups (6 animals per group). The first group of mice served as the control group and was treated with the phosphate-buffered saline (PBS) vehicle. Five groups were treated with conjugates C-166a, C-719, C-720, C-723 and T-DM1 respectively at dose of 6 mg/Kg administered intravenously. Three dimensions of the tumor were measured every 3 or 4 days (twice a week) and the tumor volumes were calculated using the formula tumor volume =1/2(length × width × height). The weight of the animals was also measured at the same time. A mouse was sacrificed when any one of the following criteria was met: (1) loss of body weight of more than 20% from pretreatment weight, (2) tumor volume larger than 1500 mm³, (3) too sick to reach food and water, or (4) skin necrosis. A mouse was considered to be tumor-free if no tumor was palpable. The results were plotted in Figs. 23A and 23B.

Example **187.** The toxicity study of the conjugate in comparison with conjugates C-166a, C-719, C-720, C-723 and T-DM1. 66 female ICR mice, 6-7 weeks old, were separated into 11 groups. Each group included 6 mice for the liver toxicity study. The first group of mice served as the control group and was treated with the phosphate-buffered saline (PBS) vehicle. 10 groups were treated with conjugates C-166a, C-719, C-720, C-723 and T-DM1 respectively at dose of 75 mg/Kg and 150 mg/Kg administered intravenously. The body weight changes for each animal were measured every day for 12 days. The blood collection was followed the NCI's Guidelines for Rodent Blood Collection. Basically, Blood samples were collected through retro-orbital sinuses of the mice, and centrifuged to obtain the sera on Day 5 after administration. The levels of aspartate aminotransferase (AST), alanine aminotransferase (ALT) and alkaline phosphatase (ALP) were analyzed using PUS-2018 semi-automatic biochemistry analyzer with a commercial kid (using aspartate and alanine as substrates, respectively). Reference values were established by following reactive dynamics, according to manufacturer's recommendations. After blood collection, the mice were sacrificed and the mice livers were sliced for pathogen studies. The results of AST and ALT on average were shown in Table 2, and the results of the animal body weight changes on average and the pathogen pictures were shown in Figs. 23A and 24B and FIGS. 24A-24F, respectively.

The results of liver toxicity plus the body weight changes indicated that at the much higher dose of 75 mg/Kg and 150 mg/Kg, the conjugates C-166a, and C-719 were less toxic than T-DM1, the conjugate C-720 had somehow the similar toxicity to T-DM1, and the conjugate C-723 was much more toxic than T-DM1. The toxicity order at the tested doses was: C-723 > T-DM1 ≥ C-720 > C-719 > C-166a > PBS. Since conjugates C-166a, C-719, C-720, and C-723 had a similar in vivo activities and all of them had better in vivo activity than T-DM1 as indicated in Figs. 23A and 23B, therefore the therapeutical windows for conjugates C-166a and C-719 would be much better than T-DM1. In summary, the replacement of N-alkyl-piperidine-2-carboxylic group on the left side of tubulysin by 2-N-alkyl-2,2-dialkyl-acetic group can dramatically reduce animal side toxicity while maintaining the in vivo activities of the tubulysin analogs.

**Table 2. The results of AST and ALT on average of the tested animals.**

| | AST (IU/L) | | ALT (IU/L) | |
|---|---|---|---|---|
| | Day 5 | Day 12 | Day 5 | Day 12 |
| PBS | 91.3±11.4 | 95.9±11.0 | 36.3±18.5 | 27.9±8.0 |
| T-DM1, 75 mg/Kg | 1349.7±321.5 | 303.2±157.8 | 154.4±96.5 | 164.6±61.4 |
| T-DM1, 150 mg/Kg | 3276.6±724.4 | 1509.6±399.3 | 305.9±142.9 | 407.3±53.8 |
| C-166a, 75 mg/Kg | 173.6±13.1 | 100.5±16.1 | 56.8±13.3 | 48.3±9.5 |
| C-166a, 150 mg/Kg | 480.3±50.5 | 131.5±29.3 | 126.5±38.1 | 71.7±15.3 |
| C-719, 75 mg/Kg | 185.6±14.8 | 111.5±19.2 | 62.8±14.8 | 52.7±11.9 |
| C-719, 150 mg/Kg | 543.5±67.5 | 159.5±38.5 | 137.5±43.7 | 83.4±19.2 |
| C-720, 75 mg/Kg | 904.5±231.8 | 264.4±49.6 | 145.6±60.7 | 139.8±28.9 |
| C-720, 150 mg/Kg | 3083.1±803.0 | 1576.6±34.9 | 401.8±59.0 | 335.9±41.5 |
| C-723, 75 mg/Kg | 1673.4±335.5 | 1093.1±351.6 | 206.8±84.1 | 196.0±41.6 |
| C-723, 150 mg/Kg | 4083.4±353.9 | 1861.8±787.1 | 587.6±111.2 | 483.9±220.9 |

## Claims

1. A conjugate of a cell binding molecule with a cytotoxic agent having a structure of Formula (I): 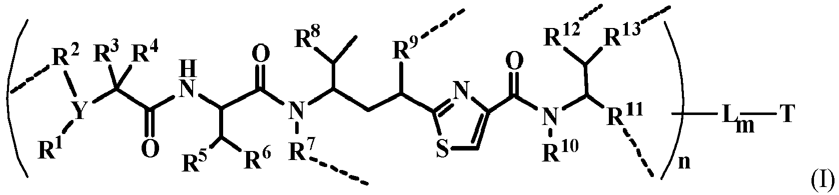
or their pharmaceutically acceptable salts, hydrates, or hydrated salts; or the polymorphic crystalline structures of these compounds; or their optical isomers, racemates, diastereomers or enantiomers thereof;
wherein T is a targeting or cell-binding molecule; L is a releasable linker; ----- is a linkage bond that L connects to an atom inside the bracket independently; n is 1∼20 and m is 1∼10;
wherein T is an antibody; a single chain antibody; an antibody fragment that binds to the target cell; a monoclonal antibody; a single chain monoclonal antibody; or a monoclonal antibody fragment that binds the target cell; a chimeric antibody; a chimeric antibody fragment that binds to the target cell; a domain antibody; a domain antibody fragment that binds to the target cell; adnectins that mimic antibodies; DARPins; a lymphokine; a hormone; a vitamin; a growth factor; a colony stimulating factor; or a nutrient-transport molecule (a transferrin); a binding peptide, or protein, or antibody, or small molecule attached on albumin, polymers, dendrimers, liposomes, nanoparticles, vesicles, (viral) capsids;
wherein the linker L has the formula: --Ww-(Aa)r--Vv-; wherein: --W-- is a Stretcher unit; w is 0 or 1; each --Aa-- is independently an Amino Acid unit; r is independently an integer ranging from 0 to 12; --V-- is a Spacer unit; and v is 0, 1 or 2; the Stretcher unit (--W--), when present, links a targeted binding molecular unit (T) to an amino acid unit (--Aa--), or links V when an Aa is not present; the Stretcher unit W independently contains a self-immolative spacer, peptide units, a hydrazone bond, disulfide or thiolether bonds; W linked to T have a structure of: 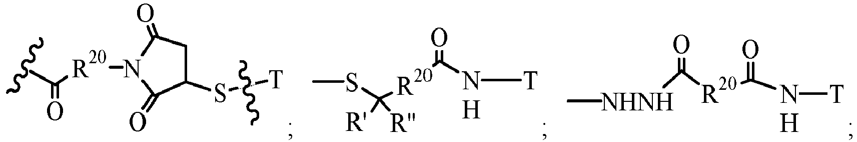 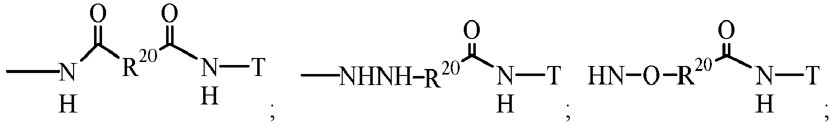 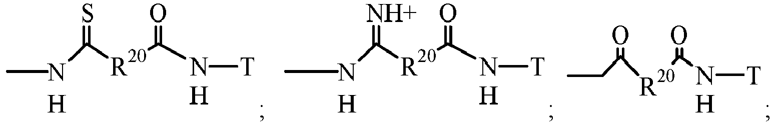 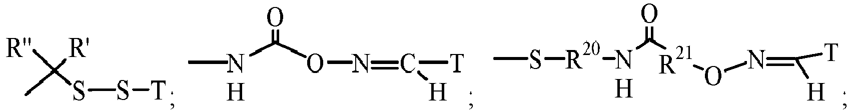 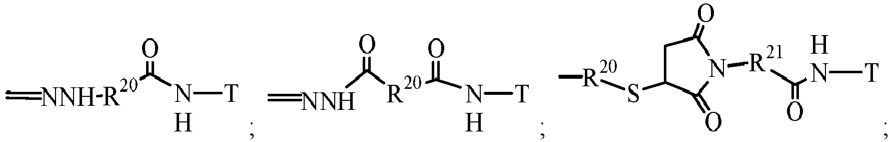 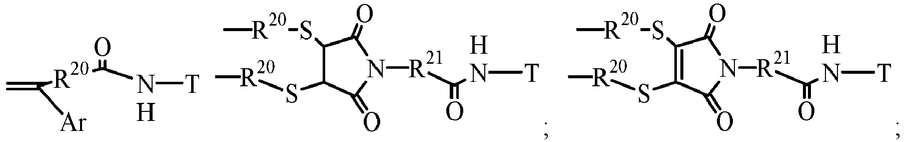 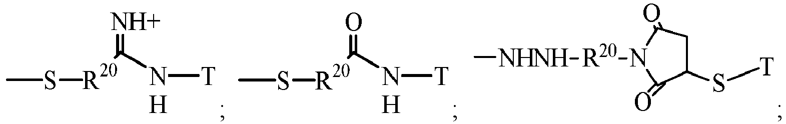 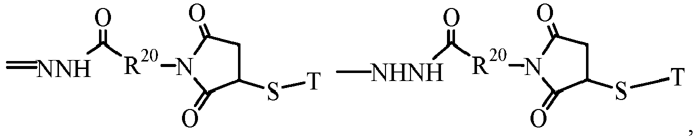 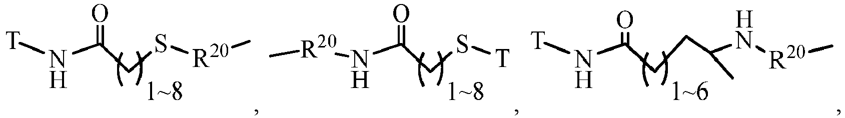 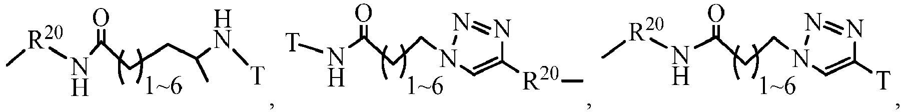 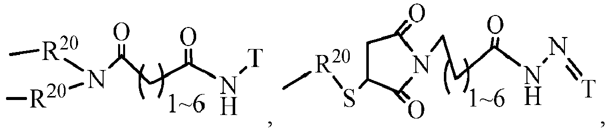 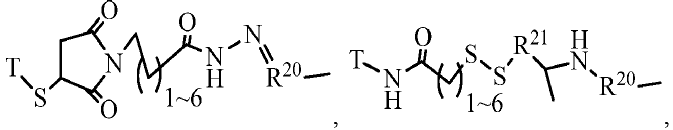 or 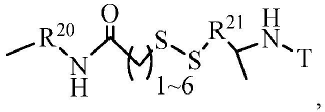 wherein R²⁰ and R²¹ are selected from -C₁∼C₉ alkylene-, -C₁∼C₇ carbocyclo-, -O-(C₁∼C₈ alkyl)-, -arylene-, -C₁∼C₉ alkylene-arylene-, -arylene, -C₁∼C₉ alkylene-, -C₁∼C₉ alkylene-(C₁∼C₈ carbocyclo)-, -(C₃∼C₇ carbocyclo)-C₁∼C₉ alkylene-, -C₃∼C₈ heterocyclo-, -C₁∼C₁₀ alkylene-(C₃∼C₈ heterocyclo)-, -(C₃∼C₈ heterocyclo)- C₁∼C₉ alkylene-, -(CH₂CH₂O)ₖ-,-(CH(CH₃)CH₂O)ₖ-, and -(CH₂CH₂O)ₖ-CH₂-; k is an integer ranging from 1-20.; R' and R" are independently H or CH₃;
the Spacer unit (--V--), when present, links an Amino Acid unit to the antimitotic agent when an Amino Acid unit is present or the Spacer unit links the Stretcher unit to antimitotic agent when the Amino Acid unit is absent, or the Spacer unit links antimitotic agent to the binding molecule (T) when both the Amino Acid unit and Stretcher unit are absent; the spacer linkers contains a function group that substantially increase the water solubility, biological transport, preferential renal clearance, uptake, absorption, biodistribution, and/or bioavailability of the conjugate are described herein; the Spacer unit is a self-immolative or non-self-immolative; the non-self-immolative Spacer unit is one in which part or all of the Spacer unit remains bound to the antimitotic agent after cleavage of the Amino Acid unit from the antimitotic agent-Linker-binding molecule conjugate or the antimitotic agent-Linker Compound; the self-immolative unit includes anaromatic compound that is electronically similar to a para-aminobenzyl-carbamoyl (PAB) group, 2-aminoimidazol-5-methanol group, heterocyclic PAB group, beta-glucuronide, or ortho or para-aminobenzylacetal; or one of the following structures: 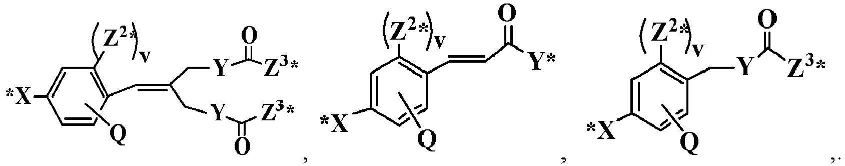 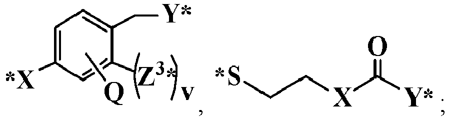 wherein the (*) atom is the point of attachment of additional spacer or releasable linker units, the antimitotic agent, and/or the binding molecule (T); X, Y and Z³ are independently NH, O, or S; Z² is H, NH, O or S independently. v is 0 or 1; Q is independently H, OH, C₁∼C₆ alkyl, (OCH₂CH₂)ₙ F, Cl, Br, I, OR¹⁷, or SR¹⁷, NR¹⁷R¹⁸, N=NR¹⁷, N=R¹⁷, NR¹⁷R¹⁸, NO₂, SOR¹⁷R¹⁸, SO₂R¹⁷, SO₃R¹⁷, OSO₃R¹⁷, PR¹⁷R¹⁸, POR¹⁷R¹⁸, PO₂R¹⁷R¹⁸, OPO(OR¹⁷)(OR¹⁸), or OCH₂PO(OR¹⁷(OR¹⁸) wherein R¹⁷, R¹⁸ are independently H, C₁∼C₈ of alkyl; C₂∼C₈ of alkenyl, alkynyl, heteroalkyl; C₃∼C₈ of aryl, heterocyclic, carbocyclic, cycloalkyl, heterocycloalkyl, heteroaralkyl, alkylcarbonyl; or a pharmaceutical cation salt thereof;
the non-self-immolative spacer unit having a structure of: 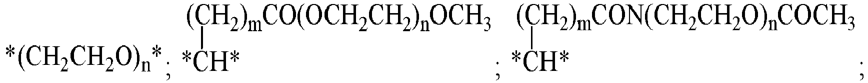 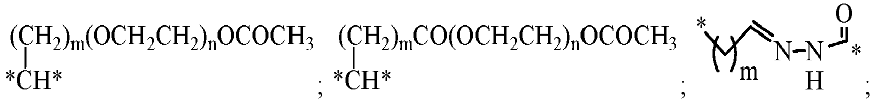 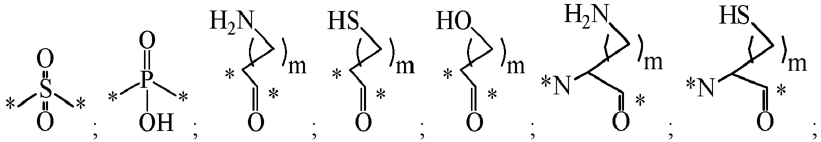 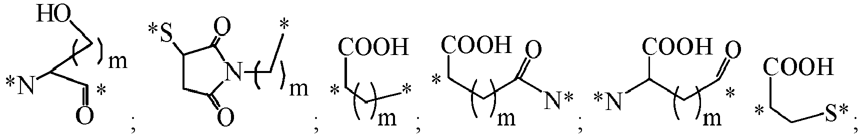 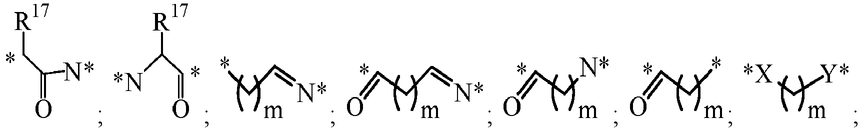 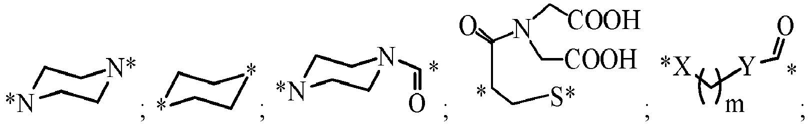 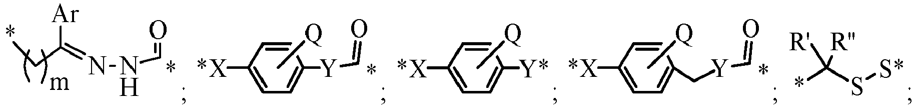 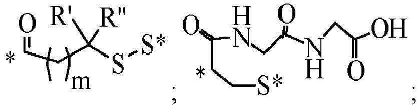 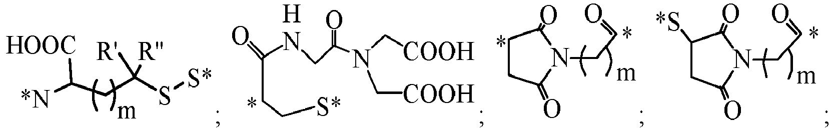 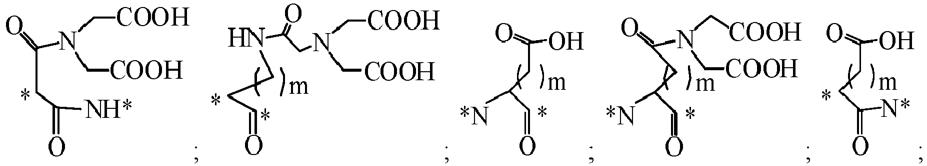 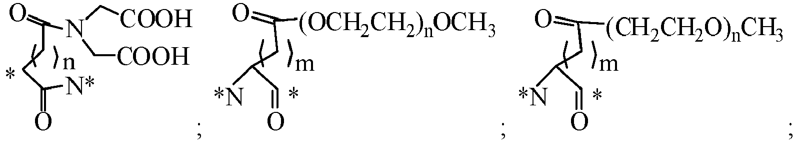 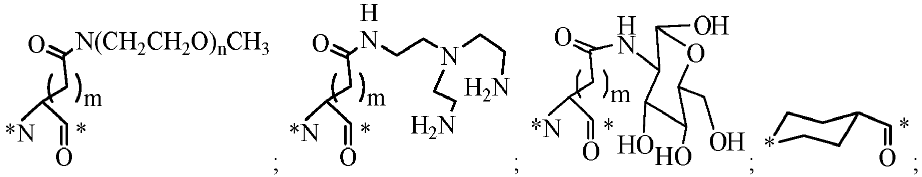 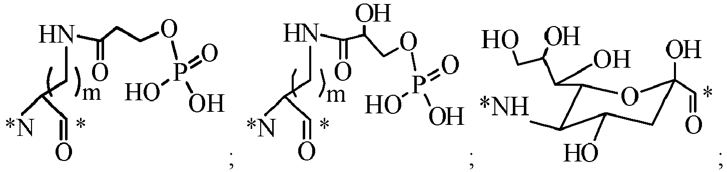 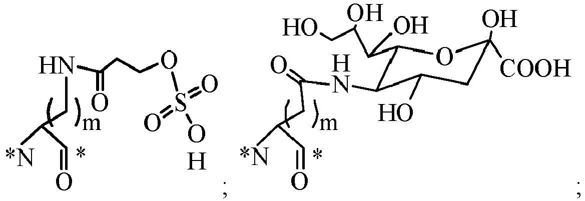 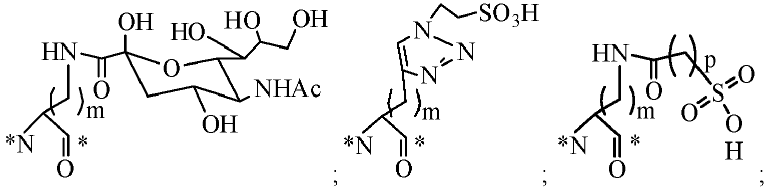 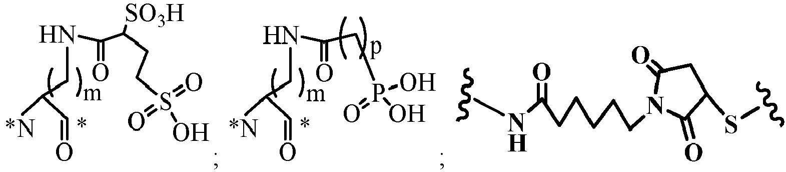 6-maleimidocaproyl (MC), 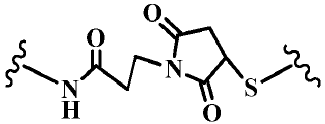
maleimido propanoyl (MP), cit), 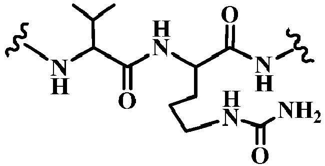 valine-citrulline (val- 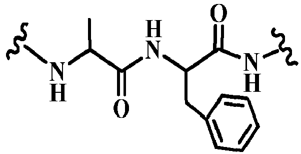 alanine-phenylalanine (ala-phe), 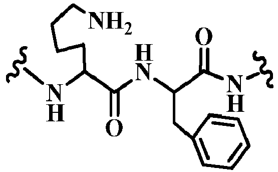
lysine-phenylalanine (lys-phe), 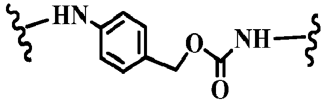 p-aminobenzyloxycarbonyl (PAB), 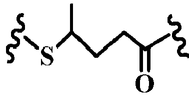 4-thio-pentanoate (SPP), 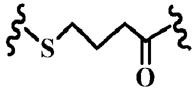 4-thio-butyrate (SPDB), 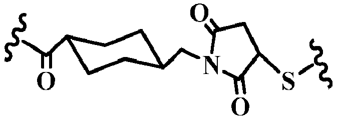 4-(N-maleimidomethyl)cyclo-hexane-1-carboxylate (MCC), 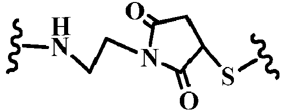 maleimidoethyl (ME), 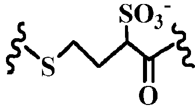 4-thio-2-hydroxysulfonyl-butyrate (2-Sulfo-SPDB), 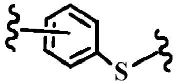 aryl-thiol (PySS), 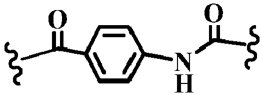 (4-acetyl)aminobenzoate (SIAB), 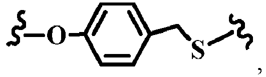 oxylbenzylthio, 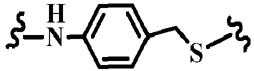 aminobenzylthio, 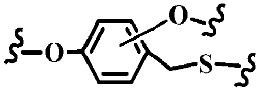 dioxylbenzylthio, 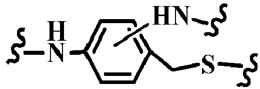 diaminobenzylthio, 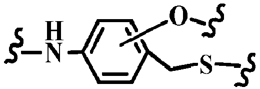 amino-oxylbenzylthio, 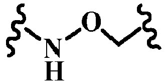 alkoxy amino (AOA), 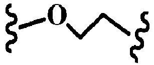 ethyl eneoxy (EO), 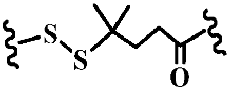 4-methyl-4-dithio-pentanoic (MPDP), 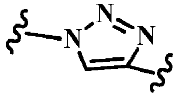 triazole, 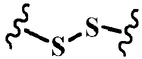 dithio, 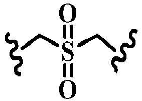 alkylsulfonyl, 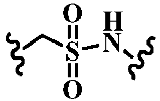 alkylsulfonamide, 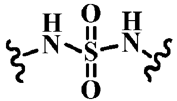 sulfon-bisamide, 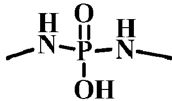 Phosphondiamide, 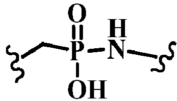 alkylphosphonamide, 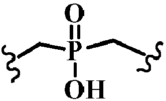 phosphinic acid, 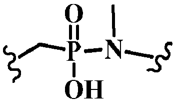 N-methylphosphonamidic acid, 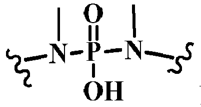 N,N' -dimethylphosphon-amidic acid, 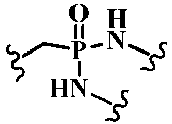 N,N' -dimethylphosphondiamide, 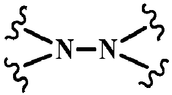 hydrazine, 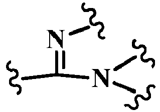 acetimidamide; 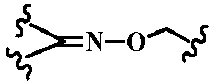 oxime, 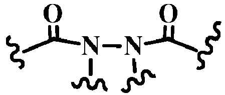 acetylacetohydrazide, 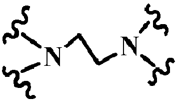 aminoethyl-amine, 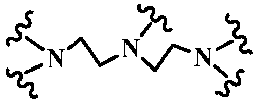 aminoethyl-aminoethyl-amine, 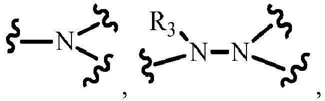 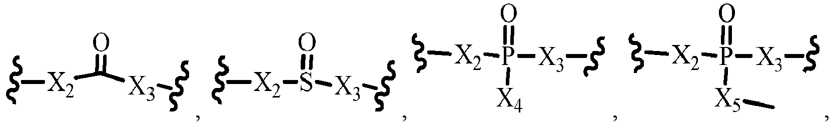 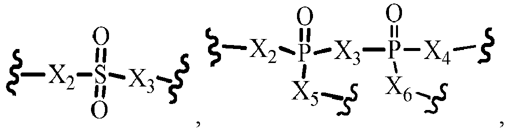 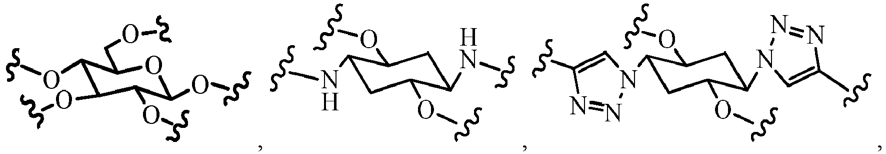 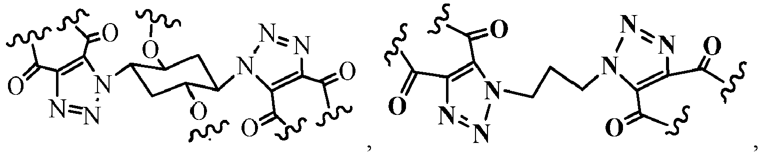 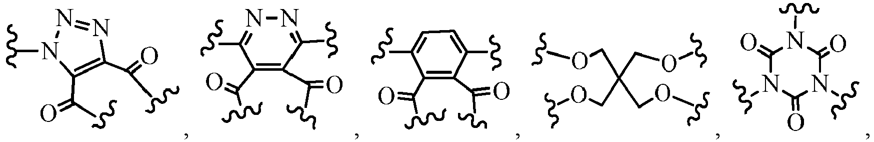 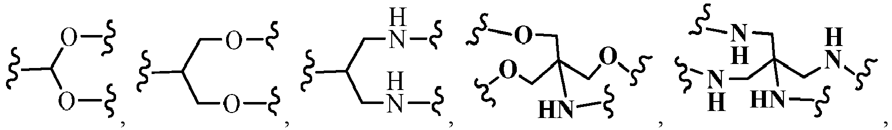 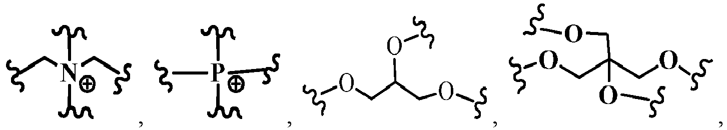 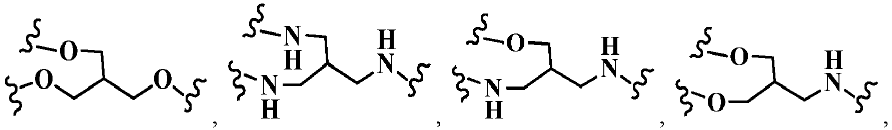 or L- or D-, natural or unnatural peptides containing 1-20 the same or different amino acids;
wherein the "*" and " 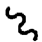 " atom are the point of attachment of additional spacer or releasable linkers, the antimitotic agents, and/or the binding molecules; m is 1∼10; n is 1∼20; X₂, X₃, X₄, X₅, or X₆, are independently selected from NH; NHNH; N(R₁₂); N(R₁₂)N(R_{12'}); O; S; C₁-C₆ of alkyl; C₂-C₆ of heteroalkyl, alkylcycloalkyl, heterocycloalkyl; C₃-C₈ of aryl, Ar-alkyl, heterocyclic, carbocyclic, cycloalkyl, heteroalkylcycloalkyl, alkylcarbonyl, heteroaryl; CH₂OR₁₂, CH₂SR₁₂, CH₂NHR₁₂, or 1∼8 amino acids; wherein R₁₂ and R_{12'} are independently H;C₁-C₈ of alkyl; C₂-C₈ of hetero-alkyl, alkylcycloalkyl, heterocycloalkyl; C₃-C₈ of aryl, Ar-alkyl, heterocyclic, carbocyclic, cycloalkyl, heteroalkylcycloalkyl, alkylcarbonyl, heteroaryl; or 1-8 carbon atoms of esters, ether, or amide; or polyethyleneoxy unit of formula (OCH₂CH₂)ₚ or (OCH₂CH(CH₃))ₚ, wherein p is an integer from 0 to about 1000, or combination above thereof;
a releasable component of the linker L that at least one bond in L can be broken under physiological conditions: a pH-labile, acid-labile, base-labile, oxidatively labile, metabolically labile, biochemically labile or enzyme-labile bond, which having one of the following structures:
-(CR₁₅R₁₆)ₘ(Aa)r(CR₁₇R₁₈)ₙ(OCH₂CH₂)ₜ-, -(CR₁₅R₁₆)ₘ(CR₁₇R₁₈)ₙ(Aa)ᵣ(OCH₂CH₂)ₜ-, -(Aa)ᵣ-(CRₗ₅R₁₆)ₘ(CR₁₇R₁₈)ₙ(OCH₂CH₂)ₜ-, -(CR₁₅R₁₆)ₘ(CR₁₇R₁₈)ₙ(OCH₂CH₂)ᵣ(Aa)ₜ-,-(CR₁₅R₁₆)ₘ(CR₁₇=CR₁₈)(CR₁₉R₂₀)ₙ(Aa)ₜ(OCH₂CH₂)ᵣ-, -(CR₁₅R₁₆)ₘ(NR₁₁CO)(Aa)ₜ(CR₁₉R₂₀)ₙ-(OCH₂CH₂)ᵣ-, -(CR₁₅R₁₆)ₘ(Aa)ₜ(NR₂₁CO)(CR₁₉R₂₀)ₙ(OCH₂CH₂)ᵣ-, -(CR₁₅R₁₆)ₘ(OCO)(Aa)ₜ-(CR₁₉R₂₀)ₙ(OCH₂CH₂)ᵣ-, -(CR₁₅R₁₆)ₘ(OCNR₁₇)(Aa)ₜ(CR₁₉R₂₀)ₙ(OCH₂CH₂)ᵣ-,-(CR₁₅R₁₆)ₘ-(CO)(Aa)ₜ-(CR₁₉R₂₀)ₙ(OCH₂CH₂)ᵣ-, -(CR₁₅R₁₆)ₘ(NR₂₁CO)(Aa)ₜ(CR₁₉R₂₀)ₙ(OCH₂CH₂)ᵣ-, -(CR₁₅R₁₆)ₘ-(OCO)(Aa)ₜ(CR₁₉R₂₀)ₙ-(OCH₂CH₂)ᵣ-, -(CR₁₅R₁₆)ₘ(OCNR₁₇)(Aa)ₜ(CR₁₉R₂₀)ₙ-(OCH₂CH₂)ᵣ-,-(CR₁₅R₁₆)ₘ(CO)(Aa)ₜ(CR₁₉R₂₀)ₙ-(OCH₂CH₂)ᵣ-, -(CR₁₅R₁₆)ₘ-phenyl-CO(Aa)ₜ-(CR₁₇R₁₈)ₙ-,-(CR₁₅R₁₆)ₘ-furyl-CO(Aa)ₜ(CR₁₇R₁₈)ₙ-, -(CR₁₅R₆)ₘ-oxazolyl-CO(Aa)ₜ(CR₁₇R₁₈)ₙ-,-(CR₁₅R₁₆)ₘ-thiazolyl-CO(Aa)ₜ(CCR₁₇R₁₈)ₙ-, -(CR₁₅R₁₆)ₜ-thienyl-CO(CR₁₇R₁₈)ₙ-,-(CR₁₅R₁₆)ₜ-imidazolyl-CO-(CR₁₇R₁₈)ₙ-, -(CR₁₅R₁₆)ₜ-morpholino-CO(Aa)ₜ-(CR₁₇R₁₈)ₙ-,-(CR₁₅R₁₆)ₜ-piperazino-CO(Aa)ₜ(CR₁₇R₁₈)ₙ-, -(CR₁₅R₁₆)ₜ-N-methylpiperazin-CO(Aa)ₜ(CR₁₇R₁₈)ₙ-, -(CR₁₅R₁₆)ₘ-(Aa)ₜphenyl-, -(CR₁₅R₁₆)ₘ-(Aa)tfuryl-, -(CR₁₅R₁₆)ₘ-oxazolyl(Aa)ₜ-, -(CR₁₅R₁₆)ₘ-thiazolyl(Aa)ₜ-, -(CR₁₅R₁₆)ₘ-thienyl-(Aa)ₜ-, -(CR₁₅R₁₆)ₘ-imidazolyl(Aa)ₜ, -(CR₁₅R₁₆)ₘ-morpholino-(Aa)ₜ-, -(CR₁₅R₁₆)ₘ-piperazino-(Aa)t-, -(CR₁₅R₁₆)ₘ-N-methylpiperazino-(Aa)ₜ-,-K(CR₁₅R₁₆)ₘ(Aa)r(CR₁₇R₁₈)ₙ(OCH₂CH₂)ₜ-,-K(CR₁₅R₁₆)ₘ(CR₁₇R₁₈)ₙ(Aa)ᵣ(OCH₂CH₂)ₜ-,-K(Aa)ᵣ-(CR₁₅R₁₆)ₘ(CR₁₇R₁₈)ₙ(OCH₂CH₂)ₜ,-K(CR₁₅R₁₆)ₘ(CR₁₇R₁₈)ₙ(OCH₂CH₂)ᵣ(Aa)ₜ-, -K(CR₁₅R₁₆)ₘ-(CR₁₇=CR₁₈)(CR₁₉R₂₀)ₙ(Aa)ₜ(OCH₂CH₂)ᵣ, -K(CR₁₅R₁₆)ₘ(NR₁₁CO)(Aa)ₜ-(CR₁₉R₂₀ )ₙ(OCH₂CH₂)ᵣ-, -K(CR₅R₆)ₘ(Aa)ₜ(NR₂₁CO)(CR₁₉R₂₀)ₙ(OCH₂CH₂)ᵣ-, -K(CR₁₅R₁₆)ₘ-(OCO)(Aa)t(CR₁₉R₂₀)ₙ-(OCH₂CH₂)ᵣ-, -K(CR₁₅R₁₆)ₘ(OCNR₁₇)(Aa)t(CR₁₉R₂₀)ₙ-(OCH₂CH₂)ᵣ-, -K(CR₁₅R₁₆)ₘ(CO)(Aa)ₜ-(CR₁₉R₂₀)ₙ(OCH₂CH₂)ᵣ-, -K(CR₁₅R₁₆)ₘ(NR₂₁CO)(Aa)ₜ₋(CR₁₉R₂₀)ₙ-(OCH₂CH₂)ᵣ-, -K(CR₁₅R₁₆)ₘ-(OCO)(Aa)ₜ(CR₁₉R₂₀)ₙ(OCH₂CH₂)ᵣ-, -K(CR₁₅R₁₆)ₘ-(OCNR₁₇)(Aa)ₜ-(CR₁₉R₂₀)ₙ(OCH₂CH₂)ᵣ-, -K-(CR₁₅R₁₆)ₘ(CO)(Aa)ₜ(CR₁₉R₂₀)ₙ(OCH₂CH₂)ᵣ-, -K(CR₁₅R₁₆)ₘ-phenyl-CO(Aa)ₜ(CR₁₇R₁₈)ₙ-, -K-(CR₁₅R₁₆)ₘ-furyl-CO(Aa)ₜ(CR₁₇R₁₈)ₙ-, -K(CR₁₅R₁₆)ₘ-oxazolyl-CO(Aa)ₜ(CR₁₇R₁₈)ₙ-, -K(CR₁₅R₁₆)ₘ-thiazolyl-CO(Aa)ₜ-(CR₁₇R₁₈)ₙ-, -K(CR₁₅R₁₆)ₜ-thienyl-CO(CR₁₇R₁₈)ₙ-, -K(CR₁₅R₁₆)ₜimidazolyl-CO-(CR₁₇R₁₈)ₙ-, -K(CR₅R₆)ₜmorpholino-CO(Aa)ₜ-(CR₁₇R₁₈)ₙ-, -K(CR₁₅R₁₆)ₜ-piperazino-CO(Aa)ₜ-(CR₁₇R₁₈)ₙ-, -K(CR₁₅R₁₆)ₜ-N-methylpiperazin-CO(Aa)ₜ(CR₁₇R₁₈)ₙ-, -K(CR₁₅R₁₆)ₘ-(Aa)tphenyl, -K-(CR₁₅R₁₆)ₘ-(Aa)tfuryl-, -K(CR₁₅R₁₆)ₘ-oxazolyl-(Aa)ₜ, -K(CR₁₅R₁₆)ₘ-thiazolyl(Aa)ₜ, -K(CR₁₅R₁₆)ₘ-thienyl-(Aa)t-, -K(CR₁₅R₁₆)ₘ-imidazolyl(Aa)ₜ-, -K(CR₁₅R₁₆)ₘ-morpholino(Aa)ₜ-, -K(CR₁₅R₁₆)ₘpiperazino(Aa)ₜG, -K(CR₅R₆)ₘ-N-methyl-piperazino(Aa)ₜ-; wherein m, Aa, m, n, R₁₃, R₁₄, and R₁₅ are described above; t and r here are 0 - 100 independently; R₁₆, R₁₇, R₁₈, R₁₉, and R₂₀ are independently chosen from H; halide; C₁∼C₈ of alkyl or heteroalkkyl, C₂∼C₈ of aryl, alkenyl, alkynyl, ether, ester, amine or amide, C₃∼C₈ of aryl, which optionally substituted by one or more halide, CN, NR₁₂R_{12'}, CF₃, OR₁₂, Aryl, heterocycle, S(O)R₁₂, SO₂R₁₂, -CO₂H, -SO₃H, -OR₁₂, -CO₂R₁₂, -CONR₁₂, -PO₂R₁₂R₁₃,-PO₃H or P(O)R₁₂R₁₂R₁₃; K is NR₁₂, -SS-, -C(=O)-, -C(=O)NH-, -C(=O)O-, -C=NH-O-,-C=N-NH-, -C(=O)NH-NH-, O, S, Se, B, Het (heterocyclic or heteroaromatic ring having C₃-C₁₂); or peptides containing the same or different 1- 20 amino acids;
inside the bracket of the Formula (I) is a potent antimitotic agent wherein R¹, R², R³, and R⁴ are independently linear or branched C₁-C₈ of alkyl, alkylalcohol; C₂-C₈ of heteroalkyl, alkylcycloalkyl, heterocycloalkyl, alkyl ether, alkyl carboxylate, alkyl amine, alkyl ester, alkyl amide; C₃-C₈ of aryl, Ar-alkyl, heterocyclic, carbocyclic, cycloalkyl, heteroalkylcycloalkyl, alkylcarbonyl; or two R's: R¹R², R³R⁴, R⁵R⁶, or R¹²R¹³ together form a 3∼7 membered carbocyclic, cycloalkyl, heterocyclic, heterocycloalkyl, aromatic or heteroaromatic ring system; Y is N or C; R¹, R², R³, and R⁴ may optionally be independently absent;
wherein R⁵, R⁶, R⁸ and R¹⁰ are independently selected from H and linear or branched C₁-C₄ of alkyl or C₂-C₄ of heteroalkyl;
wherein R⁷ is selected from H, R¹⁴, or -R¹⁴C(=O)X¹R¹⁵; -R¹⁴X¹R¹⁵; X¹ is selected from O, S, S-S, NH, or NR¹⁴;
wherein R⁹ is H, -O-, -OR¹⁴, -OC(=O)R¹⁴-, -OC(=O)NHR¹⁴-, -OC(=O)NR¹⁴R¹⁵-,-OC(=O)R¹⁴SSR¹⁵-, OP(=O)(OR¹⁴)-, or OR¹⁴OP(=O)(OR¹⁵);
wherein R¹¹ is H, R¹⁴, -R¹⁴C(=O)R¹⁶, -R¹⁴C(=O)X²R¹⁶, -R¹⁴X²R¹⁶, -R¹⁴C(=O)X², wherein X² is -O-, -S-, -NH-, -NHS(O₂), -NHS(O), -N(R¹⁴)-, -O-R¹⁴-, -S-R¹⁴-, -S(=O)-R¹⁴-, or -NHR¹⁴-;
wherein R¹² is H, R¹⁴, -O-, -S-, -N-, =N-, =NNH-, -OH, -SH, -NH₂, =NH, =NNH₂, -NH(R¹⁴), -OR¹⁴, -C(O)O-, -C(O)OR¹⁶-,-COR¹⁶, -COOR¹⁴-, C(O)NH-, C(O)NH₂, C(O)NHR¹⁴, -SR¹⁴, -S(=O)R¹⁴, -P(=O)(OR¹⁶)₂, -OP(=O)(OR¹⁶)₂, -CH₂OP(=O)(OR¹⁶)₂,-SO₂R¹⁶;
wherein R¹³ is linear or branched C₁-C₁₀ of alkyl, alkyl acid, alkyl amide, alkyl amine; or C₂-C₁₀ of heteroalkyl; or C₃-C₁₀ of Ar; Ar is an aromatic or hetero aromatic group, composed of one or several rings, comprising four to ten carbon atoms, the hetero aromatic group is an aromatic group that has one or several carbon atoms replaced by hetero atoms, the aryl or Ar is an aromatic group, wherein one or several H atoms can be replaced independently by R¹⁷, F, Cl, Br, I, OR¹⁶, SR¹⁶, NR¹⁶R¹⁷, N=NR¹⁶, N=R¹⁶, NR¹⁶R¹⁷ , NO₂, SOR¹⁶R¹⁷, SO₂R¹⁶, SO₃R¹⁶, OSO₃R¹⁶, PR¹⁶R¹⁷, POR¹⁶R¹⁷, PO₂R¹⁶R¹⁷, OP(O)(OR¹⁷)₂, OCH₂OP(O)(OR¹⁷)₂, OC(O)OP(O)(OR¹⁷)₂, PO(OR¹⁶)(OR¹⁷), OP(O)(OR¹⁷)OP(O)(OR¹⁷)₂, OC(O)R¹⁷ or OC(O)NHR¹⁷;
wherein R¹⁴ and R¹⁵ are independently H; linear or branched C₁-C₈ of alkyl; C₂-C₈ of alkenyl, alkynyl, heteroalkyl, heterocyclic, carbocyclic; C₃-C₈ of aryl, cycloalkyl, alkylcycloalkyl, heterocycloalkyl, heteroaralkyl, heteroalkylcycloalkyl, alkylcarbonyl;
wherein when R¹⁴ is bivalent, R¹⁴ is further connected to an additional functional group of one to four amino acid units, or (CH₂CH₂O)ᵣ, r is an integer ranging from 0 to 12, or C₄-C₁₂ of glycosides, or C₁-C₈ of carboxylic acid;
wherein R¹⁶ is H, OH, R¹⁴ or one to four amino acid units;
wherein R¹⁷ is H, linear or branched C₁-C₈ of alkyl; C₂-C₈ of alkenyl, alkynyl, heteroalkyl, heterocyclic; C₃-C₈ of aryl, carbocyclic, cycloalkyl, alkylcycloalkyl, heterocycloalkyl, heteroalkylcycloalkyl, heteroaralkyl, alkylcarbonyl or C₄-C₁₂ of glycoside, or a pharmaceutical salt.

2. The conjugate according to claim 1 having Formula (II): 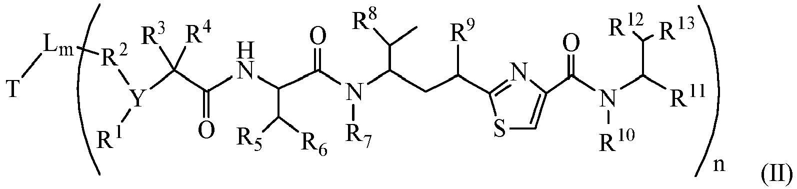
or a pharmaceutically acceptable salt, hydrate, or hydrated salt thereof; or a polymorphic crystalline structure thereof; or an optical isomer, racemate, diastereomer or enantiomer thereof;
wherein T, L, n, m, Y, R¹, R², R³, R⁴, R⁵, R⁶, R⁸, R¹⁰, R¹³, R¹⁴, R¹⁵, R¹⁶ and R¹⁷ are defined the same as in the Formula (I);
wherein R⁷ is selected from H, R¹⁴, or -R¹⁴C(=O)X¹R¹⁵; -R¹⁴X¹R¹⁵; X¹ is selected from O, S, S-S, NH, or NR¹⁴;
wherein R⁹ is H, -OH, -OR¹⁴, -OC(=O)R¹⁴, -OC(=O)NHR¹⁴, -OC(=O)NR¹⁴R¹⁵,-OC(=O)R¹⁴SSR¹⁵, OP(=O)(OR¹⁴)₂, or OR¹⁴OP(=O)(OR¹⁵);
wherein R¹¹ is H, R¹⁴, -R¹⁴C(=O)R¹⁶, -R¹⁴C(=O)X²R¹⁶, -R¹⁴X²R¹⁶, -R¹⁴C(=O)X², wherein X² is -O-, -S-, -NH-, -NHS(O₂), -N(R¹⁴)-, -O-R¹⁴-, -S-R¹⁴-, -S(=O)-R¹⁴-, or-NHR¹⁴-;
wherein R¹² is H, R¹⁴, -O-, -S-, -N-, =N-, =NNH-, -OH, -SH, -NH₂, =NH, =NNH₂, -NH(R¹⁴), -OR¹⁴, -C(O)O-, -C(O)OR¹⁶-, -COR¹⁶, -COOR¹⁴-, C(O)NH-, C(O)NH₂, C(O)NHR¹⁴, -SR¹⁴, -S(=O)R¹⁴, -P(=O)(OR¹⁶)₂, -OP(=O)(OR¹⁶)₂, -CH₂OP(=O)(OR¹⁶)₂, or -SO₂R¹⁶.

3. The conjugate according to claim 1 having Formula (III): 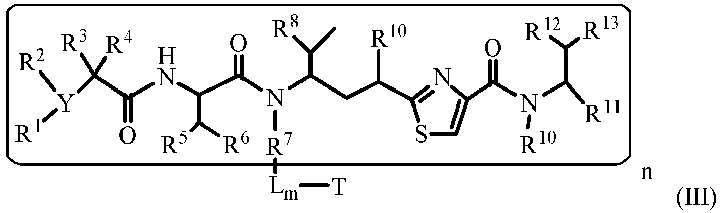
or a pharmaceutically acceptable salt, hydrate, or hydrated salt thereof; or a polymorphic crystalline structure thereof; or an optical isomer, racemate, diastereomer or enantiomer thereof;
wherein T, L, m, Y, R¹, R², R³, R⁴, R⁵, R⁶, R⁸ , R⁹, R¹⁰, R¹¹, R¹², R¹³ and n are defined the same as in Formula (I);
wherein R⁷ is independently -R¹⁴-, or -R¹⁴C(=O)X¹R¹⁵- or -R¹⁴X¹R¹⁵-, wherein R¹⁴ and R¹⁵ are independently linear or branched C₁∼C₈ of alkyl, heteroalkyl; C₂-C₈ of alkenyl, alkynyl; C₃∼C₈ of aryl, heterocyclic, carbocyclic, cycloalkyl, heterocycloalkyl, heteroaralkyl heteroalkylcycloalkyl, alkylcarbonyl; X¹ is O, S, S-S, NH, or NR¹⁴.

4. The conjugate according to claim 1 having Formula (IV): 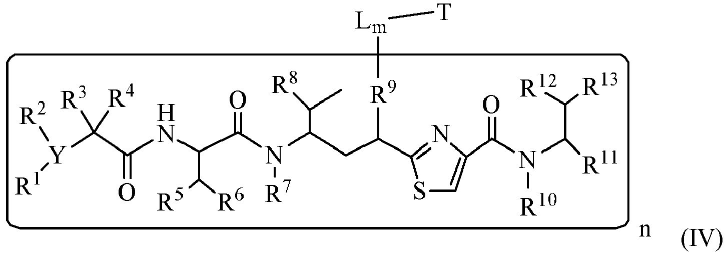
or a pharmaceutically acceptable salt, hydrate, or hydrated salt thereof; or a polymorphic crystalline structure thereof; or an optical isomer, racemate, diastereomer or enantiomer thereof;
wherein T, L, m, R¹, R², R³, R⁴, R⁵, R⁶, R⁷, R⁸, R¹⁰, R¹¹, R¹², R¹³ and n are defined the same as in Formula (I);
wherein R⁹ is independently H, -O-, -OR¹⁴-, -OC(=O)R¹⁴-, -OC(=O)NHR¹⁴-,-OC(=O)NR¹⁴R¹⁵-, -OC(=O)R¹⁴SSR¹⁵-, -OP(=O)(OR¹⁴)O-, wherein R¹⁴, R¹⁵ are independently H, C₁∼C₈ of alkyl, heteroalkyl; C₃∼C₈ of aryl, heteroaryl, heterocyclic, carbocyclic, cycloalkyl, alkylcycloalkyl, heterocycloalkyl, heteroalkylcycloalkyl, heteroaralkyl, alkylcarbonyl or a pharmaceutical salt.

5. The conjugate according to claim 1 having Formula (V): 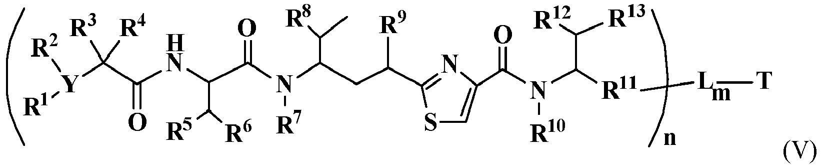
or a pharmaceutically acceptable salt, hydrate, or hydrated salt thereof; or a polymorphic crystalline structure thereof; or an optical isomer, racemate, diastereomer or enantiomer thereof;
wherein T, L, m, Y, R¹, R², R³, R⁴, R⁵, R⁶, R⁷, R⁸, R⁹, R¹⁰, R¹², R¹³ and n are defined the same as in Formula (I);
wherein R¹¹ is -R¹⁴-, -R¹⁴C(=O)R¹⁷-, -R¹⁴C(=O)X²R¹⁷-, -R¹⁴X²R¹⁷-, - R¹⁴C(=O)X²-, wherein R¹⁷ is independently H, OH, C₁∼C₈ of alkyl; C₂∼C₈ of alkenyl, alkynyl, heteroalkyl; C₃∼C₈ of aryl, arylene, heterocyclic, carbocyclic, heterocycloalkyl; or an amino acid, or two amino acid units; X² is -O-, -S-, -NH-, -NHS(O₂)-, -NHS(O)-, -N(R¹⁴)-, -O-R¹⁴-, -S-R¹⁴-, -S(=O)-R¹⁴-, or -NHR¹⁴-; R¹⁴ is H, C₁∼C₈ of alkyl, heteroalkyl; C₂-C₈ of alkenyl, alkynyl; C₃∼C₈ of aryl, heterocyclic, carbocyclic, cycloalkyl, alkylcycloalkyl, heterocycloalkyl, heteroalkylcycloalkyl, heteroaralkyl, or alkylcarbonyl.

6. The conjugate according to claim 1 having Formula (VI): 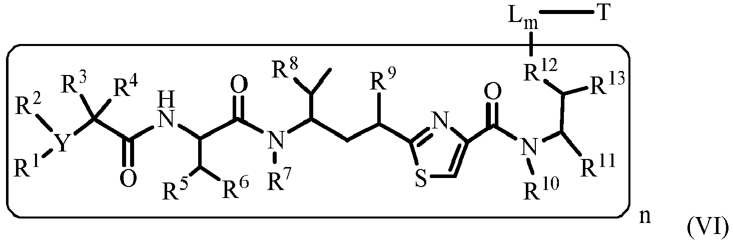
or a pharmaceutically acceptable salt, hydrate, or hydrated salt thereof; or a polymorphic crystalline structure thereof; or an optical isomer, racemate, diastereomer or enantiomer thereof;
wherein T, L, m, Y, R¹, R², R³, R⁴, R⁵, R⁶, R⁷, R⁸, R⁹, R¹¹, R¹³ and n are defined the same as in Formula (I);
wherein R¹² is independently R¹⁴, -O-, -S-, -NH-, =N-, =NNH-, -N(R¹⁴)-, -OR¹⁴-, C(O)O-, C(O)NH-, C(O)NR¹⁴-, -SR¹⁴-, -S(=O)R¹⁴- -NHR¹⁴-, -CH₂OP(=O)(OR¹⁵)-,-P(=O)(OR¹⁵)-,. -OP(=O)(OR¹⁵)O-, -SO₂R¹⁴, R¹⁴, R¹⁵ are independently C₁∼C₈ of alkyl, heteroalkyl; C₂-C₈ of alkenyl, alkynyl; C₃∼C₈ of aryl, heterocyclic, carbocyclic, cycloalkyl, alkylcycloalkyl, heterocycloalkyl, heteroaralkyl, heteroalkylcycloalkyl, alkylcarbonyl.

7. The conjugate according to claim 1 having Formula (VII): 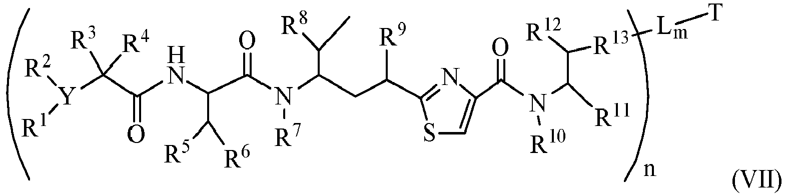
or a pharmaceutically acceptable salt, hydrate, or hydrated salt; or a polymorphic crystalline structure thereof; or an optical isomer, racemate, diastereomer or enantiomer thereof,
wherein T, L, n, m, Y, R¹, R^{1'}, R², R³, R⁴, R⁵, R⁶, R⁸, R¹⁰, R¹¹, and R¹² are defined the same as in Formula (I);
wherein R¹³ is C₁∼C₁₀ of alkyl, heteroalkyl, alkyl acid, alkyl amide, alkyl amine, or Ar; Ar refers to a aromatic or hetero aromatic group, composed of one or several rings, comprising four to ten carbon atoms; the hetero aromatic group is an aromatic group wherein one, or more carbon atoms are replaced by O, N, Si, Se, P or S, the aryl or Ar is an aromatic group, wherein one or several H atoms are replaced independently by R¹⁸, F, Cl, Br, I, OR¹⁶, SR¹⁶; NR¹⁶R¹⁸, N=NR¹⁶, N=R¹⁶, NR¹⁶R¹⁸, NO₂, SOR¹⁶R¹⁸, SO₂R¹⁶, SO₃R¹⁶, OSO₃R¹⁶, PR¹⁶R¹⁸, POR¹⁶R¹⁸, PO₂R¹⁶R¹⁸, OPO₃R¹⁶R¹⁸, or PO₃R¹⁶R¹⁸ wherein R¹⁶, R¹⁸ are independently H, C₁∼C₈ of alkyl; C₂∼C₈ of alkenyl, alkynyl, heteroalkyl; C₃∼C₈ of aryl, heterocyclic, carbocyclic, cycloalkyl, alkylcycloalkyl, heterocycloalkyl, heteroaralkyl, heteroalkylcycloalkyl, alkylcarbonyl; or C₄ ∼ C₁₂ glycosides; or a pharmaceutical salt.

8. The conjugate according to claim 1, wherein the cytotoxic compound inside the bracket of Formula (I) has a structure represented by one of the following Formulae II-01 ∼II-69, III-01 ∼III-68, IV-01 ∼IV-68, V-01 ∼V-68, VI-01 ∼VI-12, and VII-01 ∼VII-77: 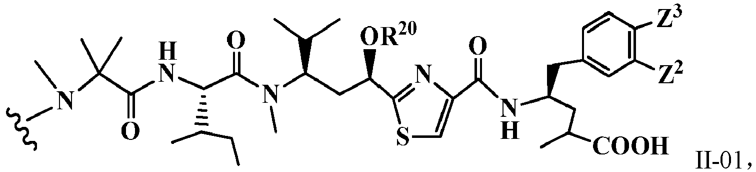 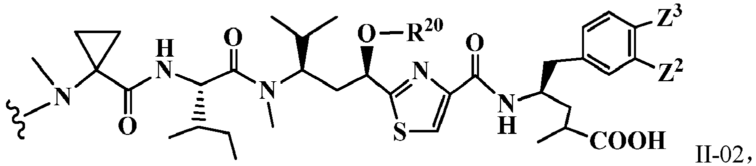 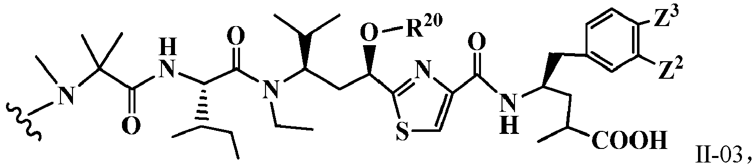 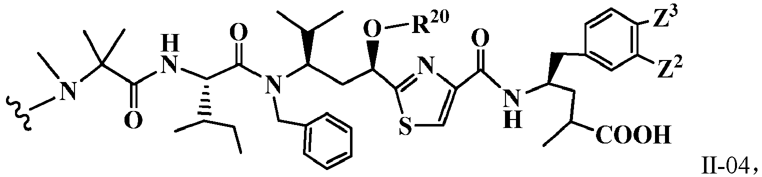 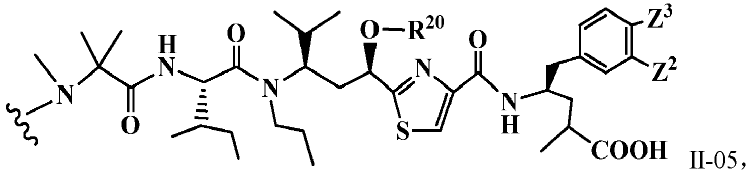 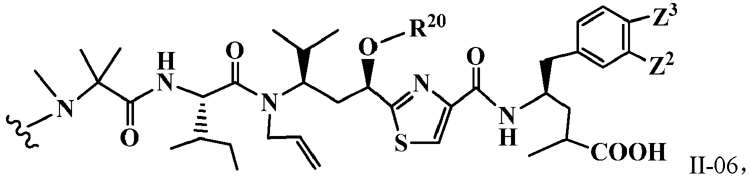 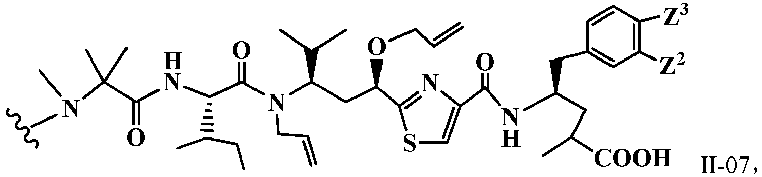 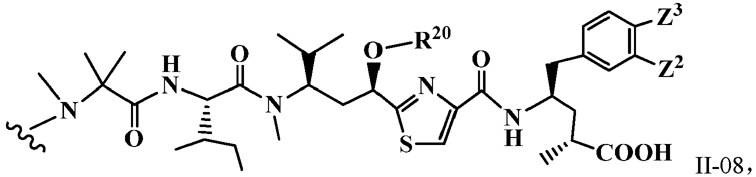 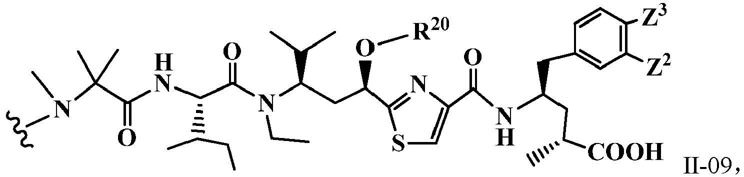 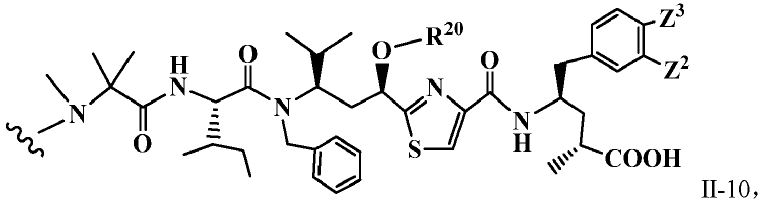 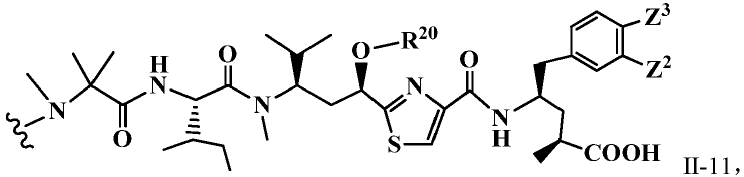 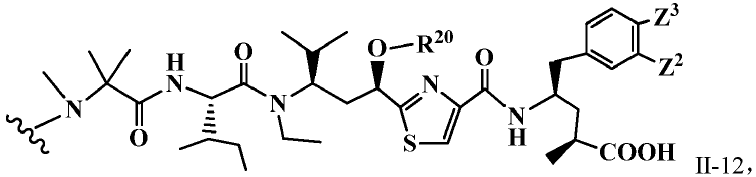 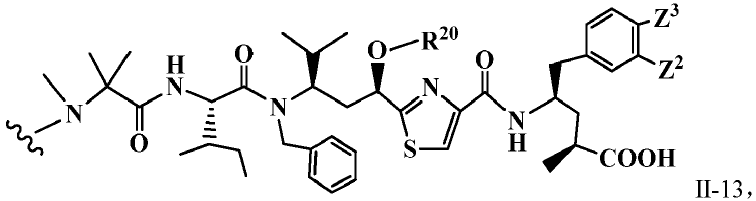 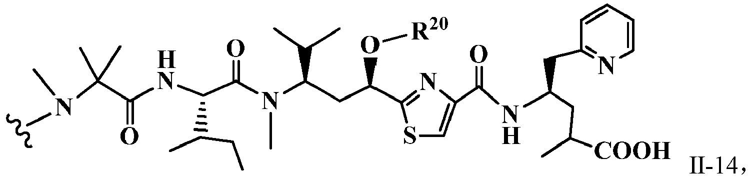 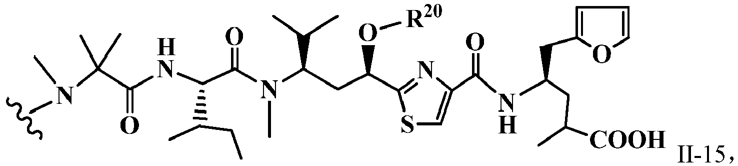 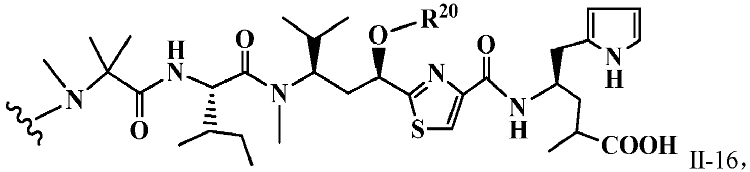 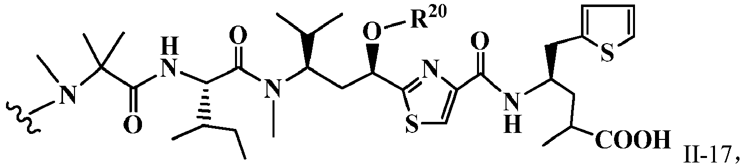 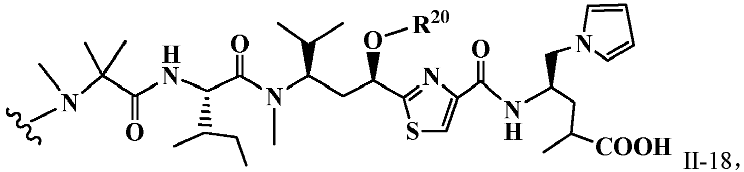 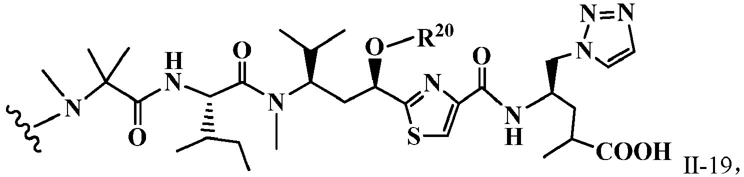 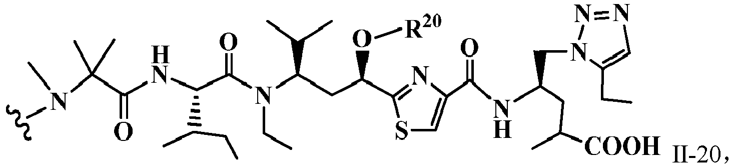 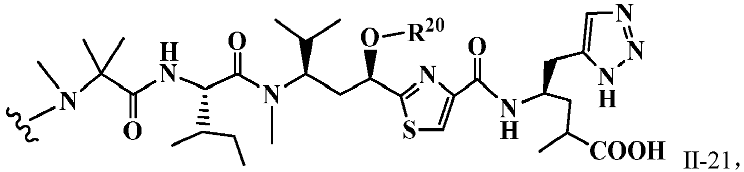 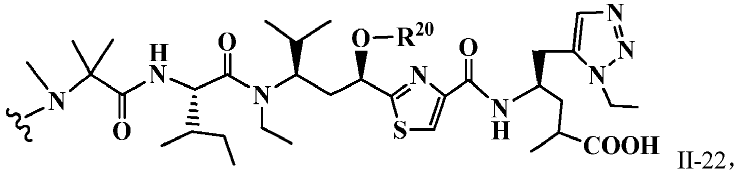 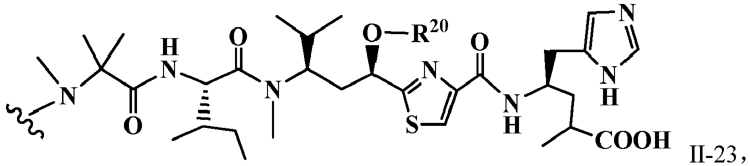 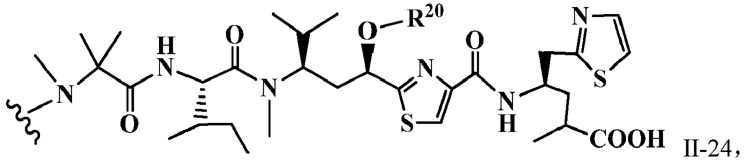 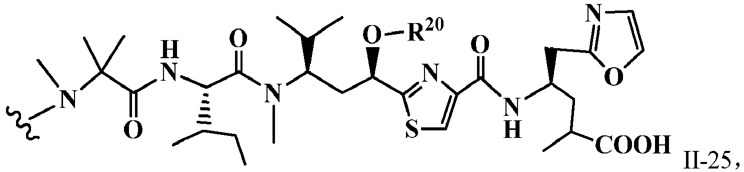 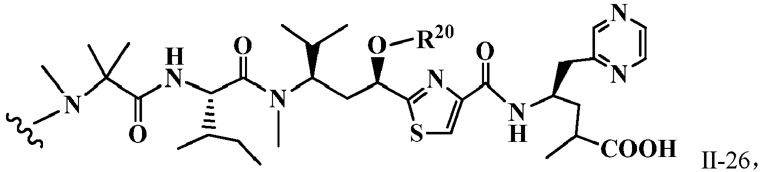 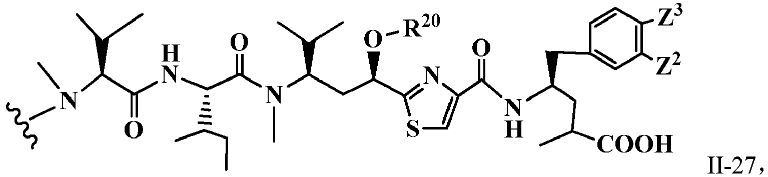 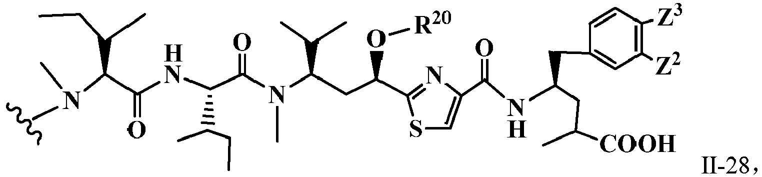 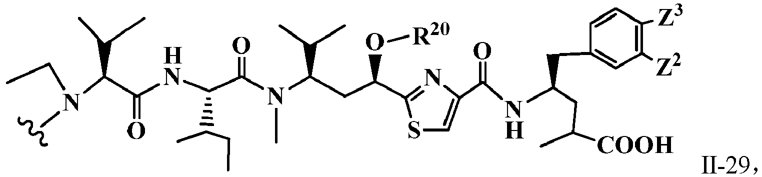 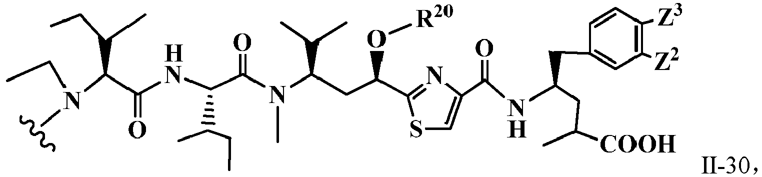 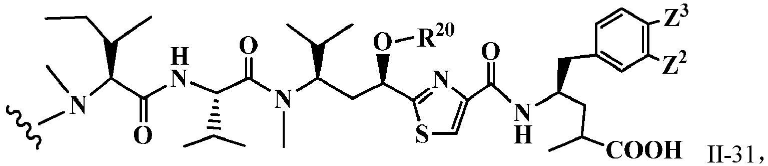 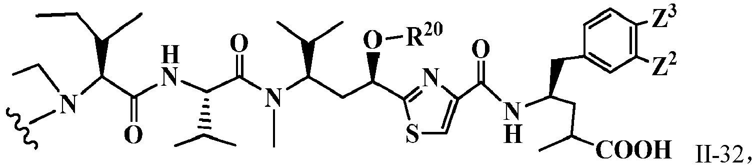 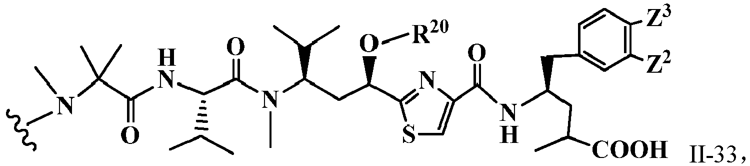 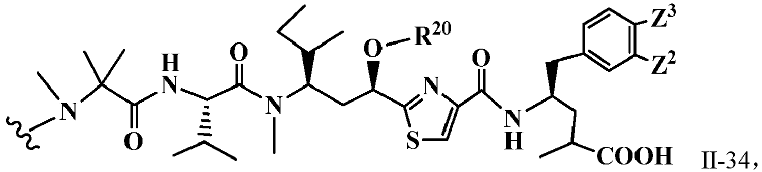 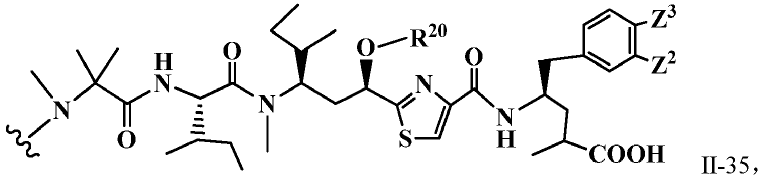 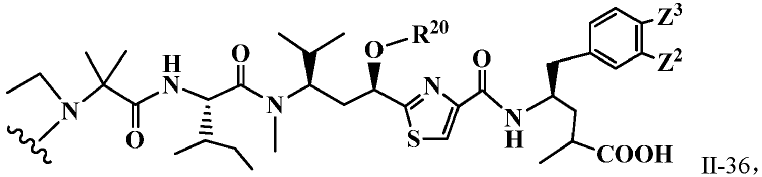 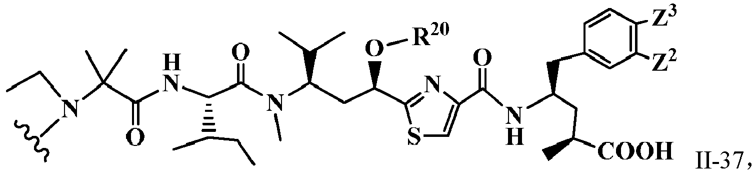 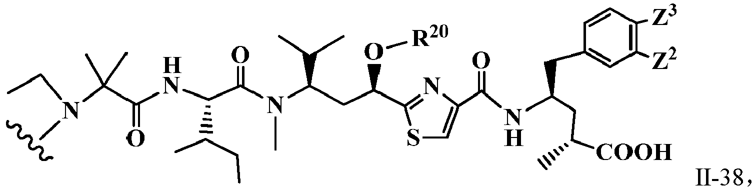 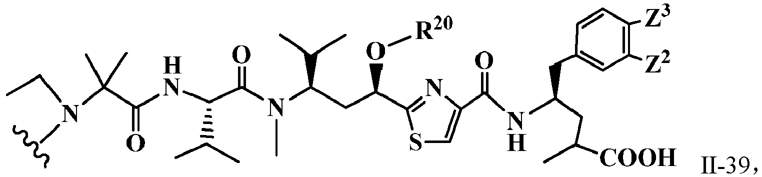 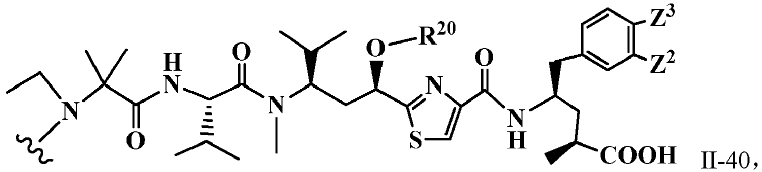 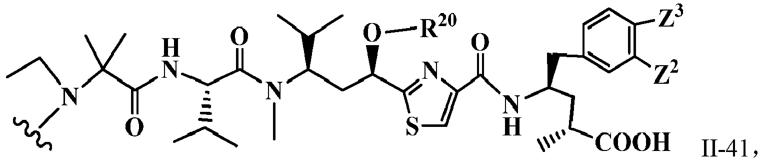 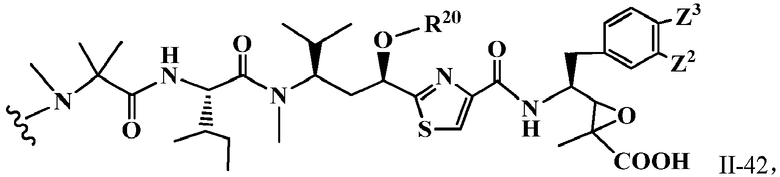 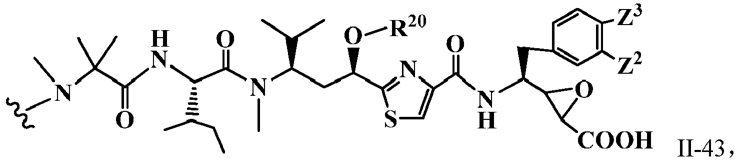 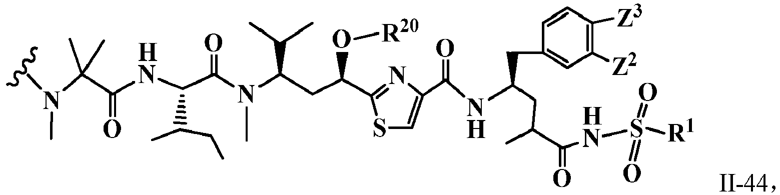 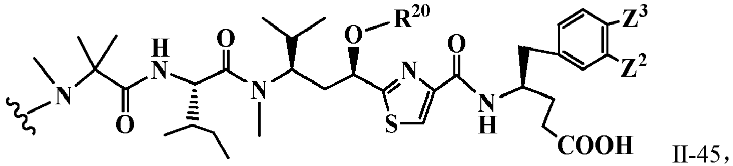 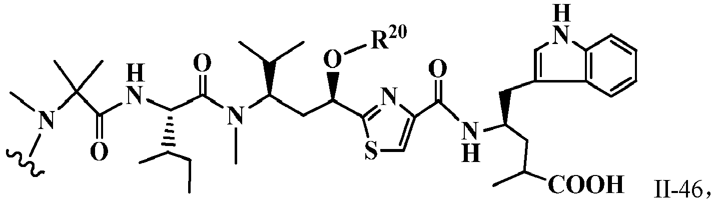 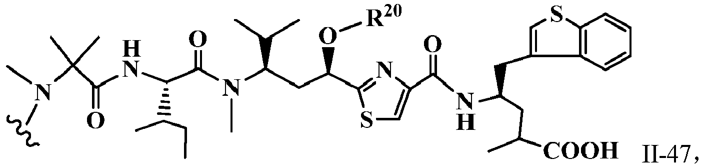 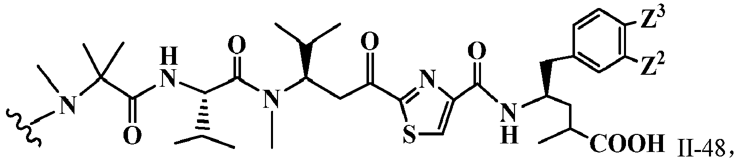 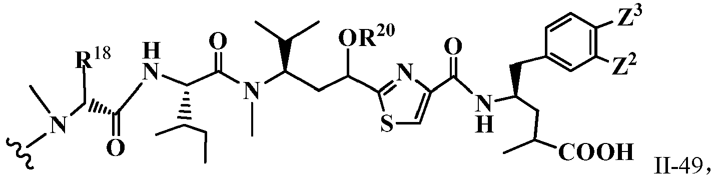 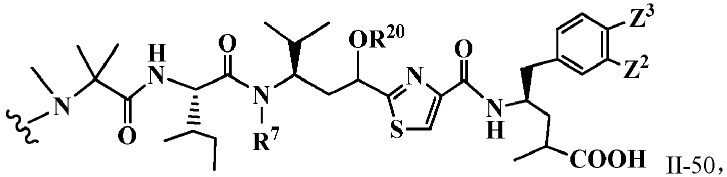 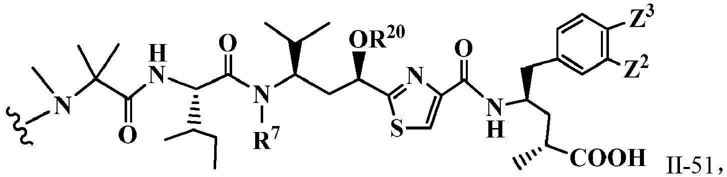 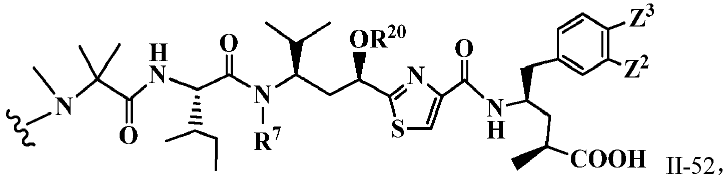 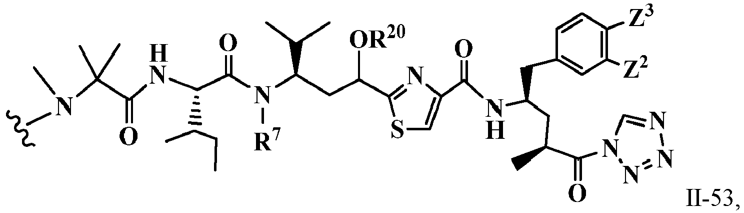 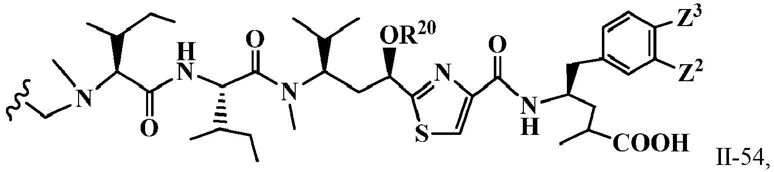 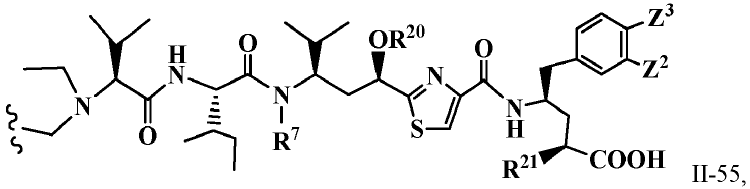 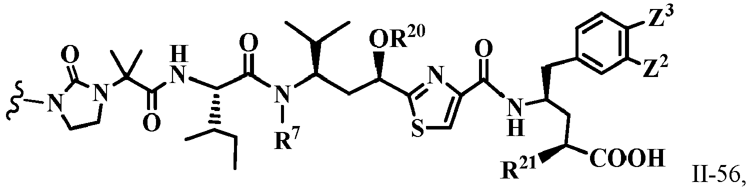 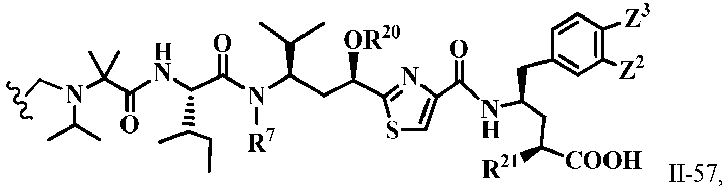 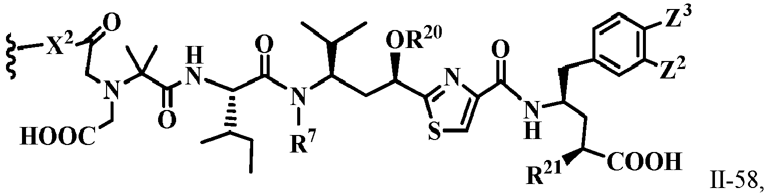 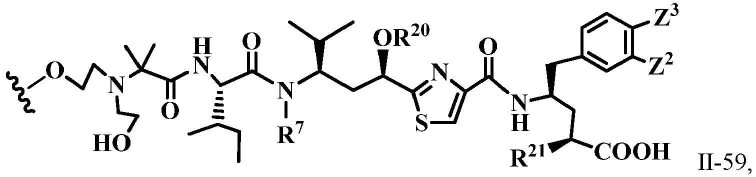 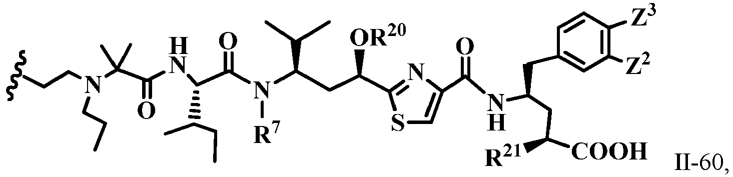 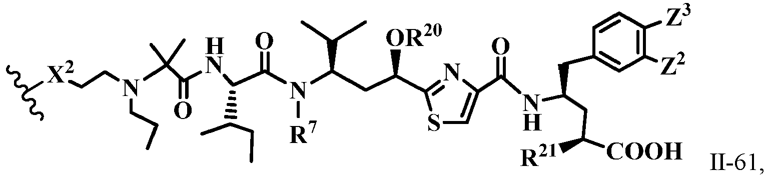 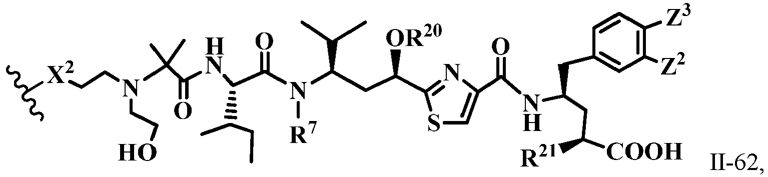 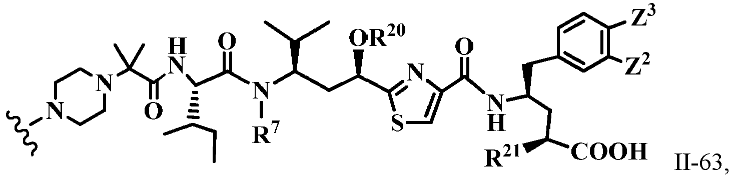 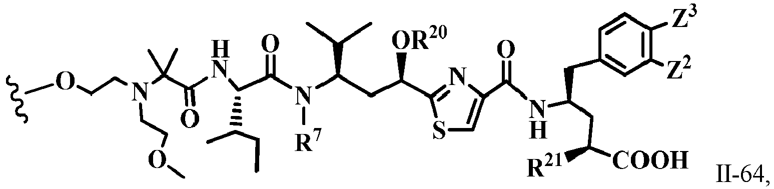 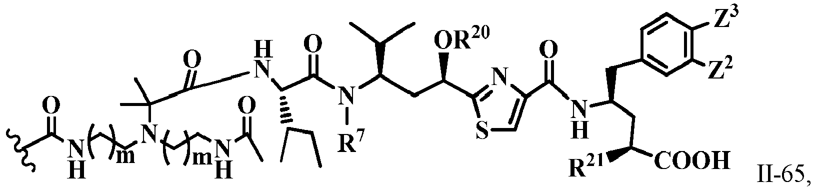 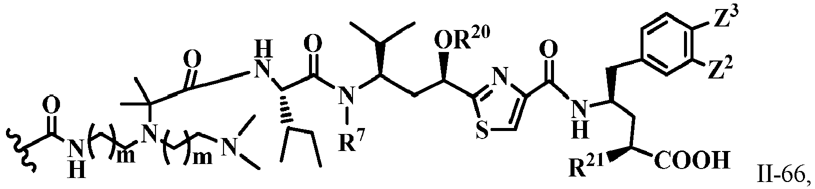 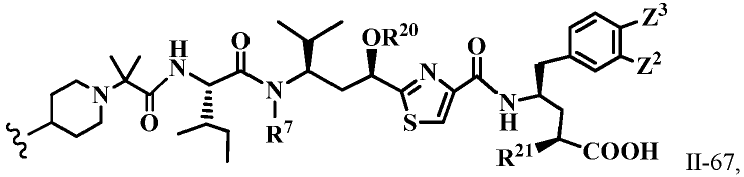 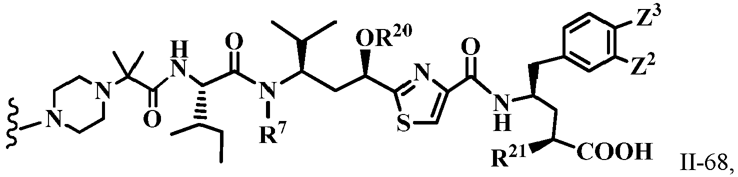 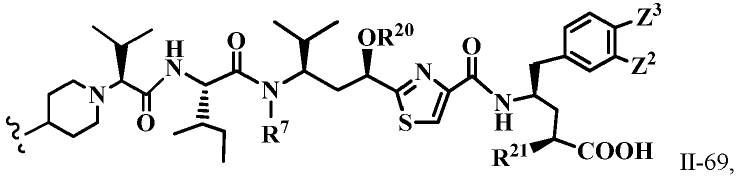 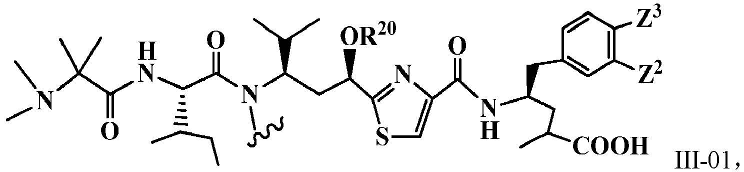 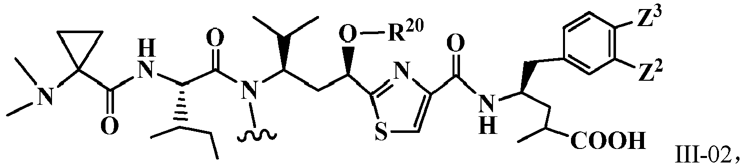 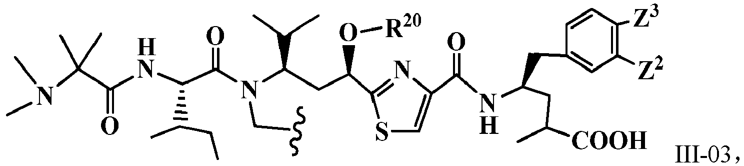 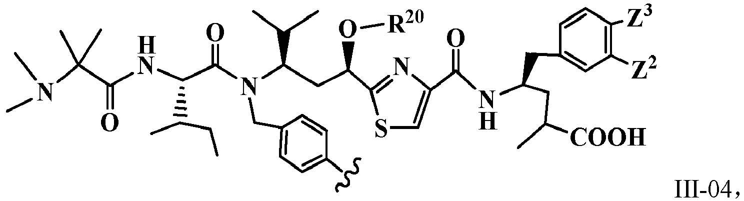 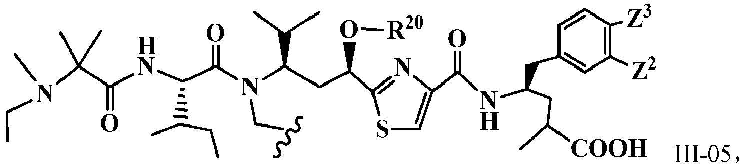 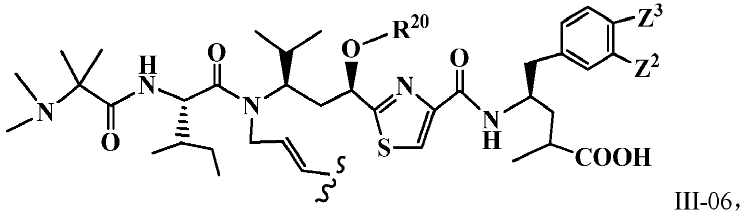 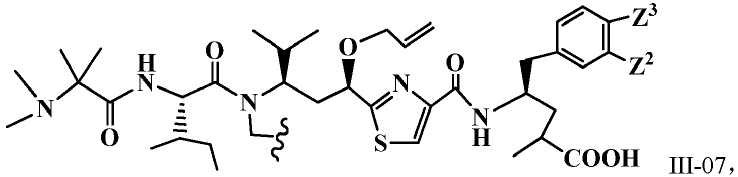 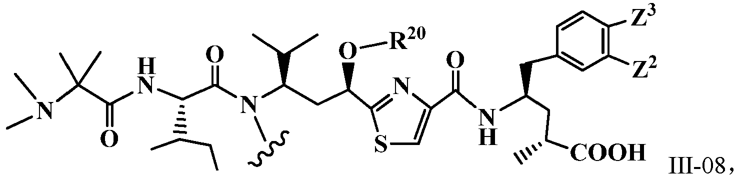 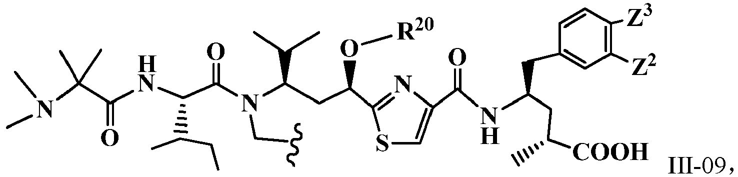 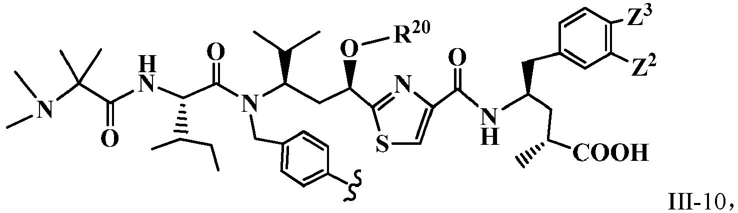 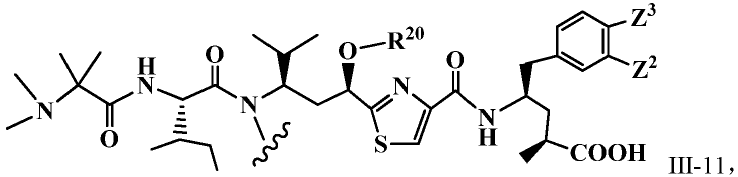 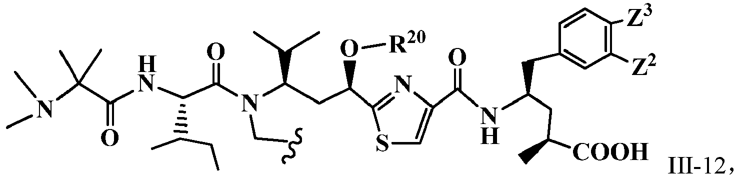 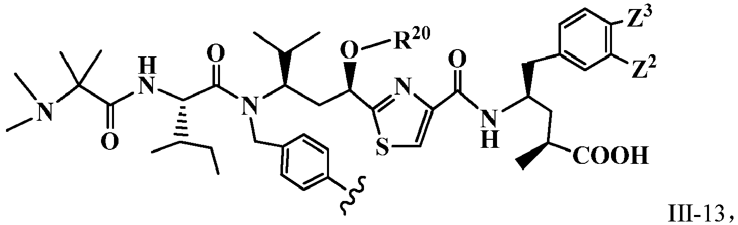 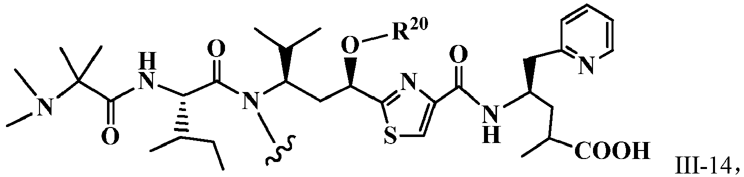 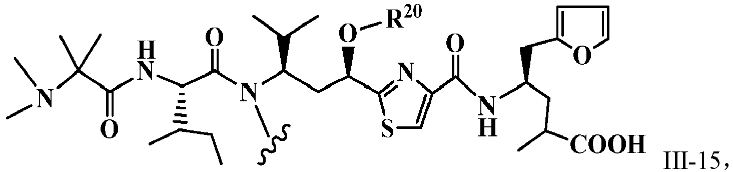 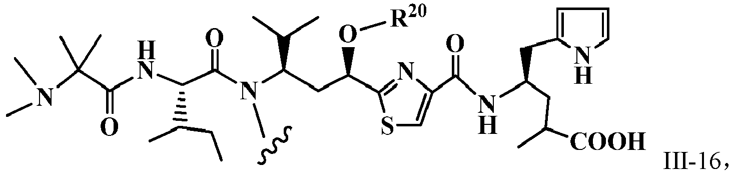 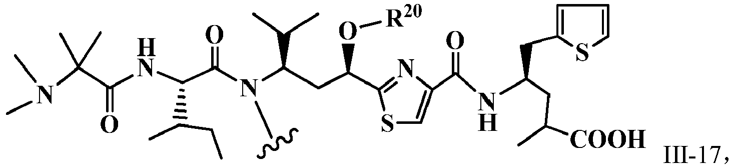 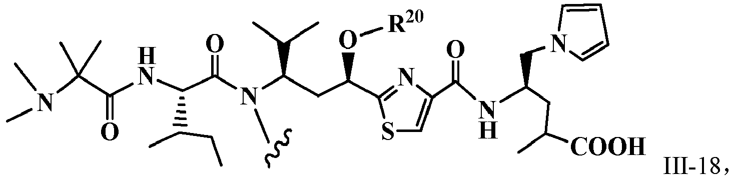 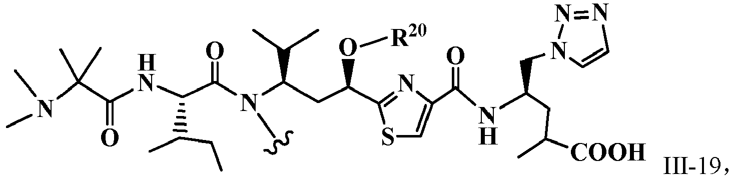 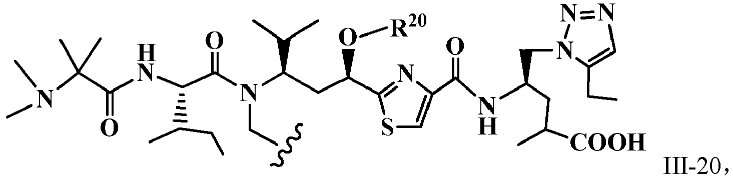 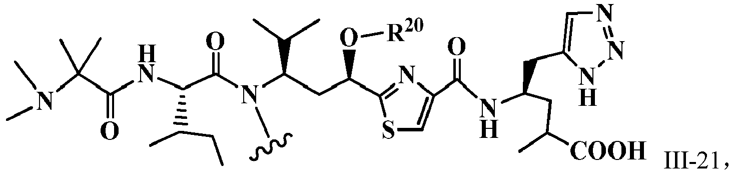 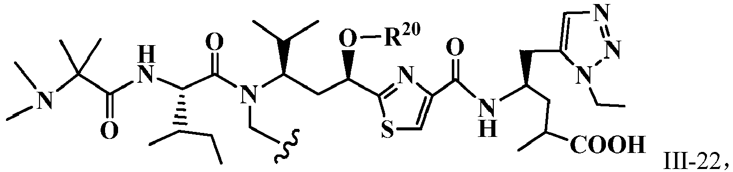 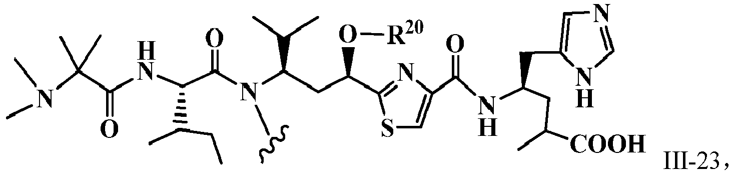 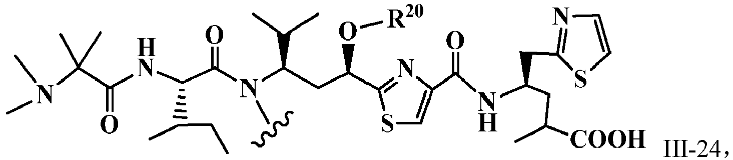 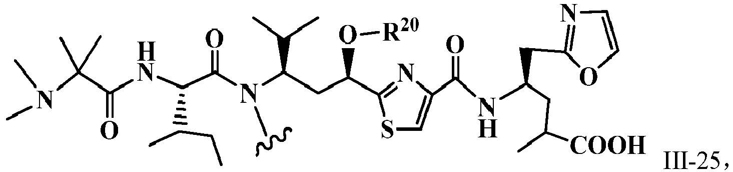 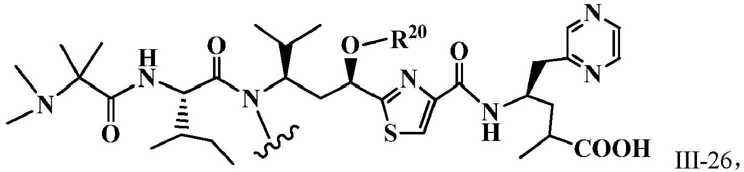 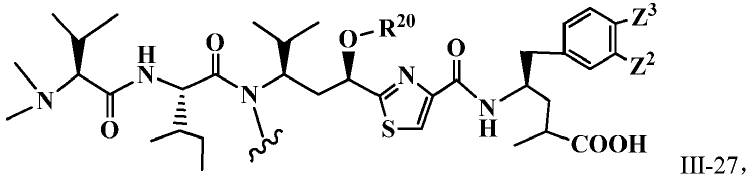 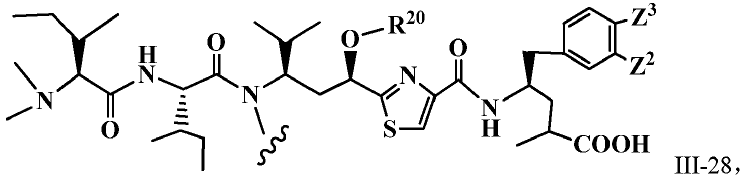 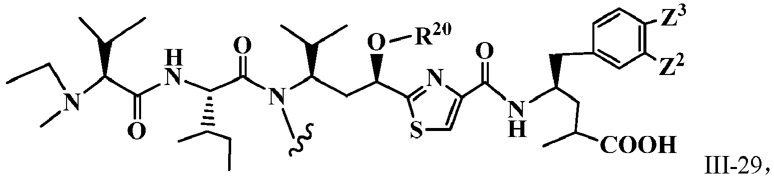 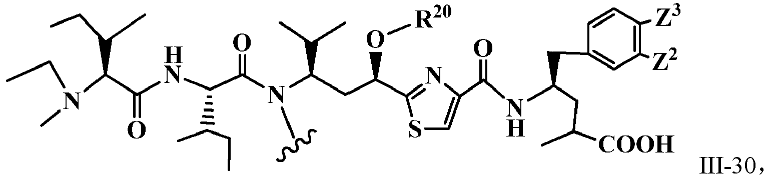 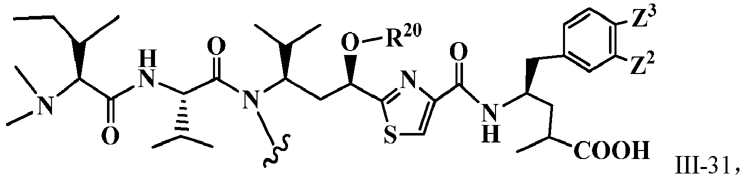 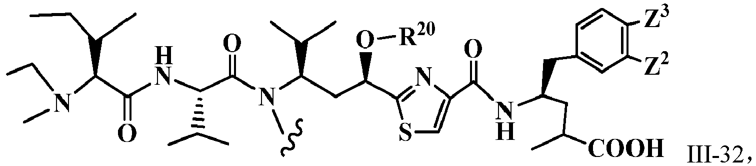 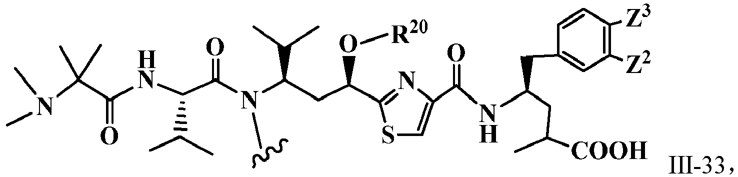 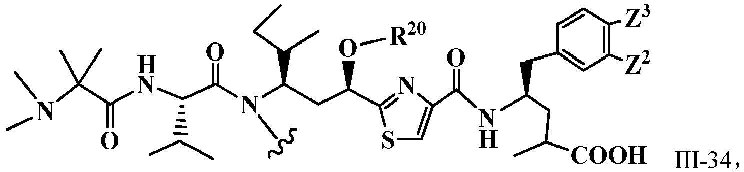 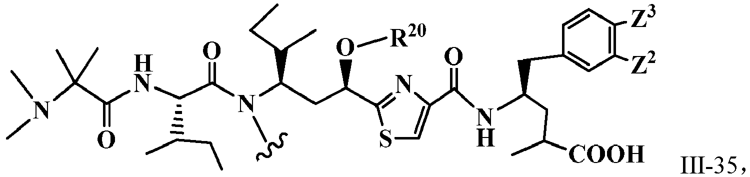 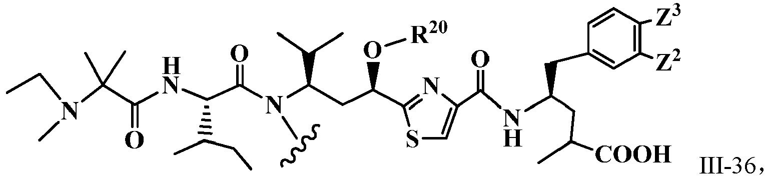 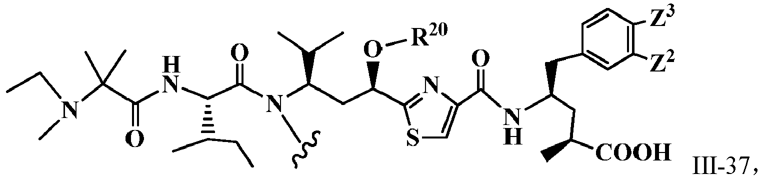 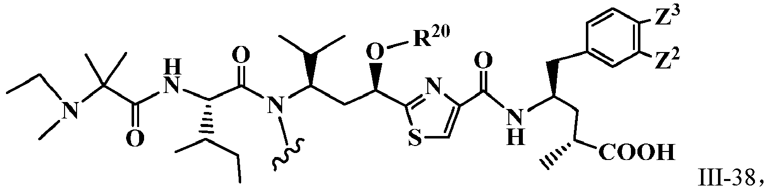 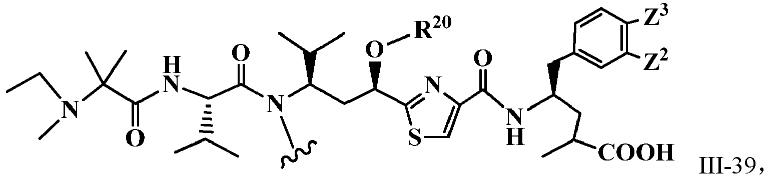 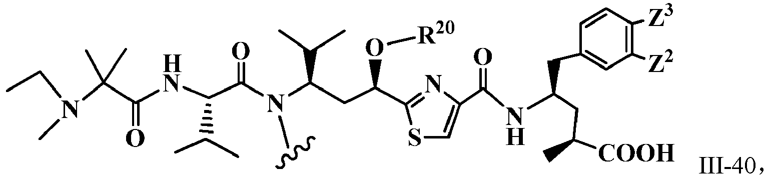 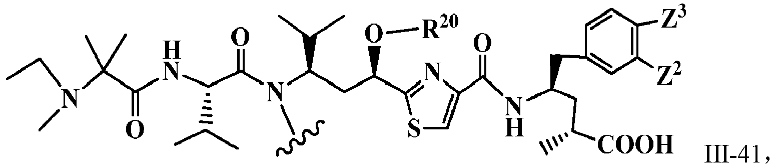 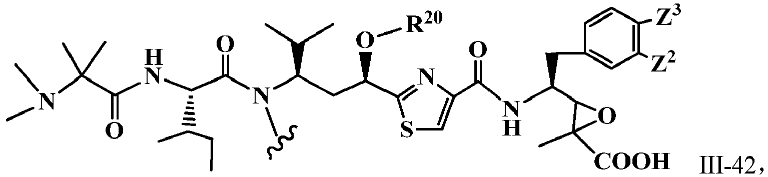 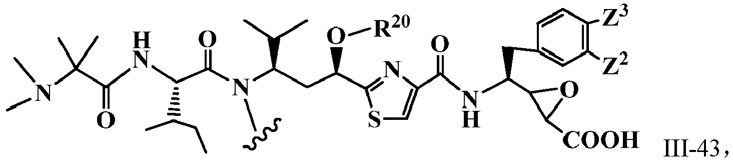 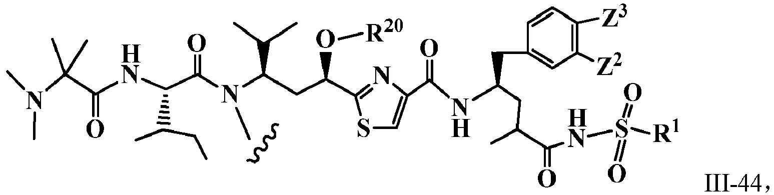 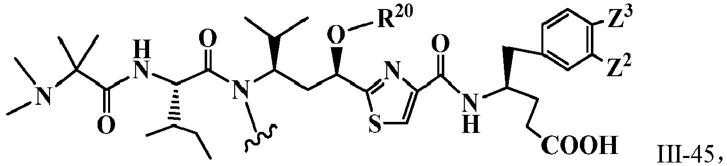 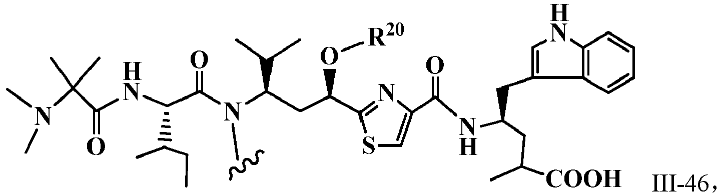 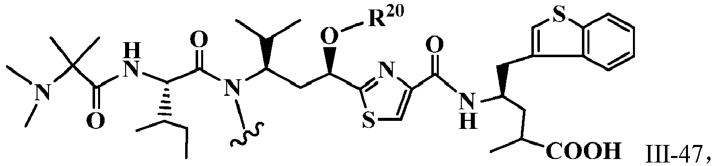 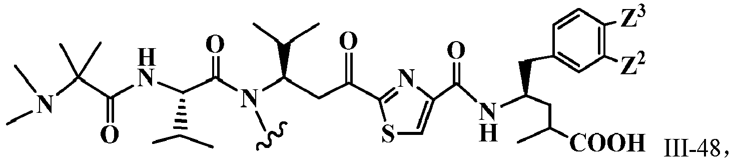 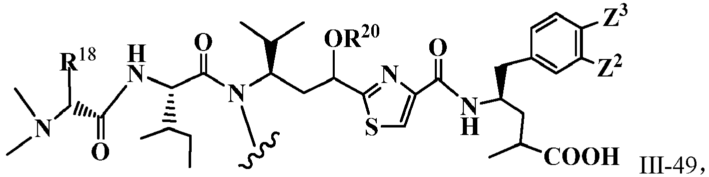 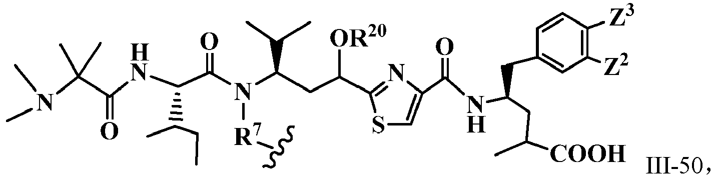 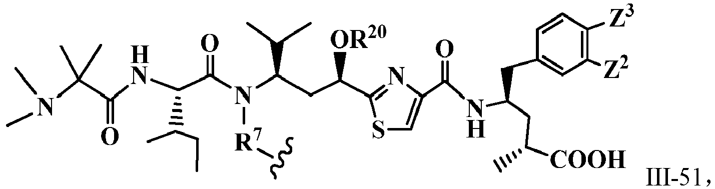 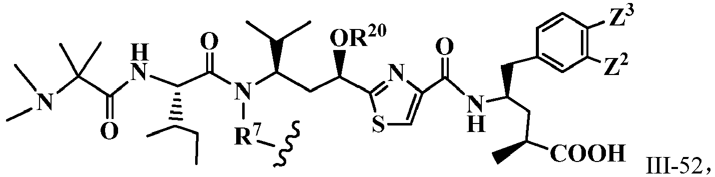 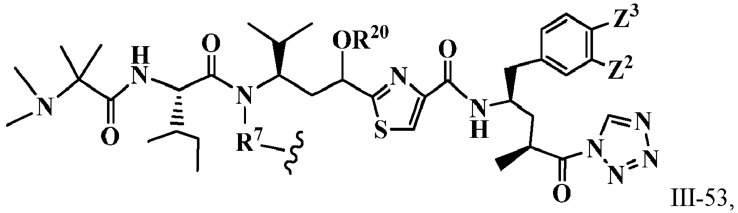 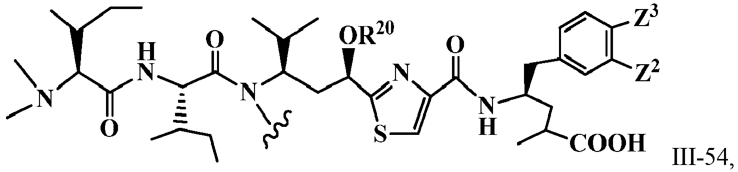 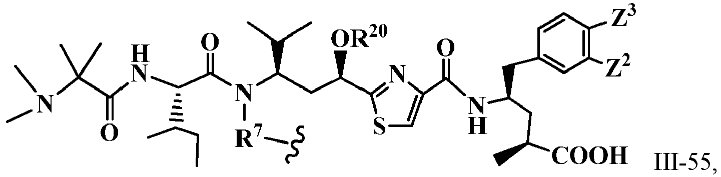 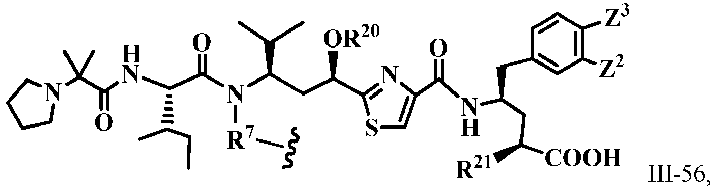 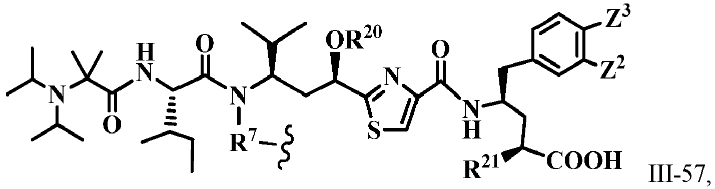 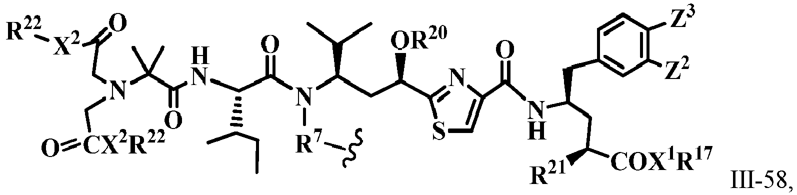 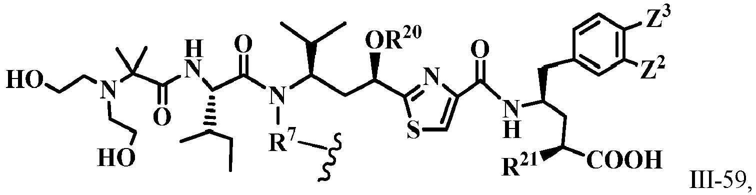 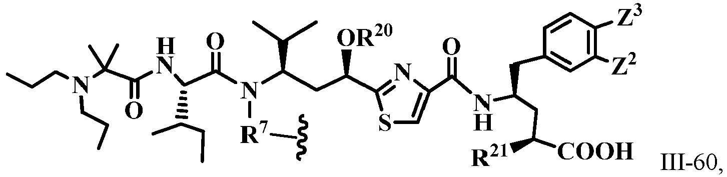 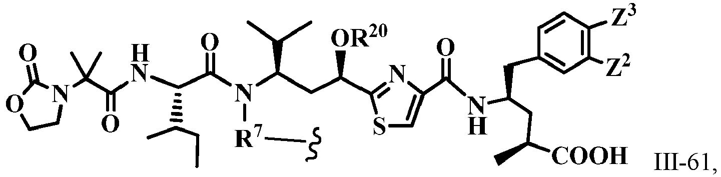 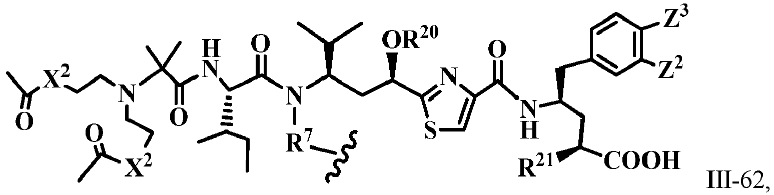 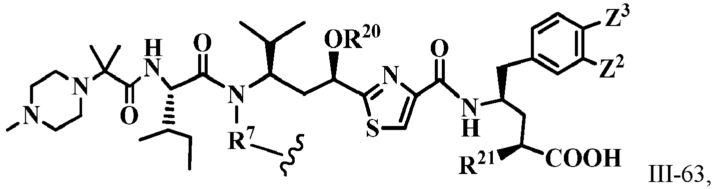 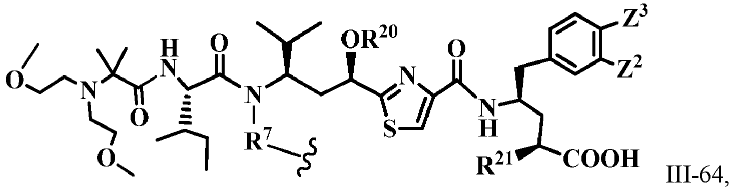 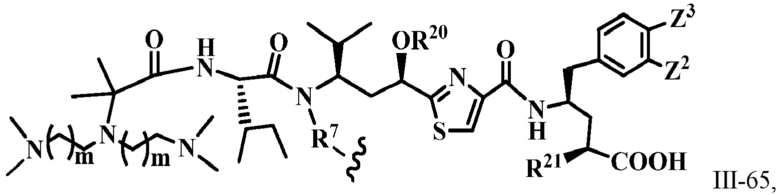 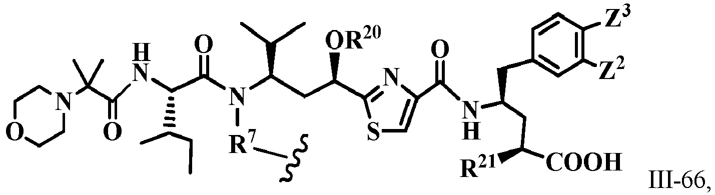 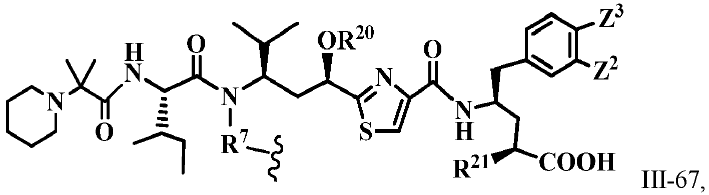 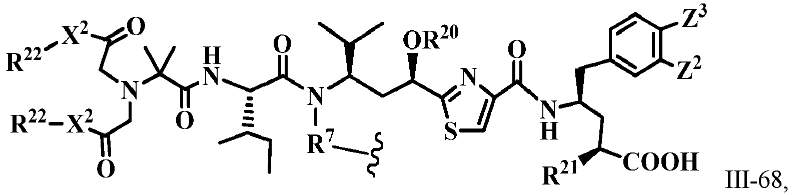 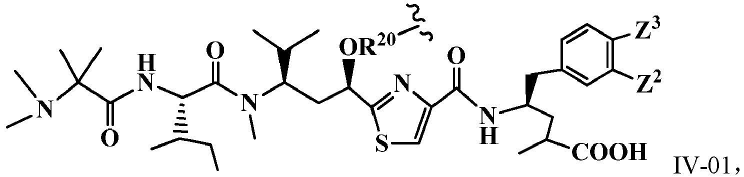 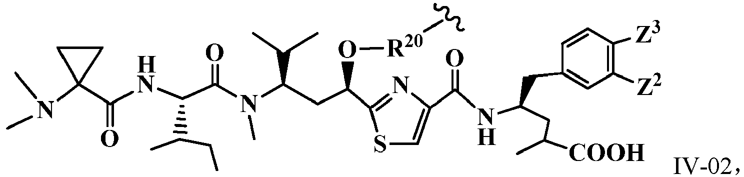 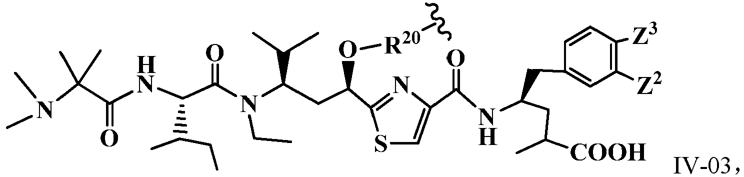 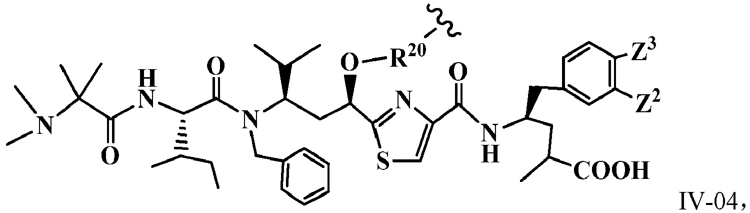 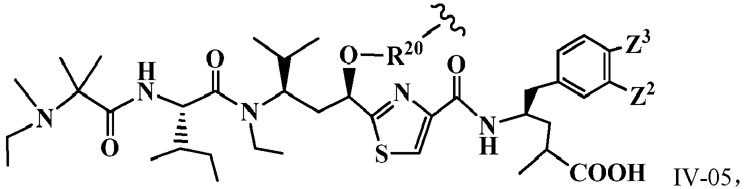 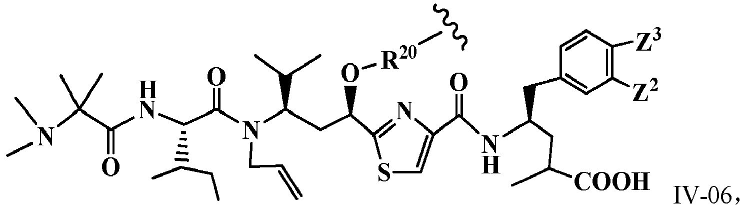 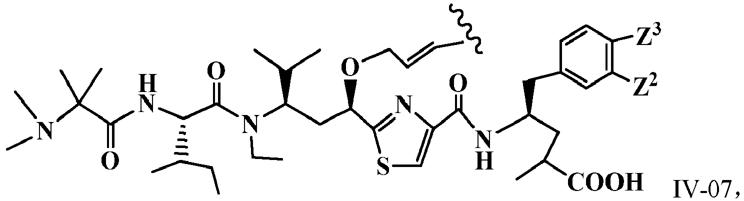 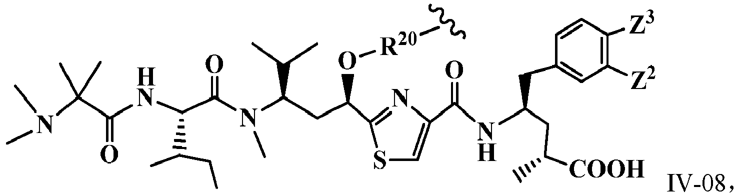 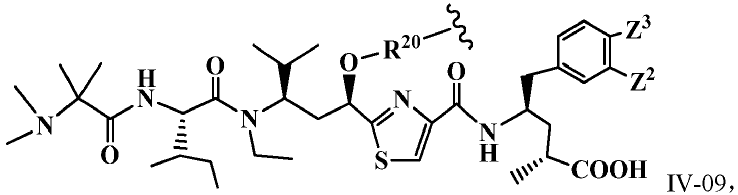 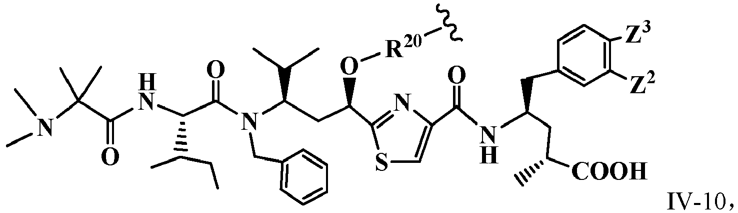 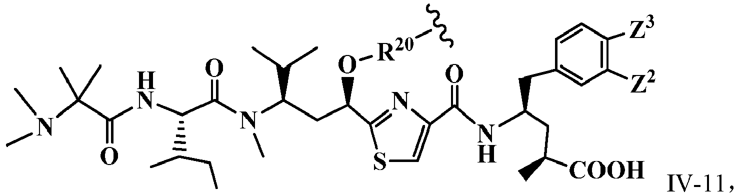 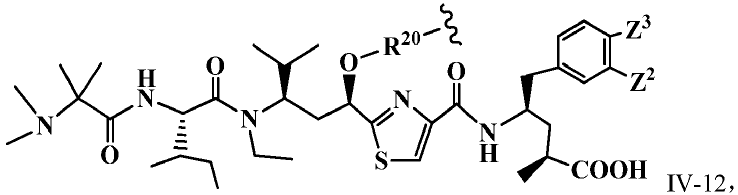 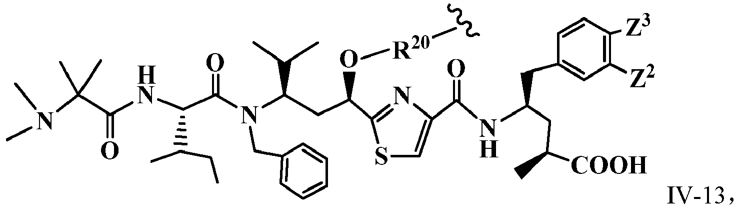 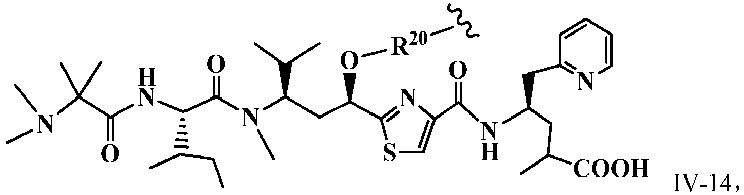 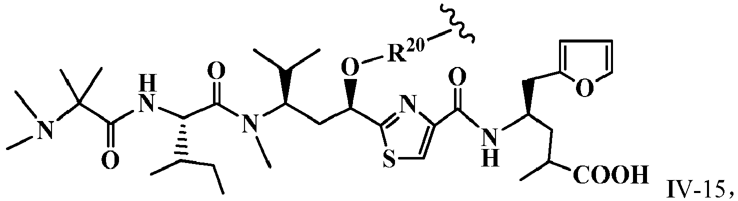 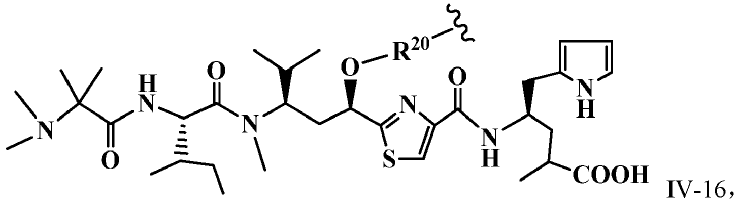 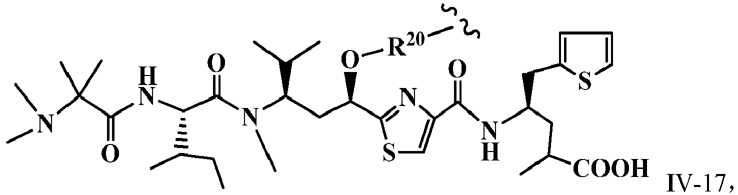 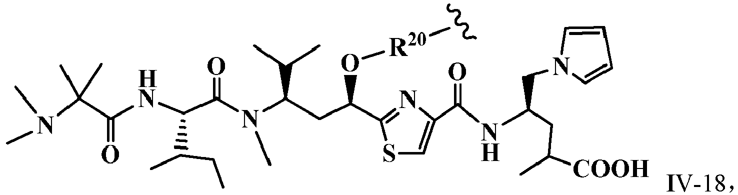 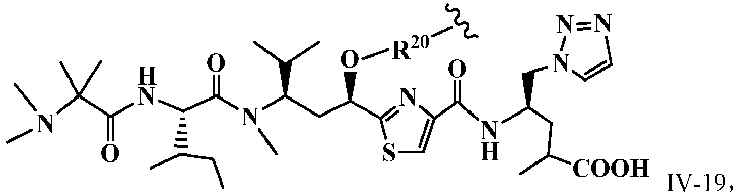 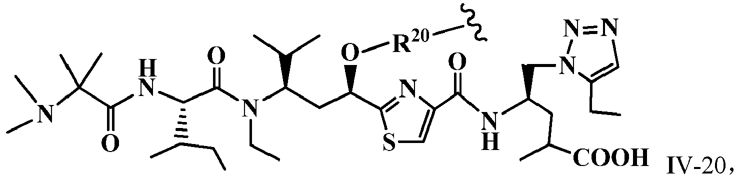 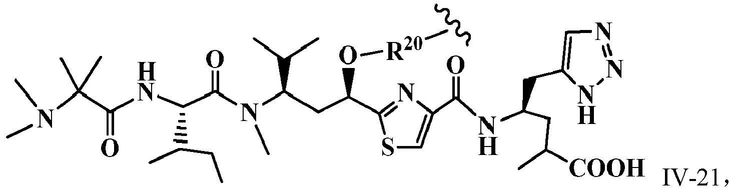 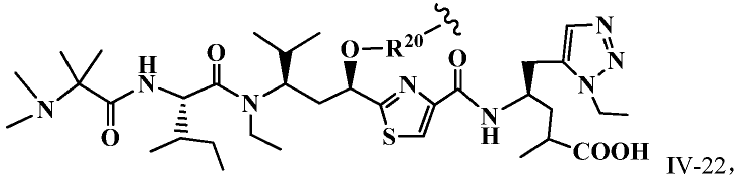 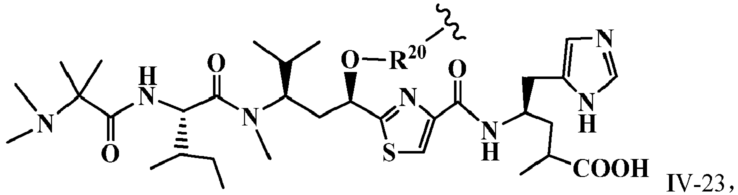 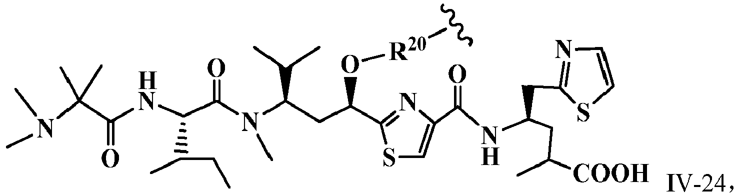 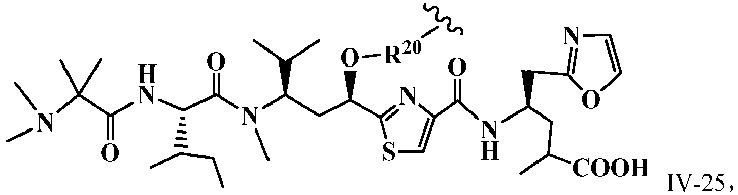 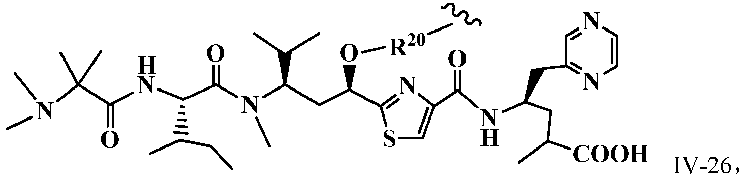 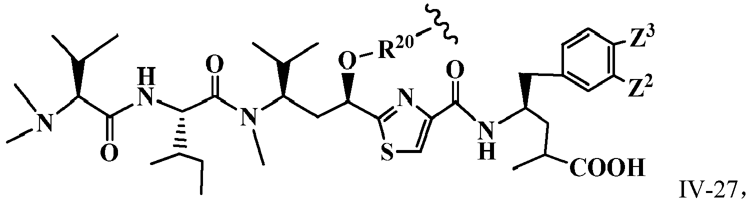 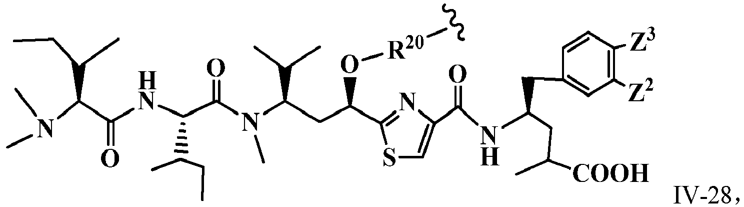 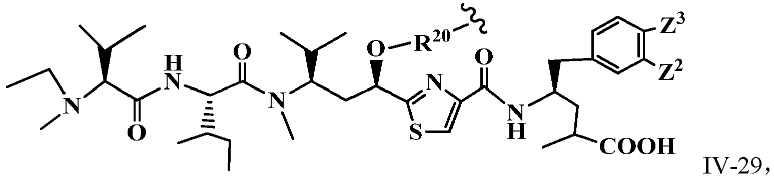 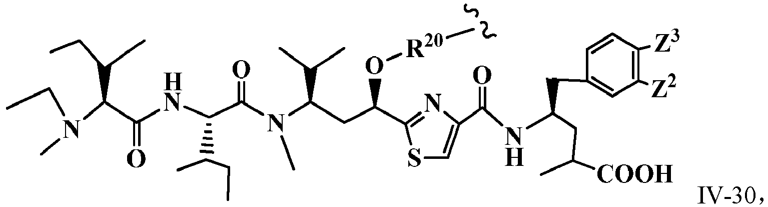 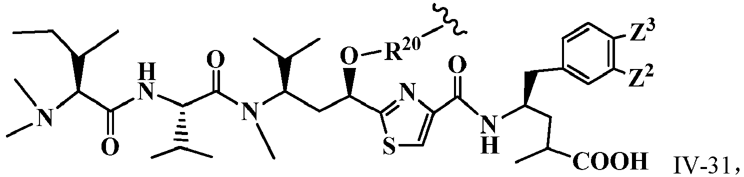 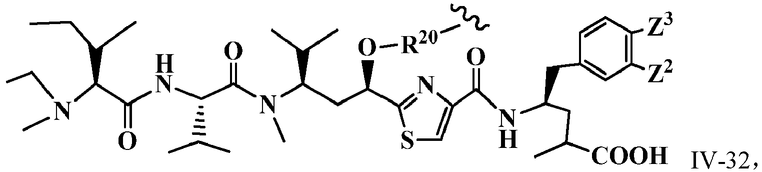 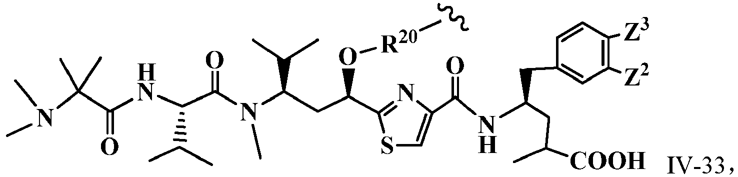 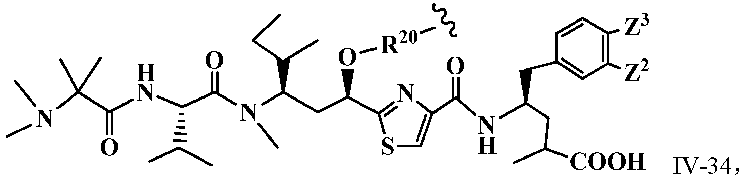 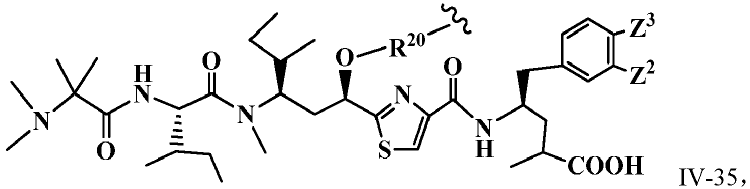 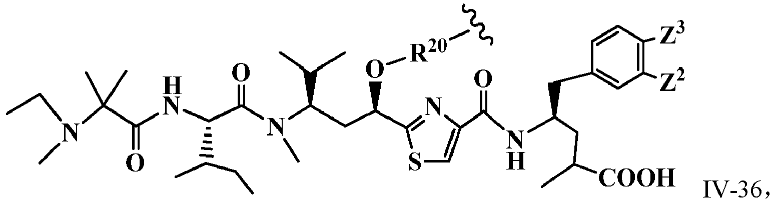 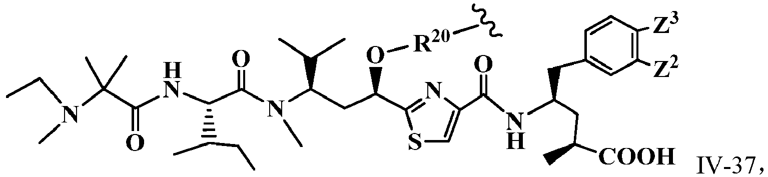 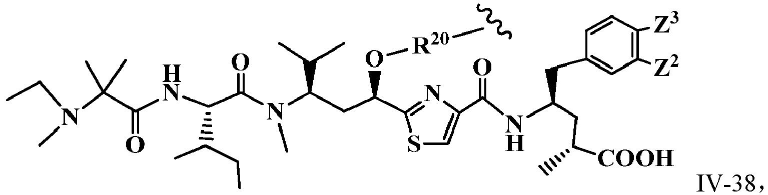 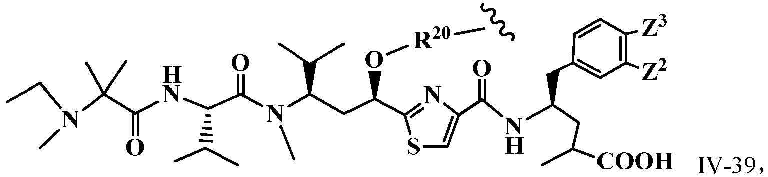 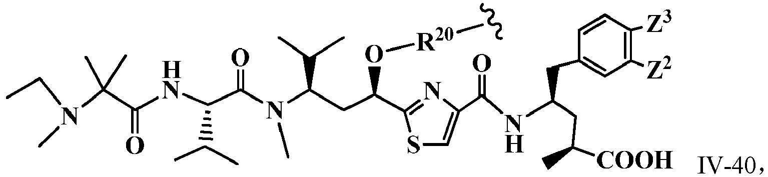 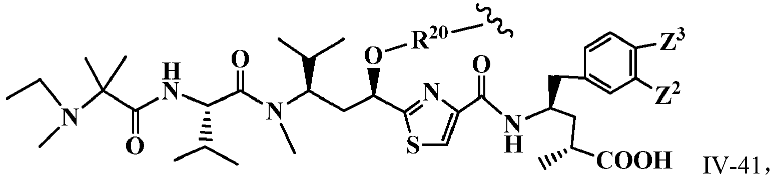 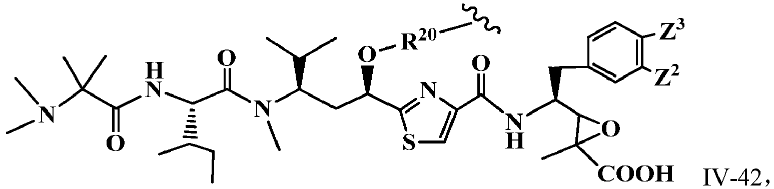 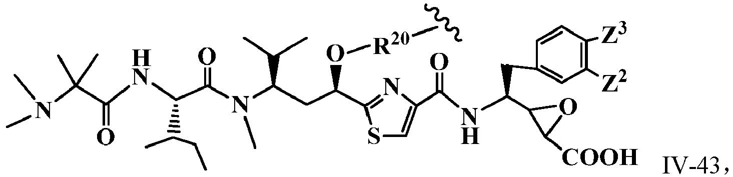 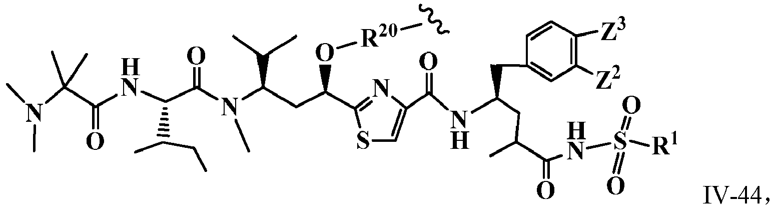 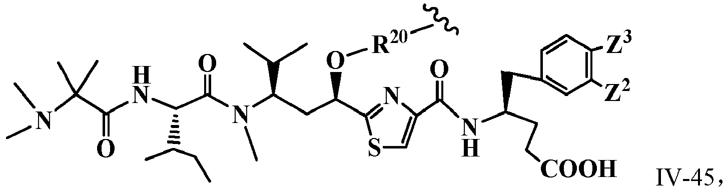 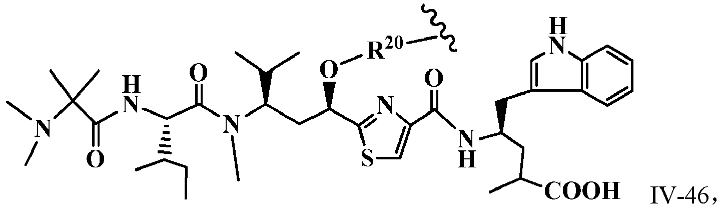 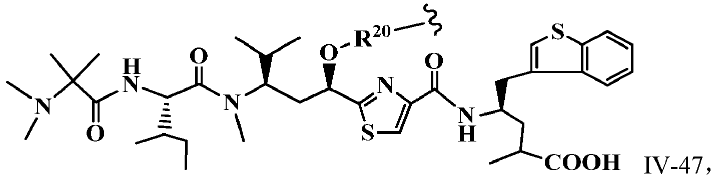 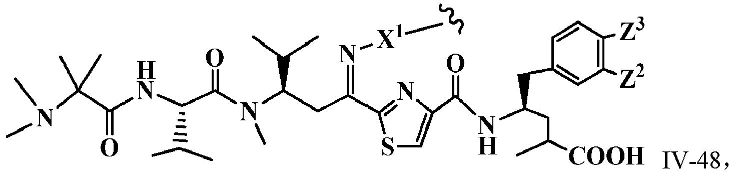 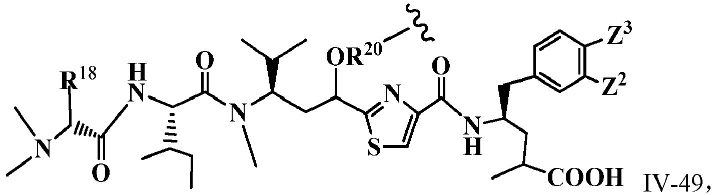 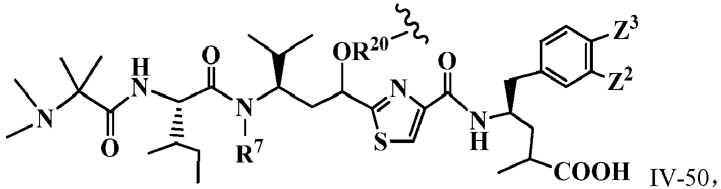 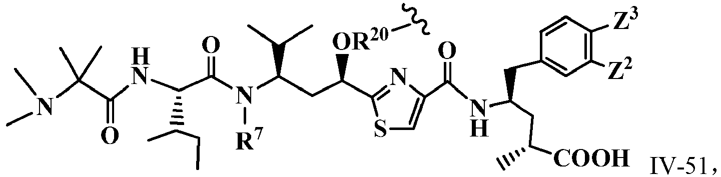 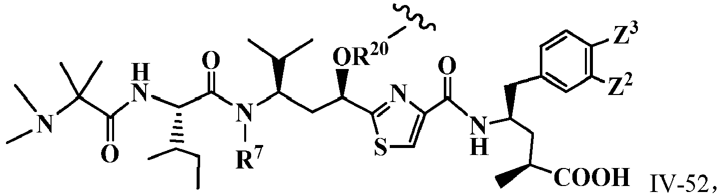 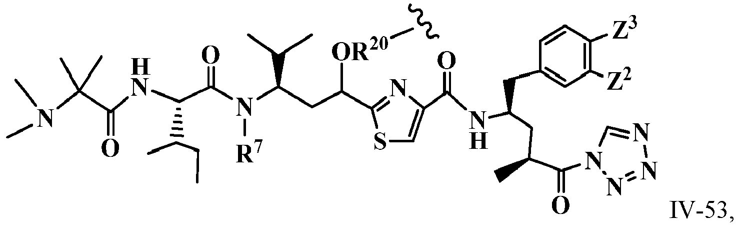 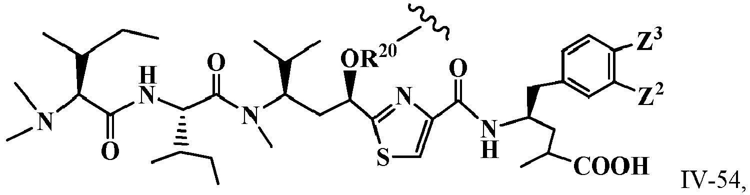 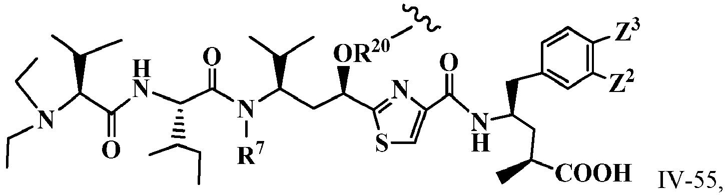 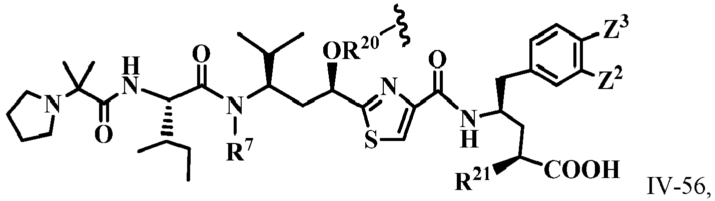 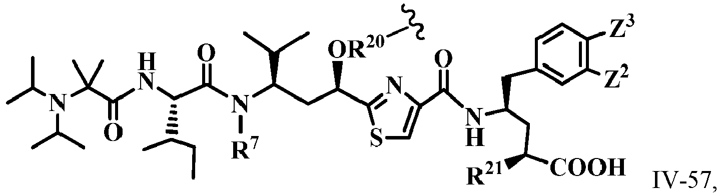 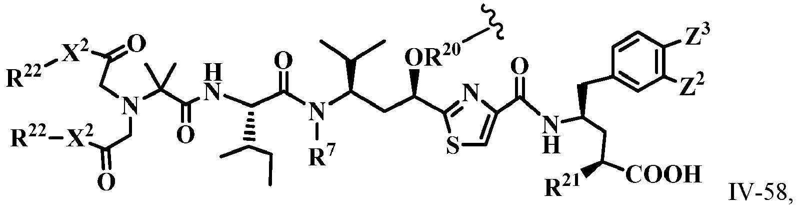 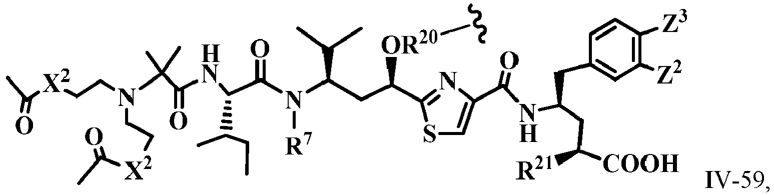 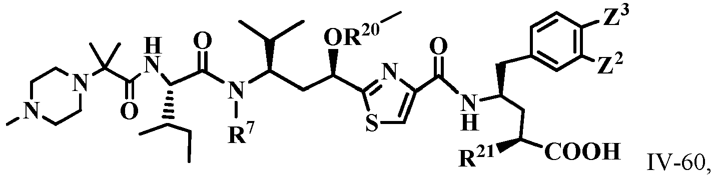 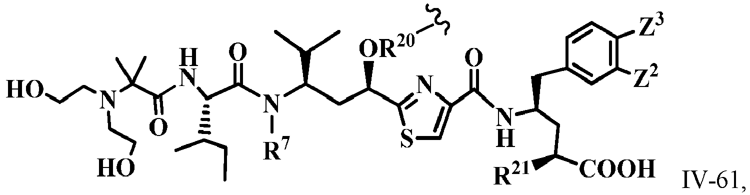 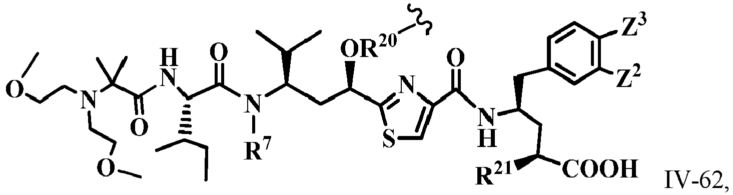 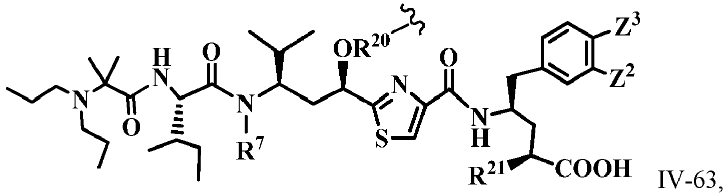 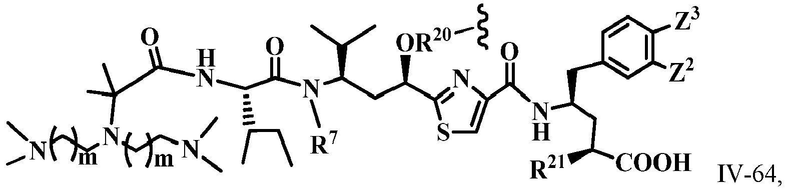 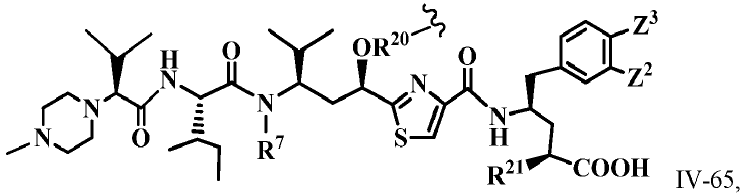 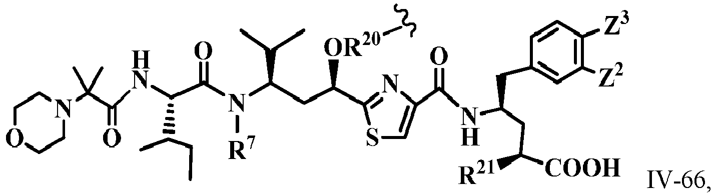 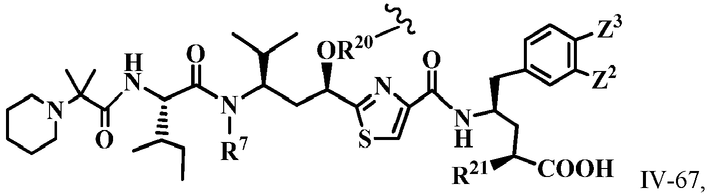 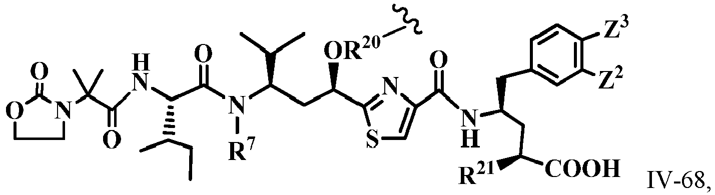 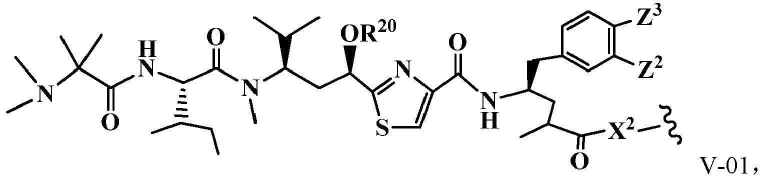 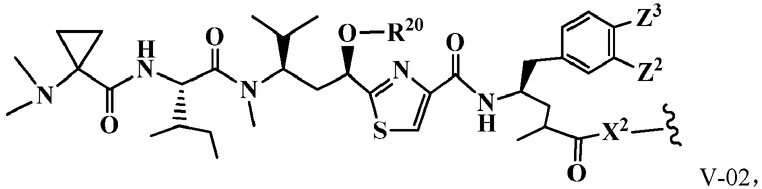 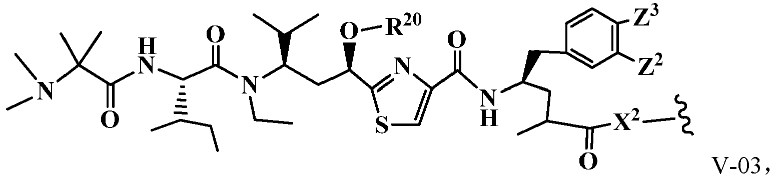 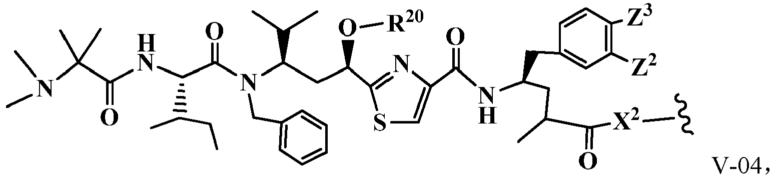 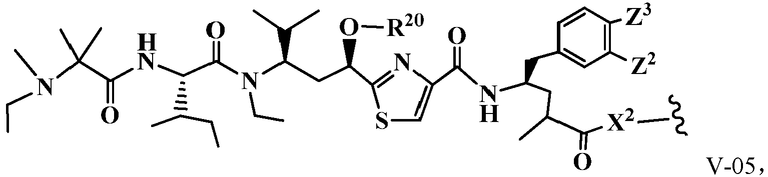 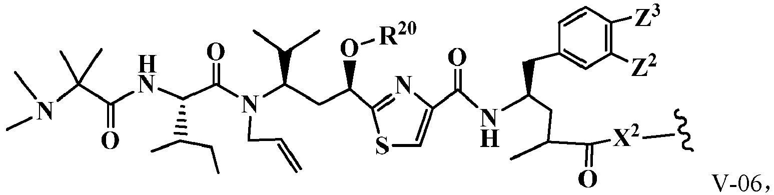 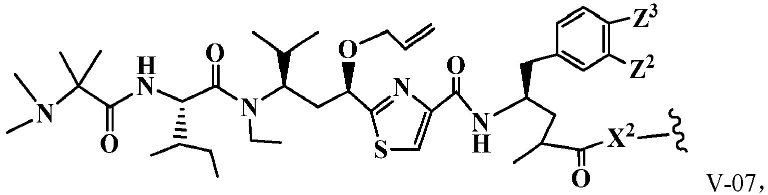 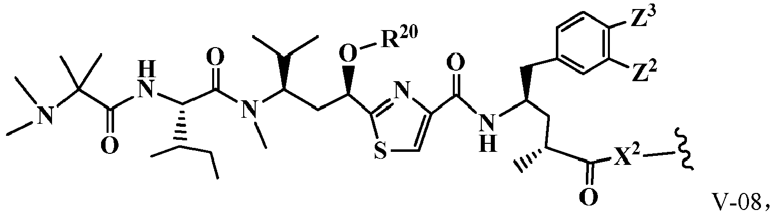 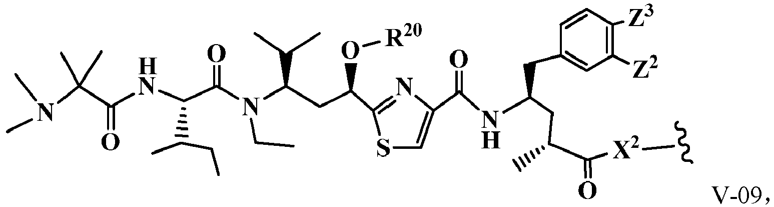 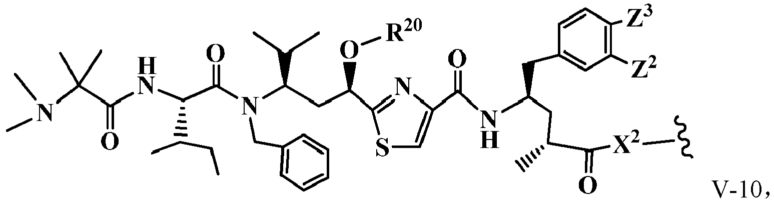 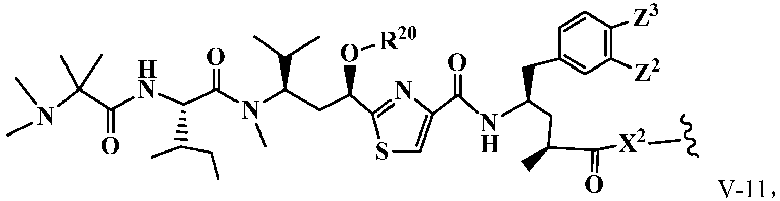 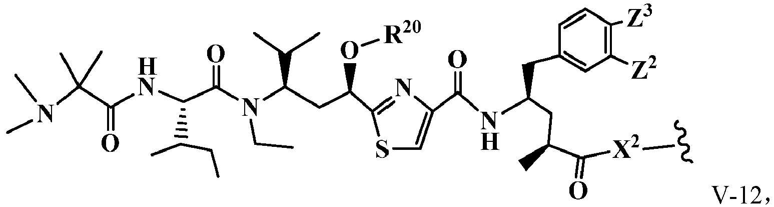 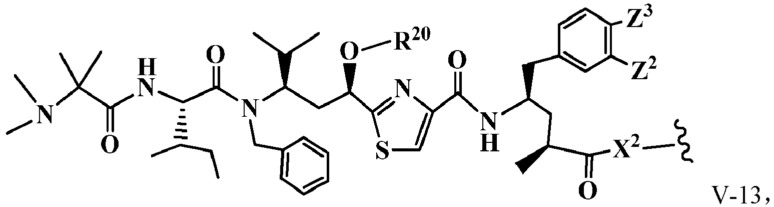 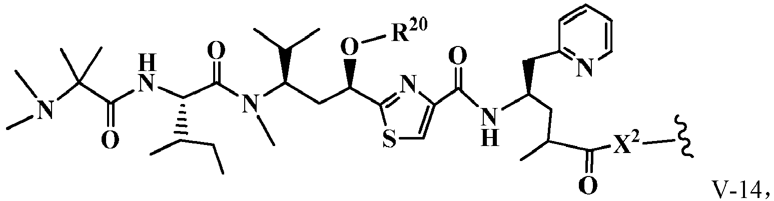 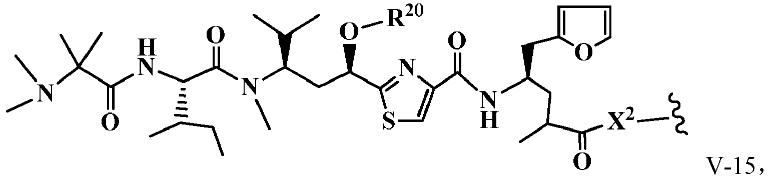 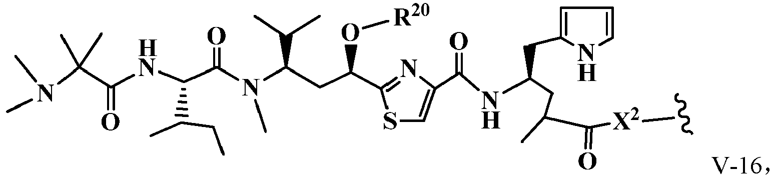 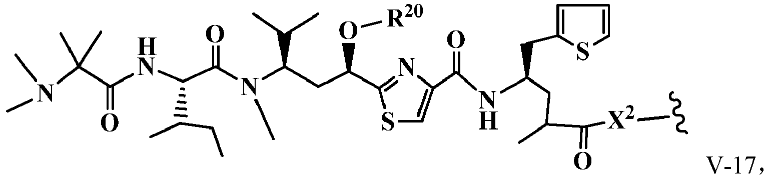 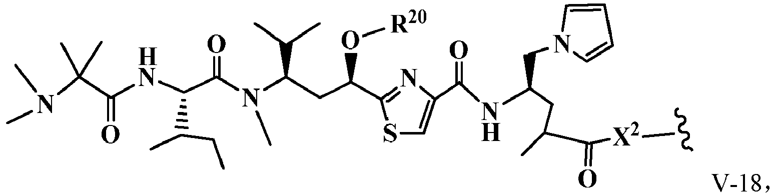 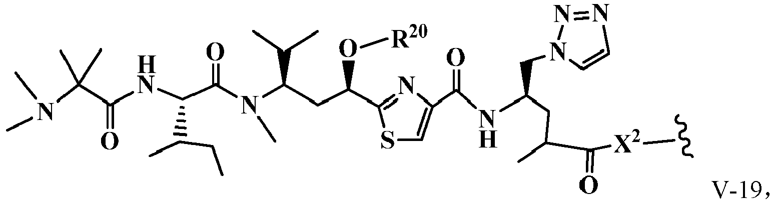 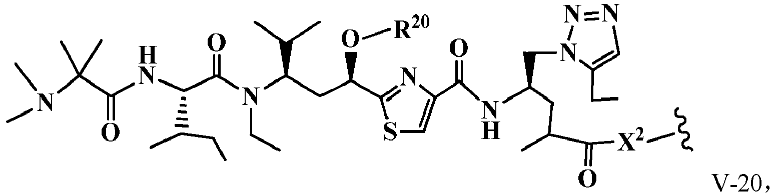 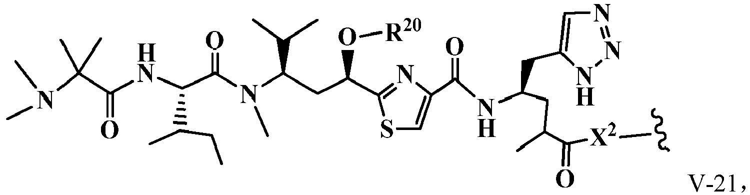 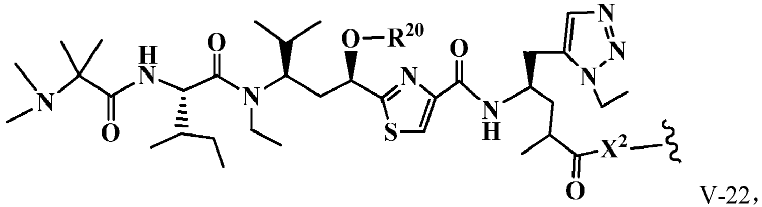 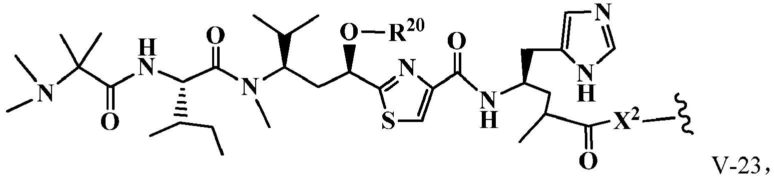 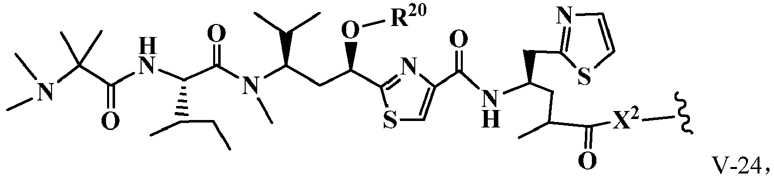 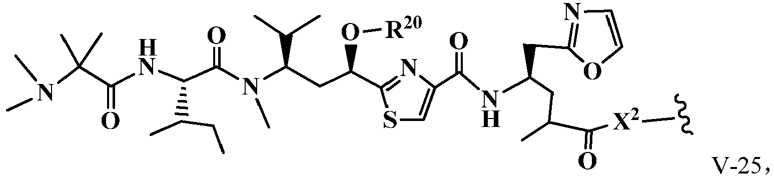 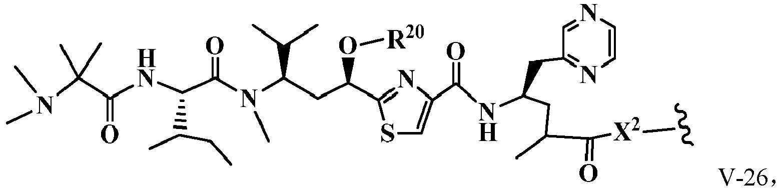 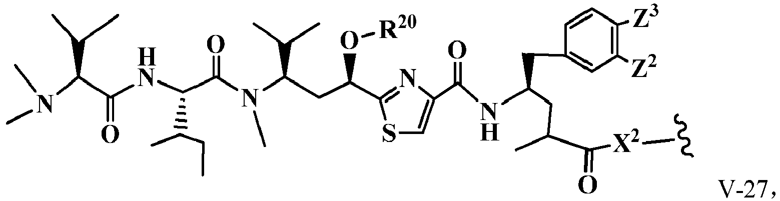 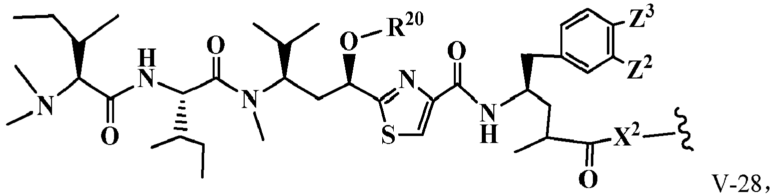 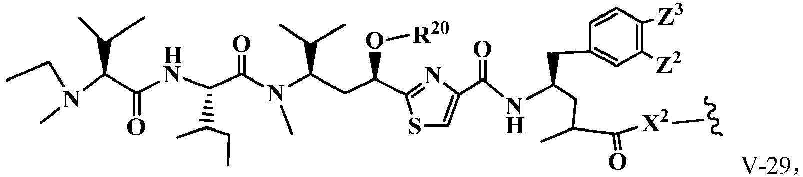 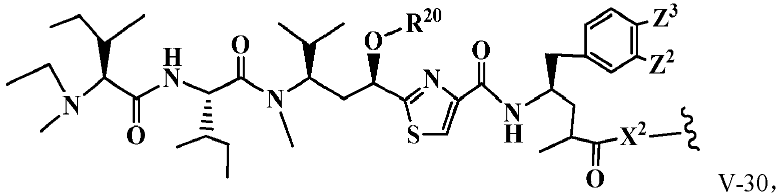 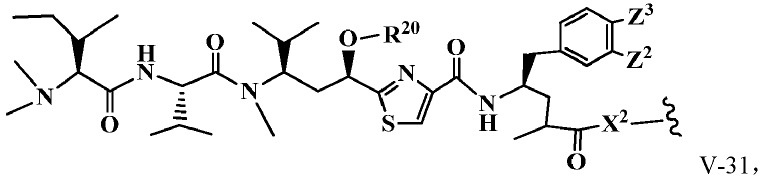 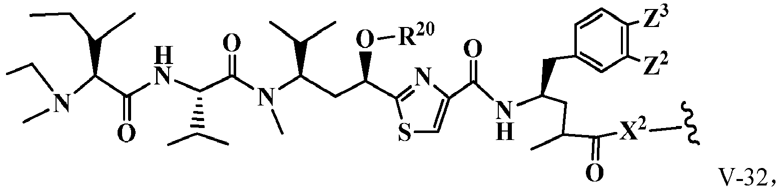 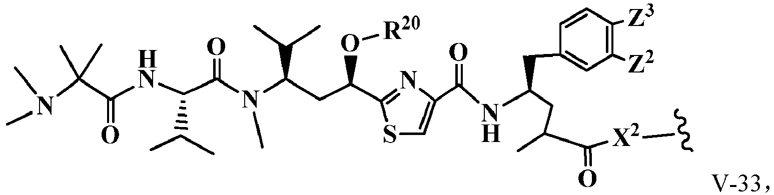 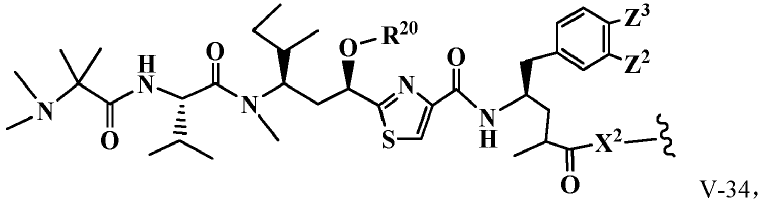 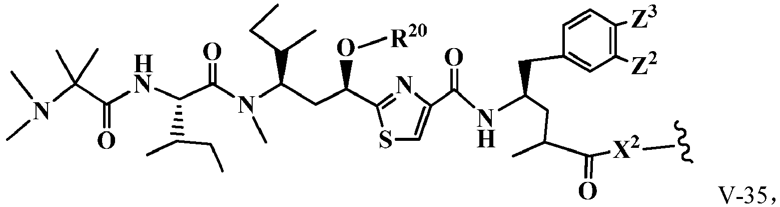 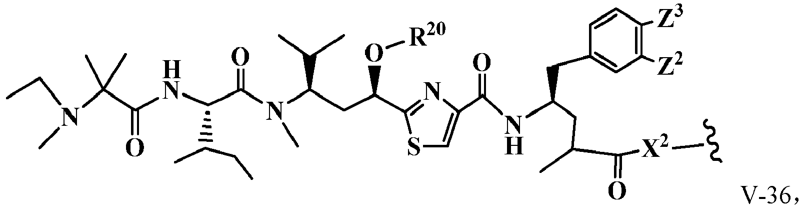 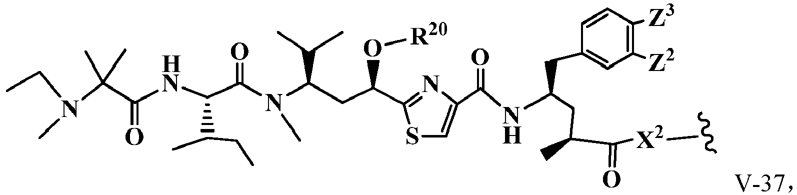 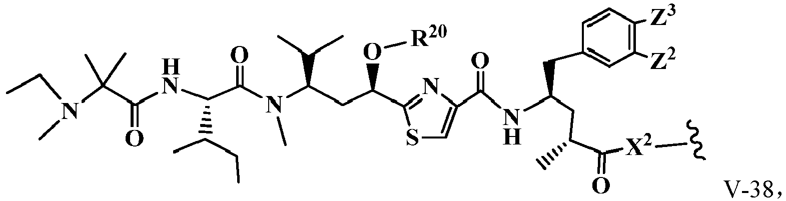 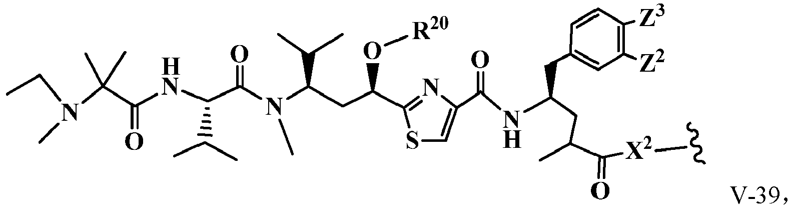 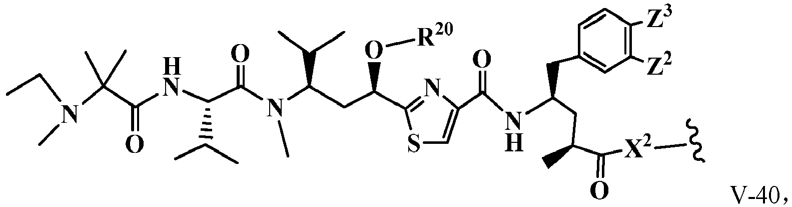 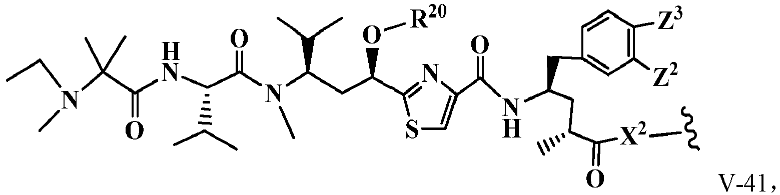 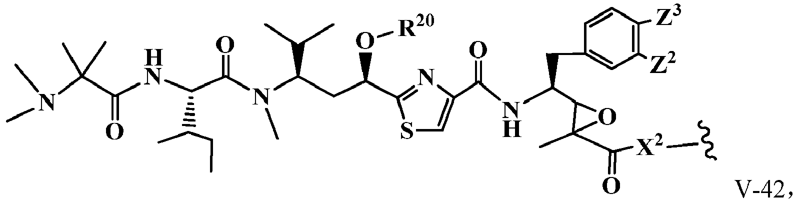 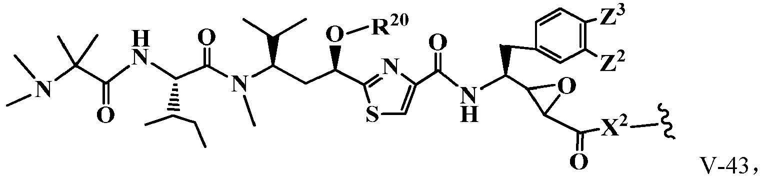 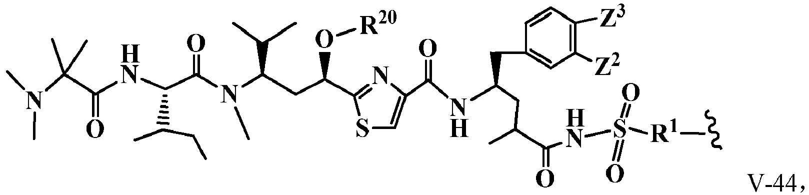 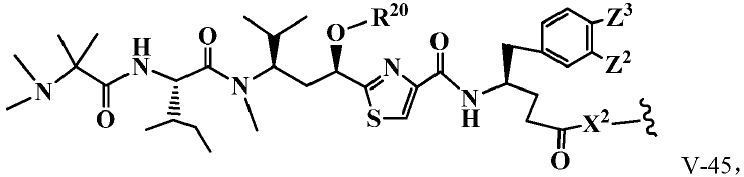 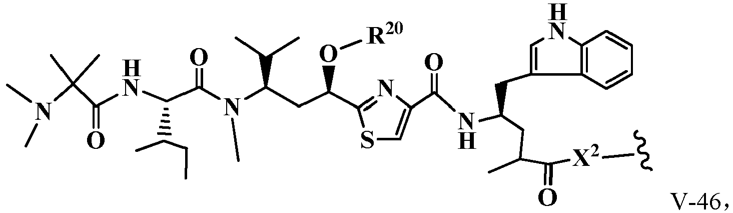 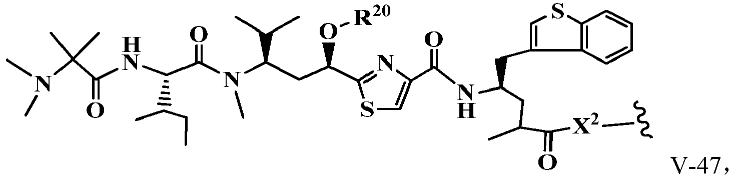 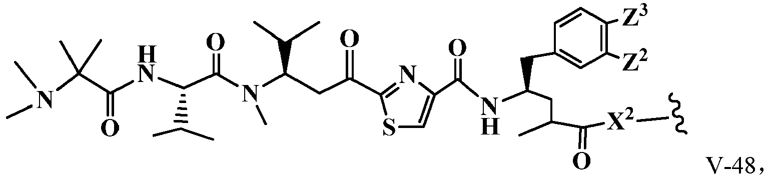 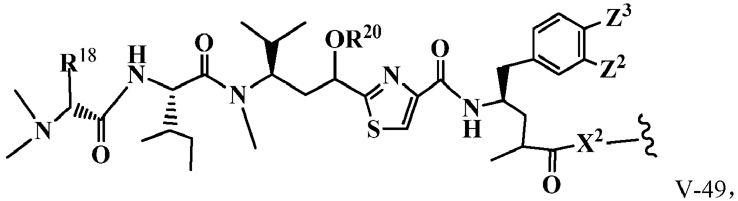 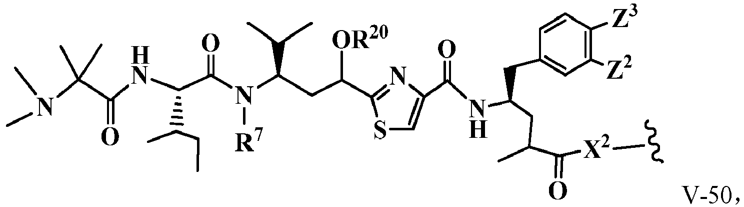 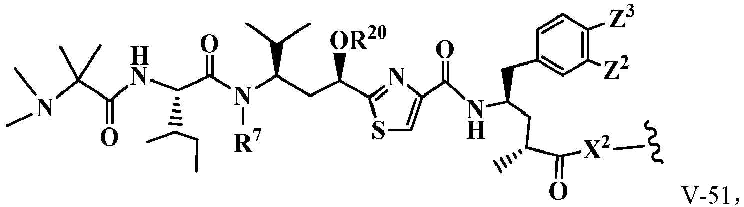 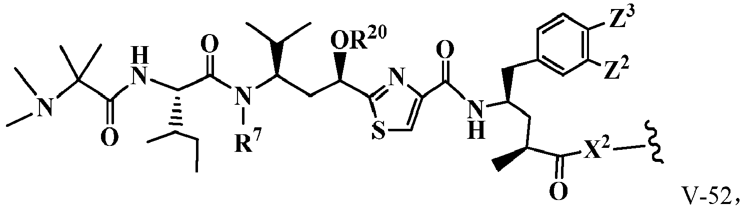 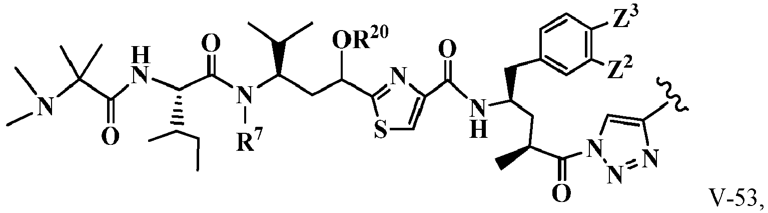 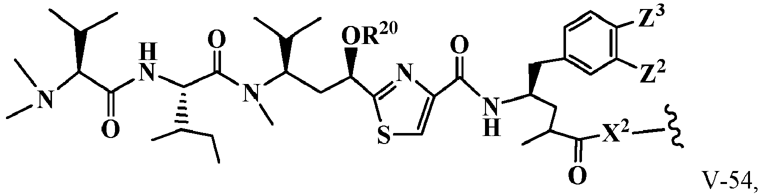 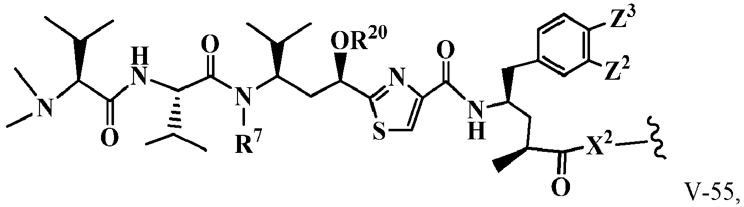 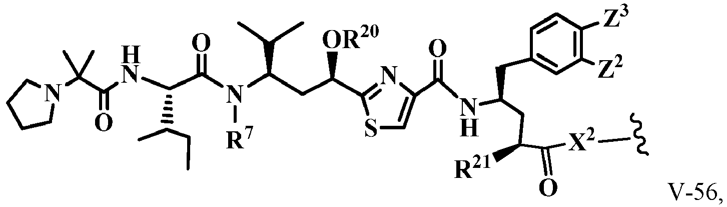 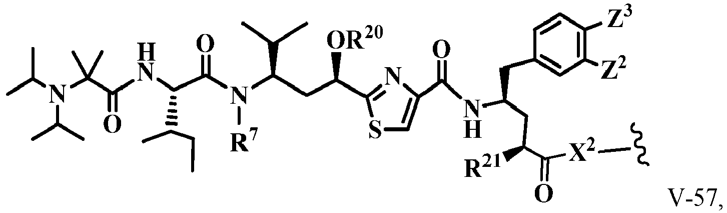 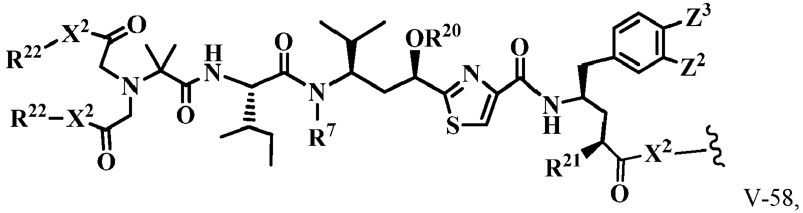 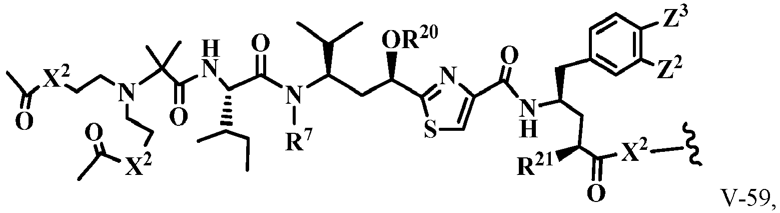 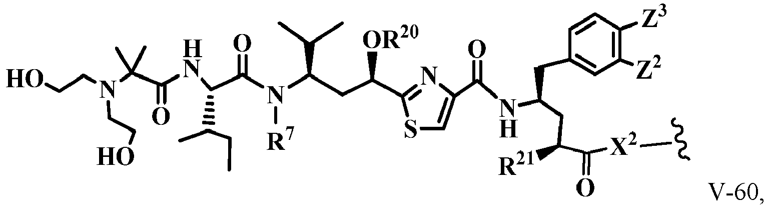 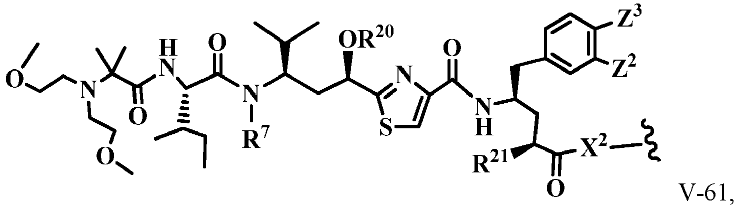 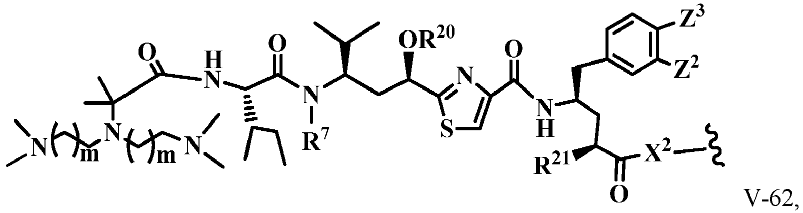 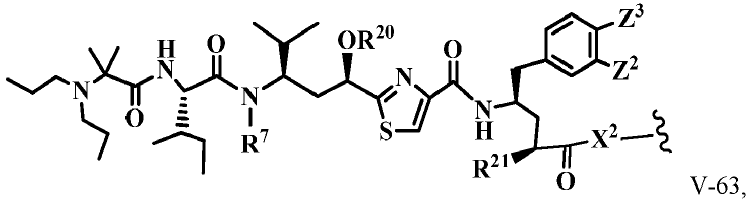 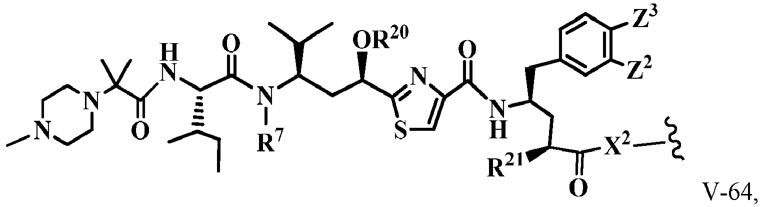 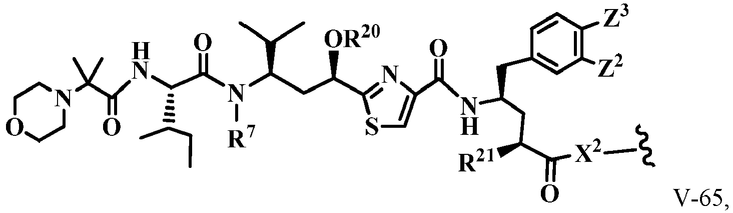 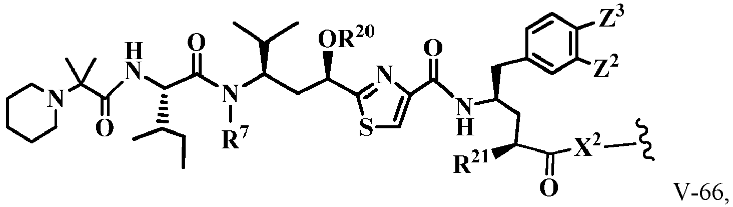 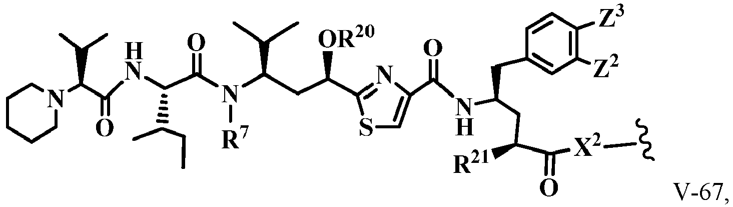 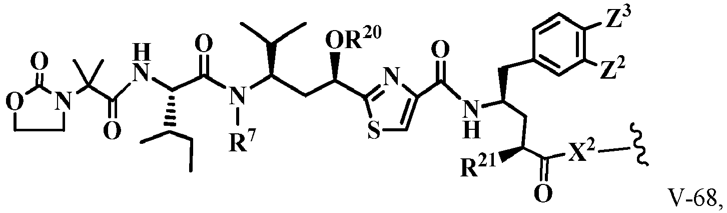 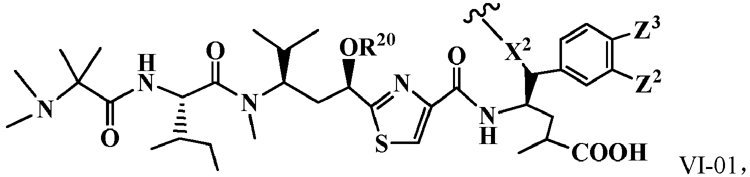 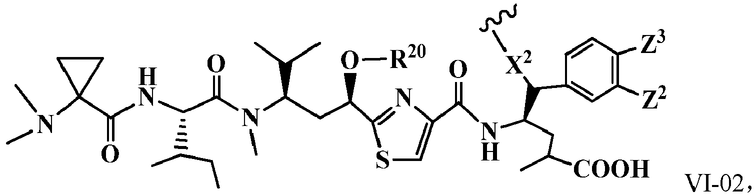 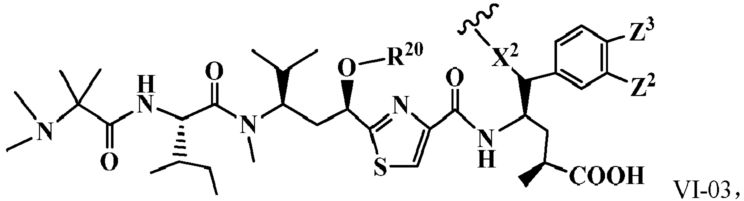 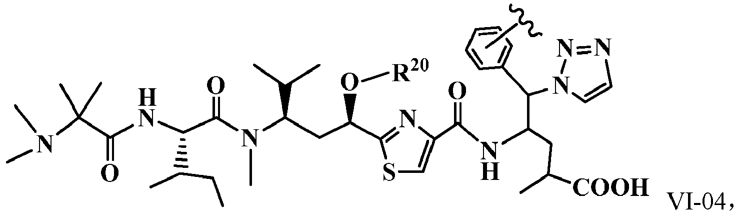 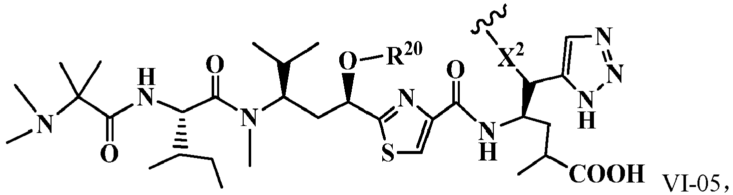 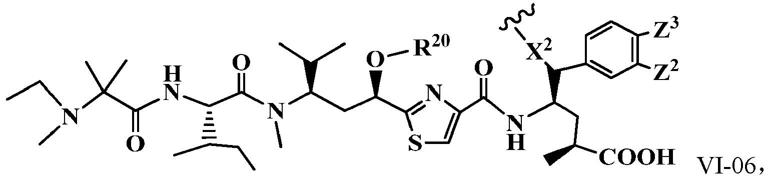 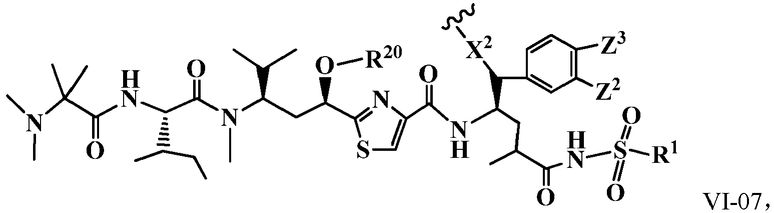 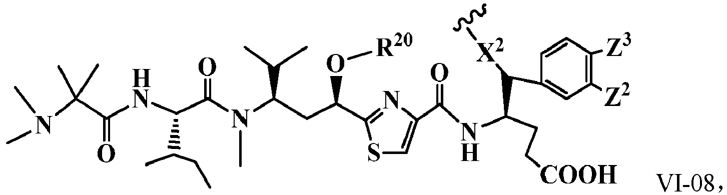 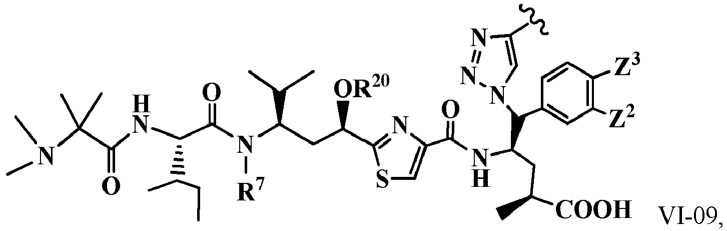 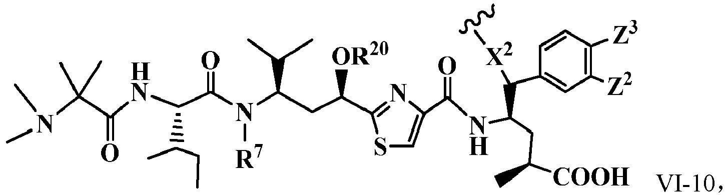 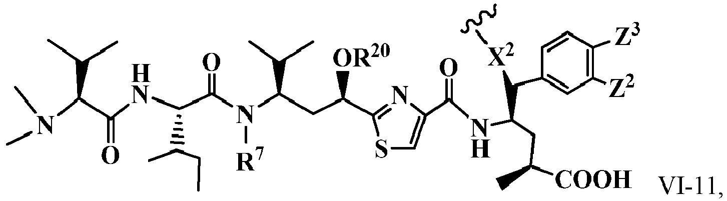 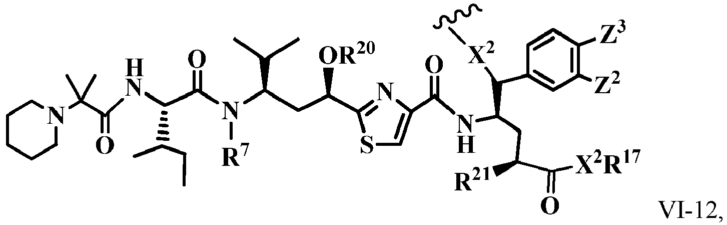 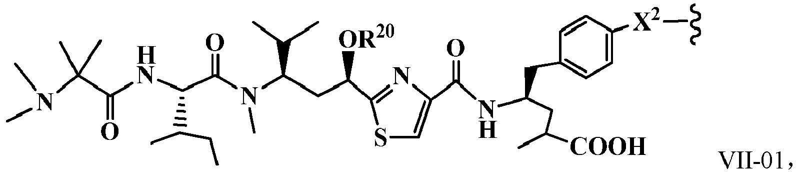 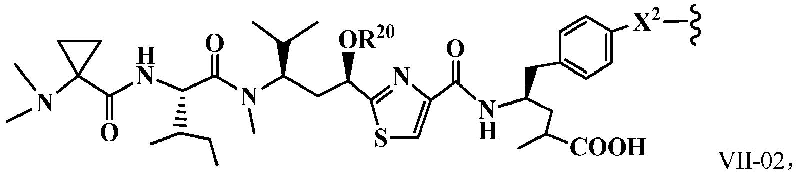 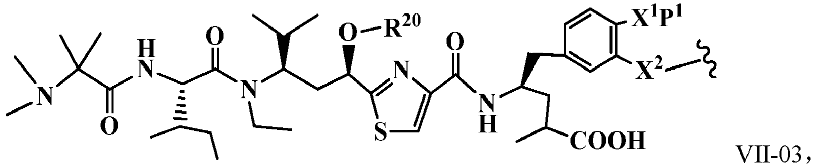 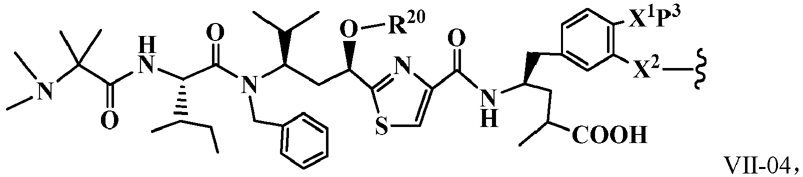 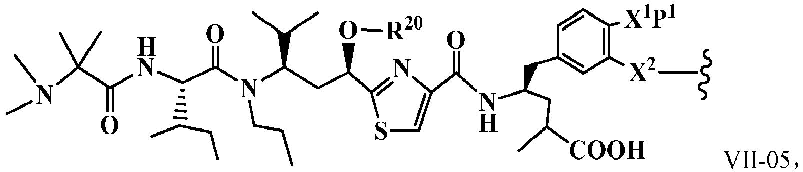 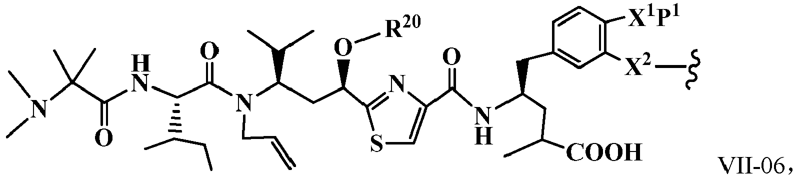 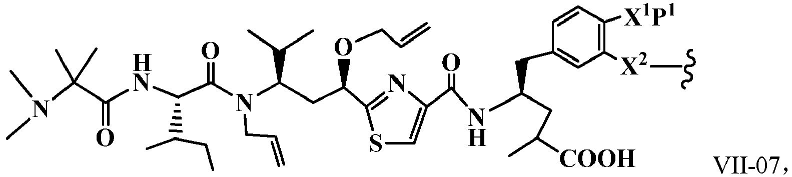 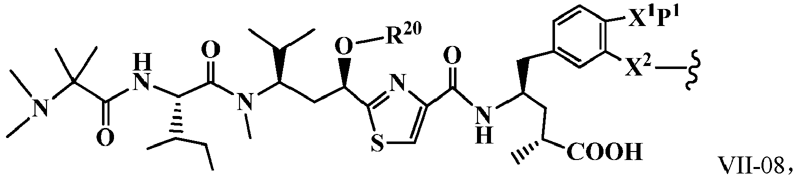 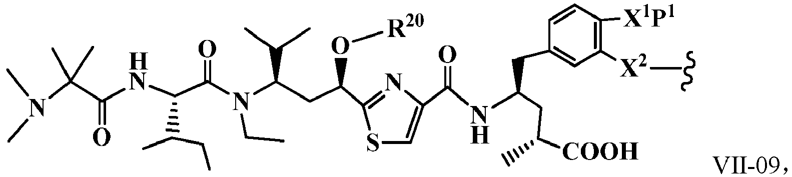 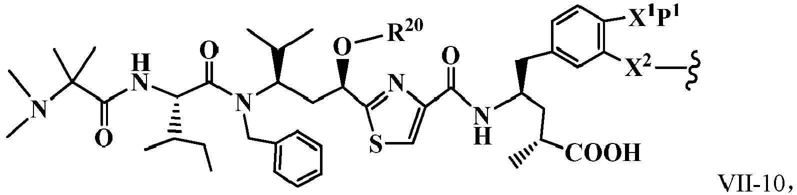 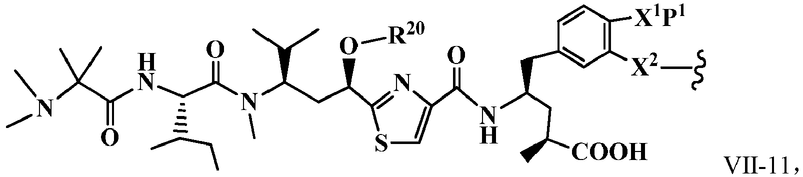 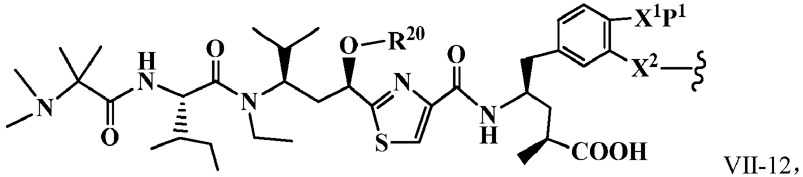 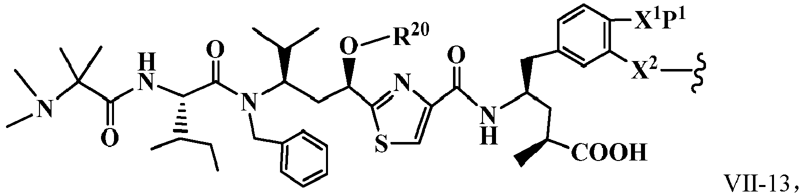 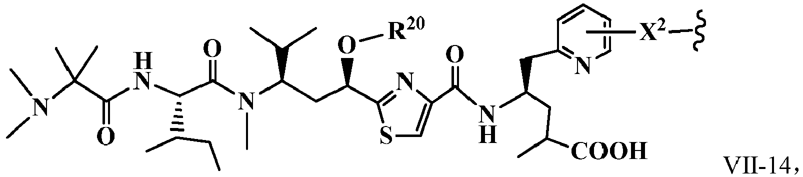 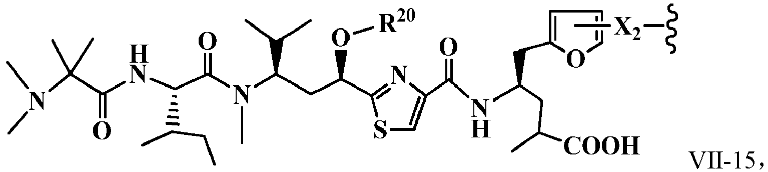 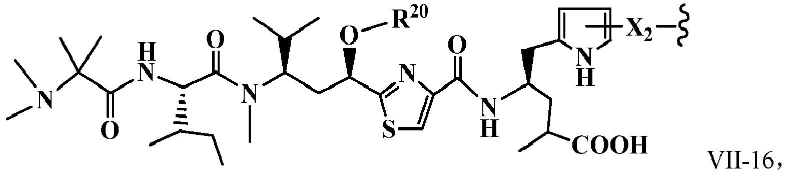 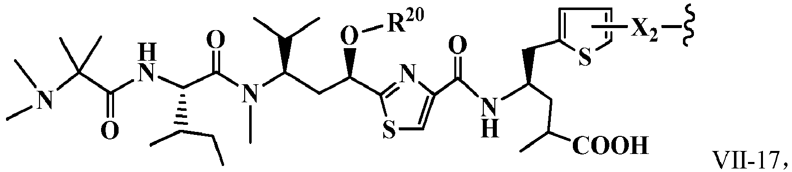 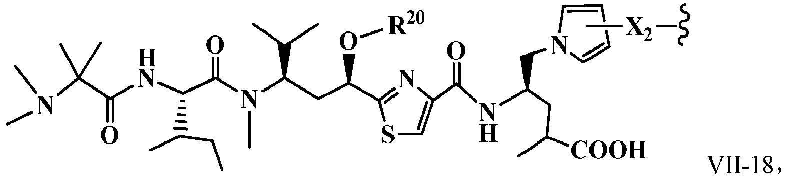 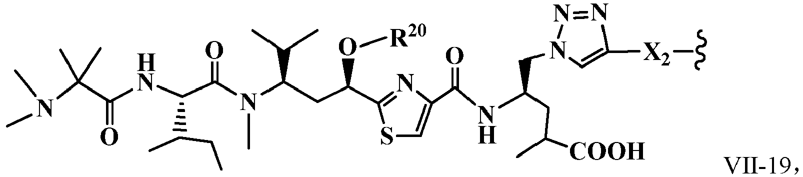 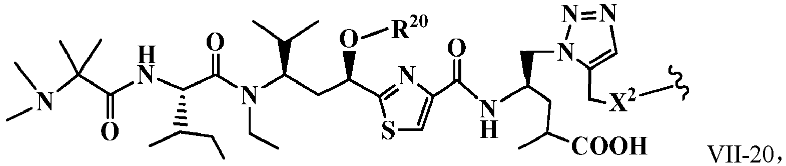 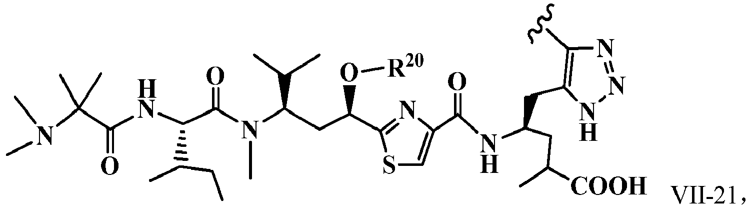 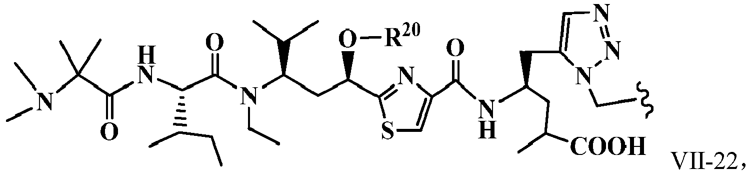 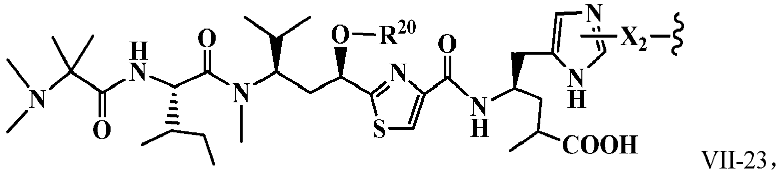 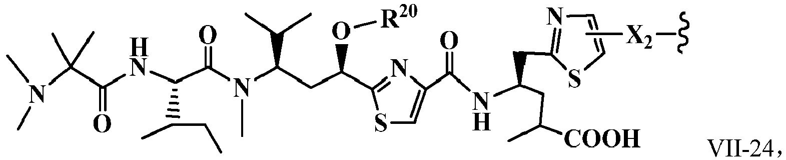 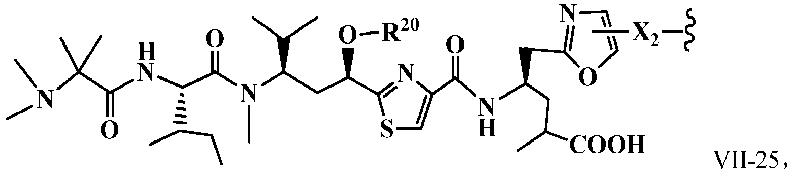 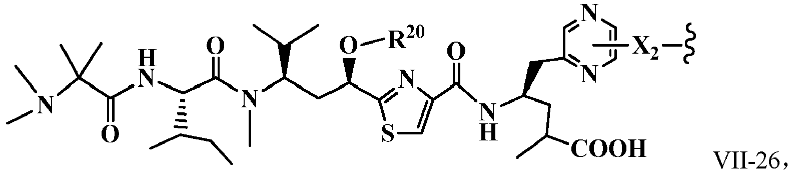 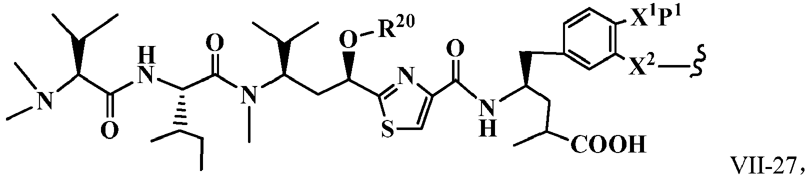 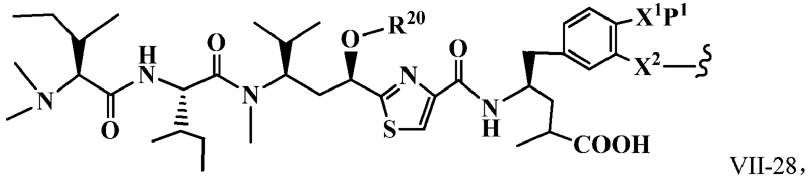 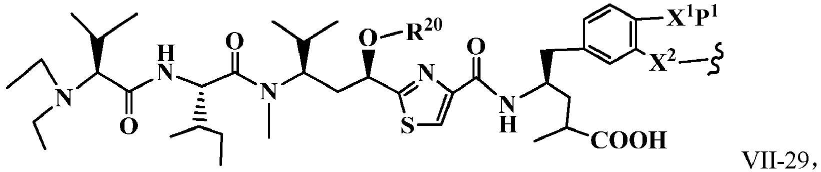 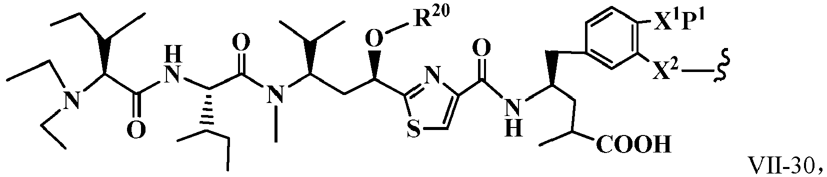 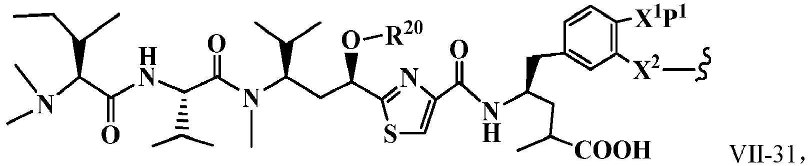 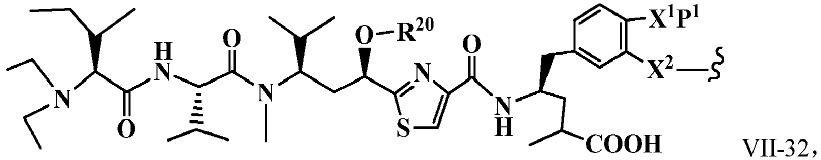 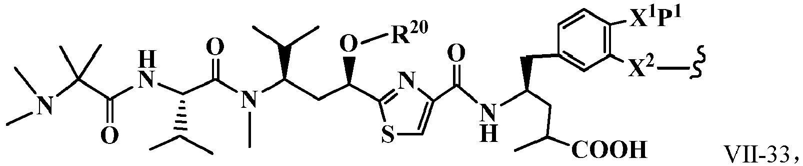 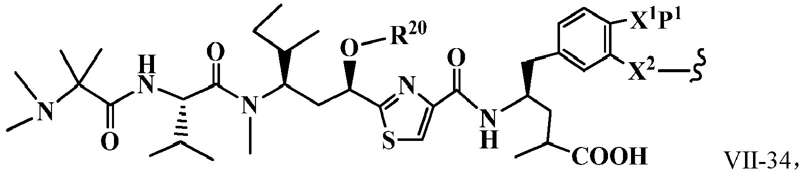 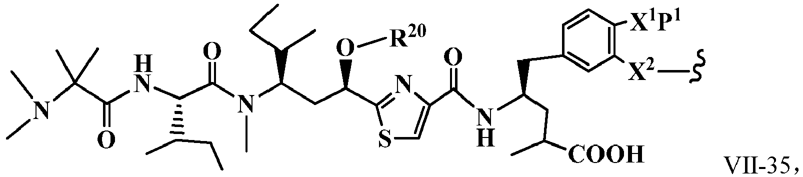 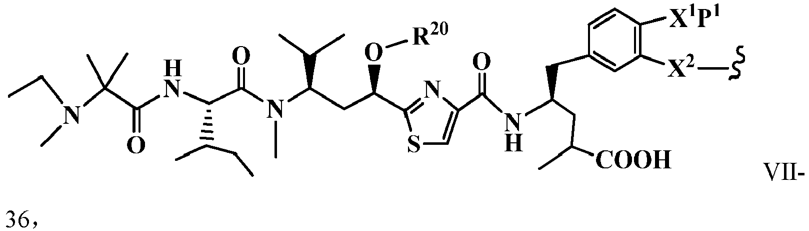 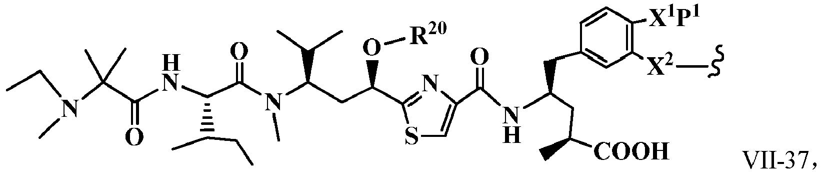 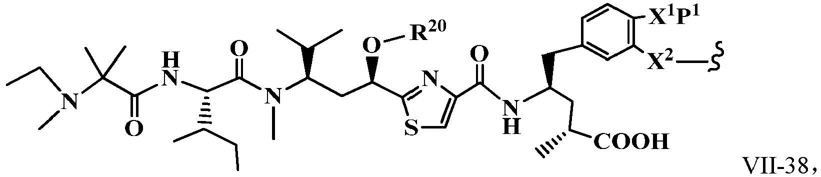 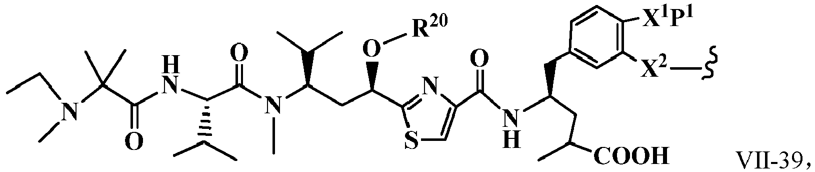 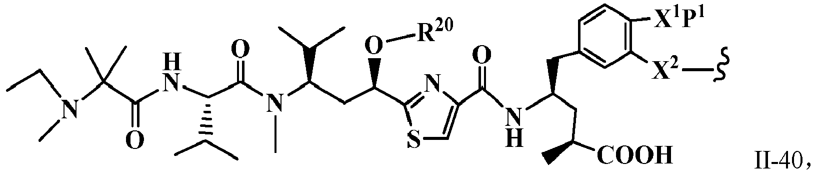 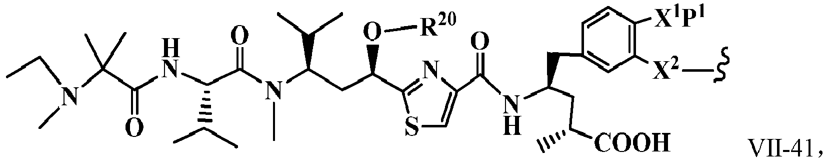 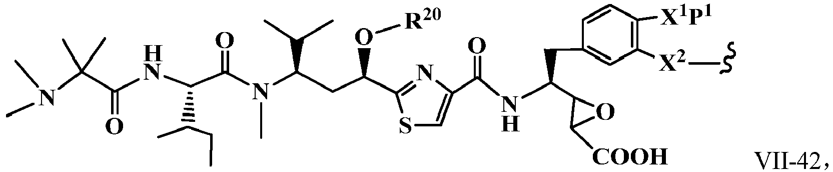 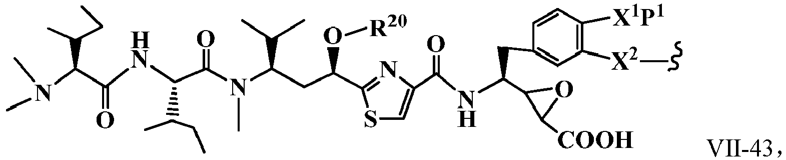 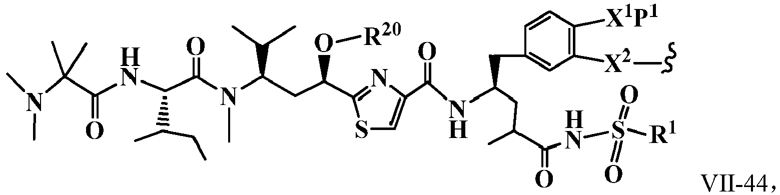 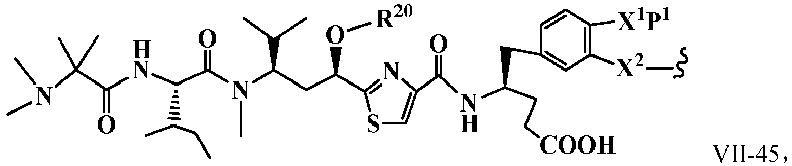 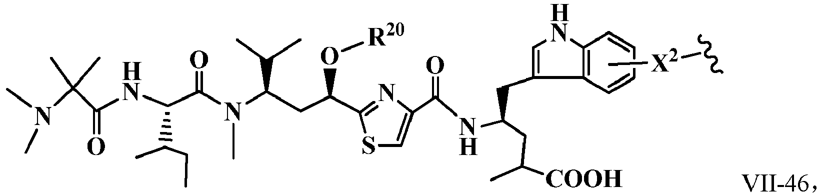 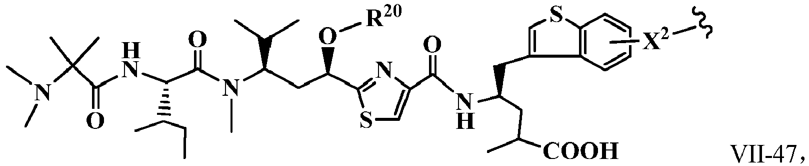 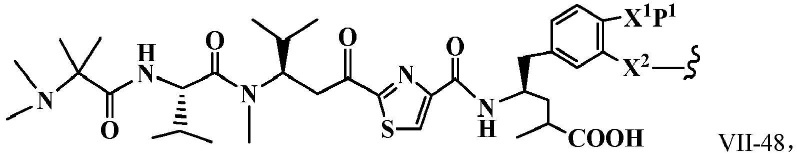 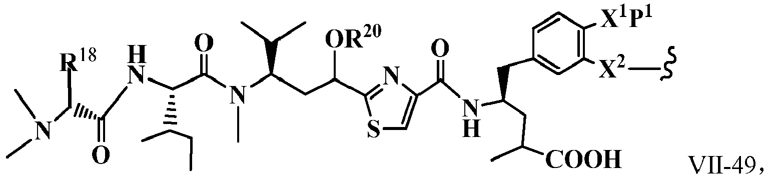 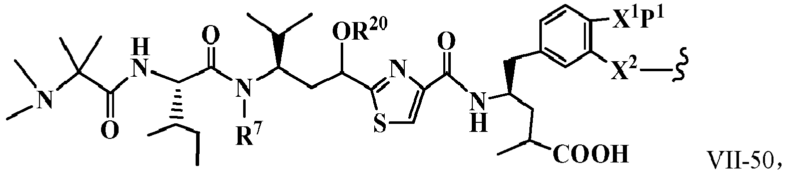 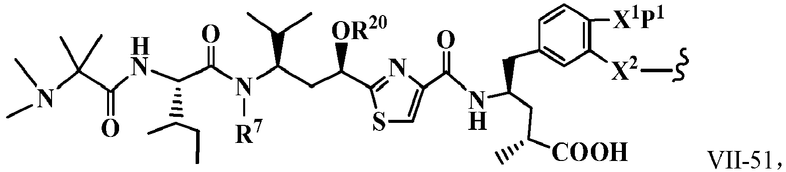 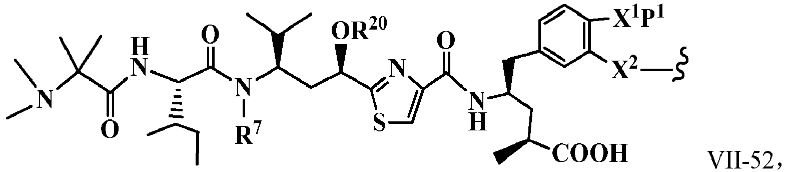 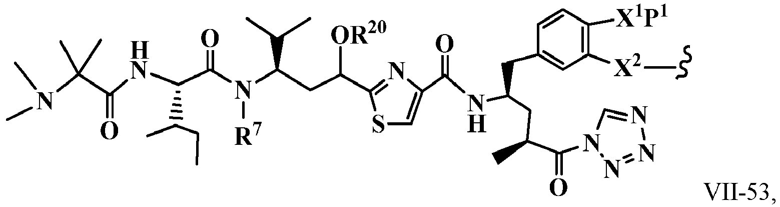 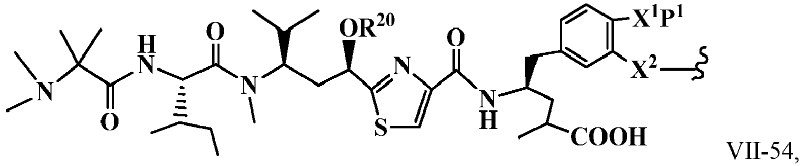 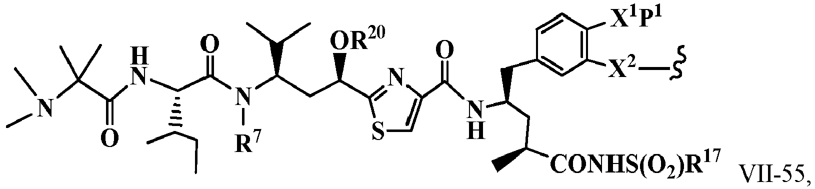 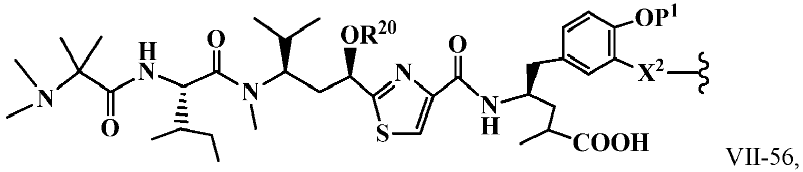 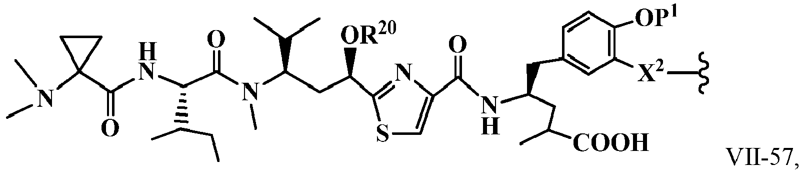 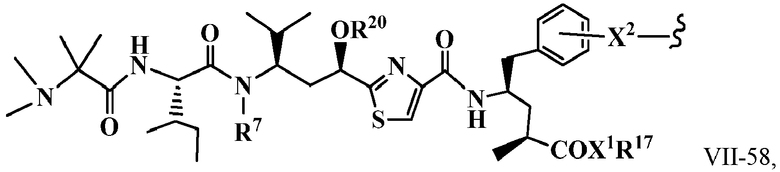 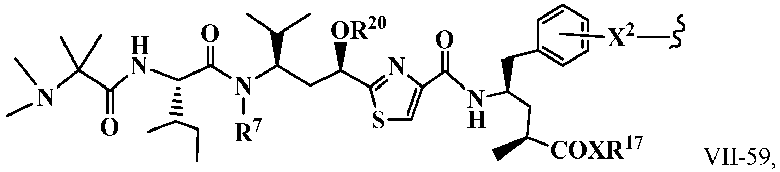 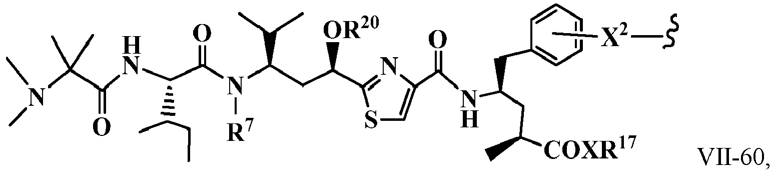 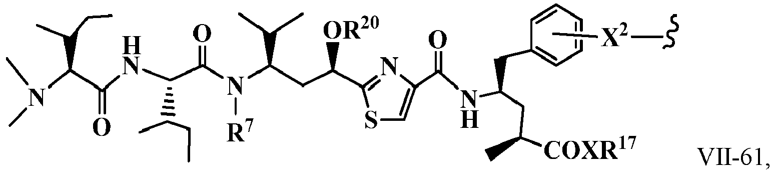 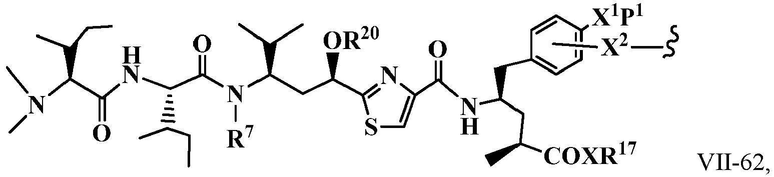 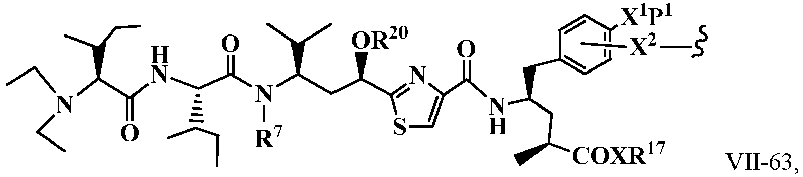 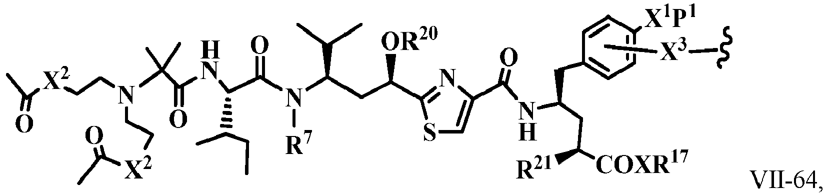 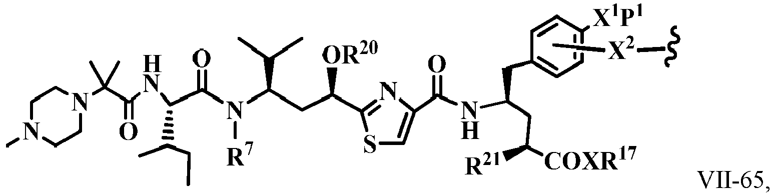 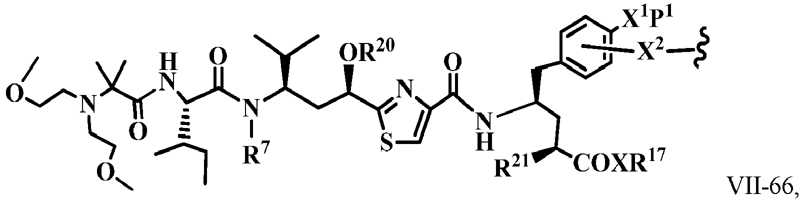 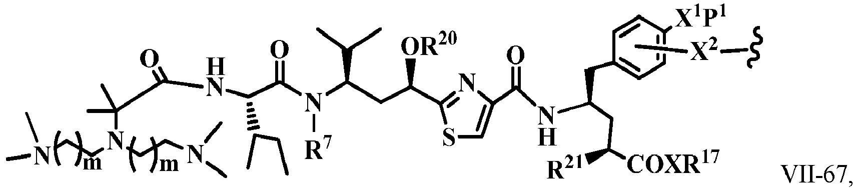 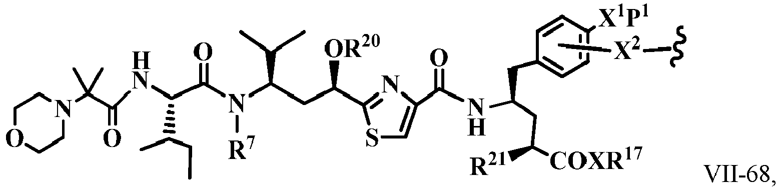 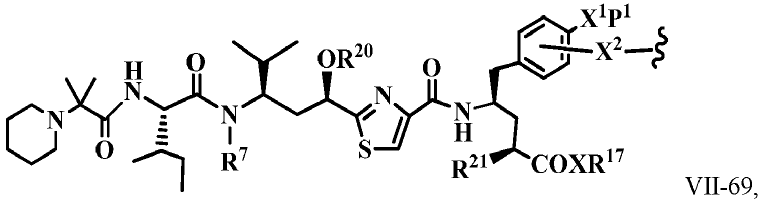 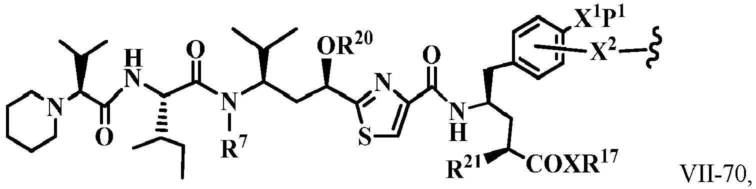 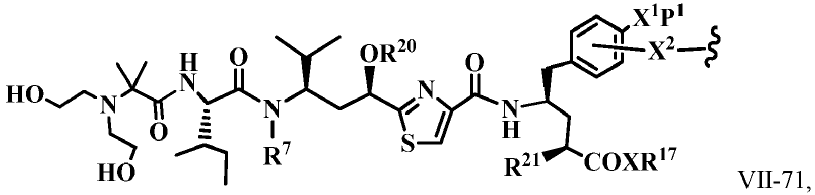 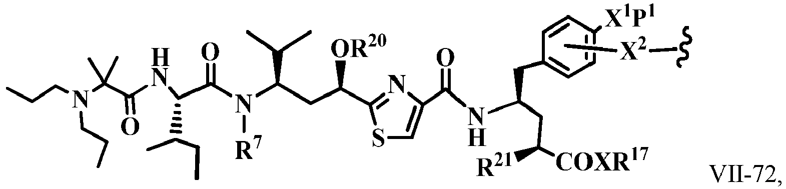 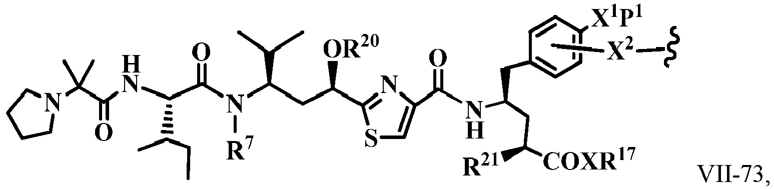 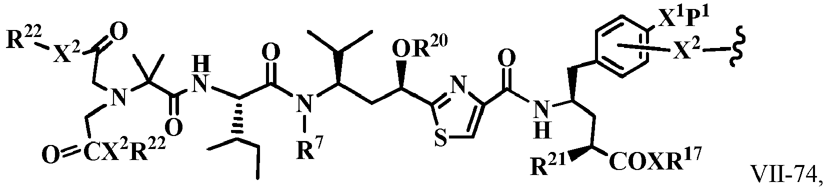 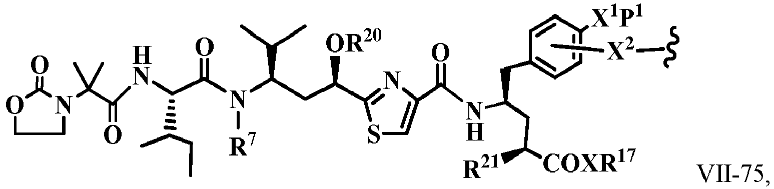 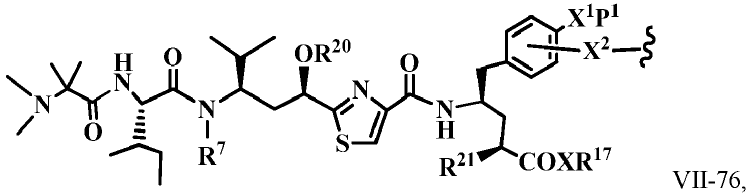 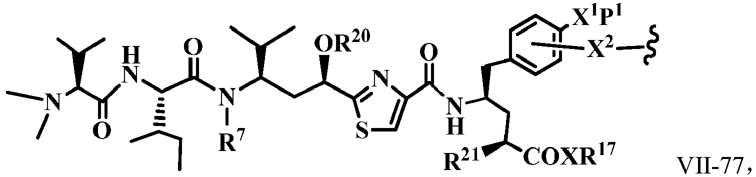
or a pharmaceutically acceptable salt, hydrate, or hydrated salt thereof; or a polymorphic crystalline structure thereof; or an optical isomer, racemate, diastereomer or enantiomer thereof;
wherein R₂₀ is H; C₁-C₈ of linear or branched alkyl, heteroalkyl, or acyl (-C(O)R¹⁷); C₂-C₈ of linear or branched alkenyl, alkynyl, alkylcycloalkyl, heterocycloalkyl; C₃-C₈ of linear or branched aryl, Ar-alkyl, heterocyclic, carbocyclic, cycloalkyl, heteroalkylcycloalkyl, alkylcarbonyl, heteroaryl; carbonate (-C(O)OR¹⁷), carbamate (-C(O)NR¹⁷R¹⁸); or C₁-C₈ of carboxylate, esters, ether, or amide; or 1∼8 amino acids; or polyethyleneoxy unit of formula (OCH₂CH₂)ₚ or (OCH₂CH(CH₃))ₚ, wherein p is an integer from 0 to about 1000; or R₂₀ is absent and the oxygen forms a ketone, or combination above thereof; wherein R²¹ is H, C₁-C₈ of linear or branched alkyl; X, X¹, X², and X³, are independently O, S, NH, NHNH, NHR¹⁷, CH₂ or absent; P¹ is H, R¹⁷, P(O)(OH)₂, P(O)(X¹R¹⁷)₂, CH₂P(O)(OH)₂, S(O²)(X¹R¹⁷), C₆H₁₂O₅ (glycoside), (CH₂CH₂O)ₚR¹⁷, wherein p is selected from 0-100,and R¹⁷ is defined above; in addition X¹P¹ can be absent (together is H);
wherein Z² and Z³ are independently H, OH, NH₂, OR¹⁷, NHR¹⁷, COOH, COOR¹⁷, C(O)R¹⁷, C(O)NHR¹⁷, C(O)NHNHR¹⁷, C(O)NH₂, R¹⁸, OCH₂OP(O)(OR¹⁸)₂, OC(O)OP(O)(OR¹⁸)₂, OPO(OR¹⁸)₂, NHPO(OR¹⁸)₂, OP(O)(OR¹⁸)OP(O)(OR¹⁸)₂, OC(O)R¹⁸, OC(O)NHR¹⁸, OSO₂(OR¹⁸), O-(C₄-C₁₂-glycoside), C₁-C₈ of linear or branched alkyl or heteroalkyl; C₂-C₈ of linear or branched alkenyl, alkynyl, alkylcycloalkyl, heterocycloalkyl; C₃-C₈ linear or branched of aryl, Ar-alkyl, heterocyclic, carbocyclic, cycloalkyl, heteroalkylcycloalkyl, alkylcarbonyl, heteroaryl; carbonate (-C(O)OR¹⁷), carbamate (-C(O)NR¹⁷R¹⁸); R¹⁷ and R¹⁸ are independently H, C₁-C₈ linear or branched alkyl or heteroalkyl; C₂-C₈ of linear or branched alkenyl, alkynyl, alkylcycloalkyl, heterocycloalkyl; C₃-C₈ linear or branched of aryl, Ar-alkyl, heterocyclic, carbocyclic, cycloalkyl, heteroalkylcycloalkyl, alkylcarbonyl, heteroaryl; carbonate (-C(O)OR¹⁷), carbamate (-C(O)NR¹⁷R¹⁸); R¹⁹ is H, OH, NH₂, OSO₂(OR¹⁸), XCH₂OP(O)(OR¹⁸)₂, XPO(OR¹⁸)₂, XC(O)OP(O)(OR¹⁸)₂, XC(O)R¹⁸, XC(O)NHR¹⁸, C₁∼C₈ alkyl or carboxylate; C₂∼C₈ alkenyl, alkynyl, alkylcycloalkyl, heterocycloalkyl; C₃∼C₈ aryl or alkylcarbonyl; or pharmaceutical salts; X is O, S, NH, NHNH, NHR¹⁷, or CH₂; R⁷ is defined the same above;
wherein " 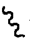 " is the site that linked to a linker L.

9. The conjugate according to claim 1 having one of the following structures: 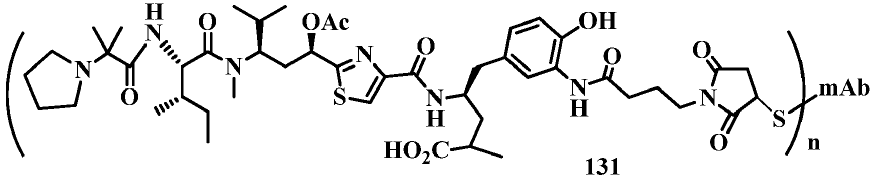 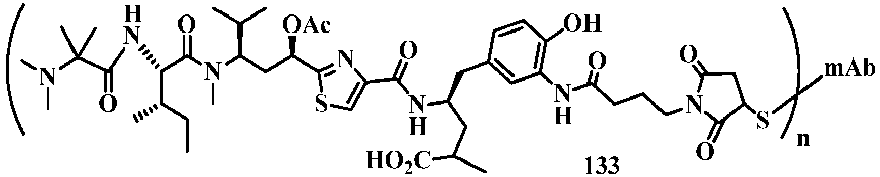 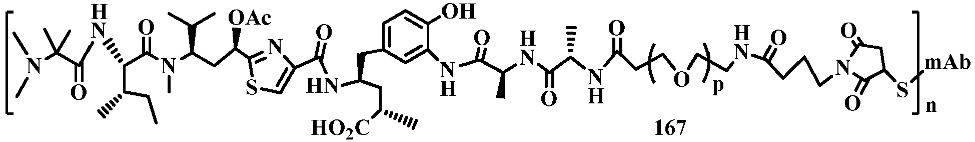 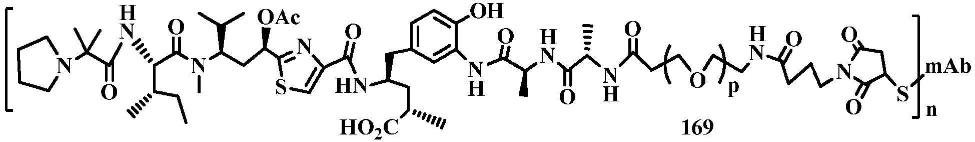 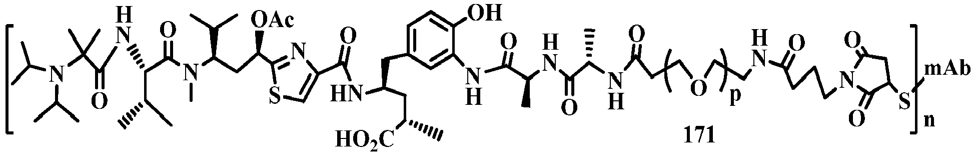 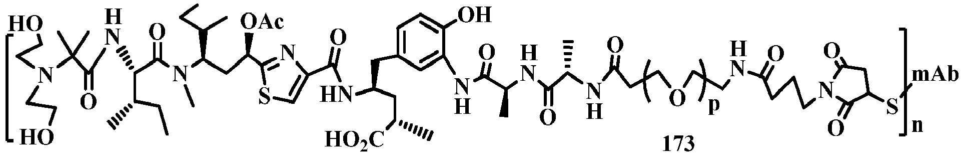 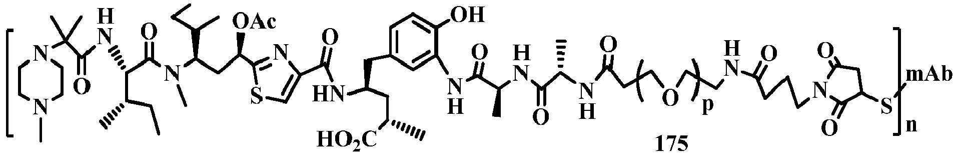 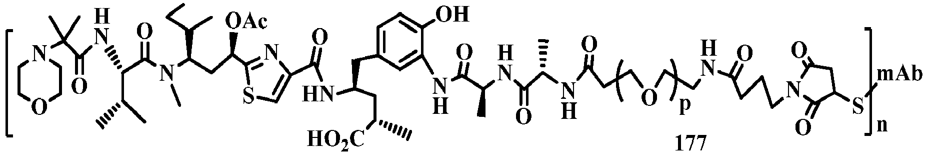 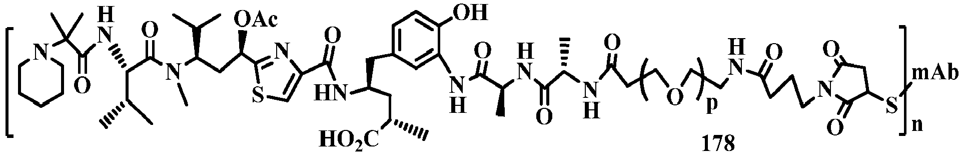 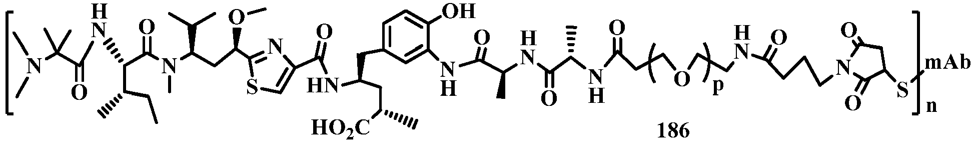 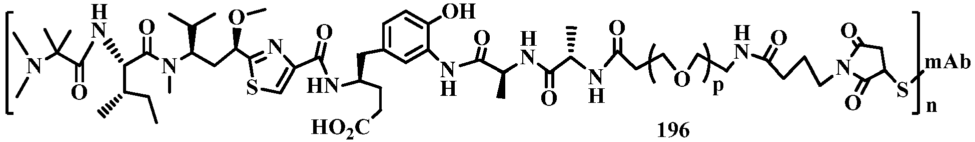 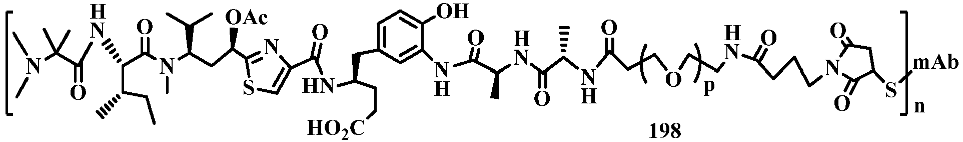 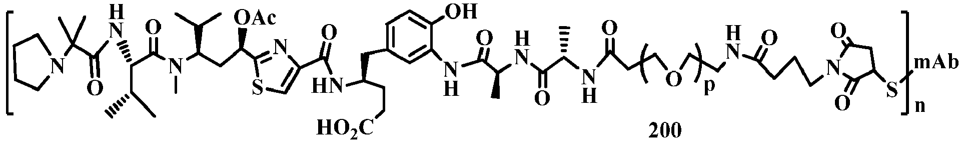 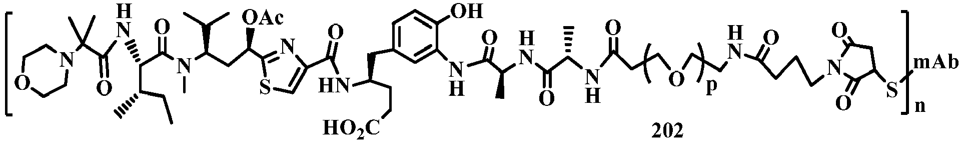 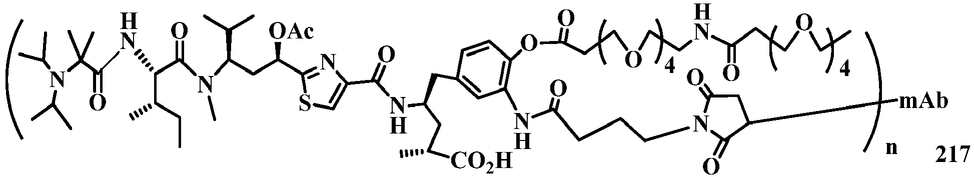 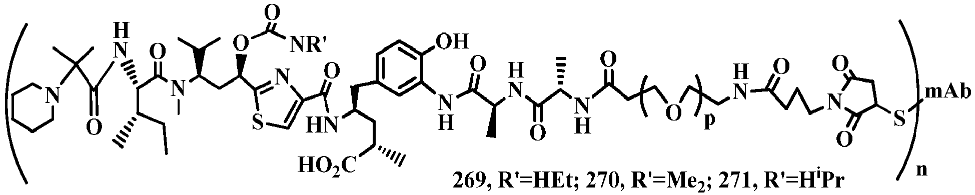 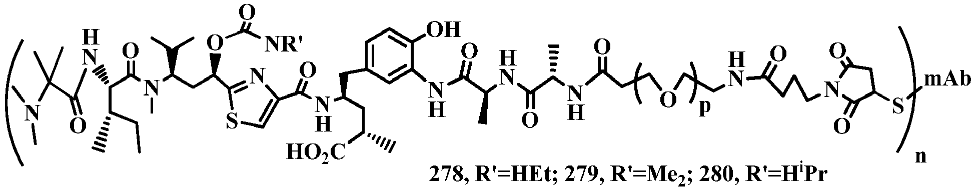 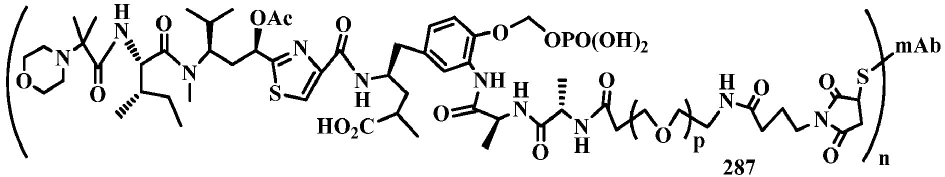 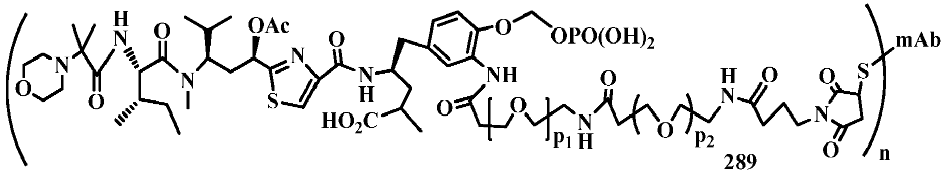 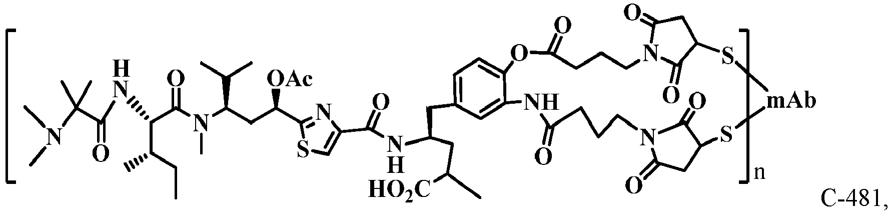 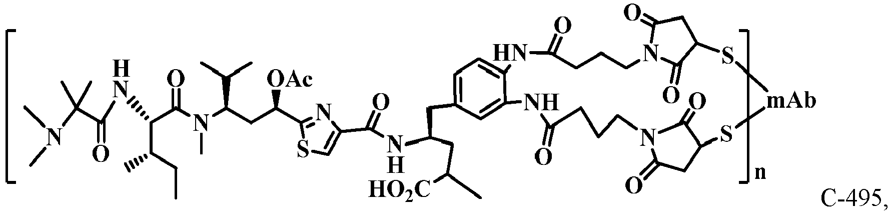 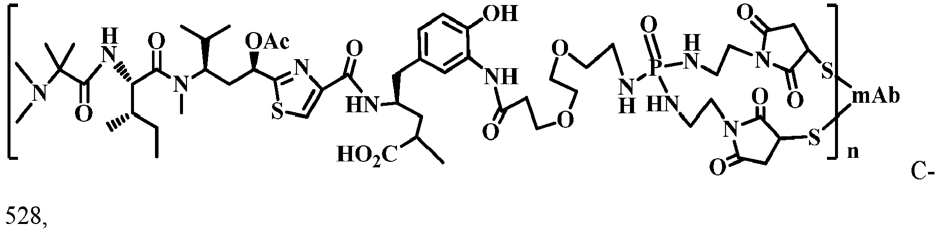 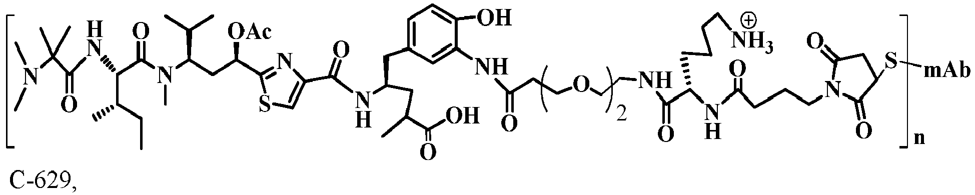 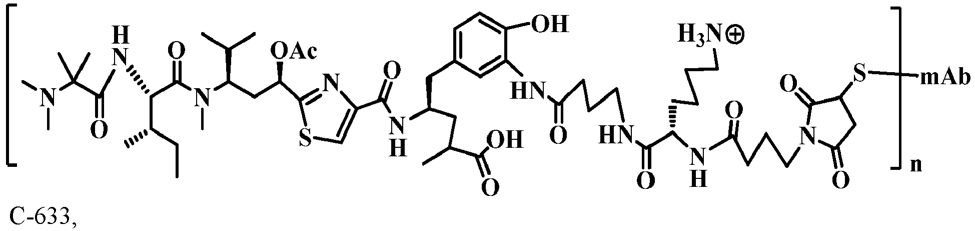 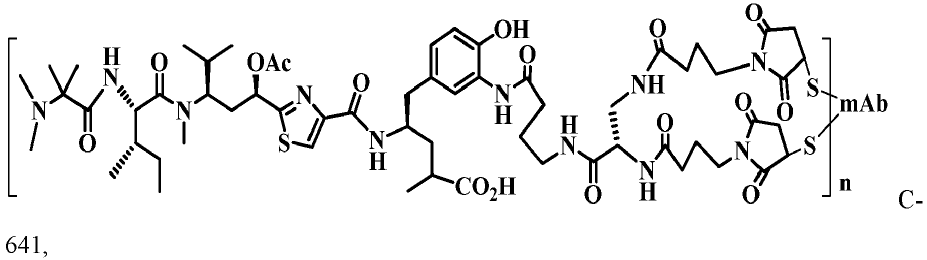 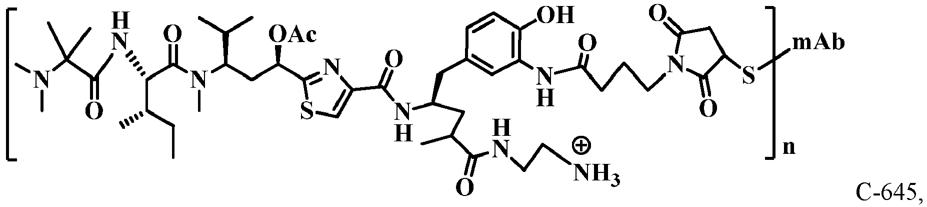 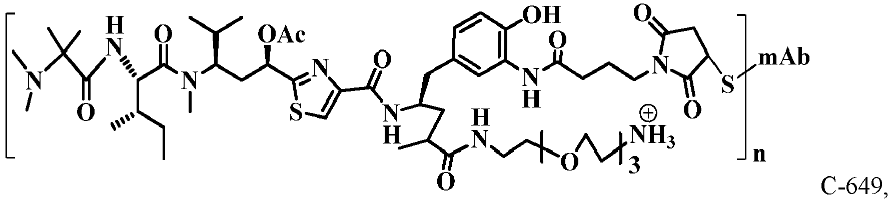 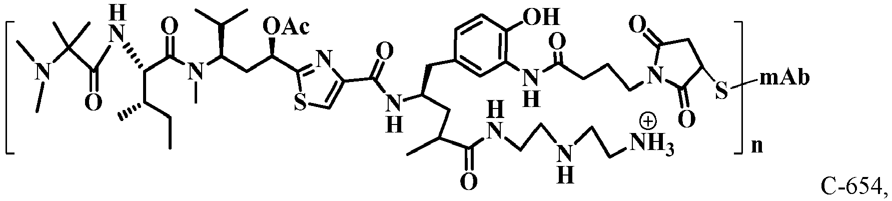 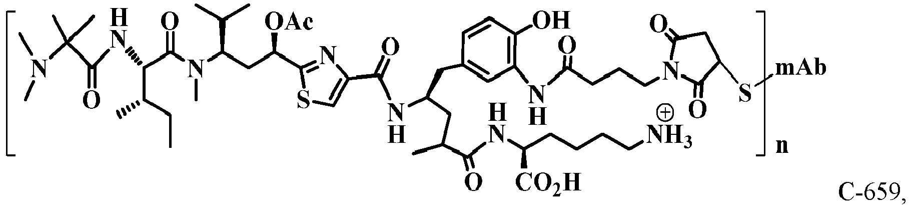 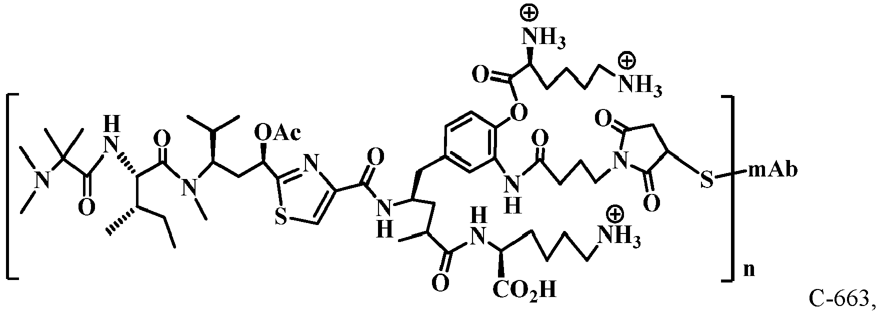 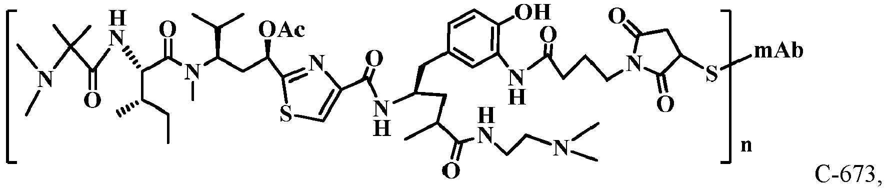 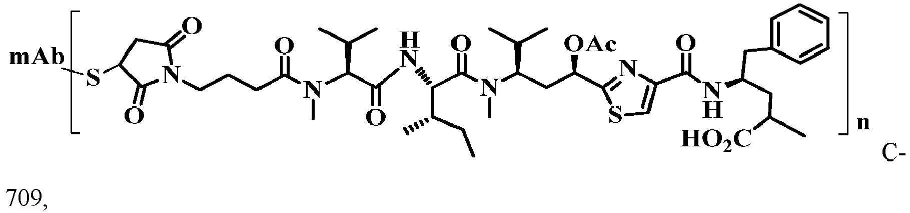 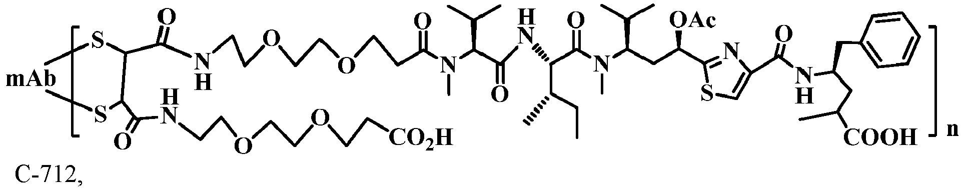 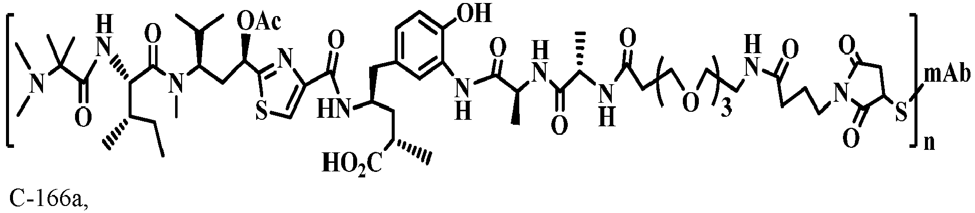 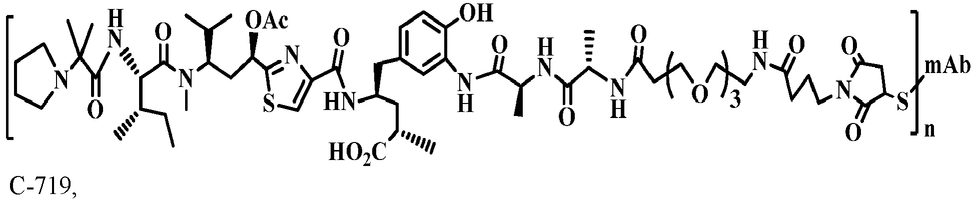 wherein mAb is an antibody; n is 1 ∼20; and p is 0∼100.

10. A method for preparing the conjugate according to claim 9, comprising reacting the antibody with a compound having one of the following strutures:

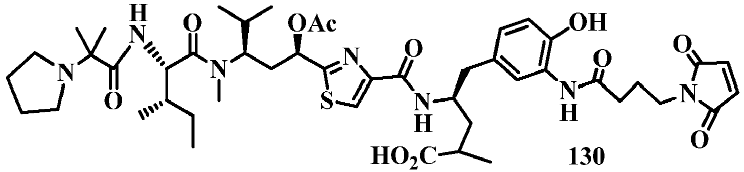

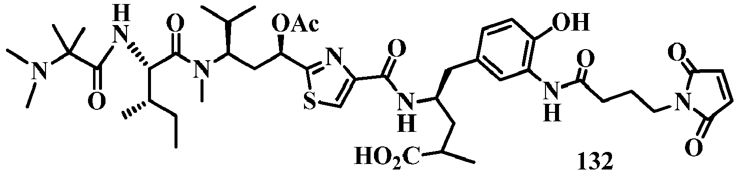

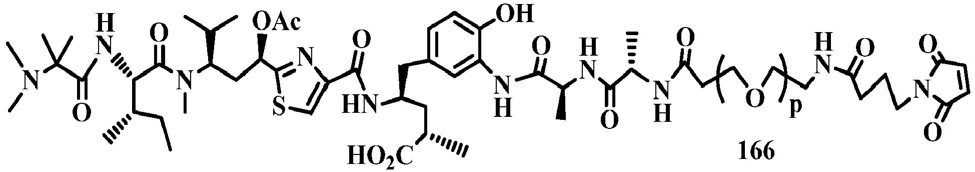

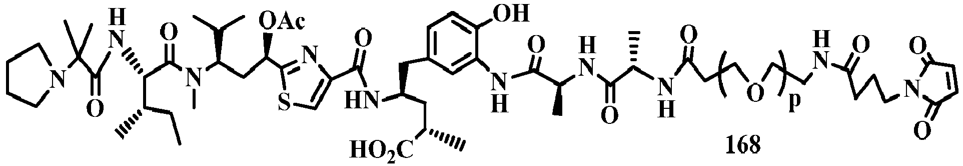

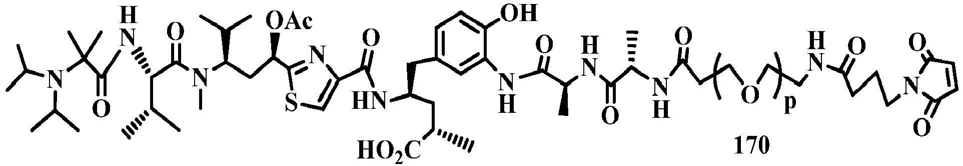

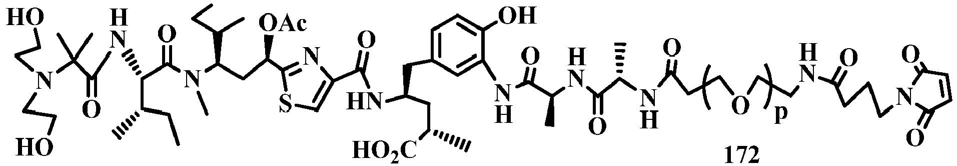

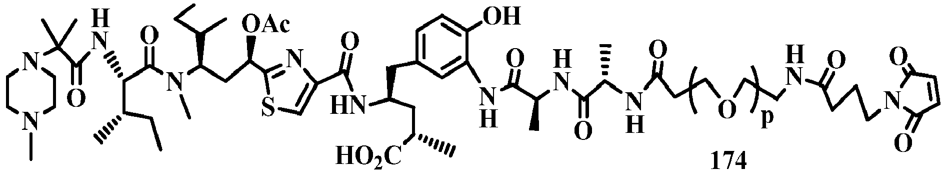

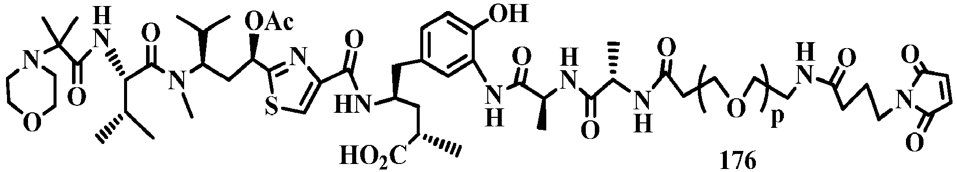

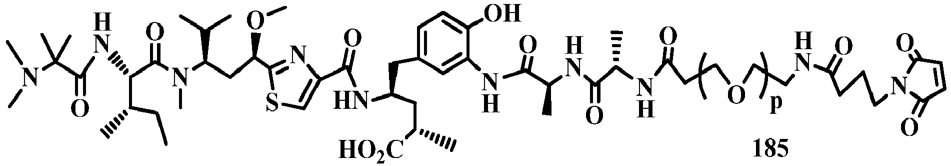

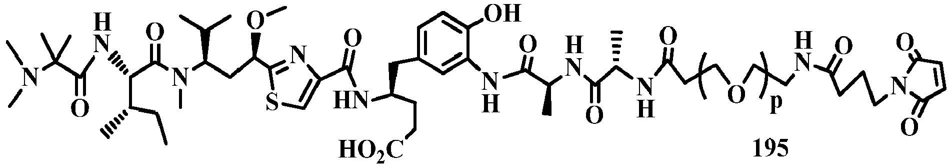

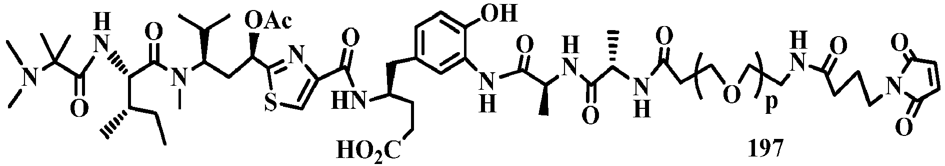

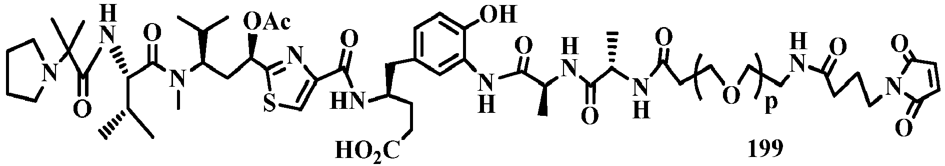

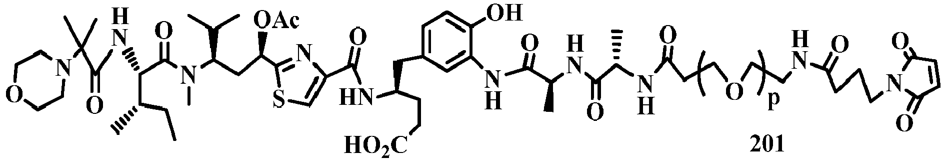

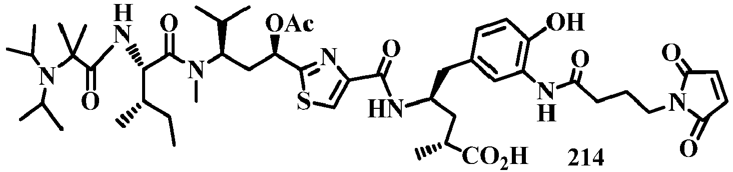

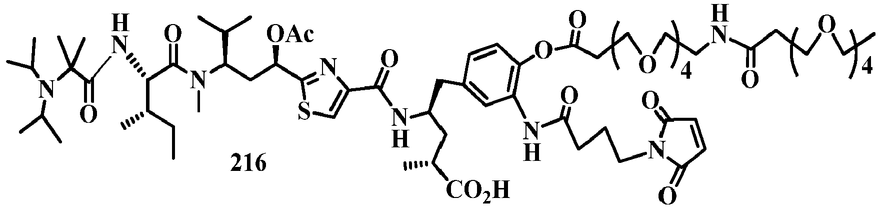

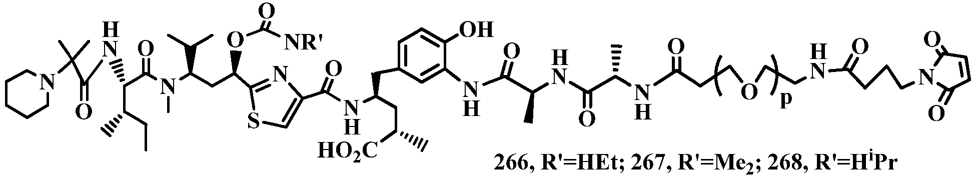

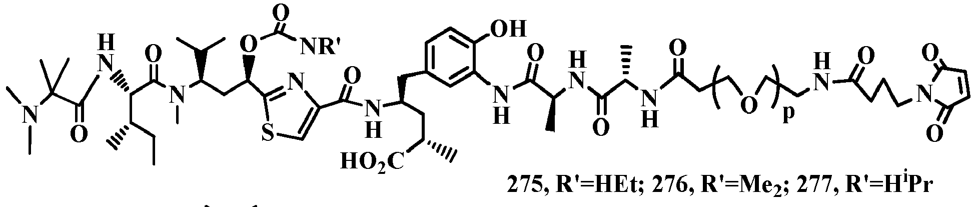

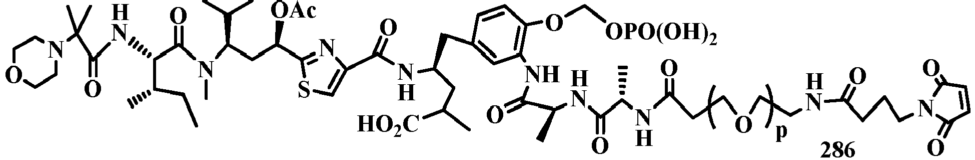

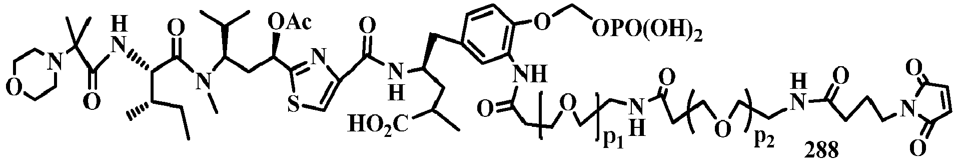

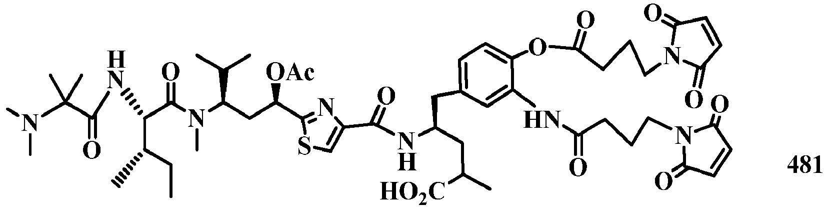

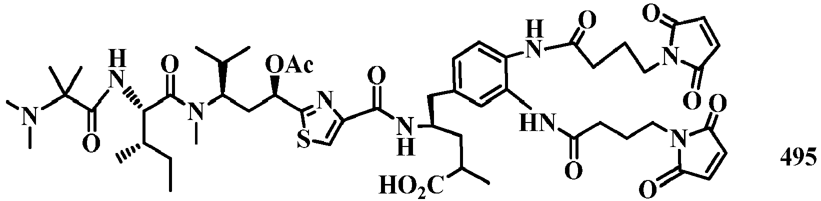

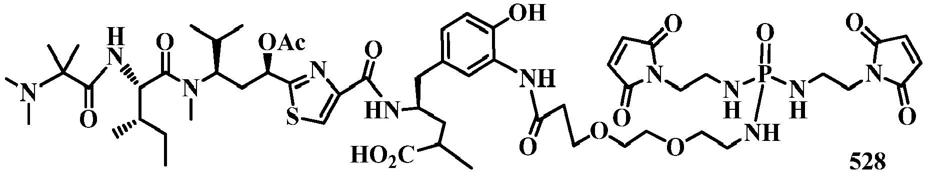

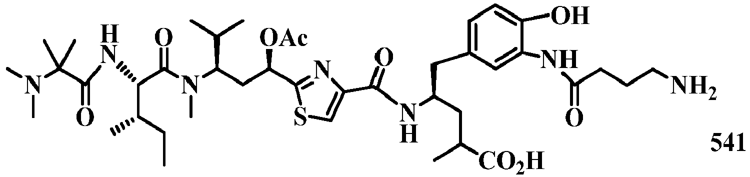

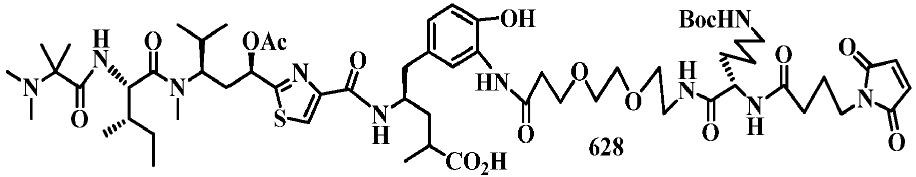

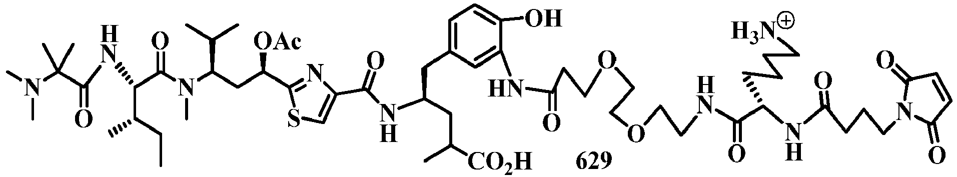

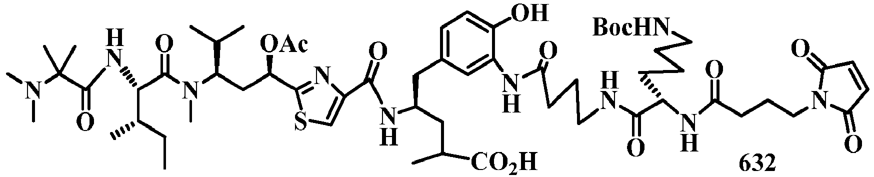

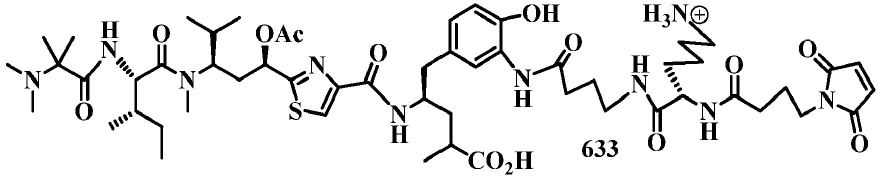

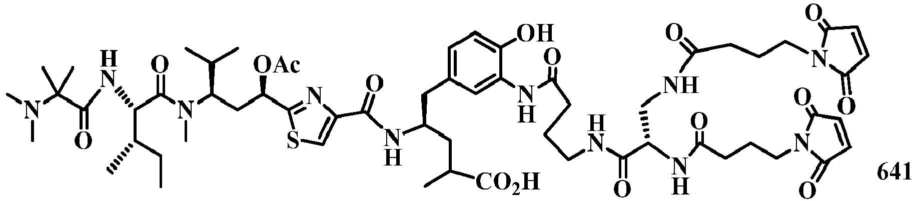

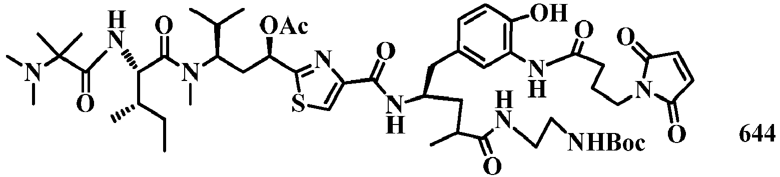

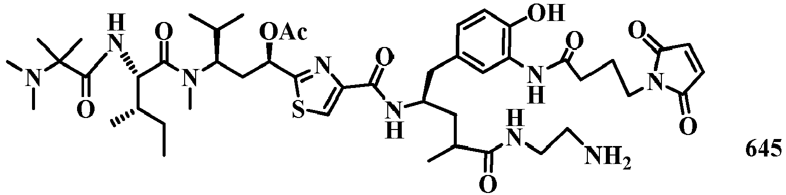

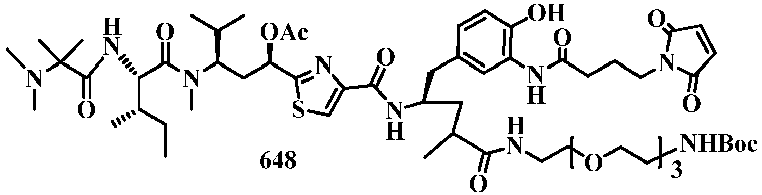

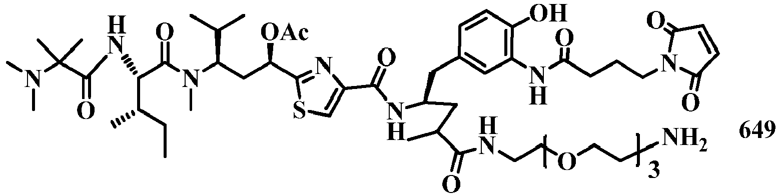

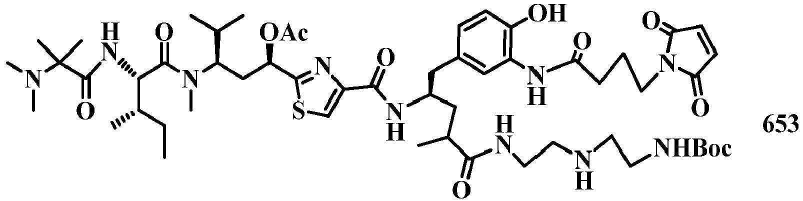

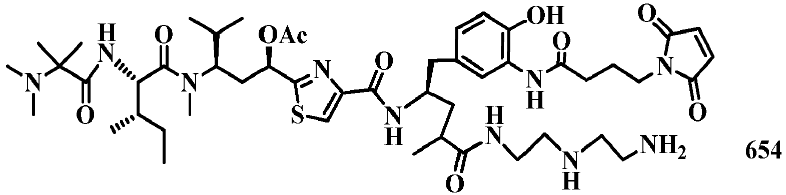

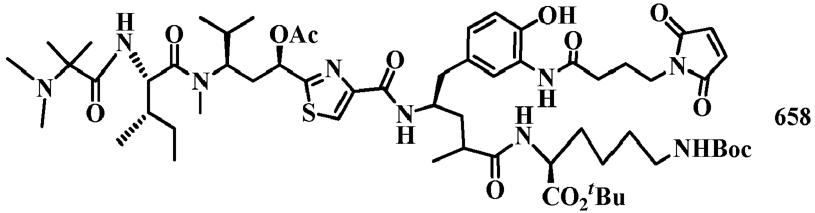

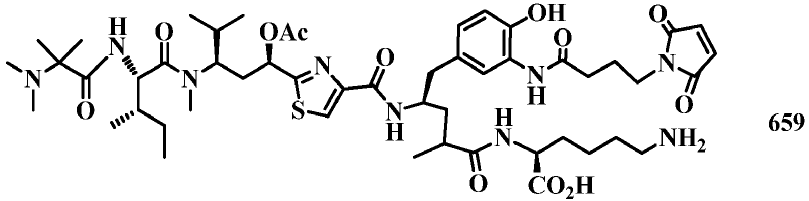

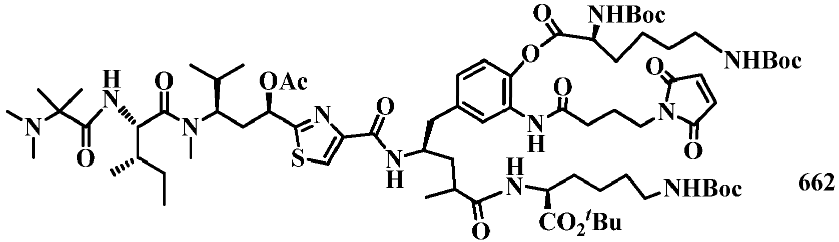

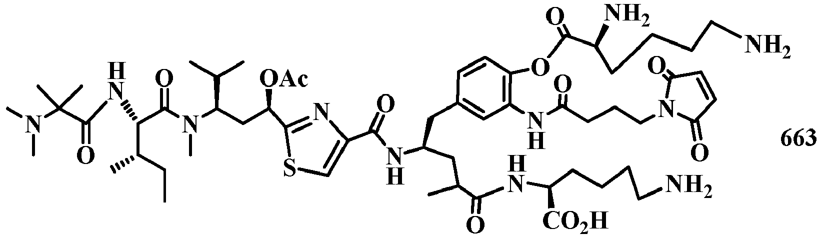

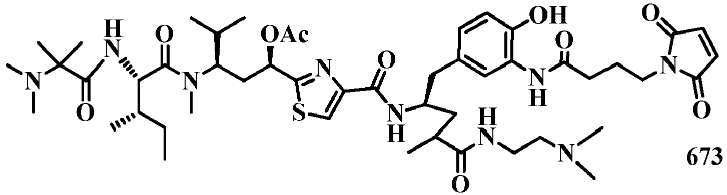

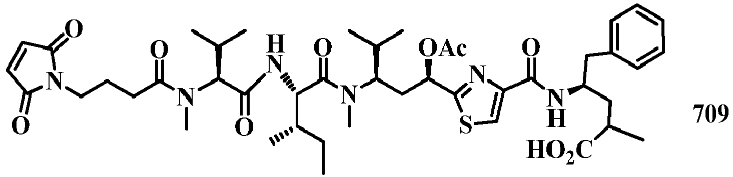

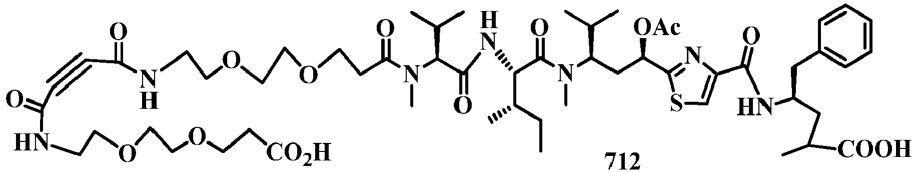

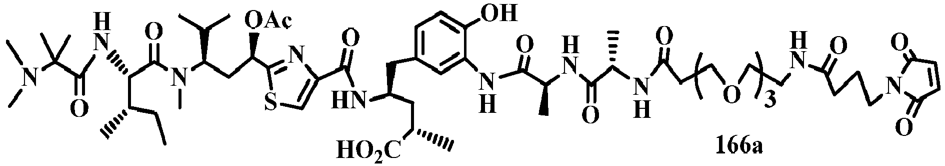

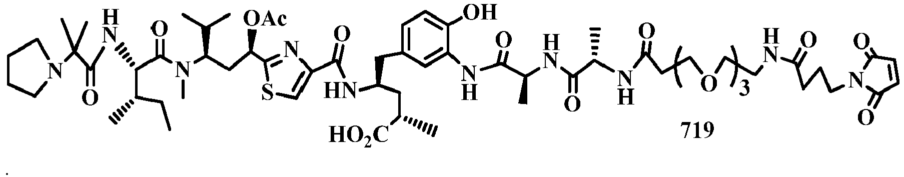

11. The conjugate according to claim 1, wherein the cell binding agent is capable of targeting against a tumor cell, a virus infected cell, a microorganism infected cell, a parasite infected cell, an autoimmune disease cell, an activated tumor cells, a myeloid cell, an activated T-cell, an affecting B cell, or a melanocyte, or any cells expressing any one of the following antigens or receptors: CD2, CD2R, CD3, CD3gd, CD3e, CD4, CD5, CD6, CD7, CD8, CD8a, CD8b, CD9, CD10, CD11a, CD11b, CD11c, CD12, CD12w, CD13, CD14, CD15, CD15s, CD15u, CD16, CD16a, CD16b, CD17, CDw17, CD18, CD19, CD20, CD21, CD22, CD23, CD24, CD25, CD26, CD27, CD28, CD29, CD30, CD31, CD32, CD33, CD34, CD35, CD36, CD37, CD38, CD39, CD40, CD41, CD42, CD42a, CD42b, CD42c, CD42d, CD43, CD44, CD44R, CD45, CD45RA, CD45RB, CD45RO, CD46, CD47, CD47R, CD48, CD49a, CD49b, CD49c, CD49e, CD49f, CD50, CD51, CD52, CD53, CD54, CD55,CD56, CD57, CD58, CD59, CD60, CD60a, CD60b, CD60c, CD61, CD62E, CD62L, CD62P, CD63, CD64, CD65, CD65s, CD66, CD66a, CD66b, CD66c, CD66d, CD66e, CD66f, CD67, CD68, CD69, CD70, CD71, CD72, CD73, CD74, CD74, CD75, CD75s, CD76, CD77, CD78, CD79, CD79a, CD79b, CD80, CD81, CD82, CD83, CD84, CDw84, CD85, CD86, CD87, CD88, CD89, CD90, CD91, CD92, CDw92, CD93, CD94, CD95, CD96, CD97, CD98, CD99, CD99R, CD100, CD101, CD102, CD103, CD104, CD105, CD106, CD107, CD107a, CD107b, CD108, CD109, CD110, CD111, CD112, CD113, CDw113, CD114, CD115, CD116, CD117, CD118, CD119, CDw119, CD120a, CD120b, CD121a, CD121b, CDw121b, CD122, CD123, CDw123, CD124, CD125, CDw125, CD126, CD127, CD128, CDw128, CD129, CD130, CD131, CDw131, CD132, CD133, CD134, CD135, CD136, CDw136, CD137, CDw137, CD138, CD139, CD140a, CD140b, CD141, CD142, CD143, CD144, CD145, CDw145, CD146, CD147, CD148, CD149, CD150, CD151, CD152, CD153, CD154, CD155, CD156a, CD156b, CDw156c, CD157, CD158a, CD158b, CD159a, CD159b, CD159c, CD160, CD161, CD162, CD162R, CD163, CD164, CD165, CD166, CD167, CD167a, CD168, CD169, CD170, CD171, CD172a, CD172b, CD172g, CD173, CD174, CD175, CD175s, CD176, CD177, CD178, CD179, CD180, CD181, CD182, CD183, CD184, CD185, CD186, CDw186, CD187, CD188, CD189, CD190, Cd191, CD192, CD193, CD194, CD195, CD196, CD197, CD198, CDw198, CD199, CDw199, CD200, CD200a, CD200b, CD201, CD202, CD202b, CD203, CD203c, CD204, CD205, CD206, CD207, CD208, CD209, CD210, CDw210, CD212, CD213a1, CD213a2, CDw217, CDw218a, CDw218b, CD220, CD221, CD222, CD223, CD224, CD225, CD226, CD227, CD228, CD229, CD230, CD231, CD232, CD233, CD234, CD235a, CD235ab, CD235b, CD236, CD236R, CD238, CD239, CD240, CD240CE, CD240D, CD241, CD242, CD243, CD244, CD245, CD246, CD247, CD248, CD249, CD252, CD253, CD254, CD256, CD257, CD258, CD261, CD262, CD263, CD265, CD266, CD267, CD268, CD269, CD271, CD273, CD274, CD275, CD276 (B7-H3), CD277, CD278, CD279, CD280, CD281, CD282, CD283, CD284, CD289, CD292, CDw293, CD294, CD295, CD296, CD297, CD298, CD299, CD300a, CD300c, CD300e, CD301, CD302, CD303, CD304, CD305, CD306, CD309, CD312, CD314, CD315, CD316, CD317, CD318, CD319, CD320, CD321, CD322, CD324, CDw325, CD326, CDw327, CDw328, CDw329, CD331, CD332, CD333, CD334, CD335, CD336, CD337, CDw338, CD339, CD340, CD341, CD342, CD343, CD344, CD345, CD346, CD347, CD348, CD349, CD350, CD351, CD352, CD353, CD354, CD355, CD356, CD357, CD358, CD359, CD360, CD361, CD362, CD363, CD364, CD365, CD366, CD367, CD368, CD369, CD370, CD371, CD372, CD373, CD374, CD375, CD376, CD377, CD378, CD379, CD381, CD382, CD383, CD384, CD385, CD386, CD387, CD388, CD389, CRIPTO, CRIPTO, CR, CR1, CRGF, CRIPTO, CXCR5, LY64, TDGF1, 4-1BB, APO2, ASLG659, BMPR1B, 4-1BB, 5AC, 5T4 (Trophoblastic glycoprotein, TPBG, 5T4, Wnt-Activated Inhibitory Factor 1 or WAIF1), Adenocarcinoma antigen, AGS-5, AGS-22M6, Activin receptor-like kinase 1, AFP, AKAP-4, ALK, Alpha integrin, Alpha v beta6, Amino-peptidase N, Amyloid beta, Androgen receptor, Angiopoietin 2, Angiopoietin 3, Annexin A1, Anthrax toxin protective antigen, Anti-transferrin receptor, AOC3 (VAP-1), B7-H3, Bacillus anthracis anthrax, BAFF (B-cell activating factor), BCMA, B-lymphoma cell, bcr-abl, Bombesin, BORIS, C5, C242 antigen, CA125 (carbohydrate antigen 125, MUC16), CA-IX (or CAIX, carbonic anhydrase 9), CALLA, CanAg, Canis lupus familiaris IL31, Carbonic anhydrase IX, Cardiac myosin, CCL11(C-C motif chemokine 11), CCR4 (C-C chemokine receptor type 4), CCR5, CD3E (epsilon), CEA (Carcinoembryonic antigen), CEACAM3, CEACAM5 (carcino-embryonic antigen), CFD (Factor D), Ch4D5, Cholecystokinin 2 (CCK2R), CLDN18 (Claudin-18), CLDN18.1 (Claudin-18.1), CLDN18.2 (Claudin-18.2), Clumping factor A, cMet, CRIPTO, FCSF1R (Colony stimulating factor 1 receptor), CSF2 (colony stimulating factor 2, Granulocyte-macrophage colony-stimulating factor (GM-CSF)), CSP4, CTLA4 (cytotoxic T-lymphocyte-associated protein 4), CTAA16.88 tumor antigen, CXCR4, C-X-C chemokine receptor type 4, cyclic ADP ribose hydrolase, Cyclin B1, CYP1B1, Cytomegalovirus, Cytomegalovirus glycoprotein B, Dabigatran, DLL3 (delta-like-ligand 3), DLL4 (delta-like-ligand 4), DPP4 (Dipeptidyl-peptidase 4), DR5 (Death receptor 5), E. coli shiga toxin type-1, E. coli shiga toxin type-2, ED-B, EGFL7 (EGF-like domain-containing protein 7), EGFR, EGFRII, EGFRvIII, Endoglin, Endothelin B receptor, Endotoxin, EpCAM (epithelial cell adhesion molecule), EphA2, Episialin, ERBB2 (Epidermal Growth Factor Receptor 2), ERBB3, ERG (TMPRSS2 ETS fusion gene), Escherichia coli, ETV6-AML, FAP (Fibroblast activation protein alpha), fibroblast surface antigen, FCGR1, alpha-Fetoprotein, Fibrin II, beta chain, Fibronectin extra domain-B, FOLR (folate receptor), Folate receptor alpha, Folate hydrolase, Fos-related antigen IF protein of respiratory syncytial virus, Frizzled receptor, Fucosyl GM1, GD2 ganglioside, G-28 (a cell surface antigen glyvolipid), GD3 idiotype, GloboH, Glypican 3, N-glycolylneuraminic acid, GM3, GMCSF receptor α-chain, Growth differentiation factor, GP100, GPNMB (Trans-membrane glycoprotein NMB), GUCY2C (Guanylate cyclase 2C, guanylyl cyclase C(GC-C), intestinal Guanylate cyclase, Guanylate cyclase-C receptor, Heat-stable enterotoxin receptor (hSTAR)), Heat shock proteins, Hemagglutinin, Hepatitis B surface antigen, Hepatitis B virus, HER1 (human epidermal growth factor receptor 1), HER2, HER2/neu, HER3 (ERBB-3), IgG4, HGF/SF (Hepatocyte growth factor/scatter factor), HHGFR, HIV-1, Histone complex, HLA-DR (human leukocyte antigen), HLA-DR10, HLA-DRB , HMWMAA, Human chorionic gonadotropin, HNGF, Human scatter factor receptor kinase, HPV E6/E7, Hsp90, hTERT, ICAM-1 (Intercellular Adhesion Molecule 1), Idiotype, IGF1R (IGF-1, insulin-like growth factor 1 receptor), IGHE, IFN-γ, Influenza hemagglutinin, IgE, IgE Fc region, IGHE, interleukins (comprising IL-1, IL-2, IL-3, IL-4, IL-5, IL-6, IL-6R, IL-7, IL-8, IL-9, IL-10, IL-11, IL-12, IL-13, IL-15, IL-17, IL-17A, IL-18, IL-19, IL-20, IL-21, IL-22, IL-23, IL-27, or IL-28), IL31RA, ILGF2 (Insulin-like growth factor 2), Integrins (α4, α_{IIb}β₃, αvβ3, α₄β₇, α5β1, α6β4, α7β7, αllβ3, α5β5, αvβ5), Interferon gamma-induced protein, ITGA2, ITGB2, KIR2D, Kappa Ig, LCK, Le, Legumain, Lewis-Y antigen, LFA-1 (Lymphocyte function-associated antigen 1, CD11a), LHRH, LINGO-1, Lipoteichoic acid, LIV1A, LMP2, LTA, MAD-CT-1, MAD-CT-2, MAGE-1, MAGE-2, MAGE-3, MAGE A1, MAGE A3, MAGE 4, MART1, MCP-1, MIF (Macrophage migration inhibitory factor, or glycosylation-inhibiting factor (GIF)), MS4A1 (membrane-spanning 4-domains subfamily A member 1), MSLN (mesothelin), MUC1(Mucin 1, cell surface associated (MUC1) or polymorphic epithelial mucin (PEM)), MUC1-KLH, MUC16 (CA125), MCP1(monocyte chemotactic protein 1), MelanA/MART1, ML-IAP, MPG, MS4A1 (membrane-spanning 4-domains subfamily A), MYCN, Myelin-associated glycoprotein, Myostatin, NA17, NARP-1, NCA-90 (granulocyte antigen), Nectin-4 (ASG-22ME), NGF, Neural apoptosis-regulated proteinase 1, NOGO-A, Notch receptor, Nucleolin, Neu oncogene product, NY-BR-1, NY-ESO-1, OX-40, OxLDL (Oxidized low-density lipoprotein), OY-TES1, P21, p53 nonmutant, P97, Page4, PAP, Paratope of anti-(N-glycolylneuraminic acid), PAX3, PAX5, PCSK9, PDCD1 (PD-1, Programmed cell death protein 1), PDGF-Rα (Alpha-type platelet-derived growth factor receptor), PDGFR-β, PDL-1, PLAC1, PLAP-like testicular alkaline phosphatase, Platelet-derived growth factor receptor beta, Phosphate-sodium co-transporter, PMEL 17, Polysialic acid, Proteinase3 (PR1), Prostatic carcinoma, PS (Phosphatidylserine), Prostatic carcinoma cells, Pseudomonas aeruginosa, PSMA, PSA, PSCA, Rabies virus glycoprotein, RHD (Rh polypeptide 1 (RhPI)), Rhesus factor, RANKL, RhoC, Ras mutant, RGS5, ROBO4, Respiratory syncytial virus, RON, ROR1, Sarcoma translocation breakpoints, SART3, Sclerostin, SLAMF7 (SLAM family member 7), Selectin P, SDC1 (Syndecan 1), sLe(a), Somatomedin C, SIP (Sphingosine-1-phosphate), Somatostatin, Sperm protein 17, SSX2, STEAP1 (six-transmembrane epithelial antigen of the prostate 1), STEAP2, STn, TAG-72 (tumor associated glycoprotein 72), Survivin, T-cell receptor, T cell transmembrane protein, TEM1 (Tumor endothelial marker 1), TENB2, Tenascin C (TN-C), TGF-α, TGF-β (Transforming growth factor beta), TGF-β1, TGF-β2 (Transforming growth factor-beta 2), Tie (CD202b), Tie2, TIM-1 (CDX-014), Tn, TNF, TNF-α, TNFRSF8, TNFRSF10B (tumor necrosis factor receptor superfamily member 10B), TNFRSF-13B (tumor necrosis factor receptor superfamily member 13B), TPBG (trophoblast glycoprotein), TRAIL-R1 (Tumor necrosis apoptosis Inducing ligand Receptor 1), TRAILR2 (Death receptor 5 (DR5)), tumor-associated calcium signal transducer 2, tumor specific glycosylation of MUC1, TWEAK receptor, TYRP1 (glycoprotein 75), TRP-1 (Trop1), TRP-2 (Trop2), Tyrosinase, VCAM-1, VEGF, VEGF-A, VEGF-2, VEGFR-1, VEGFR2, or vimentin, WT1, XAGE 1, or cells expressing any insulin growth factor receptors, or any epidermal growth factor receptors.

12. The conjugateaccording to claim **11,** wherein the tumor cell is selected from the group consisting of lymphoma cells, myeloma cells, renal cells, breast cancer cells, prostate cancer cells, ovarian cancer cells, colorectal cancer cells, gastric cancer cells, squamous cancer cells, small-cell lung cancer cells, none small-cell lung cancer cells, testicular cancer cells, malignant cells, and cells that grow and divide at an unregulated, quickened pace to cause cancers.

13. A pharmaceutical composition comprising a therapeutically effective amount of one or mores of the conjugate of claim **1,** and a pharmaceutically acceptable salt, carrier, diluent, or excipient therefor.

14. The pharmaceutical composition of Claim **13,** having in vitro, in vivo or ex vivo cell killing activity.

15. A method for synergistically treating a cancer, an autoimmune disease, or an infectious disease, comprising administering concurrently the pharmaceutical composition according to Claim **13,** with a synergistic agent selected from chemotherapeutic agent, a radiation therapy, an immunotherapy agent, an autoimmune disorder agent, or an anti-infectious agents .

16. The method according to Claim **15,** wherein the synergistic agent is one or more selected from the following agents:
(1). a chemotherapeutic agent consisting of:
a). an alkylating agent: selected from the group consisting of nitrogen mustards: chlorambucil, chlornaphazine, cyclophosphamide, dacarbazine, estramustine, ifosfamide, mechlorethamine, mechlorethamine oxide hydrochloride, mannomustine, mitobronitol, melphalan, mitolactol, pipobroman, novembichin, phenesterine, prednimustine, thiotepa, trofosfamide, uracil mustard; CC-1065 and adozelesin, carzelesin, bizelesin or their synthetic analogues; duocarmycin and its synthetic analogues, KW-2189, CBI-TMI, or CBI dimers; benzodiazepine dimers or pyrrolobenzodiazepine (PBD) dimers, tomaymycin dimers, indolinobenzodiazepine dimers, imidazobenzothiadiazepine dimers, or oxazolidinobenzodiazepine dimers; Nitrosoureas: comprising carmustine, lomustine, chlorozotocin, fotemustine, nimustine, ranimustine; Alkylsulphonates: comprising busulfan, treosulfan, improsulfan and piposulfan); Triazenes or dacarbazine; Platinum containing compounds: comprising carboplatin, cisplatin, and oxaliplatin; aziridines, benzodopa, carboquone, meturedopa, or uredopa; ethylenimines and methylamelamines including altretamine, triethylenemelamine, trietylenephosphoramide, triethylenethiophosphoramide and trimethylolomelamine;
b). a plant alkaloid: selected from the group consisting of Vinca alkaloids: comprising vincristine, vinblastine, vindesine, vinorelbine, and navelbin; Taxoids: comprising paclitaxel, docetaxol and their analogs, Maytansinoids comprising DM1, DM2, DM3, DM4, DM5, DM6, DM7, maytansine, ansamitocins and their analogs, cryptophycins (including the group consisting of cryptophycin 1 and cryptophycin 8); epothilones, eleutherobin, discodermolide, bryostatins, dolostatins, auristatins, tubulysins, cephalostatins; pancratistatin; a sarcodictyin; spongistatin;
c). a DNA topoisomerase inhibitor: selected from the groups of Epipodophyllins: comprising 9-aminocamptothecin, camptothecin, crisnatol, daunomycin, etoposide, etoposide phosphate, irinotecan, mitoxantrone, novantrone, retinoic acids (or retinols), teniposide, topotecan, 9-nitrocamptothecin or RFS 2000; and mitomycins and their analogs;
d). an antimetabolite: selected from the group consisting of {[Anti-folate: (DHFR inhibitors: comprising methotrexate, trimetrexate, denopterin, pteropterin, aminopterin (4-aminopteroic acid) or folic acid analogues); IMP dehydrogenase Inhibitors: (comprising mycophenolic acid, tiazofurin, ribavirin, EICAR); Ribonucleotide reductase Inhibitors: (comprising hydroxyurea, deferoxamine)]; [Pyrimidine analogs: Uracil analogs: (comprising ancitabine, azacitidine, 6-azauridine, capecitabine (Xeloda), carmofur, cytarabine, dideoxyuridine, doxifluridine, enocitabine, 5-Fluorouracil, floxuridine, ratitrexed (Tomudex)); Cytosine analogs: (comprising cytarabine, cytosine arabinoside, fludarabine); Purine analogs: (comprising azathioprine, fludarabine, mercaptopurine, thiamiprine, thioguanine)]; folic acid replenisher, frolinic acid};
e). a hormonal therapy: selected from the group consisting of {Receptor antagonists: [Anti-estrogen: (comprising megestrol, raloxifene, tamoxifen); LHRH agonists: (comprising goscrclin, leuprolide acetate); Anti-androgens: (comprising bicalutamide, flutamide, calusterone, dromostanolone propionate, epitiostanol, goserelin, leuprolide, mepitiostane, nilutamide, testolactone, trilostane and other androgens inhibitors)]; Retinoids/Deltoids: [Vitamin D3 analogs: (comprising CB 1093, EB 1089 KH 1060, cholecalciferol, ergocalciferol); Photodynamic therapies: (comprising verteporfin, phthalocyanine, photosensitizer Pc4, demethoxyhypocrellin A); Cytokines: (comprising Interferon-alpha, Interferon-gamma, tumor necrosis factor (TNFs), human proteins containing a TNF domain)]};
f). a kinase inhibitor, selected from the group consisting of BIBW 2992 (anti-EGFR/Erb2), imatinib, gefitinib, pegaptanib, sorafenib, dasatinib, sunitinib, erlotinib, nilotinib, lapatinib, axitinib, pazopanib. vandetanib, E7080 (anti-VEGFR2), mubritinib, ponatinib (AP24534), bafetinib (INNO-406), bosutinib (SKI-606), cabozantinib, vismodegib, iniparib, ruxolitinib, CYT387, axitinib, tivozanib, sorafenib, bevacizumab, cetuximab, Trastuzumab, Ranibizumab, Panitumumab, ispinesib;
g). a poly (ADP-ribose) polymerase (PARP) inhibitors selected from the group consisting of olaparib, niraparib, iniparib, talazoparib, veliparib, CEP 9722 (Cephalon's), E7016 (Eisai's), BGB-290 (BeiGene's), or 3-aminobenzamide.
h). an antibiotic, selected from the group consisting of an enediyne antibiotic (selected from the group consisting of calicheamicin, calicheamicin γ1, δ1, α1 or β1; dynemicin, including dynemicin A and deoxydynemicin; esperamicin, kedarcidin, C-1027, maduropeptin, or neocarzinostatin chromophore and related chromoprotein enediyne antibiotic chromomophores), aclacinomycins, actinomycin, authramycin, azaserine, bleomycins, cactinomycin, carabicin, carminomycin, carzinophilin; chromomycins, dactinomycin, daunorubicin, detorubicin, 6-diazo-5-oxo-L-norleucine, doxorubicin, morpholino-doxorubicin, cyanomorpholino-doxorubicin, 2-pyrrolino-doxorubicin and deoxydoxorubicin, epirubicin, eribulin, esorubicin, idarubicin, marcellomycin, nitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin;
i). a polyketide (acetogenin), bullatacin and bullatacinone; gemcitabine, epoxomicins andcarfilzomib, bortezomib, thalidomide, lenalidomide, pomalidomide, tosedostat, zybrestat, PLX4032, STA-9090, Stimuvax, allovectin-7, Xegeva, Provenge, Yervoy, Isoprenylation inhibitors and Lovastatin, Dopaminergic neurotoxins and1-methyl-4-phenylpyridinium ion, Cell cycle inhibitors (selected from staurosporine), Actinomycins (comprising Actinomycin D, dactinomycin), amanitins, Bleomycins (comprising bleomycin A2, bleomycin B2, peplomycin), Anthracyclines (comprising daunorubicin, doxorubicin (adriamycin), idarubicin, epirubicin, pirarubicin, zorubicin, mtoxantrone, MDR inhibitors or verapamil, Ca²⁺ ATPase inhibitors or thapsigargin, Histone deacetylase inhibitors ((comprising Vorinostat, Romidepsin, Panobinostat, Valproic acid, Mocetinostat (MGCD0103), Belinostat, PCI-24781, Entinostat, SB939, Resminostat, Givinostat, AR-42, CUDC-101, sulforaphane, Trichostatin A) ; Thapsigargin, Celecoxib, glitazones, epigallocatechin gallate, Disulfiram, Salinosporamide A.; Anti-adrenals, selected from the group consisting of aminoglutethimide, mitotane, trilostane; aceglatone; aldophosphamide glycoside; aminolevulinic acid; amsacrine; arabinoside, bestrabucil; bisantrene; edatraxate; defofamine; demecolcine; diaziquone; eflornithine (DFMO), elfomithine; elliptinium acetate, etoglucid; gallium nitrate; gacytosine, hydroxyurea; ibandronate, lentinan; lonidamine; mitoguazone; mitoxantrone; mopidamol; nitracrine; pentostatin; phenamet; pirarubicin; podophyllinic acid; 2-ethylhydrazide; procarbazine; PSK®; razoxane; rhizoxin; sizofiran; spirogermanium; tenuazonic acid; triaziquone; 2, 2',2"-trichlorotriethylamine; trichothecenes (including the group consisting ofT-2 toxin, verrucarin A, roridin A and anguidine); urethane, siRNA, antisense drugs;
(2). an anti-autoimmune disease agent: cyclosporine, cyclosporine A, aminocaproic acid, azathioprine, bromocriptine, chlorambucil, chloroquine, cyclophosphamide, corticosteroids (including the group consisting of amcinonide, betamethasone, budesonide, hydrocortisone, flunisolide, fluticasone propionate, fluocortolone danazol, dexamethasone, Triamcinolone acetonide, beclometasone dipropionate), DHEA, enanercept, hydroxychloroquine, infliximab, meloxicam, methotrexate, mofetil, mycophenylate, prednisone, sirolimus, tacrolimus.
(3). an anti-infectious disease agents comprising:
a). aminoglycosides: amikacin, astromicin, gentamicin (netilmicin, sisomicin, isepamicin), hygromycin B, kanamycin (amikacin, arbekacin, bekanamycin, dibekacin, tobramycin), neomycin (framycetin, paromomycin, ribostamycin), netilmicin, spectinomycin, streptomycin, tobramycin, verdamicin;
b). amphenicols: azidamfenicol, chloramphenicol, florfenicol, thiamphenicol;
c). ansamycins: geldanamycin, herbimycin;
d). carbapenems: biapenem, doripenem, ertapenem, imipenem/cilastatin, meropenem, panipenem;
e). cephems: carbacephem (loracarbef), cefacetrile, cefaclor, cefradine, cefadroxil, cefalonium, cefaloridine, cefalotin or cefalothin, cefalexin, cefaloglycin, cefamandole, cefapirin, cefatrizine, cefazaflur, cefazedone, cefazolin, cefbuperazone, cefcapene, cefdaloxime, cefepime, cefminox, cefoxitin, cefprozil, cefroxadine, ceftezole, cefuroxime, cefixime, cefdinir, cefditoren, cefepime, cefetamet, cefmenoxime, cefodizime, cefonicid, cefoperazone, ceforanide, cefotaxime, cefotiam, cefozopran, cephalexin, cefpimizole, cefpiramide, cefpirome, cefpodoxime, cefprozil, cefquinome, cefsulodin, ceftazidime, cefteram, ceftibuten, ceftiolene, ceftizoxime, ceftobiprole, ceftriaxone, cefuroxime, cefuzonam, cephamycin (cefoxitin, cefotetan, cefmetazole), oxacephem (flomoxef, latamoxef);
f). glycopeptides: bleomycin, vancomycin (oritavancin, telavancin), teicoplanin (dalbavancin), ramoplanin;
g). glycylcyclines: tigecycline;
h). β-lactamase inhibitors: penam (sulbactam, tazobactam), clavam (clavulanic acid);
i). lincosamides: clindamycin, lincomycin;
j). lipopeptides: daptomycin, A54145, calcium-dependent antibiotics (CDA);
k). macrolides: azithromycin, cethromycin, clarithromycin, dirithromycin, erythromycin, flurithromycin, josamycin, ketolide (telithromycin, cethromycin), midecamycin, miocamycin, oleandomycin, rifamycins (rifampicin, rifampin, rifabutin, rifapentine), rokitamycin, roxithromycin, spectinomycin, spiramycin, tacrolimus (FK506), troleandomycin, telithromycin;
l). monobactams: aztreonam, tigemonam;
m). oxazolidinones: linezolid;
n). penicillins: amoxicillin, ampicillin, pivampicillin, hetacillin, bacampicillin, metampicillin, talampicillin, azidocillin, azlocillin, benzylpenicillin, benzathine benzylpenicillin, benzathine phenoxymethylpenicillin, clometocillin, procaine benzylpenicillin, carbenicillin (carindacillin), cloxacillin, dicloxacillin, epicillin, flucloxacillin, mecillinam (pivmecillinam), mezlocillin, meticillin, nafcillin, oxacillin, penamecillin, penicillin, pheneticillin, phenoxymethylpenicillin, piperacillin, propicillin, sulbenicillin, temocillin, ticarcillin;
o). polypeptides: bacitracin, colistin, polymyxin B;
p). quinolones: alatrofloxacin, balofloxacin, ciprofloxacin, clinafloxacin, danofloxacin, difloxacin, enoxacin, enrofloxacin, floxin, garenoxacin, gatifloxacin, gemifloxacin, grepafloxacin, kano trovafloxacin, levofloxacin, lomefloxacin, marbofloxacin, moxifloxacin, nadifloxacin, norfloxacin, orbifloxacin, ofloxacin, pefloxacin, trovafloxacin, grepafloxacin, sitafloxacin, sparfloxacin, temafloxacin, tosufloxacin, trovafloxacin;
q). streptogramins: pristinamycin, quinupristin/dalfopristin;
r). sulfonamides: mafenide, prontosil, sulfacetamide, sulfamethizole, sulfanilimide, sulfasalazine, sulfisoxazole, trimethoprim, trimethoprim-sulfamethoxazole (co-trimoxazole);
s). steroid antibacterials: selected from fusidic acid;
t). tetracyclines: doxycycline, chlortetracycline, clomocycline, demeclocycline, lymecycline, meclocycline, metacycline, minocycline, oxytetracycline, penimepicycline, rolitetracycline, tetracycline, glycylcyclines (including tigecycline);
u). antibiotics: selected from the group consisting of annonacin, arsphenamine, bactoprenol inhibitors (Bacitracin), DADAL/AR inhibitors (cycloserine), dictyostatin, discodermolide, eleutherobin, epothilone, ethambutol, etoposide, faropenem, fusidic acid, furazolidone, isoniazid, laulimalide, metronidazole, mupirocin, mycolactone, NAM synthesis inhibitors (fosfomycin), nitrofurantoin, paclitaxel, platensimycin, pyrazinamide, quinupristin/dalfopristin, rifampicin (rifampin), tazobactam tinidazole, uvaricin;
(4). anti-viral drugs comprising:
a). entry/fusion inhibitors: aplaviroc, maraviroc, vicriviroc, *gp41* (enfuvirtide), PRO 140, CD4 (ibalizumab);
b). integrase inhibitors: raltegravir, elvitegravir, globoidnan A;
c). maturation inhibitors: bevirimat, vivecon;
d). neuraminidase inhibitors: oseltamivir, zanamivir, peramivir;
e). nucleosides &_nucleotides: abacavir, aciclovir, adefovir, amdoxovir, apricitabine, brivudine, cidofovir, clevudine, dexelvucitabine, didanosine (ddl), elvucitabine, emtricitabine (FTC), entecavir, famciclovir, fluorouracil (5-FU), 3'-fluoro-substituted 2', 3'-dideoxynucleoside analogues (including the group consisting of 3'-fluoro-2',3'-dideoxythymidine (FLT) and 3'-fluoro-2',3'-dideoxyguanosine (FLG), fomivirsen, ganciclovir, idoxuridine, lamivudine (3TC), 1-nucleosides (including the group consisting of *β*-l-thymidine and *β*-l-2'-deoxycytidine), penciclovir, racivir, ribavirin, stampidine, stavudine (d4T), taribavirin (viramidine), telbivudine, tenofovir, trifluridine valaciclovir, valganciclovir, zalcitabine (ddC), zidovudine (AZT);
f). non-nucleosides: amantadine, ateviridine, capravirine, diarylpyrimidines (etravirine, rilpivirine), delavirdine, docosanol, emivirine, efavirenz, foscarnet (phosphonoformic acid), imiquimod, interferon alfa, loviride, lodenosine, methisazone, nevirapine, NOV-205, peginterferon alfa, podophyllotoxin, rifampicin, rimantadine, resiquimod (R-848), tromantadine;
g). protease inhibitors: amprenavir, atazanavir, boceprevir, darunavir, fosamprenavir, indinavir, lopinavir, nelfinavir, pleconaril, ritonavir, saquinavir, telaprevir (VX-950), tipranavir;
h). anti-virus drugs: abzyme, arbidol, calanolide a, ceragenin, cyanovirin-n, diarylpyrimidines, epigallocatechin gallate (EGCG), foscarnet, griffithsin, taribavirin (viramidine), hydroxyurea, KP-1461, miltefosine, pleconaril, portmanteau inhibitors, ribavirin, seliciclib.
(5). a radioisotope for radiotherapy that can be selected from the group consisting of (radionuclides) ³H, ¹¹C, ¹⁴C, ¹⁸F, ³²P, ³⁵S, ⁶⁴Cu, ⁶⁸Ga, ⁸⁶Y, ⁹⁹Tc, ¹¹¹In, ¹²³I, ¹²⁴I, ¹²⁵I, ¹³¹I, ¹³³Xe, ¹⁷⁷Lu , ²¹¹At, or ²¹³Bi.
(6). a cell-binding molecule-drug conjugate having a cytotoxic agent of a tubulysin analog, maytansinoid analog, taxanoid (taxane) analog, CC-1065 analog, daunorubicin and doxorubicin compound, amatoxin analog, benzodiazepine dimer (dimers of (pyrrolobenzodiazepine (PBD), tomaymycin, anthramycin, indolinobenzodiazepines, imidazobenzothiadiazepines, or oxazolidinobenzodiazepines)), calicheamicins and the enediyne antibiotic compound, actinomycin, azaserine, bleomycins, epirubicin, tamoxifen, idarubicin, dolastatins, auristatins (monomethyl auristatin E, MMAE, MMAF, auristatin PYE, auristatin TP, Auristatins 2-AQ, 6-AQ, EB (AEB), and EFP (AEFP)), duocarmycins, geldanamycins, methotrexates, thiotepa, vindesines, vincristines, hemiasterlins, nazumamides, microginins, radiosumins, topoisomerase I inhibitors, alterobactins, microsclerodermins, theonellamides, esperamicins, PNU-159682, and their analogues and derivatives above thereof;
(7). pharmaceutically acceptable salts, acids or derivatives of any of the above drugs.
